# EUROPEAN PATENT APPLICATION

(11) **EP 4 361 163 A1**
(43) Date of publication of application: **01.05.2024**
(21) Application number: 22827721.6
(22) Date of filing: 27.06.2022
(51) Int. Cl.: C07F 9/6558, C07D 401/14, C07D 417/14, C07D 405/14, C07D 487/04, A61K 31/675, A61K 31/506, A61K 31/519, A61K 31/501, A61K 31/496, A61K 31/53, A61P 35/00, A61P 35/02

(54) **PROTEIN INHIBITOR OR DEGRADING AGENT, PHARMACEUTICAL COMPOSITION CONTAINING SAME AND PHARMACEUTICAL USE**

(30) Priority: 25.06.2021 CN 202110716210; 24.08.2021 CN 202110979581; 10.03.2022 CN 202210234529; 20.05.2022 CN 202210556400
(71) Applicant: JING MEDICINE TECHNOLOGY (SHANGHAI) LTD., Shanghai 201203 (CN)
(72) Inventor: YE, Guozhong, Shanghai 201203 (CN); DU, Yong, Shanghai 201203 (CN); ZHANG, Yuhua George, Shanghai 201203 (CN); CHEN, Jinju, Shanghai 201203 (CN); CHEN, Shaojun, Shanghai 201203 (CN); LIU, Zhi, Shanghai 201203 (CN); CHEN, Yongfeng, Shanghai 201203 (CN); ZHAO, Cunliang, Shanghai 201203 (CN); LIU, Yao, Shanghai 201203 (CN); FANG, Tao, Shanghai 201203 (CN); YU, Miao, Shanghai 201203 (CN); LU, Xiaorong, Shanghai 201203 (CN); WANG, Yuxia, Shanghai 201203 (CN); WANG, Zhaofu, Shanghai 201203 (CN); WU, Qiang, Shanghai 201203 (CN); KONG, Desheng, Shanghai 201203 (CN); ZHANG, Li, Shanghai 201203 (CN)
(74) Representative: Maiwald GmbH
(86) International application number: PCT/CN2022/101684
(87) International publication number: WO 2022/268229

(57) **Abstract**

A protein inhibitor or degrading agent, a pharmaceutical composition comprising same and a pharmaceutical use. Provided are a compound represented by general formula (I), and a pharmaceutical composition containing same. Also disclosed are an application of the compound of the general formula as a protein inhibitor and/or a protein degrading agent and a pharmaceutical use.

**PIN - Linker - E** **(I)**

## Description

The present application claims the priority to the following applications:
Chinese Patent Application No. 2021107162100 filed on Jun. 25, 2021;
Chinese Patent Application No. 2021109795818 filed on Aug. 24, 2021;
Chinese Patent Application No. 2022102345294 filed on Mar. 10, 2022; and
Chinese Patent Application No. 2022105564005 filed on May 20, 2022,
which are incorporated herein by reference in their entireties.

### TECHNICAL FIELD

The present invention relates to the technical field of pharmaceutics, and particularly, to a PROTAC molecule targeting ALK, ROS1 and EGFR protein and mutein thereof, a pharmaceutical composition, and pharmaceutical use thereof.

### BACKGROUND

PROTAC (proteolysis targeting chimera) has been an emerging hot spot in the research field in recent years. PROTAC molecules can be generally divided into three parts: a war head that binds to a specific target protein, an E3 ligase ligand having ubiquitination functionality, and a linker that connects the two. PROTAC molecules utilize the ubiquitin-proteasome pathway in cells to selectively degrade target proteins. Specifically, since the two ends of PROTAC molecules are ligand fragments of the target protein and E3 ligase, the PROTAC molecule can simultaneously bind to the target protein and E3 ligase, thus promoting ubiquitination of the target protein, such that the target protein is recognized and degraded by a proteasome.

Lung cancer is a serious disease threatening human health, and ranks the first among all malignant tumors in mortality. In patients with non-small cell lung cancer, the proportions of activated mutations of EGFR (epidermal growth factor receptor), ALK (anaplastic lymphoma kinase), and ROS1 (ROS proto-oncogene 1 receptor tyrosine kinase) are approximately 30%, 8% and 2%, respectively.

International Patents Nos. WO2020249048, WO2019113071, WO2019042444, WO2017204445, WO2020069106, WO2019114770, etc., design PROTAC molecules targeting ALK.

Further development of PROTAC molecules having higher efficacy and/or selectivity for diseases resistant to existing EGFR inhibitors, ALK inhibitors, and ROS1 inhibitors may have great research value and potential practical value.

### SUMMARY

The present invention is intended to develop novel compounds that inhibit and induce EGFR degradation. The present invention provides a compound of formula (I), or a pharmaceutically acceptable salt, an enantiomer, a diastereomer, a racemate, a solvate, a hydrate, a polymorph, a prodrug or an isotopic variant thereof, and a mixture thereof,

PIN-Linker-E (I)

wherein PIN is a ligand that binds to EGFR and represents: 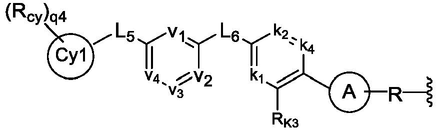 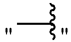 denotes the connection point of PIN with Linker;
ring Cy1 is a C₆₋₁₀ aryl or 5- to 14-membered heteroaryl, and q4 is 0, 1, 2, 3, or 4;
R_{cy} is selected from -O-R_{cy2}, -S-R_{cy2}, -NR₀₀-R_{cy2}, and -(CR¹⁰R¹¹)ₘ₃-R_{cy2};
R_{cy2} is selected from hydrogen, deuterium, cyano, carboxy, nitro, halogen, C₁₋₁₀ alkyl, C₁₋₁₀ haloalkyl, C₁₋₁₀ hydroxyalkyl, C₁₋₁₀ alkoxy, C₁₋₁₀ alkylthio, C₂₋₁₀ alkenyl, C₂₋₁₀ alkynyl, NRₐR_{b}, -C(O)R¹², -S(O)R¹², SO₂R¹², - C(O)NR³¹R³², -S(O)NR³¹R³², -SO₂NR³¹R³², -NR₀₀C(O)R¹², -NR₀₀S(O)R¹², -NR₀₀SO₂R¹², - NR₀₀C(O)NR³¹R³², -NR₀₀S(O)NR³¹R³², -NR₀₀SO₂NR³¹R³², -C(O)OR¹², -S(O)OR¹², S(O)₂OR¹², -OC(O)R¹², -OS(O)R¹², -OS(O)₂R¹², -OC(O)OR¹², -OS(O)OR¹², -OS(O)₂OR¹², -P(O)R³¹R³², C₃₋₈ cycloalkyl, 3- to 8-membered heterocyclyl, C₆₋₁₀ aryl, or 5- to 14-membered heteroaryl, wherein the C₁₋₁₀ alkyl, C₁₋₁₀ haloalkyl, C₁₋₁₀ hydroxyalkyl, C₁₋₁₀ alkoxy, C₁₋₁₀ alkylthio, C₂₋₁₀ alkenyl, C₂₋₁₀ alkynyl, C₃₋₈ cycloalkyl, 3- to 8-membered heterocyclyl, C₆₋₁₀ aryl, and 5- to 14-membered heteroaryl are optionally substituted with 0, 1, 2, 3, or 4 substituents selected from R²¹;
R¹⁰, R¹¹, R¹² , R³¹, and R³² are each independently selected from the group consisting of hydrogen, deuterium, hydroxy, cyano, carboxy, nitro, halogen, acetyl, -C(O)NRₐR_{b}, NRₐR_{b}, -C(O)R¹³, -S(O)R¹³, SO₂R¹³, - C(O)NRₐR_{b}, -S(O)NRₐR_{b}, -SO₂NRₐR_{b}, C₁₋₁₀ alkyl, C₁₋₁₀ haloalkyl, C₁₋₁₀ hydroxyalkyl, C₁₋₁₀ alkoxy, C₁₋₁₀ alkylthio, C₂₋₁₀ alkenyl, C₂₋₁₀ alkynyl, C₃₋₈ cycloalkyl, 3- to 8-membered heterocyclyl, C₆₋₁₀ aryl, or 5- to 14-membered heteroaryl; or, R¹⁰ and R¹¹, together with the C atom to which they are attached, form a C₃₋₈ cycloalkyl or 3- to 8-membered heterocyclyl, wherein the C₁₋₁₀ alkyl, C₁₋₁₀ haloalkyl, C₁₋₁₀ hydroxyalkyl, C₁₋₁₀ alkoxy, C₁₋₁₀ alkylthio, C₂₋₁₀ alkenyl, C₂₋₁₀ alkynyl, C₃₋₈ cycloalkyl, 3- to 8-membered heterocyclyl, C₆₋₁₀ aryl and 5- to 14-membered heteroaryl, and the C₃₋₈ cycloalkyl or 3- to 8-membered heterocyclyl is optionally substituted with 1, 2, 3, or 4 substituents selected from R²¹;
m3 is 0, 1, 2, 3, 4, 5, 6, 7, 8, 9, or 10;
V₁ is N or CR₁, V₂ is N or CR₂, V₃ is N or CR₃, and V₄ is N or CR₄;
R₁, R₂, R₃, and R₄ are each independently selected from: -X²-(CR¹⁸₂)_{q1}-X³-(CR¹⁴₂)_{q2}-R¹⁵;
X² and X³ are each independently selected from: a bond, O, S, NR₀₀, -C(O)-, -S(O)-, SO₂, -C(O)NR₀₀-, - S(O)NR₀₀-, -SO₂NR₀₀-, -NR₀₀C(O)-, -NR₀₀SO₂-, -NR₀₀C(O)NR₀₀-, -NR₀₀SO₂NR₀₀-, -NR₀₀C(O)O-, - OC(O)O-, -C(O)O-, -OC(O)-, -SO₂-O-, -OSO₂-, -OSO₂O-, C₂₋₁₀ alkenylene, C₂₋₁₀ alkynylene, -(CR¹⁶R¹⁷)_{q3}-, C₃₋₈ cycloalkylene, 3- to 8-membered heterocyclylene, C₆₋₁₀ arylene, or 5- to 14-membered heteroarylene, wherein the C₂₋₁₀ alkenylene, C₂₋₁₀ alkynylene, C₃₋₈ cycloalkylene, 3- to 8-membered heterocyclylene, C₆₋₁₀ arylene, and 5- to 14-membered heteroarylene are optionally substituted with 1, 2, 3, or 4 substituents selected from R²¹;
R¹⁴, R¹⁵, R¹⁶, R¹⁷, and R¹⁸ are each independently selected from: hydrogen, deuterium, hydroxy, cyano, carboxy, nitro, halogen, NRₐR_{b}, -C(O)NRₐR_{b}, acetyl, C₁₋₁₀ alkyl, C₁₋₁₀ haloalkyl, C₁₋₁₀ alkoxy, C₁₋₁₀ alkylthio, C₂₋₁₀ alkenyl, C₂₋₁₀ alkynyl, C₃₋₈ cycloalkyl, 3- to 8-membered heterocyclyl, C₆₋₁₀ aryl, or 5- to 14-membered heteroaryl, wherein the C₁₋₁₀ alkyl, C₁₋₁₀ haloalkyl, C₁₋₁₀ alkoxy, C₁₋₁₀ alkylthio, C₂₋₁₀ alkenyl, C₂₋₁₀ alkynyl, C₃₋₈ cycloalkyl, 3- to 8-membered heterocyclyl, C₆₋₁₀ aryl, or 5- to 14-membered heteroaryl is optionally substituted with 1, 2, 3, or 4 substituents selected from R²¹;
or, V₃ and V₄, together with the substituents to which they are attached, form a 5- to 6-membered heteroaryl or benzene; or V₂ and V₃, together with the substituents to which they are attached, form a 5- to 6-membered heteroaryl or benzene, wherein the 5- to 6-membered heteroaryl or benzene is optionally substituted with 1, 2, 3, or 4 substituents selected from R²¹;
K₁ is N or CRₖ₁, K₂ is N or CRₖ₂, and K₄ is N or CRₖ₄,
Rₖ₁, Rₖ₂, and Rₖ₄ are each independently selected from hydrogen, deuterium, hydroxy, cyano, acetyl, - C(O)NRₐR_{b}, halogen, C₁₋₁₀ alkyl, C₁₋₅ alkylene-OC₁₋₅ alkyl, C₁₋₁₀ haloalkyl, C₁₋₁₀ alkoxy, C₁₋₁₀ alkylthio, C₂₋₁₀ alkenyl, C₂₋₁₀ alkynyl, NRₐR_{b}, C₃₋₈ cycloalkyl, C₃₋₈ heterocyclyl, -O-C₃₋₈ cycloalkyl, -O-3- to 8-membered heterocyclyl, C₆₋₁₀ aryl, and 5- to 14-membered heteroaryl;
Rₖ₃ is not hydrogen, and Rₖ₃ represents -X₁-Rx,
X₁ is selected from O, S, NR₀₀, -C(O)-, -S(O)-, SOz, -C(O)NR₀₀-, -NR₀₀C(O)-, -SO₂NR₀₀-, -NR₀₀SO₂-, - NR₀₀C(O)NR₀₀-, -NR₀₀C(O)O-, -OC(O)O-, -C(O)O-, -OC(O)-, -CH=CH-, -C≡C-, -(CRₓ₁Rₓ₂)ₓ-, C₃₋₈ cycloalkylene, 3- to 8-membered heterocyclylene, C₆₋₁₀ arylene, and 5- to 14-membered heteroarylene;
Rx represents -(CRₓ₃Rₓ₄)ₓ₂-Rₓₙ,
Rₓ₁, Rₓ₂, Rₓ₃, Rₓ₄, and Rₓₙ are each independently selected from hydrogen, deuterium, cyano, hydroxy, acetyl, halogen, nitro, formyl, carboxy, amino, C₁₋₁₀ alkyl, C₁₋₁₀ haloalkyl, C₁₋₁₀ alkoxy, C₁₋₁₀ alkylthio, C₂₋₁₀ alkenyl, C₂₋₁₀ alkynyl, C₃₋₈ cycloalkyl, 3- to 8-membered heterocyclyl, C₆₋₁₀ aryl, and 5- to 14-membered heteroaryl; or, Rₓ₁ and Rₓ₂, together with the C atom to which they are attached, form a 3- to 8-membered cycloalkyl or 3- to 8-membered heterocyclyl;
x and x2 are each independently 0, 1, 2, 3, 4, or 5, and x and x2 are not both 0;
or, R_{J3} and Rₖ₃ are connected to form a bond;
L₅ and L₆ are each independently selected from a bond, O, S, NR₀₀, C(O)NR₀₀, NR₀₀C(O), C₁₋₁₀ alkylene, C₁₋₁₀ haloalkylene, C₁₋₁₀ alkyleneoxy, C₂₋₁₀ alkenylene, or C₂₋₁₀ alkynylene, wherein, when L₅ is NH, at least one Rcy is selected from -S(O)R¹², SO₂R¹², -C(O)NR³¹R³², -S(O)NR³¹R³², -SO₂NR³¹R³², -NR₀₀C(O)R¹²,-NR₀₀S(O)R¹², -NR₀₀SO₂R¹², -NR₀₀C(O)NR³¹R³², -NR₀₀S(O)NR³¹R³², -NR₀₀SO₂NR³¹R³², and -P(O)R³¹R³²; ring A is a bond, or a saturated or unsaturated monocyclic alkylene, monocyclic heterocyclylene, polycyclic alkylene or polycyclic heterocyclylene; or, when ring A is present and is a substituted ring, Rₖ₃ and the ring A substituent, together with the ring atoms to which they are attached, form a C₅₋₇ cycloalkyl or 6- to 8-membered heterocyclyl;
R is a bond, or a saturated or unsaturated monocyclic alkylene, monocyclic heterocyclylene, polycyclic alkylene or polycyclic heterocyclylene;
Linker is absent, or represents: a C₁₋₂₀ alkylene chain, and any one or more methylene groups may optionally be substituted by one or more R^{L}s, R^{L}s are identical or different, and R^{L} is selected from a bond, O, S, NR₀₀, - C(O)-, SO, SOz, -C(O)NR₀₀-, -NR₀₀C(O)-, -SO₂NR₀₀-, -NR₀₀SO₂-, -NR₀₀C(O)NR₀₀-, -NR₀₀C(O)O-, -OC(O)-, -C(O)O-, -OC(O)O-, -CR₀₀=CR₀₀-, -C≡C-, -(CH₂)ᵣ-, C₃₋₈ cycloalkylene, 3- to 8-membered heterocyclylene, C₆₋₁₀ arylene, and 5- to 14-membered heteroarylene; and any one methylene group may be substituted by 1 or 2 R^{L'}s, and R^{L'}s are identical or different and are hydrogen, deuterium, halogen, amino, nitro, cyano, acetyl, C₁₋₁₀ alkyl, C₁₋₁₀ alkoxy, C₁₋₁₀ haloalkyl, C₃₋₈ cycloalkyl, 3- to 8-membered heterocyclyl, C₆₋₁₀ aryl, or 5- to 14-membered heteroaryl, or two R^{L'}s, together with the C atoms to which they are attached, form a C₃₋₈ cycloalkyl or 3- to 8-membered heterocyclyl; r is 1, 2, 3, 4, or 5;
E represents: 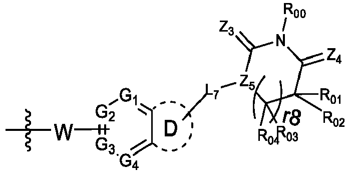
wherein G₁ is N or CR_{G1}, G₂ is N or CR_{G2}, G₃ is N or CR_{G3}, and G₄ is N or CR_{G4};
one of R_{G1}, R_{G2}, R_{G3}, and R_{G4} is attached to W, and the others are each independently selected from the group consisting of hydrogen, deuterium, hydroxy, cyano, acetyl, acylamino, halogen, C₁₋₁₀ alkyl, C₁₋₁₀ haloalkyl, C₁₋₁₀ alkoxy, C₁₋₁₀ alkylthio, NRₐR_{b}, C₃₋₈ cycloalkyl, 3- to 8-membered heterocyclyl, C₆₋₁₀ aryl, and 5- to 14-membered heteroaryl;
ring D is absent, such that L₇ is attached to a C atom or an N atom on the aromatic ring on which G₁ is located, or ring D is a substituted or unsubstituted C₆₋₁₀ aryl or a substituted or unsubstituted 5- to 14-membered heteroaryl; the substitution refers to that the C₆₋₁₀ aryl or 5- to 14-membered heteroaryl is optionally substituted, within their respective valence-permitted ranges, with 1, 2, or 3 substituents selected from hydrogen, deuterium, halogen, C₁₋₁₀ alkyl, C₁₋₁₀ alkoxy, C₁₋₁₀ haloalkyl, C₁₋₁₀ haloalkoxy, C₂₋₁₀ alkenyl, C₂₋₁₀ alkynyl, cyano, nitro, acylamino, amino, and acetyl;
or, ring D is 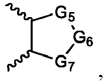
G₅, G₆, and G₇ are O, S, N, or C atoms optionally substituted by 1 or 2 R^{G}s; R^{G} is hydrogen, deuterium, hydroxy, amino, cyano, acetyl, carboxy, nitro, halogen, C₁₋₁₀ alkyl, C₁₋₁₀ haloalkyl, C₁₋₁₀ alkoxy, C₁₋₁₀ alkylthio, C₂₋₁₀ alkenyl, C₂₋₁₀ alkynyl, C₃₋₈ cycloalkyl, 3- to 8-membered heterocyclyl, C₆₋₁₀ aryl, or 5- to 14-membered heteroaryl; or two R^{G}s, together with the C atoms to which they are attached, form C=O, 3- to 8-membered cycloalkyl, or 3- to 8-membered heterocyclyl;
Z₃ and Z₄ are each independently selected from O, S, and NR₀₀; Z₅ is CR₀₀ or N; or the ring in which Z₅ is located is absent;
L₇ is a bond, O, S, NR₀₀, C(O)NR₀₀, NR₀₀C(O), C₁₋₅ alkylene, C₁₋₅ haloalkylene, C₁₋₅ alkyleneoxy, C₂₋₆ alkenylene, or C₂₋₆ alkynylene;
W is a bond, O, S, NR₀₀, -C(O)-, -S(O)-, SO₂, -C(O)NR₀₀-, -NR₀₀C(O)-, -SO₂NR₀₀-, -NR₀₀SO₂-, - NR₀₀C(O)NR₀₀-, -NR₀₀C(O)O-, -OC(O)O-, -C(O)O-, -OC(O)-, -CR₀₀=CR₀₀-, -C≡C-, -(CR₂₃R₂₄)ᵣ₂-, C₃₋₈ cycloalkylene, 3- to 8-membered heterocyclylene, C₆₋₁₀ arylene, or 5- to 14-membered heteroarylene;
Rₐ, R_{b}, R₀₀, R₀₁, R₀₂, R₀₃, R₀₄, R₂₃, R₂₄, R¹³, and R²¹ are each independently selected from hydrogen, deuterium, cyano, hydroxy, carboxy, nitro, halogen, C₁₋₁₀ alkyl, C₁₋₁₀ haloalkyl, C₁₋₁₀ hydroxyalkyl, C₁₋₁₀ alkoxy, C₁₋₁₀ alkylthio, NR_{c}R_{d}, C(O)NR_{c}R_{d}, C(O)R_{d}, -SO₂NR_{c}R_{d}, C₃₋₈ cycloalkyl, 3- to 8-membered heterocyclyl, C₆₋₁₀ aryl, 5- to 14-membered heteroaryl, -OC₃₋₈ cycloalkyl, -O-3- to 8-membered heterocyclyl, -O-C₁₋₁₀ alkylene-C₃₋₈ cycloalkyl, -O-C₁₋₁₀ alkylene-3- to 8-membered heterocyclyl, -C₁₋₁₀ alkylene-NR_{c}R_{d}, -C₁₋₁₀ alkylene-C(O)NR_{c}R_{d}, -C₁₋₁₀ alkylene-C(O)R_{d}, -C₁₋₁₀ alkylene-C₃₋₈ cycloalkyl, -C₁₋₁₀ alkylene-3- to 8-membered heterocyclyl, -C₁₋₁₀ alkylene-C₆₋₁₀ aryl, and -C₁₋₁₀ alkylene-5- to 14-membered heteroaryl;
or, R₀₁, R₀₂ or R₀₃, R₀₄ or R₂₃, and R₂₄, together with the C atoms to which they are attached, form a 3- to 8-membered cycloalkyl or 3- to 8-membered heterocyclyl; or Rₐ and R_{b}, together with the N atom to which they are attached, form a 3- to 8-membered heterocyclyl;
r2 is 0, 1, 2, 3, or 4;
R_{c} and R_{d} are each independently hydrogen, deuterium, C₁₋₅ alkyl, C₁₋₅ alkoxy, or C₁₋₅ haloalkyl; or R_{c} and R_{d}, together with the N atom to which they are attached, form a 3- to 8-membered heterocyclyl;
the above C₁₋₁₀ alkyl, C₁₋₁₀ haloalkyl, C₁₋₁₀ alkoxy, C₁₋₁₀ alkylthio, C₁₋₅ alkyl, C₁₋₅ haloalkyl, C₁₋₅ alkoxy, C₃₋₈ cycloalkyl, 3- to 8-membered heterocyclyl, C₆₋₁₀ aryl or 5- to 14-membered heteroaryl, or saturated or unsaturated monocycle, monocyclic heterocycle, polycycle or polycyclic heterocycle is unsubstituted or, within a valence-permitted range, each independently substituted by 1, 2, 3, 4, or more groups selected from the following B2 groups, and the B2 group is selected from hydrogen, deuterium, cyano, hydroxy, carboxy, nitro, halogen, C₁₋₁₀ alkyl, C₁₋₁₀ haloalkyl, C₁₋₁₀ hydroxyalkyl, C₁₋₁₀ alkoxy, C₁₋₁₀ alkylthio, NRₑR_{f}, C(O)NRₑR_{f}, C(O)R_{g}, -SO₂NRₑR_{f}, C₃₋₈ cycloalkyl, 3- to 8-membered heterocyclyl, C₆₋₁₀ aryl, 5- to 14-membered heteroaryl, -OC₃₋₈ cycloalkyl, -O-3- to 8-membered heterocyclyl, -O-C₁₋₁₀ alkylene-C₃₋₈ cycloalkyl, -O-C₁₋₁₀ alkylene-3-to 8-membered heterocyclyl, -C₁₋₁₀ alkylene-NRₑR_{f}, -C₁₋₁₀ alkylene-C(O)NRₑR_{f}, -C₁₋₁₀ alkylene-C(O)R_{g}, -C₁₋₁₀ alkylene-C₃₋₈ cycloalkyl, -C₁₋₁₀ alkylene-3- to 8-membered heterocyclyl, -C₁₋₁₀ alkylene-C₆₋₁₀ aryl, and -C₁₋₁₀ alkylene-5- to 14-membered heteroaryl, wherein Rₑ, R_{f}, and R_{g} are each independently hydrogen, deuterium, C₁₋₅ alkyl, C₁₋₅ alkoxy, or C₁₋₅ haloalkyl.

The present invention further provides a compound of formula (I), or a pharmaceutically acceptable salt, an enantiomer, a diastereomer, a racemate, a solvate, a hydrate, a polymorph, a prodrug or an isotopic variant thereof, and a mixture thereof,

PIN-Linker-E (I)

wherein E is a compound capable of binding to ubiquitin ligase;
Linker is a connecting part and is covalently connected with at least one PIN and at least one E;
wherein,
PIN represents: 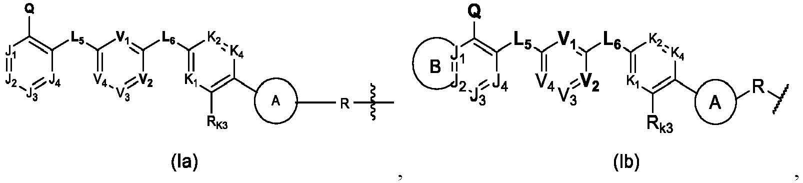 or 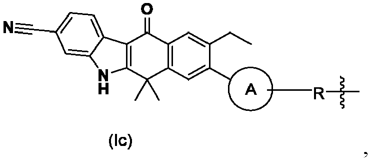
wherein Q is C(R^{q1})₃, C(O)R^{q1}, S(O)R^{q1}, SO₂R^{q1}, P(O)R^{q1}R^{q2}, or NR^{q1}R^{q2}, and R^{q1} and R^{q2} are each independently selected from hydrogen, amino, -C₁₋₅ alkylene-R_{qn}, -C₁₋₅ haloalkylene-R_{qn}, -C₁₋₅ alkyleneoxy-R_{qn}, -C₃₋₈ cycloalkylene-R_{qn}, -3- to 8-membered heterocycloalkylene-R_{qn}, -C(O)R_{qn}, -S(O)R_{qn}, SO₂R_{qn}, - C(O)NR₀₀R_{qn}, -NR₀₀C(O)R_{qn}, and -SO₂NR₀₀R_{qn};
R_{qn} is selected from hydrogen, C₁₋₁₀ alkyl, C₁₋₁₀ haloalkyl, C₁₋₁₀ alkoxy, C₁₋₁₀ alkylthio, C₂₋₁₀ alkenyl, C₂₋₁₀ alkynyl, NRₐR_{b}, C₃₋₈ cycloalkyl, 3- to 8-membered heterocycloalkyl, C₆₋₁₀ aryl, and 5- to 14-membered heteroaryl;
J₁ is N or CR_{J1}, J₂ is N or CR_{J2}, J₃ is N or CR_{J3}, and J₄ is N or CR_{J4};
V₁ is N or CR₁, V₂ is N or CR₂, V₃ is N or CR₃, and V₄ is N or CR₄;
R₁, R₂, R₃, R₄, R_{J1}, R_{J2}, R_{J3}, and R_{J4} are each independently selected from hydrogen, hydroxy, cyano, acetyl, carboxy, nitro, halogen, amino, C₁₋₁₀ alkyl, C₁₋₁₀ haloalkyl, C₁₋₁₀ alkoxy, C₁₋₁₀ alkylthio, C₂₋₁₀ alkenyl, C₂₋₁₀ alkynyl, NRₐR_{b}, C₃₋₈ cycloalkyl, 3- to 8-membered heterocycloalkyl, C₆₋₁₀ aryl, and 5- to 14-membered heteroaryl;
or, R₃ and R₄, together with the atoms to which they are attached, form a substituted or unsubstituted 5- to 6-membered heteroaryl or benzene;
or, R_{J2} and R_{J3}, together with the atoms to which they are attached, form a substituted or unsubstituted 5- to 6-membered heteroaryl or benzene, or R_{J3} and R_{J4}, together with the atoms to which they are attached, form a substituted or unsubstituted 5- to 6-membered heteroaryl or benzene; the substitution refers to that the 5- to 6-membered heteroaryl or benzene is optionally substituted with 1, 2, 3, or 4 substituents selected from hydrogen, halogen, amino, cyano, acetyl, C₁₋₅ alkyl, C₁₋₅ alkoxy, C₁₋₃ haloalkyl, C₃₋₈ cycloalkyl, and 3- to 8-membered heterocycloalkyl;
K₁ is N or CRₖ₁, K₂ is N or CRₖ₂, and K₄ is N or CRₖ₄,
Rₖ₁, Rₖ₂, and Rₖ₄ are identical or different, and are each independently selected from hydrogen, hydroxy, cyano, acetyl, -C(O)NRₐR_{b}, halogen, C₁₋₁₀ alkyl, C₁₋₅ alkylene-OC₁₋₅ alkyl, C₁₋₁₀ haloalkyl, C₁₋₁₀ alkoxy, C₁₋₁₀ alkylthio, C₂₋₁₀ alkenyl, C₂₋₁₀ alkynyl, NRₐR_{b}, C₃₋₈ cycloalkyl, 3- to 8-membered heterocycloalkyl, -O-C₃₋₈ cycloalkyl, -O-3- to 8-membered heterocycloalkyl, C₆₋₁₀ aryl, and 5- to 14-membered heteroaryl;
Rₖ₃ is not hydrogen, and Rₖ₃ represents -X₁-Rx,
X₁ is selected from O, S, NR₀₀, -C(O)-, -S(O)-, SOz, -C(O)NR₀₀-, -NR₀₀C(O)-, -SO₂NR₀₀-, -NR₀₀SO₂-, - NR₀₀C(O)NR₀₀-, -NR₀₀C(O)O-, -OC(O)O-, -C(O)O-, -OC(O)-, -CH=CH-, -C≡C-, -(CRₓ₁Rₓ₂)ₓ-, C₃₋₈ cycloalkylene, 3- to 8-membered heterocycloalkylene, C₆₋₁₀ arylene, and 5- to 14-membered heteroarylene; Rx represents -(CRₓ₃Rₓ₄)ₓ₂-Rₓₙ or deuterium,
Rₓ₁, Rₓ₂, Rₓ₃, Rₓ₄, and Rₓₙ are each independently selected from hydrogen, cyano, hydroxy, acetyl, halogen, nitro, formyl, carboxy, amino, C₁₋₁₀ alkyl, C₁₋₁₀ haloalkyl, C₁₋₁₀ alkoxy, C₁₋₁₀ alkylthio, C₂₋₁₀ alkenyl, C₂₋₁₀ alkynyl, C₃₋₈ cycloalkyl, 3- to 8-membered heterocycloalkyl, C₆₋₁₀ aryl, and 5- to 14-membered heteroaryl; or, Rₓ₁ and Rₓ₂, together with the C atom to which they are attached, form a 3- to 8-membered cycloalkyl or 3- to 8-membered heterocycloalkyl;
x and x2 are each independently 0, 1, 2, 3, 4, or 5, and x and x2 are not both 0;
or, R_{J3} and Rₖ₃ are connected to form a bond;
L₅ and L₆ are each independently selected from O, S, NR₀₀, C(O)NR₀₀, NR₀₀C(O), C₁₋₁₀ alkylene, C₁₋₁₀ haloalkylene, C₁₋₁₀ alkyleneoxy, C₂₋₁₀ alkenylene, and C₂₋₁₀ alkynylene;
ring A is a saturated or unsaturated monocyclic alkylene, monocyclic heterocyclylene, polycyclic alkylene, or polycyclic heterocyclylene;
ring B is a saturated or unsaturated monocyclic alkylene, monocyclic heterocyclylene, polycyclic alkylene, or polycyclic heterocyclylene;
R is a bond, a saturated or unsaturated monocyclic alkylene, monocyclic heterocyclylene, polycyclic alkylene or polycyclic heterocyclylene, or a 7- to 11-membered mono-spiroheterocyclylene, 3- to 8-membered monocyclic heterocyclylene and 8- to 10-membered bicyclic heteroarylene substituted with 1, 2, or 3 R₀s, wherein R₀ is selected from hydrogen, deuterium, halogen, =O, C₁₋₆ alkyl, C₁₋₆ haloalkyl, C₁₋₆ alkoxy, C₃₋₈ cycloalkyl, and 3- to 8-membered heterocyclyl, or two R₀s, together with the C atom to which they are attached, form a 3- to 6-membered cycloalkyl; the heteroatom of the mono-spiroheterocyclylene is selected from one or more of nitrogen, oxygen, and sulfur, and the number of the heteroatom is 1, 2, or 3; the heteroatom of the bicyclic heteroarylene is selected from one or more of nitrogen, oxygen, and sulfur, and the number of the heteroatom is 1, 2, or 3; the heteroatom of the monocyclic heterocyclylene is selected from one or more of nitrogen, oxygen, and sulfur, and the number of the heteroatom is 1, 2, or 3; the heteroatom of the 3- to 8-membered heterocyclyl is selected from one or more of nitrogen, oxygen, and sulfur, and the number of the heteroatom is 1, 2, or 3;
Linker is absent, or represents: a C₁₋₂₀ alkylene chain, and any one or more methylene groups may optionally be substituted by one or more R^{L}s, R^{L}s are identical or different, and R^{L} is selected from O, S, NR₀₀, -C(O)-, SO, SOz, -C(O)NR₀₀-, -NR₀₀C(O)-, -SO₂NR₀₀-, -NR₀₀SO₂-, -NR₀₀C(O)NR₀₀-, -NR₀₀C(O)O-, -OC(O)-, C(O)O-, -OC(O)O-, -CR₀₀=CR₀₀-, -C≡C-, -(CH₂)ᵣ-, C₃₋₈ cycloalkylene or 3- to 8-membered heterocyclylene, C₆₋₁₀ arylene, and 5- to 14-membered heteroarylene; and any one methylene group may be substituted by 1 or 2 R^{L'}s, and R^{L'}s are identical or different and are hydrogen, halogen, amino, nitro, cyano, acetyl, C₁₋₁₀ alkyl, C₁₋₁₀ alkoxy, C₁₋₁₀ haloalkyl, C₃₋₈ cycloalkyl, 3- to 8-membered heterocyclyl, C₆₋₁₀ aryl, or 5- to 14-membered heteroaryl, or two R^{L'}s, together with the C atoms to which they are attached, form a C₃₋₈ cycloalkyl or 3- to 8-membered heterocyclyl; r is 1, 2, 3, 4, or 5;
E represents: 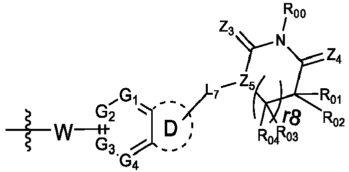
wherein G₁ is N or CR_{G1}, G₂ is N or CR_{G2}, G₃ is N or CR_{G3}, and G₄ is N or CR_{G4};
one of R_{G1}, R_{G2}, R_{G3}, and R_{G4} is attached to W, and the others are each independently selected from the group consisting of hydrogen, hydroxy, cyano, acetyl, acylamino, halogen, C₁₋₁₀ alkyl, C₁₋₁₀ haloalkyl, C₁₋₁₀ alkoxy, C₁₋₁₀ alkylthio, NRₐR_{b}, C₃₋₈ cycloalkyl, 3- to 8-membered heterocyclyl, C₆₋₁₀ aryl, and 5- to 14-membered heteroaryl;
ring D is absent, such that L₇ is attached to a C atom or an N atom on the aromatic ring on which G₁ is located, or ring D is a substituted or unsubstituted C₆₋₁₀ aryl or a substituted or unsubstituted 5- to 14-membered heteroaryl; the substitution refers to that the C₆₋₁₀ aryl or 5- to 14-membered heteroaryl is optionally substituted, within their respective valence-permitted ranges, with 1, 2, or 3 substituents selected from hydrogen, halogen, C₁₋₁₀ alkyl, C₁₋₁₀ alkoxy, C₁₋₁₀ haloalkyl, C₁₋₁₀ haloalkoxy, C₂₋₁₀ alkenyl, C₂₋₁₀ alkynyl, cyano, nitro, acylamino, amino, and acetyl;
or, ring D is 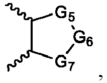
G₅, G₆, and G₇ are O, S, N, or C atoms optionally substituted by 1 or 2 R^{G}s; R^{G} is hydrogen, hydroxy, amino, cyano, acetyl, carboxy, nitro, halogen, C₁₋₁₀ alkyl, C₁₋₁₀ haloalkyl, C₁₋₁₀ alkoxy, C₁₋₁₀ alkylthio, C₂₋₁₀ alkenyl, C₂₋₁₀ alkynyl, C₃₋₈ cycloalkyl, 3- to 8-membered heterocyclyl, C₆₋₁₀ aryl, or 5- to 14-membered heteroaryl; or two R^{G}s, together with the C atoms to which they are attached, form C=O, 3- to 8-membered cycloalkyl, or 3-to 8-membered heterocyclyl;
Z₃ and Z₄ are each independently selected from O, S, or NR₀₀; Z₅ is CR₀₀ or N; or the ring in which Z₅ is located is absent;
L₇ is a bond, O, S, NR₀₀, C(O)NR₀₀, NR₀₀C(O), C₁₋₅ alkylene, C₁₋₅ haloalkylene, C₁₋₅ alkyleneoxy, C₂₋₆ alkenylene, or C₂₋₆ alkynylene;
W is a bond, O, S, NR₀₀, -C(O)-, -S(O)-, SO₂, -C(O)NR₀₀-, -NR₀₀C(O)-, -SO₂NR₀₀-, -NR₀₀SO₂-, - NR₀₀C(O)NR₀₀-, -NR₀₀C(O)O-, -OC(O)O-, -C(O)O-, -OC(O)-, -CR₀₀=CR₀₀-, -C≡C-, -(CR₂₃R₂₄)ᵣ₂-, C₃₋₈ cycloalkylene, 3- to 8-membered heterocyclylene, C₆₋₁₀ arylene, or 5- to 14-membered heteroarylene;
Rₐ, R_{b}, R₀₀, R₀₁, R₀₂, R₀₃, R₀₄, R₂₃, and R₂₄ are each independently selected from hydrogen, cyano, acetyl, hydroxy, carboxy, nitro, halogen, C₁₋₁₀ alkyl, C₁₋₁₀ haloalkyl, C₁₋₁₀ alkoxy, C₁₋₁₀ alkylthio, NR_{c}R_{d}, C₃₋₈ cycloalkyl, 3- to 8-membered heterocyclyl, C₆₋₁₀ aryl, and 5- to 14-membered heteroaryl;
or R₀₁ and R₀₂, or R₀₃ and R₀₄, together with the C atom to which they are attached, form a 3- to 8-membered cycloalkyl or 3- to 8-membered heterocycloalkyl; or R₂₃ and R₂₄, together with the C atom to which they are attached, form a 3- to 8-membered cycloalkyl or 3- to 8-membered heterocyclyl; or Rₐ and R_{b}, together with the N atom to which they are attached, form a 3- to 8-membered heterocyclyl;
r2 is 0, 1, 2, 3, or 4;
R_{c} and R_{d} are each independently hydrogen, C₁₋₅ alkyl, C₁₋₅ alkoxy, or C₁₋₅ haloalkyl; or R_{c} and R_{d}, together with the N atom to which they are attached, form a 3- to 8-membered heterocyclyl;
the above C₁₋₁₀ alkyl, C₁₋₁₀ haloalkyl, C₁₋₁₀ alkoxy, C₁₋₁₀ alkylthio, C₁₋₅ alkyl, C₁₋₅ haloalkyl, C₁₋₅ alkoxy, C₃₋₈ cycloalkyl, 3- to 8-membered heterocyclyl, C₆₋₁₀ aryl, or 5- to 14-membered heteroaryl, saturated or unsaturated monocycle, monocyclic heterocycle, polycycle or polycyclic heterocycle are unsubstituted or, within a valence-permitted range, each independently substituted by 1, 2, 3, 4, or more groups selected from the following B2 groups, and the B2 group is selected from hydrogen, =O, cyano, acetyl, hydroxy, carboxy, nitro, halogen, C₁₋₅ alkyl, C₁₋₅ alkoxy, C₁₋₅ alkylthio, NRₑR_{f}, 3- to 8-membered cycloalkyl, 3- to 8-membered heterocyclyl, C₆₋₁₀ aryl, or 5- to 14-membered heteroaryl, wherein Rₑ and R_{f} are each independently hydrogen, C₁₋₅ alkyl, C₁₋₅ alkoxy, and C₁₋₅ haloalkyl;
provided that:
   when PIN is formula (Ia) and Q is S(O)₂-isopropyl, neither Rₖ₃ nor Rₖ₄ is methyl;
   when PIN is formula (Ib), ring B is 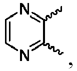 Q is P(O)(CH₃)₂, J₁, J₂, J₃, J₄, V₃ and K₁ are CH, and V₁ and V₂ are N, R₄ is not bromine, or neither Rₖ₃ nor Rₖ₄ is methyl;
   when PIN is formula (Ic) and A-R is 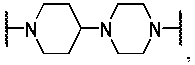 Linker is an all-carbon chain system, W contains no heteroatoms, and Linker is not methylene, ethylene and propylene.

In one aspect, the present invention provides a compound of formula (I), or a pharmaceutically acceptable salt, an enantiomer, a diastereomer, a racemate, a solvate, a hydrate, a polymorph, a prodrug or an isotopic variant thereof, and a mixture thereof,

PIN-Linker-E (I)

wherein, PIN represents: 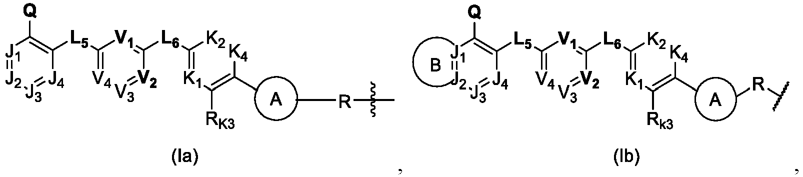 or 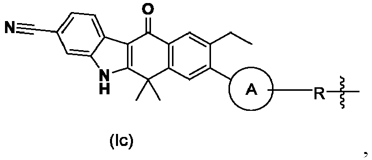 and
E represents: 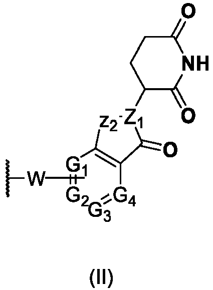
wherein Q is C(R^{q1})₃, C(O)R^{q1}, S(O)R^{q1}, SO₂R^{q1}, P(O)R^{q1}R^{q2}, or NR^{q1}R^{q2}, and R^{q1} and R^{q2} are each independently selected from hydrogen, amino, -C₁₋₅ alkylene-R_{qn}, -C₁₋₅ haloalkylene-R_{qn}, -C₁₋₅ alkyleneoxy-R_{qn}, -C₃₋₈ cycloalkylene-R_{qn}, -3- to 8-membered heterocycloalkylene-R_{qn}, -C(O)R_{qn}, -S(O)R_{qn}, SO₂R_{qn}, - C(O)NR₀₀R_{qn}, -NR₀₀C(O)R_{qn}, and -SO₂NR₀₀R_{qn};
R_{qn} is selected from hydrogen, C₁₋₁₀ alkyl, C₁₋₁₀ haloalkyl, C₁₋₁₀ alkoxy, C₁₋₁₀ alkylthio, C₂₋₁₀ alkenyl, C₂₋₁₀ alkynyl, NRₐR_{b}, C₃₋₈ cycloalkyl, 3- to 8-membered heterocycloalkyl, C₆₋₁₀ aryl, or 5- to 14-membered heteroaryl;
J₁ is N or CR_{J1}, J₂ is N or CR_{J2}, J₃ is N or CR_{J3}, and J₄ is N or CR_{J4};
V₁ is N or CR₁, V₂ is N or CR₂, V₃ is N or CR₃, and V₄ is N or CR₄;
R₁, R₂, R₃, R₄, R_{J1}, R_{J2}, R_{J3}, and R_{J4} are each independently selected from hydrogen, hydroxy, cyano, acetyl, carboxy, nitro, halogen, amino, C₁₋₁₀ alkyl, C₁₋₁₀ haloalkyl, C₁₋₁₀ alkoxy, C₁₋₁₀ alkylthio, C₂₋₁₀ alkenyl, C₂₋₁₀ alkynyl, NRₐR_{b}, C₃₋₈ cycloalkyl, 3- to 8-membered heterocycloalkyl, C₆₋₁₀ aryl, and 5- to 14-membered heteroaryl;
or, R₃ and R₄, together with the atoms to which they are attached, form a substituted or unsubstituted 5- to 6-membered heteroaryl or benzene;
or, R_{J2} and R_{J3}, together with the atoms to which they are attached, form a substituted or unsubstituted 5- to 6-membered heteroaryl or benzene, or R_{J3} and R_{J4}, together with the atoms to which they are attached, form a substituted or unsubstituted 5- to 6-membered heteroaryl or benzene,
wherein the substitution refers to that the 5- to 6-membered heteroaryl or benzene is optionally substituted with 1, 2, 3, or 4 substituents selected from hydrogen, halogen, amino, cyano, acetyl, C₁₋₅ alkyl, C₁₋₅ alkoxy, C₁₋₃ haloalkyl, C₃₋₈ cycloalkyl, or 3- to 8-membered heterocycloalkyl;
K₁ is N or CRₖ₁, K₂ is N or CRₖ₂, and K₄ is N or CRₖ₄,
Rₖ₁, Rₖ₂, and Rₖ₄ are identical or different, and are each independently selected from hydrogen, hydroxy, cyano, acetyl, amino, halogen, C₁₋₁₀ alkyl, C₁₋₅ alkylene-OC₁₋₅ alkyl, C₁₋₁₀ haloalkyl, C₁₋₁₀ alkoxy, C₁₋₁₀ alkylthio, C₂₋₁₀ alkenyl, C₂₋₁₀ alkynyl, NRₐR_{b}, C₃₋₈ cycloalkyl, 3- to 8-membered heterocycloalkyl, -O-C₃₋₈ cycloalkyl, -O-3-to 8-membered heterocycloalkyl, C₆₋₁₀ aryl, and 5- to 14-membered heteroaryl;
Rₖ₃ represents -X₁-Rx,
X₁ is selected from O, S, NR₀₀, -C(O)-, -S(O)-, SO₂, -C(O)NR₀₀-, -NR₀₀C(O)-, -SO₂NR₀₀-, -NR₀₀SO₂-, - NR₀₀C(O)NR₀₀-, -NR₀₀C(O)O-, -OC(O)O-, -C(O)O-, -OC(O)-, -CH=CH-, -C≡C-, -(CRₓ₁Rₓ₂)ₓ-, C₃₋₈ cycloalkylene, 3- to 8-membered heterocycloalkylene, C₆₋₁₀ arylene, and 5- to 14-membered heteroarylene; Rx represents -(CRₓ₃Rₓ₄)ₓ₂-Rₓₙ,
Rₓ₁, Rₓ₂, Rₓ₃, Rₓ₄, and Rₓₙ are each independently selected from hydrogen, cyano, hydroxy, acetyl, halogen, nitro, formyl, carboxy, amino, C₁₋₁₀ alkyl, C₁₋₁₀ haloalkyl, C₁₋₁₀ alkoxy, C₁₋₁₀ alkylthio, C₂₋₁₀ alkenyl, C₂₋₁₀ alkynyl, C₃₋₈ cycloalkyl, 3- to 8-membered heterocycloalkyl, C₆₋₁₀ aryl, and 5- to 14-membered heteroaryl; or, Rₓ₁ and Rₓ₂, together with the C atom to which they are attached, form a 3- to 8-membered cycloalkyl or 3- to 8-membered heterocycloalkyl;
x and x2 are each independently 0, 1, 2, 3, 4, or 5, and x and x2 are not both 0;
or, R_{J3} and Rₖ₃ are connected to form a bond;
ring A is a saturated or unsaturated monocyclic alkylene, monocyclic heterocyclylene, polycyclic alkylene, or polycyclic heterocyclylene;
ring B is a saturated or unsaturated monocyclic alkylene, monocyclic heterocyclylene, polycyclic alkylene, or polycyclic heterocyclylene;
R is a bond, or a saturated or unsaturated monocyclic alkylene, monocyclic heterocyclylene, polycyclic alkylene or polycyclic heterocyclylene;
Linker represents 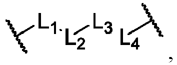 wherein
L₁, L₂, L₃, and L₄ each independently represents -R_{L1}-(CR₂₁R₂₂)ₙ₃-R_{L2}-(CR₃₁R₃₂)ₙ₂-;
R_{L1} and R_{L2} are each independently a bond, O, S, NR₀₀, -C(O)-, SO, SO₂, -C(O)NR₀₀-, -NR₀₀C(O)-, - SO₂NR₀₀-, -NR₀₀SO₂-, -NR₀₀C(O)NR₀₀-, -NR₀₀C(O)O-, -OC(O)-, -C(O)O-, -OC(O)O-, -CH=CH-, -C≡C-, - (CR₄₁R₄₂)ᵣ-, -(CR₄₁R₄₂)ᵣ-O-, -O-(CR₄₁R₄₂)ᵣ-, C₃₋₈ cycloalkylene or 3- to 8-membered heterocycloalkylene, C₆₋₁₀ arylene, or 5- to 14-membered heteroarylene;
n2, n3, and r are each independently 0, 1, 2, 3, or 4;
R₂₁, R₂₂, R₃₁, R₃₂, R₄₁, and R₄₂ are each independently selected from hydrogen, cyano, acetyl, hydroxy, halogen, C₁₋₁₀ alkyl, C₁₋₁₀ haloalkyl, C₁₋₁₀ alkoxy, C₁₋₁₀ alkylthio, NRₐR_{b}, C₃₋₈ cycloalkyl, 3- to 8-membered heterocycloalkyl, C₆₋₁₀ aryl, and 5- to 14-membered heteroaryl; or R₂₁ and R₂₂, together with the C atom to which they are attached, form a 3- to 8-membered cycloalkyl or 3- to 8-membered heterocycloalkyl;
L₅ and L₆ are each independently selected from O, S, NR₀₀, C(O)NR₀₀, NR₀₀C(O), C₁₋₁₀ alkylene, C₁₋₁₀ haloalkylene, C₁₋₁₀ alkyleneoxy, C₂₋₁₀ alkenylene, and C₂₋₁₀ alkynylene;
W is a bond, O, S, NR₀₀, -C(O)-, -S(O)-, SO₂, -C(O)NR₀₀-, -NR₀₀C(O)-, -SO₂NR₀₀-, -NR₀₀SO₂-, - NR₀₀C(O)NR₀₀-, -NR₀₀C(O)O-, -OC(O)O-, -C(O)O-, -OC(O)-, -CH=CH-, -C≡C-, -(CR₂₃R₂₄)ᵣ₂-, C₃₋₈ cycloalkylene, 3- to 8-membered heterocycloalkylene, C₆₋₁₀ arylene, or 5- to 14-membered heteroarylene; R₂₃ and R₂₄ are each independently selected from hydrogen, cyano, acetyl, hydroxy, halogen, C₁₋₁₀ alkyl, C₁₋₁₀ haloalkyl, C₁₋₁₀ alkoxy, C₁₋₁₀ alkylthio, NRₐR_{b}, C₃₋₈ cycloalkyl, 3- to 8-membered heterocycloalkyl, C₆₋₁₀ aryl, and 5- to 14-membered heteroaryl; or R₂₃ and R₂₄, together with the C atom to which they are attached, form a 3- to 8-membered cycloalkyl or 3- to 8-membered heterocycloalkyl; r2 is 0, 1, 2, 3 or 4;
Z₁ is CH or N, and Z₂ is CR_{z1}R_{z2}, NR₀₀, or C(O);
R_{z1} and R_{z2} are each independently selected from hydrogen, hydroxy, cyano, acetyl, halogen, C₁₋₁₀ alkyl, C₁₋₁₀ haloalkyl, C₁₋₁₀ alkoxy, C₁₋₁₀ alkylthio, NRₐR_{b}, C₃₋₈ cycloalkyl, 3- to 8-membered heterocycloalkyl, C₆₋₁₀ aryl, and 5- to 14-membered heteroaryl; or R_{z1} and R_{z2}, together with the C atoms to which they are attached, form a 3- to 8-membered cycloalkyl or 3- to 8-membered heterocycloalkyl;
G₁ is N or CR_{G1}, G₂ is N or CR_{G2}, G₃ is N or CR_{G3}, and G₄ is N or CR_{G4};
one of R_{G1}, R_{G2}, R_{G3}, and R_{G4} is attached to W, and the others are each independently selected from the group consisting of hydrogen, hydroxy, cyano, acetyl, halogen, amino, C₁₋₁₀ alkyl, C₁₋₁₀ haloalkyl, C₁₋₁₀ alkoxy, C₁₋₁₀ alkylthio, NRₐR_{b}, C₃₋₈ cycloalkyl, 3- to 8-membered heterocycloalkyl, C₆₋₁₀ aryl, and 5- to 14-membered heteroaryl;
R₀₀, Rₐ, and R_{b} are each independently hydrogen, cyano, acetyl, hydroxy, carboxy, nitro, halogen, C₁₋₁₀ alkyl, C₁₋₁₀ haloalkyl, C₁₋₁₀ alkoxy, C₁₋₁₀ alkylthio, NR_{c}R_{d}, C₃₋₈ cycloalkyl, 3- to 8-membered heterocycloalkyl, C₆₋₁₀ aryl or 5- to 14-membered heteroaryl; or Rₐ and R_{b}, together with the N atom to which they are attached, form a 3- to 8-membered heterocycloalkyl;
R_{c} and R_{d} are each independently hydrogen, C₁₋₅ alkyl, C₁₋₅ alkoxy, or C₁₋₅ haloalkyl; or R_{c} and R_{d}, together with the N atom to which they attached, form a 3- to 8-membered heterocycloalkyl;
the above C₁₋₁₀ alkyl, C₁₋₁₀ haloalkyl, C₁₋₁₀ alkoxy, C₁₋₁₀ alkylthio, C₁₋₅ alkyl, C₁₋₅ haloalkyl, C₁₋₅ alkoxy, C₃₋₈ cycloalkyl, 3- to 8-membered heterocycloalkyl, C₆₋₁₀ aryl, or 5- to 14-membered heteroaryl, saturated or unsaturated monocycle, monocyclic heterocycle, polycycle or polycyclic heterocycle are unsubstituted or, within a valence-permitted range, each independently substituted by 1, 2, 3, 4, or more groups selected from the following B2 groups, and the B2 group is selected from hydrogen, cyano, acetyl, hydroxy, carboxy, nitro, halogen, C₁₋₅ alkyl, C₁₋₅ alkoxy, C₁₋₅ alkylthio, NRₑR_{f}, 3- to 8-membered cycloalkyl, 3- to 8-membered heterocycloalkyl, C₆₋₁₀ aryl, or 5- to 14-membered heteroaryl, wherein Rₑ and R_{f} are each independently hydrogen, C₁₋₅ alkyl, C₁₋₅ alkoxy, and C₁₋₅ haloalkyl;
provided that:
   when PIN is formula (Ia) and Q is S(O)₂-isopropyl, neither Rₖ₃ nor Rₖ₄ is methyl;
   when PIN is formula (Ib) and ring B is pyrazine, neither Rₖ₃ nor Rₖ₄ is methyl;
   when PIN is formula (Ic) and A-R is 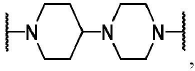 Linker is an all-carbon chain system, W contains no heteroatoms, and Linker is not methylene, ethylene and propylene.
Rₖ₁ and Rₖ₂ are each independently hydrogen, F, Cl, Br, I, amino, hydroxy, cyano, acetyl, C₁₋₅ alkyl, C₁₋₅ haloalkyl, C₁₋₅ alkoxy, C₁₋₃ alkylene-OC₁₋₃ alkyl, C₃₋₆ cycloalkyl, or C₃₋₆ heterocycloalkyl; preferably, Rₖ₂ is hydrogen, methyl, ethyl, propyl, isopropyl, methoxy, ethoxy, propoxy, isopropoxy, cyclopropyl, cyclobutyl, or -O-oxetanyl; preferably, Rₖ₂ and Rₖ₄ are each independently selected from hydrogen, deuterium, halogen, cyano, acylamino, C₁₋₅ alkyl, C₁₋₅ haloalkyl, C₁₋₅ alkoxy, C₁₋₃ alkylene-OC₁₋₃ alkyl, C₃₋₆ cycloalkyl, or C₃₋₆ heterocycloalkyl, wherein the C₁₋₅ alkyl, C₁₋₅ haloalkyl, C₁₋₅ alkoxy, C₁₋₃ alkylene-OC₁₋₃ alkyl, C₃₋₆ cycloalkyl, or C₃₋₆ heterocycloalkyl is optionally substituted with 0, 1, 2, 3, or more substituents selected from deuterium, hydroxy, cyano, C₁₋₅ alkyl, C₁₋₅ haloalkyl, or C₁₋₅ alkoxy; preferably R_{K4} is selected from hydrogen, deuterium, acylamino, cyano, or C₁₋₅ alkyl, preferably methyl, ethyl, propyl, isopropyl, C₁₋₅ haloalkyl, and C₁₋₅ alkoxy.

In some embodiments, the pharmaceutically acceptable salt may be a hydrochloride, a formate, or a trifluoroacetate.

In some embodiments, Rₖ₁ and Rₖ₂ are each independently hydrogen, F, Cl, Br, I, amino, hydroxy, cyano, acetyl, C₁₋₅ alkyl, C₁₋₅ haloalkyl, -CONH₂, C₁₋₅ alkoxy, C₁₋₃ alkylene-OC₁₋₃ alkyl, C₃₋₆ cycloalkyl, or C₃₋₆ heterocycloalkyl; preferably, Rₖ₂ is hydrogen, methyl, ethyl, propyl, isopropyl, methoxy, ethoxy, propoxy, isopropoxy, cyclopropyl, cyclobutyl, or -O-oxetanyl; preferably, Rₖ₂ and Rₖ₄ are each independently selected from hydrogen, deuterium, halogen, cyano, acylamino, C₁₋₅ alkyl, C₁₋₅ haloalkyl, C₁₋₅ alkoxy, C₁₋₃ alkylene-OC₁₋₃ alkyl, C₃₋₆ cycloalkyl, and C₃₋₆ heterocycloalkyl, wherein the C₁₋₅ alkyl, C₁₋₅ haloalkyl, C₁₋₅ alkoxy, C₁₋₃ alkylene-OC₁₋₃ alkyl, C₃₋₆ cycloalkyl, or C₃₋₆ heterocycloalkyl is optionally substituted with 0, 1, 2, 3, or more substituents selected from deuterium, halogen, hydroxy, cyano, C₁₋₅ alkyl, C₁₋₅ haloalkyl, and C₁₋₅ alkoxy; preferably R_{K4} is selected from hydrogen, deuterium, acylamino, cyano, and C₁₋₅ alkyl, preferably methyl, ethyl, propyl, isopropyl, C₁₋₅ haloalkyl, and C₁₋₅ alkoxy.

In some embodiments, Rₖ₂ is hydrogen, cyano, F, Cl, Br, I, C₁₋₅ alkyl, -CONH₂, C₁₋₅ haloalkyl, or C₁₋₅ alkoxy, wherein the C₁₋₅ alkyl or C₁₋₅ alkoxy is optionally substituted with 1, 2, 3, 4, or more groups selected from the following B2 groups, and the B2 group is selected from deuterium, halo, hydroxy, cyano, C₁₋₅ alkyl, C₁₋₅ haloalkyl, and C₁₋₅ alkoxy.

In some embodiments, Rₖ₂ is hydrogen, F, Cl, Br, I, C₁₋₅ alkyl, -CONH₂, C₁₋₅ haloalkoxy, or C₁₋₅ alkoxy. In some embodiments, Rₖ₄ is hydrogen, F, Cl, Br, I, cyano, acetyl, or C₁₋₅ alkoxy, preferably hydrogen or C₁₋₅ alkoxy.

In some embodiments, Rₖ₂ is -CONH₂.

In some embodiments, X₁ is selected from O, S, NR₀₀, -C(O)-, -S(O)-, SO₂, -C(O)NR₀₀-, -NR₀₀C(O)-, - SO₂NR₀₀-, -NR₀₀SO₂-, -NR₀₀C(O)NR₀₀-, -NR₀₀C(O)O-, -OC(O)O-, -CH=CH-, -C≡C-, C₁₋₅ alkylene, C₃₋₆ cycloalkylene, 3- to 6-membered heterocycloalkylene, phenylene, 5- to 6-membered heteroarylene, and 8- to 10-membered bicyclic heteroarylene,
wherein R₀₀ is selected from hydrogen or C₁₋₃ alkyl (preferably methyl, ethyl, or propyl; preferably hydrogen or methyl).

In some embodiments, X₁ is -(CRₓ₁Rₓ₂)ₓ-,
wherein Rₓ₁ and Rₓ₂ are each independently selected from hydrogen, C₁₋₅ alkyl (preferably C₁₋₃ alkyl, more preferably methyl, ethyl, propyl or isopropyl) and C₁₋₅ alkoxy,
or Rₓ₁ and Rₓ₂, together with the C atoms to which they are attached, form a C₃₋₆ cycloalkyl (preferably cyclopropyl or cyclobutyl) or 3- to 6-membered heterocycloalkyl (preferably oxetanyl);
x is 0, 1, 2, 3, or 4.

Preferably, the hydrogen of the compound of formula (I) described above is optionally substituted by deuterium.

In some embodiments, Rx is hydrogen, cyano, hydroxy, acetyl, halogen, amino, C₁₋₅ alkyl, C₁₋₅ haloalkyl, C₁₋₅ alkoxy, C₂₋₁₀ alkenyl, C₂₋₁₀ alkynyl, C₃₋₆ cycloalkyl, 3- to 6-membered heterocycloalkyl, benzene, 5- to 6-membered heteroaryl, or 8- to 10-membered bicyclic heteroaryl.

In some embodiments, Rₖ₃ is selected from F, Cl, Br, I, hydroxy, cyano, formyl, nitro, carboxy, acetyl, SOC₁₋₅ alkyl, SO₂C₁₋₅ alkyl, C₁₋₅ alkyl, C₁₋₅ haloalkyl, C₁₋₅ alkoxy, C₁₋₅ alkylene-OC₁₋₅ alkyl, C₃₋₆ cycloalkyl, 3- to 6-membered heterocycloalkyl, -O-C₃₋₆ cycloalkyl, and -O-3- to 6-membered heterocycloalkyl;

preferably, Rₖ₃ is hydroxy, cyano, formyl, nitro, carboxy, acetyl, -CO-amino, or -SO₂-methyl;

preferably, Rₖ₃ is F, Cl, Br, I, methyl, ethyl, propyl, isopropyl, methoxy, ethoxy, propoxy, isopropoxy, cyclopropyl, cyclobutyl, oxetanyl, fluoromethyl, difluoromethyl, trifluoromethyl, -CH₂CH₂F, -CHFCH₃, - CH₂CHF₂, -CF₂CH₃, -CHFCH₂F, -CH₂CF₃, -CF₂CH₂F, or -CHFCHF₂.

Preferably, Rₖ₃ is a C₁₋₆ alkyl, C₁₋₆ haloalkyl, C₁₋₆ alkoxy, C₁₋₅ alkylene-OC₁₋₅ alkyl, C₃₋₆ cycloalkyl, 3- to 6-membered heterocycloalkyl, -O-C₃₋₆ cycloalkyl, or -O-3- to 6-membered heterocycloalkyl, wherein the C₁₋₆ alkyl, C₁₋₆ haloalkyl, C₁₋₆ alkoxy, C₁₋₅ alkylene, C₁₋₅ alkyl, C₃₋₆ cycloalkyl, or 3- to 6-membered heterocycloalkyl is optionally substituted with 0, 1, 2, 3, 4, or 5 substituents selected from deuterium, halogen, hydroxy, cyano, C₁₋₃ alkyl, C₁₋₃ haloalkyl, and C₁₋₃ alkoxy; preferably C₁₋₆ alkyl; more preferably C₁₋₃ alkyl; preferably methyl, and more preferably ethyl; more preferably propyl, even more preferably cyclopropyl, and still more preferably isopropyl; more preferably C₃₋₆ cycloalkyl; more preferably a halogen, and even more preferably trifluoromethyl; more preferably ethyl substituted with 1, 2, 3, 4, or 5 deuterium; preferably cyano; preferably C₁₋₆ alkoxy; more preferably C₁₋₆ haloalkyl; more preferably C₁₋₃ alkylene-OC₁₋₃ alkyl; more preferably a 5-membered heterocyclyl substituted with 1 or 2 substituents selected from a halogen, C₁₋₃ alkyl, and C₁₋₃ haloalkyl.

In some embodiments, Rₖ₃ represents -X₁-Rx,
X₁ is selected from C₃₋₈ cycloalkylene, 3- to 8-membered heterocycloalkylene, C₆₋₁₀ arylene, or 5- to 14-membered heteroarylene;
Rx represents -(CRₓ₃Rₓ₄)ₓ₂-Rₓₙ,
Rₓ₃, Rₓ₄, and Rₓₙ are each independently selected from hydrogen, cyano, hydroxy, acetyl, halogen, nitro, formyl, carboxy, amino, C₁₋₅ alkyl, C₁₋₅ haloalkyl, C₁₋₅ alkoxy, C₁₋₅ alkylthio, C₃₋₈ cycloalkyl, 3- to 8-membered heterocycloalkyl, C₆₋₁₀ aryl, and 5- to 14-membered heteroaryl; or Rₓ₁ and Rₓ₂, together with the C atom to which they are attached, form a 3- to 8-membered cycloalkyl or 3- to 8-membered heterocycloalkyl; x2 is 0, 1, 2, 3, or 4.

In some embodiments, X₁ is a 5- to 6-membered heteroarylene or 8- to 10-membered bicyclic heteroarylene, and
Rx is H, F, Cl, Br, I, methyl, ethyl, propyl, isopropyl, methoxy, ethoxy, propoxy, isopropoxy, cyclopropyl, cyclobutyl, oxetanyl, fluoromethyl, difluoromethyl, trifluoromethyl, -CH₂CH₂F, -CHFCH₃, -CH₂CHF₂, - CF₂CH₃, -CHFCH₂F, -CH₂CF₃, -CF₂CH₂F, or -CHFCHF₂.

In some embodiments, Rₖ₃ is selected from: 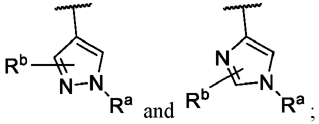 preferably Rₖ₃ is selected from 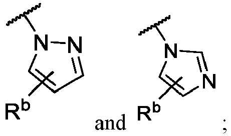 R^{a} and R^{b} are H, F, Cl, Br, I, methyl, ethyl, propyl, isopropyl, methoxy, ethoxy, propoxy, isopropoxy, cyclopropyl, cyclobutyl, oxetanyl, fluoromethyl, difluoromethyl, trifluoromethyl, -CH₂CH₂F, -CHFCH₃, - CH₂CHF₂, -CF₂CH₃, -CHFCH₂F, -CH₂CF₃, -CF₂CH₂F, or -CHFCHF₂.

Preferably, Rₖ₃ is a 5- or 6-membered heterocyclyl substituted with 0, 1, or 2 substituents selected from R^{b}, wherein R^{b} is selected from hydrogen, deuterium, halogen, cyano, C₁₋₅ alkyl, C₁₋₅ haloalkyl, and C₁₋₅ alkoxy.

Preferably, Rₖ₃ is selected from 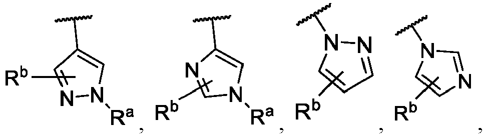 halogen, cyano, acetyl, C₁₋₅ alkyl, C₁₋₅ alkylene-OC₁₋₅ alkyl, and C₃₋₆ cycloalkyl, wherein the C₁₋₅ alkyl is optionally substituted with 0, 1, 2, 3, 4, or 5 substituents selected from deuterium, halogen, hydroxy, cyano, C₁₋₃ alkyl, C₁₋₃ haloalkyl, and C₁₋₃ alkoxy; R^{a} and R^{b} are H, F, Cl, Br, I, methyl, ethyl, propyl, isopropyl, methoxy, ethoxy, propoxy, isopropoxy, cyclopropyl, cyclobutyl, oxetanyl, fluoromethyl, difluoromethyl, trifluoromethyl, -CH₂CH₂F, - CHFCH₃, -CH₂CHF₂, -CF₂CH₃, -CHFCH₂F, -CH₂CF₃, -CF₂CH₂F, or -CHFCHF₂; R^{a} and R^{b} are H, F, Cl, Br, I, methyl, ethyl, propyl, isopropyl, methoxy, ethoxy, propoxy, isopropoxy, cyclopropyl, cyclobutyl, oxetanyl, fluoromethyl, difluoromethyl, trifluoromethyl, -CH₂CH₂F, -CHFCH₃, -CH₂CHF₂, -CF₂CH₃, -CHFCH₂F, - CH₂CF₃, -CF₂CH₂F, or -CHFCHF₂.

Preferably, Rₖ₃ is selected from 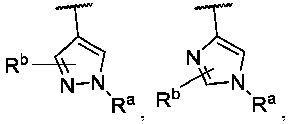 F, Cl, Br, I, cyano, acetyl, ethyl, C₁₋₅ alkylene-OC₁₋₅ alkyl, and C₃₋₆ cycloalkyl, wherein the C₁₋₅ alkyl is optionally substituted with 0, 1, 2, 3, 4, or 5 substituents selected from deuterium, hydroxy, cyano, C₁₋₃ alkyl, and C₁₋₃ alkoxy; R^{a} is H, methyl, ethyl, propyl, or isopropyl; R^{b} is H, methyl, ethyl, propyl, isopropyl, methoxy, ethoxy, propoxy, isopropoxy, cyclopropyl, cyclobutyl, oxetanyl, fluoromethyl, difluoromethyl, trifluoromethyl, -CH₂CH₂F, -CHFCH₃, - CH₂CHF₂, -CF₂CH₃, -CHFCH₂F, -CH₂CF₃, -CF₂CH₂F, or -CHFCHF₂.

Preferably, Rₖ₃ is selected from 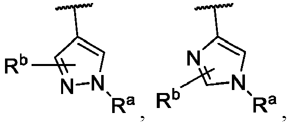 F, Cl, Br, I, cyano, acetyl, ethyl, C₁₋₅ alkylene-OC₁₋₅ alkyl, and C₃₋₆ cycloalkyl, wherein the C₁₋₅ alkyl is optionally substituted with 0, 1, 2, or 3 substituents selected from deuterium, hydroxy, cyano, C₁₋₃ alkyl, and C₁₋₃ alkoxy; R^{a} is H, methyl, ethyl, propyl, or isopropyl; R^{b} is H, methyl, ethyl, propyl or isopropyl.

In some embodiments, Rₖ₁ and Rₖ₄ are hydrogen.

In some embodiments, R₄ is hydroxy, cyano, acetyl, carboxy, nitro, halogen, amino, C₁₋₆ alkyl, C₁₋₆ haloalkyl, C₁₋₆ alkoxy, C₁₋₆ alkylthio, C₂₋₆ alkenyl, C₂₋₆ alkynyl, NRₐR_{b}, C₃₋₈ cycloalkyl, 3- to 8-membered heterocyclyl, C₆₋₁₀ aryl, or 5- to 14-membered heteroaryl, wherein the C₁₋₆ alkyl, C₁₋₆ haloalkyl, C₁₋₆ alkoxy, C₁₋₆ alkylthio, C₂₋₆ alkenyl, C₂₋₆ alkynyl, C₃₋₈ cycloalkyl, 3- to 8-membered heterocyclyl, C₆₋₁₀ aryl, or 5- to 14-membered heteroaryl is optionally substituted with 0, 1, 2, or 3 R⁴¹s;
preferably, R₄ is halogen, C(O)NRₐR_{b}, C₃₋₈ cycloalkyl, 3- to 8-membered heterocyclyl, phenyl, 5- or 6-membered heteroaryl, 7- to 11-membered spirocyclyl, or 8- to 12-membered spiroheterocyclyl, wherein the C₃₋₈ cycloalkyl, 3- to 8-membered heterocyclyl, phenyl, 5- or 6-membered heteroaryl, 7- to 11-membered spirocyclyl, or 8- to 12-membered spiroheterocyclyl is optionally substituted with 0, 1, 2, or 3 R⁴¹s;
preferably, R₄ is selected from: fluorine, chlorine, bromine, amino, acylamino, acetyl, cyclopropyl, cyclobutyl, oxetanyl, cyclopentyl, trifluoromethyl, and C₁₋₅ alkyl; preferably, R₄ is hydrogen, -O-C₀₋₅ alkylene-C₃₋₈ cycloalkyl, -OC₀₋₅ alkylene-3- to 8-membered heterocyclyl; preferably R₄ is phenyl or 5- or 6-membered heteroaryl substituted with 0, 1, or 2 R⁴¹s;
preferably, R₄ is selected from furanyl, thienyl, pyrrolyl, pyrazolyl, imidazolyl, thiazolyl, isothiazolyl, oxazolyl, isoxazolyl, triazolyl, phenyl, pyridyl, pyrimidinyl, cyclopentyl, tetrahydrofuranyl, tetrahydrothienyl, tetrahydropyrrolyl, cyclohexyl, oxacyclohexyl, piperidinyl, piperazinyl, and morpholinyl substituted with 0, 1, or 2 R⁴¹s; R⁴¹ is selected from hydrogen, deuterium, halogen, C₁₋₆ alkyl, C₁₋₆ haloalkyl, C₁₋₆ alkoxy, C₁₋₆ alkylthio, C₃₋₈ cycloalkyl, 3- to 8-membered heterocyclyl, OC₃₋₈ cycloalkyl, and -O-3- to 8-membered heterocyclyl (R⁴¹ is preferably hydrogen, deuterium, hydroxy, methyl, ethyl, propyl, isopropyl, trifluoromethyl, trifluoroethyl, methoxy, ethoxy, propoxy, cyclopropyl, cyclobutyl, oxetanyl, cyclopentyl, azacyclopentyl,

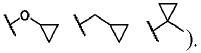

In some embodiments, R₄ is hydrogen, hydroxy, cyano, C(O)NRₐR_{b}, halogen, C₁₋₁₀ alkyl substituted with 0, 1, or 2 R⁴¹s, or 5- to 6-membered heteroaryl substituted with 0, 1, or 2 R⁴¹s; the heteroatom of the 5- to 6-membered heteroaryl is selected from one or more of nitrogen, oxygen, and sulfur, and the number of the heteroatom is 1, 2, or 3;
or, R₃ and R₄, together with the atoms to which they are attached, form a 5- to 6-membered heteroaryl or benzene substituted with 0, 1, or 2 R⁴¹s; the heteroatom of the 5- to 6-membered heteroaryl is selected from one or more of nitrogen, oxygen, and sulfur, and the number of the heteroatom is 1, 2, or 3;
R^{a} and R^{b} are each independently hydrogen, cyano, acetyl, hydroxy, carboxy, nitro, halogen, C₁₋₁₀ alkyl, C₁₋₁₀ haloalkyl, C₁₋₁₀ alkoxy, C₁₋₁₀ alkylthio, NR_{c}R_{d}, C₃₋₈ cycloalkyl, 3- to 8-membered heterocycloalkyl, C₆₋₁₀ aryl or 5- to 14-membered heteroaryl; or Rₐ and R_{b}, together with the N atom to which they are attached, form a 3-to 8-membered heterocycloalkyl; the heteroatom of the 5- to 14-membered heteroaryl is selected from one or more of nitrogen, oxygen, and sulfur, and the number of the heteroatom is 1, 2, or 3; the heteroatom of the 3-to 8-membered heterocycloalkyl is selected from one or more of nitrogen, oxygen, and sulfur, and the number of the heteroatom is 1, 2, or 3;
R⁴¹ is selected from hydrogen, deuterium, halogen, C₁₋₆ alkyl, C₁₋₆ haloalkyl, C₁₋₆ alkoxy, C₁₋₆ alkylthio, C₃₋₈ cycloalkyl, 3- to 8-membered heterocyclyl, OC₃₋₈ cycloalkyl, and -O-3- to 8-membered heterocyclyl; the heteroatom of the 3- to 8-membered heterocycloalkyl is selected from one or more of nitrogen, oxygen, and sulfur, and the number of the heteroatom is 1, 2, or 3. In some embodiments, R₄ is hydrogen, hydroxy, cyano, C(O)NRₐR_{b}, halogen, C₁₋₁₀ alkyl substituted with 0, 1, or 2 R⁴¹s, or 5- to 6-membered heteroaryl substituted with 0, 1, or 2 R⁴¹s; the heteroatom of the 5- to 6-membered heteroaryl is nitrogen and/or oxygen, and the number of the heteroatom is 1, 2, or 3;
or, R₃ and R₄, together with the atoms to which they are attached, form a 5- to 6-membered heteroaryl or benzene substituted with 0, 1, or 2 R⁴¹s; the heteroatom of the 5- to 6-membered heteroaryl is nitrogen and/or oxygen, and the number of the heteroatom is 1, 2, or 3;
R^{a} and R^{b} are each independently hydrogen or C₁₋₅ alkyl;
R⁴¹ is selected from hydrogen, deuterium, halogen, C₁₋₅ alkyl, C₁₋₅ haloalkyl, and C₁₋₅ alkoxy.
In some embodiments, R₄ is hydrogen, halogen, or 5- to 6-membered heteroaryl; the heteroatom of the 5- to 6-membered heteroaryl is nitrogen, and the number of the heteroatom is 1, 2, or 3;
or, R₃ and R₄, together with the atoms to which they are attached, form a 5- to 6-membered heteroaryl or benzene; the heteroatom of the 5- to 6-membered heteroaryl is nitrogen, and the number of the heteroatom is 1, 2, or 3;
R^{a} and R^{b} are each independently hydrogen or C₁₋₁₀ alkyl.

In some embodiments, ring A is a 3- to 8-membered monocyclic heterocyclylene, 7- to 16-membered spiroheterocyclylene, 7- to 14-membered fused heterocyclylene, 7- to 10-membered bridged heterocyclylene, phenylene, 5- or 6-membered monocyclic heteroarylene, or 8- to 10-membered bicyclic heteroarylene; preferably ring A is a 5- to 7-membered monocyclic heterocyclylene; preferably, ring A is a 7- to 11-membered mono-spiroheterocyclylene. Preferably, R is a 7- to 11-membered mono-spiroheterocyclylene or 8- to 10-membered bicyclic heteroarylene substituted with 0, 1, 2, or 3 R₀s; R₀ is selected from hydrogen, deuterium, halogen, =O, C₁₋₅ alkyl, C₁₋₅ haloalkyl, C₁₋₅ alkoxy, C₃₋₈ cycloalkyl, or 3- to 8-membered heterocyclyl, or two R₀s, together with the C atom to which they are attached, form a 3- to 6-membered cycloalkyl or heterocyclyl. In some embodiments, ring A is a 3- to 8-membered monocyclic heterocyclylene or 7 -to 16-membered spiroheterocyclylene; the heteroatom of the monocyclic heterocyclylene is selected from one or more of nitrogen, oxygen, and sulfur, and the number of the heteroatom is 1, 2, or 3; the heteroatom of the spiroheterocyclylene is selected from one or more of nitrogen, oxygen, and sulfur, and the number of the heteroatom is 1, 2, or 3.

In some embodiments, ring A is a 3- to 6-membered monocyclic heterocyclylene or 7 -to 11-membered spiroheterocyclylene; the heteroatom of the monocyclic heterocyclylene is nitrogen, oxygen, or sulfur, and the number of the heteroatom is 1, 2, or 3; the heteroatom of the spiroheterocyclylene is nitrogen, oxygen, or sulfur, and the number of the heteroatom is 1, 2, or 3.

In some embodiments, ring A is a 3- to 8-membered monocyclic heterocyclylene or 7 -to 11-membered spiroheterocyclylene; the heteroatom of the monocyclic heterocyclylene is N, and the number of the heteroatom is 1 or 2; the heteroatom of the spiroheterocyclylene is N, and the number of the heteroatom is 1 or 2.

In some embodiments, R is a bond, or a 7- to 11-membered mono-spiroheterocyclylene, 3- to 8-membered monocyclic heterocyclylene, and 8- to 10-membered bicyclic heteroarylene substituted with 0, 1, 2, or 3 R₀s, wherein R₀ is selected from hydrogen, deuterium, halogen, =O, C₁₋₆ alkyl, C₁₋₆ haloalkyl, C₁₋₆ alkoxy, C₃₋₈ cycloalkyl, and 3- to 8-membered heterocyclyl, or two R₀s, together with the C atom to which they are attached, form a 3- to 6-membered cycloalkyl; the heteroatom of the mono-spiroheterocyclylene is selected from one or more of nitrogen, oxygen, and sulfur, and the number of the heteroatom is 1, 2, or 3; the heteroatom of the bicyclic heteroarylene is selected from one or more of nitrogen, oxygen, and sulfur, and the number of the heteroatom is 1, 2, or 3; the heteroatom of the monocyclic heterocyclylene is selected from one or more of nitrogen, oxygen, and sulfur, and the number of the heteroatom is 1, 2, or 3; the heteroatom of the 3- to 8-membered heterocyclyl is selected from one or more of nitrogen, oxygen, and sulfur, and the number of the heteroatom is 1, 2, or 3.

In some embodiments, R is a bond, or a 7- to 11-membered mono-spiroheterocyclylene, 3- to 8-membered monocyclic heterocyclylene, and 8- to 10-membered bicyclic heteroarylene substituted with 0, 1, 2, or 3 R₀s, wherein R₀ is selected from hydrogen, =O, and C₁₋₆ alkyl, or two R₀s, together with the C atom to which they are attached, form a 3- to 6-membered cycloalkyl; the heteroatom of the bicyclic heteroarylene is nitrogen, oxygen, or sulfur, and the number of the heteroatom is 1, 2, or 3; the heteroatom of the spiroheterocyclylene is nitrogen, oxygen, or sulfur, and the number of the heteroatom is 1, 2, or 3; the heteroatom of the monocyclic heterocyclylene is nitrogen, oxygen, or sulfur, and the number of the heteroatom is 1, 2, or 3.

In some embodiments, R is a bond, or a 7- to 11-membered mono-spiroheterocyclylene, 5- or 6-membered monocyclic heterocyclylene, and 8- to 10-membered bicyclic heteroarylene substituted with 0, 1, 2, or 3 R₀s, wherein R₀ is selected from hydrogen, =O, and C₁₋₆ alkyl, or two R₀s, together with the C atom to which they are attached, form a 3- to 6-membered cycloalkyl; the heteroatom of the mono-spiroheterocyclylene is N, and the number of the heteroatom is 1 or 2; the heteroatom of the bicyclic heteroarylene is N, and the number of the heteroatom is 1 or 2; the heteroatom of the 6-membered monocyclic heterocyclylene is N, and the number of the heteroatom is 1 or 2.

In one aspect, the present invention provides a compound of formula (I), or a pharmaceutically acceptable salt, an enantiomer, a diastereomer, a racemate, a solvate, a hydrate, a polymorph, a prodrug or an isotopic variant thereof, and a mixture thereof,

PIN-Linker-E (I)

wherein, PIN represents: 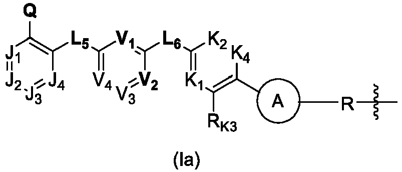
wherein R is a bond, or a 7- to 11-membered mono-spiroheterocyclylene, 3- to 8-membered monocyclic heterocyclylene, and 8- to 10-membered bicyclic heteroarylene substituted with 0, 1, 2, or 3 R₀s, wherein R₀ is selected from hydrogen, deuterium, halogen, =O, C₁₋₆ alkyl, C₁₋₆ haloalkyl, C₁₋₆ alkoxy, C₃₋₈ cycloalkyl, and 3- to 8-membered heterocyclyl, or two R₀s, together with the C atom to which they are attached, form a 3- to 6-membered cycloalkyl; the heteroatom of the mono-spiroheterocyclylene is selected from one or more of nitrogen, oxygen, and sulfur, and the number of the heteroatom is 1, 2, or 3; the heteroatom of the bicyclic heteroarylene is selected from one or more of nitrogen, oxygen, and sulfur, and the number of the heteroatom is 1, 2, or 3; the heteroatom of the monocyclic heterocyclylene is selected from one or more of nitrogen, oxygen, and sulfur, and the number of the heteroatom is 1, 2, or 3; the heteroatom of the 3- to 8-membered heterocyclyl is selected from one or more of nitrogen, oxygen, and sulfur, and the number of the heteroatom is 1, 2, or 3;
Q, V₁, V₂, V₃, V₄, J₁, J₂, J₃, J₄, K₁, K₂, K₄, Rₖ₃, L₅, L₆, ring A, E and Linker are defined in any one of the above items.

In one aspect, the present invention provides a compound of formula (I), or a pharmaceutically acceptable salt, an enantiomer, a diastereomer, a racemate, a solvate, a hydrate, a polymorph, a prodrug or an isotopic variant thereof, and a mixture thereof,

PIN-Linker-E (I)

wherein, PIN represents: 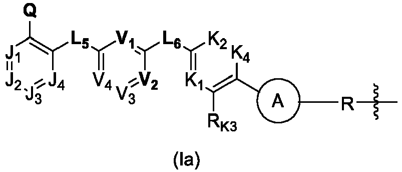
wherein Rₖ₃ is selected from 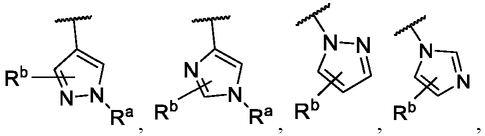 halogen, cyano, acetyl, C₁₋₅ alkyl, C₁₋₅ alkylene-OC₁₋₅ alkyl, or C₃₋₆ cycloalkyl, wherein the C₁₋₅ alkyl is optionally substituted with 0, 1, 2, 3, 4, or 5 substituents selected from deuterium, halogen, hydroxy, cyano, C₁₋₃ alkyl, C₁₋₃ haloalkyl, and C₁₋₃ alkoxy; R^{a} and R^{b} are H, F, Cl, Br, I, methyl, ethyl, propyl, isopropyl, methoxy, ethoxy, propoxy, isopropoxy, cyclopropyl, cyclobutyl, oxetanyl, fluoromethyl, difluoromethyl, trifluoromethyl, -CH₂CH₂F, - CHFCH₃, -CH₂CHF₂, -CF₂CH₃, -CHFCH₂F, -CH₂CF₃, -CF₂CH₂F, or -CHFCHF₂; R^{a} and R^{b} are H, F, Cl, Br, I, methyl, ethyl, propyl, isopropyl, methoxy, ethoxy, propoxy, isopropoxy, cyclopropyl, cyclobutyl, oxetanyl, fluoromethyl, difluoromethyl, trifluoromethyl, -CH₂CH₂F, -CHFCH₃, -CH₂CHF₂, -CF₂CH₃, -CHFCH₂F, - CH₂CF₃, -CF₂CH₂F, and -CHFCHF₂;
Q, V₁, V₂, V₃, V₄, J₁, J₂, J₃, J₄, K₁, K₂, K₄, R, L₅, L₆, ring A, E, and Linker are defined in any one of the above items.

In one aspect, the present invention provides a compound of formula (I), or a pharmaceutically acceptable salt, an enantiomer, a diastereomer, a racemate, a solvate, a hydrate, a polymorph, a prodrug or an isotopic variant thereof, and a mixture thereof,

PIN-Linker-E (I)

wherein, PIN represents: 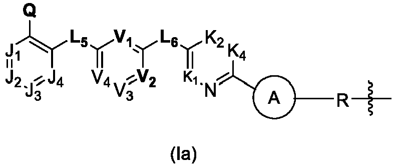 wherein Q, V₁, V₂, V₃, V₄, J₁, J₂, J₃, J₄, K₁, K₂, K₄, R, L₅, L₆, ring A, E, and Linker are defined in any one of the above items.

In some embodiments, ring A represents: 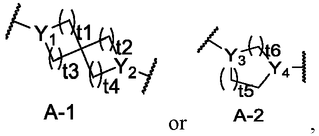 wherein, Y₁ and Y₂ are each independently CH or N, Y₃ and Y₄ are each independently CH or N, t1, t2, t3 and t4 are each independently 0, 1, 2 or 3, and t5 and t6 are each independently 0, 1, 2 or 3; t1 and t3 are not both 0, and t2 and t4 are not both 0.

In some embodiments, ring A is selected from:

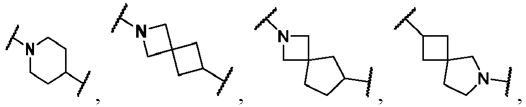

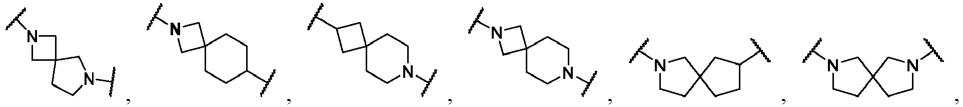

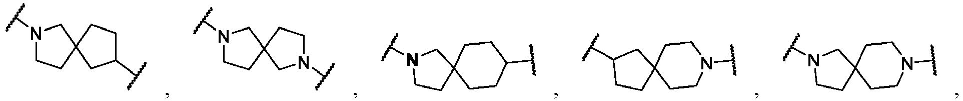

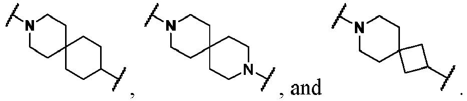

In some embodiments, R is a bond, or R is a 3- to 8-membered monocyclic heterocyclylene, 7- to 16-membered spiro heterocyclylene, 7- to 10-membered bicyclic fused heterocyclylene, 7- to 10-membered bridged heterocyclylene, 5- or 6-membered heteroarylene, or 8- to 10-membered bicyclic heteroarylene; preferably, R is a bond; preferably, R is a 5- to 7-membered monocyclic heterocyclylene; preferably, R is a 7- to 11-membered mono-spiroheterocyclylene or 8- to 10-membered bicyclic heteroarylene.

In some embodiments, R represents: 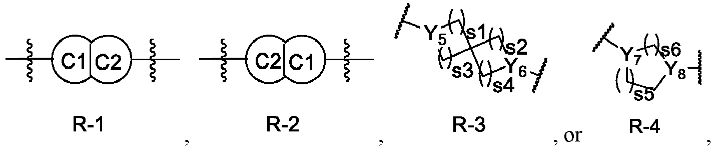 wherein
ring C1 is a 5- to 6-membered heteroaryl or benzene, and ring C2 is a 5- to 6-membered saturated or unsaturated monocycle or a 5- to 6-membered saturated or unsaturated monoheterocycle, wherein the 5- to 6-membered heteroaryl, benzene, 5- to 6-membered saturated or unsaturated monocycle, or 5- to 6-membered saturated or unsaturated monoheterocycle is optionally substituted with 0, 1, 2, 3, or more substituents selected from hydrogen, D, halogen, amino, hydroxy, cyano, acetyl, C₁₋₅ alkyl, C₁₋₅ alkoxy, C₃₋₈ cycloalkyl, 5- to 7-membered heterocycloalkyl, 6- to 10-membered aryl, and 5- to 14-membered heteroaryl; preferably, ring C2 is a 5- to 7-membered azacycle;
Y₅ and Y₆ are each independently CH or N, Y₇ and Y₈ are each independently CH or N, s1, s2, s3 and s4 are each independently 0, 1, 2, or 3, s5 and s6 are each independently 0, 1, 2, or 3; s1 and s3 are not both 0, and s2 and s4 are not both 0.

In some embodiments, ring C1 is selected from furan, thiophene, pyrrole, pyrazole, imidazole, thiazole, isothiazole, oxazole, isoxazole, triazole, benzene, pyridine, pyrimidine, pyrazine, or pyridazine; ring C2 is selected from cyclopentane, tetrahydrofuran, tetrahydrothiophene, tetrahydropyrrole, furan, thiophene, pyrrole, pyrazole, imidazole, thiazole, isothiazole, oxazole, isoxazole, triazole, cyclohexane, oxacyclohexane, piperidine, piperazine, morpholine, benzene, pyridine, pyrimidine, pyrazine, and pyridazine.

In some embodiments, R is

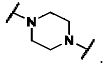

In some embodiments, R is selected from 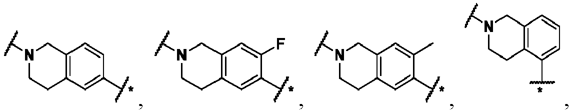 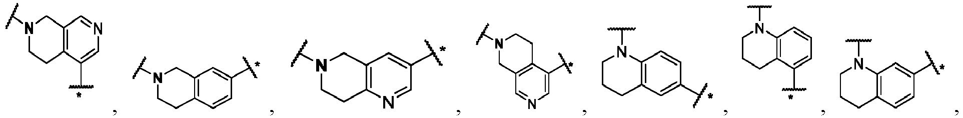 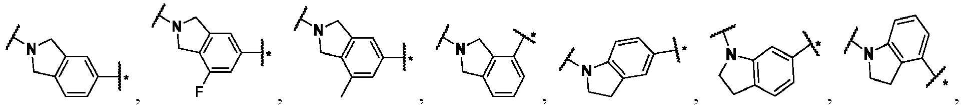 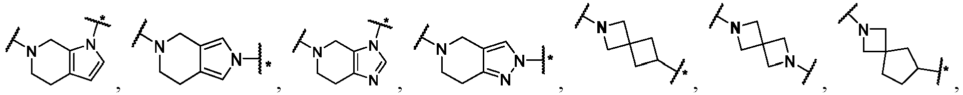 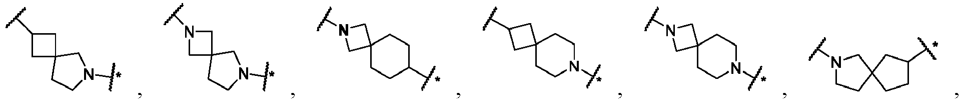 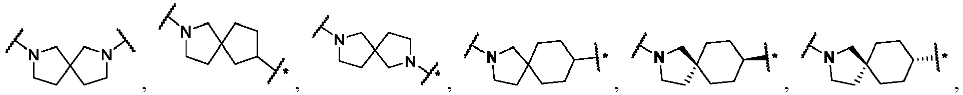 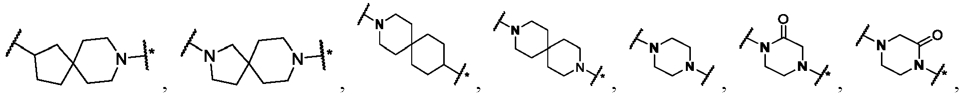 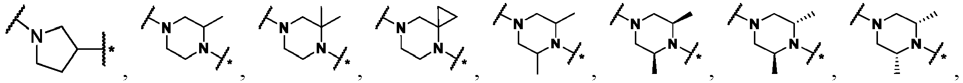 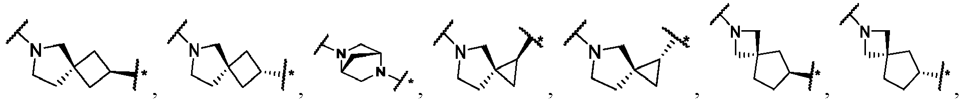 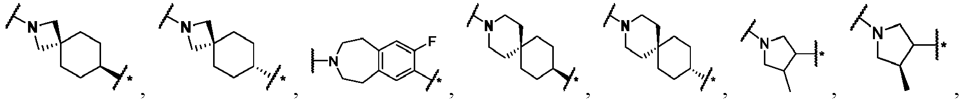 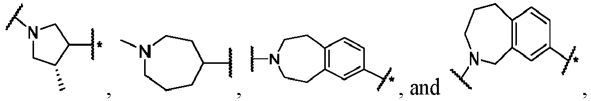 preferably, R is selected from 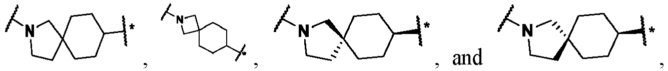 wherein 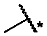 is connected with Linker while the other end is connected with ring A, or 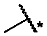 is connected with ring A while the other end is connected with Linker.

In some embodiments, L₁, L₂, L₃, and L₄ are each independently selected from -R_{L1}-(CH₂)ₙ₅-R_{L2}-(CH₂)ₙ₆-, wherein R_{L1} and R_{L2} are each independently O, S, NR₀₀, -C(O)-, -C(O)NR₀₀-, -NR₀₀C(O)-, -SO₂NR₀₀-, - NR₀₀SO₂-, -NR₀₀C(O)NR₀₀-, -NR₀₀C(O)O-, -OC(O)-, -C(O)O-, -OC(O)O-, and (CH₂)ᵣ₄; n5, n6, and r4 are each independently 0, 1, 2, 3, or 4,
R₀₀ is a C₁₋₃ alkyl (preferably methyl), or R₀₀ is hydrogen.

Preferably, R_{L1} and R_{L2} are each independently selected from a bond, 5- or 6-membered heteroarylene, phenylene, C₃₋₈ cycloalkylene, C₃₋₈ heterocyclylene, CH₂, CH₂CH₂, OCH₂CH₂, CH₂CH₂O, CH₂CH₂CH₂, ethenylene, ethynylene, 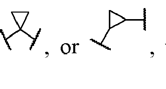 , wherein, the 5- or 6-membered heteroarylene, phenylene, C₃₋₈ cycloalkylene, C₃₋₈ heterocyclylene, or any methylene is optionally substituted, within their respective valence-permitted ranges, with 0, 1, 2, or 3 substituents selected from hydroxy, cyano, halogen, C₁₋₅ alkyl, C₁₋₅ haloalkyl, C₁₋₅ alkoxy, C₁₋₅ alkylthio, C₂₋₆ alkenyl, C₂₋₆ alkynyl, NRₐR_{b}, acetyl, -C(O)NRₐR_{b}, C₃₋₈ cycloalkyl, 3- to 8-membered heterocyclyl, C₆₋₁₀ aryl, and 5- to 14-membered heteroaryl.

In some embodiments, L₁ is (CR₂₁R₂₂)ₙ₃, wherein R₂₁ and R₂₂ are each independently hydrogen, methyl, ethyl, propyl, isopropyl, cyclopropyl, cyclobutyl, oxetanyl, methoxy, ethoxy, or propoxy, and n3 is 0, 1, 2, or 3;
preferably, L₁ is (CR₂₁R₂₂)ₙ₃, wherein R₂₁ and R₂₂, together with the C atom to which they are attached, form cyclopropyl, cyclobutyl, cyclopentyl, or oxetanyl, and n₃ is 1 or 2; more preferably -C(CH₃)₂-, and even more preferably 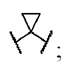
preferably, L₁ is O, S, NR₀₀, C(O), C(O)NR₀₀, NR₀₀C(O), -C(O)O-, -OC(O)-, SO, SO₂, SO₂NR₀₀, or NR₀₀SO₂; R₀₀ is hydrogen or methyl; preferably, L₁ is -CH=CH- or -C=C-; preferably, L₁ is CH₂, CH₂CH₂, or CH₂CH₂CH₂ (preferably CH₂, more preferably CH₂CH₂, and even more preferably CH₂CH₂CH₂); preferably L₁ is O, preferably L₁ is NH or N(CH₃), more preferably C(O), even more preferably CH₂CH₂O, and still more preferably a bond.

In some embodiments, L₁ is a C₃₋₆ cycloalkylene, 4- to 7-membered heterocyclylene, phenylene, or 5- or 6-membered heteroarylene (preferably cyclopropylene, cyclobutylene, cyclopentylene, azacyclopentylene, cyclohexylene, piperidinylene, piperazinylene, or 1,4-phenylene, more preferably 1,3-cyclobutylene, even more preferably 1,4-piperidylene or 1,4-piperazinylene).

In some embodiments, L₂ is (CR₂₁R₂₂)ₙ₃, wherein R₂₁ and R₂₂ are each independently hydrogen, methyl, ethyl, propyl, isopropyl, cyclopropyl, cyclobutyl, oxetanyl, methoxy, ethoxy, or propoxy, and n3 is 0, 1, 2, or 3;
preferably, L₂ is (CR₂₁R₂₂)ₙ₃, wherein R₂₁ and R₂₂, together with the C atom to which they are attached, form cyclopropyl, cyclobutyl, cyclopentyl, or oxetanyl, and n₃ is 1 or 2; more preferably, -C(CH₃)₂-, and even more preferably, 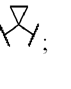
preferably, L₂ is O, S, NR₀₀, C(O), C(O)NR₀₀, NR₀₀C(O), -C(O)O-, -OC(O)-, SO, SO₂, SO₂NR₀₀, or NR₀₀SO₂; R₀₀ is hydrogen or methyl; preferably, L₂ is -CH=CH- or -C≡C-; preferably, L₂ is CH₂, CH₂CH₂, or CH₂CH₂CH₂ (preferably CH₂, more preferably CH₂CH₂, even more preferably CH₂CH₂CH₂); preferably L₂ is O, preferably L₂ is NH or N(CH₃), preferably C(O), more preferably CH₂CH₂O; preferably, L₂ is a bond;
preferably, L₂ is a C₃₋₆ cycloalkylene, 4- to 7-membered heterocyclylene, phenylene, or 5- or 6-membered heteroarylene, preferably 1,3-cyclobutylene, preferably 1,4-piperidylene or 1,4-piperazinylene.

In some embodiments, L₃ is (CR₂₁R₂₂)ₙ₃, wherein R₂₁ and R₂₂ are each independently hydrogen, methyl, ethyl, propyl, isopropyl, cyclopropyl, cyclobutyl, oxetanyl, methoxy, ethoxy, or propoxy, and n3 is 0, 1, 2, or 3;
preferably, L₃ is (CR₂₁R₂₂)ₙ₃, wherein R₂₁ and R₂₂, together with the C atom to which they are attached, form cyclopropyl, cyclobutyl, cyclopentyl, or oxetanyl, and n₃ is 1 or 2; more preferably -C(CH₃)₂-, and even more preferably 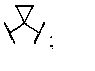
preferably, L₃ is O, S, NR₀₀, C(O), C(O)NR₀₀, NR₀₀C(O), -C(O)O-, -OC(O)-, SO, SO₂, SO₂NR₀₀, or NR₀₀SO₂; R₀₀ is hydrogen or C₁₋₃ alkyl (preferably methyl, ethyl, or propyl); preferably, L₃ is -CH=CH- or -C≡C-; preferably, L₃ is CH₂, CH₂CH₂, or CH₂CH₂CH₂ (preferably CH₂, more preferably CH₂CH₂, even more preferably CH₂CH₂CH₂); preferably L₃ is O, preferably L₃ is NH or N(CH₃), preferably C(O), more preferably CH₂CH₂O; preferably, L₃ is a bond;
preferably, L₃ is a C₃₋₆ cycloalkylene, 4- to 7-membered heterocyclylene, phenylene, or 5- or 6-membered heteroarylene, more preferably 1,3-cyclobutylene, more preferably 1,4-piperidylene or 1,4-piperazinylene. In some embodiments, L₄ is (CR₂₁R₂₂)ₙ₃, wherein R₂₁ and R₂₂ are each independently hydrogen, methyl, ethyl, propyl, isopropyl, cyclopropyl, cyclobutyl, oxetanyl, methoxy, ethoxy, or propoxy, and n3 is 0, 1, 2, or 3; preferably, L₄ is (CR₂₁R₂₂)ₙ₃, wherein R₂₁ and R₂₂, together with the C atom to which they are attached, form cyclopropyl, cyclobutyl, cyclopentyl, or oxetanyl, and n₃ is 1 or 2; more preferably -C(CH₃)₂-, and even more preferably 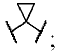
preferably, L₄ is O, S, NR₀₀, C(O), C(O)NR₀₀, NR₀₀C(O), -C(O)O-, -OC(O)-, SO, SOz, SO₂NR₀₀, or NR₀₀SO₂; R₀₀ is hydrogen or methyl; preferably, L₄ is -CH=CH- or -C≡C-; preferably, L₄ is CH₂, CH₂CH₂, or CH₂CH₂CH₂ (preferably CH₂, more preferably CH₂CH₂, even more preferably CH₂CH₂CH₂); preferably L₄ is O, preferably L₄ is NH or N(CH₃), preferably C(O), more preferably CH₂CH₂O; preferably, L₄ is a bond.

In some embodiments, L₄ is a C₃₋₆ cycloalkylene, 4- to 7-membered heterocyclylene, phenylene, or 5- or 6-membered heteroarylene (preferably cyclopropylene, cyclobutylene, cyclopentylene, azacyclopentylene, cyclohexylene, piperidinylene, piperazinylene, or 1,4-phenylene, more preferably 1,3-cyclobutylene, even more preferably 1,4-piperidylene or 1,4-piperazinylene).

In some embodiments, Linker represents: 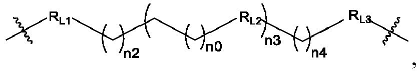 wherein
R_{L1} and R_{L3} are each independently O, S, CO, SO, SOz, N(R₀₀), or (CR₂₁R₂₂)ᵣ; R₂₁ and R₂₂ are hydrogen, acetyl, halogen, amino, C₁₋₁₀ alkyl, C₁₋₁₀ haloalkyl, C₁₋₁₀ alkoxy, C₂₋₁₀ alkenyl, C₂₋₁₀ alkynyl, C₃₋₈ cycloalkyl, 3- to 8-membered heterocycloalkyl, C₆₋₁₀ aryl, or 5- to 14-membered heteroaryl; or, R₂₁ and R₂₂, together with the C atom to which they are attached, form a 3- to 8-membered cycloalkyl or 3- to 8-membered heterocycloalkyl;
R_{L2}s are identical or different, and R_{L2} is O, S, CO, N(R₀₀), C(O)O, OC(O), C(O)NH, NHC(O) or NHC(O)NH, phenylene, alkynylene, cyclopropylene, 1,4-piperazinylene, or triazolylene; R₀₀ is hydrogen or C₁₋₁₀ alkyl, and n2 and n4 are each independently 0, 1, 2, 3, 4, 5, 6, 7, 8, 9, or 10;
r, n0, and n3 are 0, 1, 2, 3, or 4.

In some embodiments, Linker represents: 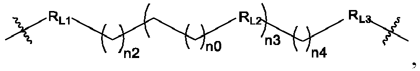 wherein
R_{L1} and R_{L3} are each independently O, CO, N(R₀₀), or (CR₂₁R₂₂)ᵣ; R₂₁ and R₂₂ are hydrogen, acetyl, C₁₋₅ alkyl, C₆₋₁₀ aryl, or C₁₋₅ haloalkyl; or, R₂₁ and R₂₂, together with the C atom to which they are attached, form a 3- to 8-membered cycloalkyl;
R_{L2}s are identical or different, and R_{L2} is O, CO, N(R₀₀), alkynylene 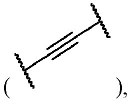 or cyclopropylene; R₀₀ is hydrogen or C₁₋₅ alkyl, and
n2 and n4 are each independently 0, 1, 2, or 3;
r, n0, and n3 are 0, 1, 2, 3, or 4.

Linker represents 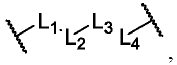 wherein
L₁, L₂, L₃, and L₄ each independently represents -R_{L1}-(CR₂₁R₂₂)ₙ₃-R_{L2}-(CR₃₁R₃₂)ₙ₂-,
R_{L1} and R_{L2} are each independently a bond, O, NR₀₀, -C(O)-, -CH=CH-, -C≡C-, -(CR₄₁R₄₂)ᵣ-, or C₃₋₈ cycloalkylene;
n2, n3, and r are each independently 0, 1, 2, 3, or 4;
R₄₁ and R₄₂ are each independently selected from hydrogen, acetyl, halogen, C₁₋₅ alkyl, C₁₋₅ haloalkyl, or C₆₋₁₀ aryl; or, R₂₁ and R₂₂, together with the C atom to which they are attached, form a 3- to 8-membered cycloalkyl.

In some embodiments, Linker represents: 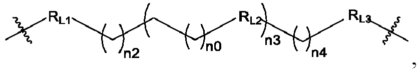 wherein
R_{L1} and R_{L3} are each independently O, N(R₀₀), or (CR₂₁R₂₂)ᵣ, wherein R₂₁ and R₂₂ are hydrogen or C₁₋₅ alkyl; or, R₂₁ and R₂₂, together with the C atom to which they are attached, form a 3- to 8-membered cycloalkyl;
R_{L2}s are identical or different, and R_{L2} is O, CO, N(R₀₀), alkynylene 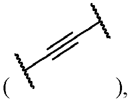 alkenylene 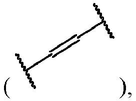 or cyclopropylene 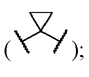 R₀₀ is hydrogen or C₁₋₅ alkyl,
n2 and n4 are each independently 0, 1, 2, or 3;
r, n0, and n3 are 0, 1, 2, 3, or 4.

Linker represents 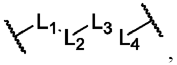 wherein
L₁, L₂, L₃, and L₄ each independently represents -R_{L1}-(CR₂₁R₂₂)ₙ₃-R_{L2}-(CR₃₁R₃₂)ₙ₂-,
each R_{L1} is independently a bond, O, NR₀₀, -C(O)-, -(CR₄₁R₄₂)₄-, or C₃₋₈ cycloalkylene;
each R_{L2} is independently a bond, O, NR₀₀, -C(O)-, -CH=CH-, -C≡C-, or C₃₋₈ cycloalkylene;
n2, n3, and r are each independently 0, 1, 2, 3, or 4;
R₄₁ and R₄₂ are each independently selected from hydrogen or C₁₋₅ alkyl; or, R₂₁ and R₂₂, together with the C atom to which they are attached, form a 3- to 8-membered cycloalkyl.

In some embodiments, Linker represents: 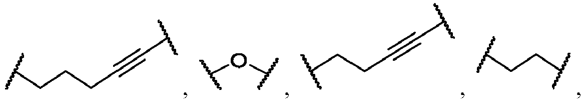 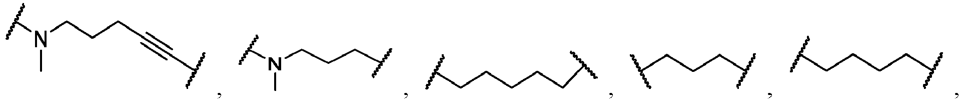 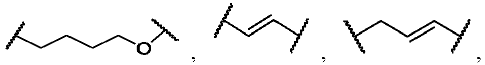 methylene,

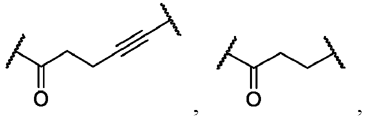

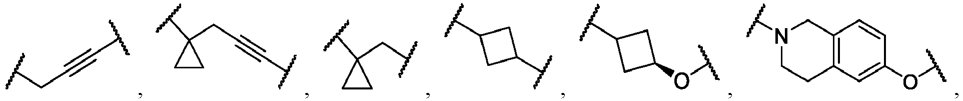

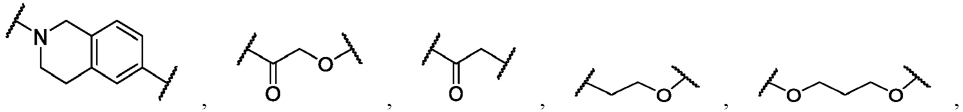

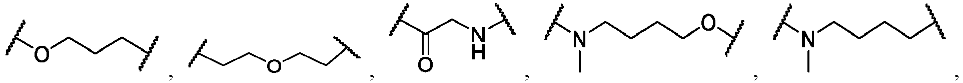

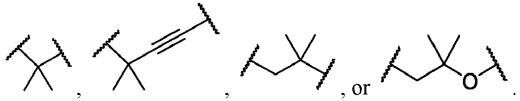

In some embodiments, R_{L1} is a bond, O, S, CO, SO, SO₂, NH, N(CH₃), CONH, NHCO, methylene, 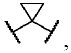 or ethenylene; R_{L2} is a bond, O, S, CO, SO, SO₂, NH, N(CH₃), CONH, NHCO, methylene, 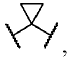 1,4-phenylene, or 1,4-piperazinylidene; R_{L3} is a bond, O, S, CO, SO, SO₂, NH, N(CH₃), CONH, NHCO, methylene, 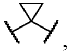 or ethenylene.

In some embodiments, W is a bond, O, S, NR₀₀, -C(O)-, -S(O)-, SO₂, -C(O)NR₀₀-, -NR₀₀C(O)-, - NR₀₀C(O)NR₀₀-, -SO₂NR₀₀-, -NR₀₀SO₂-, -NR₀₀C(O)O-, -C(O)O-, -OC(O)-, -OC(O)O-, -CH=CH-, -C≡C-, - (CR₄₃R₄₄)ₙ₈-, C₃₋₆ cycloalkylene, 3- to 6-membered heterocycloalkylene, phenylene, 5- to 6-membered heteroarylene, or 8- to 10-membered arylene or heteroarylene, wherein R₀₀, R₄₃, and R₄₄ are each independently hydrogen, methyl, ethyl, propyl, isopropyl, methoxy, ethoxy, isopropoxy, cyclopentyl, or cyclohexyl, or R₄₃ and R₄₄, together with the C atom to which they are attached, form cyclopropyl, cyclobutyl, cyclopentyl, or oxetanyl, and n₈ is 0, 1, 2, or 3; preferably, W is 1,2-phenylene, 1,3-phenylene, 1,4-phenylene, piperazinylene, or pyridinylene; preferably, W is CH₂, O, S, -N(methyl)-, -CH=CH-, or C≡C; preferably, W is CH₂; preferably, W is O or S; preferably, W is -CH=CH- or C≡C, preferably C≡C.

In some embodiments, ring B is selected from a 3- to 8-membered saturated or unsaturated cycloalkyl, 3- to 8-membered saturated or unsaturated heterocycloalkyl, 6- to 10-membered aryl, or 5- to 14-membered heteroaryl; preferably, ring B is a 5- to 7-membered saturated monocyclic heterocyclyl, benzene, or 5- to 6-membered heteroaryl; preferably, ring B is benzene or 5- to 6-membered heteroaryl; preferably, ring B is a 5-to 7-membered saturated monoheterocyclyl; preferably, ring B is cyclopentane, tetrahydropyrrole, 1,3-dioxolane, oxolane, 1,4-dioxane, furan, thiophene, thiazole, isothiazole, oxazole, isoxazole, pyrrole, imidazole, pyrazole, piperidine, piperazine, benzene, pyridine, pyridazine, or pyrimidine; preferably, ring B is 1,3-dioxolane, oxolane, 1,4-dioxane, imidazole, benzene, or pyrimidine; preferably, ring B is a 5- to 6-membered heteroaryl; preferably, ring B is 1,4-dioxane or 1,3-dioxolane.

In some embodiments, ring B is selected from 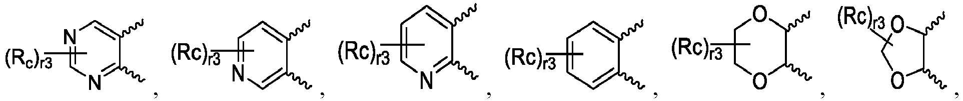 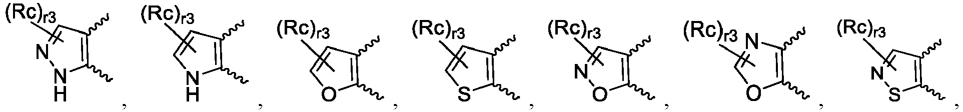 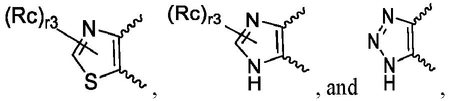 "
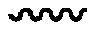 " represents a connection site;
Rc is hydrogen, F, Cl, Br, I, hydroxy, cyano, formyl, nitro, carboxy, acetyl, SOC₁₋₅ alkyl, SO₂C₁₋₅ alkyl, C₁₋₅ alkyl, C₁₋₅ haloalkyl, C₁₋₅ alkoxy, C₁₋₅ alkyl-OC₁₋₅ alkyl, C₃₋₆ cycloalkyl, 3- to 6-membered heterocycloalkyl, -O-C₃₋₆ cycloalkyl, or -O-3- to 6-membered heterocycloalkyl;
r3 is 0, 1, 2, or 3.

In some embodiments, V₁ and V₂ are both N, V₃ is CR₃, and V₄ is CR₄, wherein R₃ is hydrogen, and R₄ is hydrogen, halogen, amino, cyano, acetyl, C₁₋₃ alkyl, C₁₋₃ alkoxy, or C₁₋₃ haloalkyl (preferably fluorine or chlorine);
preferably, V₁ and V₂ are both N, V₃ is CR₃, and V₄ is CR₄, wherein R₃ and R₄, together with the atoms to which they are attached, form a substituted or unsubstituted 5- to 6-membered heteroaryl; the substitution refers to that the 5- to 6-membered heteroaryl is optionally substituted with 1, 2, or 3 substituents selected from hydrogen, halogen, amino, cyano, acetyl, C₁₋₃ alkyl, C₁₋₃ alkoxy, or C₁₋₃ haloalkyl;
preferably, R₃ and R₄, together with the atoms to which they are attached, form 1*H*-pyrrole, 1*H*-pyrazole, or 1*H*-imidazole.

In some embodiments, L₅ and L₆ are each independently selected from a bond, NH, CONH, NHCO, C₁₋₃ alkylene (preferably methylene or ethylene), C₂₋₆ alkenylene, or C₂₋₆ alkynylene.

In some embodiments, E further represents: 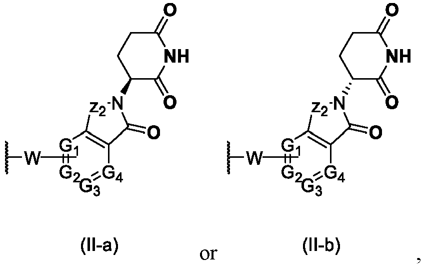 wherein
Z₂, G₁, G₂, G₃, G₄, and W are as defined above.

In some embodiments, G₁ is N, G₂ is CR_{G2}, G₃ is CR_{G3}, and G₄ is CR_{G4}; preferably, G₁ is CR_{G1}, G₂ is N, G₃ is CR_{G3}, and G₄ is CR_{G4}; preferably, G₁ is CR_{G1}, G₂ is CR_{G2}, G₃ is N, and G₄ is CR_{G4}; preferably, G₁ is CR_{G1}, G₂ is CR_{G2}, G₃ is CR_{G3}, and G₄ is N; preferably, G₁ is N, G₂ is N, G₃ is CR_{G3}, and G₄ is CR_{G4}; preferably, G₁ is N, G₂ is CR_{G2}, G₃ is N, and G₄ is CR_{G4}; preferably, G₁ is N, G₂ is CR_{G2}, G₃ is CR_{G3}, and G₄ is N; preferably, G₁ is CR_{G1}, G₂ is N, G₃ is N, and G₄ is CR_{G4}; preferably, G₁ is CR_{G1}, G₂ is N, G₃ is CR_{G3}, and G₄ is N; preferably, G₁ is CR_{G1}, G₂ is CR_{G2}, G₃ is N, and G₄ is N; preferably, G₁ is N, G₂ is N, G₃ is N, and G₄ is CR_{G4}; preferably, G₁ is N, G₂ is N, G₃ is CR_{G3}, and G₄ is N; preferably, G₁ is N, G₂ is CR_{G2}, G₃ is N, and G₄ is N; preferably, G₁ is CR_{G1}, G₂ is N, G₃ is N, and G₄ is N; preferably, G₁ is CR_{G1}, G₂ is CR_{G2}, G₃ is CR_{G3}, and G₄ is CR_{G4}; R_{G1}, R_{G2}, R_{G3}, and R_{G4} are as defined in one of the above items.

In some embodiments, one of R_{G1}, R_{G2}, R_{G3}, and R_{G4} is attached to W, the others are each independently selected from hydrogen, hydroxy, cyano, acetyl, fluorine, chlorine, bromine, amino, methyl, ethyl, propyl, isopropyl, methoxy, ethoxy, fluoromethyl, difluoromethyl, trifluoromethyl, -CH₂CH₂F, -CHFCH₃, -CH₂CHF₂, -CF₂CH₃, -CHFCH₂F, -CH₂CF₃, -CF₂CH₂F, or -CHFCHF₂; preferably, R_{G1}, R_{G2}, R_{G3}, and R_{G4} are each independently hydrogen, halogen (preferably F), C₁₋₃ alkyl, or C₁₋₃ haloalkyl.

In some embodiments, Z₁ is N, and Z₂ is CH₂ or C(O).

In some embodiments, E is further selected from 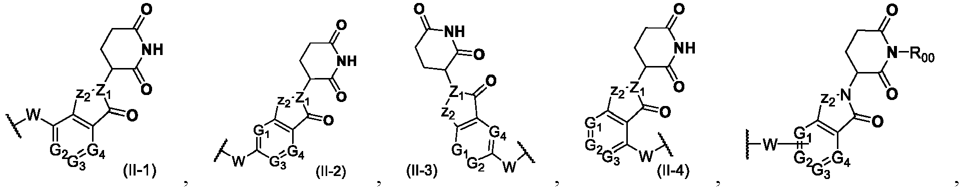 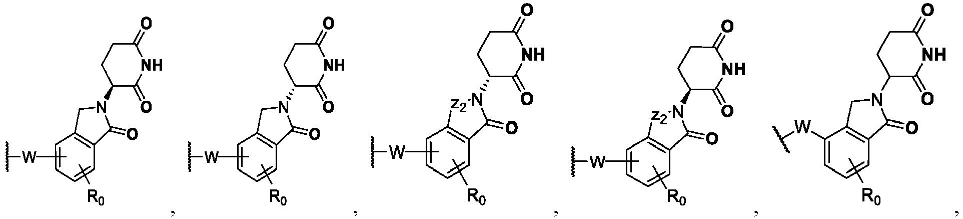 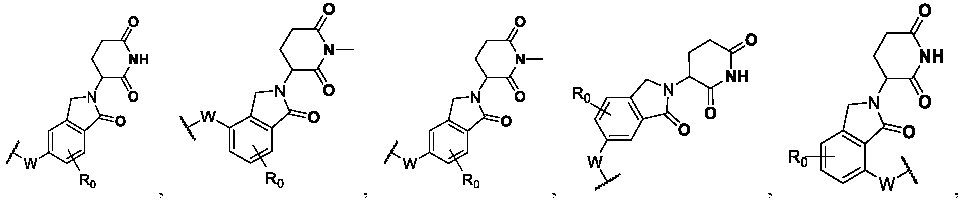 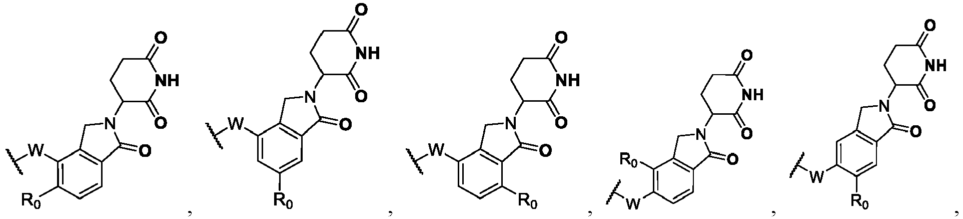 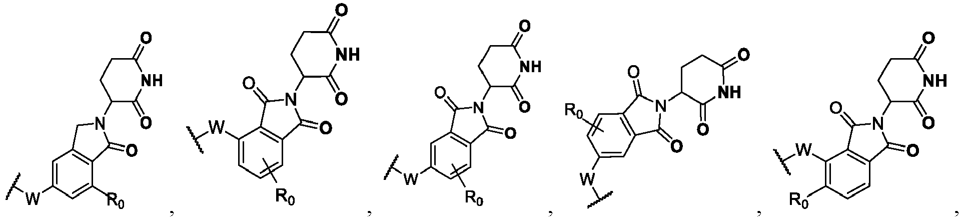 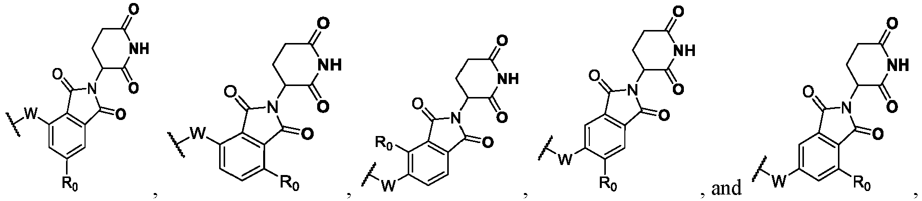 wherein Z₁, Z₂, G₁, G₂, G₃, G₄, and W are as defined in one of the above items.

Preferably, R_{G1}, R_{G2}, R_{G3}, R_{G4}, and R₀ are each independently hydrogen, deuterium, cyano, halogen (preferably F), C₁₋₃ alkyl, or C₁₋₃ haloalkyl (preferably methyl or trifluoromethyl).

In some embodiments, J₁ is N, J₂ is CR_{J2}, J₃ is CR_{J3}, and J₄ is CR_{J4}; preferably, J₁ is CR_{J1}, J₂ is N, J₃ is CR_{J3}, and J₄ is CR_{J4}; preferably, J₁ is CR_{J1}, J₂ is CR_{J2}, J₃ is N, and J₄ is CR_{J4}; preferably, J₁ is CR_{J1}, J₂ is CR_{J2}, J₃ is CR_{J3}, and J₄ is N; preferably, J₁ is N, J₂ is N, J₃ is CR_{J3}, and J₄ is CR_{J4}; preferably, J₁ is N, J₂ is CR_{J2}, J₃ is N, and J₄ is CR_{J4}; preferably, J₁ is N, J₂ is CR_{J2}, J₃ is CR_{J3}, and J₄ is N; preferably, J₁ is CR_{J1}, J₂ is N, J₃ is N, and J₄ is CR_{J4}; preferably, J₁ is CR_{J1}, J₂ is N, J₃ is CR_{J3}, and J₄ is N; preferably, J₁ is CR_{J1}, J₂ is CR_{J2}, J₃ is N, and J₄ is N; R_{J1}, R_{J2}, R_{J3}, and R_{J4} are defined in any one of the above items.

R₀ is selected from hydrogen, halogen (preferably F), C₁₋₃ alkyl, or C₁₋₃ haloalkyl, preferably F, methyl, trifluoromethyl, cyano, or difluoromethyl.

In some embodiments, R_{J1}, R_{J2}, R_{J3}, and R_{J4} are each independently selected from H, F, Cl, Br, I, methyl, ethyl, propyl, isopropyl, methoxy, ethoxy, propoxy, isopropoxy, cyclopropyl, cyclobutyl, oxetanyl, fluoromethyl, difluoromethyl, trifluoromethyl, -CH₂CH₂F, -CHFCH₃, -CH₂CHF₂, -CF₂CH₃, -CHFCH₂F, -CH₂CF₃, - CF₂CH₂F, or -CHFCHF₂;
preferably, R_{J3} and R_{J4} are hydrogen, and R_{J1} and R_{J2} are each independently selected from F, Cl, Br, I, methyl, ethyl, propyl, cyclopropyl, or substituted or unsubstituted benzene or 5- to 6-membered heteroaryl; the substitution refers to that the benzene or 5- to 6-membered heteroaryl is optionally substituted with 1, 2, or 3 substituents selected from hydrogen, halogen, amino, cyano, acetyl, C₁₋₃ alkyl, C₁₋₃ alkoxy, or C₁₋₃ haloalkyl; preferably, R_{J1}, R_{J3}, and R_{J4} are hydrogen, and R_{J2} is selected from hydrogen, F, Cl, Br, and C₁₋₅ alkyl; preferably, R_{J3} and R_{J4} are hydrogen, and R_{J1} and R_{J2} are each independently selected from hydrogen, F, Cl, Br, and C₁₋₅ alkyl;
preferably, R_{J1}, R_{J3}, and R_{J4} are hydrogen, and R_{J2} is selected from H, F, Cl, Br, methyl, ethyl, 3- to 8-membered cycloalkyl, benzene, and 5- to 6-membered heteroaryl; preferably, R_{J1}, R_{J2}, R_{J3}, and R_{J4} are hydrogen; preferably, R_{J1}, R_{J3}, and R_{J4} are hydrogen, and R_{J2} is methyl, ethyl, cyclopropyl, benzene, or 1-methyl-1*H-*pyrazol-4-yl, 1-methyl-1*H*-imidazol-4-yl, 1-ethyl-1*H-*pyrazol-4-yl, 1-ethyl-1*H*-imidazol-4-yl, 1-isopropyl-1*H-*pyrazol-4-yl, or 1-isopropyl-1*H*-imidazol-4-yl.

In some embodiments, R_{J1}, R_{J2}, and R_{J4} are hydrogen, and R_{J3} and Rₖ₃ are connected to form a bond, which is a single, double or triple bond (preferably a C-C single bond).

In some embodiments, Q is C(R^{q1})₃, C(O)R^{q1}, S(O)R^{q1}, SO₂R^{q1}, P(O)R^{q1}R^{q2}, or NR^{q1}R^{q2}, wherein R^{q1} and R^{q2} are each independently selected from methyl, ethyl, propyl, isopropyl, amino, acetyl, methylsulfonyl, acylamino, and aminoacyl. Preferably, R^{q1} and R^{q2} are each independently C₁₋₆ alkyl or C₁₋₆ alkoxy.

In some embodiments, Q is P(O)R^{q1}R^{q2}, wherein R^{q1} and R^{q2} are each independently C₁₋₆ alkyl or C₁₋₆ alkoxy.

In some embodiments, J₁ is N or CH.

In some embodiments, J₂ is N or CH.

In some embodiments, J₃ is N or CH.

In some embodiments, J₄ is N or CH.

In some embodiments, V₁ is N or CH.

In some embodiments, V₂ is N or CH.

In some embodiments, V₃ is N or CR³, R₃ is hydrogen, or R₃ and R₄, together with the atoms to which they are attached, form a 5- to 6-membered heteroaryl or benzene substituted with 0, 1, or 2 R⁴¹s; the heteroatom of the 5- to 6-membered heteroaryl is nitrogen, and the number of the heteroatom is 1, 2, or 3.

In some embodiments, K₁ is N or CH.

In some embodiments, K₂ is N or CRₖ₂.

In some embodiments, L₅ is NH.

In some embodiments, L₆ is NH. In some embodiments, 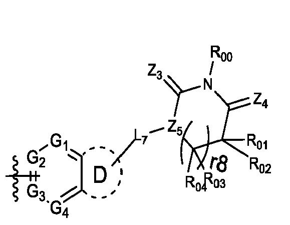 is

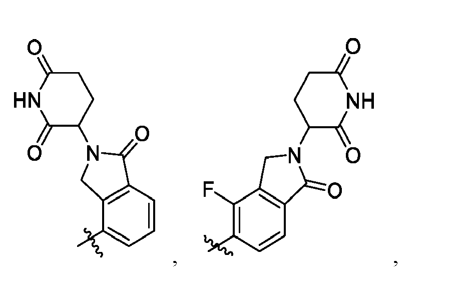

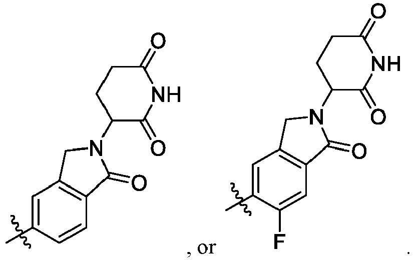

In some embodiments, W is O or -C≡C-.

In some embodiments, Q is P(O)R^{q1}R^{q2}, wherein R^{q1} and R^{q2} are each independently C₁₋₆ alkyl or C₁₋₆ alkoxy;
J₁ is N or CH;
J₂ is N or CH;
J₃ is N or CH;
J₄ is N or CH;
V₁ is N or CH;
V₂ is N or CH;
V₃ is N or CR³; R₃ is hydrogen,
V₄ is N or CR₄, wherein R₄ is hydrogen, hydroxy, cyano, C(O)NRₐR_{b}, halogen, C₁₋₁₀ alkyl substituted with 0, 1, or 2 R⁴¹s, or 5- to 6-membered heteroaryl substituted with 0, 1, or 2 R⁴¹s; the heteroatom of the 5- to 6-membered heteroaryl is selected from one or more of nitrogen, oxygen, and sulfur, and the number of the heteroatom is 1, 2, or 3;
or, R₃ and R₄, together with the atoms to which they are attached, form a 5- to 6-membered heteroaryl or benzene substituted with 0, 1, or 2 R⁴¹s; the heteroatom of the 5- to 6-membered heteroaryl is selected from one or more of nitrogen, oxygen, and sulfur, and the number of the heteroatom is 1, 2, or 3;
Rₐ and R_{b} are each independently hydrogen, cyano, acetyl, hydroxy, carboxy, nitro, halogen, C₁₋₁₀ alkyl, C₁₋₁₀ haloalkyl, C₁₋₁₀ alkoxy, C₁₋₁₀ alkylthio, NR_{c}R_{d}, C₃₋₈ cycloalkyl, 3- to 8-membered heterocycloalkyl, C₆₋₁₀ aryl or 5- to 14-membered heteroaryl; or Rₐ and R_{b}, together with the N atom to which they are attached, form a 3-to 8-membered heterocycloalkyl; the heteroatom of the 5- to 14-membered heteroaryl is selected from one or more of nitrogen, oxygen, and sulfur, and the number of the heteroatom is 1, 2, or 3; the heteroatom of the 3-to 8-membered heterocycloalkyl is selected from one or more of nitrogen, oxygen, and sulfur, and the number of the heteroatom is 1, 2, or 3;
R⁴¹ is selected from hydrogen, deuterium, halogen, C₁₋₆ alkyl, C₁₋₆ haloalkyl, C₁₋₆ alkoxy, C₁₋₆ alkylthio, C₃₋₈ cycloalkyl, 3- to 8-membered heterocyclyl, OC₃₋₈ cycloalkyl, or -O-3- to 8-membered heterocyclyl; the heteroatom of the 3- to 8-membered heterocycloalkyl is selected from one or more of nitrogen, oxygen, and sulfur, and the number of the heteroatom is 1, 2, or 3;
K₂ is N or CRₖ₂; Rₖ₂ is hydrogen, F, Cl, Br, I, amino, hydroxy, cyano, acetyl, C₁₋₅ alkyl, C₁₋₅ haloalkyl, - CONH₂, C₁₋₅ alkoxy, C₁₋₃ alkylene-OC₁₋₃ alkyl, C₃₋₆ cycloalkyl, or C₃₋₆ heterocycloalkyl optionally substituted with 0, 1, 2, 3, or more substituents selected from deuterium, halogen, hydroxy, cyano, C₁₋₅ alkyl, C₁₋₅ haloalkyl, and C₁₋₅ alkoxy; preferably R_{K4} is selected from hydrogen, deuterium, acylamino, cyano, and C₁₋₅ alkyl;
Rₖ₃ is selected from 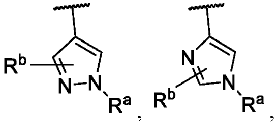 halogen, cyano, acetyl, C₁₋₅ alkyl, C₁₋₅ alkylene-OC₁₋₅ alkyl, or C₃₋₆ cycloalkyl, wherein the C₁₋₅ alkyl is optionally substituted with 0, 1, 2, 3, 4, or 5 substituents selected from deuterium, halogen, hydroxy, cyano, C₁₋₃ alkyl, C₁₋₃ haloalkyl, and C₁₋₃ alkoxy; Rₐ and R_{b} are H, F, Cl, Br, I, methyl, ethyl, propyl, isopropyl, methoxy, ethoxy, propoxy, isopropoxy, cyclopropyl, cyclobutyl, oxetanyl, fluoromethyl, difluoromethyl, trifluoromethyl, -CH₂CH₂F, -CHFCH₃, -CH₂CHF₂, - CF₂CH₃, -CHFCH₂F, -CH₂CF₃, -CF₂CH₂F, or -CHFCHF₂; Rₐ and R_{b} are H, F, Cl, Br, I, methyl, ethyl, propyl, isopropyl, methoxy, ethoxy, propoxy, isopropoxy, cyclopropyl, cyclobutyl, oxetanyl, fluoromethyl, difluoromethyl, trifluoromethyl, -CH₂CH₂F, -CHFCH₃, -CH₂CHF₂, -CF₂CH₃, -CHFCH₂F, -CH₂CF₃, - CF₂CH₂F, or -CHFCHF₂;
K₄ is N or CRₖ₄; Rₖ₄ is hydrogen, F, Cl, Br, I, amino, hydroxy, cyano, acetyl, C₁₋₅ alkyl, C₁₋₅ haloalkyl, - CONH₂, C₁₋₅ alkoxy, C₁₋₃ alkylene-OC₁₋₃ alkyl, C₃₋₆ cycloalkyl, or C₃₋₆ heterocycloalkyl optionally substituted with 1, 2, 3, 4, or more groups selected from the following B2 groups, and the B2 group is selected from deuterium, halo, hydroxy, cyano, C₁₋₅ alkyl, C₁₋₅ haloalkyl, and C₁₋₅ alkoxy;
L₅ is NH;
L₆ is NH;
ring A is a 3- to 8-membered monocyclic heterocyclylene or 7 -to 16-membered spiroheterocyclylene; the heteroatom of the monocyclic heterocyclylene is selected from one or more of nitrogen, oxygen, and sulfur, and the number of the heteroatom is 1, 2, or 3; the heteroatom of the spiroheterocyclylene is selected from one or more of nitrogen, oxygen, and sulfur, and the number of the heteroatom is 1, 2, or 3;
R is a bond, or a 7- to 11-membered mono-spiroheterocyclylene, 3- to 8-membered monocyclic heterocyclylene and 8- to 10-membered bicyclic heteroarylene substituted with 0, 1, 2, or 3 R₀s, wherein R₀ is selected from hydrogen, deuterium, halogen, =O, C₁₋₅ alkyl, C₁₋₅ haloalkyl, C₁₋₅ alkoxy, C₃₋₈ cycloalkyl, and 3- to 8-membered heterocyclyl, or two R₀s, together with the C atom to which they are attached, form a 3- to 6-membered cycloalkyl; the heteroatom of the mono-spiroheterocyclylene is selected from one or more of nitrogen, oxygen, and sulfur, and the number of the heteroatom is 1, 2, or 3; the heteroatom of the bicyclic heteroarylene is selected from one or more of nitrogen, oxygen, and sulfur, and the number of the heteroatom is 1, 2, or 3; the heteroatom of the monocyclic heterocyclylene is selected from one or more of nitrogen, oxygen, and sulfur, and the number of the heteroatom is 1, 2, or 3; the heteroatom of the 3- to 8-membered heterocyclyl is selected from one or more of nitrogen, oxygen, and sulfur, and the number of the heteroatom is 1, 2, or 3;
Linker represents: 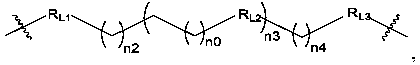 wherein
   R_{L1} and R_{L3} are each independently O, CO, N(R₀₀), or (CR₂₁R₂₂)ᵣ; R₂₁ and R₂₂ are hydrogen, acetyl, C₁₋₅ alkyl, C₆₋₁₀ aryl, or C₁₋₅ haloalkyl; or, R₂₁ and R₂₂, together with the C atom to which they are attached, form a 3- to 8-membered cycloalkyl;
R_{L2}s are identical or different, and R_{L2} is O, CO, N(R₀₀), alkynylene 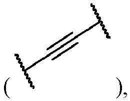 or cyclopropylene; R₀₀ is hydrogen or C₁₋₅ alkyl, and
n2 and n4 are each independently 0, 1, 2, or 3;
r, n0, and n3 are 0, 1, 2, 3, or 4.

In some embodiments, Q is P(O)R^{q1}R^{q2}, wherein R^{q1} and R^{q2} are each independently C₁₋₅ alkyl or C₁₋₅ alkoxy;
J₁ is N or CH;
J₂ is N or CH;
J₃ is N or CH;
J₄ is N or CH;
V₁ is N or CH;
V₂ is N or CH;
V₃ is N or CR³; R₃ is hydrogen;
V₄ is N or CR₄; R₄ is hydrogen, hydroxy, cyano, C(O)NRₐR_{b}, halogen, C₁₋₁₀ alkyl substituted with 0, 1, or 2 R⁴¹s, or 5- to 6-membered heteroaryl substituted with 0, 1, or 2 R⁴¹s; the heteroatom of the 5- to 6-membered heteroaryl is nitrogen and/or oxygen, and the number of the heteroatom is 1, 2, or 3;
or, R₃ and R₄, together with the atoms to which they are attached, form a 5- to 6-membered heteroaryl or benzene substituted with 0, 1, or 2 R⁴¹s; the heteroatom of the 5- to 6-membered heteroaryl is nitrogen and/or oxygen, and the number of the heteroatom is 1, 2, or 3;
Rₐ and R_{b} are each independently hydrogen or C₁₋₅ alkyl;
R⁴¹ is selected from hydrogen, deuterium, halogen, C₁₋₅ alkyl, C₁₋₅ haloalkyl, or C₁₋₅ alkoxy;
K₁ is N or CH;
K₂ is N or CRₖ₂; Rₖ₂ is hydrogen, cyano, F, Cl, Br, I, C₁₋₅ alkyl, -CONH₂, C₁₋₅ haloalkyl, or C₁₋₅ alkoxy, wherein the C₁₋₅ alkyl and C₁₋₅ alkoxy are optionally substituted with 1, 2, 3, 4, or more groups selected from the following B2 groups, and the B2 group is selected from deuterium, halo, hydroxy, cyano, C₁₋₅ alkyl, C₁₋₅ haloalkyl, and C₁₋₅ alkoxy;
Rₖ₃ is selected from 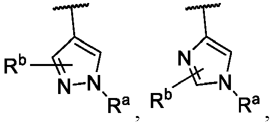 F, Cl, Br, I, cyano, acetyl, ethyl, C₁₋₅ alkylene-OC₁₋₅ alkyl, or C₃₋₆ cycloalkyl, wherein the C₁₋₅ alkyl is optionally substituted with 0, 1, 2, 3, 4, or 5 substituents selected from deuterium, hydroxy, cyano, C₁₋₃ alkyl, and C₁₋₃ alkoxy; R^{a} is H, methyl, ethyl, propyl, or isopropyl; R^{b} is H, methyl, ethyl, propyl, isopropyl, methoxy, ethoxy, propoxy, isopropoxy, cyclopropyl, cyclobutyl, oxetanyl, fluoromethyl, difluoromethyl, trifluoromethyl, -CH₂CH₂F, -CHFCH₃, -CH₂CHF₂, -CF₂CH₃, -CHFCH₂F, - CH₂CF₃, -CF₂CH₂F, or -CHFCHF₂;
K₄ is N or CRₖ₄; Rₖ₄ is hydrogen, F, Cl, Br, I, cyano, acetyl, or C₁₋₅ alkoxy, preferably hydrogen, F, Cl, Br, I, or C₁₋₅ alkoxy;
L₅ is NH;
L₆ is NH;
ring A is a 3- to 6-membered monocyclic heterocyclylene or 7 -to 11-membered spiroheterocyclylene; the heteroatom of the monocyclic heterocyclylene is nitrogen, oxygen, or sulfur, and the number of the heteroatom is 1, 2, or 3; the heteroatom of the spiroheterocyclylene is nitrogen, oxygen, or sulfur, and the number of the heteroatom is 1, 2, or 3;
R is a bond, or a 7- to 11-membered mono-spiroheterocyclylene, 3- to 8-membered monocyclic heterocyclylene and 8- to 10-membered bicyclic heteroarylene substituted with 0, 1, 2, or 3 R₀s, wherein R₀ is selected from hydrogen, =O, and C₁₋₅ alkyl, or two R₀s, together with the C atom to which they are attached, form a 3- to 6-membered cycloalkyl; the heteroatom of the bicyclic heteroarylene is nitrogen, oxygen, or sulfur, and the number of the heteroatom is 1, 2, or 3; the heteroatom of the spiroheterocyclylene is nitrogen, oxygen, or sulfur, and the number of the heteroatom is 1, 2, or 3; the heteroatom of the monocyclic heterocyclylene is nitrogen, oxygen, or sulfur, and the number of the heteroatom is 1, 2, or 3;
Linker represents: 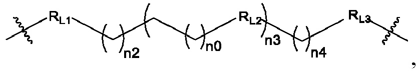 wherein
   R_{L1} and R_{L3} are each independently O, N(R₀₀), or (CR₂₁R₂₂)ᵣ, wherein R₂₁ and R₂₂ are hydrogen or C₁₋₅ alkyl; or, R₂₁ and R₂₂, together with the C atom to which they are attached, form a 3- to 8-membered cycloalkyl or 3- to 8-membered heterocycloalkyl;
R_{L2}s are identical or different, and R_{L2} is O, CO, N(R₀₀), alkynylene, alkenylene, or cyclopropylene; R₀₀ is hydrogen or C₁₋₅ alkyl;
n2 and n4 are each independently 0, 1, 2, or 3;
r, n0, and n3 are 0, 1, 2, 3, or 4.

In some embodiments, Q is P(O)R^{q1}R^{q2}, wherein R^{q1} and R^{q2} are each independently C₁₋₅ alkyl or C₁₋₅ alkoxy;
J₁ is N or CH;
J₂ is N or CH;
J₃ is N or CH;
J₄ is N or CH;
V₁ is N or CH;
V₂ is N or CH;
V₃ is N or CR³; R₃ is hydrogen;
V₄ is N or CR₄; R₄ is hydrogen, halogen, or 5- to 6-membered heteroaryl; the heteroatom of the 5- to 6-membered heteroaryl is nitrogen, and the number of the heteroatom is 1, 2, or 3;
or, R₃ and R₄, together with the atoms to which they are attached, form a 5- to 6-membered heteroaryl or benzene; the heteroatom of the 5- to 6-membered heteroaryl is nitrogen, and the number of the heteroatom is 1, 2, or 3;
Rₐ and R_{b} are each independently hydrogen or C₁₋₁₀ alkyl; Rₐ and R_{b} are each independently hydrogen or C₁₋₁₀ alkyl;
K₁ is N or CH;
K₂ is N or CRₖ₂; Rₖ₂ is hydrogen, F, Cl, Br, I, C₁₋₅ alkyl, C₁₋₅ haloalkoxy, or C₁₋₅ alkoxy;
Rₖ₃ is selected from 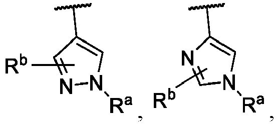 F, Cl, Br, I, cyano, acetyl, ethyl, C₁₋₅ alkylene-OC₁₋₅ alkyl, or C₃₋₆ cycloalkyl, wherein the C₁₋₅ alkyl is optionally substituted with 0, 1, 2, or 3 substituents selected from deuterium, hydroxy, cyano, C₁₋₃ alkyl, or C₁₋₃ alkoxy; R^{a} is H, methyl, ethyl, propyl, or isopropyl; R^{b} is H, methyl, ethyl, propyl or isopropyl;
K₄ is N or CRₖ₄; Rₖ₄ is hydrogen or C₁₋₅ alkoxy;
L₅ is NH;
L₆ is NH;
ring A is a 3- to 8-membered monocyclic heterocyclylene or 7 -to 11-membered spiroheterocyclylene; the heteroatom of the monocyclic heterocyclylene is N, and the number of the heteroatom is 1 or 2; the heteroatom of the spiroheterocyclylene is N, and the number of the heteroatom is 1 or 2;
R is a bond, or a 7- to 11-membered mono-spiroheterocyclylene, 6-membered monocyclic heterocyclylene and 8- to 10-membered bicyclic heteroarylene substituted with 0, 1, 2, or 3 R₀s, wherein R₀ is selected from hydrogen, =O, and C₁₋₅ alkyl, or two R₀s, together with the C atom to which they are attached, form a 3- to 6-membered cycloalkyl; the heteroatom of the mono-spiroheterocyclylene is N, and the number of the heteroatom is 1 or 2; the heteroatom of the bicyclic heteroarylene is N, and the number of the heteroatom is 1 or 2; the heteroatom of the 6-membered monocyclic heterocyclylene is N, and the number of the heteroatom is 1 or 2.

In some embodiments, PIN further represents: 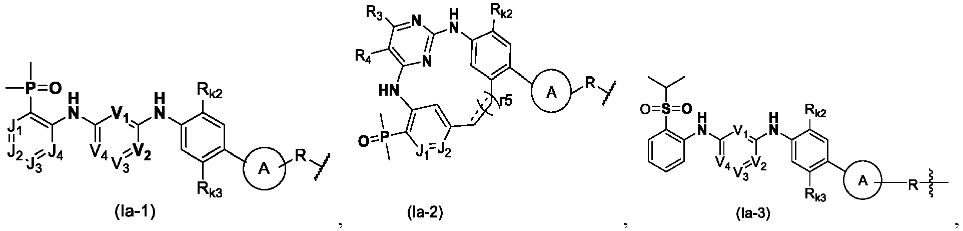 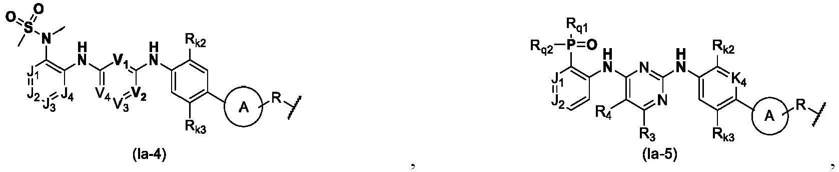 or 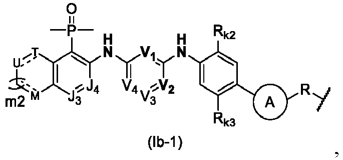 wherein
V₁, V₂, V₃, V₄, J₁, J₂, J₃, J₄, Rₖ₂, Rₖ₃, R₃, R₄, ring A, and R are defined in any one of the above items;
or, R₃ and R₄, together with the atoms to which they are attached, form 1H pyrrole;
"---" is a single bond or absent; r5 is 0, 1, 2, 3, 4, or 5;
U, T, and M are each independently C, N, O, or S, and U, T, and M are each independently substituted, within a valence-permitted range, with 1 or 2 R⁰s, wherein R⁰ is selected from hydrogen, hydroxy, halogen, cyano, acetyl, C₁₋₅ alkyl (preferably C₁₋₃ alkyl), C₁₋₅ alkoxy (preferably C₁₋₃ alkoxy), C₁₋₅ haloalkyl (preferably C₁₋₃ haloalkyl), C₃₋₆ cycloalkyl, or 3- to 6-membered heterocycloalkyl; m₂ is 0, 1, or 2; preferably R⁰ is hydrogen or halogen (preferably fluorine or chlorine).

In some embodiments, it further represents the following formulas: 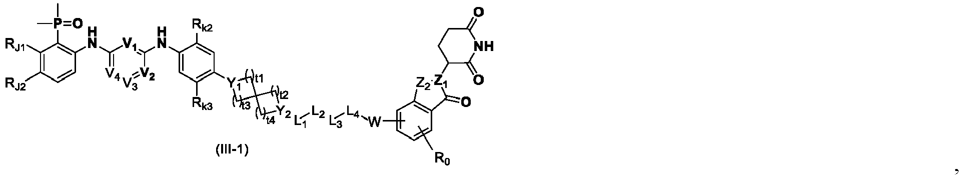 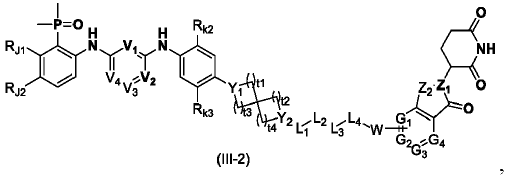 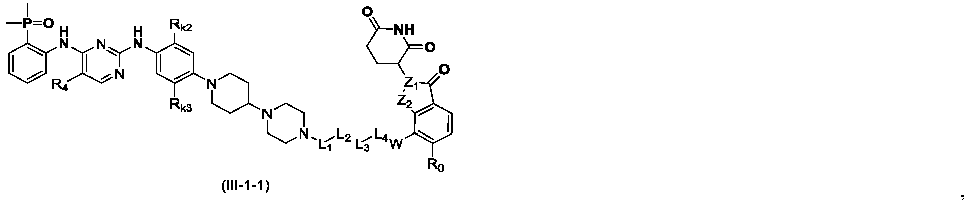 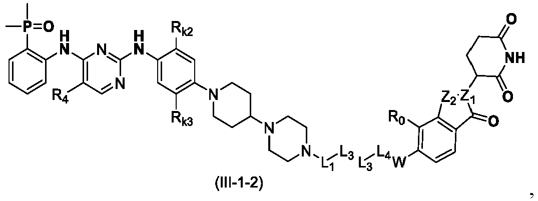 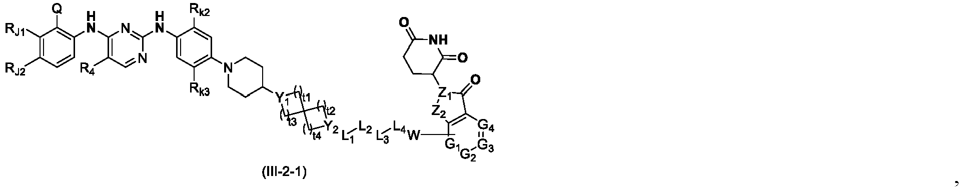 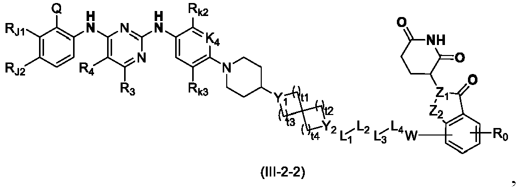 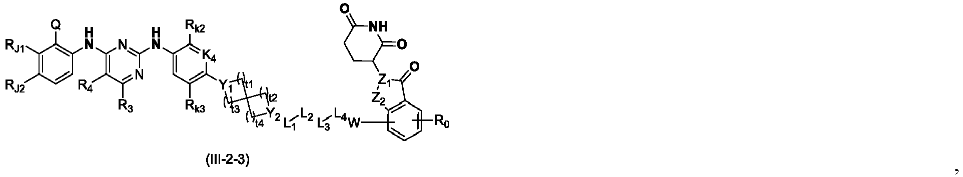 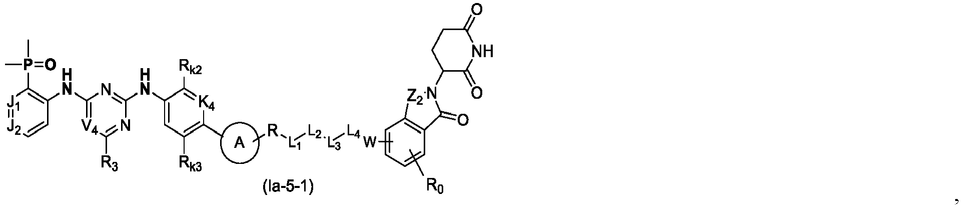 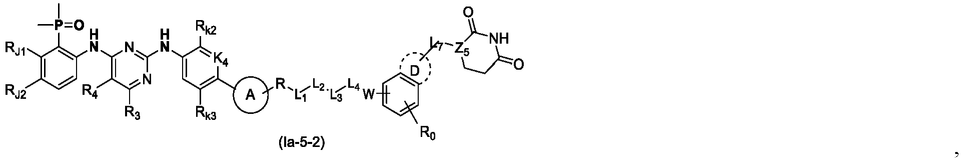 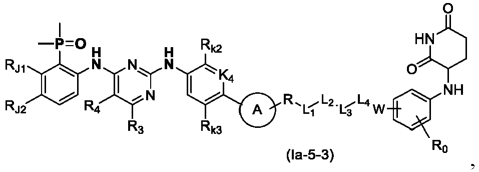 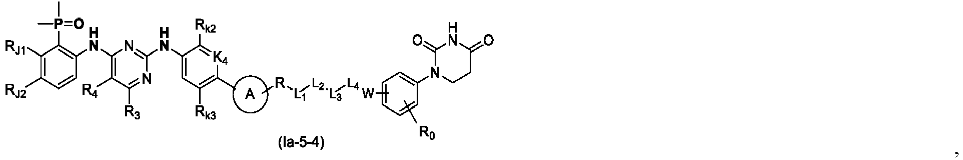 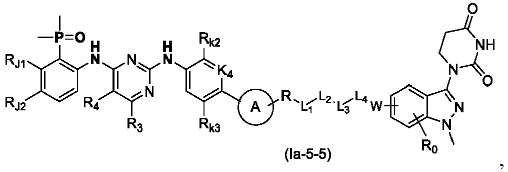 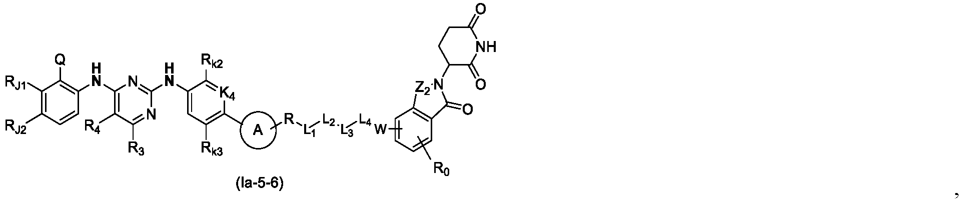 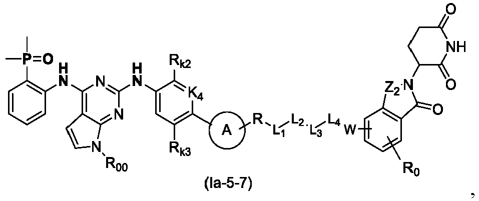 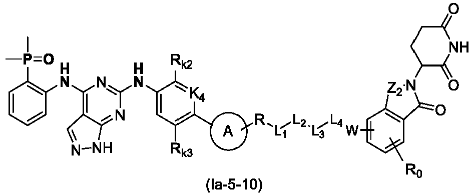 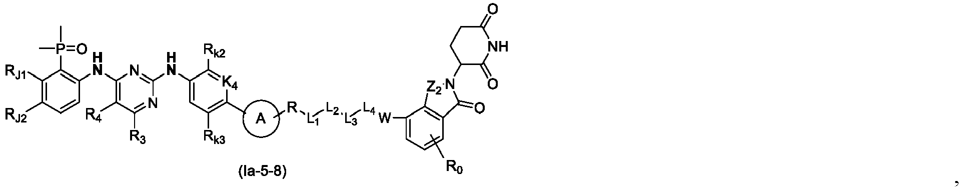 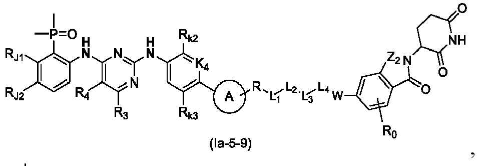 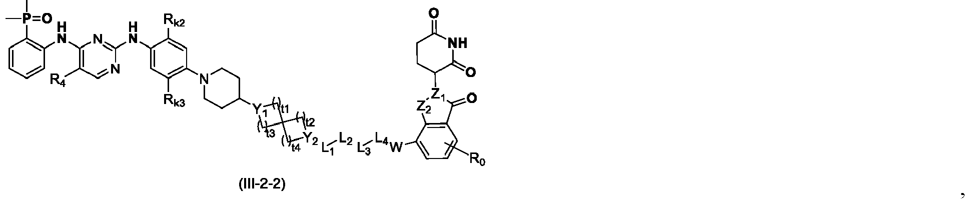 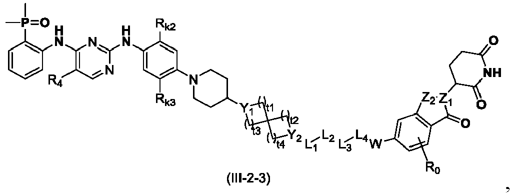 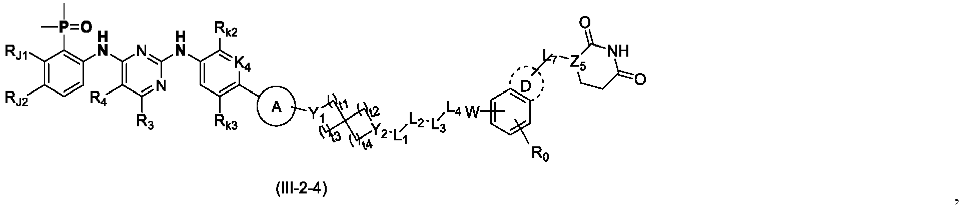 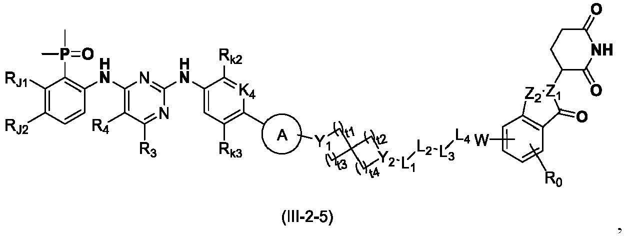 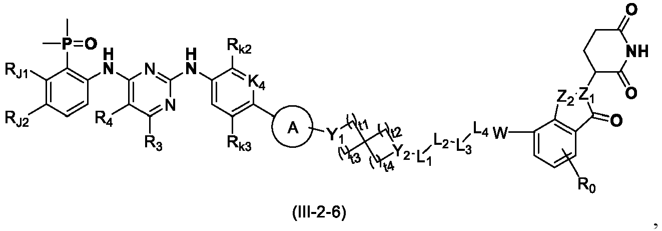 or 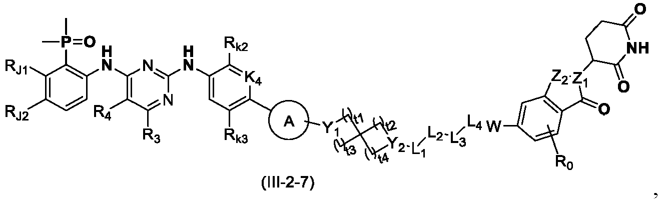 wherein
R_{J1}, R_{J2}, V₁, V₂, V₃, V₄, Rₖ₂, Rₖ₃, Y₁, Y₂, t1, t2, t3, t4, L₁, L₂, L₃, L₄, W, G₁, G₂, G₃, G₄, Z₁, Z₂, and R₀ are as defined in any one of the above items; preferably, hydrogen of the compound of formula (I) may optionally be substituted with 1, 2, 3, 4, 5, or more deuterium. In some embodiments, the compound of formula (I), or the pharmaceutically acceptable salt, the enantiomer, the diastereomer, the racemate, the solvate, the hydrate, the polymorph, the prodrug or the isotopic variant thereof, and the mixture thereof are further represented by formula (III-1-1) or (III-1-2): 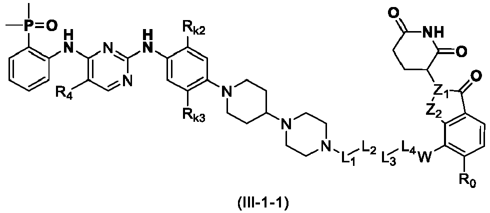 or 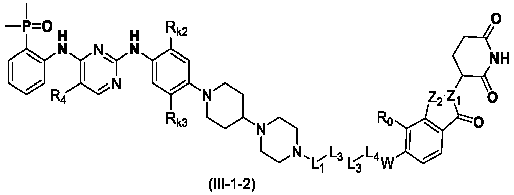
wherein R₄ is halogen (preferably chlorine or bromine);
Rₖ₂ is hydrogen or C₁₋₅ alkoxy (preferably C₁₋₃ alkoxy, more preferably methoxy);
Rₖ₃ is halogen, acetyl, cyano, C₁₋₅ alkyl, C₃₋₆ cycloalkyl, C₁₋₅ haloalkyl, or C₁₋₅ alkoxy;
L₁ is a bond, CH₂, CH₂CH₂, CH₂CH₂CH₂, CO, NH, CONH, NHCO, -N(methyl)-, O, C(O)O, OC(O), or (CR₂₁R₂₂)n₃, wherein R₂₁ and R₂₂ are each independently hydrogen or methyl, or R₂₁ and R₂₂, together with the C atom to which they are attached, form a C₃₋₆ cycloalkyl or 3- to 6-membered heterocycloalkyl, and n₃ is 0, 1, 2, or 3;
L₂ is a bond, CH₂, CH₂CH₂, CH₂CH₂CH₂, CO, NH, CONH, NHCO, O, C(O)O, OC(O), or (CR₂₁R₂₂)n₃, wherein R₂₁ and R₂₂ are each independently hydrogen or methyl, or R₂₁ and R₂₂, together with the C atom to which they are attached, form a C₃₋₆ cycloalkyl or 3- to 6-membered heterocycloalkyl, and n₃ is 0, 1, 2, or 3; L₃ is a bond, CH₂, CH₂CH₂, CH₂CH₂CH₂, CO, NH, CONH, NHCO, O, C(O)O, OC(O), or (CR₂₁R₂₂)n₃, wherein R₂₁ and R₂₂ are each independently hydrogen or methyl, or R₂₁ and R₂₂, together with the C atom to which they are attached, form a C₃₋₆ cycloalkyl or 3- to 6-membered heterocycloalkyl, and n₃ is 0, 1, 2, or 3; L₄ is a bond, CH₂, CH₂CH₂, CH₂CH₂CH₂, CO, NH, CONH, NHCO, O, C(O)O, OC(O), or (CR₂₁R₂₂)n₃, wherein R₂₁ and R₂₂ are each independently hydrogen or methyl, or R₂₁ and R₂₂, together with the C atom to which they are attached, form a C₃₋₆ cycloalkyl or 3- to 6-membered heterocycloalkyl, and n₃ is 0, 1, 2, or 3; Z₁ is CH or N, Z₂ is CH₂ or C(O); W is CH₂, O, S, alkenylene, or C≡C;
R₀ is H, D, halogen, hydroxy, cyano, C₁₋₃ alkyl, or C₁₋₃ alkoxy.

Further, in the above formula (III-1-1) or (III-1-2),
R₄ is chlorine or bromine (preferably chlorine); Rₖ₂ is hydrogen or methoxy; Rₖ₃ is selected from fluorine, chlorine, bromine, acetyl, methyl, ethyl, isopropyl, propyl, cyclopropyl, -O-oxetanyl, trifluoromethyl, - CH₂CH₂F, -CHFCH₃, -CH₂CHF₂, -CF₂CH₃, -CHFCH₂F, -CH₂CF₃, -CF₂CH₂F, and -CHFCHF₂;
L₁, L₂, L₃, and L₄ are each independently a bond, CH₂, or CH₂CH₂ (preferably, L₁, L₂, L₃, and L₄ are each independently CH₂, more preferably, L₁ and L₂ are each a bond or CH₂, and L₃ and L₄ are CH₂); Z₁ is CH or N, Z₂ is CH₂ or C(O); W is CH₂, O, S, or C≡C (preferably CH₂ or C≡C, more preferably C≡C); R₀ is H, D, fluorine, chlorine, bromine, hydroxy, cyano, methyl, ethyl, propyl, or isopropyl, R₀ is preferably hydrogen, more preferably F, even more preferably C₁₋₃ alkyl or C₁₋₃ haloalkyl, and still more preferably trifluoromethyl. In some embodiments, the compound of formula (I), or the pharmaceutically acceptable salt, the enantiomer, the diastereomer, the racemate, the solvate, the hydrate, the polymorph, the prodrug or the isotopic variant thereof, and the mixture thereof are further represented by formula (III-2-1): 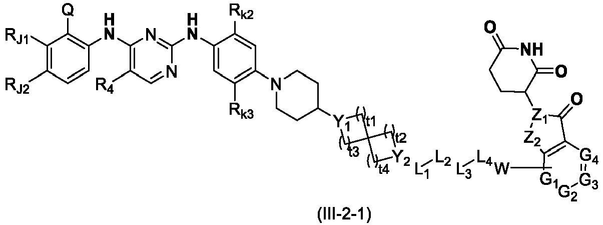 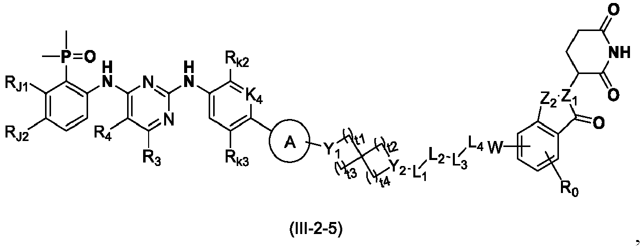 wherein
   Q is P(O)(C₁₋₃ alkyl)₂ or N(C₁₋₃ alkyl)methylsulfonyl;
Y₁ is CH or N, Y₂ is CH or N, and t1, t2, t3, and t4 are each independently 1 or 2;
ring A is a 5- to 7-membered heterocyclylene or 3- to 8-membered cycloalkylene; R₀ is selected from hydrogen, deuterium, cyano, F, C₁₋₆ alkyl, and C₁₋₆ alkoxy;
R_{J1}, R_{J2}, Rₖ₂, Rₖ₃, R₄, L₁, L₂, L₃, L₄, W, G₁, G₂, G₃, G₄, Z₁, and Z₂ are defined in any one of the above items. In some embodiments, the compound of formula (I), or the pharmaceutically acceptable salt, the enantiomer, the diastereomer, the racemate, the solvate, the hydrate, the polymorph, the prodrug or the isotopic variant thereof, and the mixture thereof are further represented by formula (III-2-2) or (III-2-3): 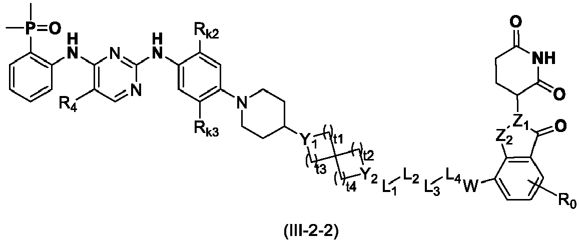 or 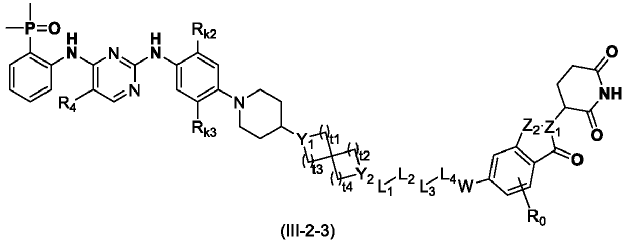
wherein R₄ is chlorine or bromine;
Y₁ is N, Y₂ is CH, and t1, t2, t3, and t4 are each independently 1 or 2;
Rₖ₂ is hydrogen, methoxy, ethoxy, or O-oxetanyl;
Rₖ₃ is selected from fluorine, chlorine, bromine, cyano, acetyl, methyl, ethyl, isopropyl, propyl, cyclopropyl, -O-oxetanyl, trifluoromethyl, -CH₂CH₂F, -CHFCH₃, -CH₂CHF₂, -CF₂CH₃, -CHFCH₂F, -CH₂CF₃, -CF₂CH₂F, or -CHFCHF₂;
L₁, L₂, L₃, and L₄ are each independently a bond, CH₂, or CH₂CH₂ (preferably, L₁, L₂, L₃, and L₄ are each independently CH₂, more preferably, L₁ and L₂ are each a bond or CH₂, and L₃ and L₄ are CH₂, even more preferably, L₁, L₂, L₃, and L₄ are each independently a bond);
W is CH₂, O, or S (preferably O);
Z₁ is CH or N, and Z₂ is CH₂ or C(O);
R₀ is selected from hydrogen, fluorine, chlorine, or methyl.

In some embodiments, the compound of formula (I), or the pharmaceutically acceptable salt, the enantiomer, the diastereomer, the racemate, the solvate, the hydrate, the polymorph, the prodrug or the isotopic variant thereof, and the mixture thereof are further represented by formula (Ib-1) or (Ib-2): 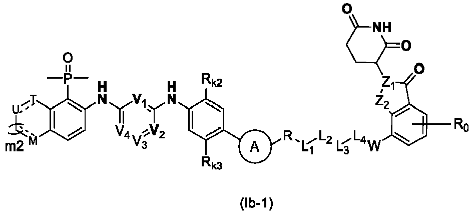 or 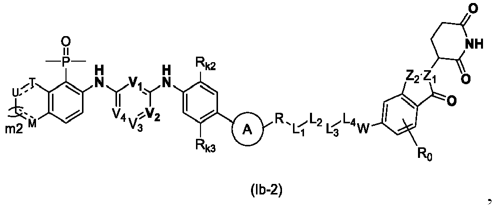 wherein
U, T, M, V₁, V₂, V₃, V₄, Rₖ₂, Rₖ₃, A, R, L₁, L₂, L₃, L₄, W, Z₁, Z₂, R₀, and m2 are defined in any one of the above items.

In some embodiments, the compound of formula (I), or the pharmaceutically acceptable salt, the enantiomer, the diastereomer, the racemate, the solvate, the hydrate, the polymorph, the prodrug or the isotopic variant thereof, and the mixture thereof are further represented by formula (IV) or (IV-1): 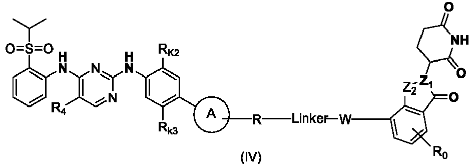 or 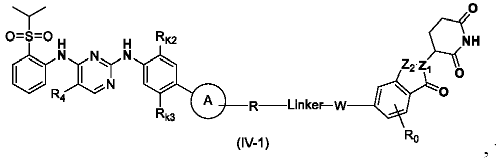 wherein
R₄, Rₖ₂, Rₖ₃, A, R, Linker, W, Z₁, Z₂, and R₀ are defined in any one of the above items.

In some embodiments, the compound of formula (I), or the pharmaceutically acceptable salt, the enantiomer, the diastereomer, the racemate, the solvate, the hydrate, the polymorph, the prodrug or the isotopic variant thereof, and the mixture thereof are further represented by formula (VIII): 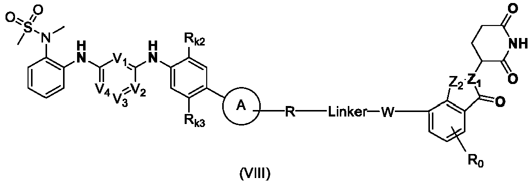 wherein V₁, V₂, V₃, V₄, Rₖ₂, Rₖ₃, A, R, W, Z₁, and Z₂ are defined in any one of the above items.

In some embodiments, T is O, S, or C, preferably, T is O or N. In some embodiments, M is O, S, or C, preferably, M is O or N. In some embodiments, U is C or N.

In some embodiments, m2 is 0 or 1; R⁰ is selected from hydrogen, fluorine, chlorine, bromine, iodine, cyano, acetyl, C₁₋₅ alkyl, C₁₋₅ alkoxy, C₁₋₅ haloalkyl, C₃₋₆ cycloalkyl, or 3- to 6-membered heterocycloalkyl; preferably, R⁰ is selected from fluorine, chlorine, and bromine; preferably, R⁰ is selected from methyl, ethyl, propyl, isopropyl, methoxy, or ethoxy.

In some embodiments, the compound of formula (V), or the pharmaceutically acceptable salt, the enantiomer, the diastereomer, the racemate, the solvate, the hydrate, the polymorph, the prodrug or the isotopic variant thereof, and the mixture thereof are further represented by (V-1) or (V-2): 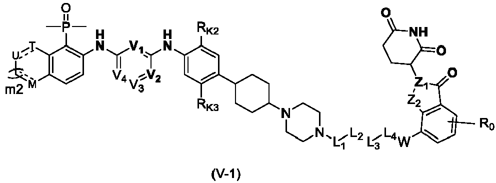 or 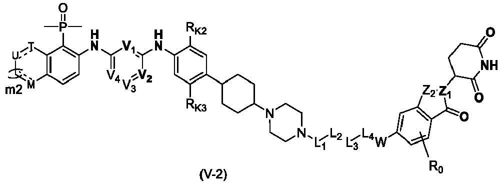
wherein V₁ and V₂ are both N, V₃ is CR₃, V₄ is CR₄, R₃ is hydrogen, and R₄ is chlorine or bromine; Rₖ₂ is hydrogen, C₁₋₃ alkyl, C₁₋₃ haloalkyl, or C₁₋₃ alkoxy (preferably methoxy), C₃₋₆ cycloalkyl, or 3- to 6-membered heterocycloalkyl; Rₖ₃ is acetyl, cyano, fluorine, chlorine, bromine, methyl, ethyl, propyl, isopropyl, cyclopropyl, O-oxetanyl, or C₁₋₃ haloalkyl; L₁, L₂, L₃, and L₄ are each independently a bond, CH₂, or CH₂CH₂; W is CH₂, O, S, or C≡C; Z₁ is CH, and Z₂ is CO or CH₂; R₀ is H, D, fluorine, chlorine, bromine, C₁₋₃ alkyl, or C₁₋₃ alkoxy;
U, T, M, and m2 are defined in any one of the above items.

In some embodiments, E further represents (II-i-1): 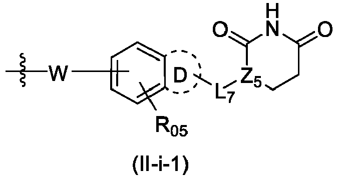
wherein L₇ is a bond or NH, and Z₅ is N or CH;
R₀₅ is hydrogen, halogen, cyano, acetyl, acylamino, C₁₋₃ alkyl, C₁₋₃ alkoxy, C₁₋₃ haloalkyl, or C₁₋₃ haloalkoxy; ring D is absent, L₇ is attached to a benzene ring, or ring D is selected from benzene, pyridine, pyrimidine, pyrazine, pyridazine, cyclohexane, oxocyclohexane, cyclopentane, tetrahydrofuran, tetrahydrothiophene, tetrahydropyrrole, furan, thiophene, pyrrole, pyrazole, imidazole, thiazole, isothiazole, oxazole, isoxazole, and triazole; and ring D is substituted, within a valence-permitted range, with 1, 2, or 3 substituents selected from hydrogen, C₁₋₃ alkyl, C₁₋₃ alkoxy, C₁₋₃ haloalkyl, C₁₋₃ haloalkoxy, C₃₋₆ cycloalkyl, C₃₋₆ heterocycloalkyl, and halogen; W is as defined in any one of the above embodiments.

In some embodiments, E is selected from: 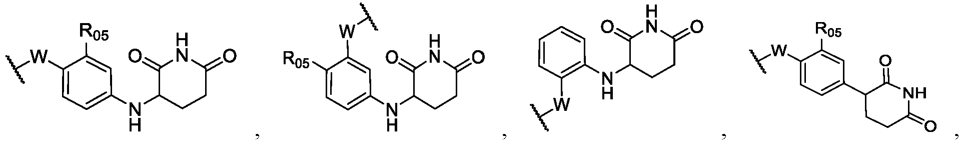 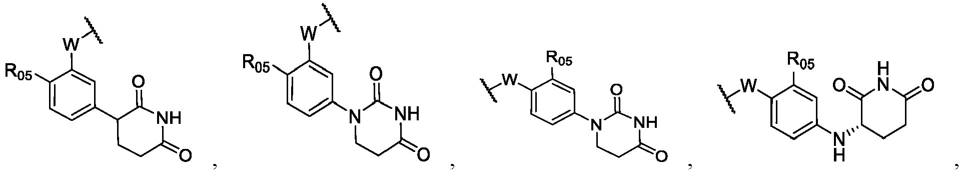 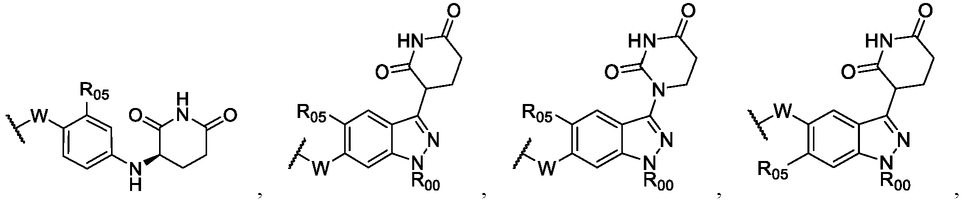 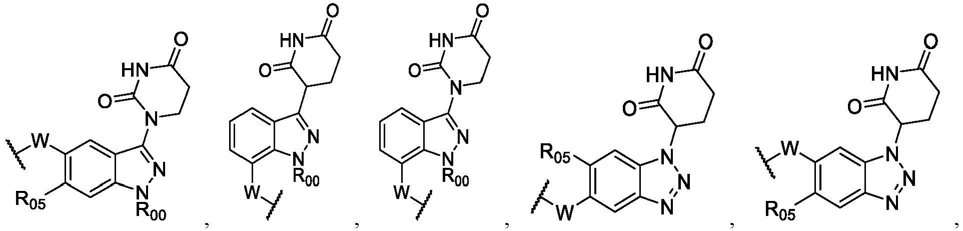 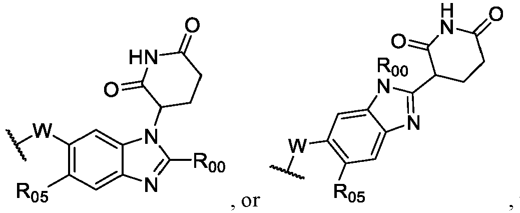 wherein
R₀₅ is hydrogen, fluorine, cyano, acetyl, acylamino, fluoromethyl, difluoromethyl, or trifluoromethyl, preferably, R₀₅ is hydrogen, deuterium, C₁₋₆ alkyl, C₁₋₆ alkoxy, cyano, or C₁₋₆ haloalkyl;
R₀₀ is hydrogen, methyl, ethyl, propyl, isopropyl, or cyclopropyl;
W is CH₂, O, S, NH, -N(methyl)-, or -C≡C.

In some embodiments, L₅ is a bond or NH; ring Cy1 is selected from phenyl, 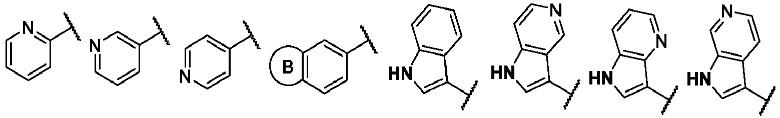 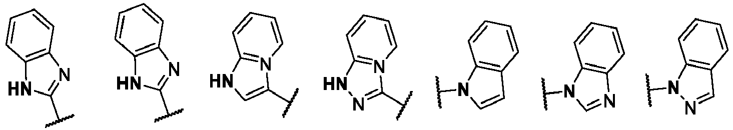 Rcy is selected from: hydrogen, deuterium, halogen, C₁₋₆ alkyl, C₁₋₆ haloalkyl, C₁₋₆ alkoxy, C₁₋₆ haloalkoxy, C₁₋₆ hydroxyalkoxy, cyano, amino, -P(O)(CH₃)₂, -N(CH₃)S(O)₂CH₃, or -S(O)₂(CH)(CH₃)₂.

In some embodiments, the compound is selected from Table 1, and the pharmaceutically acceptable salt of the compound of formula I is selected from a hydrochloride or formate salt of a compound in Table 1:

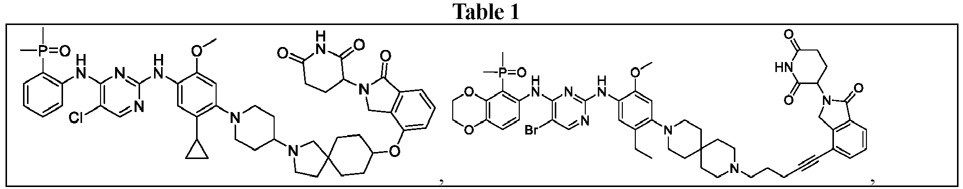

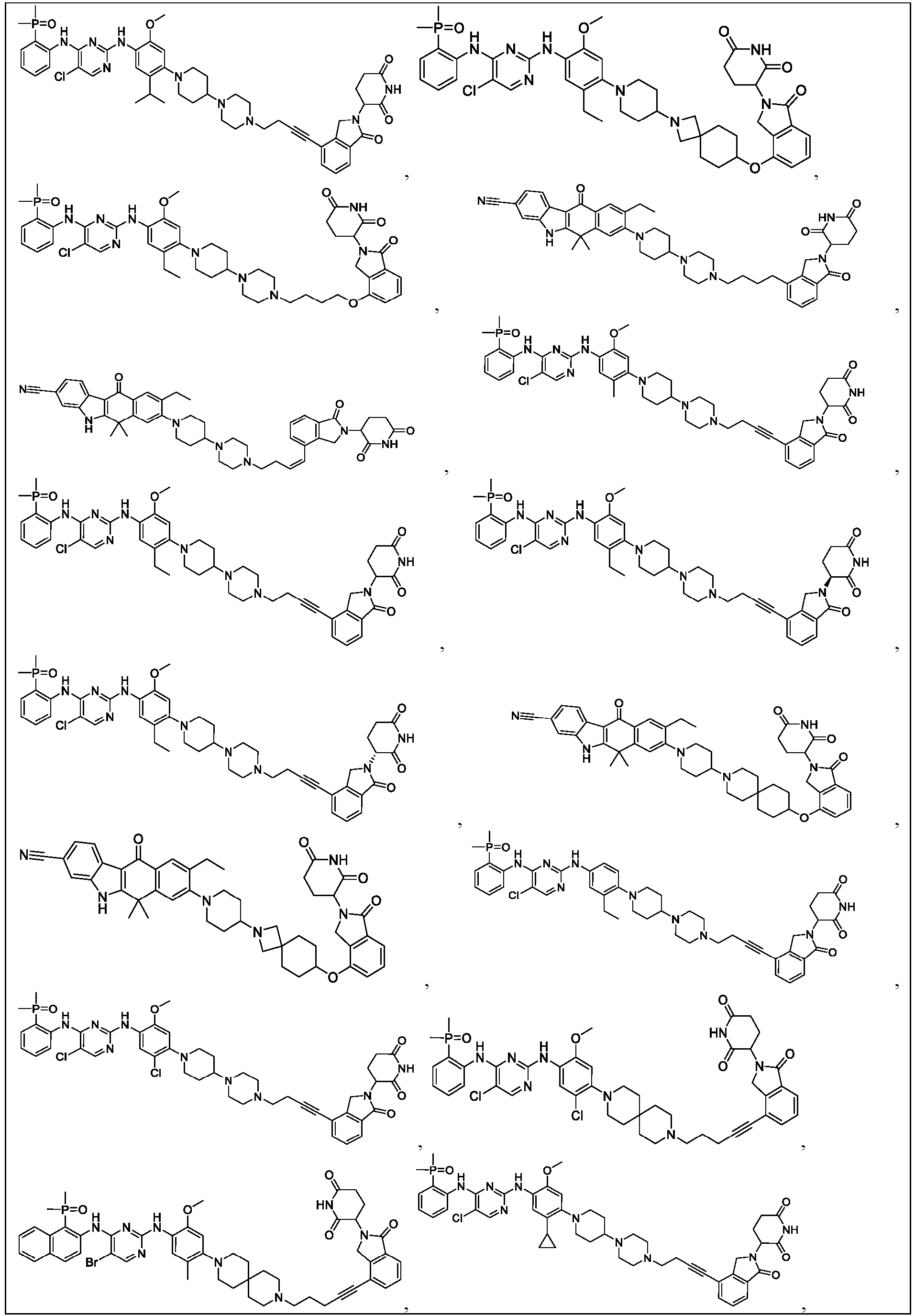

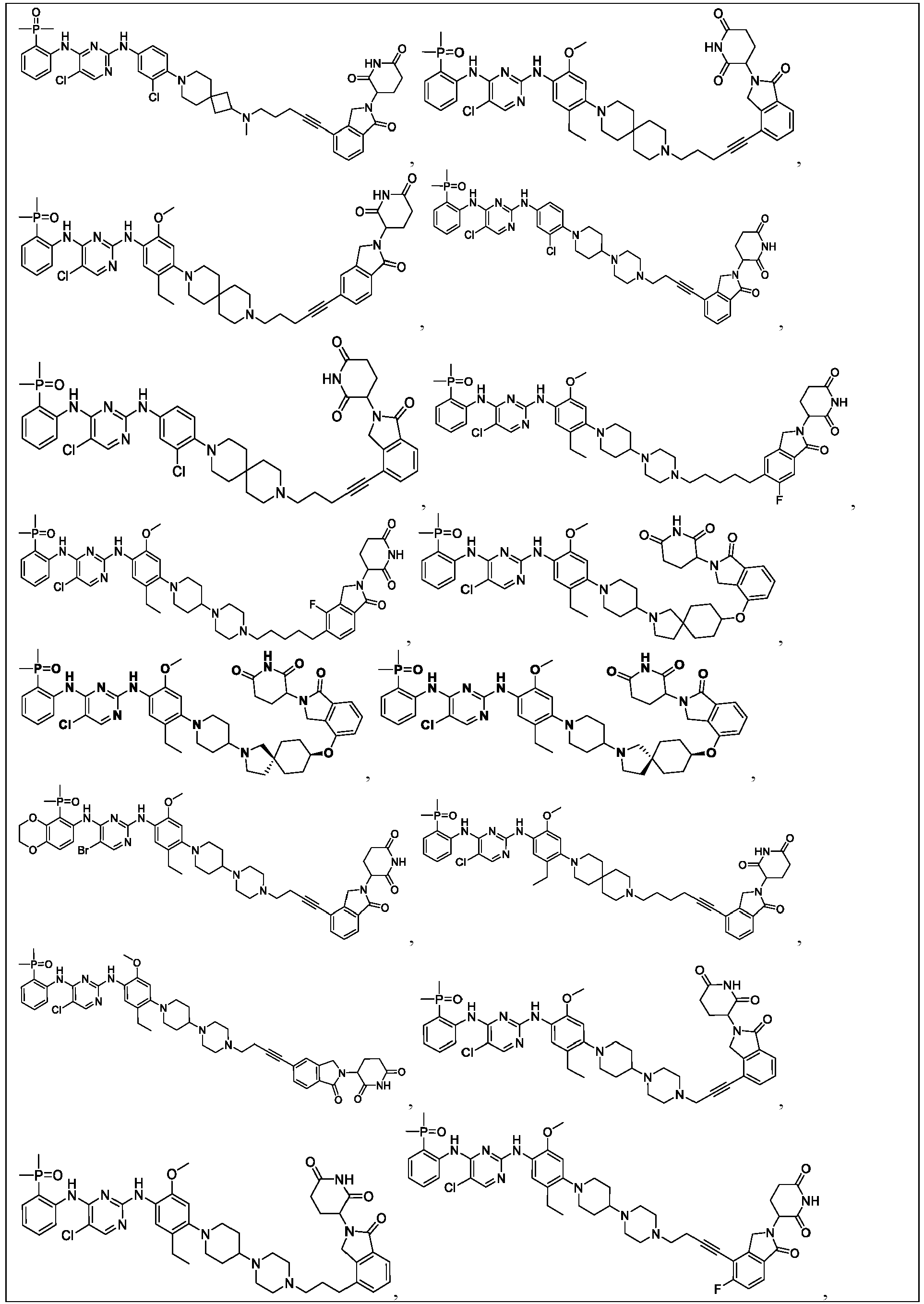

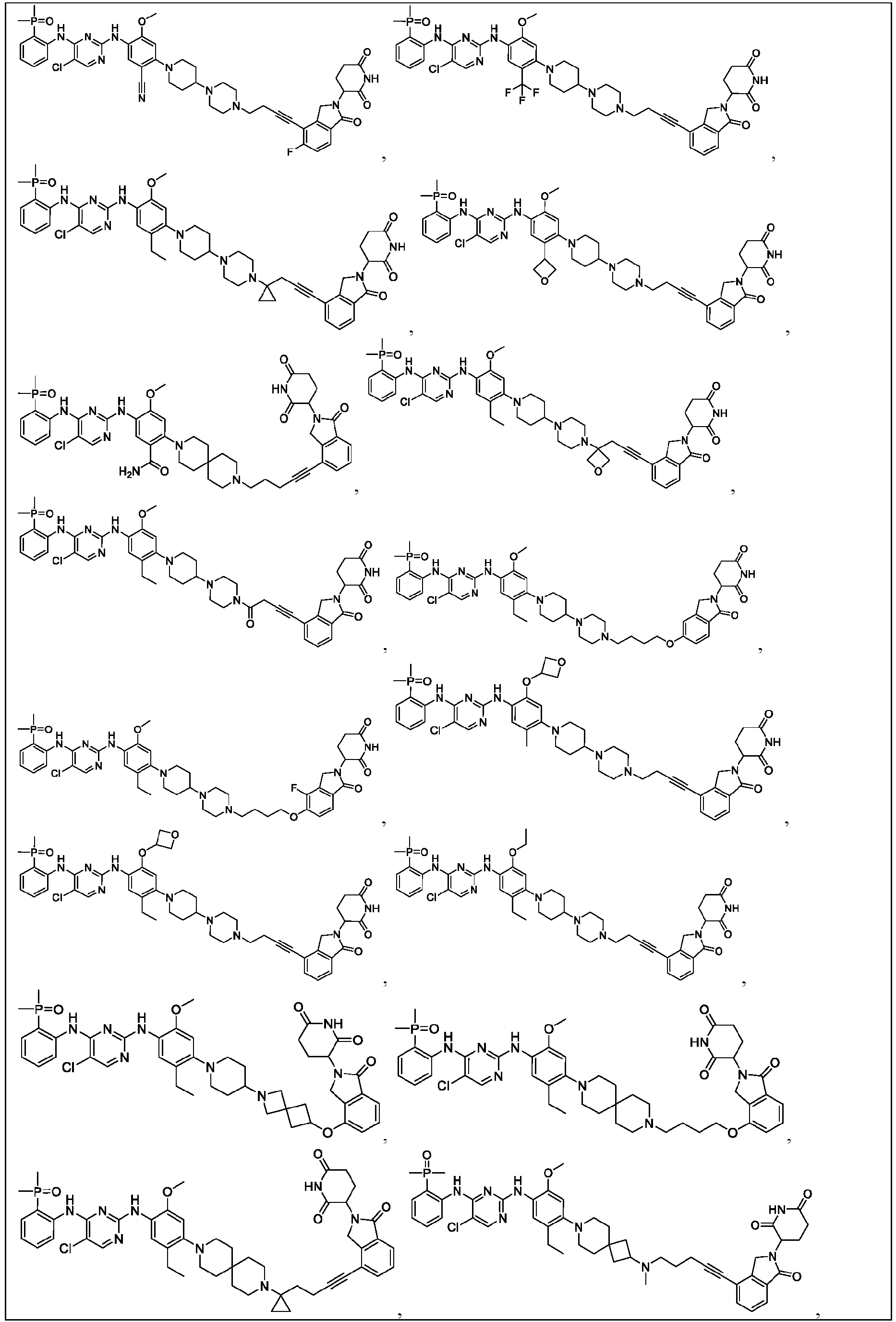

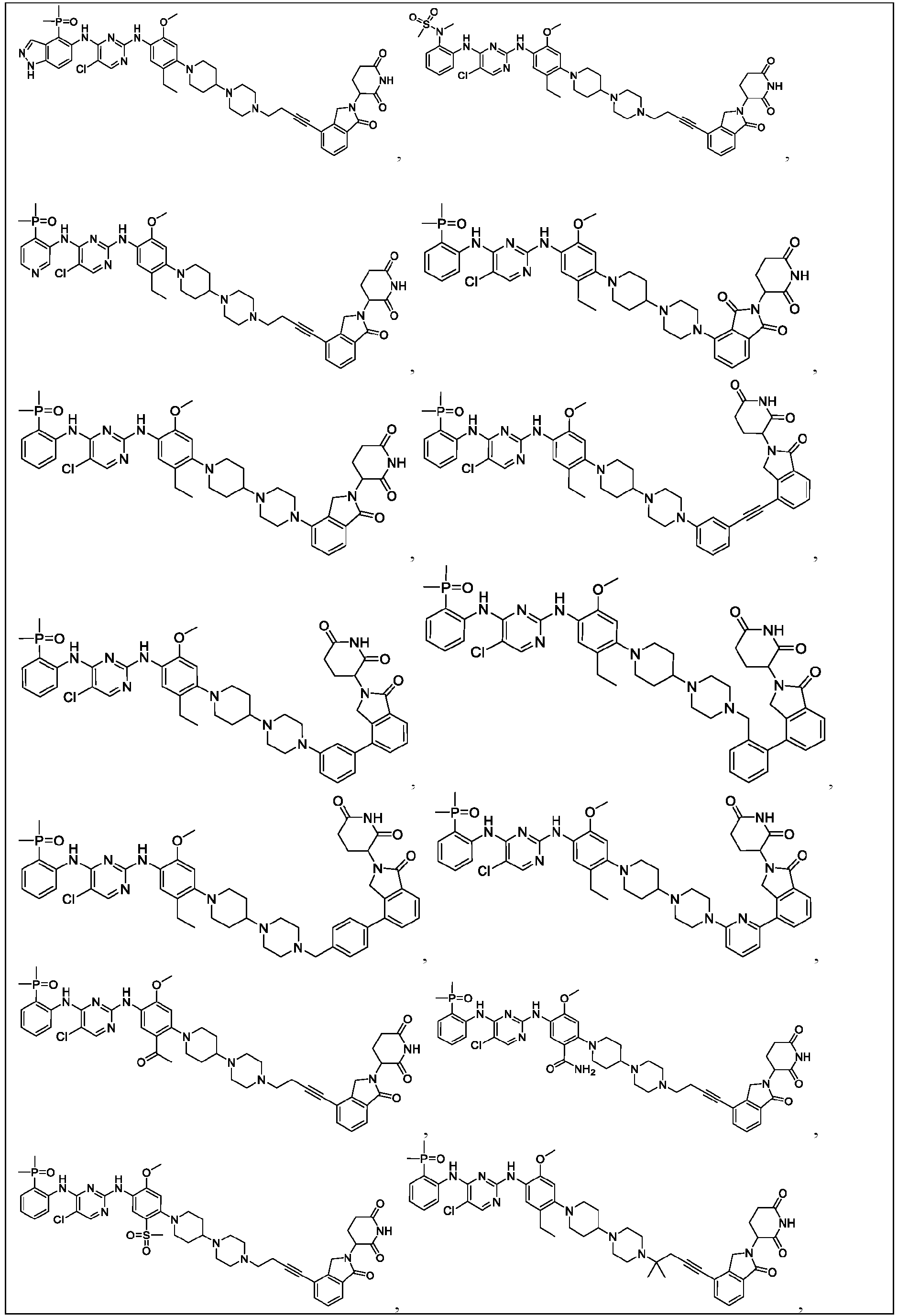

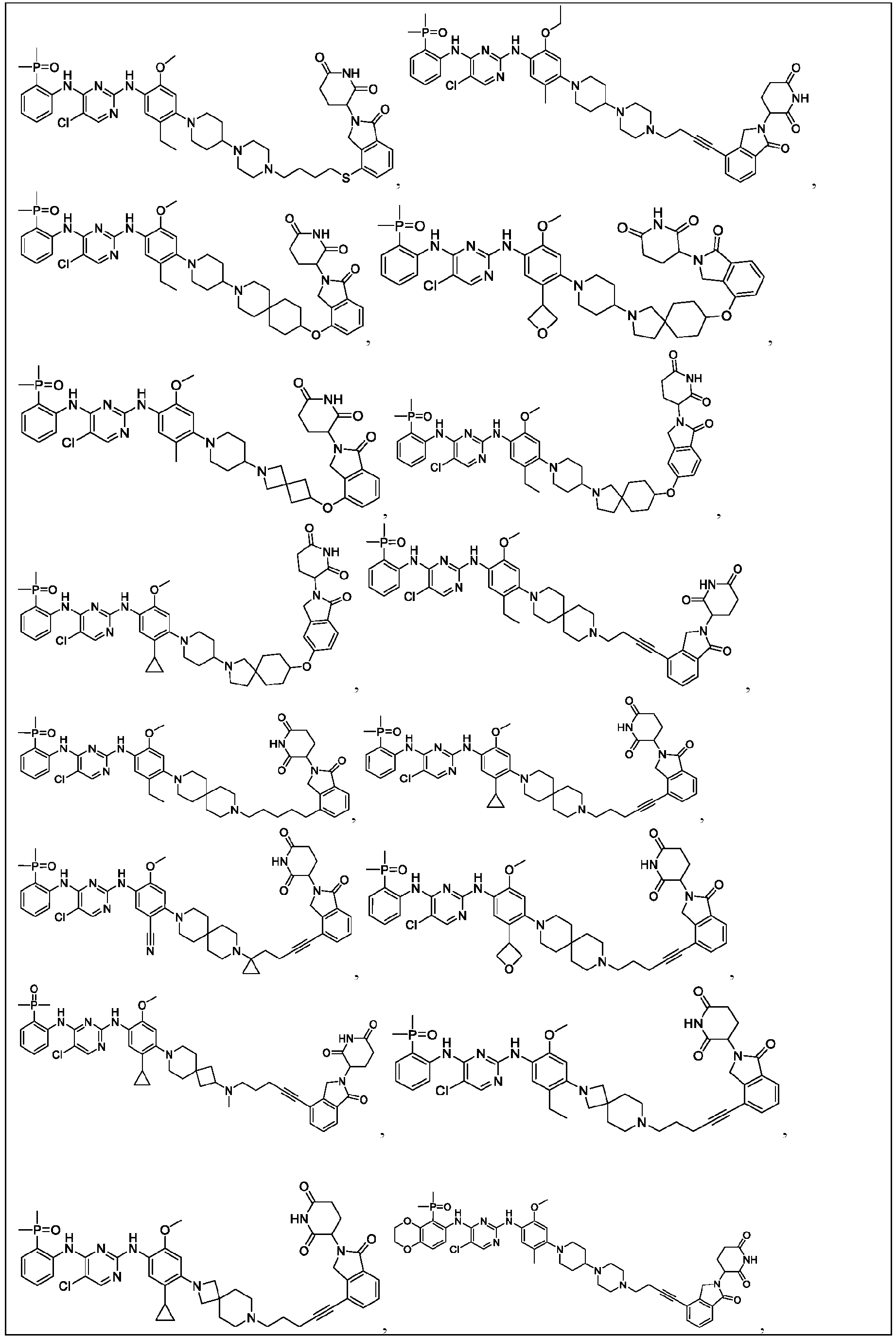

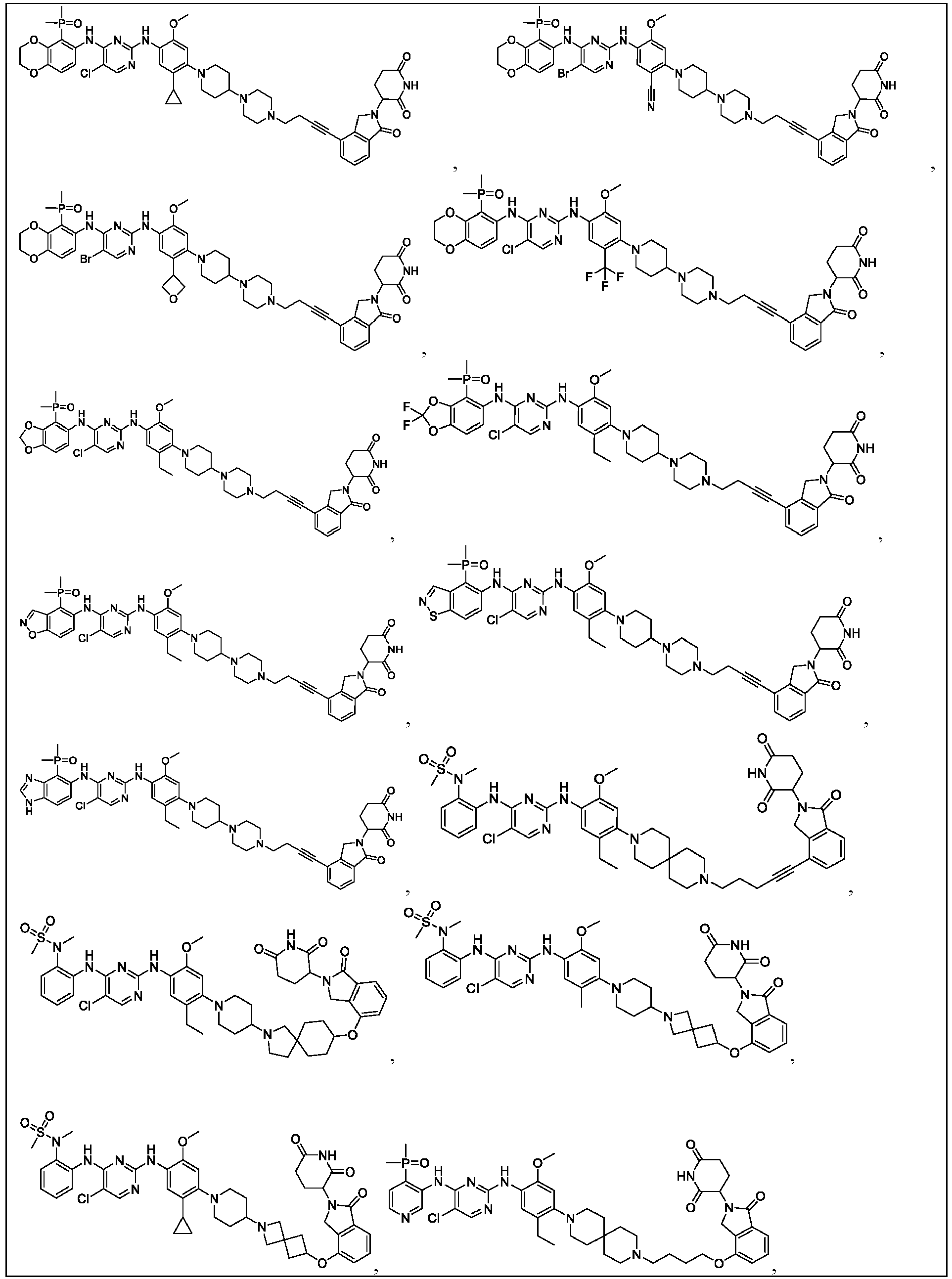

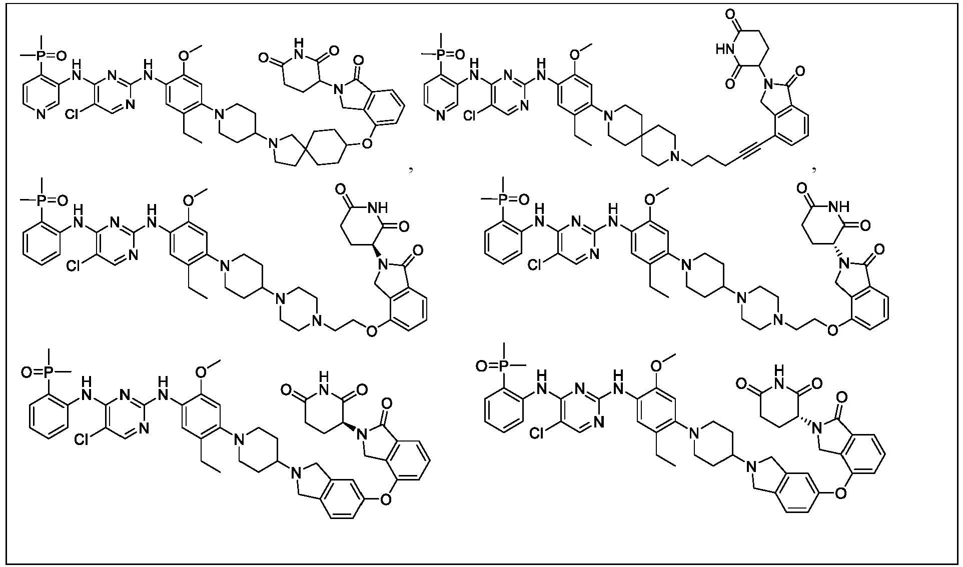

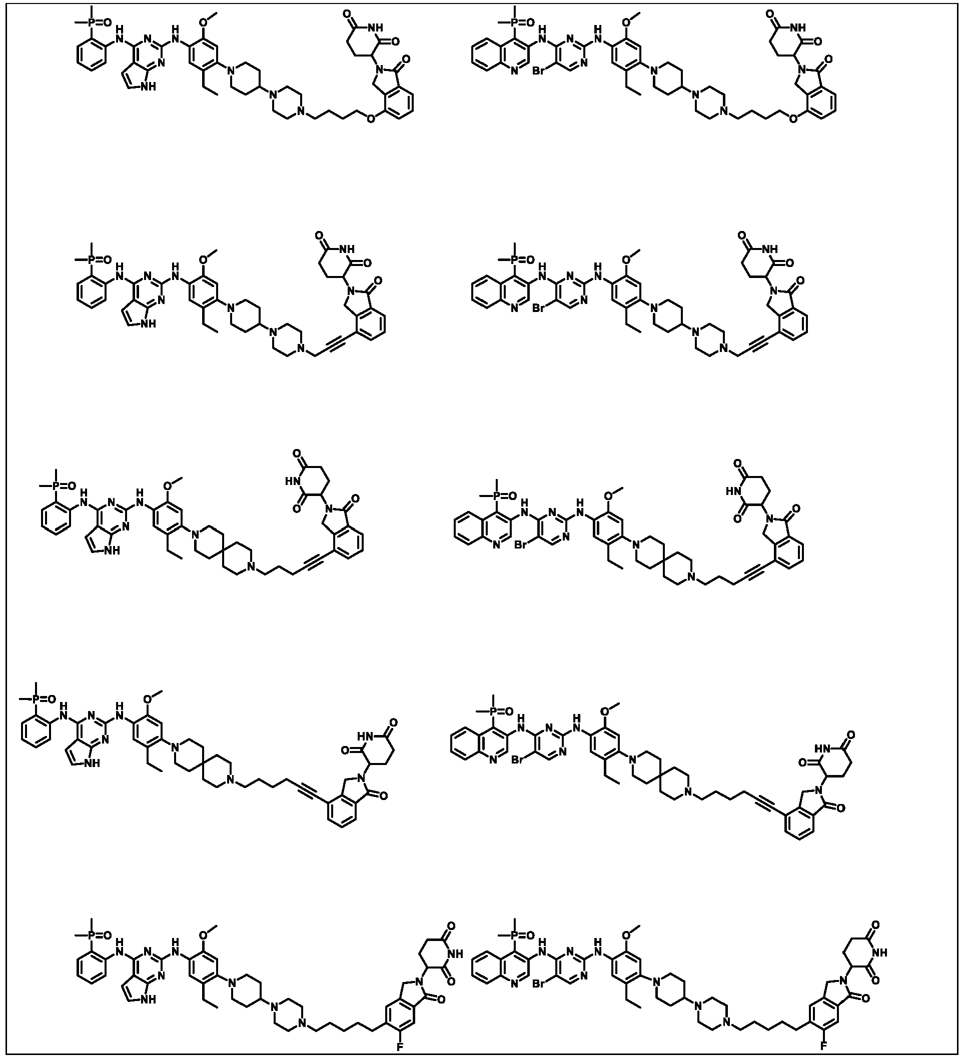

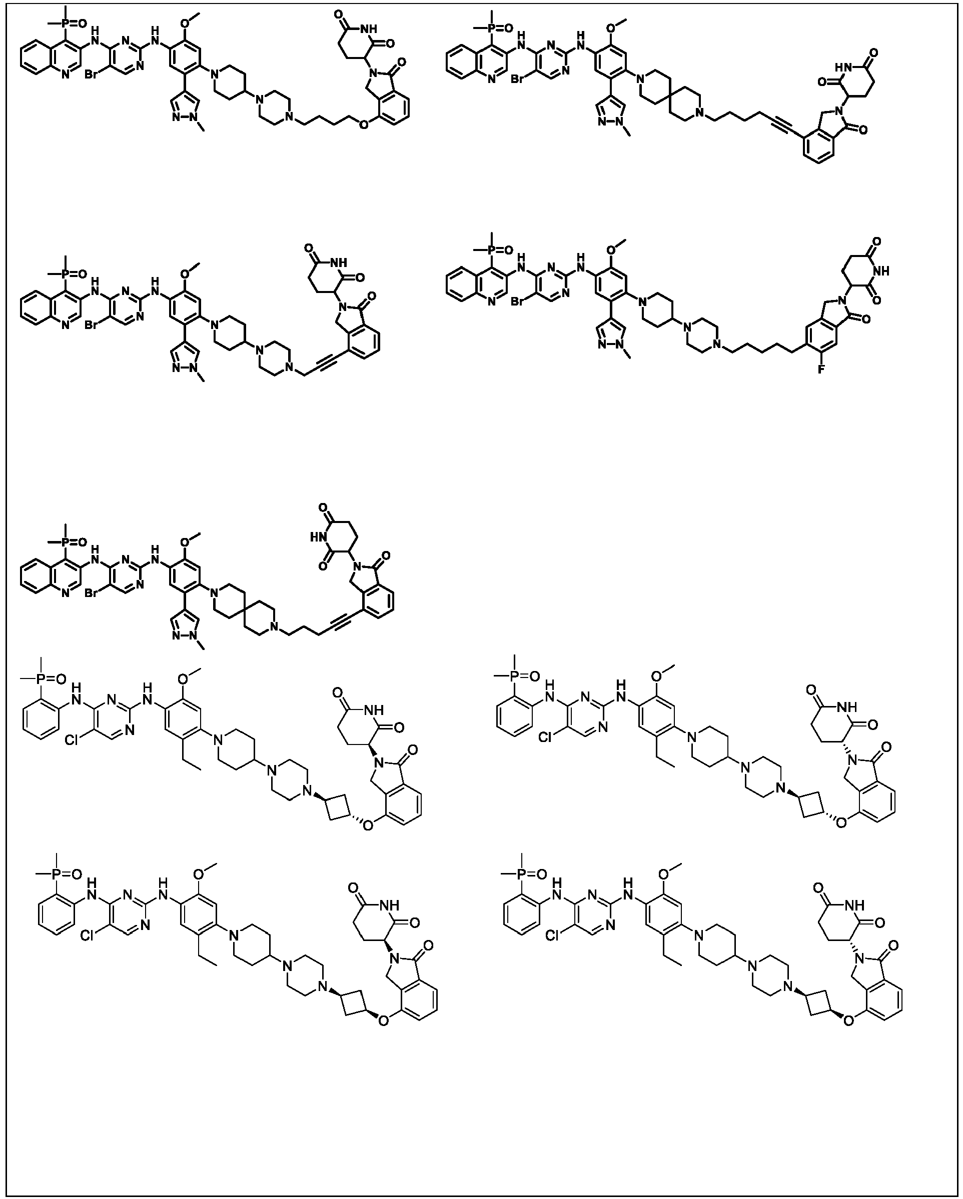

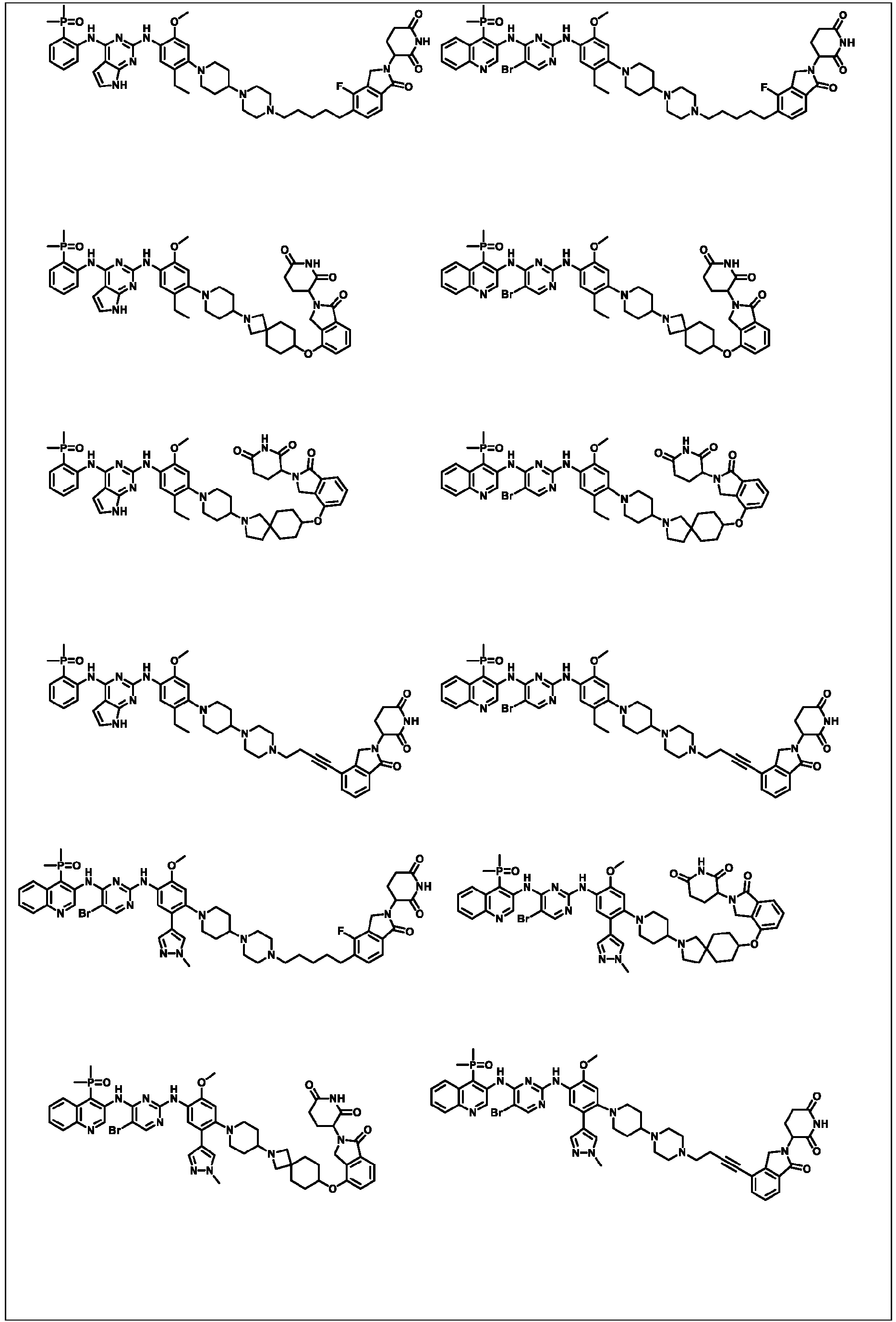

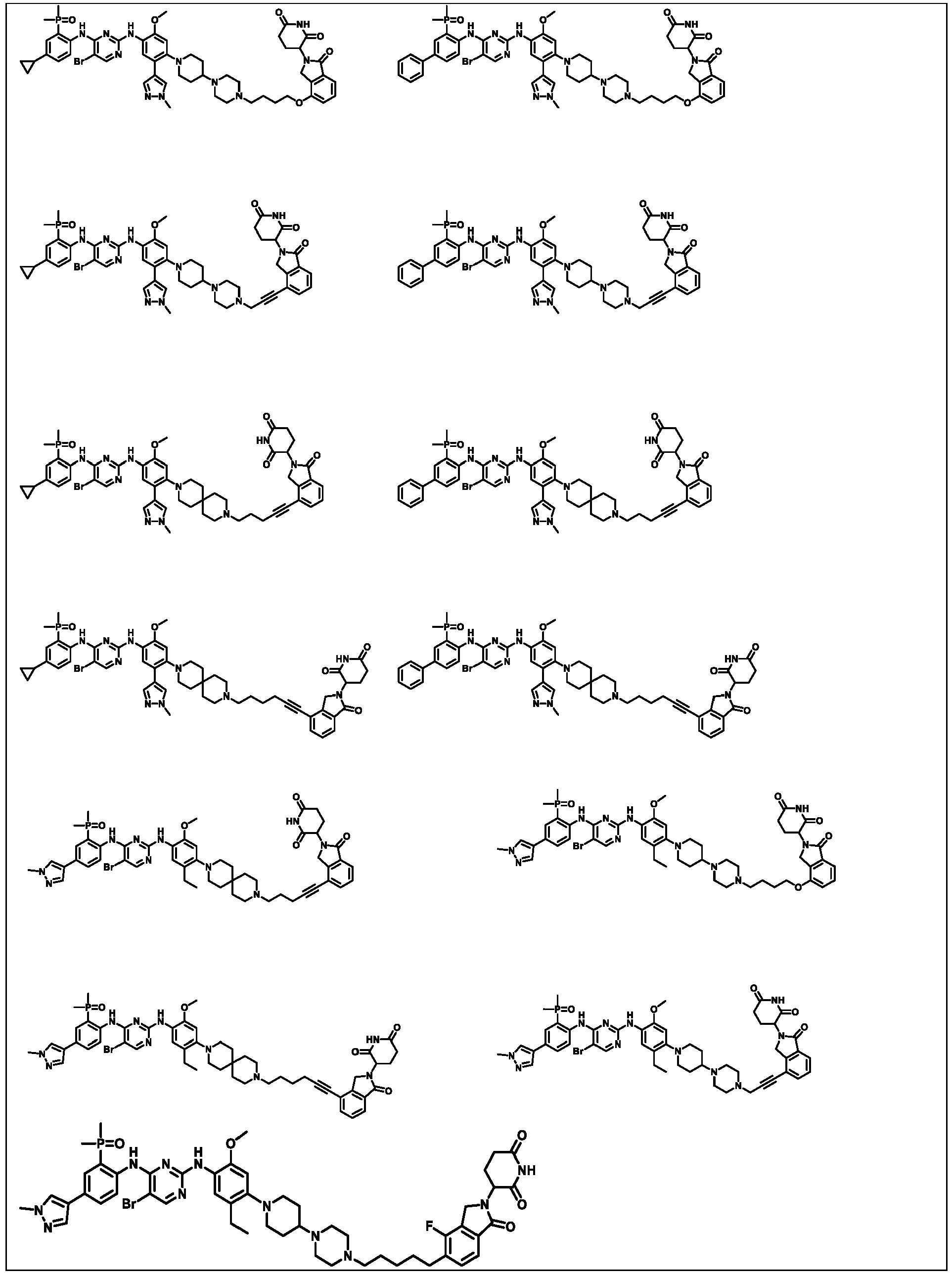

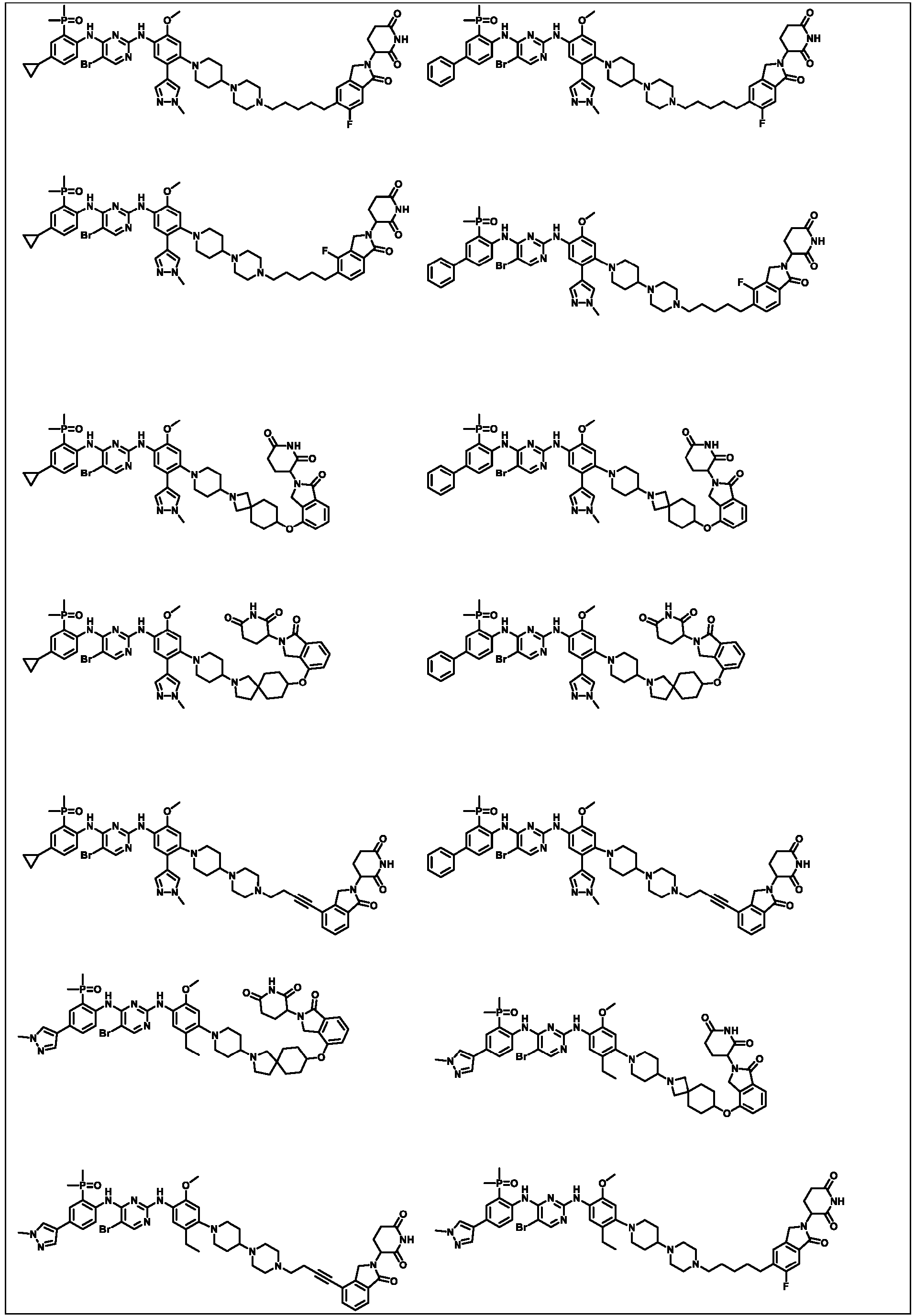

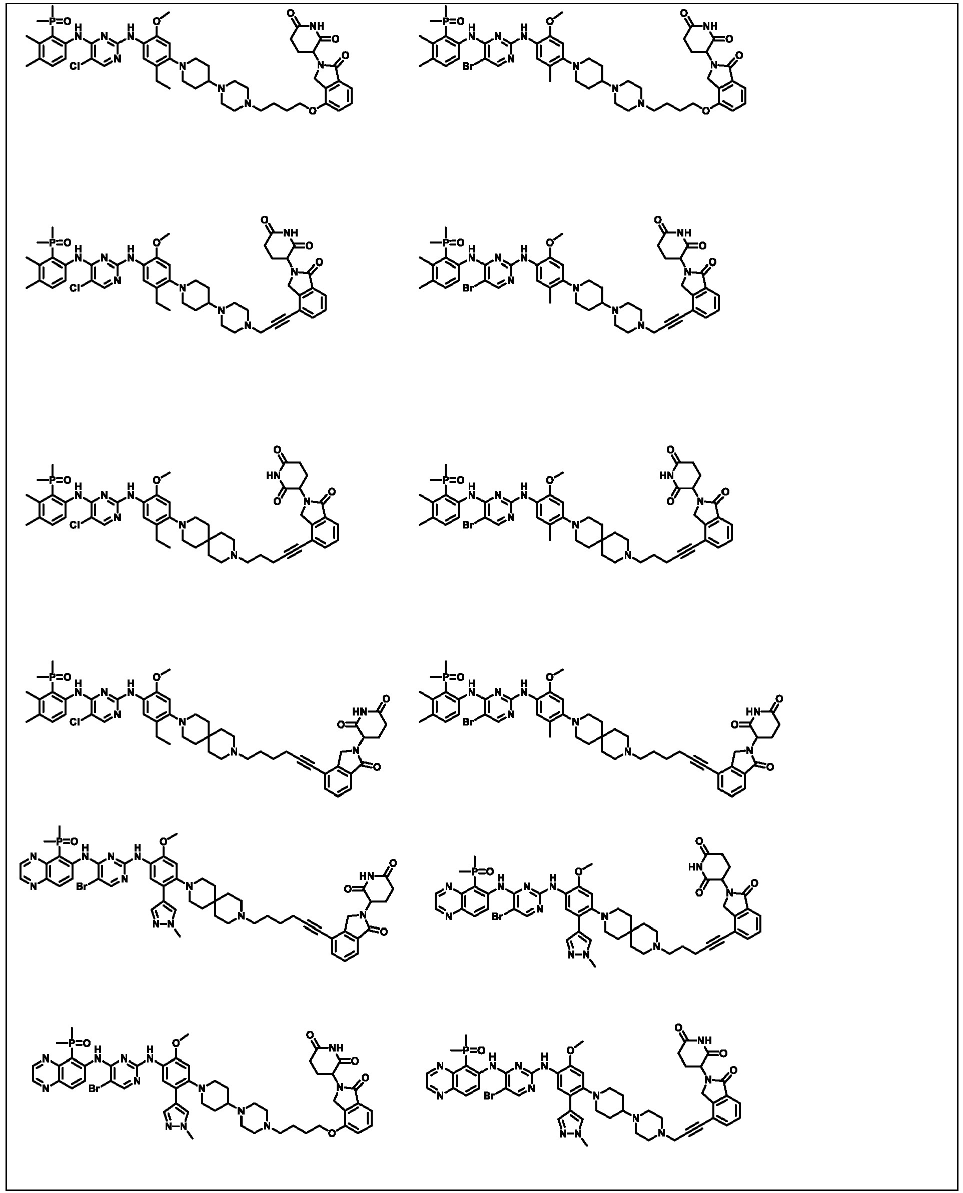

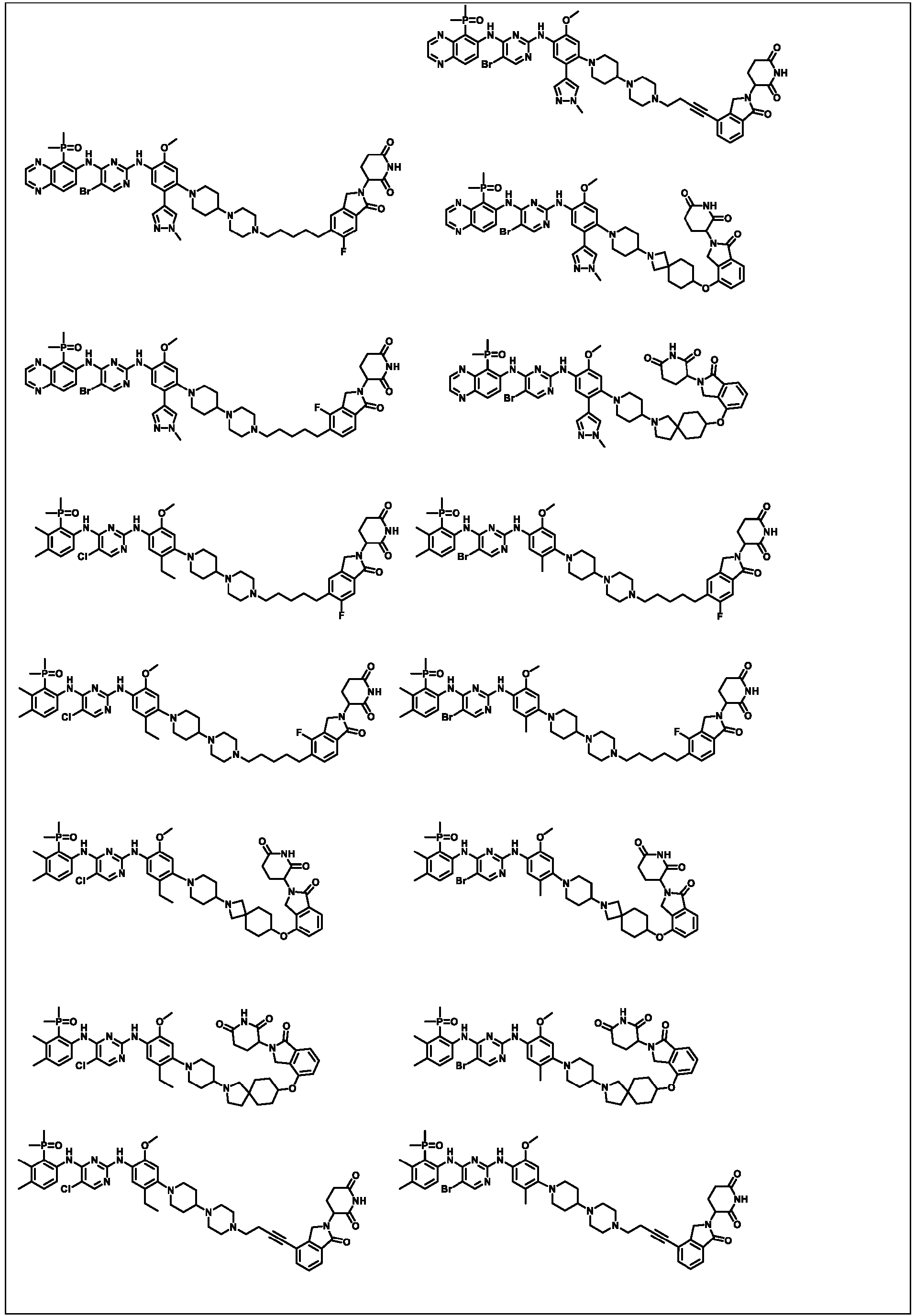

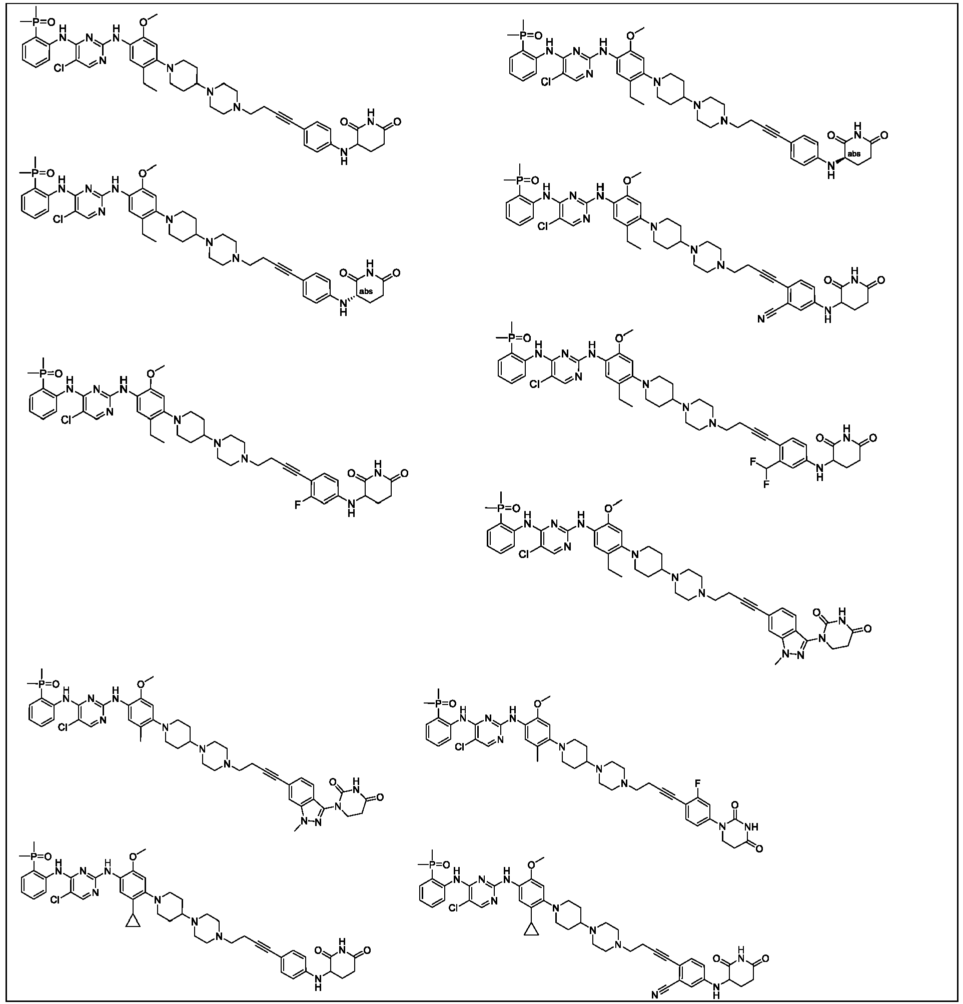

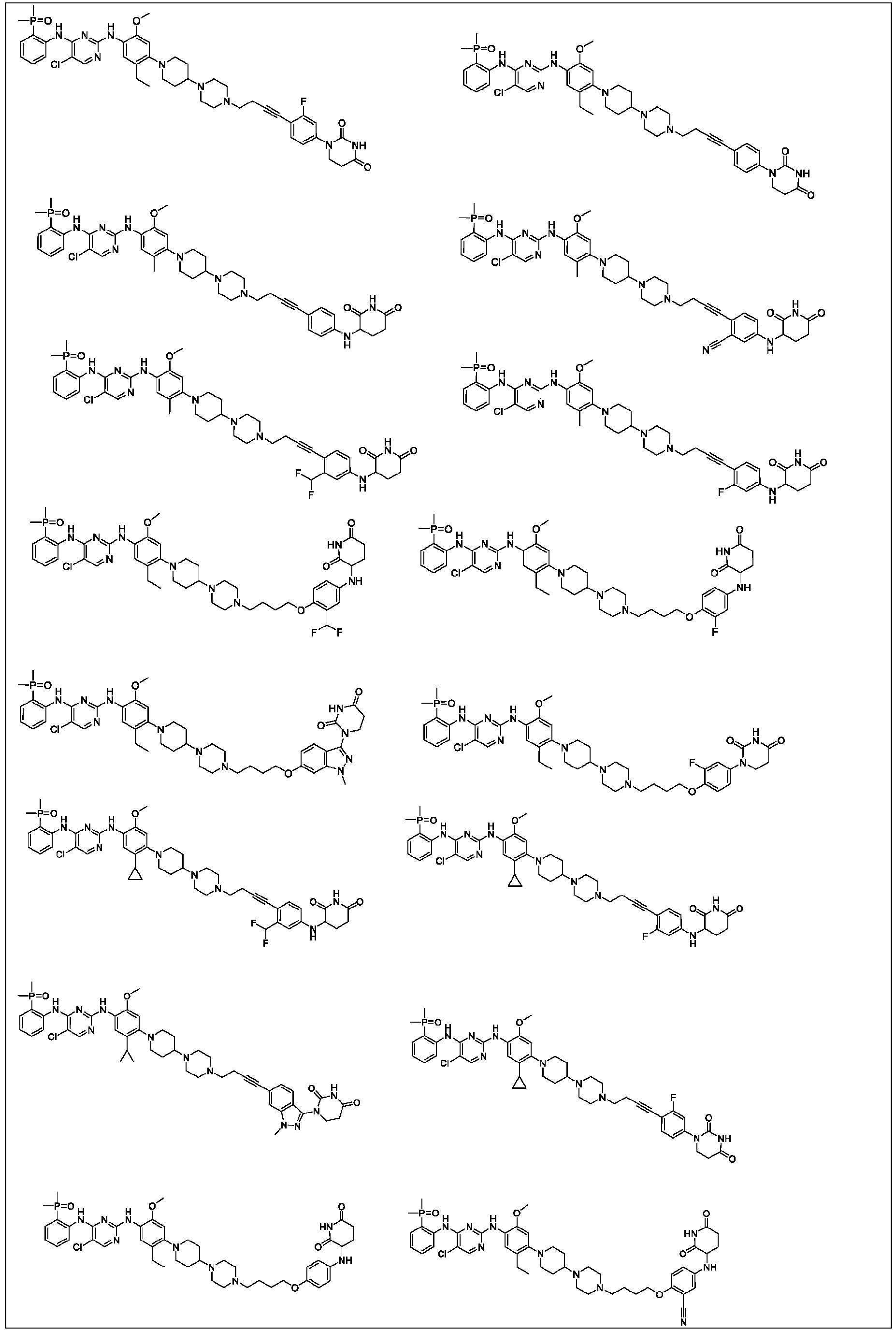

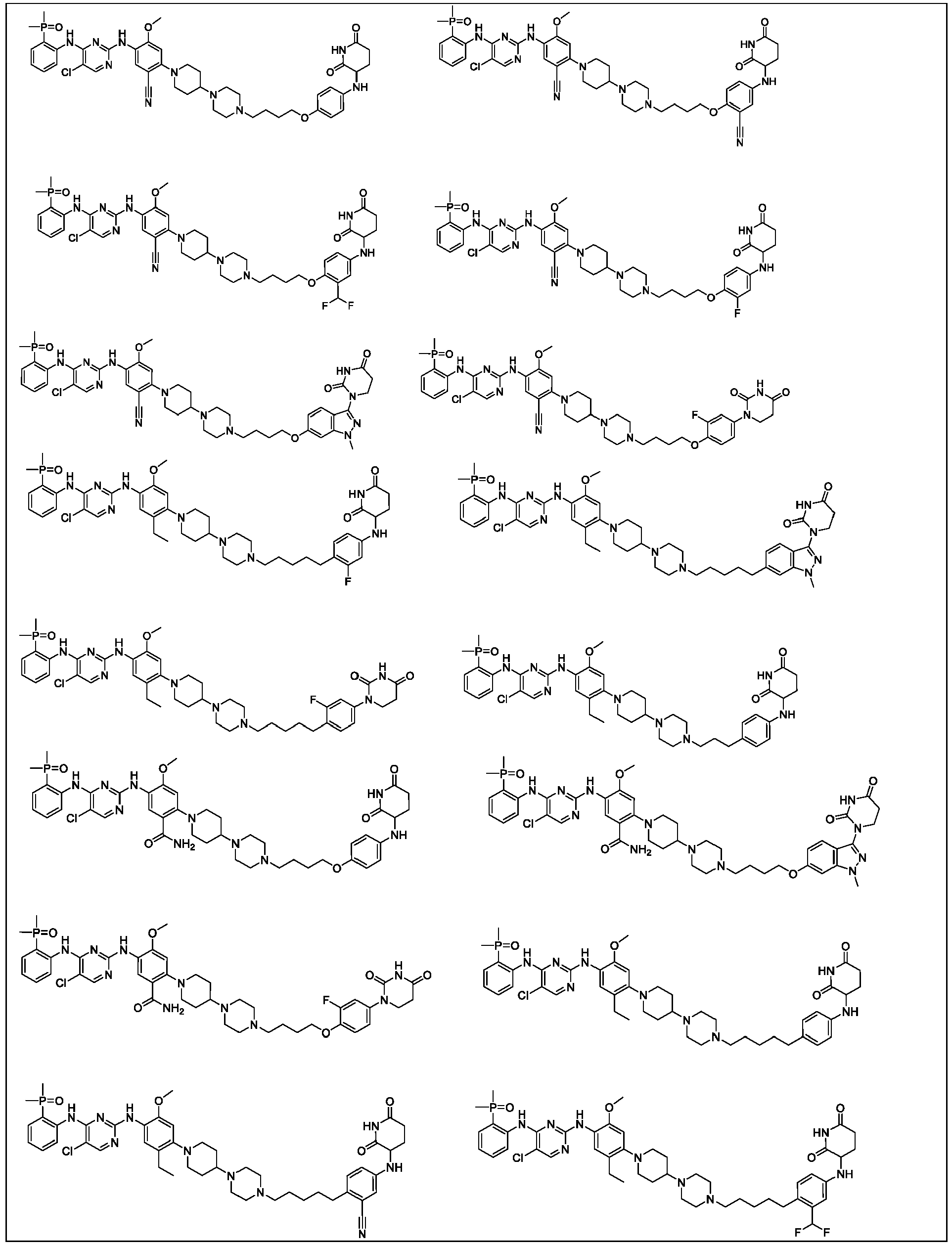

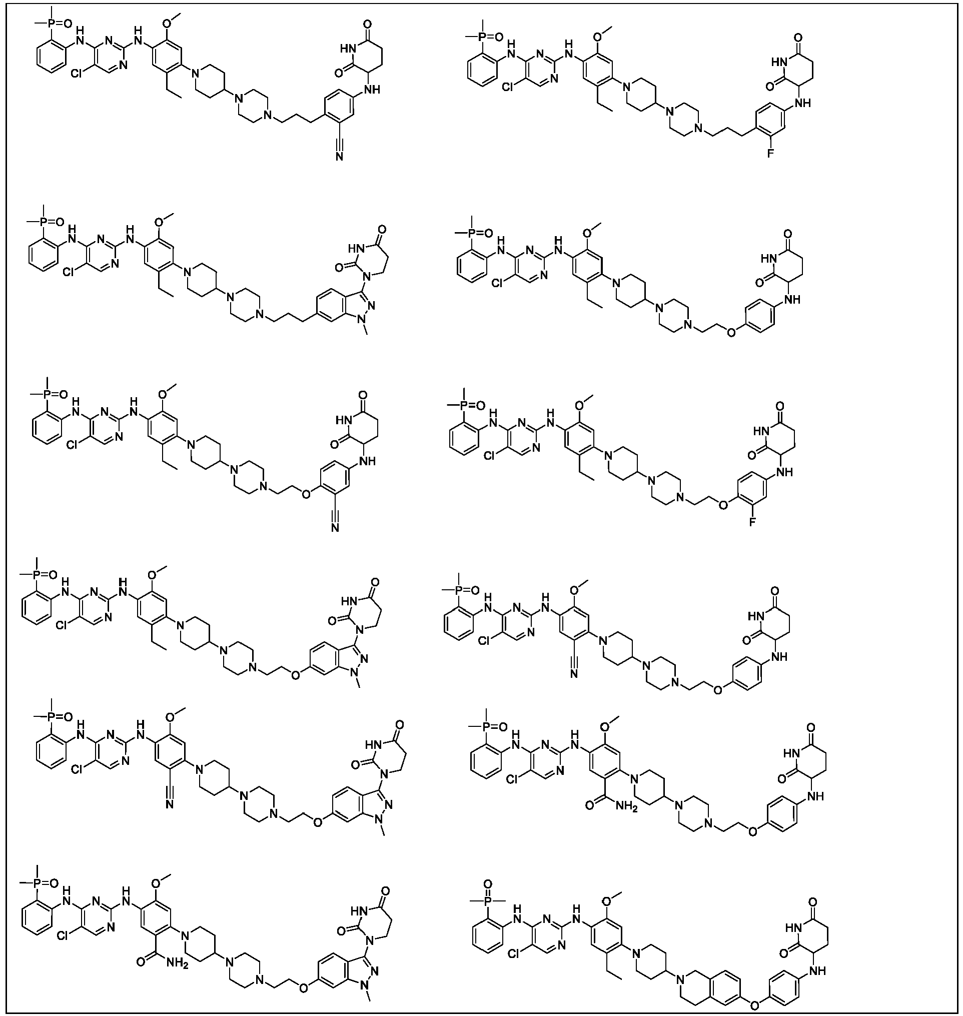

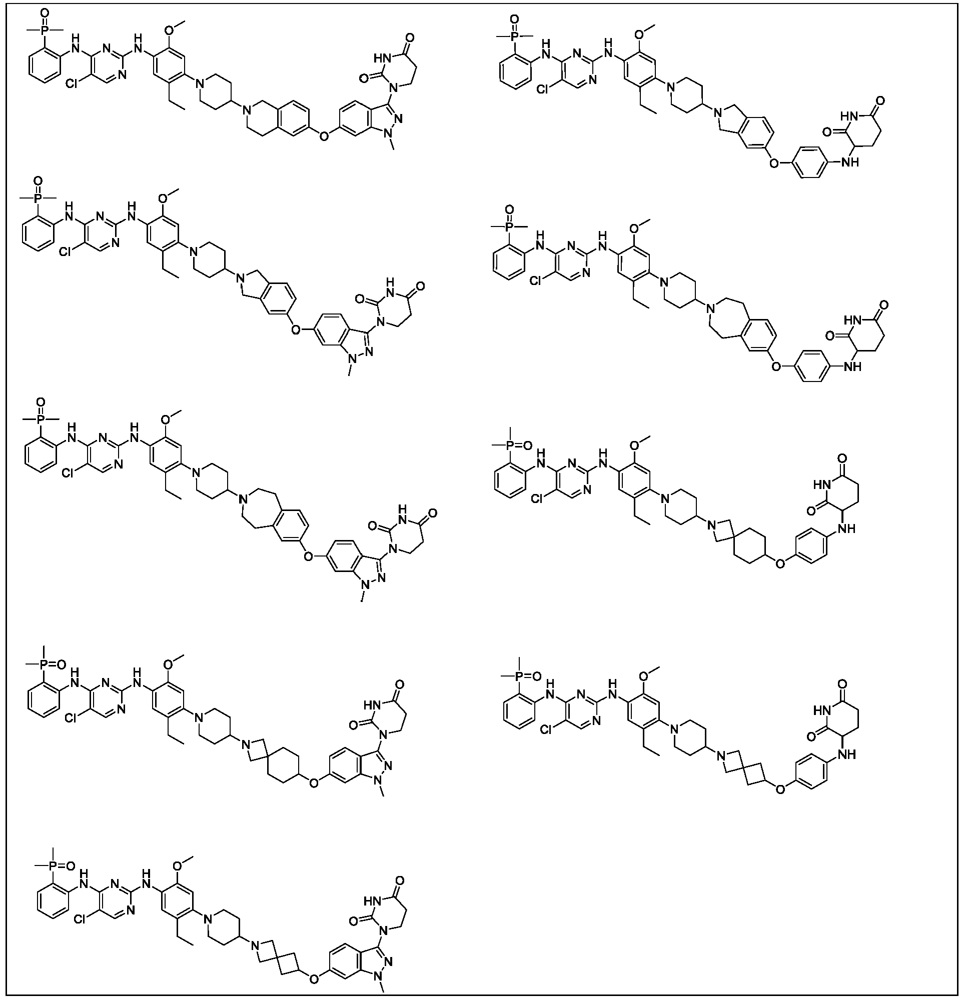

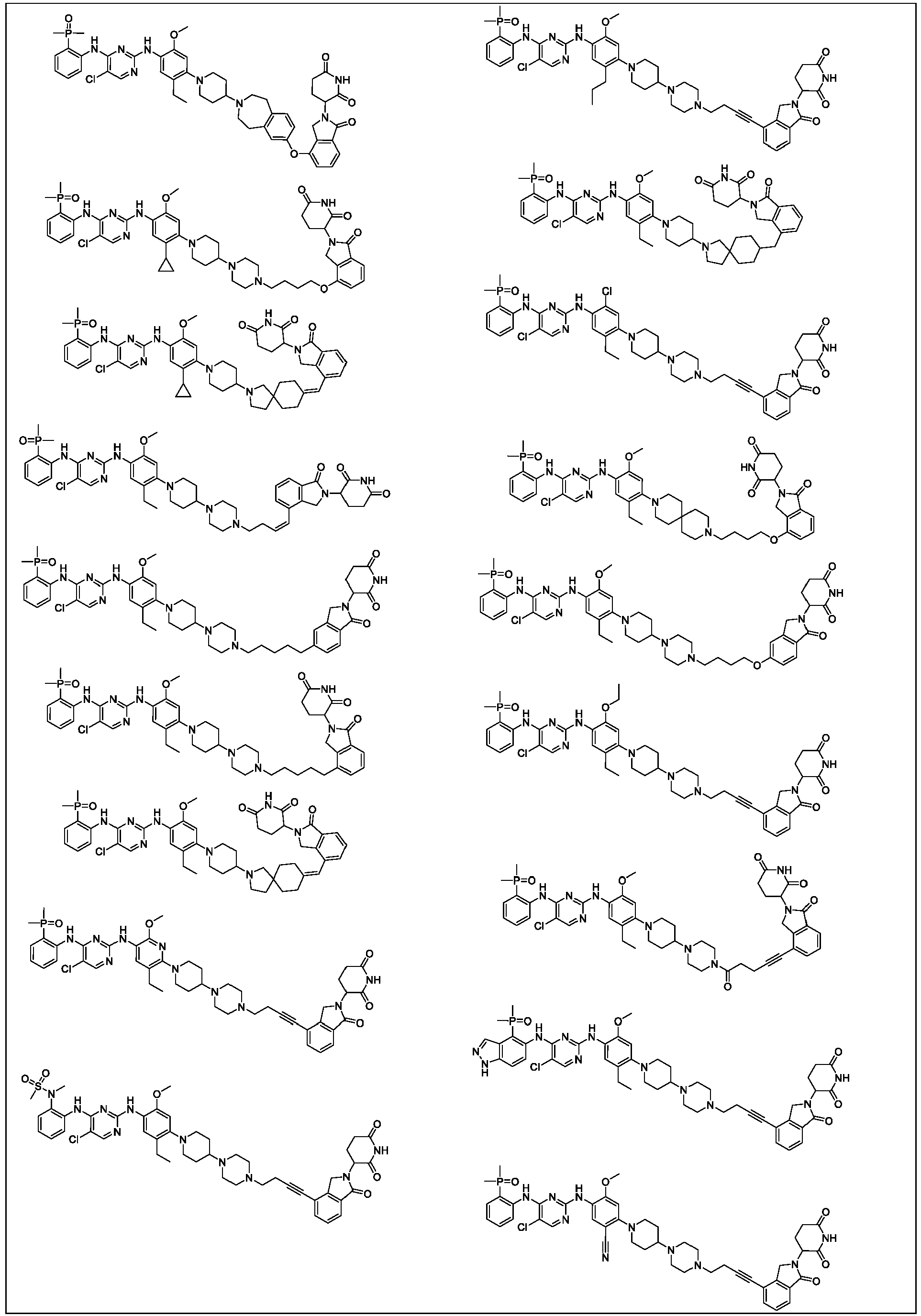

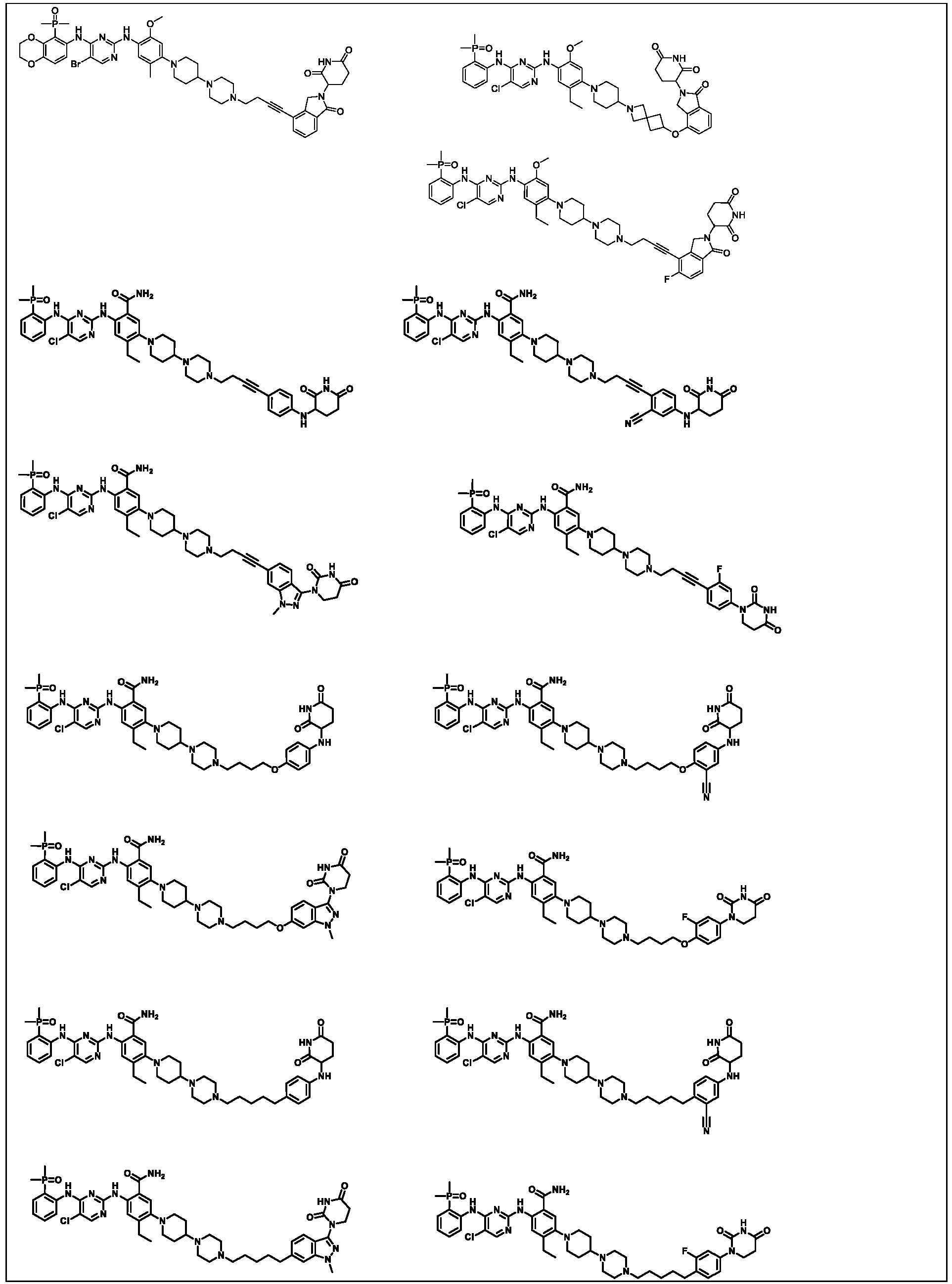

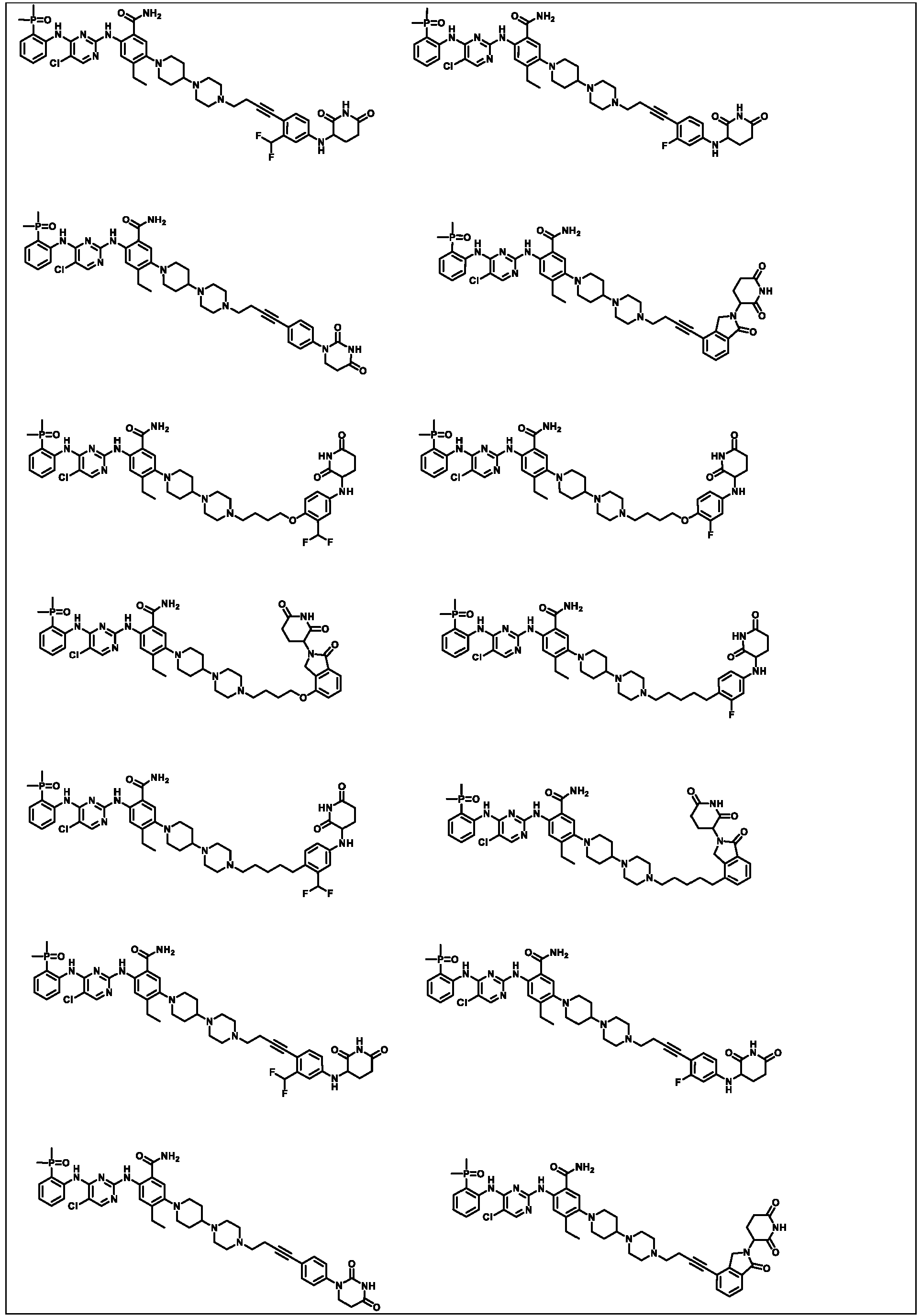

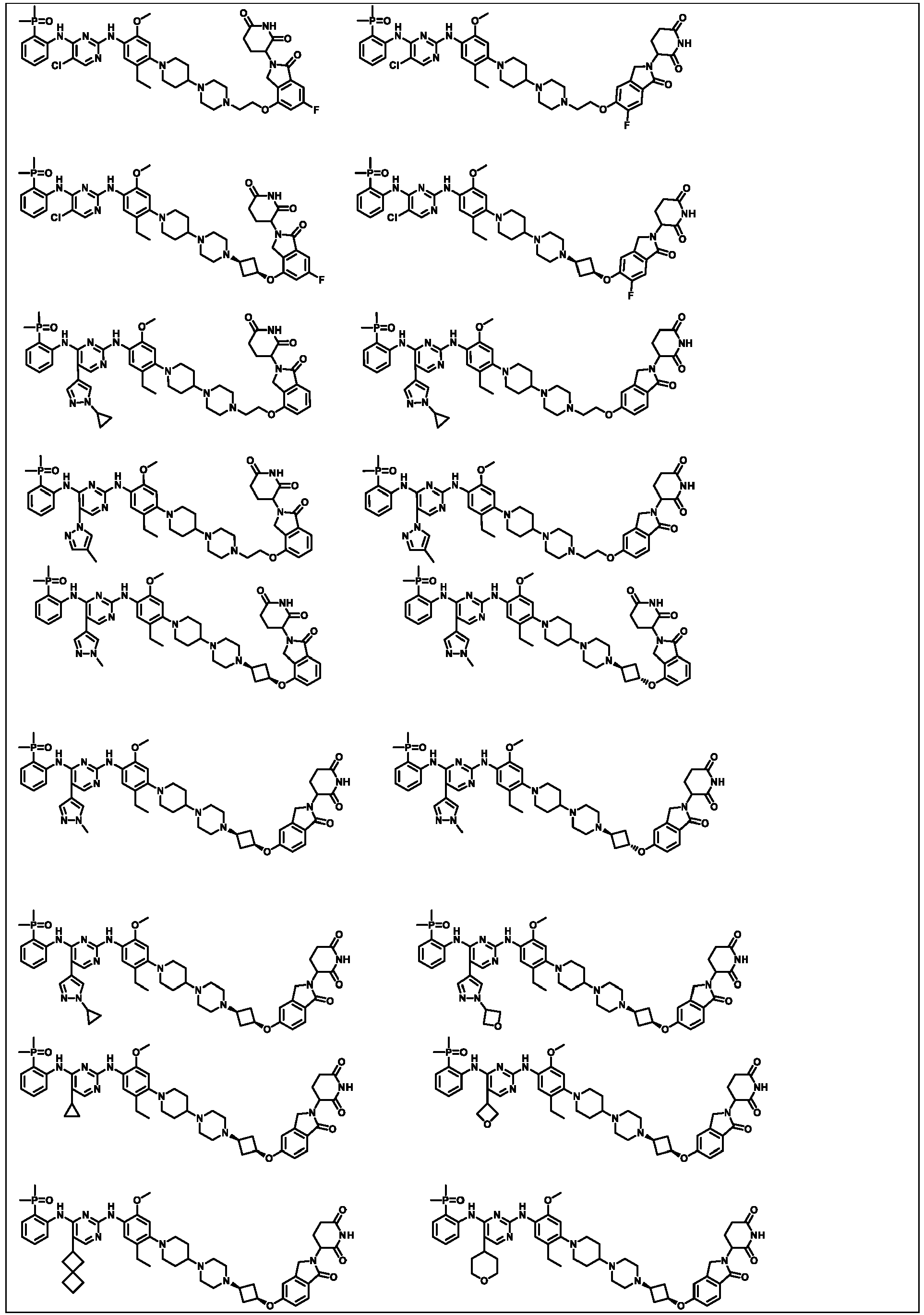

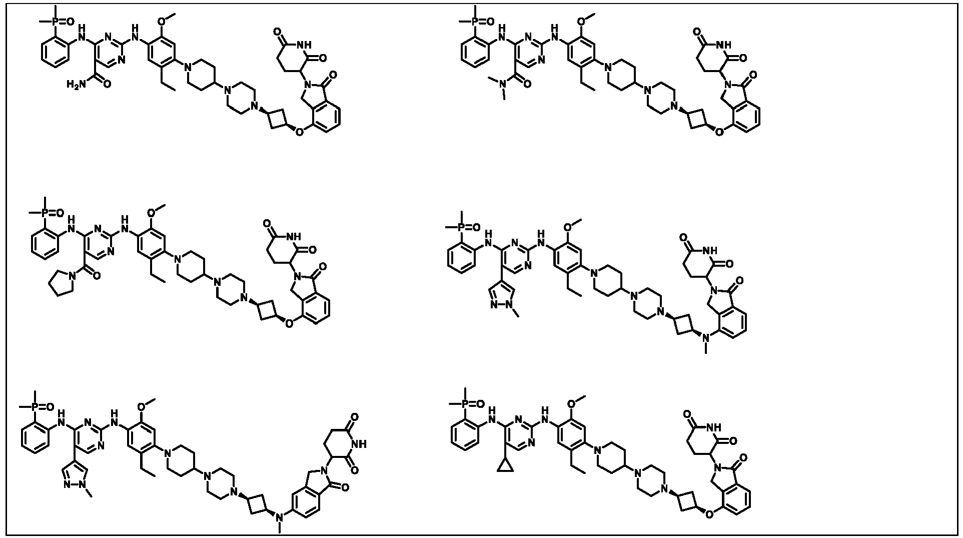

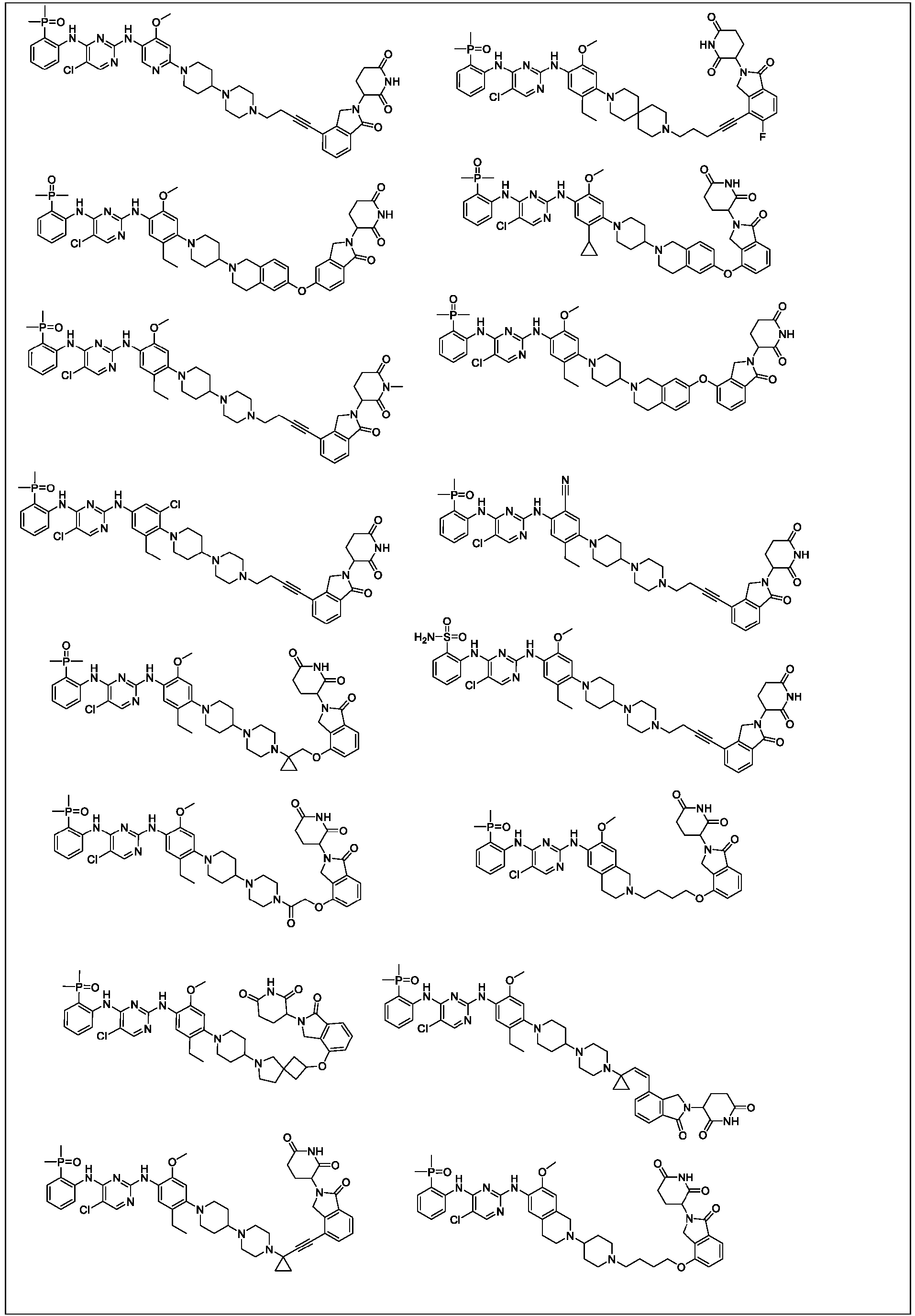

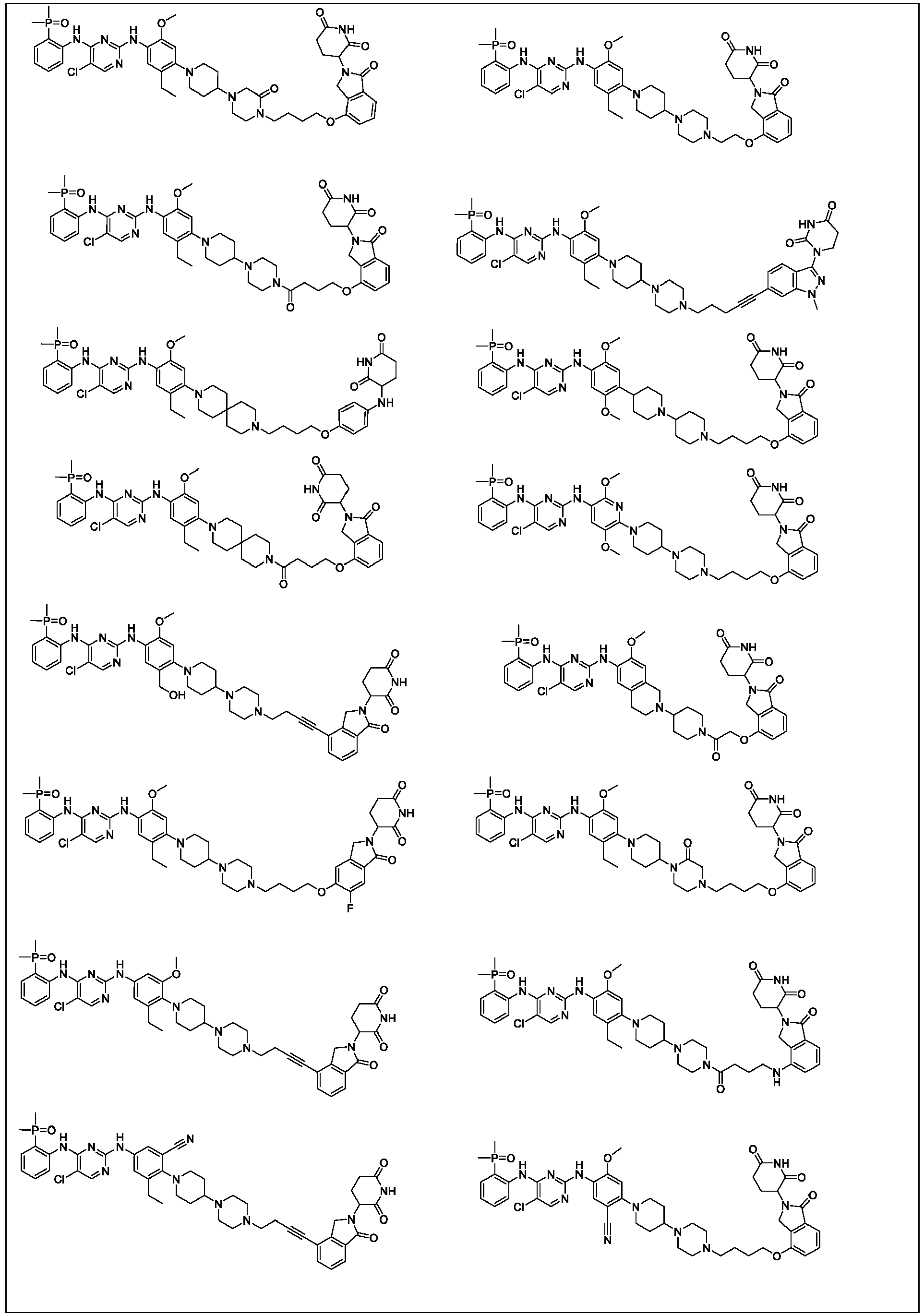

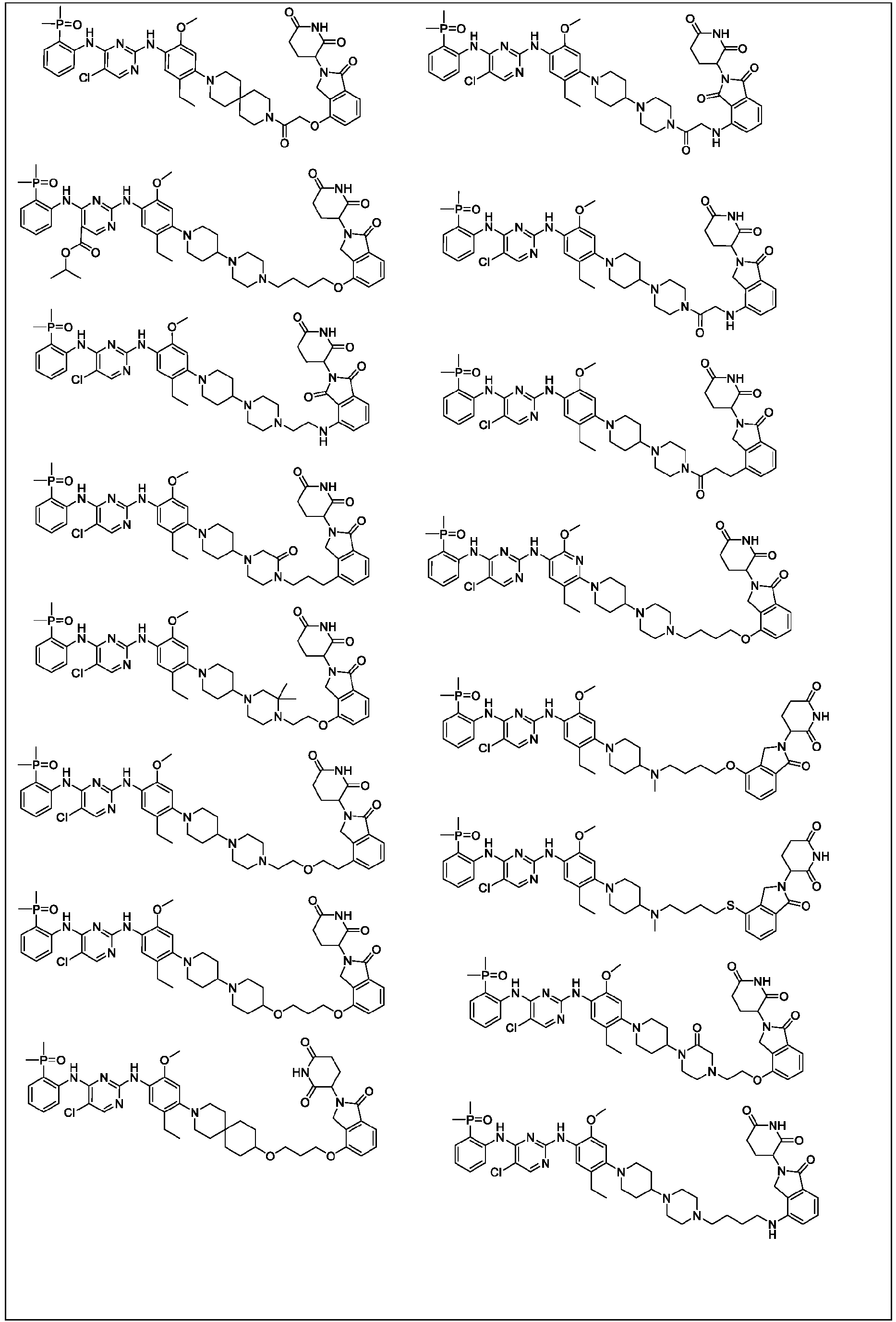

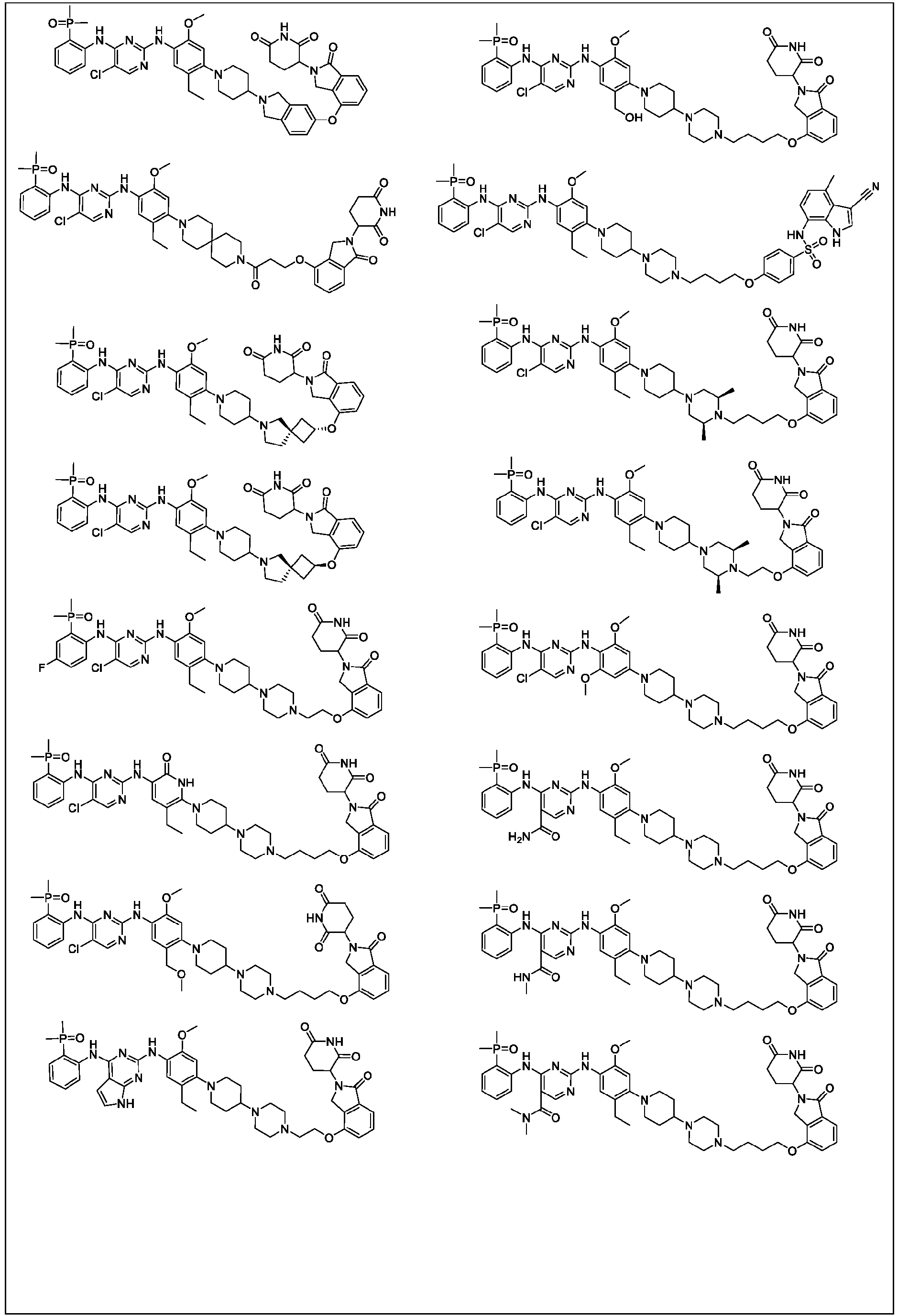

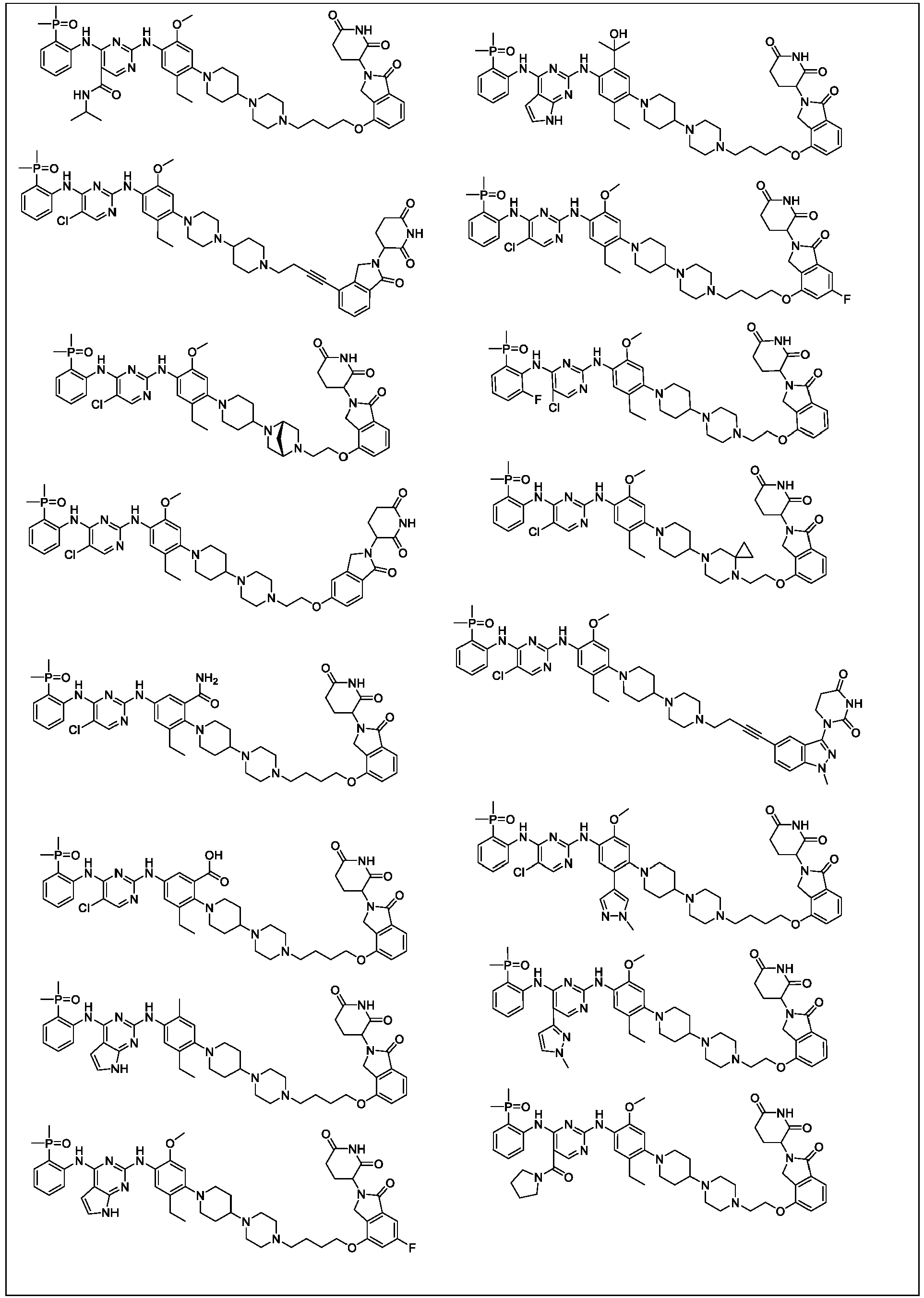

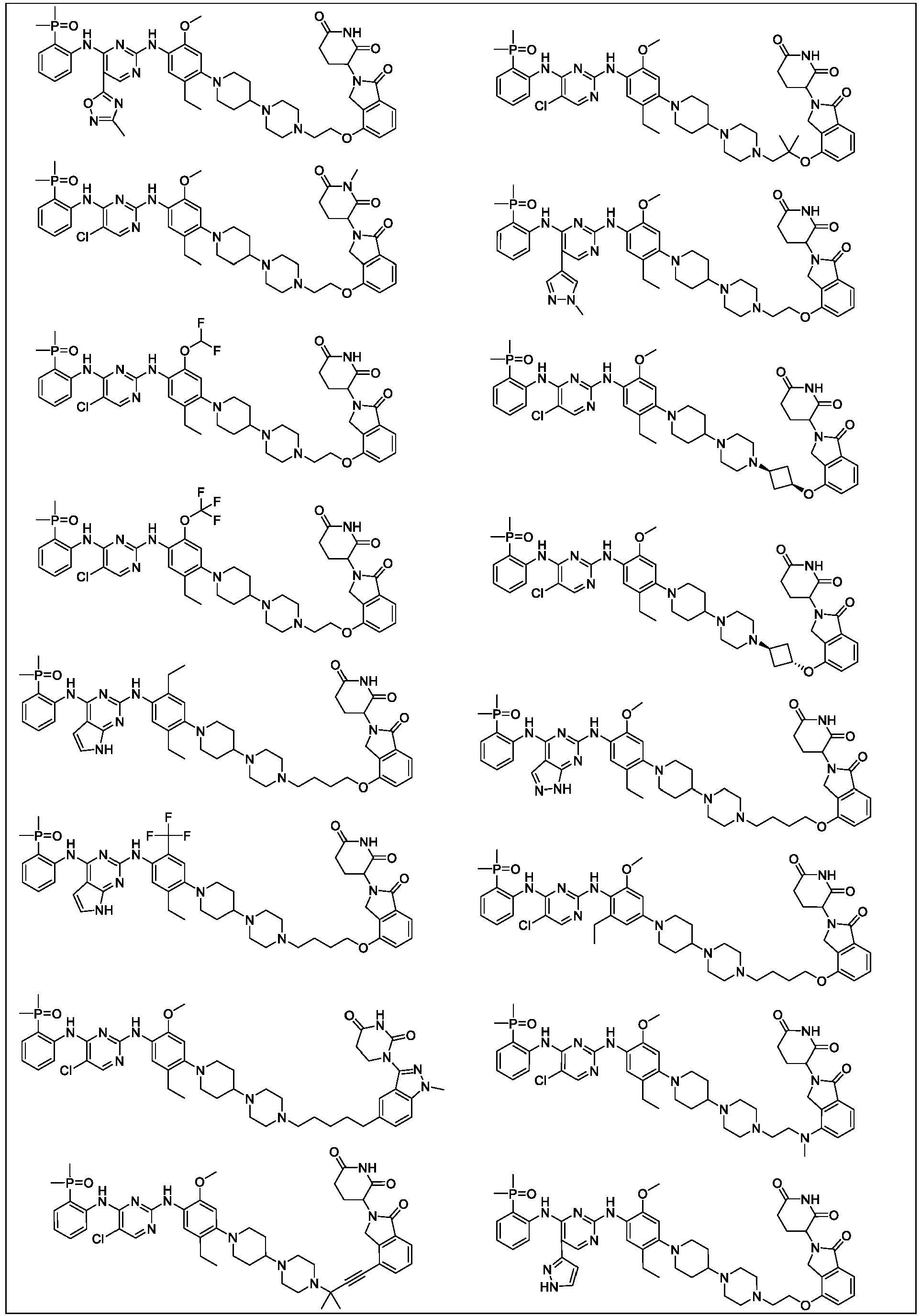

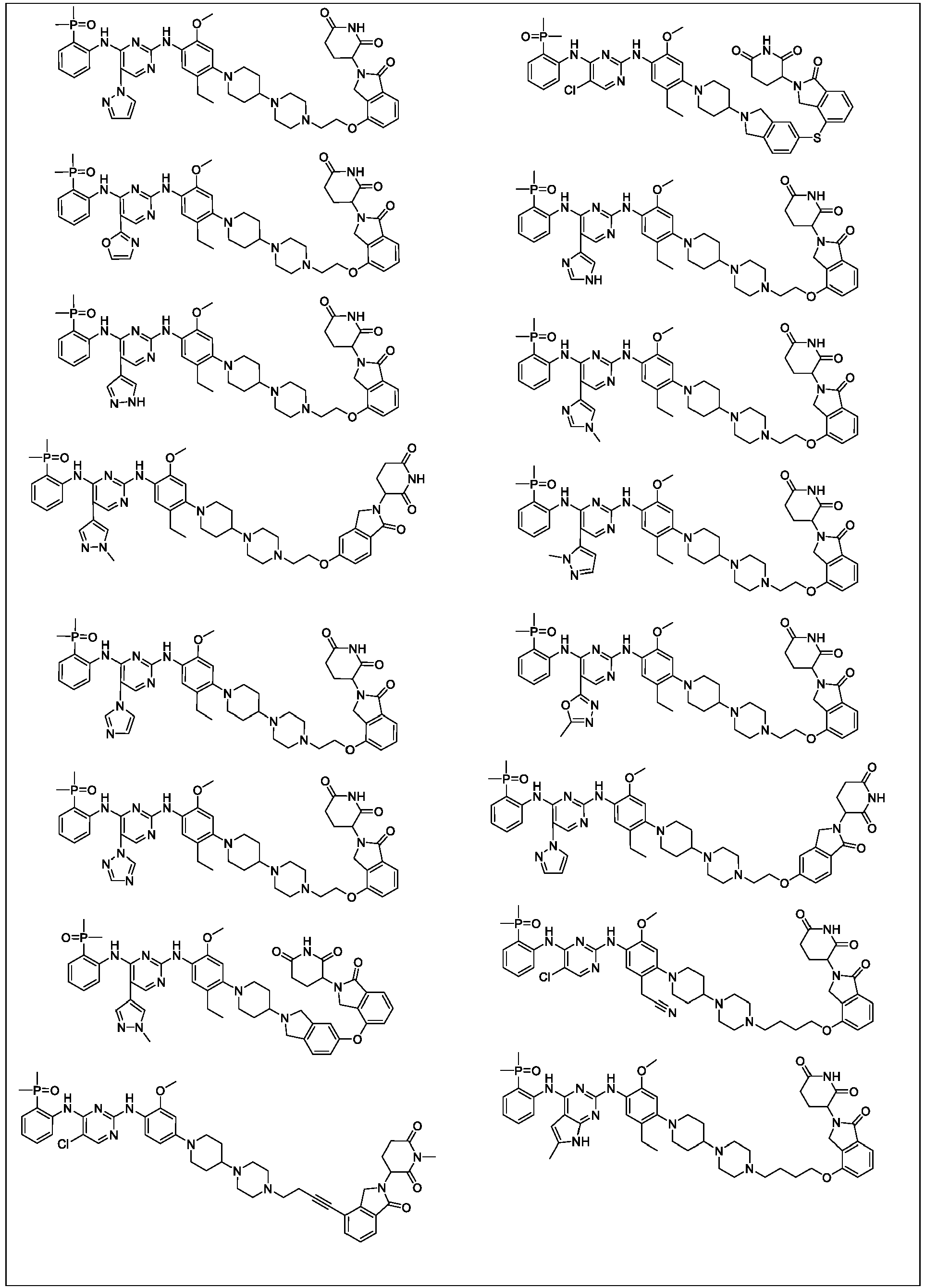

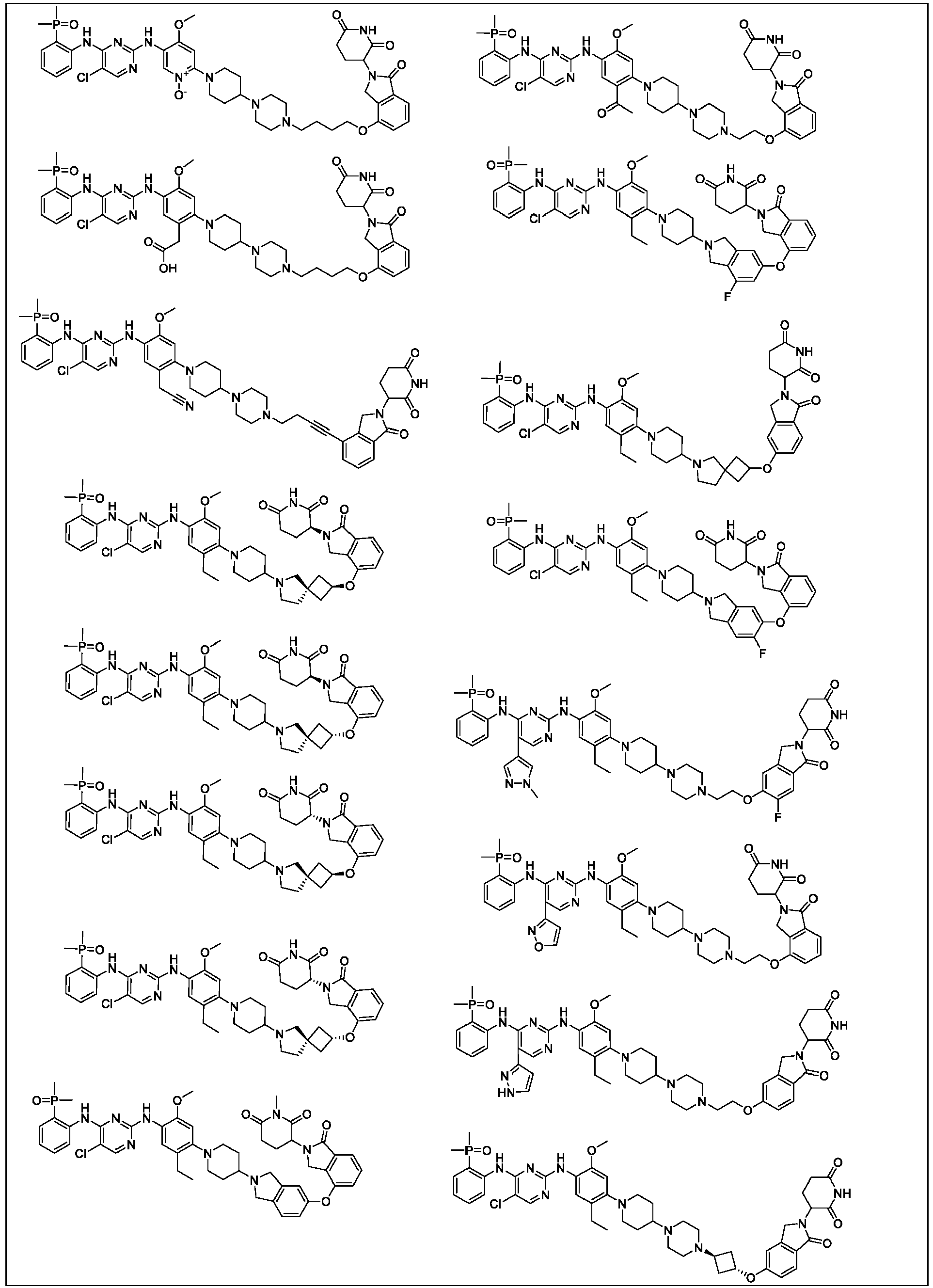

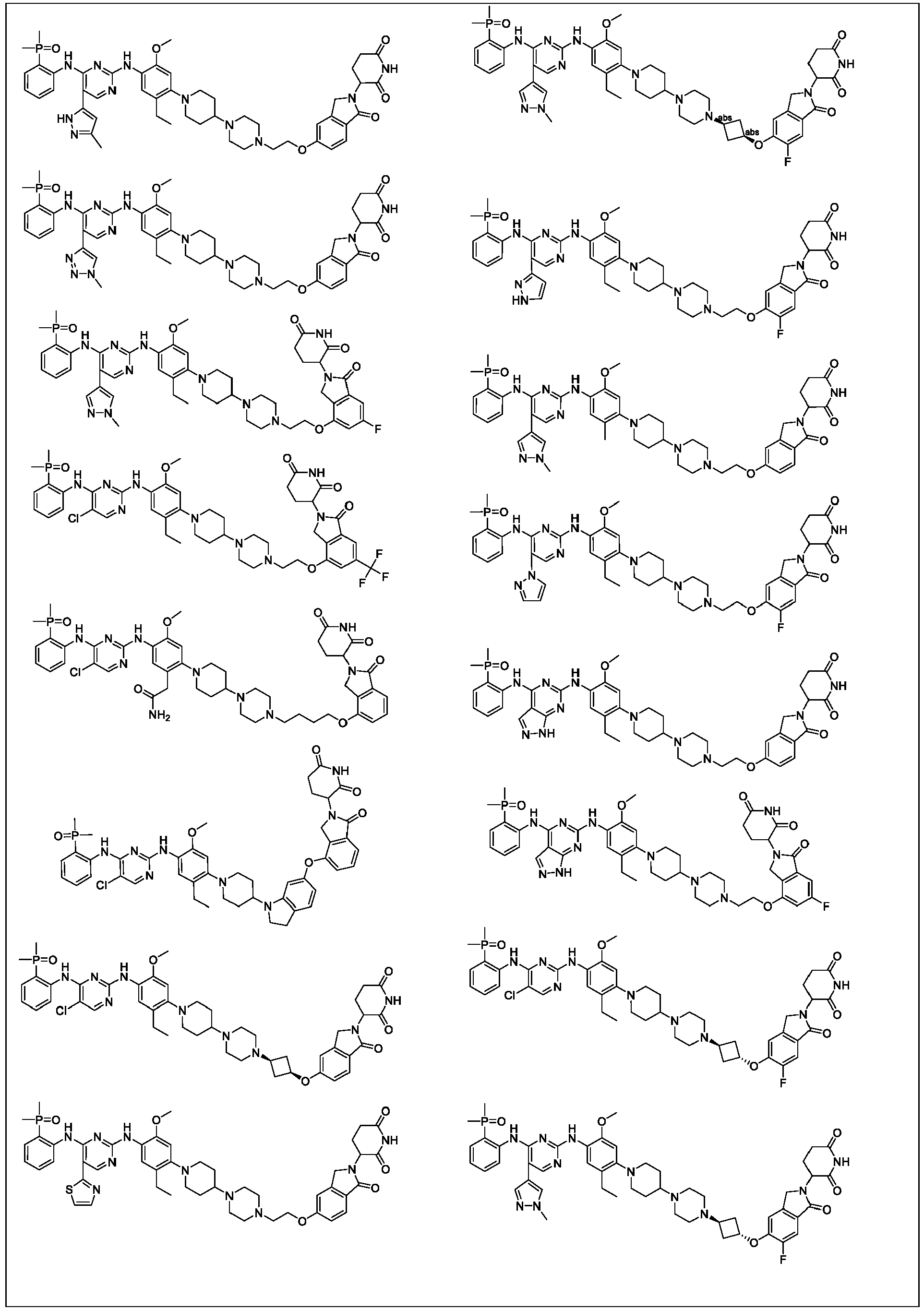

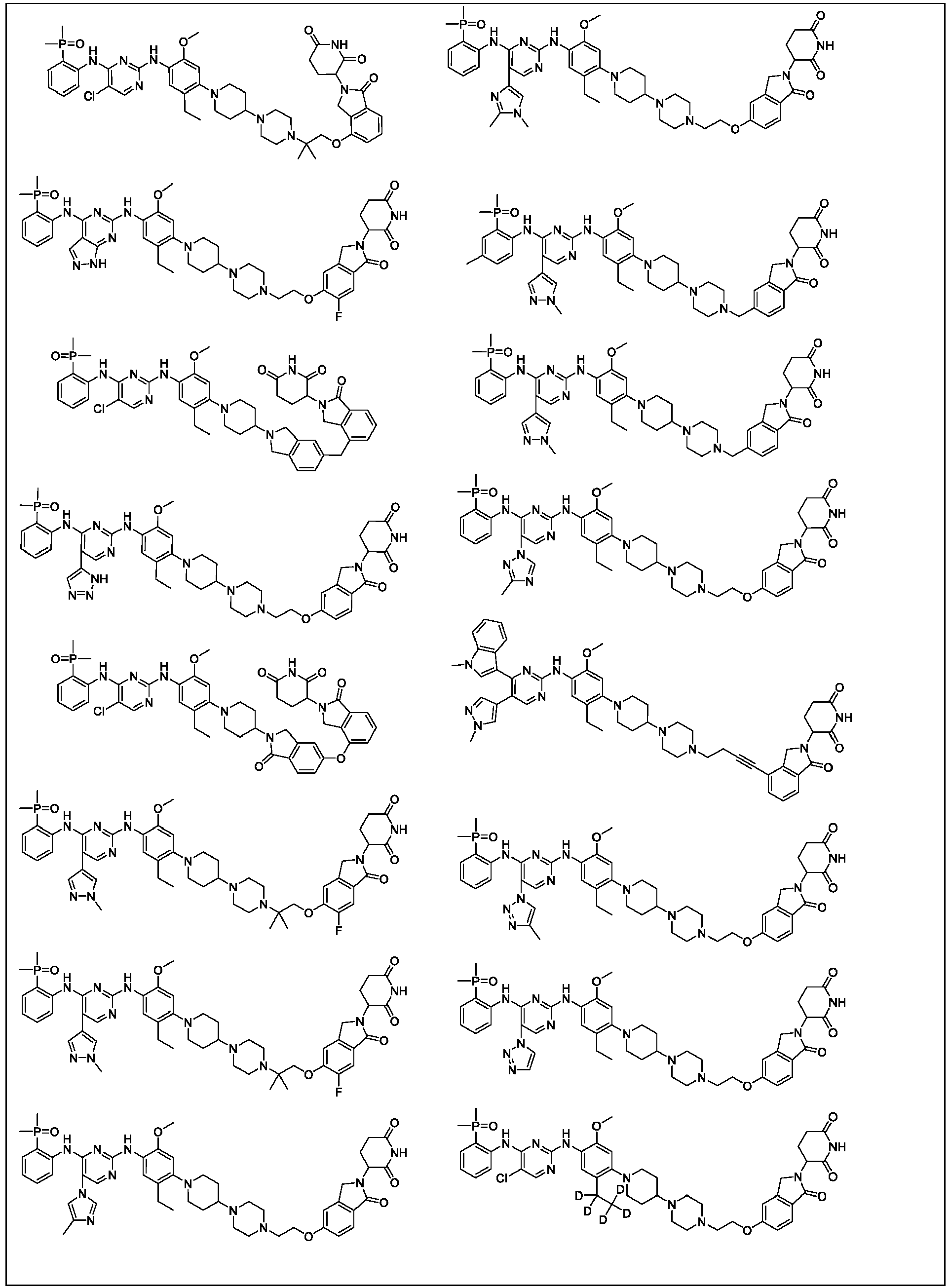

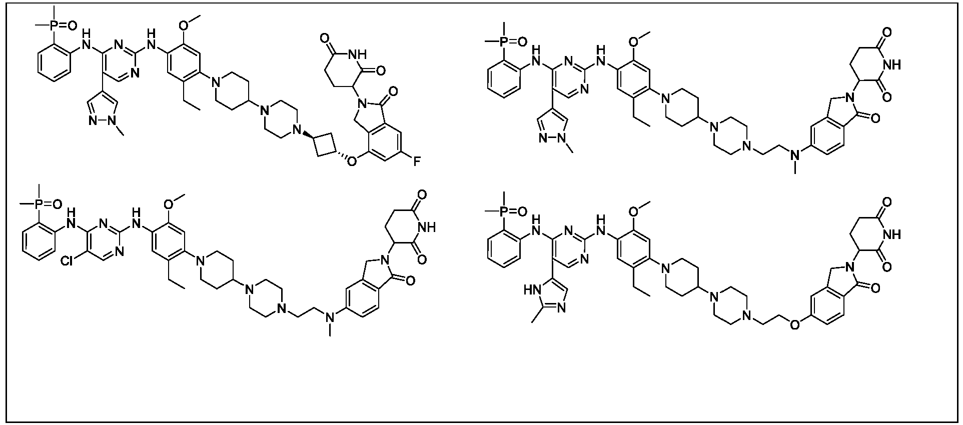

In another aspect, the present invention provides a compound of (1c-1), (1e-1), (1c-1-1), (1e-1-1), (1c-1-2), or (1e-1-2:) 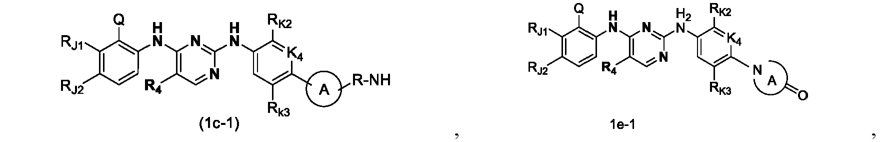 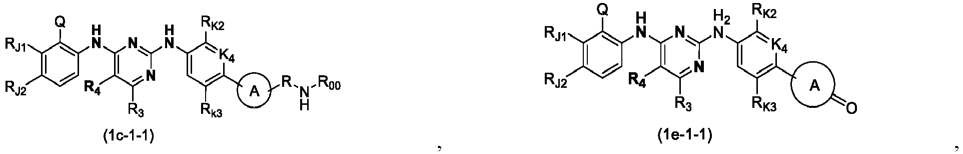 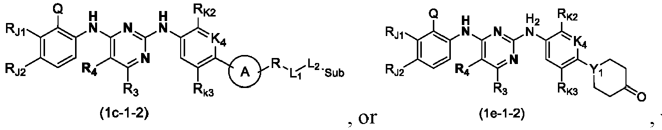 wherein
in formula (1c-1), when R is a bond, NH is NH on ring A; when R is not a bond, NH is NH on ring R;
Sub is selected from OH, SH, C(O)OH, S(O)OH, S(O)₂OH, NH, or a leaving group selected from fluorine, chlorine, bromine, iodine, boronic acid, boronate, -OMs, or -OTF; the variables in the formula are as defined in any one of the above items. Preferably, ring A is selected from a 5- to 7-membered azacycle or 7-16-membered azaspirocycle, and R is selected from a 5- to 7-membered azacycle or 7-16-membered azaspirocycle;
preferably, Sub is selected from OH, SH, C(O)OH, S(O)OH, S(O)₂OH, or NH; Sub is selected from bromine, iodine, boric acid, boronate, -OMs, or -OTF.

In another aspect, the present invention further provides a preparation method for a compound of formula (X-1) or (X-2): 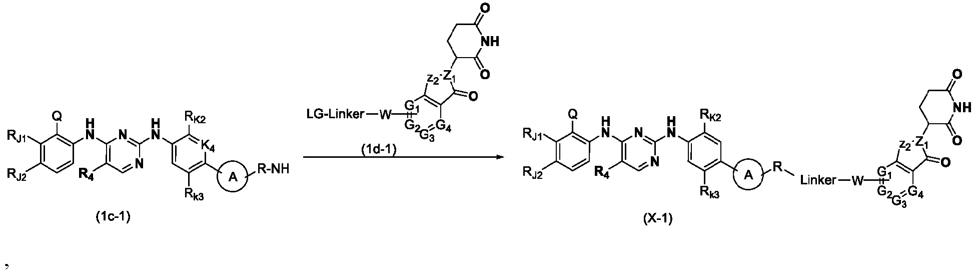 comprising reacting the compound of formula (1c-1) or (1c-2) with a compound of formula (1d-1) so as to prepare the compound of formula (X-1) or (X-2).

Specifically, the preparation method comprises: subjecting -NH in formula (1c-1) or (1c-2) to a nucleophilic substitution reaction with a leaving group of formula (1d-1) to obtain the compound of formula (X-1) or (X-2), wherein LG is the leaving group, and R_{J1}, R_{J2}, Q, R₄, Rₖ₂, Rₖ₃, ring A, R, Linker, W, G₁, G₂, G₃, G₄, Z₁, and Z₂ are defined in any one of the above items. -NH in formula (1c-1) or (1c-2) may be NH on ring R, or NH attached to R. In some embodiments, R is a bond, and then the leaving group reacts with NH of ring A.

The leaving group is preferably halogen (chlorine, bromine, or iodine), -OMs (mesylate), -OTf (triflate), -OTs (p-methylbenzenesulfonate), or the like. The nucleophilic substitution reaction is preferably conducted with a polar solvent in an alkaline system. Specifically, the polar solvent is preferably one or more of DMF, DMSO, NMP, acetonitrile, THF, and toluene, and the alkaline system includes: triethylamine, DIEA, sodium bicarbonate, potassium carbonate, sodium carbonate, cesium carbonate, cesium fluoride, and the like. In some embodiments, an alkali metal catalyst, such as NaI, KI, and CuI, may also be added as a catalyst.

In another aspect, the present invention further provides another preparation method for the compound of formula (X-1) or (X-2), 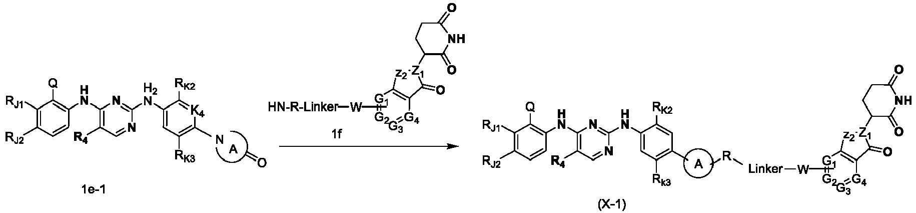 comprising reacting the compound of formula (1e-1) or (1e-2) with a compound of formula (1f) so as to prepare the compound of formula (X-1) or (X-2).

Specifically, the preparation method comprises: subjecting -C=O of formula (1e-1) or (1e-2) to a reductive amination reaction with NH of R in formula (1f) to obtain the compound of formula (X-1) or (X-2), wherein ring B, Q, R₄, Rₖ₂, Rₖ₃, ring A, R, Linker, W, G₁, G₂, G₃, G₄, Z₁, and Z₂ are defined in any one of the above items.

Preferably, the reducing agent is sodium cyanoborohydride or NaBH(OAc)₃, and the reaction is preferably conducted in a solvent, wherein the solvent is selected from 1,2-dichloroethane, dichloromethane, DMF, and the like.

In another aspect, the present invention provides a pharmaceutical composition comprising the compound of the present invention, and optionally a pharmaceutically acceptable excipient.

In another aspect, the present invention provides a pharmaceutical composition comprising the compound of the present invention and a pharmaceutically acceptable excipient, wherein the pharmaceutical composition further comprises an additional therapeutic agent.

In another aspect, the present invention provides use of the above compound or pharmaceutical composition in preparing a protein inhibitor or degrading agent, wherein the protein of the protein inhibitor or degrading agent is selected from at least one of EGFR, ROS1, or ALK.

In another aspect, the present invention provides a kit comprising the compound of the present invention, an additional therapeutic agent, and a pharmaceutically acceptable carrier, adjuvant or vehicle.

In another aspect, the present invention provides use of the compound of the present invention in preparing a medicament for treating and/or preventing a cancer.

In another aspect, the present invention provides a method for treating and/or preventing a cancer in a subject, comprising administering to the subject the compound of the present invention or the composition of the present invention.

In another aspect, the present invention provides the compound of the present invention or the composition of the present invention for use in the treatment and/or prevention of a cancer.

In a specific embodiment, the cancer is selected from lung cancer; lymphoma; inflammatory myofibroblastoma; colorectal cancer; cerebral glioma; astroblastoma; ovarian cancer; bone marrow cancer; transplantation-related cancer; neutropenia; leukemia; Wagner-Unverricht syndrome; bronchial carcinoma; prostate cancer; breast cancer; thyroid cancer; pancreatic cancer; neuroblastoma; extramedullary plasmacytoma; plasmacytoma; gastric cancer; gastrointestinal stromal tumor; esophageal cancer; large intestine adenocarcinoma; esophageal squamous cell carcinoma; liver cancer; renal cell carcinoma; bladder cancer; endometrial cancer; melanoma; brain cancer; oral cancer; sarcoma; a tumor resistant to targeted therapies; or a tumor or disease dependent on ALK, ROS1 or EGFR, or any mutein thereof.

In another specific embodiment, the cancer is selected from small cell lung cancer; non-small cell lung cancer; diffuse large B-cell lymphoma; non-Hodgkin's lymphoma; anaplastic lymphoma; anaplastic large cell lymphoma; CD20-positive lymphoma; primary lymphoma; B cell lymphoma; recurrent B-cell non-Hodgkin's lymphoma; recurrent diffuse large B-cell lymphoma; recurrent mediastinal (thymic) large B-cell lymphoma; primary mediastinal (thymic) large B-cell lymphoma; recurrent transformed non-Hodgkin's lymphoma; refractory B-cell non-Hodgkin's lymphoma; refractory diffuse large B-cell lymphoma; refractory primary mediastinal (thymic) large B-cell lymphoma; refractory transformed non-Hodgkin's lymphoma; multiple myeloma; myelodysplastic syndrome (MDS); previously treated myelodysplastic syndrome; plasma cell myeloma; smoldering myeloma; smoldering multiple myeloma; myelofibrosis; acute myeloid leukemia (AML); leukemia-associated anemia; chronic granulocytic leukemia; B cell chronic lymphocytic leukemia; Wagner-Unverricht syndrome; bronchial carcinoma; prostate cancer; triple-negative breast cancer; sporadic breast cancer; a patient with Cowden disease; thyroid cancer; pancreatic cancer; neuroblastoma; extramedullary plasmacytoma; plasmacytoma; gastric cancer; gastrointestinal stromal tumor; esophageal cancer; large intestine adenocarcinoma; esophageal squamous cell carcinoma; liver cancer; renal cell carcinoma; bladder cancer; endometrial cancer; melanoma; brain cancer; oral cancer; rhabdomyosarcoma; various adipose-derived tumors; Ewing sarcoma/primitive neuroectodermal tumor (Ewing/PNET); leiomyosarcoma; or a tumor resistant to EGFR-, ROS1- or ALK-targeted therapies.

In another specific embodiment, the cancer is selected from anaplastic lymphoma kinase (ALK) mutation-positive non-small cell lung cancer (NSCLC); ROS1-positive non-small cell lung cancer; EGFR mutant non-small cell lung cancer; lung adenocarcinoma; lung cancer resistant to EGFR-, ROS1-, or ALK-targeted therapies; lymphoma resistant to ALK-targeted therapies; or the following tumor, cancer or disease dependent on a protein selected from ALK, ROS 1 or EGFR, or any mutein thereof: lung cancer, lymphoma, inflammatory myofibroblastoma, colorectal cancer, cerebral glioma, astroblastoma, ovarian cancer, leukemia, breast cancer, thyroid cancer, neuroblastoma, extramedullary plasmacytoma, plasmacytoma, esophageal squamous cell carcinoma, renal cell carcinoma, bronchial carcinoma, prostate cancer, breast cancer, thyroid cancer, pancreatic cancer, neuroblastoma, extramedullary plasmacytoma, plasmacytoma, gastric cancer, gastrointestinal stromal tumor, esophageal cancer, large intestine adenocarcinoma, esophageal squamous cell carcinoma, liver cancer, renal cell carcinoma, bladder cancer, endometrial cancer, melanoma, brain cancer, oral cancer, or sarcoma. Other objects and advantages of the present invention will be apparent to those skilled in the art from the subsequent specific embodiments, examples, and claims.

In another aspect, the present invention further provides a compound, which is any one of the compounds in Table 2 or a salt thereof:

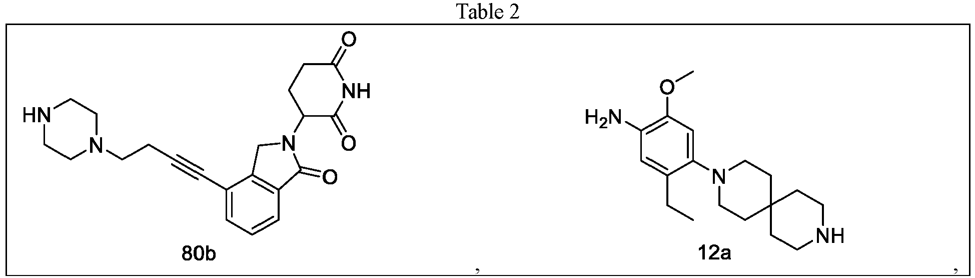

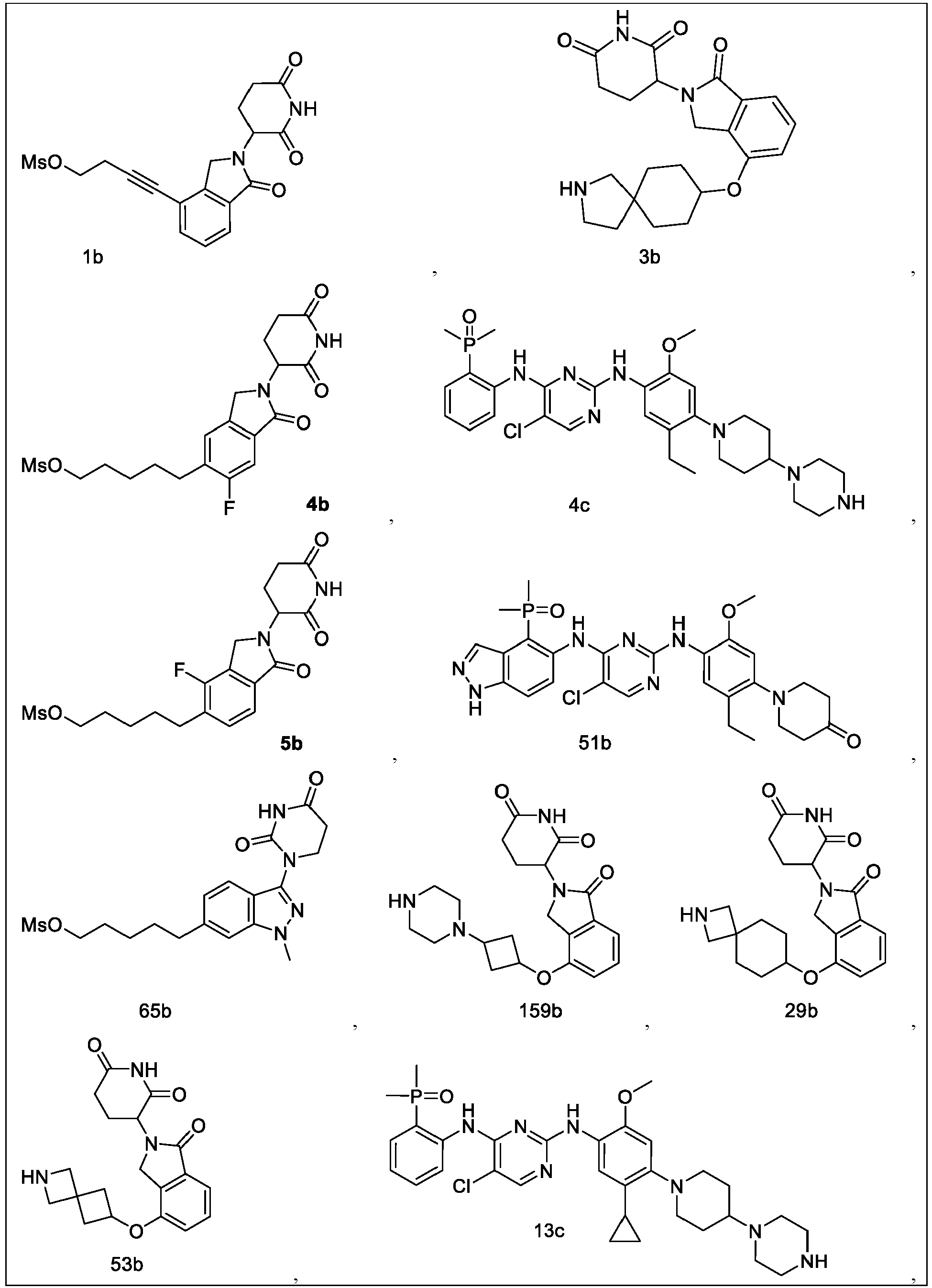

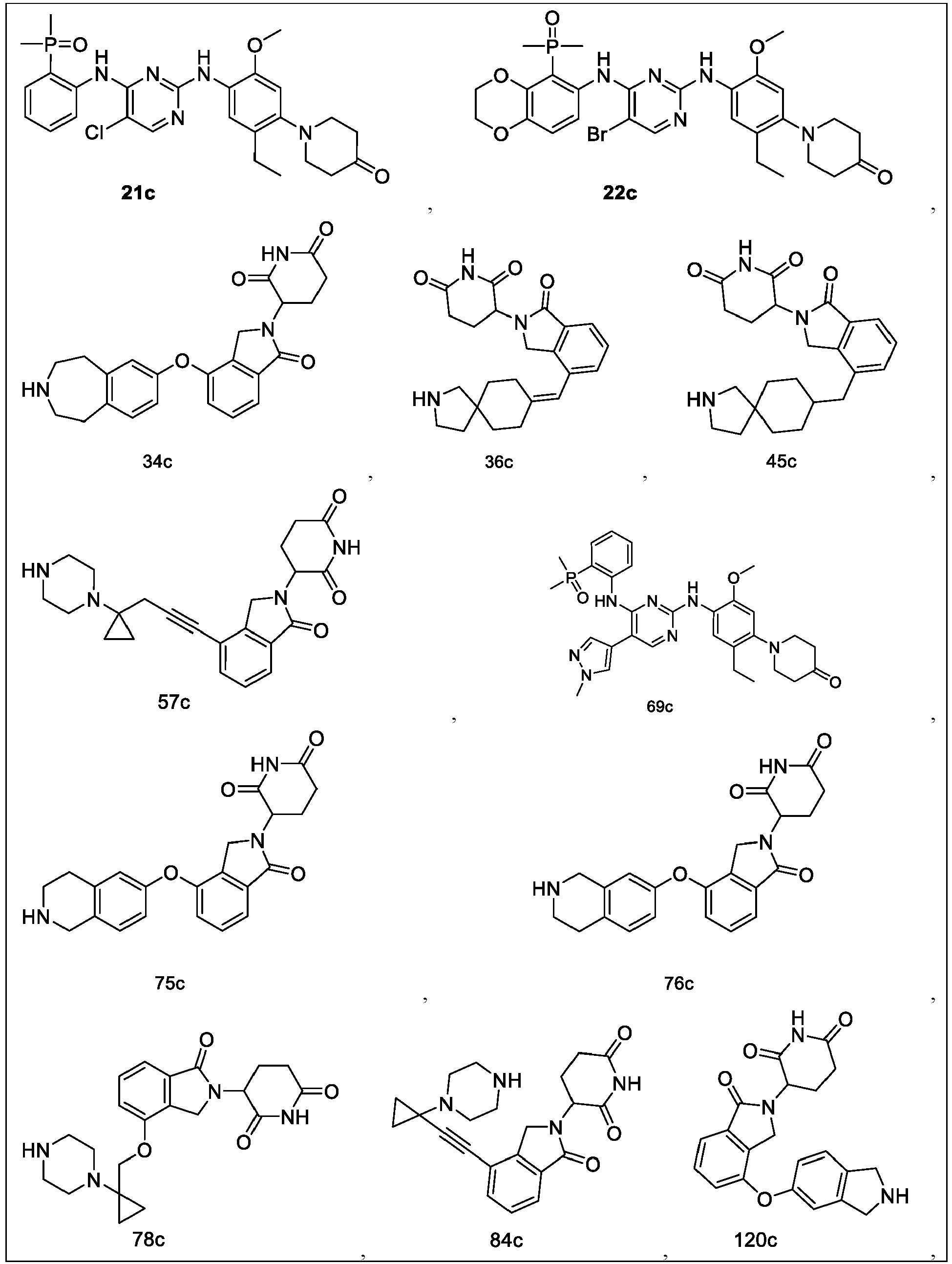

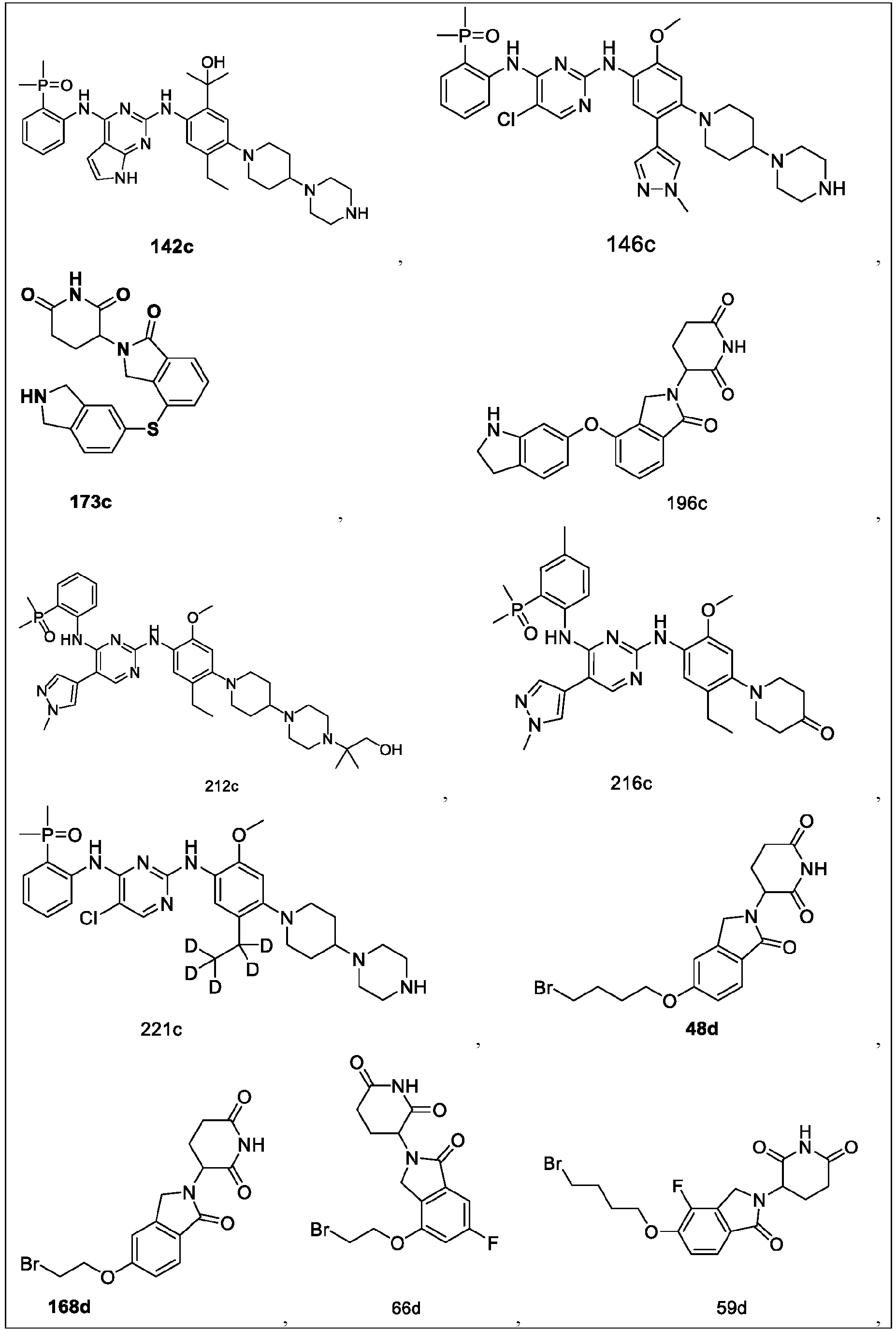

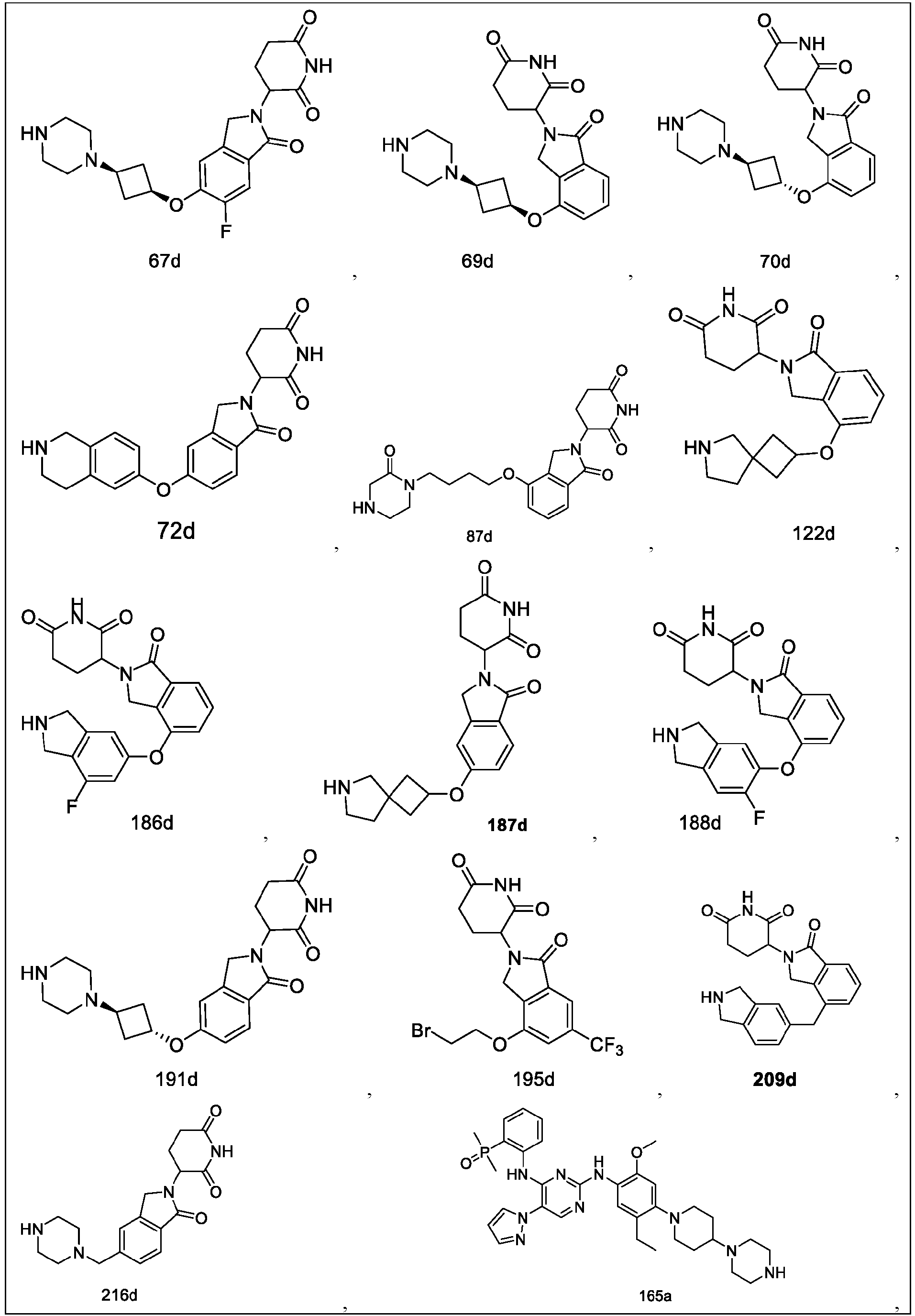

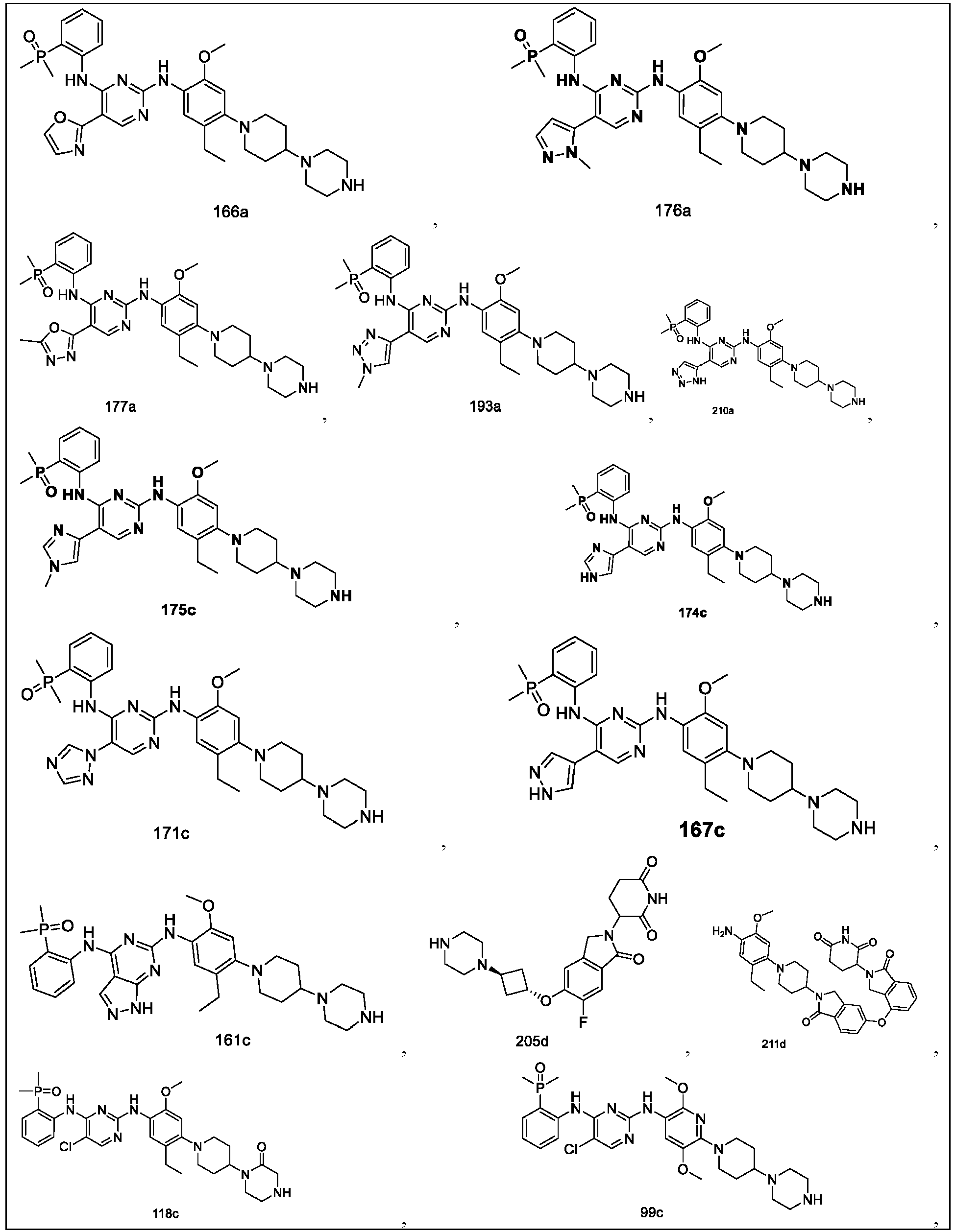

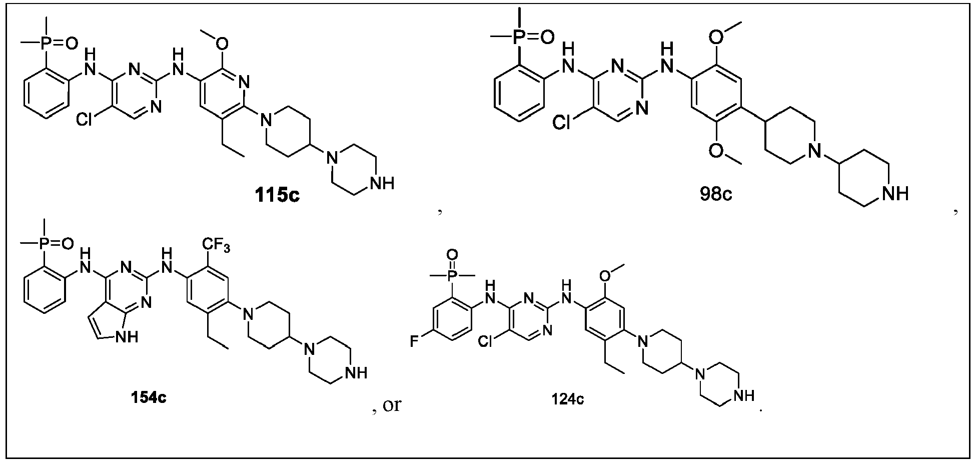

### Definitions

### Chemical definitions

Definitions of specific functional groups and chemical terms are described in more detail below.

### Chemical definitions

Definitions of specific functional groups and chemical terms are described in more detail below.

Where a numerical range is given, it is intended to include each value and every sub-range within itself. For example, "C₁₋₅ alkyl" includes C₁, C₂, C₃, C₄, C₅, C₁₋₅, C₁₋₄, C₁₋₃, C₁₋₂, C₂₋₅, C₂₋₄, C₂₋₃, C₃₋₅, C₃₋₄ and C₄₋₅ alkyl. "Alkyl" refers to linear and branched saturated aliphatic alkyl, and C₁₋₁₀ alkyl is alkyl containing 1 to 10 carbon atoms, preferably C₁₋₈ alkyl, more preferably C₁₋₅ alkyl, and even more preferably C₁₋₃ alkyl. Non-limiting examples of C₁₋₁₀ alkyl include: methyl, ethyl, n-propyl, isopropyl, n-butyl, isobutyl, tert-butyl, sec-butyl, *n-*pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n-*nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof, and the like.

"Alkylene" means a divalent group formed by the removal of another hydrogen from the above "alkyl" and may be substituted or unsubstituted. C₁₋₁₀ alkylene is preferred, C₁₋₅ alkylene is more preferred, and C₁₋₃ alkylene is even more preferred. Exemplary alkylene groups include, but are not limited to, methylene, ethylene, propylene, butylene, and the like.

"C₂₋₁₀ alkenyl" refers to a linear or branched hydrocarbon group having 2 to 10 carbon atoms and at least one carbon-carbon double bond. In some embodiments, C₂₋₆ alkenyl is preferred; in some embodiments, C₂₋₄ alkenyl is preferred. Examples of C₂₋₁₀ alkenyl include: ethenyl (C₂), 1-propenyl (C₃), 2-propenyl (C₃), 1-butenyl (C₄), 2-butenyl (C₄), butadienyl (C₄), pentenyl (C₅), pentadienyl (C₅), hexenyl (C₆), heptenyl (C₇), octenyl (C₈), and the like. The term "C₂₋₁₀ alkenyl" also encompasses heteroalkenyl where one or more (e.g., 1, 2, 3, or 4) carbon atoms are replaced with heteroatoms (e.g., oxygen, sulfur, nitrogen, boron, silicon, or phosphorus). An alkenyl group may optionally be substituted with one or more substituents, for example, 1 to 5 substituents, 1 to 3 substituents, or 1 substituent.

"C₂₋₁₀ alkynyl" refers to a linear or branched hydrocarbon group having 2 to 10 carbon atoms and at least one carbon-carbon triple bond. In some embodiments, C₂₋₆ alkynyl is preferred; in some embodiments, C₂₋₄ alkynyl is preferred. Examples of C₂₋₁₀ alkynyl include, but are not limited to: ethynyl (C₂), 1-propynyl (C₃), 2-propynyl (C₃), 1-butynyl (C₄), 2-butynyl (C₄), pentynyl (C₅), hexynyl (C₆), heptynyl (C₇), octynyl (C₈), and the like. The term "C₂₋₁₀ alkynyl" also encompasses heteroalkynyl where one or more (e.g., 1, 2, 3, or 4) carbon atoms are replaced with heteroatoms (e.g., oxygen, sulfur, nitrogen, boron, silicon, or phosphorus). An alkynyl group may optionally be substituted with one or more substituents, for example, 1 to 5 substituents, 1 to 3 substituents, or 1 substituent.

"Halo" or "halogen" refers to fluorine (F), chlorine (Cl), bromine (Br), and iodine (I).

Accordingly, "C₁₋₅ haloalkyl" refers to the above "C₁₋₅ alkyl", which is substituted with one or more halogen groups. In some embodiments, C₁₋₄ haloalkyl is particularly preferred, and C₁₋₂ haloalkyl is more preferred. Examples of the haloalkyl include, but are not limited to: -CF₃, -CH₂F, -CHF₂, -CHFCH₂F, -CH₂CHF₂, - CF₂CF₃, -CCl₃, -CH₂Cl, -CHCl₂, 2,2,2-trifluoro-1,1-dimethyl-ethyl, and the like. A haloalkyl group may be substituted at any accessible point of attachment with, for example, 1 to 5 substituents, 1 to 3 substituents, or 1 substituent.

"C₃₋₈ cycloalkyl" refers to a non-aromatic cyclic hydrocarbon group having 3 to 8 ring carbon atoms and zero heteroatoms. In some embodiments, C₃₋₆ cycloalkyl and C₃₋₅ cycloalkyl are particularly preferred. Cycloalkyl also includes ring systems where the cycloalkyl ring described above is fused with one or more aryl or heteroaryl groups, wherein the point of attachment is on the cycloalkyl ring, and in such instances, the number of carbons continues to represent the number of carbons in the cycloalkyl system. Examples of the cycloalkyl include, but are not limited to: cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), cycloheptyl (C₇), cycloheptenyl (C₇), cycloheptadienyl (C₇), cycloheptatrienyl (C₇), and the like. A cycloalkyl group may optionally be substituted with one or more substituents, for example, 1 to 5 substituents, 1 to 3 substituents, or 1 substituent.

"3- to 8-membered heterocycloalkyl" refers to a radical of a 3- to 8-membered non-aromatic ring system having ring carbon atoms and 1 to 5 ring heteroatoms, wherein each of the heteroatoms is independently selected from nitrogen, oxygen, sulfur, boron, phosphorus, and silicon, and the heteroatoms are independently preferably nitrogen and/or oxygen, e.g., nitrogen; the number of the heteroatom is preferably 1 or 2. In a heterocyclyl group containing one or more nitrogen atoms, the point of attachment may be a carbon atom or a nitrogen atom, so long as valence permits. 3- to 7-membered heterocyclyl is preferred, 3- to 6-membered heterocyclyl is preferred, 4- to 8-membered heterocyclyl is preferred, and 5- to 6-membered heterocyclyl is more preferred, which is a 5- to 6-membered non-aromatic ring system having ring carbon atoms and 1 to 3 ring heteroatoms. Exemplary 3- to 8-membered heterocycloalkyl groups include, but are not limited to: aziridinyl, oxiranyl, thiorenyl\azetidinyl, oxetanyl, thietanyl, tetrahydrofuranyl, dihydrofuranyl, tetrahydrothienyl, dihydrothienyl, pyrrolidinyl, dihydropyrrolyl, pyrrolyl-2,5-dione, dioxolanyl, oxasulfuranyl, disulfuranyl, oxazolidin-2-one, triazolinyl, oxadiazolinyl, thiadiazolinyl, piperidinyl, tetrahydropyranyl, dihydropyridinyl, thianyl, piperazinyl, morpholinyl, dithianyl, dioxanyl, and the like.

As used herein, "cycloalkyl" and "cycloalkyl ring" are used interchangeably.

"C₆₋₁₀ aryl" refers to a radical of a monocyclic or polycyclic (e.g., bicyclic) 4n+2 aromatic ring system (e.g., having 6 or 10 π electrons shared in a cyclic arrangement) having 6-10 ring carbon atoms and zero heteroatoms. In some embodiments, an aryl group has six ring carbon atoms ("C₆ aryl"; e.g., phenyl). In some embodiments, an aryl group has ten ring carbon atoms ("C₁₀ aryl"; e.g., naphthyl such as 1-naphthyl and 2-naphthyl). Aryl further includes ring systems where the aryl ring described above is fused with one or more cycloalkyl or heterocyclyl groups, wherein the point of attachment is on the aryl ring, and in such instances, the number of carbon atoms continues to represent the number of carbon atoms in the aryl ring system. An aryl group may optionally be substituted with one or more substituents, for example, 1 to 5 substituents, 1 to 3 substituents, or 1 substituent.

"5- to 14-membered heteroaryl" refers to a group having a 5- to 14-membered monocyclic or bicyclic 4n+2 aromatic ring system (e.g., having 6, 10, or 14 π electrons shared in a cyclic arrangement) having ring carbon atoms and 1-4 ring heteroatoms, wherein each of the heteroatoms is independently selected from nitrogen, oxygen, and sulfur; in the 5- to 14-membered heteroaryl, the heteroatoms are independently preferably nitrogen and/or oxygen, e.g., nitrogen; the number of the heteroatom is preferably 1, 2, or 3. In a heteroaryl group containing one or more nitrogen atoms, the point of attachment may be a carbon atom or a nitrogen atom, so long as valence permits. Heteroaryl bicyclic ring systems may include one or more heteroatoms in one or both rings. Heteroaryl further includes ring systems where the heteroaryl ring described above is fused with one or more cycloalkyl or heterocyclyl groups, wherein the point of attachment is on the heteroaryl ring, and in such instances, the number of carbon atoms continues to represent the number of carbon atoms in the heteroaryl ring system. 5- to 10-membered heteroaryl is preferred, and 5- to 6-membered heteroaryl is more preferred. Exemplary 5- to 6-membered heteroaryl groups include, but are not limited to: pyrrolyl, furanyl, thienyl, imidazolyl, pyrazolyl, oxazolyl, isoxazolyl, thiazolyl, isothiazolyl, triazolyl, oxadiazolyl (e.g., 1,2,4-oxadiazolyl), thiadiazolyl, tetrazolyl, pyridyl, pyridazinyl, pyrimidinyl, pyrazinyl, triazinyl, tetrazinyl, azepinyl, oxepinyl, and thiepinyl. Exemplary 5,6-bicyclic heteroaryl groups include, but are not limited to: indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, but are not limited to: naphthyridinyl, pteridinyl, quinolyl, isoquinolyl, cinnolinyl, quinoxalinyl, phthalazinyl, and quinazolinyl. A heteroaryl group may optionally be substituted with one or more substituents, for example, 1 to 5 substituents, 1 to 3 substituents, or 1 substituent.

"C₆₋₁₀ arylene" and "5- to 14-membered heteroarylene" refer to divalent groups formed by the removal of another hydrogen from the above "C₆₋₁₀ aryl" and "5- to 14-membered heteroaryl", respectively, and may be substituted or unsubstituted. C₆₋₁₀ arylene and 5- to 10-membered heteroarylene are preferred. Representative C₆₋₁₀ arylene groups include 1,2-phenylene, 1,3-phenylene, 1,4-phenylene, 1,2-naphthylene, 1,3-naphthylene, 1,4-naphthylene, 1,5-naphthylene, 1,6-naphthylene, 1,7-naphthylene, 1,8-naphthylene, 2,3-naphthylene, 2,5-naphthylene, 2,6-naphthylene, and 2,7-naphthylene. Representative 5- to 10-membered heteroarylene groups include 1,2-pyrrolylene, 1,3-pyrrolylene, 2,3-pyrrolylene, 2,4-pyrrolylene, 2,5-pyrrolylene, 3,4-pyrrolylene, 2,3-furanylene, 2,4-furanylene, 2,5-furanylene, 3,4-furanylene, 2,3-thienylene, 2,4-thienylene, 2,5-thienylene, 3,4-thienylene, 1,2-imidazolylene, 1,4-imidazolylene, 1,5-imidazolylene, 2,4-imidazolylene, 2,5-imidazolylene, 4,5-imidazolylene, 1,3-pyrazolylene, 1,4-pyrazolylene, 1,5-pyrazolylene, 3,4-pyrazolylene, 3,5-pyrazolylene, 4,5-pyrazolylene, 2,4-oxazolylene, 2,5-oxazolylene, 4,5-oxazolylene, 3,4-isoxazolylene, 3,5-isoxazolylene, 4,5-isoxazolylene, 2,4-thiazolylene, 2,5-thiazolylene, 4,5-thiazolylene, 3,4-isothiazolylene, 3,5-isothiazolylene, 4,5-isothiazolylene, 1,4-triazolylene, 1,5-triazolylene, 4,5-triazolylene, 1,2,4-oxadiazol-3,5-ylene, 1,2,3-oxadiazol-4,5-ylene, 1,2,4-thiadiazol-3,5-ylene, 1,2,3-thiadiazol-4,5-ylene, 2,3-pyridinylene, 2,4-pyridinylene, 2,5-pyridinylene, 2,6-pyridinylene, 3,4-pyridinylene, 3,5-pyridinylene, 3,4-pyridazinylene, 3,5-pyridazinylene, 3,6-pyridazinylene, 4,5-pyridazinylene, 2,4-pyrimidinylene, 2,5-pyrimidinylene, 4,5-pyrimidinylene, 4,6-pyrimidinylene, 2,3-pyrazinylene, 2,5-pyrazinylene, 2,6-pyrazinylene, 1,2,3-triazinyl-4,5-ylene, 1,2,3-triazinyl-4,6-ylene, 1,2,4-triazinyl-3,5-ylene, 1,2,4-triazinyl-3,6-ylene, 1,2,5-triazinyl-3,4-ylene, 1,2,5-triazinyl-3,6-ylene, 1,2,5-triazinyl-4,6-ylene, 1,3,5-triazinyl-2,4-ylene, 1,2,3,4-tetrazinyl-5,6-ylene, 1,2,3,5-tetrazinyl-4,6-ylene, 1,2,4,6-tetrazinyl-3,5-ylene, and the like.

"Heteroaryl ring" and "heteroaryl group" are used interchangeably and refer to a monocyclic or fused polycyclic (i.e., rings that share a pair of adjacent carbon atoms or heteroatoms) group containing 1 to 4 heteroatoms, having 5 to 14 ring atoms, preferably 5 to 10 ring atoms, more preferably 5, 6, 8, 9, or 10 ring atoms, wherein the heteroatoms are selected from oxygen, sulfur, and nitrogen, wherein the nitrogen and sulfur atoms may optionally be oxidized, and the nitrogen atoms may optionally be quaternized; in the "heteroaryl ring" and "heteroaryl", the heteroatoms are independently preferably nitrogen and/or oxygen, e.g., nitrogen; the number of the heteroatom is preferably 1, 2, or 3. The heteroaryl has 6, 10, or 14 π electrons shared in a ring system. At least one ring of the ring system is aromatic. In the present invention, the C₅₋₁₄ heteroaryl includes 5- or 6-membered monocyclic heteroaryl, 8- to 10-membered bicyclic heteroaryl, 11- to 14-membered tricyclic heteroaryl, a heteroaryl group formed by the fusion of a 5- or 6-membered monocyclic heteroaryl ring with a 5- to 6-membered saturated or unsaturated monoheterocycle, a heteroaryl group formed by the fusion of a 5- or 6-membered monocyclic heteroaryl ring with a 5- to 6-membered saturated or unsaturated monocycle, and a heteroaryl group formed by the fusion of a benzene ring with a 5- to 6-membered saturated or unsaturated monoheterocycle.

"5- to 6-membered heteroaryl" refers to a monocyclic heteroaryl ring containing 5 to 6 ring atoms, for example, including (but not limited to): thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine, etc.; in the 5- to 6-membered heteroaryl, the heteroatoms are independently preferably nitrogen and/or oxygen, e.g., nitrogen; the number of the heteroatom is preferably 1, 2, or 3.

"8- to 10-membered bicyclic heteroaryl" refers to a 9- or 10-membered bicyclic heteroaryl formed by the fusion of a benzene ring with a 5- or 6-membered monocyclic heteroaryl, or an 8- to 10-membered bicyclic heteroaryl formed by the fusion of a 5- or 6-membered monocyclic heteroaryl with a 5- or 6-membered monocyclic heteroaryl; wherein the 5- or 6-membered monocyclic heteroaryl is selected from: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, or pyrazine. In the 8- to 10-membered bicyclic heteroaryl, the heteroatoms are independently preferably nitrogen and/or oxygen, e.g., nitrogen; the number of the heteroatom is preferably 1, 2, or 3.

As used herein, "saturated or unsaturated monocycle" and "saturated or unsaturated monocycloalkyl" are used interchangeably and refer to a saturated or unsaturated all-carbon monocyclic ring system, wherein "unsaturated" refers to a cyclic moiety comprising at least one double or triple bond. 3- to 6-membered is preferred, 4- to 6-membered is more preferred, and 5- to 6-membered is more preferred.

"Saturated or unsaturated monoheterocycle", "saturated or unsaturated monocyclic heterocycle", and "monocyclic heterocyclyl" are used interchangeably and refer to a saturated or unsaturated monocycle in which 1, 2, or 3 ring carbon atoms are replaced by a heteroatom selected from nitrogen, oxygen or S(O)t (where t is an integer of 0 to 2), but which excludes a cyclic moiety of -O-O-, -O-S-, or -S-S-, with the remaining ring atoms being carbon. It is preferably 3- to 6-membered, more preferably 4- to 6-membered, and more preferably 5- to 6-membered. In the "saturated or unsaturated monoheterocycle", "saturated or unsaturated monocyclic heterocycle", and "monocyclic heterocyclyl", the heteroatoms are independently preferably nitrogen and/or oxygen, e.g., nitrogen; the number of the heteroatom is preferably 1, 2, or 3.

"3- to 8-membered monocyclic heterocyclyl" refers to a monocyclic heterocyclic system group containing 3 to 8 ring atoms, the heteroatoms being nitrogen, oxygen, sulfur, etc.; 5- to 7-membered monocyclic heterocycle is preferred, for example, including (but not limited to): oxolane, azacyclopentane, thiolane, 1,3-dioxolane, piperidine, piperazine, morpholine, and the like. In the "3- to 8-membered monocyclic heterocyclyl", the heteroatoms are independently preferably nitrogen and/or oxygen, e.g., nitrogen; the number of the heteroatom is preferably 1, 2, or 3.

"Polycycle" and "polycycloalkyl" are used interchangeably and refer to a saturated or unsaturated bicyclic, tricyclic, or higher polycyclic all-carbon cyclic hydrocarbon system, wherein the bicyclic system includes spiro rings and fused rings, and the tricyclic system includes bridged rings. Fused rings and spiro rings are preferred.

"Polycyclic heterocycle" and "polycyclic heteroalkyl" are used interchangeably and refer to a saturated or unsaturated polycycle in which 1, 2, 3, or 4 ring carbon atoms are replaced by a heteroatom selected from nitrogen, oxygen, or S(O)t (where t is an integer of 0 to 2), but which excludes a cyclic moiety of -O-O-, -O-S-, or -S-S-, with the remaining ring atoms being carbon. Spiro heterocycle (also known as spiroheterocyclyl) or bicyclic fused heterocyclyl is preferred, and 7- to 16-membered spiroheterocyclyl and 7- to 10-membered bicyclic fused heterocyclyl are more preferred. "5- or 6-membered monocyclic heteroaryl ring" and "5- or 6-membered monocyclic heteroaryl" are used interchangeably and both refer to a monocyclic heteroaryl ring containing 5 or 6 ring atoms, for example, including (but not limited to): a thiophene ring, a *N-*alkylcyclopyrrole ring, a furan ring, a thiazole ring, an imidazole ring, an oxazole ring, a pyrrole ring, a pyrazole ring, a triazole ring, a 1,2,3-triazole ring, a 1,2,4-triazole ring, a 1,2,5-triazole ring, a 1,3,4-triazole ring, a tetrazole ring, an isoxazole ring, an oxadiazole ring, a 1,2,3-oxadiazole ring, a 1,2,4-oxadiazole ring, a 1,2,5-oxadiazole ring, a 1,3,4-oxadiazole ring, a thiadiazole ring, a pyridine ring, a pyridazine ring, a pyrimidine ring, a pyrazine ring, and the like. In the "polycyclic heterocycle" and "polycyclic heteroalkyl", the heteroatoms are independently preferably nitrogen and/or oxygen, e.g., nitrogen; the number of the heteroatom is preferably 1, 2, or 3.

"8- to 10-membered bicyclic heteroaryl ring" and "8- to 10-membered bicyclic heteroaryl" are used interchangeably and both refer to a bicyclic heteroaryl ring containing 8 to 10 ring atoms, for example, including (but not limited to): benzofuran, benzothiophene, indole, isoindole, quinoline, isoquinoline, indazole, benzothiazole, benzimidazole, quinazoline, quinoxaline, cinnoline, phthalazine, pyrido[3,2-*d*]pyrimidine, pyrido[2,3-*d*]pyrimidine, pyrido[3,4-*d*]pyrimidine, pyrido[4,3-*d*]pyrimidine, 1,8-naphthyridine, 1,7-naphthyridine, 1,6-naphthyridine, and 1,5-naphthyridine. In the "8- to 10-membered bicyclic heteroaryl ring" and "8- to 10-membered bicyclic heteroaryl", the heteroatoms are independently preferably nitrogen and/or oxygen, e.g., nitrogen; the number of the heteroatom is preferably 1, 2, or 3.

The term "spirocycloalkyl" refers to a 5- to 20-membered polycyclic group in which monocyclic rings share one carbon atom (referred to as the spiro atom). It may contain one or more double bonds, but none of the rings has a fully conjugated π-electron system. It is preferably 6- to 14-membered, and more preferably 7- to 14-membered. According to the number of spiro atoms shared among the rings, spirocycloalkyl may be monospirocycloalkyl, bispirocycloalkyl, or polyspirocycloalkyl. Monospirocycloalkyl and bispirocycloalkyl are preferred. 4-membered/4-membered, 4-membered/5-membered, 4-membered/6-membered, 5-membered/5-membered or 5-membered/6-membered monospirocycloalkyl is more preferred. Non-limiting examples of spirocycloalkyl include:

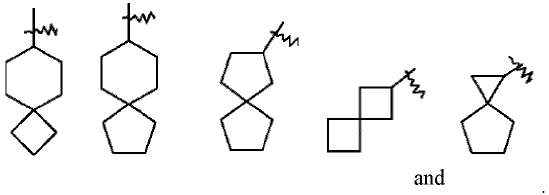

"Fused cycloalkyl" refers to a 5- to 20-membered all-carbon polycyclic group in which each ring in the system shares a pair of adjacent carbon atoms with other rings in the system, wherein one or more of the rings may contain one or more double bonds, but none of the rings has a fully conjugated π-electron system. It is preferably 6- to 14-membered, and more preferably 7- to 14-membered. According to the number of the formed rings, the fused cycloalkyl may include bicyclic, tricyclic, tetracyclic, or polycyclic fused cycloalkyl, and is preferably bicyclic. Non-limiting examples of fused cycloalkyl include:

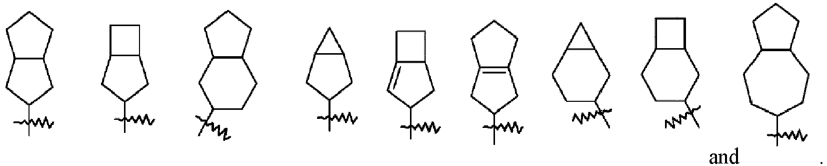

"Bridged cycloalkyl" refers to a 5- to 20-membered all-carbon polycyclic group in which any two rings share two carbon atoms that are not directly connected to each other. It may contain one or more double bonds, but none of the rings has a fully conjugated π-electron system. It is preferably 6- to 14-membered, more preferably 7- to 14-membered, and most preferably 7- to 9-membered. According to the number of the formed rings, the bridged cycloalkyl may include bicyclic, tricyclic, tetracyclic, or polycyclic, and is preferably bicyclic, tricyclic, or tetracyclic, and more preferably bicyclic or tricyclic. Non-limiting examples of bridged cycloalkyl include:

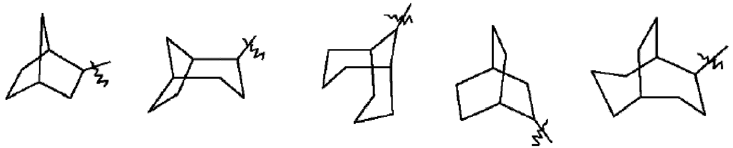

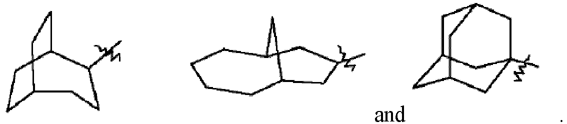

"Spiroheterocyclyl" refers to a 5- to 20-membered polycyclic heterocyclyl group in which monocyclic rings share one atom (referred to as the spiro atom), wherein one or more of the ring atoms are heteroatoms selected from nitrogen, oxygen, and S(O)_{g} (where g is an integer of 0 to 2), and the remaining ring atoms are carbon atoms. It may contain one or more double bonds, but none of the rings has a fully conjugated π-electron system. In the "spiroheterocyclyl", the heteroatoms are independently preferably nitrogen and/or oxygen, e.g., nitrogen; the number of the heteroatom is preferably 1, 2, or 3.

According to the number of spiro atoms shared among the rings, spiroheterocyclyl may include monospiroheterocyclyl, bispiroheterocyclyl, or polyspiroheterocyclyl. Monospiroheterocyclyl and bispiroheterocyclyl are preferred. 4-membered/4-membered, 4-membered/5-membered, 4-membered/6-membered, 5-membered/5-membered, 5-membered/6-membered, or 6-membered/6-membered monospiroheterocyclyl or bispiroheterocyclyl is more preferred. Non-limiting examples of spiroheterocyclyl include:

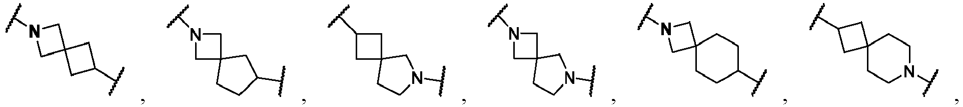

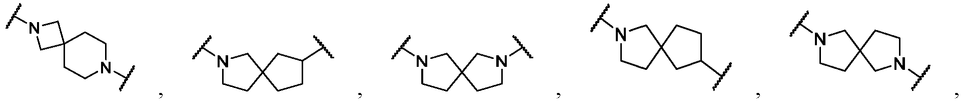

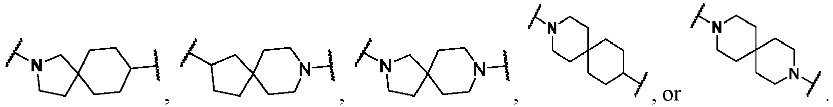

The term "fused heterocyclyl" refers to a 5- to 20-membered polycyclic heterocyclyl group in which each ring shares a pair of adjacent atoms with the other rings in the system, wherein one or more of the rings may contain one or more double bonds, but none of the rings has a fully conjugated π-electron system, and one or more of the ring atoms are heteroatoms selected from nitrogen, oxygen, and S(O)g (where g is an integer of 0 to 2), and the remaining ring atoms are carbon atoms. 7- to 14-membered fused heterocyclyl is preferred, and 7- to 10-membered (e.g., 7-, 8- or 9-membered) fused heterocyclyl is more preferred. According to the number of the formed rings, fused heterocyclyl may include bicyclic, tricyclic, tetracyclic, or polycyclic fused heterocyclyl, and is preferably bicyclic or tricyclic, and more preferably 5-membered/5-membered or 5-membered/6-membered bicyclic fused heterocyclyl. In the "fused heterocyclyl", the heteroatoms are independently preferably nitrogen and/or oxygen, e.g., nitrogen; the number of the heteroatom is preferably 1, 2, or 3.

Unless otherwise defined, the "substituents each independently selected from ..." as used herein means that when one or more hydrogen atoms on a group are substituted with substituents, the substituents may be of the same or different types, and the substituents from which the selection is made are of their respective independent types. As used herein, it should be noted that the "C₃₋₈ cycloalkylene", "3- to 8-membered heterocycloalkylene", "monocyclic alkylene", "monocyclic heterocyclylene", "polycyclic alkylene", "polycyclic heterocyclylene", "5- to 6-membered heteroarylene", "8- to 10-membered bicyclic heteroarylene", "mono-spiroheterocyclylene", and the like refer to divalent groups formed by removing another hydrogen from the above-mentioned "C₃₋₈ cycloalkyl", "3- to 8-membered heterocycloalkyl", "monocycloalkyl", "monocyclic heterocyclyl", "polycycloalkyl", "polycyclic heterocyclyl", "5- to 6-membered heteroaryl", "8-to 10-membered bicyclic heteroaryl", "monospiroheterocyclyl", and the like, respectively, and may be substituted or unsubstituted.

"Oxetanyl": 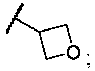 "acetyl": 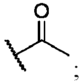 methylsulfonyl: 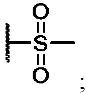 "amino": -NH₂;
"cyano": 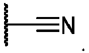 Alkyl, alkylene, alkenyl, alkynyl, cycloalkyl, cycloalkylene, heterocycloalkyl, heterocycloalkylene, aryl, arylene, heteroaryl, heteroarylene, and the like, as defined herein, are optionally substituted groups.

Exemplary substituents on carbon atoms include, but are not limited to: halogen, -CN, -NOz, -N₃, -SOzH, - SO₃H, -OH, -OR^{aa}, -ON(R^{bb})₂, -N(R^{bb})₂, -N(R^{bb})₃⁺X⁻, -N(OR^{cc})R^{bb}, -SH, -SR^{aa}, -SSR^{cc}, -C(=O)R^{aa}, -COzH, - CHO, -C(OR^{cc})₂, -CO₂R^{aa}, -OC(=O)R^{aa}, -OCO₂R^{aa}, -C(=O)N(R^{bb})₂, -OC(=O)N(R^{bb})₂, -NR^{bb}C(=O)R^{aa},-NR^{bb}CO₂R^{aa}, -NR^{bb}C(=O)N(R^{bb})₂, -C(=NR^{bb})R^{aa}, -C(=NR^{bb})OR^{aa}, -OC(=NR^{bb})R^{aa}, -OC(=NR^{bb})OR^{aa},-C(=NR^{bb})N(R^{bb})₂, -OC(=NR^{bb})N(R^{bb})₂, -NR^{bb}C(=NR^{bb})N(R^{bb})₂, -C(=O)NR^{bb}SO₂R^{aa}, -NR^{bb}SO₂R^{aa}, - SO₂N(R^{bb})₂, -SO₂R^{aa}, -SO₂OR^{aa}, -OSO₂R^{aa}, -S(=O)R^{aa}, -OS(=O)R^{aa}, -Si(R^{aa})₃, -OSi(R^{aa})₃, -C(=S)N(R^{bb})₂, - C(=O)SR^{aa}, -C(=S)SR^{aa}, -SC(=S)SR^{aa}, -SC(=O)SR^{aa}, -OC(=O)SR^{aa}, -SC(=O)OR^{aa}, -SC(=O)R^{aa}, -P(=O)₂R^{aa}, -OP(=O)₂R^{aa}, -P(=O)(R^{aa})₂, -OP(=O)(R^{aa})₂, -OP(=O)(OR^{cc})₂, -P(=O)₂N(R^{bb})₂, -OP(=O)₂N(R^{bb})₂, - P(=O)(NR^{bb})₂, -OP(=O)(NR^{bb})₂, -NR^{bb}P(=O)(OR^{cc})₂, -NR^{bb}P(=O)(NR^{bb})₂, -P(R^{cc})₂, -P(R^{cc})₃, -OP(R^{cc})₂,-OP(R^{cc})₃, -B(R^{aa})₂, -B(OR^{cc})₂, -BR^{aa}(OR^{cc}), alkyl, haloalkyl, alkenyl, alkynyl, cycloalkyl, heterocyclyl, aryl, and heteroaryl, wherein each alkyl, alkenyl, alkynyl, cycloalkyl, heterocyclyl, aryl, and heteroaryl is independently substituted with 0, 1, 2, 3, 4, or 5 R^{dd} groups;
or two geminal hydrogens on a carbon atom are replaced with the group =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb}, or =NOR^{cc};
each R^{aa} is independently selected from alkyl, haloalkyl, alkenyl, alkynyl, cycloalkyl, heterocyclyl, aryl, and heteroaryl, or two R^{aa} groups are joined to form a heterocyclyl or heteroaryl ring, wherein each alkyl, alkenyl, alkynyl, cycloalkyl, heterocyclyl, aryl, and heteroaryl is independently substituted with 0, 1, 2, 3, 4, or 5 R^{dd} groups;
each R^{bb} is independently selected from: hydrogen, -OH, -OR^{aa}, -N(R^{cc})₂, -CN, -C(=O)R^{aa}, -C(=O)N(R^{cc})₂, - CO₂R^{aa}, -SO₂R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa},-C(=S)N(R^{cc})₂, -C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, alkyl, haloalkyl, alkenyl, alkynyl, cycloalkyl, heterocyclyl, aryl, and heteroaryl, or two R^{bb} groups are joined to form a heterocyclyl or heteroaryl ring, wherein each alkyl, alkenyl, alkynyl, cycloalkyl, heterocyclyl, aryl, and heteroaryl is independently substituted with 0, 1, 2, 3, 4, or 5 R^{dd} groups;
each R^{cc} is independently selected from hydrogen, alkyl, haloalkyl, alkenyl, alkynyl, cycloalkyl, heterocyclyl, aryl, and heteroaryl, or two R^{cc} groups are joined to form a heterocyclyl or heteroaryl ring, wherein each alkyl, alkenyl, alkynyl, cycloalkyl, heterocyclyl, aryl, and heteroaryl is independently substituted with 0, 1, 2, 3, 4, or 5 R^{dd} groups;
each R^{dd} is independently selected from: halogen, -CN, -NOz, -N₃, -SO₂H, -SO₃H, -OH, -OR^{ee}, -ON(R^{ff})₂, - N(R^{ff})₂, -N(R^{ff})₃⁺X⁻, -N(OR^{ee})R^{ff}, -SH, -SR^{ee}, -SSR^{ee}, -C(=O)R^{ee}, -CO₂H, -CO₂R^{ee}, -OC(=O)R^{ee}, -OCO₂R^{ee}, - C(=O)N(R^{ff})₂, -OC(=O)N(R^{ff})₂, -NR^{ff}C(=O)R^{ee}, -NR^{ff}CO₂R^{ee}, -NR^{ff}C(=O)N(R^{ff})₂, -C(=NR^{ff})OR^{ee}, - OC(=NR^{ff})R^{ee}, -OC(=NR^{ff})OR^{ee}, -C(=NR^{ff})N(R^{ff})₂, -OC(=NR^{ff})N(R^{ff})₂, -NR^{ff}C(=NR^{ff})N(R^{ff})₂, -NR^{ff}SO₂R^{ee}, -SO₂N(R^{ff})₂, -SO₂R^{ee}, -SO₂OR^{ee}, -OSO₂R^{ee}, -S(=O)R^{ee}, -Si(R^{ee})₃, -OSi(R^{ee})₃, -C(=S)N(R^{ff})₂, -C(=O)SR^{ee}, - C(=S)SR^{ee}, -SC(=S)SR^{ee}, -P(=O)₂R^{ee}, -P(=O)(R^{ee})₂, -OP(=O)(R^{ee})₂, -OP(=O)(OR^{ee})₂, alkyl, haloalkyl, alkenyl, alkynyl, cycloalkyl, heterocyclyl, aryl, and heteroaryl, wherein each alkyl, alkenyl, alkynyl, cycloalkyl, heterocyclyl, aryl, and heteroaryl is independently substituted with 0, 1, 2, 3, 4, or 5 R^{gg} groups, or two geminal R^{dd} substituents may be joined to form =O or =S;
each R^{ee} is independently selected from alkyl, haloalkyl, alkenyl, alkynyl, cycloalkyl, aryl, heterocyclyl, and heteroaryl;
exemplary substituents on nitrogen atoms include, but are not limited to: hydrogen, -OH, -OR^{aa}, -N(R^{cc})₂, - CN, -C(=O)R^{aa}, -C(=O)N(R^{cc})₂, -CO₂R^{aa}, -SO₂R^{aa}, -C(=NR^{bb})R^{aa}, -C(=NR^{cc})OR^{aa}, -C(=NR^{cc})N(R^{cc})₂, - SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, alkyl, haloalkyl, alkenyl, alkynyl, cycloalkyl, heterocyclyl, aryl, and heteroaryl, or two R^{cc} groups attached to a nitrogen atom are joined to form a heterocyclyl or heteroaryl ring, wherein each alkyl, alkenyl, alkynyl, cycloalkyl, heterocyclyl, aryl, and heteroaryl is independently substituted with 0, 1, 2, 3, 4, or 5 R^{dd} groups, and wherein R^{aa}, R^{bb}, R^{cc}, and R^{dd} are as described above.

### Other definitions

The term "treat" or "treating" used herein relates to reversing, alleviating, or inhibiting the progression of the disorders or conditions to which the term applies, or one or more symptoms of such disorders or conditions. The noun "treatment" used herein relates to the action of the verb "treat", the latter being as just defined. The term "pharmaceutically acceptable salt" used herein denotes those carboxylate salts and amino acid addition salts of the compounds of the present invention, which are, within the scope of sound medical judgment, suitable for use in contact with the tissues of a patient without undue toxicity, irritation, allergic response, and the like. They are commensurate with a reasonable benefit/risk ratio, and are effective for their intended use. The term includes, if possible, the zwitterionic forms of the compounds of the present invention. The salts may be prepared from inorganic acids, which include sulfate, pyrosulfate, bisulfate, sulfite, bisulfite, nitrate, and the like, and representative salts include: hydrobromide, hydrochloride, sulfate, bisulfate, acetate, oxalate, palmitate, benzoate, lactate, phosphate, tosylate, citrate, maleate, fumarate, succinate, tartrate, mesylate, and the like. The salts may also be prepared from organic acids, which include acetate, propionate, octanoate, isobutyrate, oxalate, and the like. Also included are cations based on alkali metals and alkaline earth metals, such as sodium, lithium, potassium, calcium, magnesium, and the like, as well as non-toxic ammonium, quaternary ammonium, and amine cations, including, but not limited to, ammonium, tetramethylammonium, tetraethylammonium, methylamine, dimethylamine, trimethylamine, triethylamine, ethylamine, and the like. The "subject" to which the compound is administered includes, but is not limited to: a human (i.e., a male or female of any age group, e.g., a pediatric subject (e.g., an infant, a child, or an adolescent) or an adult subject (e.g., a young adult, a middle-aged adult, or an older adult)) and/or a non-human animal, e.g., a mammal, such as a primate (e.g., a cynomolgus monkey or a rhesus monkey), a cow, a pig, a horse, a sheep, a goat, a rodent, a cat, and/or a dog. In some embodiments, the subject is a human. In some embodiments, the subject is a non-human animal. The terms "human", "patient", and "subject" are used interchangeably herein.

The terms "disease", "disorder", and "condition" are used interchangeably herein.

As used herein, unless otherwise specified, the term "treatment" includes the effect on a subject who is suffering from a particular disease, disorder or condition, which reduces the severity of the disease, disorder or condition, or delays or slows the progression of the disease, disorder or condition ("therapeutic treatment"). The term also includes the effect that occurs before the subject begins to suffer from a particular disease, disorder or condition ("prophylactic treatment").

Generally, the "effective amount" of a compound refers to an amount sufficient to elicit a target biological response. As will be appreciated by those of ordinary skill in the art, the effective amount of the compound of the present invention may vary depending on, for example, the following factors: the biological target, the pharmacokinetics of the compound, the disease being treated, the mode of administration, and the age, health status and symptoms of the subject. The effective amount includes a therapeutically effective amount and a prophylactically effective amount.

The present invention also includes isotopically-labeled compounds (isotopic variants) which are equivalent to those described in formula (I), except that one or more atoms are replaced by an atom having an atomic mass or mass number different from the atomic mass or mass number usually found in nature. Examples of isotopes that can be incorporated into the compounds of the present invention include isotopes of hydrogen, carbon, nitrogen, oxygen, phosphorus, sulfur, fluorine, and chlorine, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl, respectively. The compounds of the present invention containing the above isotopes and/or other isotopes of other atoms, prodrugs thereof, and pharmaceutically acceptable salts of the compounds or prodrugs are all within the scope of the present invention. Certain isotopically-labeled compounds of the present invention, such as those into which radioisotope (e.g., ³H and ¹⁴C) are incorporated, are useful in drug and/or substrate tissue distribution assays. Tritium (i.e., ³H) and carbon-14 (i.e., ¹⁴C) isotopes are particularly preferred for their ease of preparation and detectability. Furthermore, substitution with heavier isotopes such as deuterium (i.e., ²H) may afford therapeutic benefits (e.g., increased *in vivo* half-life or reduced dose) resulting from greater metabolic stability and hence may be preferred in some circumstances. Isotopically labeled compounds of formula (I) of the present invention and prodrugs thereof can generally be prepared by using readily available isotopically-labeled reagents to replace non-isotopically-labeled reagents in the following schemes and/or the procedures disclosed in the examples and preparation examples.

The positive and progressive effects of the present invention are as follows: the compounds of the present invention have good inhibitory effects in the proliferation activity test of BaF3 cells with GFR mutants. The compounds of the present invention have good selectivity in the proliferation activity test of BaF3 cells with GFR mutants.

### DETAILED DESCRIPTION

### Preparation of intermediates

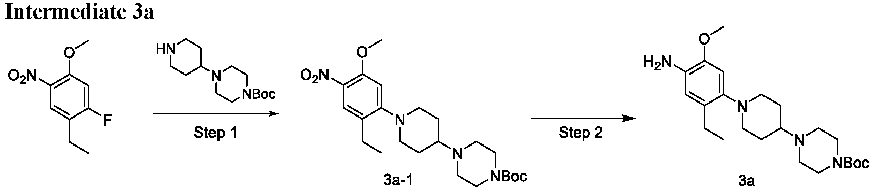

Step 1: Compound 1-ethyl-2-fluoro-4-methoxy-5-nitrobenzene (2 g, 10 mmol) and *tert*-butyl 4-(piperidin-4-yl)piperazine-1-carboxylate (4 g, 15 mmol) were added to a reaction flask before DMF (100 mL) and K₂CO₃ (4.1 g, 30 mmol) were added, and the system was stirred at 110 °C for 24 h. The reaction solution was concentrated, and then water was added. The mixture was extracted with dichloromethane and washed with water. The organic phase was concentrated and separated and purified by normal-phase column chromatography (dichloromethane:methanol = 10:1) to give compound 3a-1. MS m/z (ESI): 449.5 [M+H]⁺.

Step 2: Compound 3a-1 (2.3 g, 5.13 mmol) was added to a reaction flask before a mixture of MeOH:EA = 1:1 (150 mL) was added. Then Pd/C (1.14 g, 1.07 mmol) was added, and then the system was stirred at room temperature for 2 h in a H₂ atmosphere, filtered, and concentrated to give compound 3a. MS m/z (ESI): 419.9 [M+H]⁺.

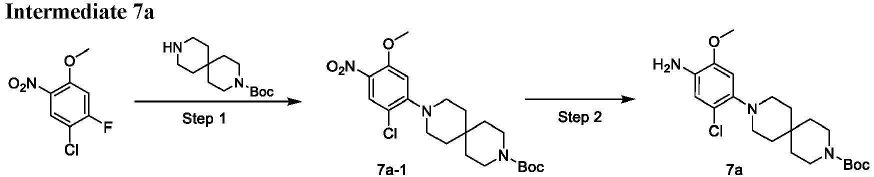

Step 1: Compound 1-chloro-2-fluoro-4-methoxy-5-nitrobenzene (0.5 g, 2.43 mmol) and compound *tert*-butyl 3,9-diazaspiro[5.5]undecane-3-carboxylate (680 mg, 2.68 mmol) were dissolved in DMF (10 mL). K₂CO₃ (672 mg, 4.86 mmol) was added with stirring, and the reaction mixture was stirred at 60 °C for 2 h. After the reaction was completed, the reaction mixture was cooled to room temperature and poured into ice water, and a solid was precipitated. The solid was then collected by filtration and dried under vacuum to give compound 7a-1, which was directly used in the next step. MS m/z (ESI): 440.4 [M+H]⁺.

Step 2: Compound 7a-1 (1 g, 2.27 mmol) was added to a mixed solution of ethanol (30 mL) and water (10 mL), and then iron powder (762 mg, 13.64 mmol) and ammonium chloride (1.22 g, 22.73 mmol) were added. In a nitrogen atmosphere, the reaction system was stirred at 90 °C for 2 h. After the reaction was completed, the reaction solution was cooled to 25 °C and filtered through celite. The filtrate was concentrated at reduced pressure to remove the ethanol, and then the aqueous phase was extracted with EA. The organic layers were combined, dried over anhydrous sodium sulfate, and then filtered. The filtrate was concentrated to give compound 7a, which was directly used in the next step. MS m/z (ESI): 411.2 [M+H]⁺.

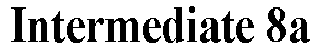

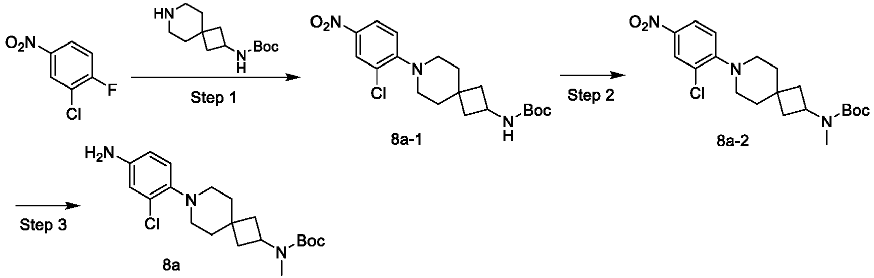

Step 1: 2-Chloro-1-fluoro-4-nitrobenzene (0.76 g, 4.33 mmol) was added to a solution of acetonitrile (10 mL), and then *tert*-butyl 7-azaspiro[3.5]nonan-2-ylcarbamate (1.04 g, 4.34 mmol) and *N*,*N*-diisopropylethylatnine (1.4 g, 10.82 mmol, 1.89 mL) were added. The reaction system was stirred in a nitrogen atmosphere at 85 °C for 5 h. After the reaction was completed, the reaction solution was directly concentrated at reduced pressure to remove the acetonitrile, diluted with water, and then extracted with ethyl acetate. The organic layers were combined, dried over anhydrous sodium sulfate, filtered, and concentrated to give compound 8a-1, which was directly used in the next step. MS m/z (ESI): 396.0 [M+H]⁺.

Step 2: Compound 8a-1 (1.6 g, 4.04 mmol, 1 eq) was added to a solution of tetrahydrofuran (16 mL), and the system was cooled to 0 °C. Sodium hydride (0.64 g, 16.16 mmol, 60% purity) was added to the reaction system, and then the reaction system was stirred in a nitrogen atmosphere at 0-25 °C for 1 h. Methyl iodide (40.42 mmol, 2.51 mL) was added, and then the reaction system was stirred for another 1 h. After the reaction was completed, a saturated aqueous ammonium chloride solution was added, and the mixture was extracted with dichloromethane. The organic layers were combined, dried over anhydrous sodium sulfate, and then filtered. The filtrate was concentrated to give compound 8a-2 as a yellow solid. MS m/z (ESI): 410.2 [M+H]⁺. Step 3: Compound 8a-2 (900 mg, 2.2 mmol) was added to a mixed solution of ethanol (18 mL) and water (18 mL), and then iron powder (306.54 mg, 5.49 mmol) and ammonium chloride (234.89 mg, 4.39 mmol) were added. In a nitrogen atmosphere, the reaction system was stirred at 80 °C for 1 h. After the reaction was completed, the reaction solution was cooled to 25 °C and filtered through celite. The filter cake was washed twice with ethanol, and the filtrate was collected, concentrated at reduced pressure to remove the ethanol, and extracted with ethyl acetate. The organic layers were combined, dried over anhydrous sodium sulfate, and then filtered. The filtrate was concentrated to give compound 8a, which was directly used in the next step. MS m/z (ESI): 380.2 [M+H]⁺.

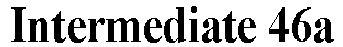

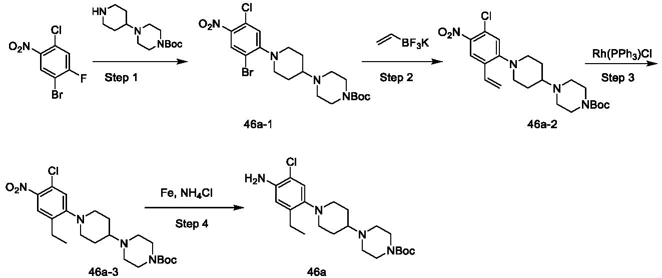

Step 1: Compound 46a-1 was prepared with reference to step 1 for intermediate 8a. MS m/z (ESI): 503.2 [M+H]⁺.

Step 2: Compound 46a-2 was prepared with reference to the method for intermediate 5d. MS m/z (ESI): 451.3 [M+H]⁺.

Step 3: Compound 46a-2 (500 mg, 1.11 mmol) was added to a reaction flask before a mixture of MeOH:THF = 1: 1 (20 mL) was added. Then Rh(PPh₃)Cl (0.1 g, 0.11 mmol) was added, and the system was stirred at room temperature for 2 h in a H₂ atmosphere, filtered, and concentrated to give compound 46a-3 (brown solid, 400 mg). MS m/z (ESI): 453.3 [M+H]⁺.

Step 4: Compound 46a was prepared with reference to step 2 for intermediate 7a. MS m/z (ESI): 423.2 [M+H]⁺.

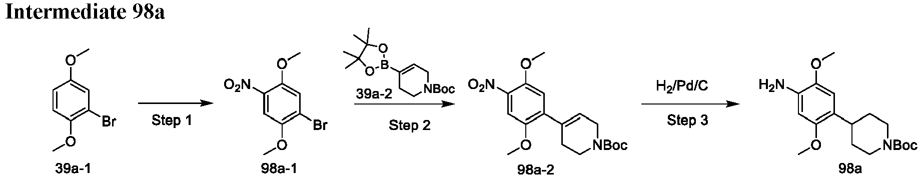

Step 1: Compound 39a-1 (1 g, 4.6 mmol) was dissolved in HOAc (6 mL). In an ice bath, concentrated HNO₃ (1.4 g, 5.0 mmol) was slowly added. The system was then stirred at 0 °C for 2 h in a nitrogen atmosphere. After the reaction was completed, the reaction solution was poured into ice water, and a solid was precipitated. The solid was then collected by filtration to give 98a-1 (yellow solid, 800 mg). MS m/z (ESI): 262.0 [M+H]⁺. Step 2: Compound 98a-2 was prepared with reference to the method for intermediate 5d. MS m/z (ESI): 365.2 [M+H]⁺.

Step 3: Compound 98awas prepared with reference to step 2 for intermediate 3a. MS m/z (ESI): 337.4 [M+H]⁺.

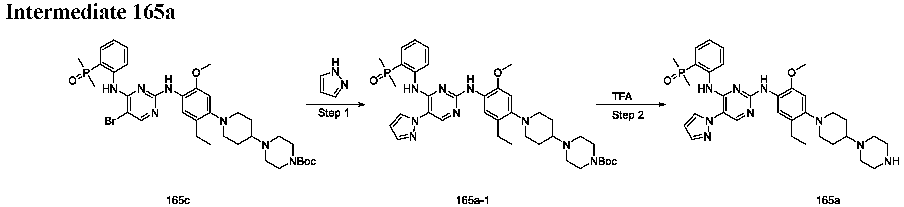

Step 1: Compound 165c (170 mg, 0.23 mmol), pyrazole (31 mg, 0.46 mmol), CuI (19 mg, 0.1 mmol), *N*1,*N*2-dimethylcyclohexane-1,2-diamine (14 mg, 0.1 mmol), Cs₂CO₃ (223 mg, 0.69 mmol), and DMF (6 mL) were added to a reaction flask. The system was purged with nitrogen for 1 min and microwaved at 150 °C for reaction for 1 h. After the reaction was completed, the reaction solution was filtered, concentrated, and subjected to normal-phase column chromatography (MeOH/DCM = 0-5%) to give compound 165a-1. MS m/z (ESI): 730.8 [M+H]⁺.

Step 2: Compound 165a-1 (60 mg, 0.08 mmol) and DCM (4 mL) were added to a reaction flask, and then TFA (2 mL) was added. The system was stirred at 20 °C for 1 h in a nitrogen atmosphere. After the reaction was completed, the reaction solution was concentrated, diluted with DCM, washed with saturated sodium bicarbonate until pH = 7-8, and then extracted with DCM. The organic phase was washed with saturated brine, dried over anhydrous sodium sulfate, and concentrated to give compound 165a. MS m/z (ESI):630.6 [M+H]⁺.

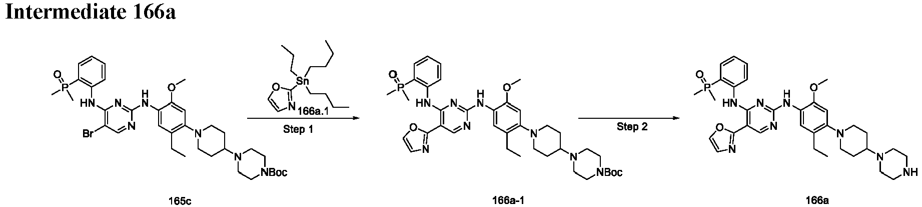

Step 1: Compound 165c (200 mg, 0.27 mmol), 166a. 1 (139 mg, 0.41 mmol), catalyst Pd(PPh₃)₄ (22.3 mg, 0.02 mmol), and 1,4-dioxane (6 mL) were added to a microwave tube. The system was purged with nitrogen for 1 min and microwaved at 150 °C for reaction for 1 h. After the reaction was completed, a saturated KF solution was added, and the mixture was stirred at room temperature for 0.5 h, subjected to liquid separation, and extracted with DCM. The organic phases were combined, dried over anhydrous sodium sulfate, filtered, concentrated, and then subjected to normal-phase column chromatography (MeOH/DCM = 0-5%) to give compound 166a-1 (yellow oil, 120 mg). MS m/z (ESI): 731.7 [M+H]⁺.

Step 2: Compound 166a was prepared with reference to step 2 for intermediate 3b. MS m/z (ESI):631.5 [M+H]⁺.

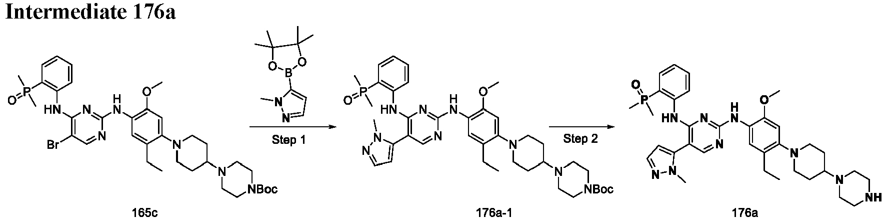

Step 1: Compound 165c (200 mg, 0.27 mmol), 1-methyl-5-(4,4,5,5-tetramethyl-1,3,2-dioxaborolan-2-yl)-1*H-*pyrazole (112 mg, 0.54 mmol), catalyst Pd(PPh₃)₄ (16 mg, 0.014 mmol), K₂CO₃ (112 mg, 0.81 mmol), 1,4-dioxane/H₂O (v/v = 6/1, 7 mL) were added to a microwave tube. The system was then purged with nitrogen for 1 min and microwaved at 130 °C for reaction for 1 h. After the reaction was completed, water was added for liquid separation, and the mixture was extracted with DCM. The organic phases were combined, dried over anhydrous sodium sulfate, filtered, concentrated, and then subjected to normal-phase column chromatography (DCM/MeOH = 0-5%) to give compound 176a-1. MS m/z (ESI): 744.8 [M+H]⁺.

Step 2: Compound 176a-1 (50 mg, 0.067 mmol) and solvent DCM (4 mL) were added to a reaction flask, and then TFA (2 mL) was added. The system was stirred at 20 °C for 1 h in a nitrogen atmosphere. After the reaction was completed, the reaction solution was concentrated, diluted with DCM, washed with saturated sodium bicarbonate until pH = 7-8, and then extracted with DCM. The organic phase was washed with saturated brine, dried over anhydrous sodium sulfate, and concentrated to give compound 176a. MS m/z (ESI): 644.5 [M+H]⁺.

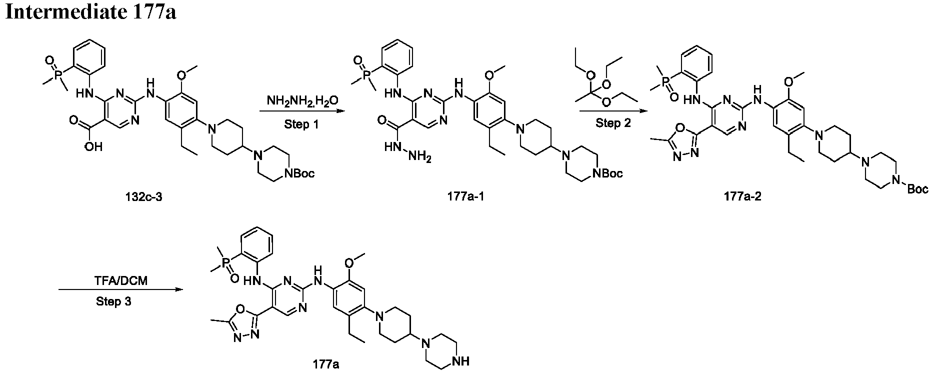

Step 1: Compound 132c-3 (200 mg, 0.28 mmol), hydrazine hydrate (33 mg, 0.56 mmol), and DCM (6 mL) were added to a three-necked flask. In a N₂ atmosphere and in an ice bath, CDI (48.6 mg, 0.3 mmol) was added, and the system was allowed to react at 20 °C for 1 h. After the reaction was completed, water was added, and the mixture was extracted with DCM. The organic phases were combined, dried over anhydrous sodium sulfate, filtered, concentrated, and then subjected to normal-phase column chromatography (DCM/MeOH = 0-5%) to give compound 177a-1 (yellow solid, 120 mg). MS m/z (ESI): 722.5 [M+H]⁺. Step 2: Compound 177a-1 (70 mg, 0.09 mmol), triethyl orthoacetate (73 mg, 0.45 mmol), and solvent NMP (3 mL) were added to a reaction flask, and the system was stirred at 150 °C for 1 h in an air atmosphere. After the reaction was completed, the reaction solution was concentrated and subjected to normal-phase column chromatography (DCM/MeOH = 0-5%) to give compound 177a-2 (yellow oil, 60 mg). MS m/z (ESI): 746.7 [M+H]⁺.

Step 3: Compound 177a was prepared with reference to step 2 for intermediate 3b. MS m/z (ESI): 646.5 [M+H]⁺.

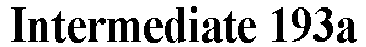

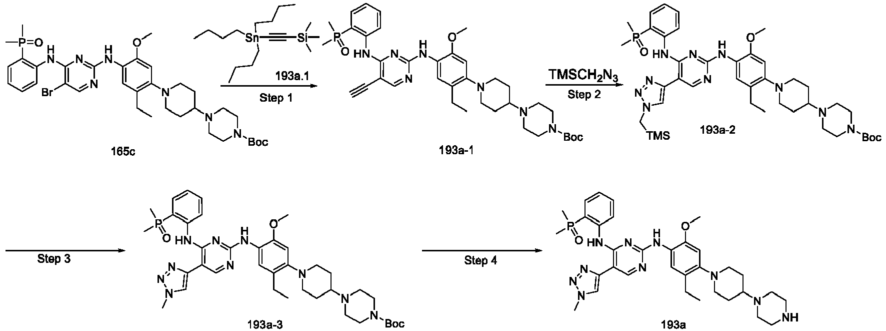

Step 1: Compound 165c (550 mg, 0.74 mmol), 193a.1 (431 mg, 1.11 mmol), catalyst Pd(PPh₃)₄ (45 mg, 0.04 mmol), and DMF (8 mL) were added to a microwave tube. The system was purged with nitrogen for 1 min and microwaved at 150 °C for reaction for 1 h. After the reaction was completed, a saturated KF solution was added, and the mixture was stirred at room temperature for 0.5 h, subjected to liquid separation, and extracted with DCM. The organic phases were combined, dried over anhydrous sodium sulfate, filtered, and then concentrated. K₂CO₃ and methanol were added, and the mixture was stirred at room temperature for 0.5 h, filtered, concentrated, and then subjected to normal-phase column chromatography (DCM/MeOH = 0-5%) to give compound 193a-1 (yellow solid, 230 mg). MS m/z (ESI): 688.5 [M+H]⁺.

Step 2: Compound 193a-1 (150 mg, 0.22 mmol), TMSN₃ (34 mg, 0.264 mmol), CuSO₄ (3 mg, 0.02 mmol), sodium ascorbate (4 mg, 0.02 mmol), and solvents DMF, water, and t-BuOH (3 mL, v/v/v =1/1/1) were added to a reaction flask. In an air atmosphere, the system was stirred at 20 °C for 2 h. After the reaction was completed, the reaction solution was filtered. DCM and water were added, and the mixture was subjected to liquid separation and extracted with DCM. The organic phase was washed with saturated brine, dried over anhydrous sodium sulfate, concentrated, and then subjected to normal-phase column chromatography (MeOH/DCM = 0-5%) to give compound 193a-2 (yellow solid, 130 mg). MS m/z (ESI):817.8. [M+H]⁺. Step 3: Compound 193a-2 (130 mg, 0.16 mmol), solvent THF (2 mL), and one drop of water were added to a reaction flask. In an ice bath, a 1.0 M solution of TBAF (1 mL, 1 mmol) in THF was dropwise added. In a nitrogen atmosphere, the system was stirred at room temperature for 1 h. After the reaction was completed, DCM and H₂O were added, and the mixture was subjected to liquid separation, extracted with DCM, dried, concentrated, and then subjected to normal-phase column chromatography (DCM/MeOH = 0-5%) to give compound 193a-3 (yellow oil, 80 mg). MS m/z (ESI): 746.1 [M+H]⁺.

Step 4: Compound 193a was prepared with reference to step 2 for 165a. MS m/z (ESI): 645.9 [M+H]⁺.

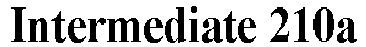

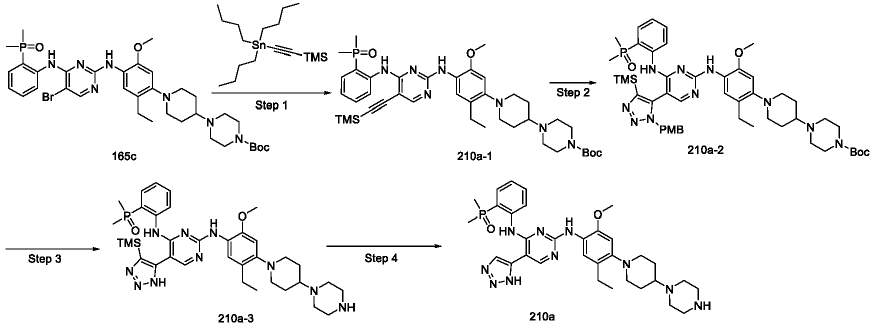

Step 1: Compound 165c (400 mg, 0.54 mmol), trimethyl((tributylstannyl)ethynyl)silane (313 mg, 0.81 mmol), and catalyst Pd(PPh₃)₄, and DMF (8 mL) were added to a 20 mL microwave tube. The system was then purged with nitrogen for 1 min and microwaved at 150 °C for reaction for 1 h. After the reaction was completed, a saturated KF solution was added, and the mixture was stirred at room temperature for half an hour, subjected to liquid separation, and extracted with DCM. The organic phases were combined, dried over anhydrous sodium sulfate, filtered, concentrated, and then subjected to normal-phase column chromatography (DCM/MeOH = 0-5%) to give compound 210a-1 (yellow solid, 250 mg). MS m/z (ESI):761.2. [M+H]⁺. Step 2: Compound 210a-1 (190 mg, 0.25 mmol), PMBN₃ (49 mg, 0.3 mmol), and toluene were added to a reaction flask. In an air atmosphere, the system was stirred at 130 °C for 16 h. After the reaction was completed, the reaction solution was concentrated and subjected to normal-phase column chromatography (MeOH/DCM = 0-5%) to give compound 210a-2 (yellow solid, 200 mg). MS m/z (ESI): 923.4 [M+H]⁺.

Step 3: Compound 210a-2 (200 mg, 0.22 mmol) and solvent DCM were added to a reaction flask. In an ice bath, TFA was dropwise added, and the system was stirred at 65 °C for 2 h. After the reaction was completed, the reaction solution was concentrated, diluted with DCM, washed with saturated sodium bicarbonate until pH = 7-8, and then extracted with DCM. The organic phase was washed with saturated brine, dried over anhydrous sodium sulfate, concentrated, and then separated and purified by prep-HPLC to give 210a-3 (pale yellow solid, 90 mg, hydrochloride). MS m/z (ESI): 704.1 [M+H]⁺.

Step 4: Compound 210a-3 (80 mg, 0.11 mmol), K₂CO₃ (45.5 mg, 0.33 mmol), and solvent MeOH were added to a reaction flask. In an air atmosphere, the system was stirred at 65 °C for 2 h. After the reaction was completed, the reaction solution was filtered, concentrated, and subjected to normal-phase column chromatography (DCM/MeOH = 0-20%) to give compound 210a. MS m/z (ESI): 631.8 [M+H]⁺.

### Preparation of intermediates of formula (a)

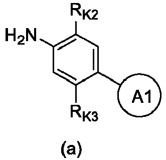

With reference to the method shown in any one of compounds 3a-46a, intermediate compounds of formula (a) were prepared from suitable substituted nitrobenzenes and amines as starting materials, as shown in Table 3 below.

**Table 3**

| Intermediate | Ring A1 | R_{K2} | R_{K3} | MS m/z (ESI) |
|---|---|---|---|---|
| 4a | 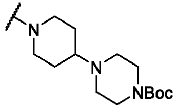 | H | -CH₂CH₃ | 389.6 [M+H]⁺ |
| 5a | 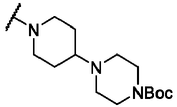 | -OMe | -Me | 405.3 [M+H]⁺ |
| 6a | 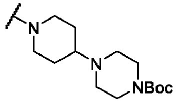 | -OMe | Cl | 426.3 [M+H]⁺ |
| 9a | 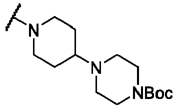 | -OMe | 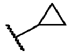 | 461.2 [M+H]⁺ |
| 10a | 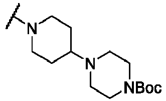 | H | Cl | 396.3 [M+H]⁺ |
| 11a | 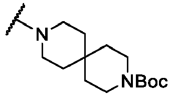 | -OMe | -Me | 390.3 [M+H]⁺ |
| 12a | 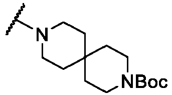 | -OMe | -CH₂CH₃ | 404.3 [M+H]⁺ |
| 13a | 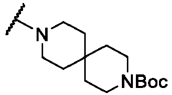 | -OMe | H | 376.3 [M+H]⁺ |
| 14a | 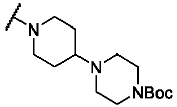 | -OMe | H | 391.3 [M+H]⁺ |
| 60a | 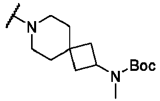 | -OMe | -CH₂CH₃ | 403.3 [M+H]⁺ |
| 140a | 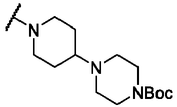 | -Me | -CH₂CH₃ | 403.3 [M+H]⁺ |
| 142a | 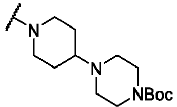 | - C(O)OCH₃ | -CH₂CH₃ | 447.3 [M+H]⁺ |
| 152a | 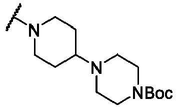 | -OCF₃ | -CH₂CH₃ | 473.3 [M+H]⁺ |

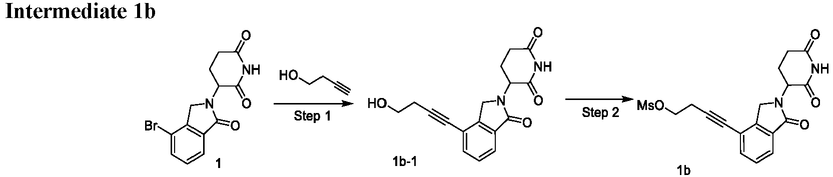

Step 1: Compound 1 (5.0 g, 15.5 mmol), but-3-yn-1-ol (3.25 g, 46.5 mmol), Pd(PPh₃)₂Cl₂ (0.22 g, 0.31 mmol), cuprous iodide (0.12 g, 0.62 mmol) were added to a reaction flask before anhydrous DMF (60 mL) and triethylamine (10 mL) were added. In a nitrogen atmosphere, the system was slowly heated to 90 °C and stirred for 16 h. After the reaction was completed, the reaction solution was filtered, and the filtrate was concentrated at reduced pressure to dryness. The residue was then triturated with ethyl acetate and petroleum ether, and the triturate was filtered at reduced pressure. The filter cake was dissolved in a mixed solution of dichloromethane and methanol, and the crude product was separated and purified by normal-phase column chromatography to give compound 1b-1 (pale yellow solid, 6.0 g). MS m/z (ESI): 313.2 [M+H]⁺.

Step 2: Compound 1b-1 (5.7 g, 18.2 mmol), triethylamine (9.2 g, 91 mmol), and anhydrous dichloromethane (1600 mL) were added in a reaction flask before methanesulfonyl chloride (6.0 g, 54.6 mmol) was slowly and dropwise added in an ice-water bath. After the dropwise addition, the system was stirred for 1 h in the ice-water bath. After the reaction was completed, water was added to quench the reaction. The mixture was extracted with dichloromethane, and the organic phases were combined, washed with saturated brine, and dried over anhydrous sodium sulfate. The solvent was removed at reduced pressure, and the crude product was triturated with petroleum ether and ethyl acetate. The triturate was filtered to give the target compound 1b (pale yellow solid, 4.5 g), which was used directly in the next step. MS m/z (ESI): 391.2 [M+H]⁺.

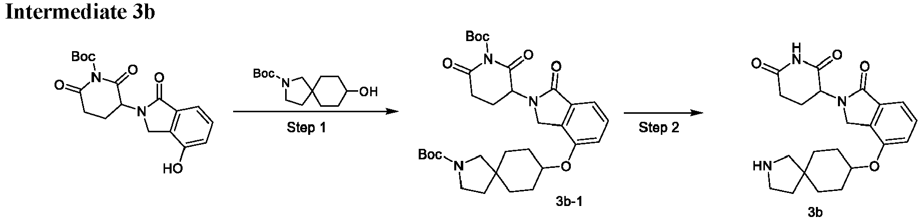

Step 1: At 25 °C, compound *tert*-butyl 3-(4-hydroxy-1-oxoisoindolin-2-yl)-2,6-dioxopiperidine-1-carboxylate (350 mg, 971.25 µmol) was added to a solution of *tert*-butyl 8-hydroxy-2-azaspiro[4.5]decane-2-carboxylate (248.01 mg, 971.25 µmol) in THF (10 mL), and then azodicarbonamide (83.62 mg, 485.62 µmol) and tributylphosphine (98.25 mg, 485.62 µmol, 119.82 µL) were slowly added in a nitrogen atmosphere. The reaction system was stirred at room temperature for 8 h. After the reaction was completed, the reaction solution was concentrated at reduced pressure to give a crude product, which was purified by column chromatography to give compound 3b-. MS m/z (ESI): 398.2 [M+H-200]⁺.

Step 2: At 25 °C, trifluoroacetic acid (2.70 mmol, 0.2 mL) was added to a solution of compound 3b-1 (100 mg, 167.31 µmol) in dichloromethane (1 mL), and then the reaction system was stirred for 1 h. The reaction solution was concentrated at reduced pressure to give compound 3b. The crude product was directly used in the next step. MS m/z (ESI): 398.2 [M+H]⁺.

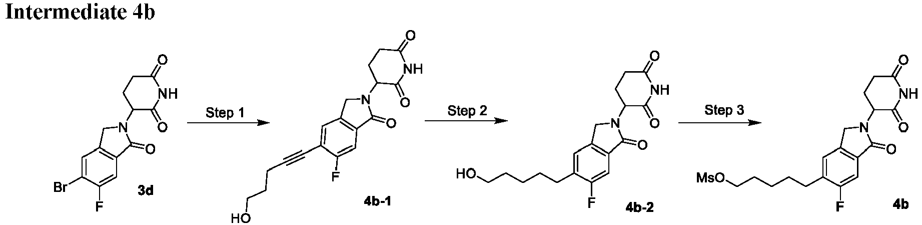

Step 1: Compound 3d (500 mg, 1.47 mmol) and compound pent-4-yn-1-ol (247 mg, 2.93 mmol) were dissolved in DMF (6 mL). Et₃N (1.06 mL, 7.33 mmol), CuI (56 mg, 0.29 mmol), and Pd(PPh₃)₂Cl₂ (103 mg, 0.15 mmol) were separately added with stirring. In a nitrogen atmosphere, the reaction system was stirred at 100 °C for 16 h. After the reaction was completed, the reaction solution was poured into ice water, and then the aqueous phase was extracted with ethyl acetate. The organic layer was washed with brine, dried over anhydrous sodium sulfate, filtered, and concentrated. The filter cake was dissolved and subjected to column chromatography (methanol/dichloromethane: 5%-10%) to give the title compound 4b-1 (white solid, 400 mg). MS m/z (ESI): 345.3 [M+H]⁺.

Step 2: Compound 4b-1 (200 mg, 0.58 mmol) was dissolved in EtOH/EA/MeOH (1:1:0.5, 25 mL). In a nitrogen atmosphere, wet Pd/C (40 mg) was added with stirring. In a hydrogen atmosphere, the system was stirred at 60 °C for 16 h. After the reaction was completed, the reaction system was filtered, and the filtrate was concentrated to give compound 4b-2 (white solid, 202 mg), which was directly used in the next step. MS m/z (ESI): 349.3 [M+H]⁺.

Step 3: Compound 4b-2 (200 mg, 0.574 mmol) was dissolved in a solution of DCM (10 mL) and THF (20 mL). Et₃N (0.5 mL, 3.44 mmol) and MsCl (197 mg, 0.14 mL, 1.72 mmol) were added with stirring, and the reaction system was stirred at room temperature for 2 h. After the reaction was completed, the reaction mixture was diluted with water to quench the reaction and then extracted with DCM. The organic layers were combined, washed with brine, dried over anhydrous sodium sulfate, filtered, and concentrated. The resulting solid was triturated with PE:EA = 10:1 (20 mL), and the triturate was filtered. The filter cake was dried under vacuum to give compound 4b. MS m/z (ESI): 427.2 [M+H]⁺.

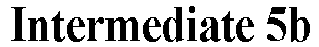

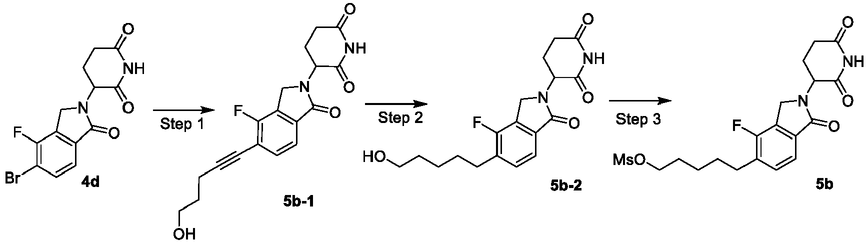

Compound 5b was prepared with reference to the method for intermediate 4b. MS m/z (ESI): 427.5 [M+H]⁺.

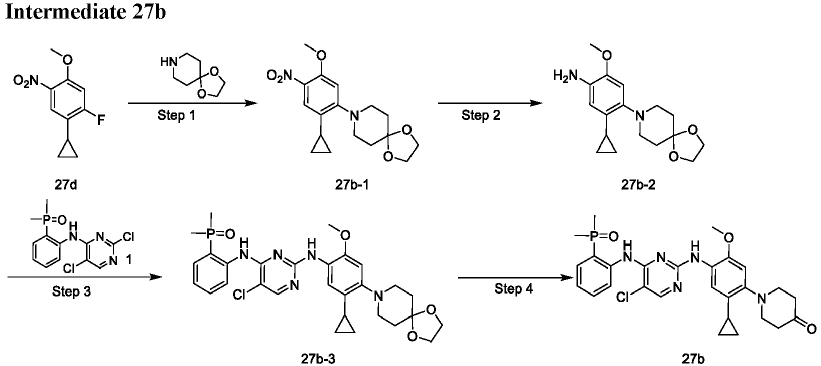

Compound 27b was prepared with reference to compound 21c. MS m/z (ESI): 540.3 [M+H]⁺.

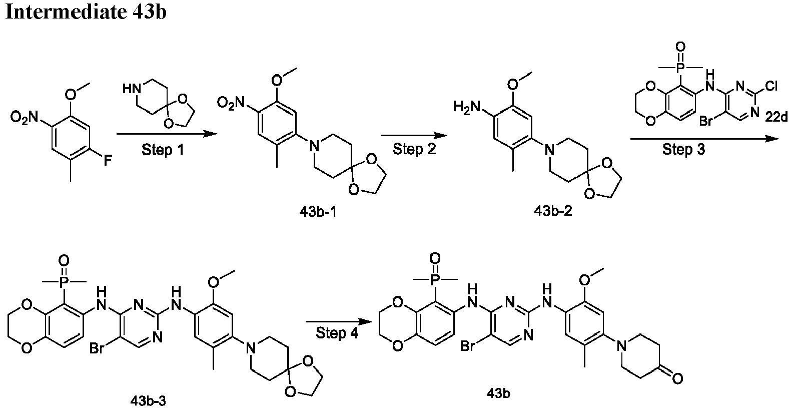

Compound 43b was prepared with reference to the method for intermediate 21c. M/S M/Z (ESI): 616.1, 618.1 [M+H]⁺.

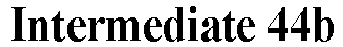

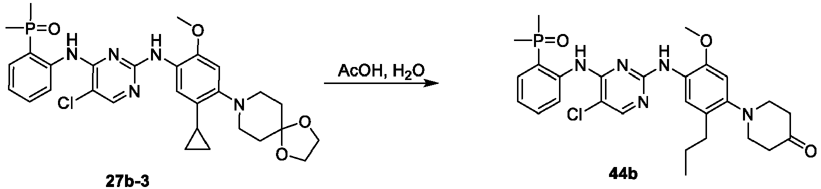

Compound 44b was prepared with reference to step 4 for intermediate 21c. MS m/z (ESI): 542.2 [M+H]⁺.

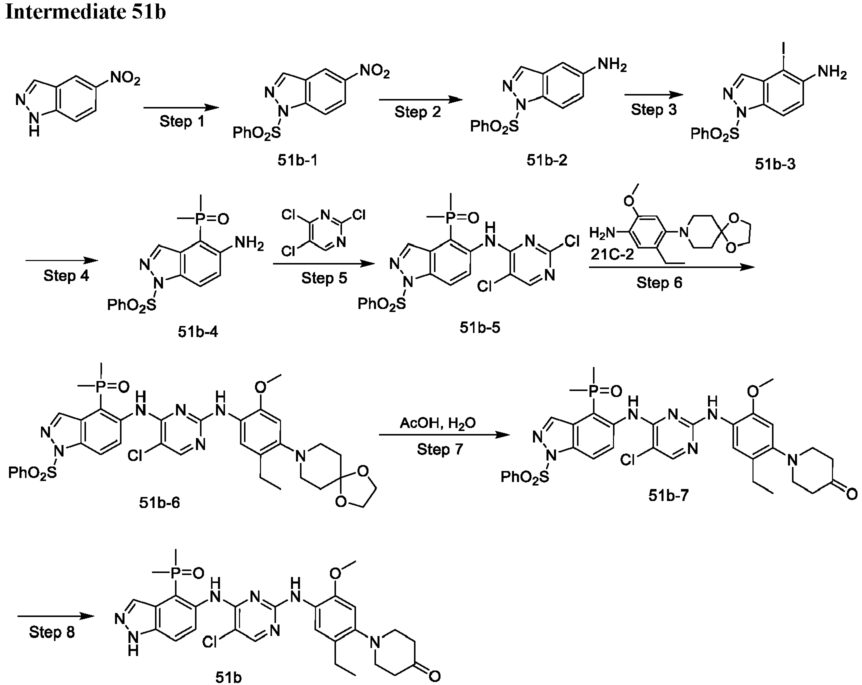

Step 1: Tetrabutylammonium hydrogen sulfate (100 mg, 294.52 µmol) and sodium hydride (1.84 g, 45.97 mmol, 60% purity) were added to a solution of 5-nitro-1*H*-indazole (3 g, 18.39 mmol) in anhydrous tetrahydrofuran (30 mL). The system was stirred at 25 °C for 1 h, and then benzenesulfonyl chloride (3.57 g, 20.23 mmol, 2.59 mL) was added. The reaction system was stirred at 25 °C for 11 h. After the reaction was completed, the reaction was quenched with an aqueous ammonium chloride solution at 0 °C. The reaction mixture was extracted with water and ethyl acetate and separated into layers. The organic layer was washed with brine, dried over anhydrous sodium sulfate, filtered, and concentrated at reduced pressure to give compound 51b-1 (brown solid, 3 g). MS m/z (ESI): 304.0 [M+H]⁺.

Step 2: Compound 51b-2 was prepared with reference to step 3 for 45c. MS m/z (ESI): 273.8 [M+H]⁺.

Step 3: NIS (1.98 g, 8.78 mmol) was added to a solution of 51b-2 (2 g, 7.32 mmol) in acetonitrile (8 mL), and then the reaction system was stirred at 25 °C for 1 h. After the reaction was completed, the reaction solution was directly filtered and concentrated at reduced pressure to give compound 51b-3. MS m/z (ESI): 399.8 [M+H]⁺.

Steps 4-5: Compound 51b-5 was prepared with reference to steps 1-2 for 12c. MS m/z (ESI): 495.8 [M+H]⁺. Steps 6-7: Compound 51b-7 was prepared with reference to steps 3-4 for 21c. MS m/z (ESI): 708.1 [M+H]⁺. Step 8: Sodium hydroxide (18.07 mg, 451.87 µmol) was added to a mixture of 51b-7 (160 mg, 225.94 µmol) in anhydrous tetrahydrofuran (5 mL) and water (1 mL), and then the system was stirred at 80 °C for 5 h. After the reaction was completed, the reaction mixture was directly concentrated at reduced pressure to give a crude product. The crude product was purified by normal-phase column chromatography to give compound 51b. MS m/z (ESI): 568.2 [M+H]⁺.

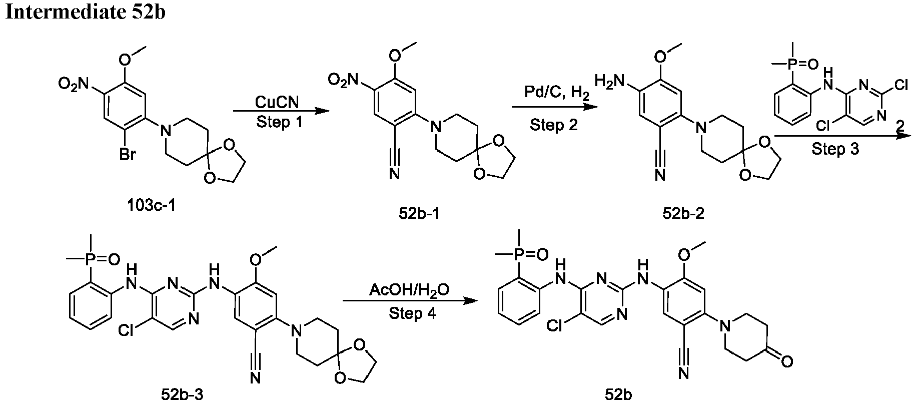

Step 1: 103c-1 (6.39 g, 17.12 mmol) and CuCN (7.67 g, 85.61 mmol, 18.70 mL) were added to DMF (50 mL), and the reaction system was stirred in an autoclave at 140 °C for 72 h. After the reaction was completed, the reaction mixture was diluted with water and then extracted with ethyl acetate. The organic layers were combined, dried over anhydrous sodium sulfate, filtered, concentrated, and separated and purified by column chromatography (PE/EA = 5/1-2/1) to give compound 52b-1 (yellow solid, 2.80 g). MS m/z (ESI): 320.1 [M+H]⁺.

Step 2: Compound 52b-2 was prepared with reference to step 3 for 45c. MS m/z (ESI): 290.2 [M+H]⁺.

Step 3: 52b-2 and 2 were added to a solution of tetrahydrofuran (25 mL), and then sodium *tert*-butoxide (1.46 g, 15.21 mmol) and tBuXPhos Pd G3 (604.02 mg, 760.38 µmol) were added. The reaction system was stirred at 65 °C for 12 h. After the reaction was completed, the reaction mixture was diluted with water and then extracted with dichloromethane. The organic layers were combined, dried over anhydrous sodium sulfate, filtered, and concentrated to give a crude product. The crude product was separated and purified by silica gel column chromatography (PE/EA = 100/1-25/1) to give compound 52b-3. MS m/z (ESI): 569.2 [M+H]⁺.

Step 4: Compound 52b was prepared with reference to step 4 for intermediate 21c. MS m/z (ESI): 525.0 [M+H]⁺.

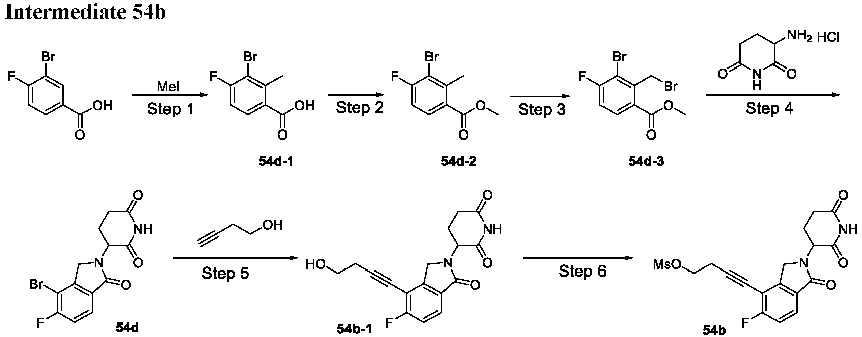

Step 1: 2,2,6,6-Tetramethylpiperidine (1.7 g, 12 mmol) and anhydrous THF (50 mL) were added in a 250 mL three-necked flask. In a nitrogen atmosphere, the reaction system was cooled to -45 °C, and *n*-butyllithium (4.8 mL, 12 mmol, 2.5 M) was slowly and dropwise added. After the dropwise addition, the system was stirred at -45 °C for 1 h. A solution of 3-bromo-4-fluorobenzoic acid (2.2 g, 10 mmol) in THF (10 mL) was then slowly and dropwise added. The system was then stirred at that temperature for another 5 h. Finally, CH₃I (0.79 mL, 12 mmol) was slowly and dropwise added, and the reaction system was allowed to gradually warm to room temperature and react at room temperature overnight. After the reaction was completed, at 0 °C, the reaction system was diluted with 3 N HCl to quench the reaction and then extracted with EA. The organic layers were combined, washed with brine, dried over anhydrous sodium sulfate, filtered, and concentrated. The crude product was purified by column chromatography (MeOH/DCM = 0-10%) to give compound 54d-1. MS m/z (ESI): 233.1 [M+H]⁺.

Step 2: Compound 54d-1 (1500 mg, 6.44 mmol) was dissolved in a single-necked flask with 20 mL of anhydrous MeOH. In an ice-water bath, SOCl₂ (920 mg, 1.29 mmol) was then slowly and dropwise added. After the dropwise addition, the system was slowly warmed to reflux and stirred for 2 h. After the reaction was completed, the system was cooled to room temperature. The reaction solution was concentrated at reduced pressure, and the crude product was separated by column chromatography (EA/PE = 0-5%) to give compound 54d-2. MS m/z (ESI): 247.1 [M+H]⁺

Steps 3-4: Compound 54d was prepared with reference to steps 2-3 for intermediate 3d. MS m/z (ESI): 341.2 [M+H]⁺.

Steps 5-6: Compound 54b was prepared with reference to steps 1-2 for intermediate 1b. MS m/z (ESI): 409.1 [M+H]⁺.

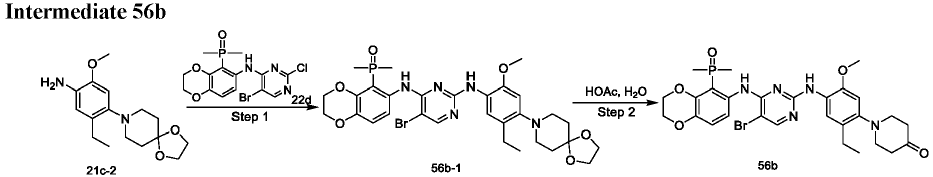

Compound 56b was prepared with reference to step 3 and step 4 for intermediate 21c. M/S M/Z (ESI): 630.2, 632.2 [M+H]⁺.

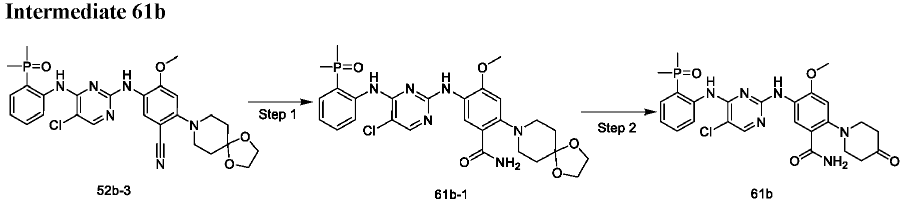

Step 1: 52b-3 (900 mg, 1.58 mmol) was added to a mixed solution of dimethyl sulfoxide (3 mL), ethanol (6 mL), and water (0.6 mL). An aqueous sodium hydroxide solution (2 M, 3.95 mL) and hydrogen peroxide (31.63 mmol, 3.04 mL, 30% purity) were added at 0 °C, and then the reaction system was stirred at 25 °C for 72 h. After the reaction was completed, sodium thiosulfate was added to the reaction mixture to quench the reaction, and then the mixture was extracted with dichloromethane. The organic layers were combined, dried over anhydrous sodium sulfate, filtered, and concentrated to give compound 61b-1. MS m/z (ESI): 587.1 [M+H]⁺.

Step 2: Compound 61b was prepared with reference to step 4 for intermediate 21c. MS m/z (ESI): 543.1 [M+H]⁺.

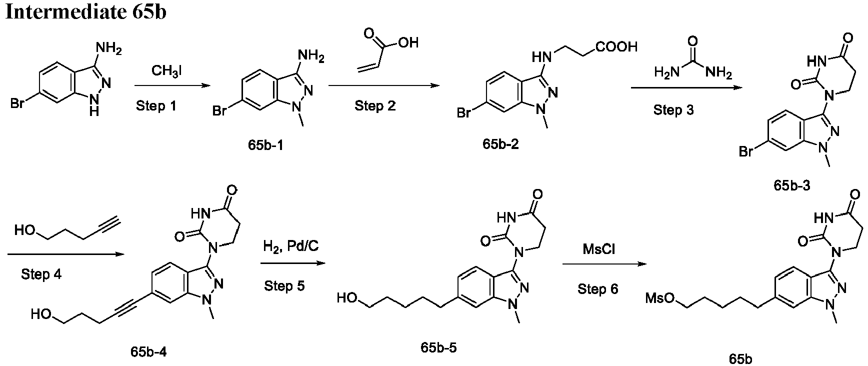

Step 1: Compound 6-bromo-1*H*-indazol-3-amine (1.0 g, 4.74 mmol) was dissolved in DMF. The system was cooled to 0 °C, and then NaH (284 mg, 7.1 mmol) was added. The system was allowed to react at 0 °C for 20 min, and then methyl iodide (1007 mg, 7.1 mmol) was added. The reaction system was allowed to react at 25 °C for 16 h. Water was added to the reaction solution, and the mixture was extracted with ethyl acetate. The organic phase was washed with brine, dried over anhydrous sodium sulfate, concentrated, and purified by column chromatography (MeOH/DCM = ~10%) to give compound 65b-1. MS m/z (ESI): 226.1 [M+H]⁺. Step 2: Compound 65b-1 (575 mg, 1.47 mmol) was dissolved in acetic acid (5 mL) and water (5 mL), and acrylic acid (212 mg, 2.94 mmol) was added. The system was heated to 105 °C for reaction for 4 h, then adjusted to pH 5 with a saturated aqueous sodium bicarbonate solution, and extracted with DCM. The organic phase was dried over anhydrous sodium sulfate, concentrated, and purified by column chromatography (MeOH/DCM = ∼10%) to give 65b-2. MS m/z (ESI): 299.1 [M+H]⁺.

Step 3: Compound 65b-2 (290 mg, 0.97 mmol) was dissolved in acetic acid (5 mL), and urea (30.5 mg, 1.95 mmol) was added. The system was heated to 120 °C for reaction for 4 h, and then concentrated hydrochloric acid was added. The reaction system was heated to reflux for 30 min. The solvent was removed by rotary evaporation, and a saturated aqueous sodium bicarbonate solution was added to adjust the pH to 7. The mixture was extracted with DCM, and the organic phase was dried over anhydrous sodium sulfate, concentrated, and purified by column chromatography (MeOH/DCM = ∼10%) to give compound 65b-3. MS m/z (ESI): 323.1 [M+H]⁺.

Steps 4-6: Compound 65b was prepared with reference to steps 1-3 for intermediate 4b. MS m/z (ESI): 409.2 [M+H]⁺.

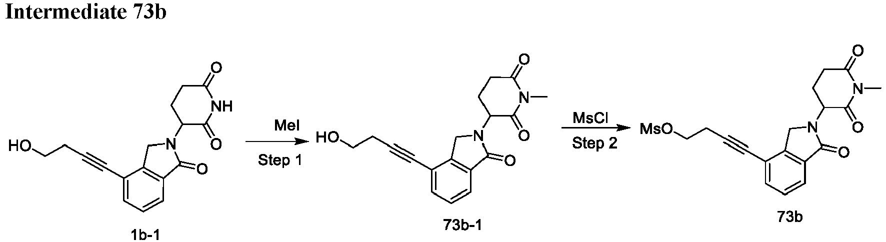

Compound 73b was prepared with reference to step 1 for compound 3d as step 1 and with reference to step 2 for compound 1b as step 2. MS m/z (ESI): 405.1 [M+H]⁺.

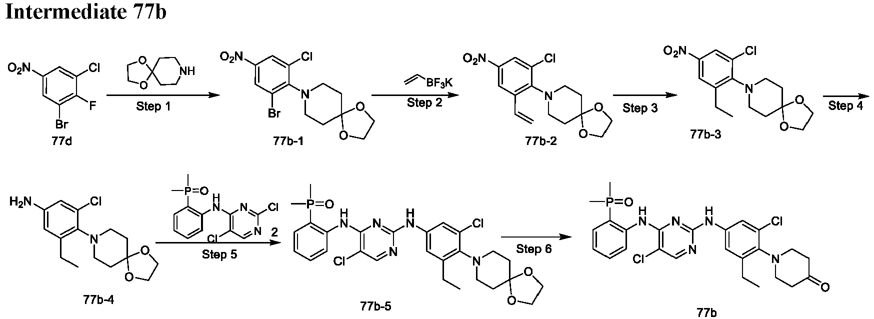

Step 1: Compound 77b-1 was prepared with reference to step 1 for intermediate 8a. MS m/z (ESI): 377.1 [M+H]⁺.

Step 2: Compound 77b-2 was prepared with reference to the preparation of intermediate 5d. MS m/z (ESI): 325.1 [M+H]⁺.

Step 3: Compound 77b-3 was prepared with reference to step 3 for intermediate 46a. MS m/z (ESI): 327.1 [M+H]⁺.

Step 4: Compound 77b-4 was prepared with reference to step 2 for intermediate 7a. MS m/z (ESI): 297.1 [M+H]⁺.

Steps 5-6: Compound 77b was prepared with reference to steps 3-4 for intermediate 21c. MS m/z (ESI): 532.2 [M+H]⁺.

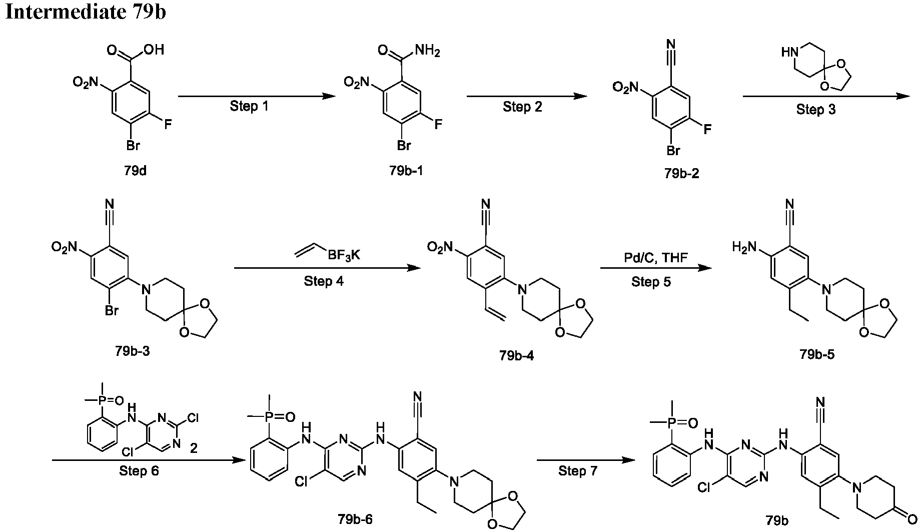

Step 1: Ammonium chloride (1.22 g, 22.73 mmol), diisopropylethylamine (2.94 g, 22.73 mmol, 3.96 mL), and HATU (4.32 g, 11.36 mmol) were added to a solution of 79d (2 g, 7.58 mmol) in DMF (20 mL), and then the reaction system was stirred at 25 °C for 4 h. After the reaction was completed, the reaction solution was concentrated and purified by column chromatography (PE/EA = 10/1-1/1) to give the title compound 79b-1 (yellow solid, 1.80 g). MS m/z (ESI): 263.1 [M+H]⁺.

Step 2: At 0 °C, triethylamine (9.12 mmol, 1.27 mL) was added to a solution of 79b-1 (1.6 g, 6.08 mmol) in anhydrous tetrahydrofuran (50 mL). Trifluoroacetic anhydride (9.12 mmol, 1.27 mL) was then added slowly, and the reaction system was stirred at 25 °C for 2 h. After the reaction was completed, the reaction mixture was extracted with water and ethyl acetate and separated into layers. The organic layer was washed with brine, dried over anhydrous sodium sulfate, filtered, and concentrated at reduced pressure to give the title compound 79b-2 (yellow solid, 1.5 g, crude product). MS m/z (ESI): 245.1 [M+H]⁺.

Step 3: Diisopropylethylamine (422.01 mg, 3.27 mmol, 568.75 µL) and 1,4-dioxa-8-azaspiro[4.5]decane (467.53 mg, 3.27 mmol, 417.43 µL) were added to a solution of 79b-2 (800 mg, 3.27 mmol) in isopropanol (10 mL), and then the reaction system was stirred at 100 °C for 1 h. After the reaction was completed, the reaction solution was concentrated at reduced pressure to give the title compound 79b-3 (yellow solid, 1.00 g, crude product). MS m/z (ESI): 367.9 [M+H]⁺.

Step 4: Compound 79b-4 was prepared with reference to the method for intermediate 5d. MS m/z (ESI): 316.1 [M+H]⁺.

Step 5: Compound 79b-5 was prepared with reference to step 4 for intermediate 94c. MS m/z (ESI): 288.1 [M+H]⁺.

Step 6: 2,5-Dichloro-*N*-(2-dimethylphosphorylphenyl)pyrimidin-4-amine (242.02 mg, 765.60 µmol), 79b-5 (220 mg, 765.60 µmol), tris(dibenzylideneacetone)dipalladium (70.11 mg, 76.56 µmol), 4,5-bis(diphenylphosphino)-9,9-dimethylxanthene (44.3 mg, 76.56 µmol), and cesium carbonate (498.89 mg, 1.53 mmol) were added to a solution of 1,4-dioxane (2 mL), and then the reaction system was stirred at 100 °C for 8 h in a nitrogen atmosphere. After the reaction was completed, the system was extracted with water and ethyl acetate and separated into layers. The organic layer was washed with brine, dried over anhydrous sodium sulfate, filtered, concentrated at reduced pressure, and purified by thin-layer chromatography (DCM/MeOH = 10/1) to give the title compound 79b-6. MS m/z (ESI): 567.2 [M+H]⁺.

Compound 79b was prepared with reference to step 4 for intermediate 21c as step 7. MS m/z (ESI): 523.1 [M+H]⁺.

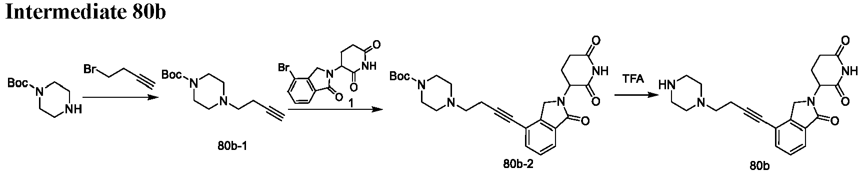

Step 1: Compound *tert*-butyl piperazine-1-carboxylate (1 g, 5.37 mmol) and compound 4-bromobut-1-yne (785 mg, 5.91 mmol) were dissolved in CH₃CN (20 mL), and then K₂CO₃ (1.11 g, 8.05 mmol) was added. The reaction system was stirred at 60 °C for 8 h. After the reaction was completed, the reaction system was concentrated. EA and H₂O were added to the residue, and the aqueous phase was extracted with EA, dried over anhydrous sodium sulfate, filtered, concentrated, and purified by column chromatography (EA/PE: ~20%) to give compound 80b-1 (colorless oily liquid, 950 mg).

Step 2: Compound 80b-2 was prepared with reference to step 1 for intermediate 1b. MS m/z (ESI): 481.5 [M+H]⁺.

Step 3: Compound 80b was prepared with reference to step 2 for intermediate 3b. MS m/z (ESI): 381.5 [M+H]⁺.

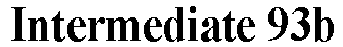

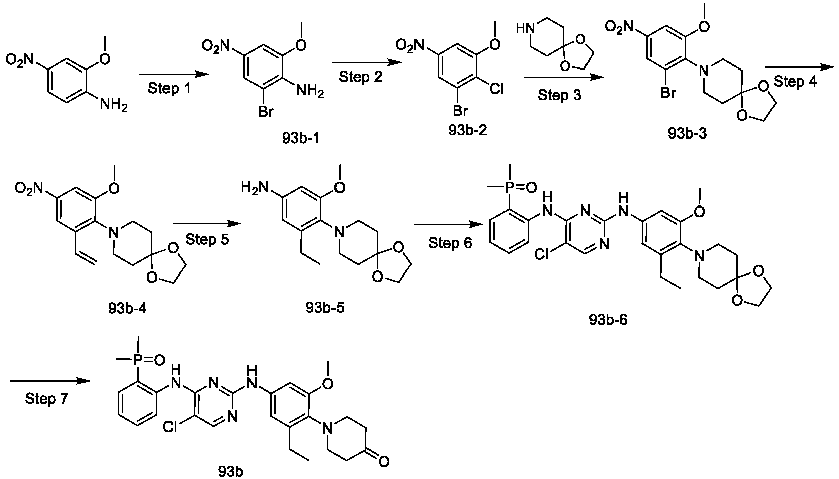

Step 1: NBS (5.29 g, 29.74 mmol) was added to a solution of 2-methoxy-4-nitroaniline (5 g, 29.74 mmol) in dichloromethane (50 mL) at 0 °C, and then the reaction system was stirred at 25 °C for 4 h. After the reaction was completed, the reaction solution was concentrated at reduced pressure and then separated and purified by normal-phase column chromatography (PE/EA = 1:0-5:1) to give the title compound 93b-1 (yellow solid, 3 g). ¹H NMR (400 MHz, DMSO-d6) δ = 7.97 (d, *J* = 2.1 Hz, 1H), 8.20-7.88 (m, 1H), 7.58 (d, *J=* 2.0 Hz, 1H), 6.41 (br s, 2H), 4.18-3.70 (m, 3H). MS m/z (ESI): 247.1 [M+H]⁺.

Step 2: At 0 °C, 93b-1 (2 g, 8.10 mmol) and sodium nitrite (1.40 g, 20.24 mmol) were dissolved in hydrochloric acid (40 mL), and then cuprous chloride (1.60 g, 16.19 mmol, 387.18 µL) was added. The reaction system was warmed to 25 °C and stirred for 5 h in a nitrogen atmosphere. After the reaction was completed, the reaction solution was diluted with water, extracted with ethyl acetate, and separated and purified by normal-phase column chromatography (PE/EA = 1:0-10:1) to give the title compound 93b-2 (white solid, 2 g). MS m/z (ESI): 266.1 [M+H]⁺.

Step 3: *N*,*N*-Diisopropylethylamine (2.91 g, 22.52 mmol) was added to a solution of 93b-2 (2 g, 7.51 mmol) and 1,4-dioxa-8-azaspiro[4.5]decane (2.15 g, 15.01 mmol) in dimethyl sulfoxide (20 mL), and then the reaction system was stirred at 80 °C for 1 h. After the reaction was completed, the reaction solution was diluted with water, extracted with EA, and separated and purified by normal-phase column chromatography (PE/EA = 1:0-5:1) to give the title compound 93b-3 (yellow solid, 2.3 g). MS m/z (ESI): 373.1 [M+H]⁺.

Step 4: Compound 93b-4 was prepared with reference to the method for intermediate 5d. MS m/z (ESI): 321.1 [M+H]⁺.

Step 5: Compound 93b-5 was prepared with reference to step 2 for intermediate 3a. MS m/z (ESI): 293.1 [M+H]⁺.

Steps 6-7: Compound 93b was prepared with reference to steps 3-4 for 21c. MS m/z (ESI): 528.4[M+H]⁺.

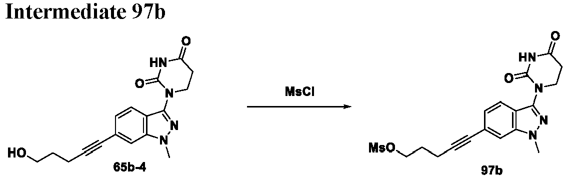

Compound 97b was prepared with reference to step 2 for intermediate 1b. MS m/z (ESI): 405.2 [M+H]⁺.

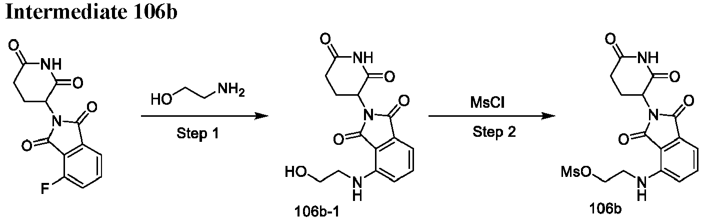

Step 1: Compound 106b-1 was prepared with reference to the synthesis for compound C-1. MS m/z (ESI): 318.1 [M+H]⁺.

Step 2: Compound 106b was prepared with reference to step 2 for intermediate 1b. MS m/z (ESI): 396.8 [M+H]⁺.

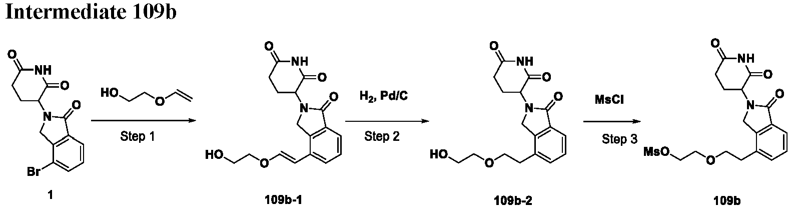

Step 1: Compound 109b-1 was prepared with reference to step 1 for intermediate 114d. MS m/z (ESI): 331.2 [M+H]⁺.

Step 2: Compound 109b-2 was prepared with reference to step 2 for intermediate 3a. MS m/z (ESI): 333.1 [M+H]⁺.

Step 3: Compound 109b was prepared with reference to step 2 for intermediate 1b. MS m/z (ESI): 411.2 [M+H]⁺.

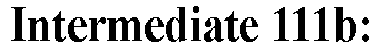

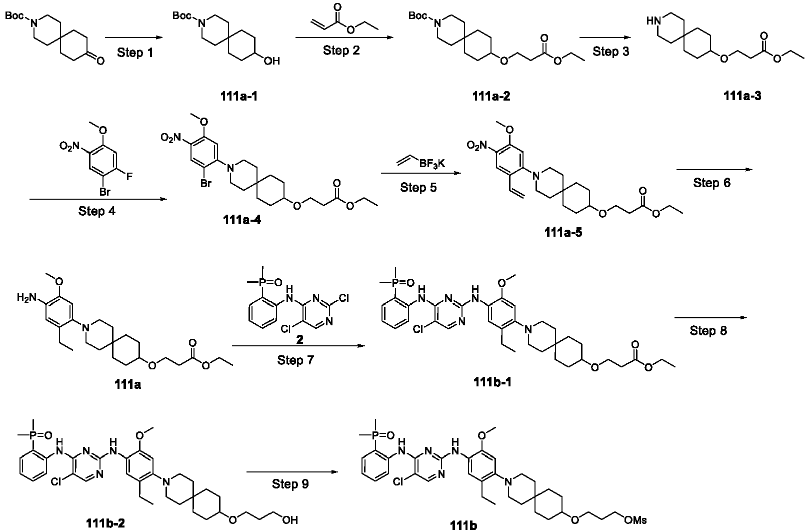

Step 1: Compound *tert*-butyl 9-oxo-3-azaspiro[5.5]undecane-3-carboxylate (3000 mg, 11.22 mmol) and anhydrous THF (30 mL) were added in a three-necked reaction flask. In a nitrogen atmosphere and in an ice-water bath, NaBH₄ (849 mg, 22.44 mmol) was added in portions. After the addition, the system was warmed to room temperature and stirred overnight. After the reaction was completed, anhydrous Na₂SO₄ was added to the reaction solution, and water was slowly and dropwise added to quench the reaction. The mixture was filtered at reduced pressure and washed with DCM, and the filtrate was concentrated to give crude product 111a-1 (pale yellow oil, 3000 mg). The crude product was directly used in the next step.

Step 2: Compound 111a-2 was prepared with reference to step 1 for intermediate 110d.

Step 3: Compound 111a-3 was prepared with reference to step 2 for intermediate 3b.

Step 4: Compound 111a-4 was prepared with reference to step 1 for intermediate 3a. MS m/z (ESI): 499.6 [M+H]⁺.

Step 5: Compound 111a-5 was prepared with reference to the method for intermediate 5d. MS m/z (ESI): 447.8 [M+H]⁺.

Step 6: Compound 111a was prepared with reference to step 2 for intermediate 3a. MS m/z (ESI): 419.7 [M+H]⁺.

Step 7: Compound 111b-1 was prepared with reference to step 1 for intermediate 4c. MS m/z (ESI): 699.0 [M+H]⁺.

Step 8: Compound 111b-2 was prepared with reference to step 2 for intermediate 110d. MS m/z (ESI): 656.2 [M+H]⁺.

Step 9: Compound 111b was prepared with reference to step 2 for intermediate 1b. MS m/z (ESI): 734.3 [M+H]⁺.

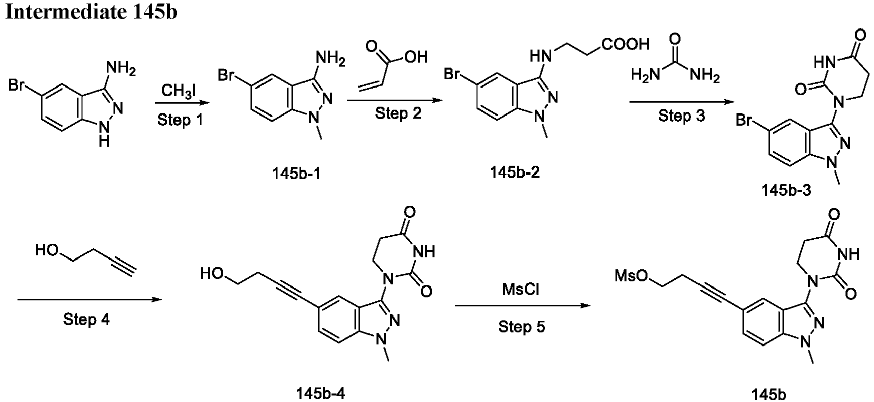

Compound 145b was prepared with reference to the method for intermediate 65b. MS m/z (ESI): 391.2 [M+H]⁺.

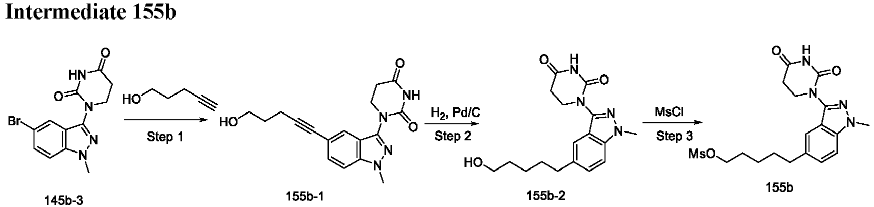

Compound 155b was prepared with reference to the method for intermediate 4b. MS m/z (ESI): 409.2 [M+H]⁺.

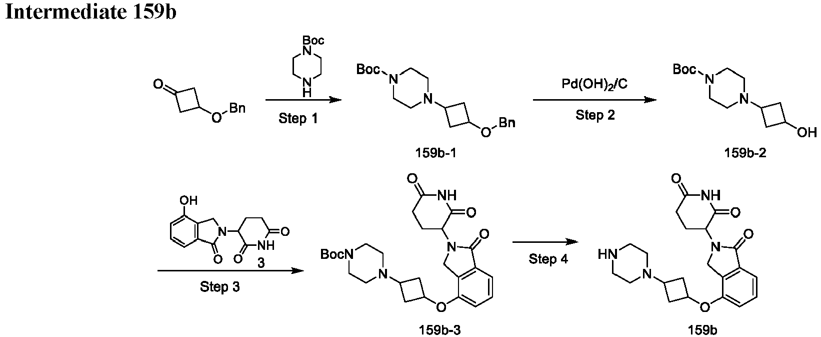

Step 1: 3-(Benzyloxy)cyclobutan-1-one (4.73 g, 26.84 mmol) and *tert*-butyl piperazine-1-carboxylate (5 g, 26.84 mmol) were dissolved in a solution of MeOH (150 mL), and AcOH (8.06 g, 134.21 mmol) was added. The system was stirred at room temperature for 0.5 h. NaBH₃CN (5.06 g, 80.53 mmol) was then added, and the reaction system was stirred at room temperature for 3.5 h. After the reaction was completed, a saturated aqueous NaHCOs solution was added to quench the reaction. The mixture was extracted with DCM and separated into layers. The organic layer was washed with brine, dried over anhydrous sodium sulfate, filtered, concentrated at reduced pressure, and purified by column chromatography (PE/EA = 5/1-3/1) to give compound 159b-1 (colorless oil, 6.5 g). ¹H NMR (500 MHz, CDCl₃) δ 7.38-7.30 (m, 5H), 4.42 (s, 2H), 3.83-3.73 (m, 1H), 3.47-3.37 (m, 4H), 2.44-2.40 (m, 2H), 2.33-2.22 (m, 5H), 2.18-2.14 (m, 1H), 1.90-1.82 (m, 2H), 1.72 (br s, 1H), 1.45 (s, 9H). MS m/z (ESI): 257.2 [M+H]⁺.

Step 2: Compound 159b-1 (6.5 g, 18.76 mmol) was dissolved in a solution of methanol (80 mL). In a nitrogen atmosphere, catalytic amounts of acetic acid (0.5 mL), Pd/C (2 g) and Pd(OH)₂/C (1 g) were added. The reaction system was purged with hydrogen and stirred at 45 °C for 48 h in the hydrogen atmosphere. After the reaction was completed, the reaction mixture was filtered. The filtrate was concentrated at reduced pressure and then purified by column chromatography (MeOH/DCM: ~3%-10%) to give compound 159b-2 (colorless oil, 3 g). MS m/z (ESI): 409.2 [M+H]⁺.

Step 3: Compound 159b-2 (95 mg, 0.36 mmol) and compound 3 (80 mg, 0.3 mmol) were dissolved in THF (10 mL), and then PPh₃ (242 mg, 0.9 mmol) and DIAD (187 mg, 0.9 mmol) were added. In a nitrogen atmosphere, the reaction system was stirred at room temperature for 16 h. After the reaction was completed, the reaction mixture was extracted with water and EA, and the organic phase was dried and concentrated at reduced pressure to give a crude product. The crude product was purified by prep-TLC (DCM:MeOH = 15:1) to give compound 159b-3 (pale yellow solid, 75 mg). MS m/z (ESI): 499.2 [M+H]⁺.

Step 4: Compound 159b was prepared with reference to step 2 for intermediate 3b. MS m/z (ESI): 399.2 [M+H]⁺.

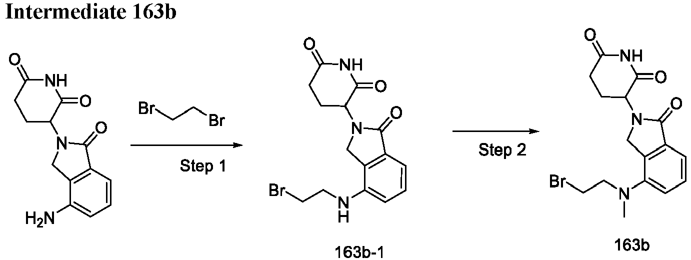

Step 1: 3-(4-Amino-1-oxoisoindolin-2-yl)piperidine-2,6-dione (300 mg, 1.16 mmol) was added to a reaction flask. NMP (mL) was added, and then DIEA (0.5 mL) and 1,2-dibromoethane (1007 mg, 5.8 mmol) were added. The system was heated to 80 °C and stirred for 16 h. Water was added to the reaction solution, and the mixture was extracted with DCM, dried over anhydrous sodium sulfate, and concentrated. The reaction mixture was concentrated and then purified by column chromatography (dichloromethane:methanol = 20:1) to give compound 163b-1 (white solid, 200 mg). MS m/z (ESI): 366.2, 368.2 [M+H]⁺.

Step 2: Compound 163b-1 (100 mg, 0.27 mmol) was dissolved in acetonitrile (5 mL) and water (0.1 mL), and a 37% aqueous formaldehyde solution (0.5 mL, 6.1 mmol) was added. The system was stirred at 20 °C for 2 h, then NaCNBH₃ (80 mg, 1.4 mmol) was added, and the system was stirred at 20 °C for another 16 h. After the reaction was completed, water was added to quench the reaction. The mixture was extracted with DCM, and the organic phase was dried over anhydrous sodium sulfate and concentrated. The reaction mixture was concentrated and then purified by column chromatography (dichloromethane:methanol = 10:1) to give 163b (white solid, 45 mg). MS m/z (ESI): 382.0 [M+H]⁺.

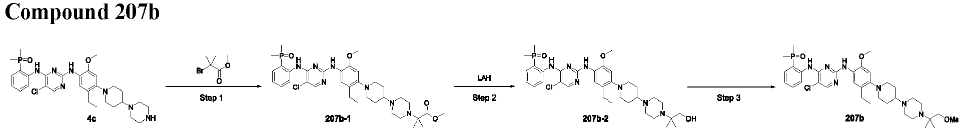

Step 1: Compound 4c (400 mg, 0.67 mmol) was dissolved in DMF (8 mL) before methyl 2-bromo-2-methylpropionate (145 mg, 0.80 mmol) and K₂CO₃ (185 mg, 1.34 mmol) were separately added. The system was then heated to 80 °C and stirred for 16 h. After the reaction was completed, as detected by LCMS, the reaction solution was filtered at reduced pressure, and the filtrate was concentrated by rotary evaporation at reduced pressure. The crude product was separated by normal-phase column chromatography (MeOH/DCM = 0-5%) to give crude compound 207b-1 (pale yellow solid, 300 mg, yield: 63%). MS m/z (ESI): 697.3 [M+H]⁺.

Step 2: Title compound 207b-2 (pale yellow oil, 130 mg, yield: 45%) was prepared with reference to the synthesis method of step 2 for compound 110d. The crude product was directly used in the next step without further purification. MS m/z (ESI): 669.3 [M+H]⁺.

Step 3: Title compound 207b (130 mg, yield: 90%) was prepared with reference to the synthesis method of step 2 for compound 1b. MS m/z (ESI): 747.3 [M+H]⁺.

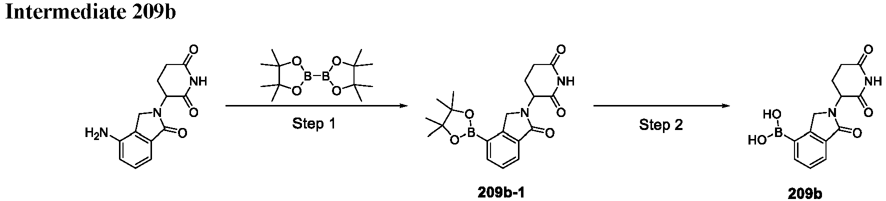

Step 1: Compound 3-(4-amino-1-oxoisoindolin-2-yl)piperidine-2,6-dione (1000 mg, 3.80 mmol), bis(pinacolato)diboron (1100 mg, 4.10 mmol), and anhydrous acetonitrile (20 mL) were added in a single-necked flask, and *tert*-butyl nitrite (600 mg, 5.70 mmol) was added slowly with stirring. After the addition, the system was stirred at room temperature for 5 h. After the reaction was completed, the reaction solution was directly evaporated to dryness at reduced pressure, and the residue was stirred with silica gel. The crude product was separated by normal-phase column chromatography to give compound 209b-1 (pale yellow solid, 700 mg). MS m/z (ESI): 371.2 [M+H]⁺.

Step 2: Compound 209b-1 (500 mg, 1.40 mmol), tetrahydrofuran (8 mL), and water (2 mL) were added to a single-necked flask before sodium periodate (870 mg, 4.20 mmol) was added slowly with thorough stirring. After the addition, the system was stirred at room temperature for 2 h, and then a 1 N diluted HCl solution (2 mL) was added. The system was then stirred at room temperature overnight. After the reaction was completed, the reaction solution was evaporated to dryness at reduced pressure before dichloromethane and water were added. The system was stirred at room temperature for 1 h, filtered at reduced pressure, washed with water, and lyophilized to give compound 209b (pale yellow solid, 320 mg). ¹H NMR (500 MHz, DMSO-*d*₆₎ *δ* 10.97 (s, 1H), 8.33 (s, 2H), 8.03 (d, *J* = 7.3 Hz, 1H), 7.76 (d, *J=* 7.3 Hz, 1H), 7.50 (t, *J* = 7.4 Hz, 1H), 5.13 (dd, *J* = 13.3, 5.1 Hz, 1H), 4.54 (d, *J=* 18.1 Hz, 1H), 4.45 (d, *J=* 18.1 Hz, 1H), 2.94-2.86 (m, 1H), 2.62-2.56 (m, 1H), 2.45-2.37 (m, 1H), 2.04-1.98 (m, 1H). MS m/z (ESI): 289.5 [M+H]⁺

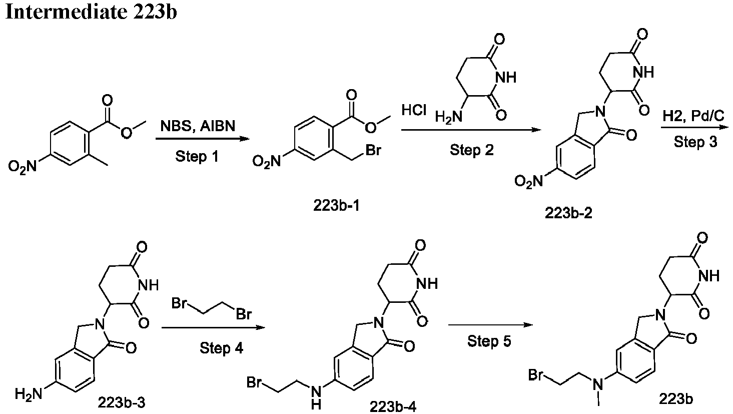

Steps 1 and 2: Compound 223b-2 was prepared with reference to steps 2 and 3 for intermediate 3d. MS m/z (ESI): 290.2 [M+H]⁺.

Step 3: Compound 223b-3 was prepared with reference to step 2 for intermediate 3a. MS m/z (ESI): 260.2 [M+H]⁺.

Steps 4 and 5: Compound 223b was prepared with reference to steps 1 and 2 for intermediate 163b. MS m/z (ESI): 382.2 [M+H]⁺.

### Preparation of compounds of formula (a-2)

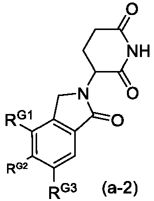

With reference to the method in any one of compounds 1b, 3b, 4b, and 5b-223b, intermediate compounds of formula (a-2) were prepared from suitable lenalidomide derivatives, as shown in Table 4 below.

**Table 4**

| Intermediate | R^{G1} | R^{G2} | R^{G3} | MS m/z (ESI) |
|---|---|---|---|---|
| 2b | 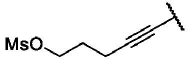 | H | H | 405.1 [M+H]⁺ |
| 6b | H | 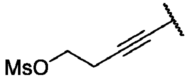 | H | 391.2 [M+H]⁺ |
| 7b | 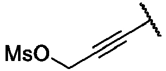 | H | H | 377.2 [M+H]⁺ |
| 8b | 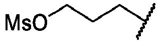 | H | H | 381.2 [M+H]⁺ |
| 9b | 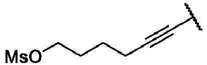 | H | H | |
| 10b | 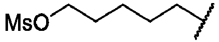 | H | H | 409.2 [M+H]⁺ |
| 29b | 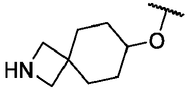 | H | H | 384.6 [M+H]⁺ |
| 38b | H | 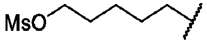 | H | 409.2 [M+H]⁺ |
| 53b | 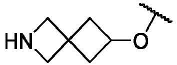 | H | H | 356.5 [M+H]⁺ |
| 74b | 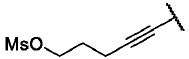 | F | H | 423.1 [M+H]⁺ |
| 95b | 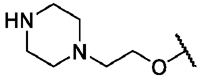 | H | H | 373.1 [M+H]⁺ |

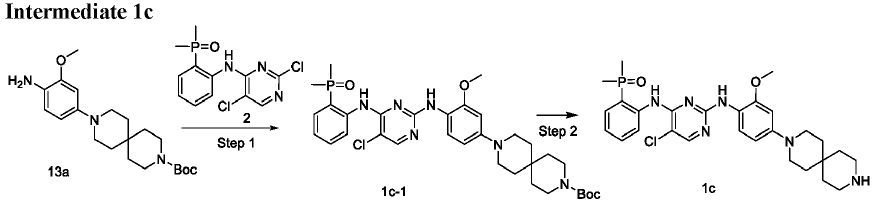

Step 1: Compound 13a (300 mg, 0.80 mmol) was added to a reaction flask before compound 2 (252 mg, 0.80 mmol), Pd(OAc)₂ (18 mg, 0.08 mmol), Xant-phos (48 mg, 0.08 mmol), anhydrous Cs₂CO₃ (781 mg, 2.40 mmol), and anhydrous DMF (8 mL) were separately added. After the addition, the reaction system was purged 3 times with argon, then heated to 105 °C, and stirred for 16 h. After the reaction was completed, the reaction mixture was filtered and concentrated. The crude product was purified by column chromatography (dichloromethane:methanol = 30:1) and concentrated by rotary evaporation to give compound 1c-1. MS m/z (ESI): 655.3 [M+H]⁺.

Step 2: Compound 1c-1 (350 mg, 0.53 mmol) was added to a single-necked flask before anhydrous dichloromethane (15 mL) and TFA (3 mL) were separately added. After the addition, the system was stirred at room temperature for 2 h. After the reaction was completed, the solvent was removed from the reaction mixture at reduced pressure. The crude product was dissolved in dichloromethane and methanol before a saturated NaHCOs solution was slowly added to the system to adjust the pH to 8-9. The mixture was then extracted with dichloromethane and methanol (10:1), and the organic phases were combined, dried over anhydrous sodium sulfate, and concentrated at reduced pressure to remove the solvent, thus giving compound 1c. MS m/z: 555.7 [M+H]⁺.

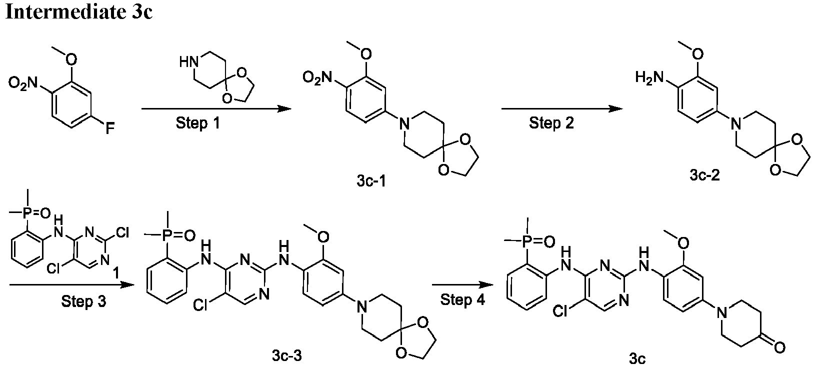

Compound 3c as a brown solid was prepared with reference to the method for intermediate 21c. MS m/z (ESI): 500.3 [M+H]⁺.

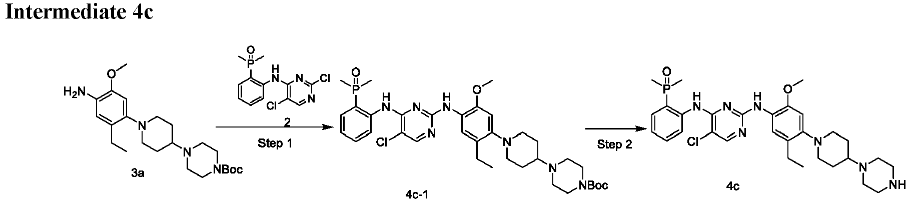

Step 1: Compound 3a (500 mg, 1.2 mmol), compound 2 (628.5 mg, 1.5 mmol), and p-toluenesulfonic acid monohydrate (412 mg, 2.4 mmol) were added to a reaction flask before i-PrOH (50 mL) was added. The reaction solution was stirred at 80 °C for 16 h and concentrated, and then a 5% NaOH solution was added. The mixture was extracted with dichloromethane, concentrated, and separated and purified by normal-phase column chromatography (dichloromethane:methanol = 10:1) to give compound 4c-1 (white solid, 500 mg). MS m/z (ESI): 698.6 [M+H]⁺.

Step 2: Compound 4c-1 (1.4 g, 20 mmol) was added to a reaction flask before DCM (30 mL) was added. TFA (6 mL) was then added, and the reaction mixture was stirred at room temperature for 0.5 h and concentrated. A saturated sodium bicarbonate solution was added, and the mixture was extracted with dichloromethane and concentrated to give compound 4c (white solid, 1 g). MS m/z (ESI): 598.7 [M+H]⁺.

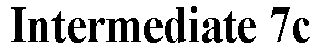

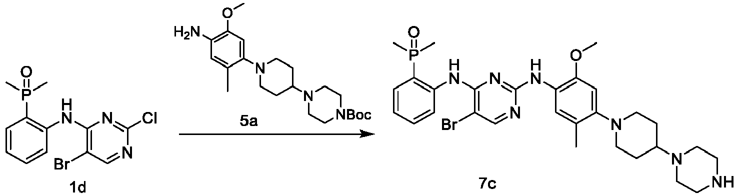

Compound 1d (200 mg, 0.55 mmol) and compound 5a (344 mg, 0.83 mmol) were added to a single-necked reaction flask before TsOH H₂O (477 mg, 2.77 mmol) and *n*-BuOH (10 mL) were added. The system was purged 3 times with argon, then heated to 140 °C, and stirred for 16 h. After the reaction was completed, the reaction mixture was concentrated. The crude product was purified by column chromatography and concentrated by rotary evaporation to give compound 7c (pale yellow solid, 0.2 g). MS (ESI) m/z: 628.3 [M+H]⁺.

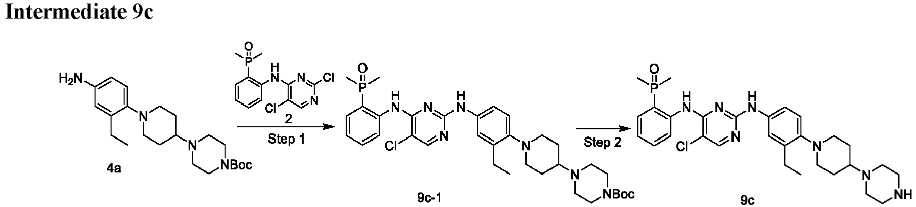

Compound 9c (brown solid, 140 mg) was prepared with reference to the method for intermediate 4c. MS m/z (ESI): 568.7 [M+H]⁺.

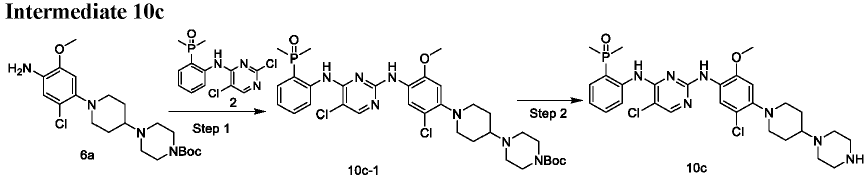

Compound 10c was prepared with reference to the method for intermediate 4c. MS m/z (ESI): 605.4 [M+H]⁺.

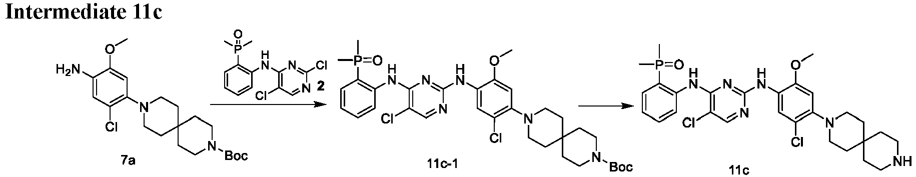

Compound 11c was prepared with reference to the method for intermediate 4c. MS m/z (ESI): 590.4 [M+H]⁺.

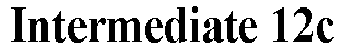

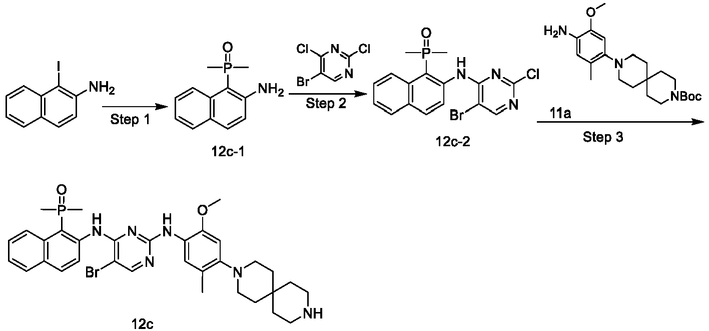

Step 1: Dimethylphosphine oxide (2.90 g, 37.16 mmol), 4,5-bis(diphenylphosphino)-9,9-dimethylxanthene (4.30 g, 7.43 mmol), palladium acetate (835.00 mg, 3.72 mmol), and potassium phosphate (15.80 g, 74.43 mmol) were added to a solution of 1-iodonaphthalen-2-amine (10 g, 37.16 mmol) in 1,4-dioxane (100 mL), and the reaction system was stirred at 100 °C for 10 h. After the reaction was completed, the reaction mixture was extracted with water (80 mL) and dichloromethane and separated into layers. The organic layer was washed with brine, dried over anhydrous sodium sulfate, filtered, and concentrated at reduced pressure to give a crude product. The crude product was purified by column chromatography to give compound 12c-1 (brown solid, 5.6 g). MS m/z (ESI): 220.3 [M+H]⁺.

Step 2: 5-Bromo-2,4-dichloropyrimidine (5.02 g, 22.03 mmol, 2.82 mL) and *N*,*N*-diisopropylethylamine (3.54 g, 27.39 mmol, 4.77 mL) were added to a solution of compound 12c-1 (3 g, 13.68 mmol) in *n*-butanol (30 mL), and the reaction system was stirred at 120 °C for 8 h. After the reaction was completed, the reaction mixture was filtered and washed with ethanol to give the title compound 12c-2 (white solid, 3.27 g). MS m/z (ESI): 409.9 [M+H]⁺.

Step 3: *p*-Toluenesulfonic acid monohydrate (231.61 mg, 1.22 mmol) was added to a solution of compound 12c-2 (500 mg, 1.22 mmol) and compound 11a in isopropanol (5 mL), and then the reaction system was stirred for 6 h at 100 °C in a nitrogen atmosphere. After the reaction was completed, the reaction solution was adjusted to pH 9 with a sodium bicarbonate solution, and then extracted with water and dichloromethane and separated into layers. The organic layer was dried over anhydrous sodium sulfate, filtered, and concentrated at reduced pressure to give compound 12c. MS m/z (ESI): 663.3 [M+H]⁺.

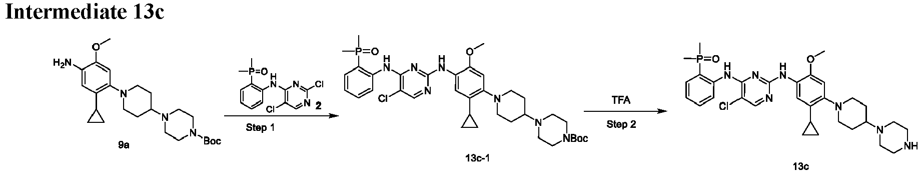

Compound 13c was prepared with reference to the method for intermediate 4c. MS m/z (ESI): 610.2 [M+H]⁺.

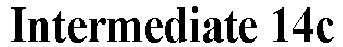

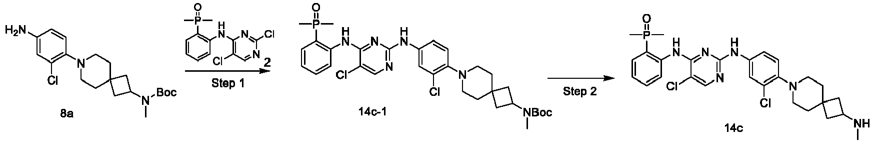

Compound 14c was prepared with reference to the method for intermediate 4c. MS m/z (ESI): 559.1 [M+H]⁺.

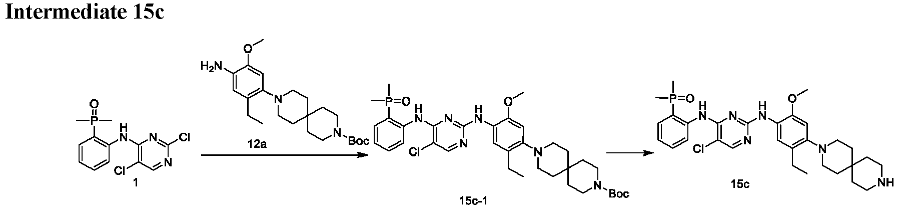

Compound 15c was prepared with reference to the method for intermediate 4c. MS m/z (ESI): 584.1 [M+H]⁺.

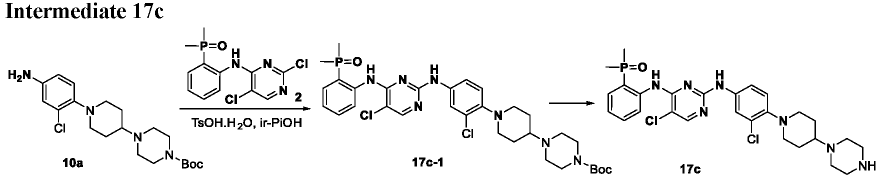

Compound 17c was prepared with reference to the method for intermediate 4c. MS m/z (ESI): 574.3 [M+H]⁺.

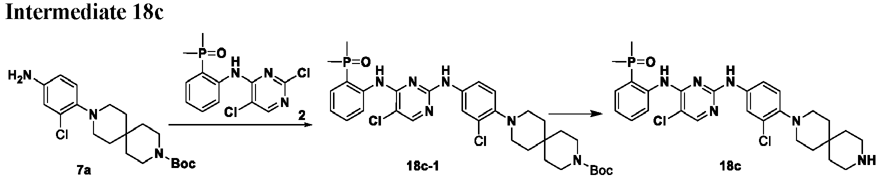

Compound 18c was prepared with reference to the method for intermediate 4c. MS m/z (ESI): MS m/z: 559.6 [M+H]⁺.

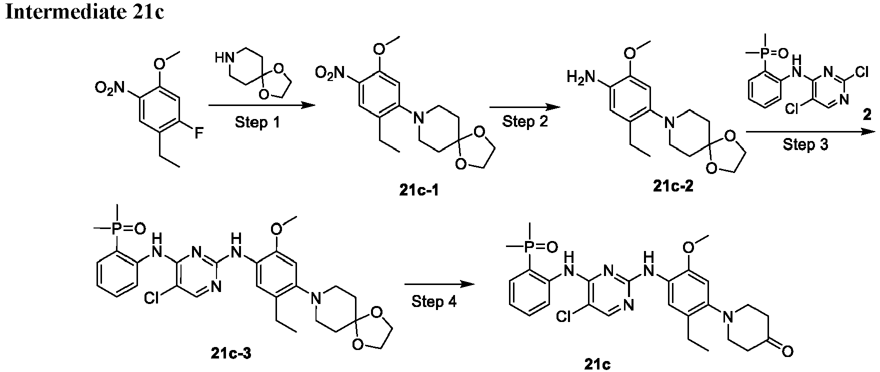

Step 1: 1-Ethyl-2-fluoro-4-methoxy-5-nitro-benzene (1 g, 5.02 mmol) and 1,4-dioxa-8-azaspiro[4.5]decane (1.44 g, 10.04 mmol, 1.28 mL) were added to a solution of DMSO (10 mL), and then Cs₂CO₃ (4.91 g, 15.06 mmol) was added. The reaction system was stirred at 110 °C for 12 h. After the reaction was completed, the reaction mixture was diluted with water and extracted with ethyl acetate. The organic layers were combined, dried over anhydrous sodium sulfate, filtered, and concentrated to give a crude product. The crude product was separated and purified by column chromatography (PE:EA = 25:1-1:1) to give compound 21c-1 (yellow oil, 1.34 g). MS m/z (ESI): 323.2 [M+H]⁺.

Step 2: Compound 21c-1 (1.2 g, 3.72 mmol) was added to a solution of ethanol (30 mL), and then wet Pd/C (200 mg, 10% purity) was added. The reaction system was stirred at 40 °C for 2 h in a hydrogen atmosphere. After the reaction was completed, the mixture was filtered, and the filtrate was concentrated to give compound 21c-2 (1.02 g, crude product), which was directly used in the next step. MS m/z (ESI): 293.2 [M+H]⁺.

Step 3: Compound 21c-2 (300 mg, 1.03 mmol) and compound 2 (324.37 mg, 1.03 mmol) were added to a solution of ethylene glycol (10 mL), and then p-toluenesulfonic acid monohydrate (292.77 mg, 1.54 mmol) was added. The reaction system was stirred at 90 °C for 12 h. After the reaction was completed, the reaction mixture was neutralized with a saturated aqueous sodium bicarbonate solution and extracted with ethyl acetate. The organic layers were combined, dried over anhydrous sodium sulfate, filtered, and concentrated to give a crude product. The crude product was separated and purified by a silica gel plate (dichloromethane:methanol = 15:1) to give compound 21c-3 (black solid, 100 mg), which was directly used in the next step. MS m/z (ESI): 572.3 [M+H]⁺.

Step 4: Compound 21c-3 (90 mg, 157.33 µmol) was added to a mixed solution of water (4.5 mL) and acetic acid (4.5 mL), and the reaction system was stirred at 90 °C for 2 h. After the reaction was completed, the reaction mixture was neutralized with a saturated aqueous sodium bicarbonate solution and extracted with DCM. The organic layers were combined, dried over anhydrous sodium sulfate, filtered, and concentrated to give compound 21c, which was directly used in the next step. MS m/z (ESI): 528.3 [M+H]⁺.

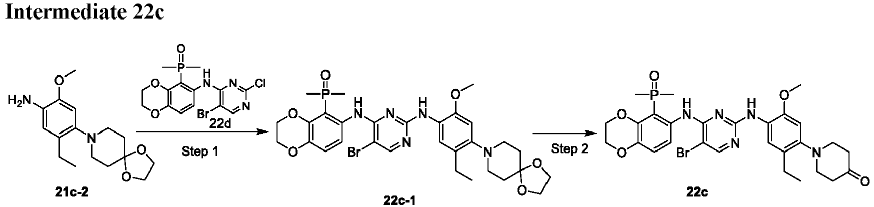

Compound 22c was prepared with reference to step 3 and step 4 for intermediate 21c. MS m/z (ESI): 630.2 [M+H]⁺.

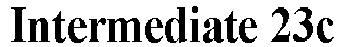

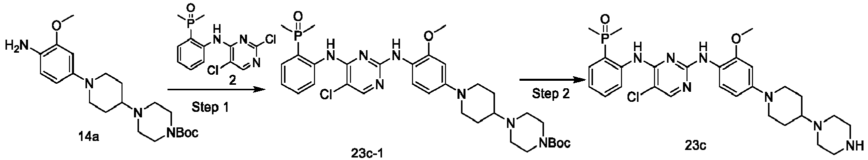

Compound 23c was prepared with reference to the method for intermediate 4c. MS m/z (ESI): 570.3 [M+H]⁺.

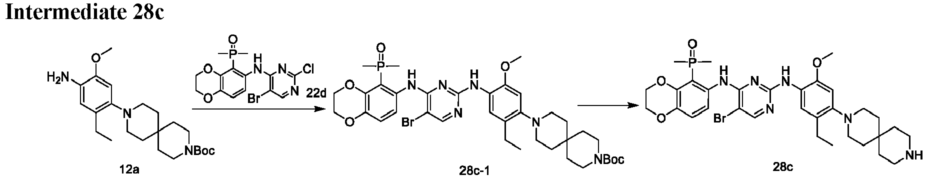

Compound 28c was prepared with reference to the method for intermediate 4c. LCMS: m/z (ESI): 685.0, 687.0[M+H]⁺.

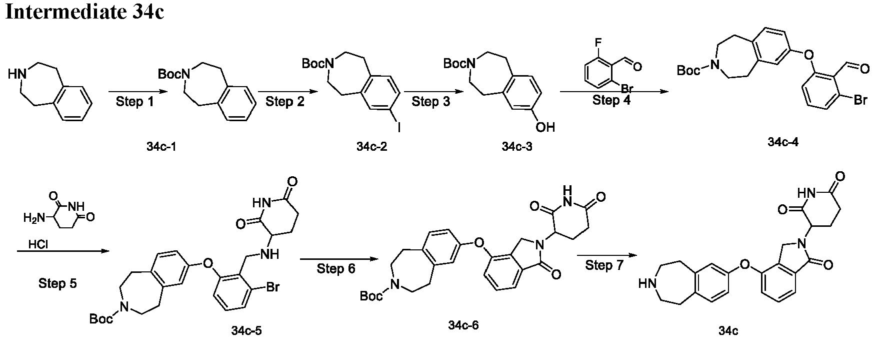

Step 1: DIEA and (Boc)₂O (5.59 g, 25.61 mmol) were added to a solution of 2,3,4,5-tetrahydro-1H-benzo[d]azepine (2.9 g, 19.70 mmol) in dichloromethane (150 mL), and then the reaction system was stirred at 25 °C for 2 h. After the reaction was completed, the reaction mixture was diluted with ethyl acetate (100 mL), washed twice with hydrochloric acid, and then washed with saturated brine. The organic phase was dried over anhydrous sodium sulfate and concentrated to give compound 34c-1. MS m/z (ESI): 192.0 [M-55]⁺. Step 2: At 25 °C, silver trifluoroacetate and iodine (4.69 g, 18.48 mmol) were added to a solution of 34c-1 (4.57 g, 18.48 mmol) in dichloromethane (50 mL). The reaction was completed after the reaction system was stirred at 25 °C for 16 h. The reaction solution was concentrated to give crude compound 34c-2 (brown oil, 0.7 g). MS m/z (ESI): 318.0 [M-55]⁺.

Step 3: Cuprous oxide (67.09 mg, 468.88 µmol), *N*,*N*-dimethylethanolamine (1.25 g, 14.07 mmol), and potassium hydroxide (789.27 mg, 14.07 mmol) were added to a mixed solution of 34c-2 (1.75 g, 4.69 mmol) in dimethyl sulfoxide (15 mL) and water (7.5 mL), and then the reaction system was stirred at 100 °C for 12 h. After the reaction was completed, the reaction solution was extracted with water and EA and separated into layers. The organic layer was washed with brine, dried over anhydrous sodium sulfate, filtered, and concentrated at reduced pressure to give a crude product. The crude product was purified by silica gel column chromatography (PE:EA= 100:1-10:1) to give the title compound 34c-3. MS m/z (ESI): 208.1 [M-55]⁺. Steps 4-7: Compound 34c was prepared with reference to steps 2-5 for intermediate 120c. MS m/z (ESI): 406.3 [M+H]⁺.

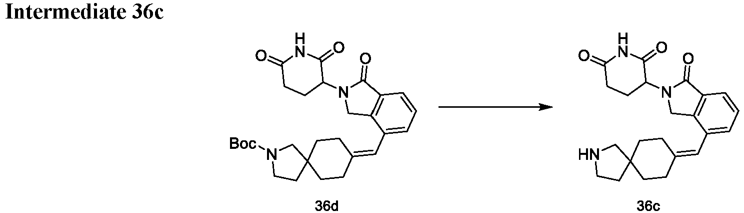

Compound 36c was prepared with reference to step 2 for intermediate 4c. MS m/z (ESI): 394.1 [M+H]⁺.

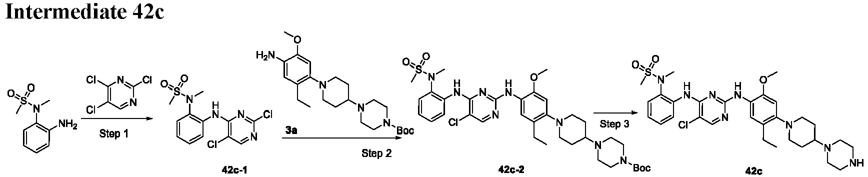

Step 1: Compound 42c-1 was prepared with reference to the synthesis method for intermediate 1d. MS m/z (ESI): 347.3 [M+H]⁺.

Steps 2-3: Compound 42c was prepared with reference to steps 1-2 for intermediate 4c. MS m/z (ESI): 629.9 [M+H]⁺.

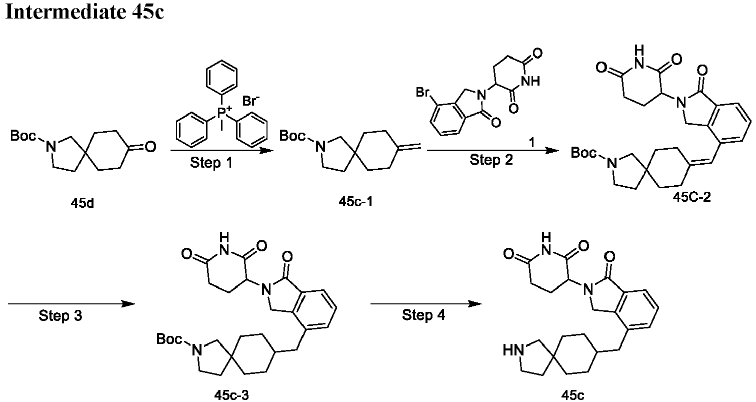

Step 1: Potassium *tert*-butoxide (1.99 g, 17.76 mmol) was added to a solution of methyl triphenyl phosphonium bromide (6.35 g, 17.76 mmol) in anhydrous tetrahydrofuran (60 mL) at -20 °C, and then the system was warmed to 0 °C and stirred for 0.5 h. In a nitrogen atmosphere, a solution of 45d (3 g, 11.84 mmol) in anhydrous tetrahydrofuran (30 mL) was dropwise added, and the reaction system was stirred at 25 °C for 3 h. After the reaction was completed, the reaction was quenched with an aqueous ammonium chloride solution. The reaction mixture was extracted with water and ethyl acetate and separated into layers, dried over anhydrous sodium sulfate, filtered, concentrated at reduced pressure, and purified by column chromatography (PE/EA = 10/1-5/1) to give compound 45c-1 (white oil, 2.38 g).

Step 2: 45c-1 (1.1 g, 4.38 mmol), compound 1 (1.56 g, 4.81 mmol), potassium chloride (326.25 mg, 4.38 mmol), tetrabutylammonium acetate (2.64 g, 8.75 mmol, 2.67 mL), palladium acetate (392.99 mg, 1.75 mmol), and potassium carbonate (1.21 g, 8.75 mmol) were added to a solution of DMF (10 mL). The reaction system was stirred at 100 °C for 5 h in a N₂ atmosphere. After the reaction was completed, the reaction solution was directly filtered and concentrated at reduced pressure to give a crude product. The crude product was separated and purified by reverse-phase chromatography (formic acid system) to give 45c-2 (yellow solid, 1 g). MS m/z (ESI): 494.2[M+H]⁺.

Step 3: Wet palladium on carbon (100 mg) was added to a solution of 45c-2 (100 mg, 202.60 µmol) in anhydrous tetrahydrofuran (2 mL), and then the reaction system was stirred at 25 °C for 1 h in a hydrogen atmosphere. After the reaction was completed, the mixture was filtered through celite and concentrated at reduced pressure to give compound 45c-2 (white solid, 92 mg). MS m/z (ESI): 396.1[M+H-100]⁺.

Step 4: Compound 45c (off-white solid, 73 mg) was prepared with reference to step 2 for 3b. MS m/z (ESI): 396.1[M+H]⁺.

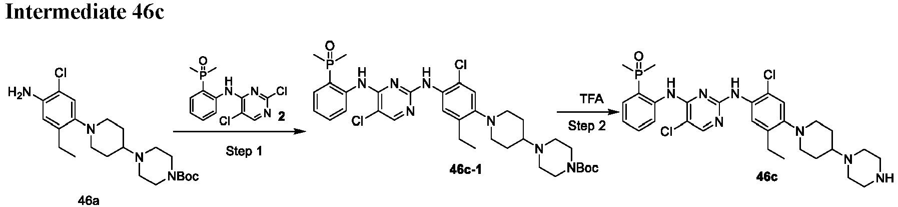

Compound 46c was prepared with reference to the method for intermediate 4c. MS m/z (ESI): 602.3 [M+H]⁺.

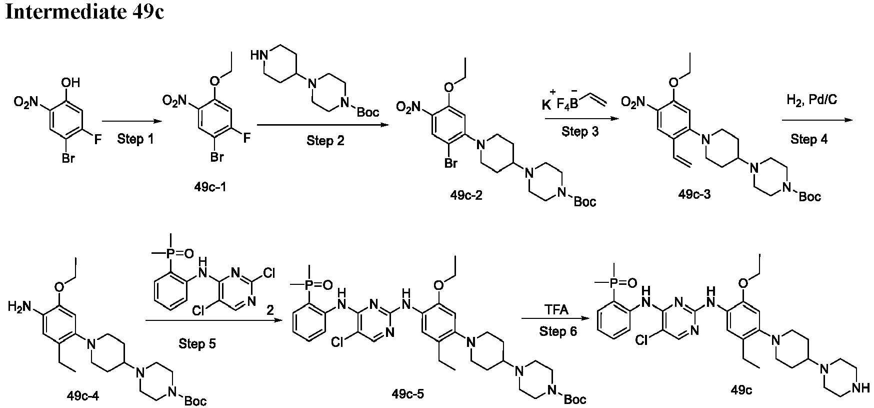

Step 1: Compound 49c-1 (790 mg, 3.3 mmol) was dissolved in DMF (8 mL), and potassium carbonate (1.15 g, mmol) and iodoethane (1.3 g, 8.37 mmol) were added. The system was then heated to 80 °C and stirred for 16 h. After the reaction was completed, water was added to quench the reaction. The precipitated solid was collected by filtration at reduced pressure and dried to give compound 49c-1 (pale yellow solid, 610 mg). MS m/z (ESI): 266.4 [M+H]⁺.

Step 2: Compound 49c-2 was prepared with reference to step 1 for intermediate 8a. MS m/z (ESI): 515.5 [M+H]⁺.

Step 3: Compound 49c-3 was prepared with reference to the preparation of intermediate 5d. MS m/z (ESI): 461.9 [M+H]⁺.

Step 4: Compound 49c-4 was prepared with reference to step 2 for intermediate 3a. MS m/z (ESI): 433.2 [M+H]⁺.

Steps 5-6: Compound 49c was prepared with reference to steps 1-2 for intermediate 1c. MS m/z (ESI): 612.2 [M+H]⁺.

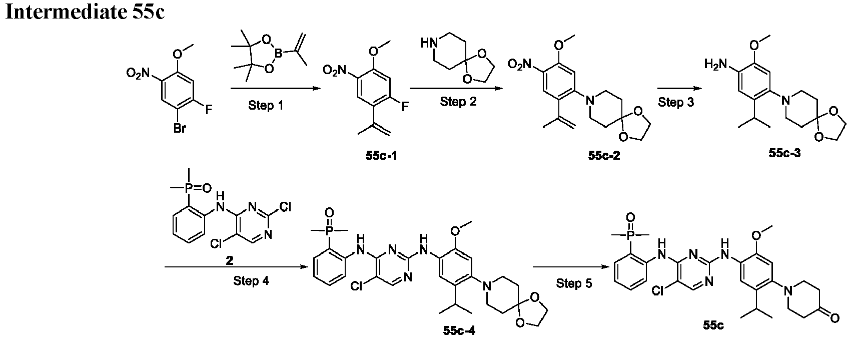

Step 1: Compound SSc-1 was separated with reference to the method for intermediate 5d. MS m/z (ESI): 212.2 [M+H]⁺.

Step 2: Compound 55c-2 was prepared with reference to step 1 for intermediate 21c. MS m/z (ESI): 335.5 [M+H]⁺.

Step 3: Compound 55c-3 was prepared with reference to step 2 for intermediate 3a. MS m/z (ESI): 307.2 [M+H]⁺.

Steps 4-5: Compound 55c was prepared with reference to steps 3-4 for intermediate 21c. MS m/z (ESI): 542.7 [M+H]⁺.

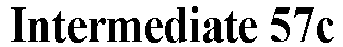

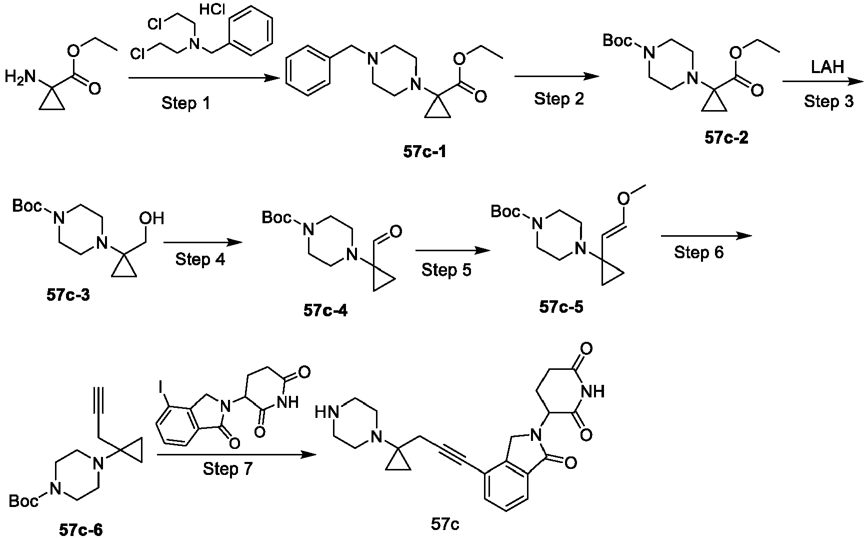

Step 1: Diisopropylethylamine (343.36 g, 2.66 mol), *N*-benzyl-2-chloro-*N*-(2-chloroethyl)ethan-1-amine hydrochloride (44 g, 265.67 mmol), and sodium iodide (79.65 g, 531.34 mmol) were added to a solution of compound ethyl 1-aminocyclopropane-1-carboxylate (71.36 g, 265.67 mmol) in ethanol (100 mL), and then the reaction system was stirred at 78 °C for 10 h. After the reaction was completed, the reaction mixture was concentrated at reduced pressure, diluted with ethyl acetate, washed with water, dried over anhydrous sodium sulfate, concentrated at reduced pressure, and then purified by silica gel column chromatography (PE:EA = 100:1-5:1) to give 57c-1 (brown oil, 49 g). MS m/z (ESI): 289.0 [M+H]⁺.

Step 2: Palladium on carbon (10 g, 169.91 mmol, 10% purity) and (Boc)₂O (41.17 g, 188.64 mmol) were added to a solution of 57c-1 (49 g, 169.91 mmol) in methanol (800 mL), and then the reaction system was stirred at 25 °C for 10 h in a hydrogen atmosphere. After the reaction was completed, the reaction solution was filtered through celite, and the filtrate was concentrated to give a crude product, which was purified by silica gel column chromatography (PE:EA = 100:1-20:1) to give 57c-2 (brown oil, 34 g, crude product). MS m/z (ESI): 299.2 [M+H]⁺.

Step 3 : A solution of 57c-2 (30 g, 100.54 mmol) in tetrahydrofuran (300 mL) was cooled to -78 °C, and lithium aluminum hydride (11.45 g, 301.63 mmol) was added slowly in portions while maintaining this temperature. The reaction system was then stirred at 0 °C for 2 h. Then the reaction was quenched with sodium sulfate decahydrate, and the reaction solution was filtered and concentrated to give the title compound 57c-3 (white solid, 20.0 g). MS m/z (ESI): 257.2 [M+H]⁺.

Step 4: Oxalyl chloride (1.49 g, 11.70 mmol) was added to a solution of dimethyl sulfoxide (2.00 g, 25.60 mmol) in dichloromethane (15 mL), and the reaction system was stirred at -78 °C for 20 min. Then a solution of 57c-3 (0.5 g, 1.95 mmol) in dichloromethane (3 mL) was added, and the system was stirred at -78 °C for 1 h. Then triethylamine (1.18 g, 11.70 mmol) was added, and the system was stirred for 10 min. After the reaction was completed, the reaction was quenched with a saturated ammonium chloride solution, and then the mixture was diluted with dichloromethane, washed with water, dried over anhydrous sodium sulfate, and concentrated at reduced pressure to give the title compound 57c-4 (colorless oil, 0.5 g, crude product).

Step 5: At 0 °C, potassium *tert*-butoxide (6.62 g, 120.4 mmol) was added to a solution of (methoxymethyl)triphenylphosphonium chloride (13.48 g, 39.32 mmol) in tetrahydrofuran (80 mL). The system was stirred for 0.5 h, and then 57c-4 (5.00 g, 19.66 mmol) was added. The reaction system was stirred at 25 °C for 16 h in a nitrogen atmosphere. After the reaction was completed, the reaction solution was directly concentrated at reduced pressure, then diluted with ethyl acetate, washed with water, and dried over anhydrous sodium sulfate. The organic phase was concentrated to give a crude product, which was purified by silica gel column chromatography (PE/EA = 100/1-5/1) to give compound 57c-5 (brown oil, 0.8 g).

Step 6: A diluted hydrochloric acid solution (0.1 M, 28.33 mL) was added to a solution of 57c-5 (0.4 g, 1.42 mmol) in acetone (4 mL), and the system was stirred at 25 °C for 16 h. After the reaction was completed, the reaction solution was directly concentrated at reduced pressure to give a crude product (0.4 g). The crude product was dissolved in methanol (5 mL), and then potassium carbonate (412.03 mg, 2.98 mmol) was added. The system was cooled to 0 °C, and then dimethyl (1-diazo-2-oxopropyl)phosphonate (572.71 mg, 2.98 mmol) was added. Then the system was warmed to room temperature for reaction for another 16 h. After the reaction was completed, the reaction solution was directly concentrated at reduced pressure, then diluted with ethyl acetate, washed three times with water, and dried over anhydrous sodium sulfate. The organic phase was concentrated to give a crude product,
which was purified by silica gel column chromatography (PE/EA = 100/1-20/1) to give compound 57c-6 (colorless oil, 0.2 g, crude product).

Step 7: Compound 57c was prepared with reference to step 1 for intermediate 4b. MS m/z (ESI): 407.2 [M+H]⁺.

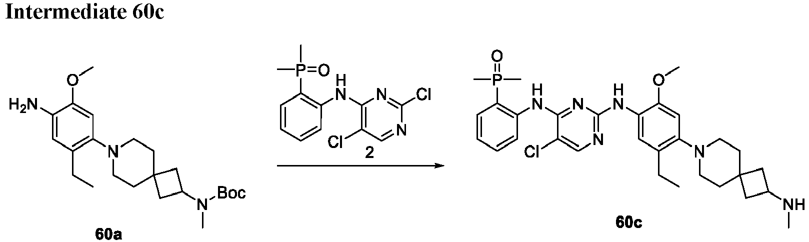

Compound 60c was prepared with reference to step 1 for intermediate 4c. MS m/z (ESI): 583.2[M+H]⁺.

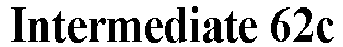

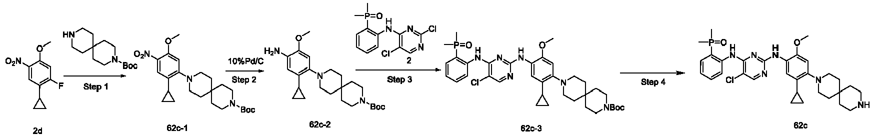

Compound 62c was prepared with reference to steps 1-2 for intermediate 8a as steps 1-2 and with reference to steps 1-2 for intermediate 4c as steps 3-4. MS m/z (ESI): 595.2[M+H]⁺.

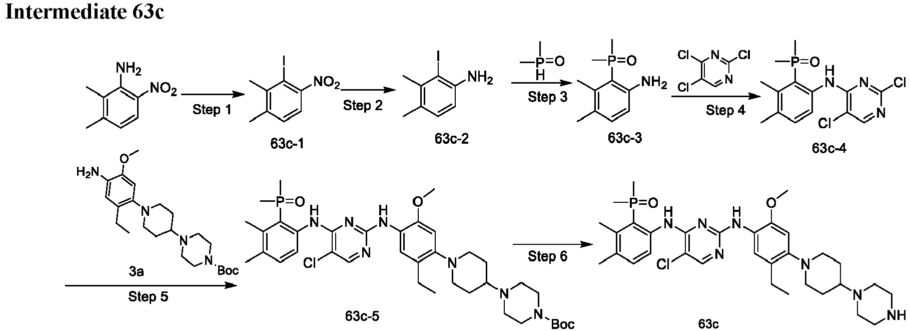

Step 1: 2,3-Dimethyl-6-nitroaniline (5 g, 30.09 mmol), methanesulfonic acid (5.78 g, 60.18 mmol, 4.28 mL), *tert*-butyl nitrite (7.76 g, 75.22 mmol, 8.95 mL), and tetrabutylammonium iodide (16.67 g, 45.13 mmol) were added to acetonitrile (50 mL) at 0 °C in a nitrogen atmosphere, and the reaction system was heated to 25 °C and stirred for 4 h. After the reaction was completed, the reaction system was concentrated at reduced pressure and separated and purified by normal-phase column chromatography (PE/EA = 1/0-5/1) to give compound 63c-1 (brown solid, 2 g). MS m/z (ESI): 278.2[M+H]⁺.

Step 2: Compound 63c-2 was prepared with reference to step 2 for 7a. MS m/z (ESI): 248.2[M+H]⁺.

Step 3: 63c-3 was prepared with reference to step 1 for 12c. MS m/z (ESI): 198.2[M+H]⁺.

Step 4: 63c-3 (1 g, 5.07 mmol), 2,4,5-trichloropyrimidine (930.07 mg, 5.07 mmol), and *N*,*N-*diisopropylethylamine (1.97 g, 15.21 mmol, 2.65 mL) were dissolved in tetrahydrofuran (10 mL), and the reaction system was stirred at 65 °C for 8 h. After the reaction was completed, the reaction solution was concentrated at reduced pressure and separated and purified by reverse-phase chromatography (formic acid system) to give the title compound 63c-4 (pale yellow solid, 300 mg). MS m/z (ESI): 344.1 [M+H]⁺.

Step 5: 63c-5 was prepared with reference to step 3 for 52b. MS m/z (ESI): 726.2 [M+H]⁺.

Step 6: 63c was prepared with reference to step 2 for 3b. MS m/z (ESI): 626.1 [M+H]⁺.

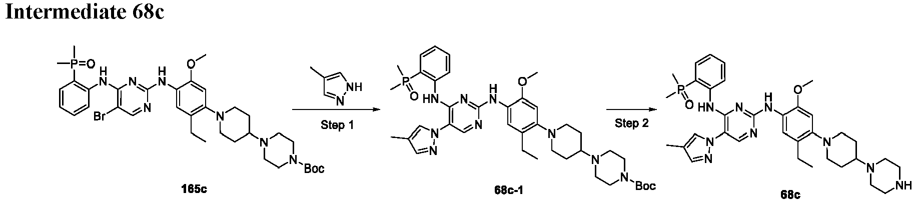

Compound 68c was prepared with reference to the method for intermediate 165a. MS m/z (ESI): 644.3 [M+H]⁺.

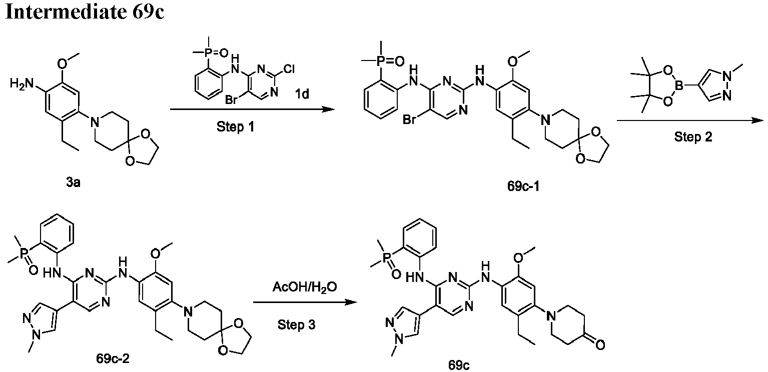

Step 1: Compound 69c-1 was prepared with reference to step 3 for intermediate 1d. MS m/z (ESI): 615.2 [M+H]⁺.

Step 2: Compound 69c-2 was prepared with reference to the method for intermediate 5d. MS m/z (ESI): 618.2 [M+H]⁺.

Step 3: Compound 69c was prepared with reference to step 4 for intermediate 21c. MS m/z (ESI): 574.2 [M+H]⁺.

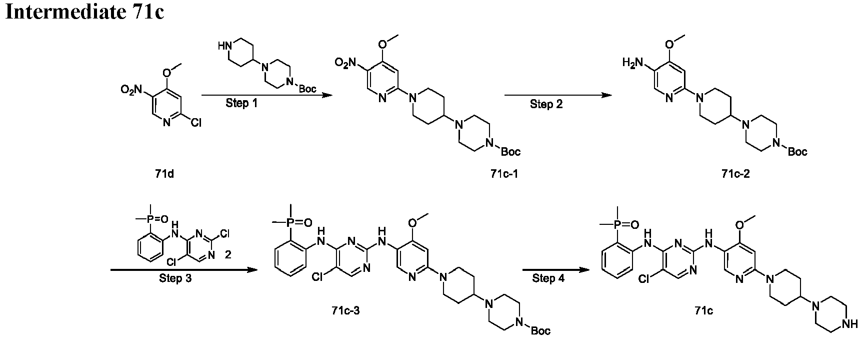

Steps 1-2: Compound 71c-2 was prepared with reference to steps 1-2 for intermediate 3a. MS m/z (ESI): 392.0 [M+H]⁺.

Steps 3-4: Compound 71c was prepared with reference to steps 1-2 for intermediate 4c. MS m/z (ESI): 571.2 [M+H]⁺.

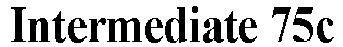

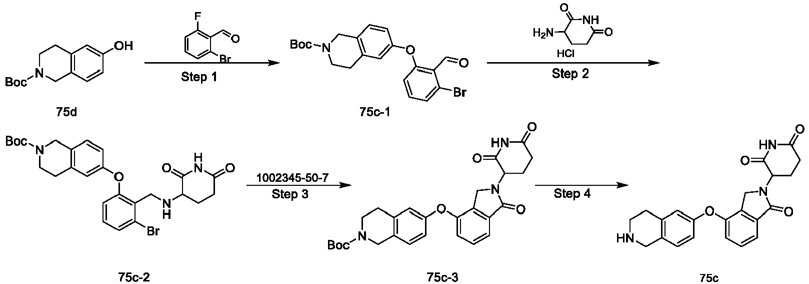

75c was prepared with reference to steps 2-5 for intermediate 120c. MS m/z (ESI): 392.2 [M+H]⁺.

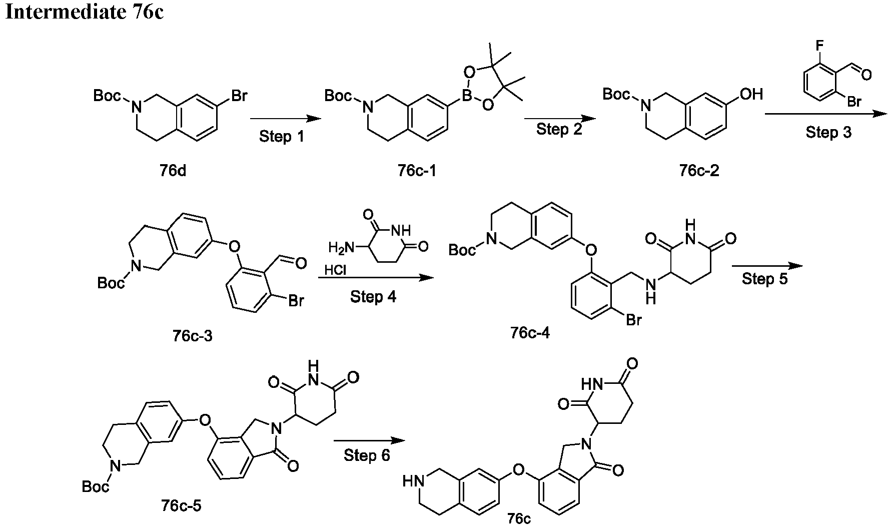

Step 1: 1,1-Bis(diphenylphosphino)ferrocene palladium(II) chloride (585.92 mg, 800.76 µmol), potassium acetate (1.57 g, 16.02 mmol), and bis(pinacolato)diboron (3.05 g, 12.01 mmol) were added to a solution of 76d (2.5 g, 8.01 mmol) in 1,4-dioxane (50 mL), and then the reaction system was stirred at 100 °C for 5 h. After the reaction was completed, the reaction mixture was directly concentrated at reduced pressure to give compound 76c-1 (yellow solid, 5.00 g, crude product). MS m/z (ESI): 304.0 [M-55]⁺.

Step 2: At 25 °C, hydrogen peroxide (15.78 g, 139.17 mmol) was added to a solution of 76c-1 (2.5 g, 6.96 mmol) in dichloromethane (50 mL), and then an aqueous solution (50 mL) of sodium hydroxide (2.78 g, 69.59 mmol) was added. The reaction system was stirred at 25 °C for 15 h. After the reaction was completed, the reaction solution was diluted with water, and then the reaction was quenched with saturated sodium thiosulfate. The mixture was then extracted with EA and dried over anhydrous sodium sulfate, and the organic phase was directly concentrated at reduced pressure to give a crude product. The crude product was purified by column chromatography (PE/EA = 100/1-10/1) to give the title compound 76c-2 (yellow solid, 0.7 g). MS m/z (ESI): 194.1 [M-55]⁺.

Steps 3-6: Compound 76c was prepared with reference to steps 2-5 for 120c. MS m/z (ESI): 392.3 [M+H]⁺.

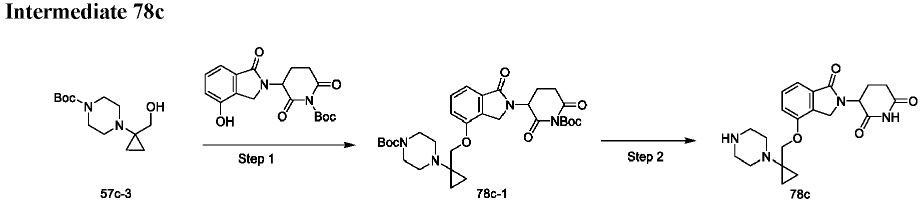

Step 1: 57c-3 (0.4 g, 1.56 mmol), *tert*-butyl 3-(4-hydroxy-1-oxoisoindolin-2-yl)-2,6-dioxopiperidine-1-carboxylate (562.32 mg, 1.56 mmol), azodicarbonamide (806.05 mg, 4.68 mmol), tributylphosphine (947.10 mg, 4.68 mmol) were dissolved in tetrahydrofuran (5 mL), and the reaction system was stirred at 25 °C for 2 h. After the reaction was completed, the reaction mixture was concentrated at reduced pressure, separated and purified by high performance liquid chromatography (hydrochloric acid system), and lyophilized to give 78c-1 (colorless liquid, 400 mg). MS m/z (ESI):599.2 [M+H]⁺.

Step 2: 78c was prepared with reference to step 2 for 3b. MS m/z (ESI): 399.1 [M+H]⁺.

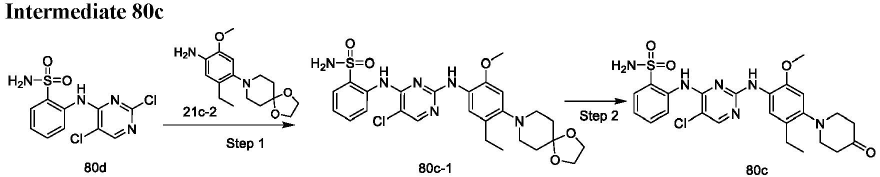

Compound 80c as a yellowish brown solid was prepared with reference to steps 3-4 for intermediate 21c. MS m/z (ESI): 531.7 [M+H]⁺.

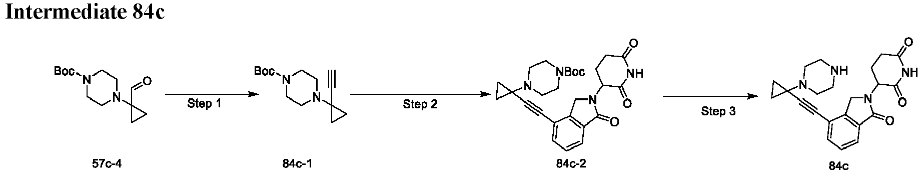

Step 1: At 0 °C, potassium carbonate (434.74 mg, 3.15 mmol) was added to a solution of 57c-4 (0.4 g, 1.57 mmol) in methanol (5 mL), and then dimethyl(1-diazo-2-oxopropyl)phosphonate (604.30 mg, 3.15 mmol) was added. The reaction system was stirred at 25 °C for 16 h in a nitrogen atmosphere. After the reaction was completed, the reaction solution was directly concentrated at reduced pressure, diluted with EA, washed with water, dried over anhydrous sodium sulfate, and concentrated to give a crude product, which was purified by silica gel column chromatography (PE/EA = 100/1-20/1) to give the title compound 84c-1 (brown oil, 0.3 g). Step 2: 84c-2 was prepared with reference to step 1 for 4b. MS m/z (ESI): 493.2 [M+H]⁺.

Step 3: 84c was prepared with reference to step 2 for 3b. MS m/z (ESI): 393.2 [M+H]⁺.

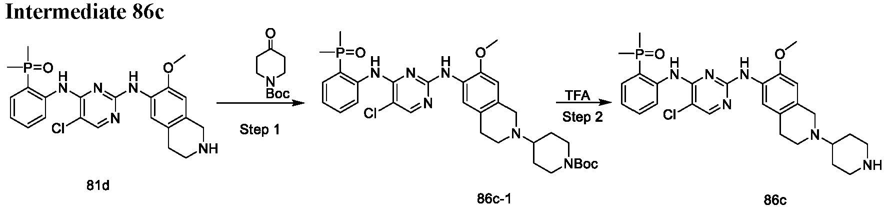

Step 1: At room temperature, HOAc (13 mg, 220 µmol) was added to a solution of compound 81d (50 mg, 110 µmol) and *tert*-butyl 4-oxopiperidine-1-carboxylate (44 mg, 220 µmol) in DCM (5 mL) before TEA (44 mg, 440 µmol) was added. The reaction system was adjusted to a weakly alkaline pH, stirred for 1 h before sodium triacetoxyborohydride (93 mg, 440 µmol) was added, and then stirred for another 12 h for reaction. After the reaction was completed, DCM and H₂O were added to the reaction solution, and the mixture was extracted with DCM. The organic phase was washed with saturated brine and dried over anhydrous sodium sulfate. The crude product was purified by silica gel column chromatography (DCM/MeOH = 100/1-20/1) to give compound 86c-1. MS m/z (ESI): 641.1 [M+H]⁺.

Step 2: Compound 86c-1 (50 mg, 0.08 mmol) and solvent DCM (4 mL) were added to a 25 mL reaction flask, and then TFA (2 mL) was added. The system was stirred at 20 °C for 1 h in a nitrogen atmosphere. After the reaction was completed, the reaction solution was concentrated, diluted with DCM, washed with saturated sodium bicarbonate until pH = 7-8, and then extracted with DCM. The organic phase was washed with saturated brine, dried over anhydrous sodium sulfate, and concentrated to give compound 86c. MS m/z (ESI): 541.1 [M+H]⁺.

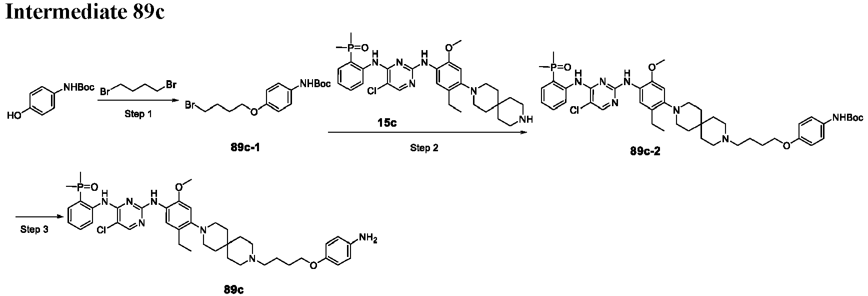

Step 1: tert-Butyl (4-hydroxyphenyl)carbamate (1000 mg, 4.78 mmol), 1,4-dibromobutane (2064 mg, 9.56 mmol), K₂CO₃ (1321 mg, 9.56 mmol), and anhydrous CH₃CN (30 mL) were added to a single-necked flask. In a nitrogen atmosphere, the reaction system was heated to 90 °C and stirred for 16 h. After the reaction was completed, water was added to quench the reaction. The mixture was extracted with EA, and the organic phases were combined, then washed with saturated brine, and dried over anhydrous sodium sulfate. The crude product was subjected to normal-phase column chromatography (EA/PE = 0-20%) to give compound 89c-1 (pale yellow solid, 1300 mg). MS m/z (ESI): 344.1 [M+H]⁺

Step 2: Compound 89c-1 (50 mg, 0.15 mmol), compound 15c (85 mg, 0.15 mmol), K₂CO₃ (41 mg, 0.30 mmol), NaI (22 mg, 0.15 mmol), and anhydrous DMF (3 mL) were added to the reaction flask, and then the system was heated to 60 °C and stirred for 16 h. After the reaction was completed, the reaction mixture was filtered. The filtrate was concentrated at reduced pressure, and then the crude product was separated by normal-phase column chromatography (MeOH/DCM = 0-5%) to give intermediate 89c-2 (pale yellow solid, 45 mg). MS m/z (ESI): 846.1 [M+H]⁺

Step 3: Crude compound 89c was prepared with reference to step 2 for intermediate 3b. MS m/z (ESI): 746 2[M+H]⁺.

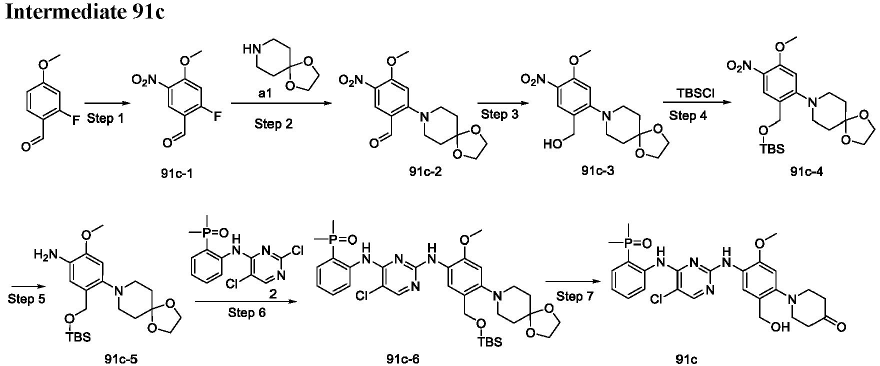

Step 1: Compound 2-fluoro-4-methoxybenzaldehyde (1 g, 6.5 mmol) was dissolved in concentrated sulfuric acid (5 mL). The system was cooled to 0 °C, and concentrated nitric acid (441 mg, 7 mmol) was dropwise added. The mixture was then stirred at 20 °C for 2 h and poured into ice water, and a large amount of solid was precipitated. The solid was then collected by filtration at reduced pressure and dried to give compound 91c-1 (pale yellow solid, 600 mg). MS m/z (ESI): 200.1 [M+H]⁺.

Step 2: Compound 91c-1 (300 mg, 1.5 mmol) was dissolved in DMF (10 mL), and compound a1 (300 mg, 2 mmol) and potassium carbonate (414 mg, 3 mmol) were sequentially added. The system was then stirred at 85 °C for 16 h. After the reaction was completed, water was added to quench the reaction. The precipitated solid was collected by filtration at reduced pressure and dried to give compound 91c-2 (pale yellow solid, 320 mg). MS m/z (ESI): 323.2 [M+H]⁺.

Step 3: Compound 91c-2 (320 mg, 0.98 mmol) was dissolved in MeOH (10 mL), and the system was cooled to 0 °C. Sodium borohydride (137.8 mg, 4.9 mmol) was added, and then the system was warmed to room temperature and stirred for 3 h. After the reaction was completed, water was added to quench the reaction, and the mixture was extracted with dichloromethane, dried over anhydrous sodium sulfate, filtered, and then concentrated at reduced pressure to give compound 91c-3 (yellow oil, 265 mg). MS m/z (ESI): 325.1 [M+H]⁺. Step 4: Compound 91c-3 (265 mg, 0.82 mmol) and imidazole (111 mg, 1.63 mmol) were dissolved in DCM (mL), and the system was cooled to 0 °C. TBS-Cl (244.5 mg, 1.63 mmol) was added, and then the system was warmed to room temperature and stirred for 3 h. After the reaction was completed, water was added to quench the reaction, and the mixture was extracted with dichloromethane, dried over anhydrous sodium sulfate, filtered, then concentrated at reduced pressure, and purified by normal-phase column chromatography (dichloromethane:methanol = 10:1) to give compound 91c-4 (yellow oil, 330 mg). MS m/z (ESI): 439.4 [M+H]⁺.

Step 5: Compound 91c-5 was prepared with reference to step 2 for intermediate 3a. MS m/z (ESI): 409.1 [M+H]⁺.

Step 6: Compound 91c-6 was prepared with reference to step 1 for intermediate 1c. MS m/z (ESI): 688.3 [M+H]⁺.

Step 7: Compound 91c-6 (100 mg, 0.15 mmol) was dissolved in THF (2 mL), and 6 N HCl (0.25 mL, 1.5 mmol) was added. The reaction system was heated to 70 °C, stirred for 3 h, cooled to room temperature, adjusted to pH 7 by adding a saturated aqueous sodium carbonate solution, and extracted with dichloromethane. The organic layer was washed with saturated brine, dried over anhydrous sodium sulfate, filtered, and concentrated by rotary evaporation. The crude product was purified by normal-phase column chromatography (dichloromethane:methanol = 10:1) to give compound 91c (yellow oil). MS m/z (ESI): 530.3 [M+H]⁺.

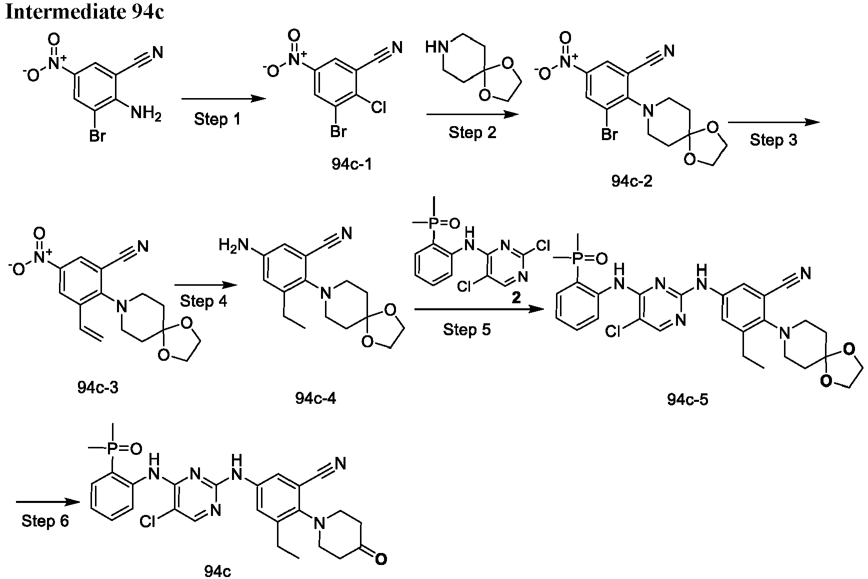

Step 1: 2-Amino-3-bromo-5-nitrobenzonitrile (10 g, 41.32 mmol) and sodium nitrite (7.13 g, 103.29 mmol) were added to hydrochloric acid (100 mL) at 0 °C, and the system was purged 3 times with nitrogen. After 1 h, cuprous chloride (6.14 g, 61.98 mmol) was added, and the reaction system was stirred at 0-25 °C for 11 h. After the reaction was completed, the reaction mixture was diluted with water and extracted with ethyl acetate. The organic layers were combined, washed with saturated brine, dried over anhydrous sodium sulfate, filtered, and concentrated to give compound 94c-1 (yellow solid, 10.0 g).

Step 2: Compound 94c-2 was prepared with reference to step 1 for intermediate 21c. MS m/z (ESI): 368.1 [M+H]⁺.

Step 3: Compound 94c-3 was prepared with reference to the method for intermediate 5d. MS m/z (ESI): 316.0 [M+H]⁺.

Step 4: 94c-3 (600 mg, 1.90 mmol) was added to tetrahydrofuran (6 mL), and then wet palladium on carbon (300 mg, 10% purity) was added. The reaction system was stirred at 25 °C for 0.5 h in a hydrogen atmosphere. After the reaction was completed, the reaction mixture was filtered and concentrated to give the title compound 94c-4 (yellow solid, 300 mg), which was directly used in the next step. MS m/z (ESI): 288.0 [M+H]⁺.

Steps 5-6: Compound 94c was prepared with reference to steps 3-4 for intermediate 21c. MS m/z (ESI): 523.0 [M+H]⁺.

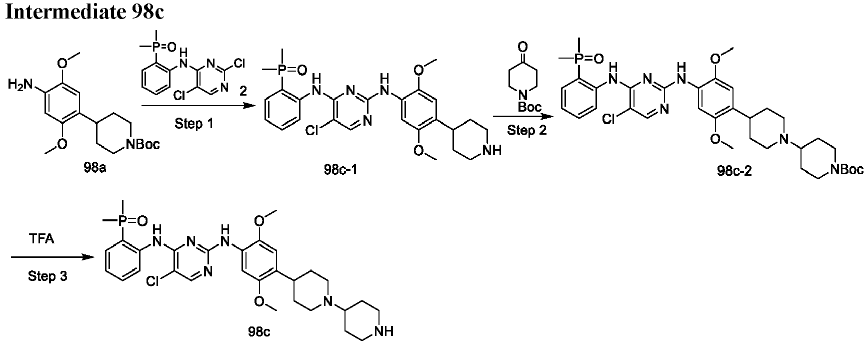

Compound 98c was prepared with reference to the method for intermediate 4c as step 1 and step 3 and with reference to the method for compound E67 as step 2. MS m/z (ESI): 599.9 [M+H]⁺.

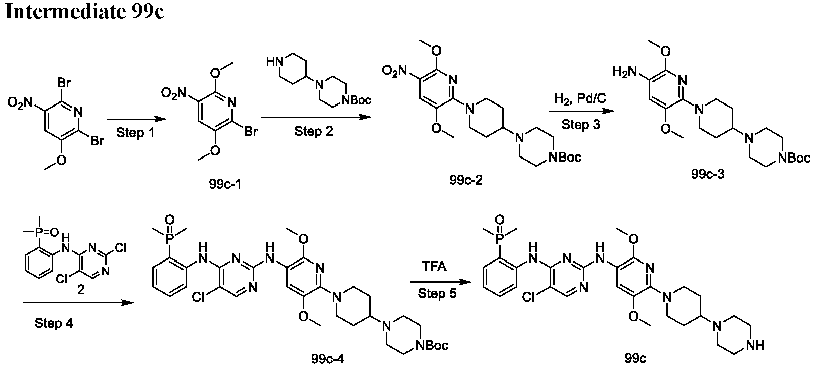

Step 1: Compound (50 mg, 0.155 mmol) was dissolved in MeOH (6 mL). In an ice bath, concentrated NaOMe (50 mg, 0.155 mmol) was added slowly. The system was then stirred at 0 °C for 12 h in a nitrogen atmosphere. After the reaction was completed, the reaction solution was concentrated. Water was added to quench the reaction, and the mixture was extracted with ethyl acetate. The organic phase was washed with saturated brine, dried over anhydrous sodium sulfate, concentrated, and subjected to normal-phase column chromatography (PE/EtOAc = 0-20%) to give compound 90c-1 (yellow solid, 60 mg). MS m/z (ESI): 263.2 [M+H]⁺.

Step 2: Compound 99c-2 was prepared with reference to step 1 for intermediate 7a. MS m/z (ESI): 452.1 [M+H]⁺.

Step 3: Compound 99c-3 was prepared with reference to step 2 for intermediate 3a. MS m/z (ESI): 422.2[M+H]⁺.

Steps 4-5: Compound 99c was prepared with reference to steps 1-2 for intermediate 4c. MS m/z (ESI): 601.9 [M+H]⁺.

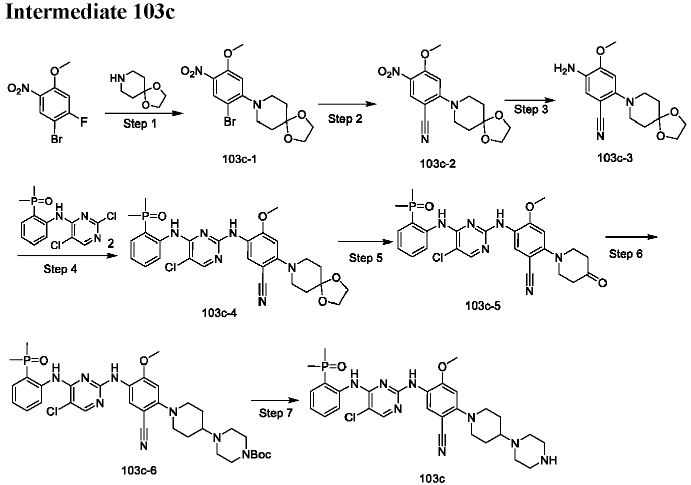

Step 1: Potassium carbonate (3.32 g, 24.00 mmol, 2 eq) and 1,4-dioxa-8-azaspiro[4.5]decane (1.89 g, 13.20 mmol, 1.69 mL) were added to a solution of 1-bromo-2-fluoro-4-methoxy-5-nitrobenzene (3 g, 12.00 mmol) in DMF (20 mL), and then the reaction system was stirred at 60 °C for 1 h. After the reaction was completed, the reaction solution was added to 300 mL of ice water at 0 °C, and a solid was precipitated. The solid was then collected by filtration and dried to give compound 103c-1 (yellow solid, 4.00 g, crude product). MS m/z (ESI): 372.8 [M+H]⁺.

Step 2: 103c-1 (6.39 g, 17.12 mmol) and cuprous cyanide (7.67 g, 85.61 mmol, 18.70 mL) were added to DMF (50 mL), and the reaction system was stirred in an autoclave at 140 °C for 72 h. After the reaction was completed, the reaction mixture was diluted with water and then extracted with ethyl acetate. The organic layers were combined and dried over anhydrous sodium sulfate. The crude product was then filtered, concentrated, and separated and purified by silica gel column chromatography (PE:EA = 5:1-2:1) to give the title compound 103c-2 (yellow solid, 2.80). MS m/z (ESI): 320.1 [M+H]⁺.

Step 3: Compound 103c-3 was prepared with reference to step 2 for intermediate 3a. MS m/z (ESI): 290.1 [M+H]⁺.

Steps 4-5: Compound 103c-5 was prepared with reference to steps 3-4 for intermediate 21c. MS m/z (ESI): 525.1 [M+H]⁺.

Step 6: Compound 103c-6 was prepared with reference to the method for reference compound C-6. MS m/z (ESI): 695.3 [M+H]⁺.

Step 7: Compound 103c was prepared with reference to step 2 for intermediate 3b. MS m/z (ESI): 595.3 [M+H]⁺.

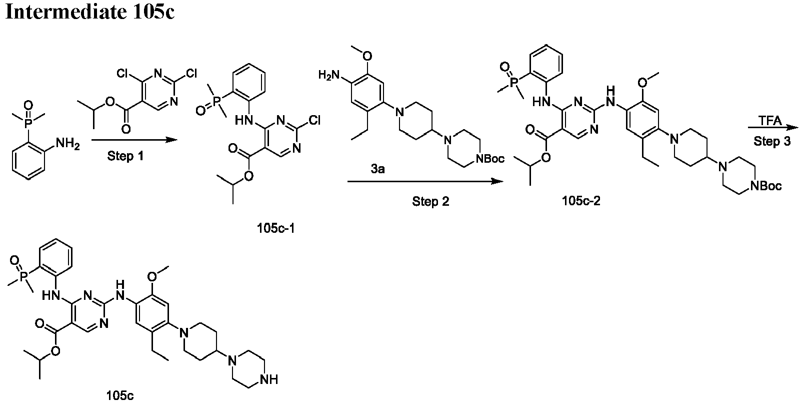

Compound 105c was prepared with reference to the method for intermediate 1d as step 1 and with reference to the method for intermediate 4c as steps 2-3. MS m/z (ESI): 650.3 [M+H]⁺.

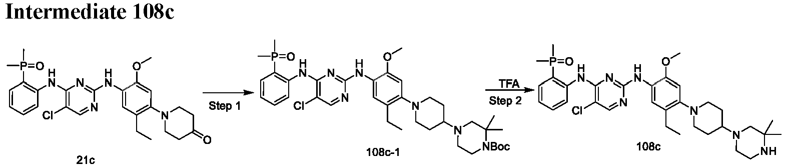

Compound 108c was prepared with reference to the method for intermediate 86c. MS m/z (ESI): 626.4 [M+H]⁺.

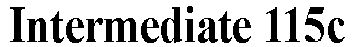

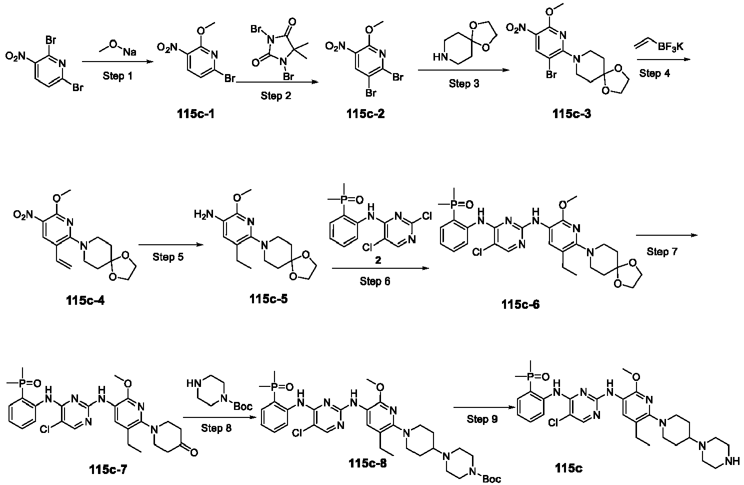

Step 1: Compound 2,6-dibromo-3-nitropyridine (3000 mg, 10.64 mmol) and anhydrous MeOH (30 mL) were added to a three-necked reaction flask. In a nitrogen atmosphere and in an ice-water bath, NaH (468 mg, 11.70 mmol, 60% wt) was added in portions. After the addition, the system was stirred in the ice-water bath for another 0.5 h before CH₃ONa (575 mg, 10.64 mmol) was added in portions. The system was then stirred in the ice-water bath for another 3 h. After the reaction was completed, saturated NH₄Cl was added to the reaction solution to quench the reaction. The mixture was then extracted with EA, and the organic phases were combined, washed with water, washed with saturated brine, dried over anhydrous Na₂SO₄, and concentrated by rotary evaporation at reduced pressure. The crude product was separated by normal-phase column chromatography (EA/PE = 0-15%) to give product 115c-1 (pale yellow solid, 2200 mg). MS m/z (ESI): 233.1 [M+H]⁺.

Step 2: Compound 115c-1 (2200 mg, 9.44 mmol) and anhydrous TFA (20 mL) were added to a single-necked flask before dibromohydantoin (3240 mg, 11.33 mmol) was added. After the addition, the system was stirred at room temperature for 16 h. After the reaction was completed, the reaction solvent was removed at reduced pressure, and the crude product was triturated with PE/EA (50:1). The triturate was filtered at reduced pressure and washed with PE. The filter cake was dried by rotary evaporation at reduced pressure to give crude compound 115c-2 (pale yellow solid, 2100 mg), which was directly used in the next step. MS m/z (ESI): 311.1 [M+H]⁺.

Step 3: Crude compound 115c-3 was prepared with reference to step 1 for intermediate 21c. MS m/z (ESI): 374.3 [M+H]⁺.

Step 4: Crude compound 115c-4 was prepared with reference to the method for intermediate 5d.MS m/z (ESI): 322.5 [M+H]⁺.

Step 5: Crude compound 115c-5 was prepared with reference to step 2 for intermediate 3a.MS m/z (ESI): 294.5 [M+H]⁺.

Step 6: Compound 115c-6 was prepared with reference to step 1 for intermediate 1c.MS m/z (ESI): 573.8 [M+H]⁺.

Step 7: Compound 115c-7 was prepared with reference to step 4 for intermediate 21c.MS m/z (ESI): 529.8 [M+H]⁺.

Step 8: Product 115c-8 was prepared with reference to compound E67. MS m/z (ESI): 700.0 [M+H]⁺.

Step 9: Crude compound 115c was prepared with reference to step 2 for intermediate 3b. MS m/z (ESI): 599.8 [M+H]⁺.

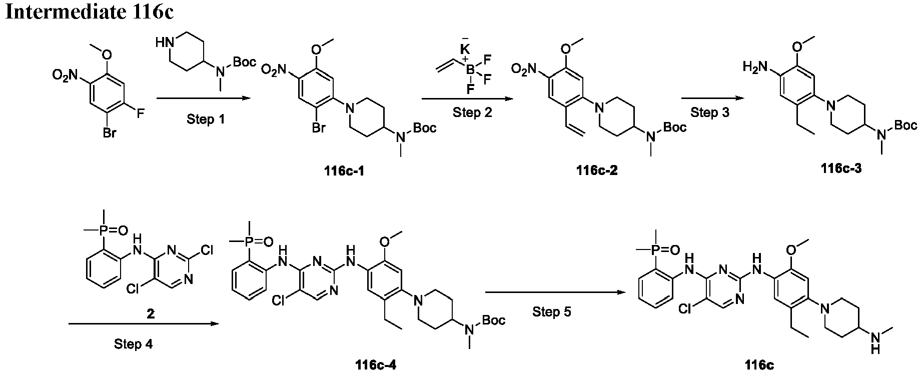

Step 1: Compound 116c-1 was prepared with reference to the method of step 1 for intermediate 3a. MS m/z (ESI): 444.1 [M+H]⁺.

Step 2: Compound 116c-2 was prepared with reference to the method for intermediate 5d. MS m/z (ESI): 392.1 [M+H]⁺.

Step 3: Compound 116c-3 was prepared with reference to the method of step 2 for intermediate 3a. MS m/z (ESI): 364.3 [M+H]⁺.

Steps 4-5: Compound 116c was prepared with reference to the methods of steps 1-2 for intermediate 1c. MS m/z (ESI): 543.7 [M+H]⁺.

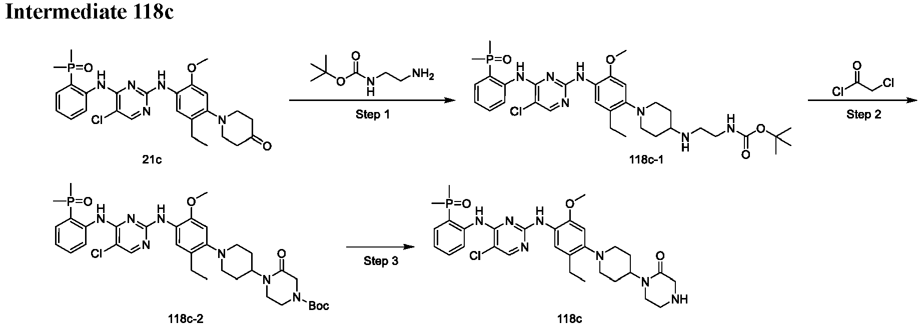

Step 1: Acetic acid (3.79 mL, 1 M in DCM) and TEA (1.1 mL, 7.58 mmol) were added to a solution of compound 21c (1 g, 1.89 mmol) and *tert*-butyl(2-aminoethyl)carbamate (607 mg, 3.79 mmol) in DCE (5 mL), and the reaction solution was stirred at 25 °C for 2 h. Then sodium triacetoxyborohydride (1.61 g, 7.58 mmol) was added, and the reaction system was stirred at 25 °C for 16 h. Saturated NaHCOs was added, and the mixture was extracted with DCM, dried, concentrated, and subjected to column chromatography (MeOH/DCM = ~10%) to give compound 118c-1 (white solid, 1.2 g). MS m/z (ESI): 673.4 [M+H]⁺.

Step 2: Compound 118c-1 (1.3 g, 1.93 mmol) was dissolved in DCM (50 mL), and then Et₃N (0.84 mL, 5.8 mmol) and chloroacetyl chloride (262 mg, 2.32 mmol) were added. The reaction system was stirred at room temperature for 16 h. After the reaction was completed, the reaction was quenched with a saturated sodium bicarbonate solution, and the mixture was extracted with DCM. The organic phase was washed with brine, dried, filtered, and concentrated to give a crude product. The crude product was dissolved in DMF (20 mL), and NaH (387 mg, 9.67 mmol) was added in portions at room temperature. The reaction system was stirred at room temperature for 16 h. After the reaction was completed, the reaction was quenched with a saturated sodium bicarbonate solution. The mixture was extracted with DCM, washed with brine, dried, filtered, and concentrated to give a crude product, which was purified by column chromatography (MeOH/DCM = ~8%) to give compound 118c-2 (white solid, 0.65 g). MS m/z (ESI): 713.3

Step 3: Compound 118c was prepared with reference to step 2 for intermediate 3b. MS m/z (ESI): 613.2 [M+H]⁺.

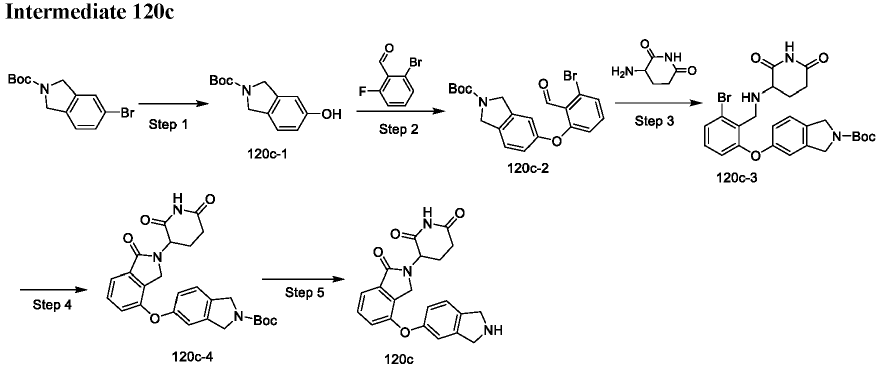

Step 1: Methanesulfonato(2-di-*tert*-butylphosphino-2,4,6-triisopropyl-1,1-biphenyl)(2-amino-1,1-biphenyl-2-yl)palladium (II) (0.52 g, 0.65 mmol) and potassium hydroxide (0.92 g, 16.42 mmol) were added to a mixed solution of tert-butyl 5-bromoisoindoline-2-carboxylate (2.5 g, 8.43 mmol) in 1,4-dioxane (50 mL) and water (20 mL), and then the reaction system was stirred at 80 °C for 18 h. After the reaction was completed, the reaction mixture was diluted with ethyl acetate and washed with water, and then the organic phase was dried over anhydrous sodium sulfate and concentrated at reduced pressure to give compound 120c-1 (yellow solid, 1.6 g, crude product). MS m/z (ESI): 180.0 [M-55]⁺.

Step 2: Potassium carbonate (822.38 mg, 5.95 mmol) and 2-bromo-6-fluorobenzaldehyde (603.99 mg, 2.98 mmol) were added to a solution of 120c-1 (0.7 g, 2.98 mmol) in DMF (4 mL), and then the reaction system was stirred at 60 °C for 2 h. After the reaction was completed, the reaction solution was extracted with water and ethyl acetate and separated into layers. The organic layer was washed with brine, dried over anhydrous sodium sulfate, filtered, and concentrated at reduced pressure to give compound 120c-2 (brown oil, 0.9 g, crude product). MS m/z (ESI): 362.3 [M-55]⁺.

Step 3: Triethylamine (302.40 mg, 2.99 mmol) was added to a solution of 120c-2 (0.5 g, 1.20 mmol) in methanol (5 mL), and then the reaction system was stirred at 25 °C for 0.5 h. Acetic acid (100.49 mg, 1.67 mmol) and 3-aminopiperidine-2,6-dione (196.75 mg, 1.20 mmol) were then added, and the system was stirred at 25 °C for another 2 h. Sodium cyanoborohydride (225.35 mg, 3.59 mmol) was then added, and the system was stirred at 25 °C for another 1 h. The reaction solution was concentrated at reduced pressure and then separated and purified by a silica gel plate (dichloromethane:methanol = 10:1) to give the title compound 120-3 (colorless oil, 0.6 g, crude product), which was used directly in the next step. MS m/z (ESI): 530.3 [M+H]⁺. Step 4: 120c-3 (0.5 g, 942.67 µmol) and 1,3-bis(dicyclohexylphosphonium)propane bis(tetrafluoroborate) (57.72 mg, 94.27 µmol) were dissolved in DMF (20 mL), and then palladium acetate (21.16 mg, 94.27 µmol) and potassium carbonate (195.43 mg, 1.41 mmol) were added. In a carbon monoxide atmosphere, the reaction system was stirred at 80 °C for 24 h. After the reaction was completed, the reaction mixture was extracted with water and ethyl acetate, and the organic phase was dried and concentrated at reduced pressure to give the title compound 120c-4 (green solid, 600 mg, crude product), which was used directly in the next step. MS m/z (ESI): 378.1 [M+H-100]⁺.

Step 5: A solution of 120c-4 (290.83 mg, 609.06 µmol) in hydrogen chloride in ethyl acetate (4 M, 10 mL) was stirred at 25 °C for reaction for 2 h. After the reaction was completed, the reaction solution was directly concentrated to give the title compound 120c (green solid, 200 mg, HCl salt). MS m/z (ESI): 378.3 [M+H]⁺.

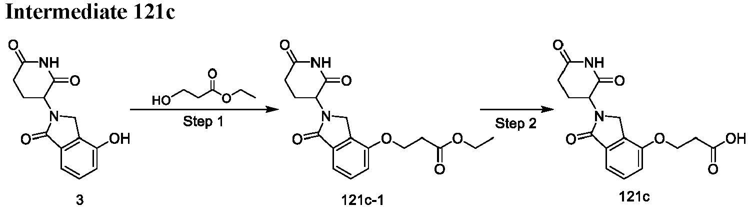

Step 1: In a nitrogen atmosphere, ethyl 3-hydroxy-propionate (90.78 mg, 768.51 µmol) was added to a solution of compound 3 (100 mg, 384.25 µmol), azodicarbonamide (198.49 mg, 1.15 mmol), and tributylphosphine (233.22 mg, 1.15 mmol, 284.42 µL) in tetrahydrofuran (3 mL), and then the reaction system was stirred at 25 °C for 10 h. After the reaction was completed, the reaction mixture was directly concentrated at reduced pressure to give a crude product. The crude product was separated and purified by thin-layer chromatography to give compound 121c-1 (white solid, 46 mg). MS m/z (ESI): 361.2 [M+H]⁺.

Step 2: A solution of hydrogen chloride in dioxane (4 M, 2 mL) was added to a solution of 121c-1 (40 mg, 111.00 µmol) in water (0.1 mL), and then the reaction system was stirred at 80 °C for 8 h. After the reaction was completed, the reaction mixture was directly concentrated at reduced pressure to give compound 121c (white solid, 24 mg). MS m/z (ESI): 333.1 [M+H]⁺.

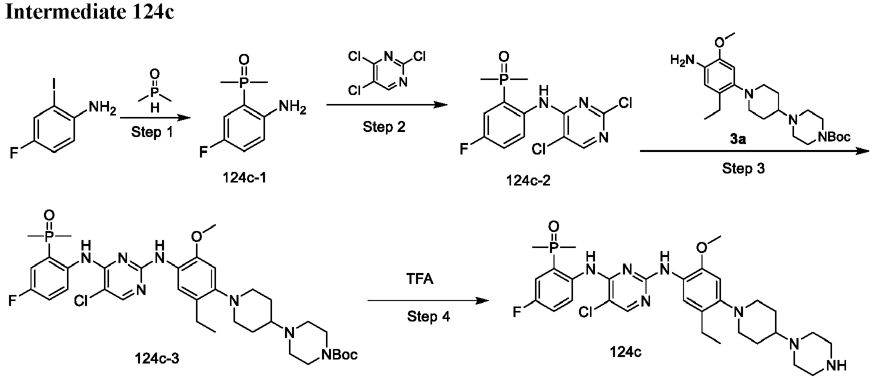

Compound 124c was prepared with reference to step 1 for intermediate 114d as step 1, with reference to the method for intermediate 1d as step 2, and with reference to the method for intermediate 4c as steps 3-4. MS m/z (ESI): 616.2 [M+H]⁺.

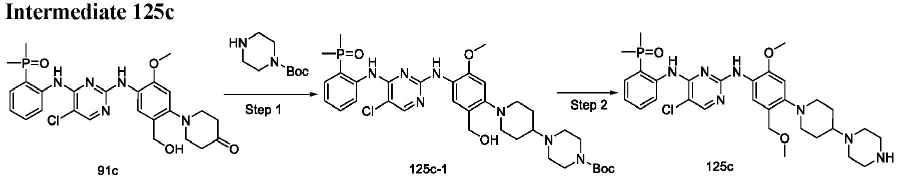

Step 1: Compound 125c-1 was prepared with reference to compound C-6. MS m/z (ESI): 601.1 [M+H]⁺.

Step 2: Compound 125c-1 (100 mg, 0.19 mmol) was dissolved in methanol (4 mL), and 4 M HCl/1,4-dioxane (0.5 mL, 2 mmol) was added. The system was heated to reflux for reaction for 4 h, then adjusted to pH 7 with a saturated aqueous sodium bicarbonate solution, and extracted with DCM. The organic phase was dried over anhydrous sodium sulfate, concentrated, and purified by column chromatography (MeOH/DCM = ~10%) to give 125c (white solid, 60 mg). MS m/z (ESI): 614.1 [M+H]⁺.

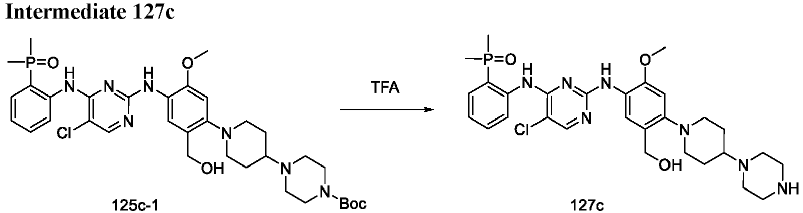

Compound 127c was prepared with reference to step 2 for intermediate 3b. MS m/z (ESI): 600.2 [M+H]⁺.

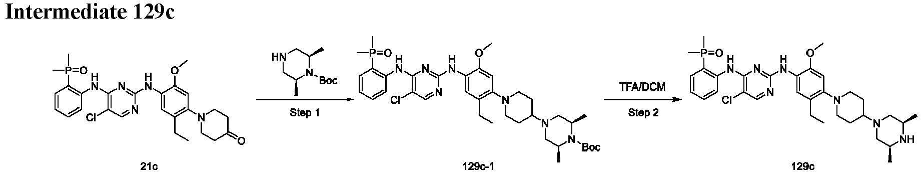

Compound 129c was prepared with reference to the method for E67 as step 1 and with reference to step 2 for intermediate 15c as step 2. MS m/z (ESI): 626.9 [M+H]⁺.

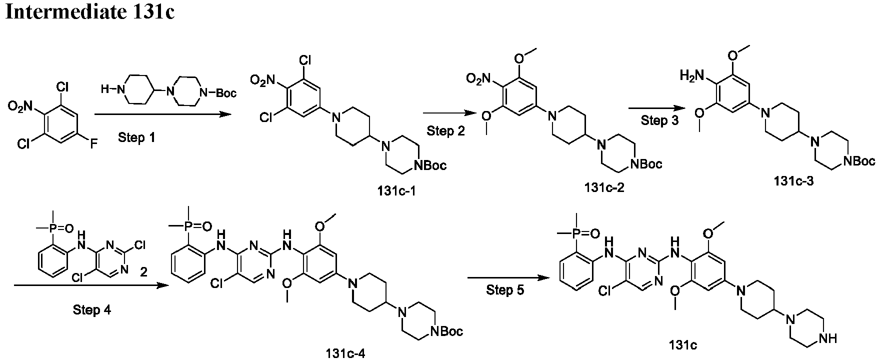

Step 1: Compound 131c-1 was prepared with reference to step 1 for intermediate 7a. MS m/z (ESI): 459.1 [M+H]⁺.

Step 2: 131c-1 (2.0 g, 4.35 mmol) was added to a mixed solution of methanol (6 mL) and 1,4-dioxane (30 mL), and then sodium methoxide (2.35 g, 43.54 mmol) and cuprous iodide (829.19 mg, 4.35 mmol) were added. The reaction system was stirred at 100 °C for 2 h in a nitrogen atmosphere. After the reaction was completed, the reaction mixture was diluted with water and then extracted with dichloromethane. The organic layers were combined, dried over anhydrous sodium sulfate, filtered, and concentrated to give a crude product. The crude product was separated and purified by silica gel column chromatography (PE/EA = 10:1-1:1) to give the title compound 131c-2. MS m/z (ESI): 451.1 [M+H]⁺.

Step 3: Compound 131c-3 was prepared with reference to step 2 for intermediate 3a. MS m/z (ESI): 421.1 [M+H]⁺.

Step 4: Compound 131c-4 was prepared with reference to step 1 for intermediate 4c. MS m/z (ESI): 700.3 [M+H]⁺.

Step 5: Compound 131c was prepared with reference to step 2 for intermediate 3b. MS m/z (ESI): 600.3 [M+H]⁺.

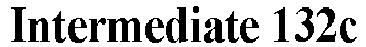

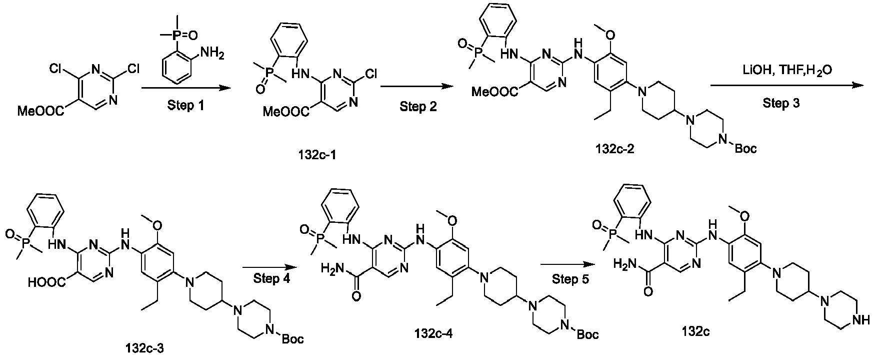

Step 1: Compound 132c-1 was prepared with reference to the method for intermediate 1d. MS m/z (ESI): 340.1 [M+H]⁺.

Step 2: Compound 132c-2 was prepared with reference to step 1 for intermediate 4c. MS m/z (ESI): 722.4 [M+H]⁺.

Step 3: Lithium hydroxide monohydrate (348.78 mg, 8.31 mmol) was added to a mixed solution of 132c-2 (1.5 g, 2.08 mmol) in tetrahydrofuran (15 mL), methanol (15 mL), and water (1.5 mL), and then the reaction system was stirred at 25 °C for 16 h in a nitrogen atmosphere. After the reaction was completed, the reaction solution was concentrated to remove the solvent. The residue was adjusted to pH 3-5 with a 1 M aqueous hydrochloric acid solution, and then the mixture was extracted with dichloromethane and separated into layers. The organic layers were combined, dried over anhydrous sodium sulfate, and concentrated at reduced pressure to give compound 132c-3 (blackish brown solid, 1.0 g). MS m/z (ESI): 708.3 [M+H]⁺.

Step 4: HATU (128.93 mg, 339.08 µmol), DIEA (109.56 mg, 847.70 µmol), and ammonium chloride (109.56 mg, 847.70 µmol) were added sequentially to a solution of 132c-3 (200 mg, 282.57 µmol) in DMF (5 mL), and then the reaction system was stirred at 25 °C for 3 h in a nitrogen atmosphere. After the reaction was completed, the reaction mixture was filtered to give a crude product. The crude product was separated and purified by reverse-phase chromatography to give the title compound 132C-4 (yellow solid, 100 mg). MS m/z (ESI): 707.4 [M+H]⁺.

Step 5: Compound 132c was prepared with reference to step 2 for intermediate 3b. MS m/z (ESI): 607.4 [M+H]⁺.

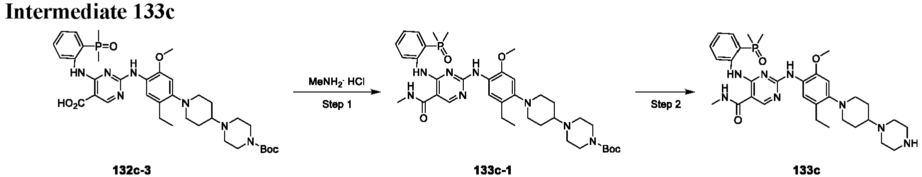

Step 1: Compound 132c-3 (150 mg, 0.22 mmol) and DMF (2 mL) were added to a single-necked flask, then HATU (120.87 mg, 0.32 mmol) and DIPEA (164.34 mg, 1.27 mmol) were added, and finally methylamine hydrochloride (42.93 mg, 0.64 mmol) was added. The reaction system was stirred at 25 °C for 12 h. After the reaction was completed, the reaction mixture was diluted with water and then extracted with DCM. The organic layers were combined, dried over anhydrous sodium sulfate, filtered, and concentrated. The crude product was separated and purified by a silica gel plate (dichloromethane:methanol = 10:1) to give the title compound 133c-1 (123 mg, crude product). MS m/z (ESI): 721.4 [M+H]⁺.

Step 2: Compound 133c was prepared with reference to step 2 for intermediate 1c. MS m/z (ESI): 621.4 [M+H]⁺.

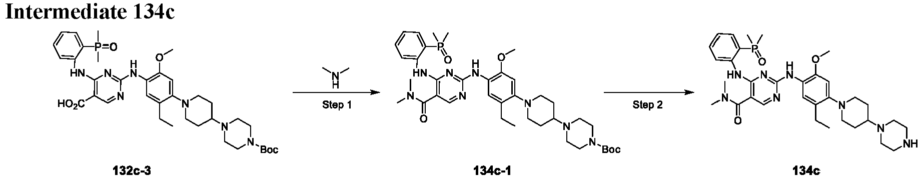

Compound 134c was prepared with reference to the method for intermediate 133c. MS m/z (ESI): 635.4 [M+H]⁺.

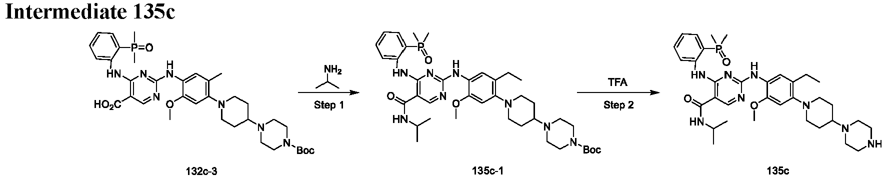

Compound 135c was prepared with reference to the method for intermediate 133c. MS m/z (ESI): 649.4 [M+H]⁺.

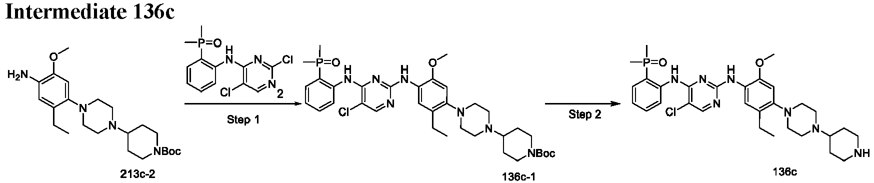

Compound 28c was prepared with reference to the method for intermediate 4c. LCMS: m/z (ESI): 598.3[M+H]⁺.

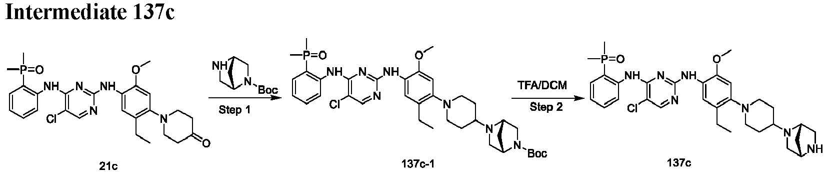

Compound 137c was prepared with reference to the method for intermediate 86c. MS m/z (ESI): 610.9 [M+H]⁺.

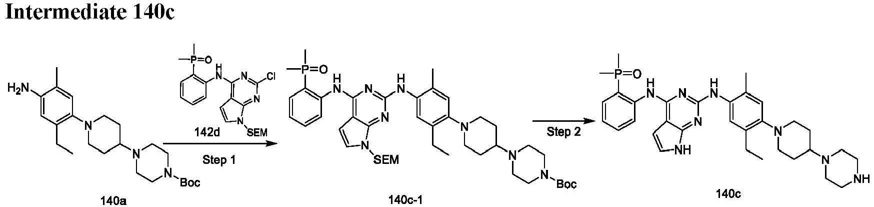

Title compound 140c was prepared with reference to steps 5 and 6 for intermediate 154c. MS m/z (ESI): 587.2 [M+H]⁺.

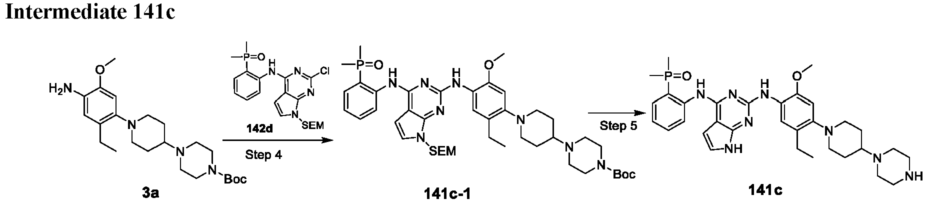

Compound 141c was prepared with reference to steps 5-6 for intermediate 154c. MS m/z (ESI): 603.3 [M+H]⁺.

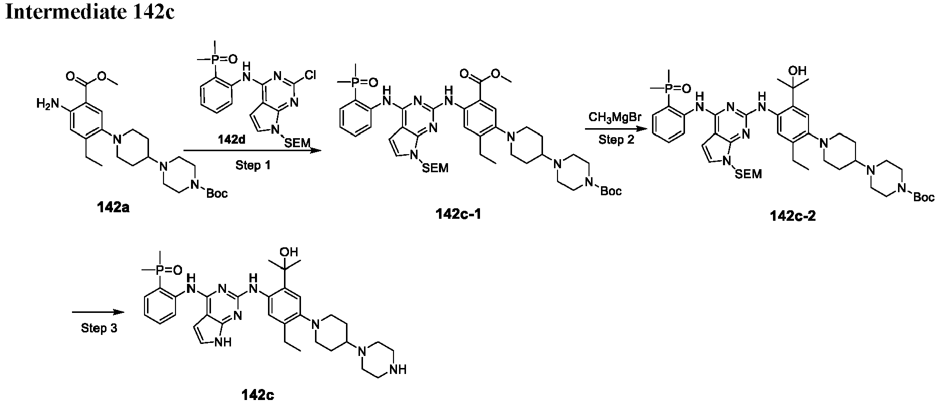

Step 1: Compound 142c-1 was prepared with reference to step 5 for intermediate 154c. MS m/z (ESI): 861.3 [M+H] ⁺.

Step 2: Compound 142c-1 (650 mg, 0.75 mmol) and anhydrous THF (10 mL) were added to a three-necked flask. In a nitrogen atmosphere and in an ice-water bath, methylmagnesium bromide (1 M, 8 mL) was slowly and dropwise added. After the dropwise addition, the reaction solution was stirred at 0 °C for 0.5 h, and then warmed to room temperature and stirred for 6 h. After the reaction was completed, water was added to the reaction solution to quench the reaction. The mixture was extracted with ethyl acetate, and the organic phase was dried over anhydrous sodium sulfate, filtered, and concentrated by rotary evaporation. The crude product was separated by column chromatography to give compound 142c-2 (pale yellow solid, 630 mg). MS m/z (ESI): 861.7 [M+H] ⁺.

Step 3: Compound 142c was prepared with reference to step 6 for intermediate 154c. MS m/z (ESI): 653.6 [M+Na] ⁺.

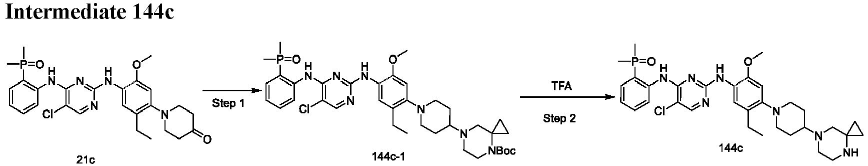

Compound 144c was prepared with reference to the method for intermediate 86c. MS m/z (ESI): 624.8 [M+H]⁺.

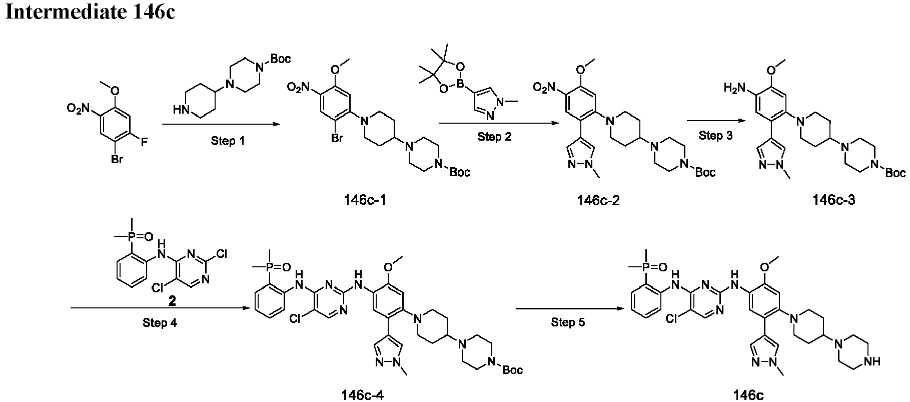

Compound 146c was prepared with reference to step 1 for intermediate 3a as step 1, with reference to the method for 5d as step 2, with reference to step 2 for 3a as step 3, and with reference to steps 1-2 for intermediate 1c as steps 4-5. MS m/z (ESI): 650.3 [M+H]⁺.

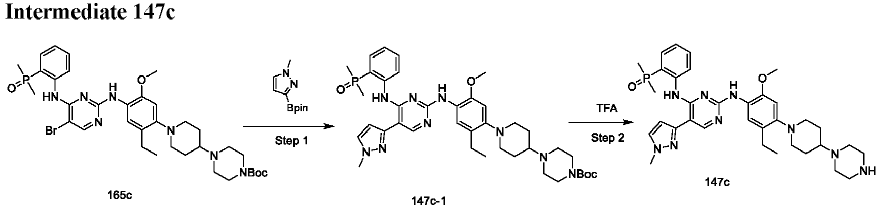

Compound 147c was prepared with reference to the method for intermediate 176a. MS m/z (ESI): 644.7 [M+H]⁺.

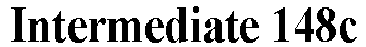

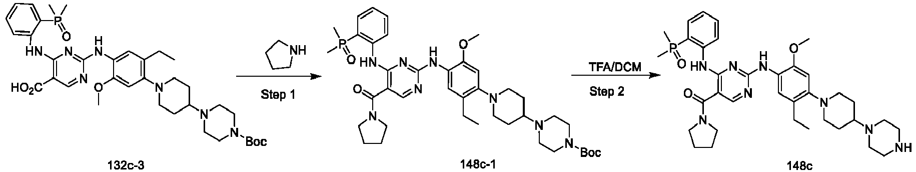

Compound 148c was prepared with reference to step 1 for intermediate 133c as step 1 and with reference to step 2 for intermediate 1c as step 2. MS m/z (ESI): 661.6 [M+H]⁺.

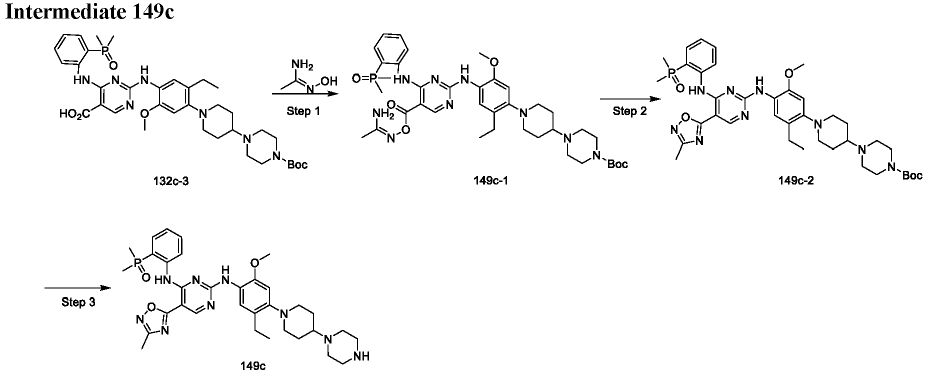

Step 1: Compound 132c-3 (200 mg, 0.28 mmol) was dissolved in THF (50 mL), and then CDI (115 mg, 0.71 mmol) was added. The reaction system was stirred at room temperature for 16 h. (*Z*)-*N*'-hydroxyacetimide (52 mg, 0.71 mmol) was then added, and the reaction system was stirred at room temperature for 16 h. After the reaction was completed, the reaction mixture was diluted with EA and poured into ice water. The mixture was extracted with EA, and the organic phase was washed with brine, dried over anhydrous sodium sulfate, filtered, and concentrated to give crude compound 149c-1 (white solid, 250 mg, crude product). MS m/z (ESI): 763.4 [M+H]⁺.

Step 2: Compound 149c-1 (215 mg, 0.28 mmol) was dissolved in DMSO (50 mL), and then KOH (47 mg, 0.84 mmol) was added. The reaction system was stirred at room temperature for 16 h. After the reaction was completed, the reaction solution was directly concentrated and purified by column chromatography (80 g, MeOH/DCM = ~8%) to give the title compound 149c-2 (white solid, 170 mg). MS m/z (ESI): 746.4 [M+H]⁺. Step 3: Compound 149c was prepared with reference to step 2 for intermediate 3b. MS m/z (ESI): 646.5 [M+H]⁺.

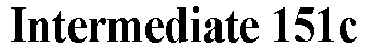

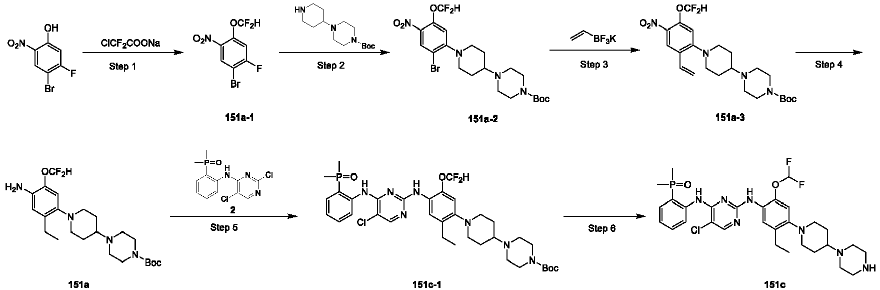

Step 1: Compound 4-bromo-5-fluoro-2-nitrophenol (0.5 g, 2.12 mmol) was dissolved in anhydrous DMF (10 mL), and then sodium difluorochloroacetate (1.13 g, 7.42 mmol) and potassium carbonate (351.38 mg, 2.54 mmol) were separately added. After the addition, the reaction solution was stirred at 90 °C for 16 h. After the reaction was completed, water was added to the reaction solution to quench the reaction. The mixture was extracted with EA, and the organic phases were combined, washed with saturated brine, dried over anhydrous sodium sulfate, filtered, and concentrated by rotary evaporation. The crude product was separated by column chromatography to give compound 151a-1 (yellow oil, 510 mg). MS m/z (ESI): 386.2 [M+H]⁺.

Step 2: Compound 151a-2 (yellow solid, 1.84 g) was prepared with reference to step 1 for intermediate 3a. MS m/z (ESI): 535.3 [M+H]⁺.

Step 3: Compound 151a-3 (yellow oil, 1.2 g) was prepared with reference to the method for intermediate 5d. MS m/z (ESI): 483.4 [M+H]⁺.

Step 4: Compound 151a (red oil, 950 mg) was prepared with reference to step 2 for intermediate 3a. MS m/z (ESI): 455.3 [M+H]⁺.

Step 5: Compound 151a (500 mg, 1.10 mmol), compound 2 (500 mg, 1.58 mmol), sodium tert-butoxide (250 mg, 2.60 mmol), and anhydrous THF (10 mL) were dissolved in a single-necked flask. The system was purged three times with nitrogen before t-Bu Xphos (100 mg, 0.24 mmol) and t-Bu Xphos G₃ (90 mg, 0.11 mmol) were separately added. After the addition, the reaction solution was stirred at 65 °C for 18 h in a nitrogen atmosphere. After the reaction was completed, the reaction solution was diluted with EA and then filtered. The filtrate was concentrated by rotary evaporation. The crude product was separated by column chromatography (EA/PE: 0-100%) to give compound 151c-1 (pale red solid, 100 mg). MS m/z (ESI): 734.3 [M+H]⁺.

Step 6: Crude compound 151c was prepared with reference to step 2 for intermediate 3b. MS m/z (ESI): 634.6 [M+H]⁺.

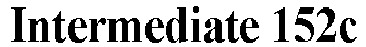

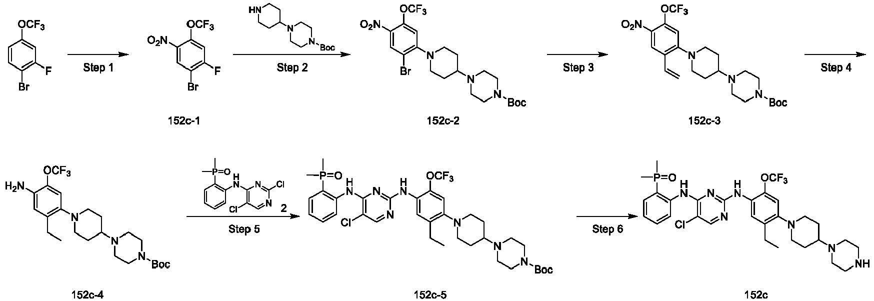

Step 1: Compound 1-bromo-2-fluoro-4-trifluoromethoxybenzene (2.5 g, 9.65 mmol) was dissolved in acetonitrile (37.5 mL), and then nitronium tetrafluoroborate (1.13 g, 7.42 mmol) was added at 0 °C. The reaction solution was stirred at 25 °C for 4 h, then diluted with water, and extracted three times with ethyl acetate. The organic phases were combined, washed with saturated brine, dried over anhydrous sodium sulfate, filtered, and concentrated by rotary evaporation. The crude product was separated by column chromatography (0-2% ethyl acetate/petroleum ether) to give compound 152c-1 (yellow oily liquid, 1.5 g). MS m/z (ESI): 303.9 [M+H]⁺.

Step 2: Compound 152c-2 was prepared with reference to step 1 for intermediate 3a. MS m/z (ESI): 553.2 [M+H]⁺.

Step 3: Compound 152c-3 was prepared with reference to the preparation of intermediate 5d. MS m/z (ESI): 501.4 [M+H] ⁺.

Step 4: Compound 152-4 was prepared with reference to step 2 for intermediate 3a. MS m/z (ESI): 473.3 [M+H]⁺.

Step 5: Compound 152c-5 was prepared with reference to step 5 for intermediate 151c. MS m/z (ESI): 753.2 [M+H]⁺.

Step 6: Compound 152c was prepared with reference to step 2 for intermediate 3b. MS m/z (ESI): 652.3 [M+H]⁺.

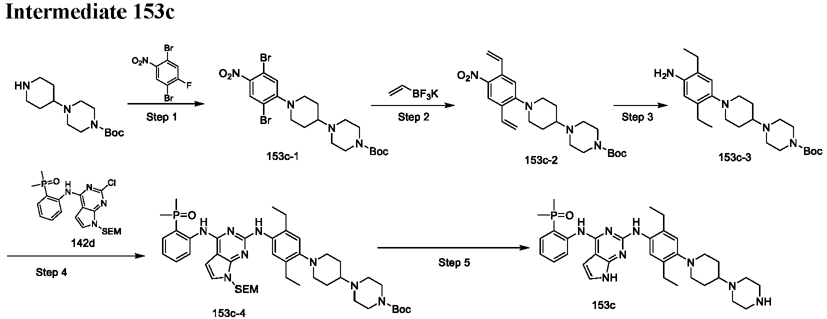

Compound 153c was prepared with reference to steps 1 and 2 for intermediate 3a as steps 1 and 3, with reference to the method for 5d as step 2, and with reference to steps 5 and 6 for intermediate 154c as steps 4 and 5. MS m/z (ESI): 601.3 [M+H]⁺.

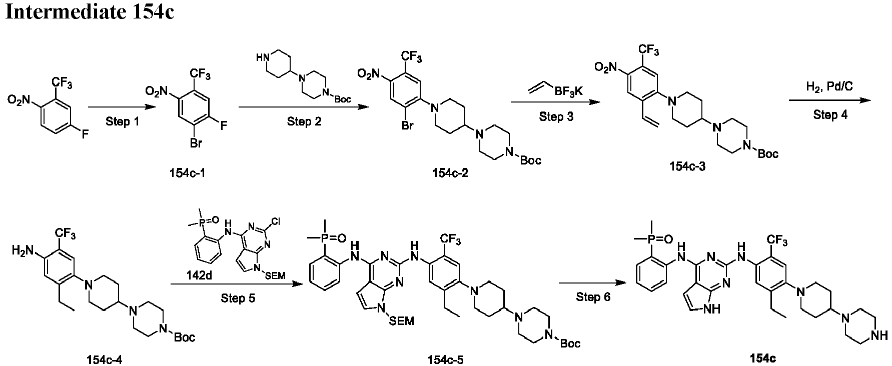

Step 1: At 20 °C, 1,3-dibromo-5,5-dimethylimidazolidine-2,4-dione (6.8 g, 23.78 mmol) was added to a solution of 4-fluoro-1-nitro-2-trifluoromethylbenzene (5.97 g, 28.54 mmol) in concentrated sulfuric acid (30 mL). The reaction solution was stirred at 20 °C for 6 h, then poured into ice water, and extracted with EA. The organic phase was washed with saturated brine, dried, and concentrated by rotary evaporation to give compound 154c-1 (pale yellow oil, 6.55 g, crude product). MS m/z (ESI): 288.3 [M+H]⁺.

Step 2: Compound 154c-2 was prepared with reference to step 1 for intermediate 3a. MS m/z (ESI): 537.3 [M+H]⁺.

Step 3: Compound 154c-3 was prepared with reference to the method for intermediate 5d. MS m/z (ESI): 485.5 [M+H]⁺.

Step 4: Compound 154c-4 was prepared with reference to step 2 for intermediate 3a. MS m/z (ESI): 457.3 [M+H]⁺.

Step 5: Compound 154c-4 (1 g, 2.19 mmol), compound 142d (1.14 g, 2.52 mmol), potassium phosphate (1.86 g, 8.76 mmol) were added to a mixture of t-Bu Xphos (400 mg, 941.97 µmol) and t-Bu Xphos Pd G₃ (350 mg, 440.60 µmol) in dioxane (12 mL) and DMF (3 mL), and the reaction system was stirred at 95 °C for 16 h in a nitrogen atmosphere. After the reaction was completed, water (100 mL) was added to the reaction solution, and the mixture was extracted with EA. The organic phases were combined, washed with saturated ammonium chloride and brine, dried over sodium sulfate, filtered, and concentrated at reduced pressure. The crude product was separated by column chromatography to give compound 154c-5 (yellow solid, 510 mg). MS m/z (ESI): 871.4 [M+H]⁺.

Step 6: A solution of compound 154c-5 (510 mg, 351.30 µmol) and trifluoroacetic acid (2.90 g, 25.44 mmol) in dichloromethane (5 mL) was stirred at 20 °C for 3 h. The reaction solution was concentrated. The crude product was dissolved in dichloromethane (3 mL), and ethylenediamine (72.49 mmol, 4.85 mL) was added. The reaction solution was stirred at 20 °C for 16 h. After the reaction was completed, the reaction solution was concentrated. The residue was dissolved in DCM, washed with brine, dried over sodium sulfate, filtered, and concentrated at reduced pressure to give compound 154c (pale yellow solid, 118 mg, crude product). MS m/z (ESI): 641.4 [M+H]⁺.

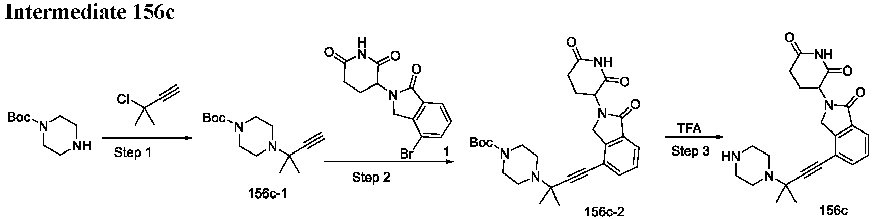

Step 1: Compound *N-*Boc piperazine (1 g, 5.37 mmol) was dissolved in THF (mL), and TEA (5 mL) and CuCl (60 mg, 0.6 mmol) were added. In a nitrogen atmosphere, 3-chloro-3-methyl-1-butyne (551 mg, 5.40 mmol) was added, and the system was stirred at room temperature for 30 min. Water was added to quench the reaction, and the mixture was extracted with dichloromethane, dried over anhydrous sodium sulfate, concentrated, and then purified by column chromatography (PE/EA = 1:1) to give compound 156c-1 (white solid, 400 mg, crude product).

Step 2: Compound 156c-2 was prepared with reference to step 1 for intermediate 1b. MS m/z (ESI): 495.3 [M+H]⁺.

Step 3: Compound 156c was prepared with reference to step 2 for intermediate 3b. MS m/z (ESI): 395.3 [M+H]⁺.

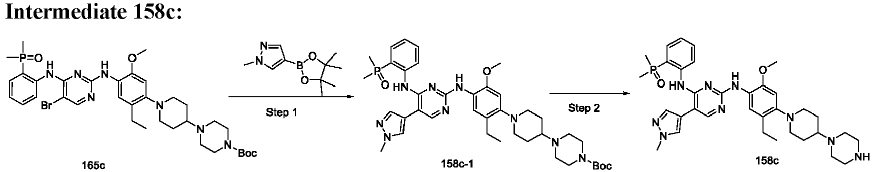

Compound 158c was prepared with reference to the method for intermediate 176a. MS m/z (ESI): 643.4 [M+H]⁺.

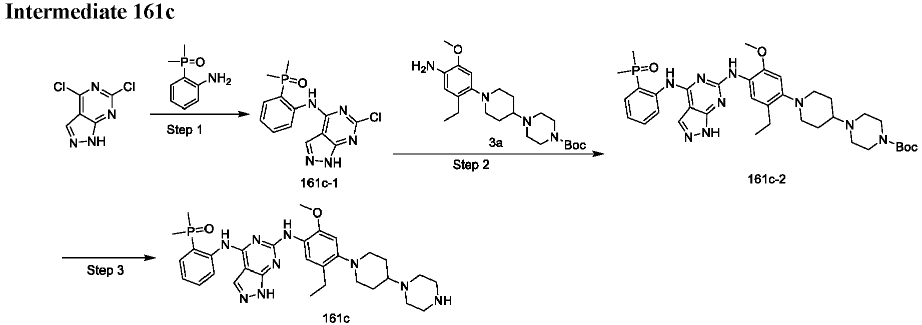

Step 1: 4,6-Dichloropyrazolo[3,4-*d*]pyrimidinc (2.9 g, 15.34 mmol) and (2-aminophenyl)dimethylphosphine oxide (1.30 g, 7.67 mmol) were added to *tert-amyl* alcohol (180 mL), and then DIEA (9.92 g, 76.72 mmol, 13.36 mL) was added. The reaction system was stirred at 100 °C for 12 h. After the reaction was completed, the reaction mixture was diluted with water and then extracted with dichloromethane/methanol. The organic layers were combined and dried over anhydrous sodium sulfate. The crude product was then filtered, concentrated, and subjected to preparative reverse-phase chromatography (hydrochloric acid system) to give compound 161c-1 (pale yellow solid, 525 mg). MS m/z (ESI): 322.0 [M+H]⁺.

Step 2: Compound 3a (2. 19 g, 3.82 mmol) and compound 161c-1 (1.23 g, 3.82 mmol) were added to ethylene glycol (80 mL), and then TsOH·H₂O (581.69 mg, 3.06 mmol) was added. The reaction system was stirred at 80 °C for 12 h. After the reaction was completed, the reaction mixture was adjusted to pH 7-8 with a saturated aqueous sodium bicarbonate solution and then extracted with dichloromethane/methanol. The organic layers were combined and dried over anhydrous sodium sulfate. The crude product was then filtered, concentrated, and subjected to preparative reverse-phase chromatography (hydrochloric acid system) to give compound 161c-2 (brown solid, 1.04 g). MS m/z (ESI): 704.4 [M+H]⁺.

Step 3: Compound 161c was prepared with reference to step 2 for intermediate 3b. MS m/z (ESI): 604.4 [M+H]⁺.

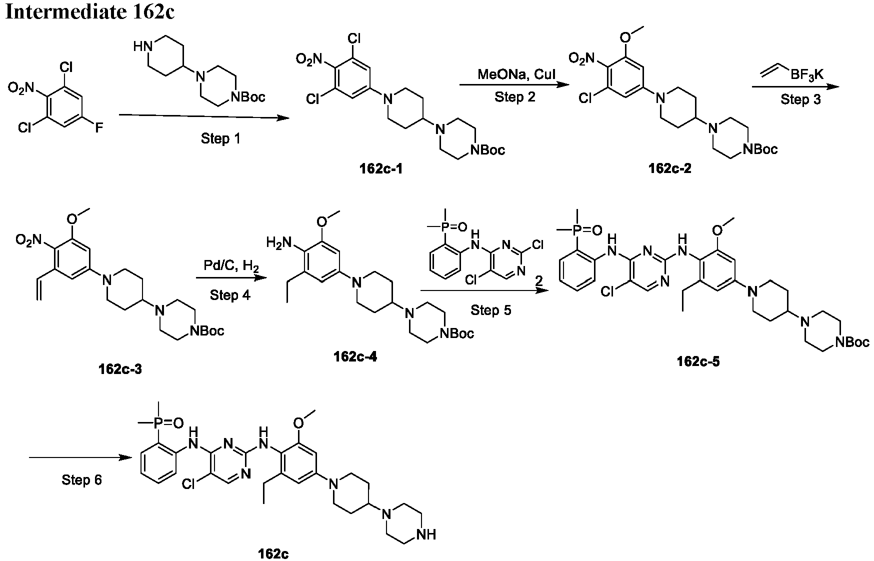

Step 1: Compound 162c-1 was prepared with reference to the method of step 1 for intermediate 3a. MS m/z (ESI): 459.2 [M+H]⁺.

Step 2: Compound 162c-1 (2.0 g, 4.35 mmol) was added to a mixed solution of methanol (6 mL) and 1,4-dioxane (30 mL), and then sodium methoxide (2.35 g, 43.54 mmol) and cuprous iodide (829.19 mg, 4.35 mmol) were added. The reaction system was stirred at 100 °C for 2 h in a nitrogen atmosphere. After the reaction was completed, the reaction mixture was diluted with water and then extracted with dichloromethane. The organic layers were combined, dried over anhydrous sodium sulfate, filtered, and concentrated to give a crude product. The crude product was separated and purified by silica gel column chromatography to give the title compound 162c-2 (yellow solid, 220 mg). MS m/z (ESI): 455.2 [M+H]⁺.

Step 3: Compound 162c-3 was prepared with reference to the method for intermediate 5d. MS m/z (ESI): 447.2 [M+H]⁺.

Step 4: Compound 162c-4 was prepared with reference to the method of step 2 for intermediate 3a. MS m/z (ESI): 419.3 [M+H]⁺.

Steps 5-6: Compound 162c was prepared with reference to the method for intermediate 4c. MS m/z (ESI): 598.4 [M+H]⁺.

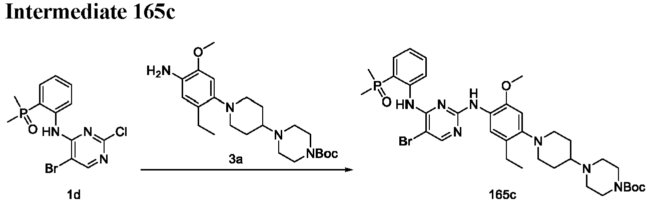

Compound 165c was prepared with reference to the method for intermediate 1d. MS m/z (ESI): 742.3, 744.3 [M+H]⁺.

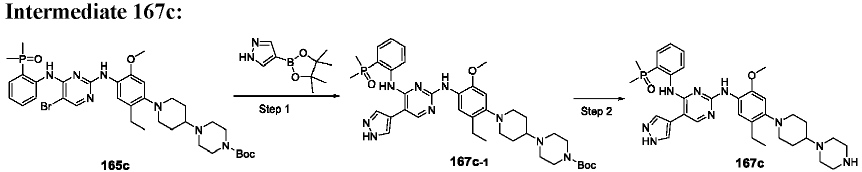

Compound 167c was prepared with reference to the method for intermediate 176a. MS m/z (ESI): 630.5 [M+H]⁺.

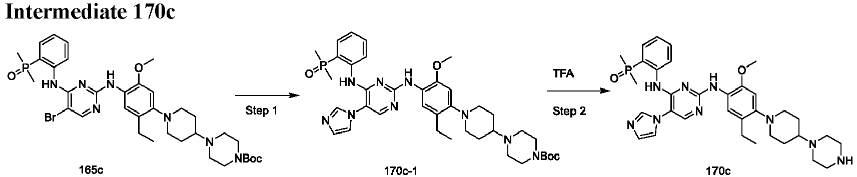

Compound 170c was prepared with reference to the method for intermediate 165a. MS m/z (ESI): 630.5 [M+H]⁺.

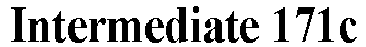

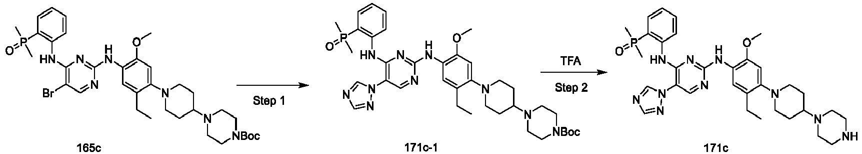

Compound 171c was prepared with reference to the method for intermediate 165a. MS m/z (ESI): 631.5 [M+H]⁺.

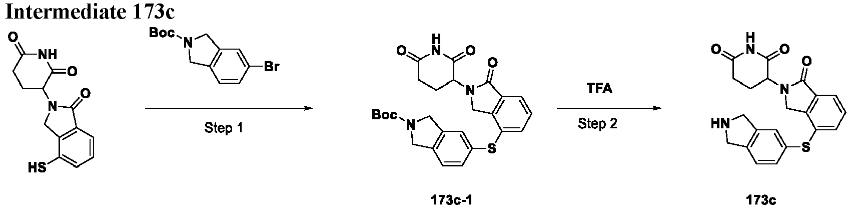

Compound 173c was prepared with reference to the method for intermediate 1c. MS m/z (ESI): 394.1 [M+H]⁺.

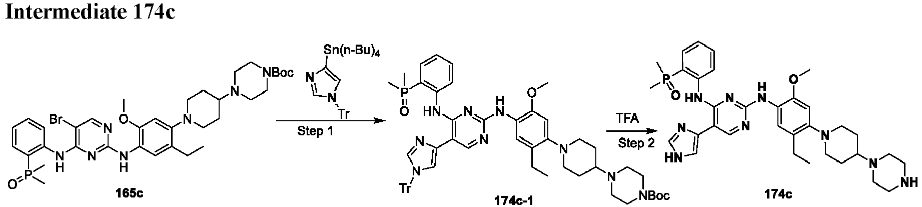

Compound 174c was prepared with reference to the method for intermediate 165a. MS m/z (ESI): 630.6 [M+H]⁺.

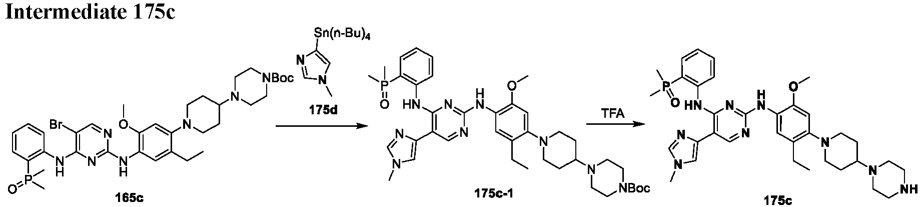

Compound 175c was prepared with reference to the method for intermediate 165a. MS m/z (ESI): 644.6 [M+H]⁺.

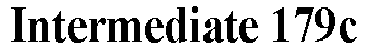

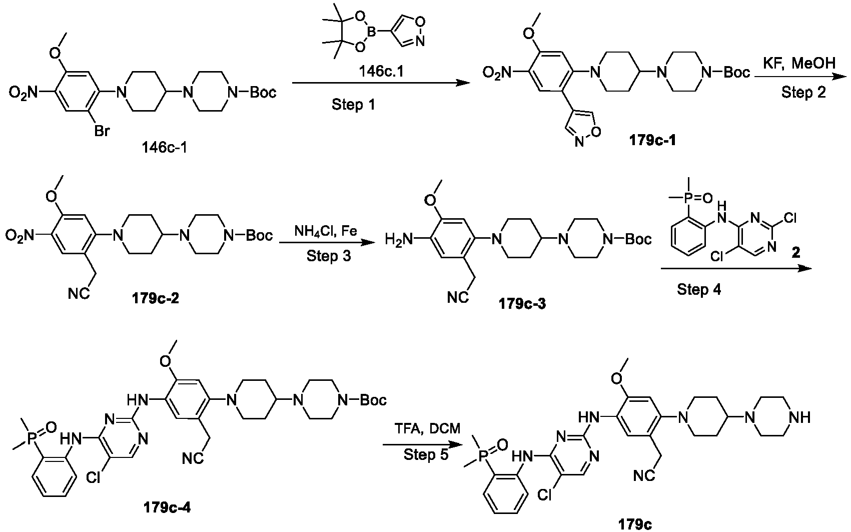

Step 1: Compound 146c-1 (3.8 g, 7.61 mmol), compound 146c.1 (1.78 g, 9.13 mmol), potassium fluoride (1 M, 30.44 mL), and Pd(dppf)Cl₂·CH₂Cl₂ (621.39 mg, 0.76 mmol) were added to dimethyl sulfoxide (150 mL), and the reaction system was stirred at 100 °C for 10 h in a nitrogen atmosphere. After the reaction was completed, the reaction mixture was diluted with water and then extracted with ethyl acetate. The organic phase was washed with water, and the organic layers were combined, dried over anhydrous sodium sulfate, filtered, and concentrated to give a crude product. The crude product was subjected to column chromatography to give compound 179c-1 (blackish brown solid, 1.7 g). MS m/z (ESI): 488.2 [M+H]⁺.

Step 2: 179c-1 (1.7 g, 3.49 mmol) and potassium fluoride (1 M, 10.46 mL) were added to a solution of methanol (25 mL), and the reaction system was stirred at 90 °C for 10 h in a nitrogen atmosphere. After the reaction was completed, the reaction mixture was concentrated to remove the methanol, and the aqueous phase was extracted with dichloromethane/methanol. The organic phase was washed with saturated brine, and the organic layers were combined, dried over anhydrous sodium sulfate, filtered, and concentrated to give compound 179c-2 (blackish brown solid, 1.3 g). MS m/z (ESI): 460.2 [M+H]⁺.

Step 3: Compound 179c-3 (blackish brown solid, 363 mg) was prepared with reference to step 2 for intermediate 7a. MS m/z (ESI): 430.2 [M+H]⁺.

Step 4: Compound 179c-3 (360 mg, 0.84 mmol) and compound 2 (264.94 mg, 0.84 mmol) were added to a solution of tetrahydrofuran (15 mL), and then sodium *tert*-butoxide (161.08 mg, 1.68 mmol) and tBuXPhos Pd G3 (66.57 mg, 83.81 µmol) were added. The reaction system was stirred at 65 °C for 12 h in a nitrogen atmosphere. After the reaction was completed, the reaction mixture was filtered. The filter cake was washed with dichloromethane/methanol, and the filtrate was concentrated to give a crude product. The crude product was purified by a silica gel plate to give compound 179c-4 (blackish brown solid, 360 mg, crude product). MS m/z (ESI): 709.4 [M+H]⁺.

Step 5: Compound 179c was prepared with reference to step 2 for intermediate 1c. MS m/z (ESI): 609.1 [M+H]⁺.

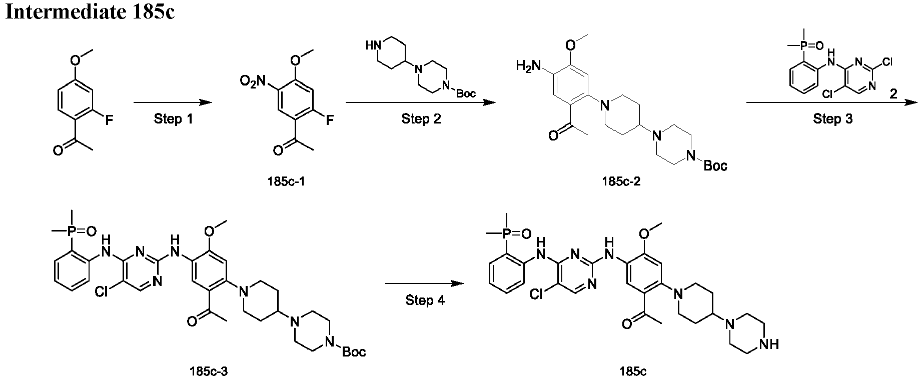

Step 1: At -20 °C, 2-fluoro-4-methoxyacetophenone (5 g, 29.73 mmol) was added to sulfuric acid (31.28 g, 318.93 mmol, 17 mL), and then nitric acid (3.75 g, 59.47 mmol, 2.68 mL) was slowly and dropwise added. The reaction system was then stirred at -20 °C for 30 min. The reaction solution was added to ice water, and the mixture was stirred for 30 min and then filtered. The filter cake was washed with water to give a pale yellow solid, which was dried to give compound 185c-1 (pale yellow solid, 6 g). MS m/z (ESI): 214.1 [M+H]⁺. Step 2: Compound 185c-2 was prepared with reference to step 1 for intermediate 8a. MS m/z (ESI): 433.3 [M+H]⁺.

Step 3: Compound 185c-2 (300 mg, 693.55 µmol), compound 2 (250 mg, 790.83 µmol), and sodium *tert-*butoxide (150 mg, 1.56 mmol) were dissolved in tetrahydrofuran (8 mL), and t-Bu Xphos (65 mg, 153.07 µmol) and t-Bu Xphos Pd G₃ (70 mg, 88.12 µmol) were added. The reaction solution was stirred at 65 °C for 18 h in a nitrogen atmosphere. The reaction solution was diluted with EA, filtered through celite, and concentrated by rotary evaporation. The crude product was separated by column chromatography to give 185c-3 (yellow oil, 200 mg). MS m/z (ESI): 712.5 [M+H] ⁺.

Step 4: Compound 185c was prepared with reference to step 2 for intermediate 3b. MS m/z (ESI): 612.4 [M+H]⁺.

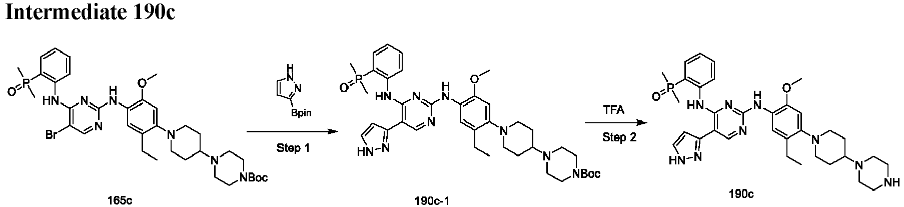

Compound 190c was prepared with reference to the method for intermediate 176a. MS m/z (ESI): 630.3 [M+H]⁺.

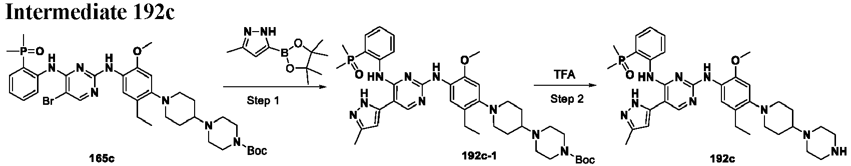

Compound 192c was prepared with reference to the method for intermediate 176a. MS m/z (ESI): 644.3 [M+H]⁺.

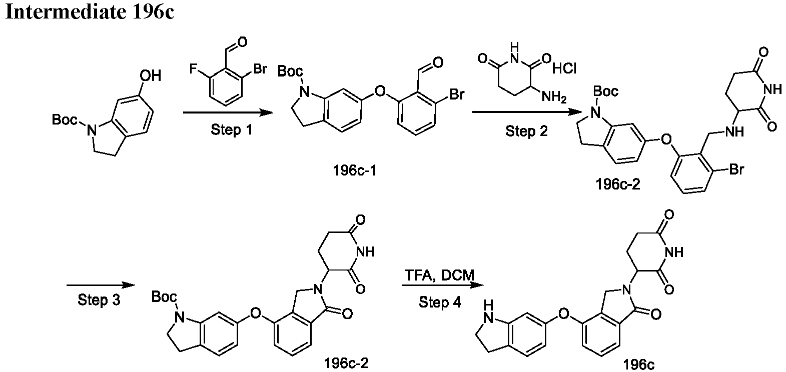

Compound 196c was prepared with reference to the method for intermediate 120c. MS m/z (ESI): 378.0 [M+H]⁺.

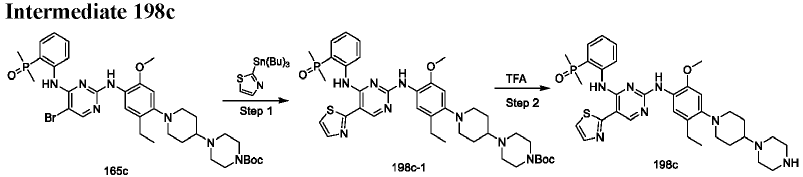

Compound 198c was prepared with reference to step 1 for intermediate 166a as step 1 and with reference to step 2 for intermediate 3b as step 2. MS m/z (ESI): 648.0 [M+H]⁺.

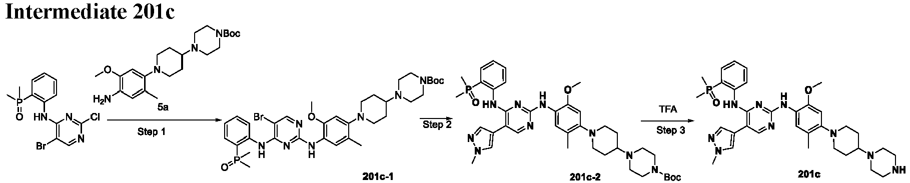

Step 1: Compound 201c-1 was prepared with reference to step 1 for intermediate 4c. MS m/z (ESI): 728.2 [M+H]⁺.

Steps 2-3: Compound 201c was prepared with reference to the method for intermediate 176a. MS m/z (ESI): 630.2 [M+H]⁺.

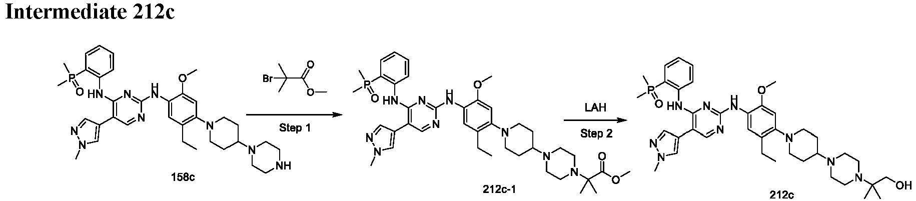

Step 1: A solution of compound 158c (0.7 g, 923.75 µmol) and potassium carbonate (1.66 g, 12.01 mmol) in acetonitrile (10 mL) was stirred at 20 °C for 1 h. Methyl 2-bromo-2-methylpropionate (1 g, 5.52 mmol) was then added. The reaction system was stirred at 80 °C for 15 h and concentrated at reduced pressure. The crude product was separated by column chromatography to give compound 212c-1 (brown solid, 440 mg). MS m/z (ESI): 744.6 [M+H]⁺.

Step 2: LAH (90 mg, 2.37 mmol) was added to a solution of compound 212c-1 (440 mg, 591.50 µmol) in THF (10 mL). The reaction system was stirred at 20 °C for 30 min. Water, a sodium hydroxide solution, and water were added to the reaction solution, and then the reaction solution was concentrated at reduced pressure. The crude product was subjected to column chromatography to give compound 212c (white solid, 170 mg). MS m/z (ESI): 716.6 [M+H]⁺.

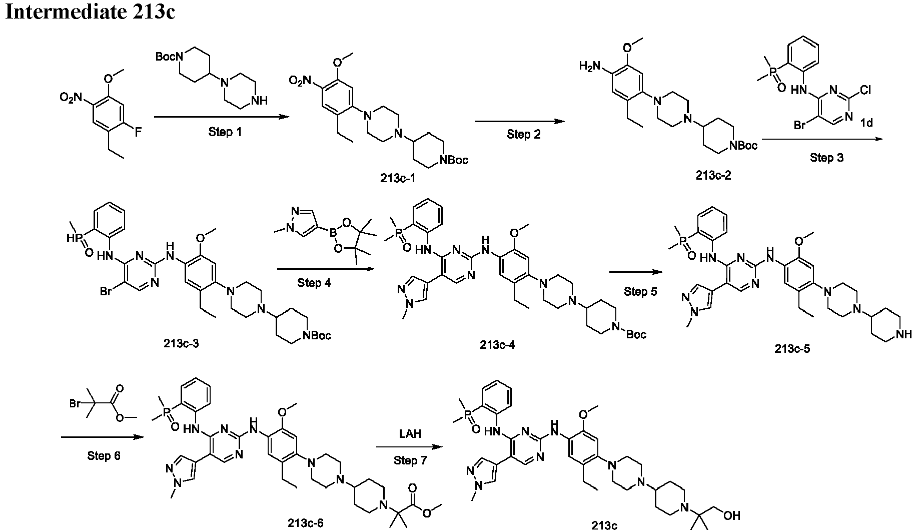

Steps 1-2: Compound 213c-2 was prepared with reference to the method for intermediate 3a. MS m/z (ESI): 419.5 [M+H]⁺.

Step 3: Compound 213c-3 was prepared with reference to the method for intermediate 165c. MS m/z (ESI): 742.2 [M+H]⁺.

Step 4 and step 5: Compound 213c-5 was prepared with reference to the method for intermediate 176a. MS m/z (ESI): 644.2 [M+H]⁺.

Step 6 and step 7: Compound 213c was prepared with reference to the method for intermediate 212c. MS m/z (ESI): 716.6 [M+H]⁺.

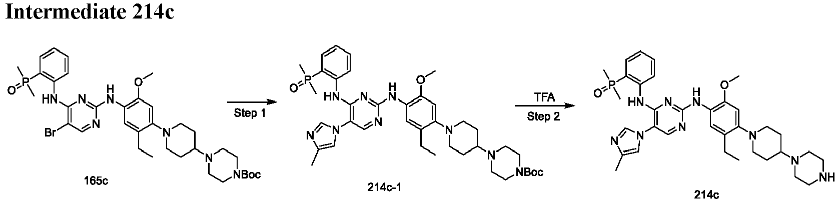

Compound 214c was prepared with reference to the method for intermediate 165a. MS m/z (ESI): 644.6 [M+H]⁺.

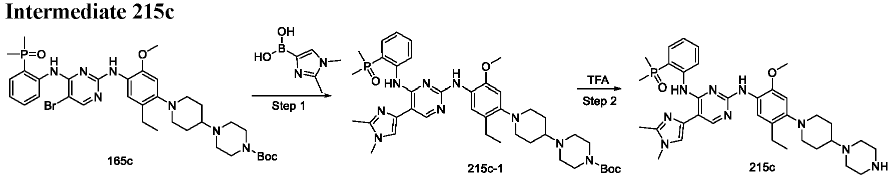

Compound 215c was prepared with reference to the method for intermediate 176a. MS m/z (ESI): 658.4 [M+H]⁺.

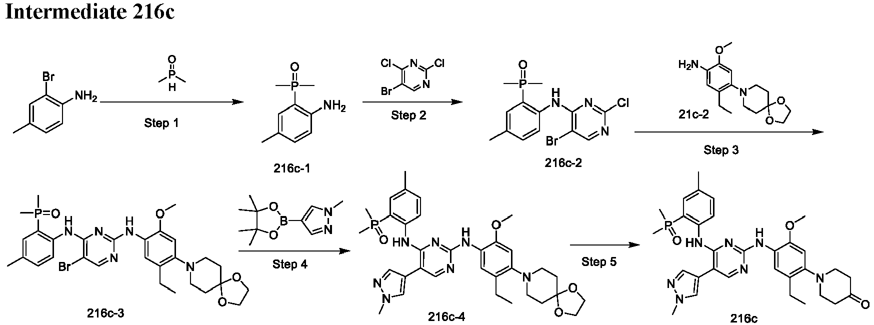

Compound 216c was prepared with reference to the method for intermediate 5d as step 4 and with reference to the method for intermediate 124c as step 1, step 2, step 3, and step 5. MS m/z (ESI): 588.3 [M+H]⁺.

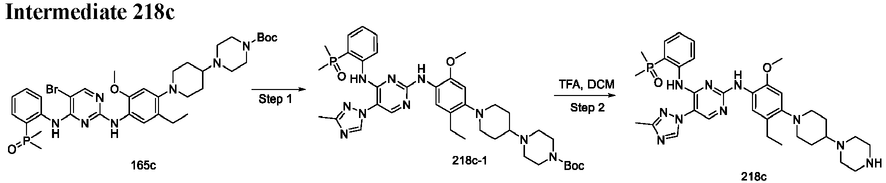

Compound 218c was prepared with reference to the method for intermediate 219c. MS m/z (ESI): 645.4 [M+H]⁺.

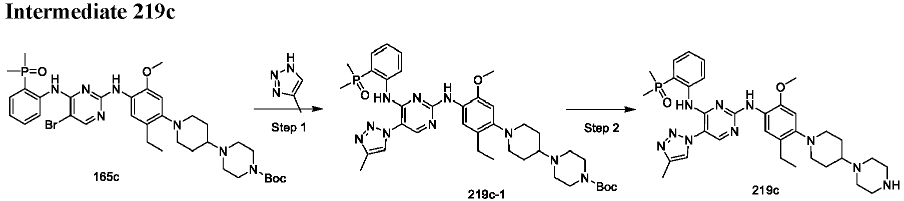

Step 1: A solution of compound 165c (850 mg, 1.14 mmol), 4-methyltriazole (300 mg, 3.61 mmol), cuprous iodide (68.00 mg, 357.05 µmol), L-proline (42.50 mg, 369.15 µmol), and cesium carbonate (1.87 g, 5.74 mmol) in DMSO (5 mL) was microwaved at 140 °C for reaction for 3 h. The reaction solution was diluted with ethyl acetate, washed with saturated brine, dried over anhydrous sodium sulfate, filtered, and concentrated by rotary evaporation. The crude product was separated by column chromatography and then subjected to prep-HPLC (FA) to give compound 219c-1. MS m/z (ESI): 745.5 [M+H]⁺.

Step 2: Compound 219c-1 (20 mg, 26.85 µmol) was dissolved in DCM (2 mL), and then TFA (1 mL) was added. The reaction system was stirred at 20 °C for 1 h. The reaction solution was concentrated by rotary evaporation to give compound 219c. MS m/z (ESI): 645.5 [M+H]⁺.

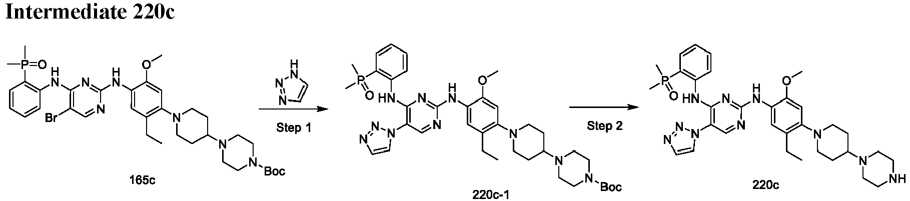

Compound 220c was prepared with reference to the synthesis method for intermediate 219c. MS m/z (ESI): 631.3 [M+H]⁺.

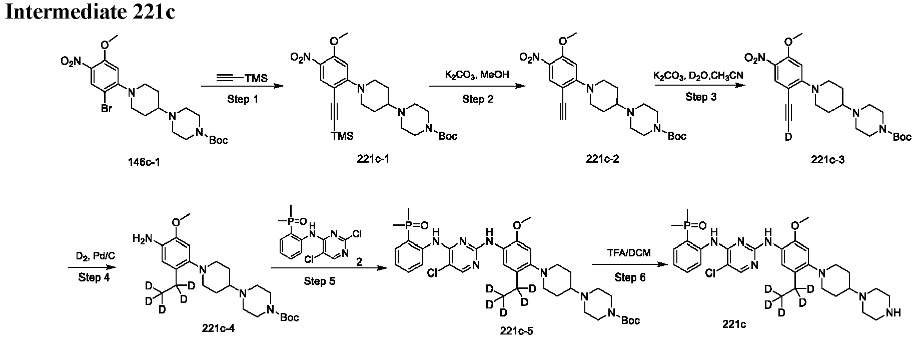

Step 1: A solution of compound 146c-1 (1 g, 2.00 mmol), ethynyltrimethylsilane (1.97 g, 20.02 mmol), cuprous iodide (50 mg, 262.54 µmol), Pd(PPh₃)₂Cl₂ (210 mg, 299.19 µmol), and TEA (1.22 g, 12.01 mmol) in DMF (10 mL) were allowed to react at 85 °C for 16 h in a nitrogen atmosphere. The reaction solution was diluted with EA, washed with saturated brine, dried over anhydrous sodium sulfate, filtered, and concentrated by rotary evaporation. The crude product was separated by column chromatography to give compound 221c-1. MS m/z (ESI): 517.4 [M+H]⁺.

Step 2: A solution of compound 221c-1 (1.82 g, 3.52 mmol) and potassium carbonate (1 g, 7.24 mmol) in methanol (20 mL) was stirred at 20 °C for 2 h. The reaction solution was concentrated. The crude product was separated by column chromatography to give compound 221c-2. MS m/z (ESI): 445.4 [M+H]⁺.

Step 3: Deuterium water (95.61 mmol, 2 mL) was added to a solution of compound 221c-2 (850 mg, 1.91 mmol) and potassium carbonate (550 mg, 3.98 mmol) in acetonitrile (8 mL). The reaction solution was allowed to react at 20 °C for 48 h, diluted with EA, and filtered. The filtrate was concentrated at reduced pressure to give compound 221c-3. MS m/z (ESI): 446.4 [M+H]⁺.

Step 4: Compound 221c-3 (750 mg, 1.68 mmol) was dissolved in deuterated methanol (100 mL) and EA (50 mL). The system was purged three times with nitrogen before palladium on carbon (200 mg, 10% purity) was added. The reaction system was stirred at 20 °C for 24 h in a deuterium atmosphere. The reaction solution was filtered through celite and then concentrated by rotary evaporation. The crude product was separated by column chromatography to give compound 221c-4. MS m/z (ESI): 424.4 [M+H]⁺.

Step 5: Compound 221c-5 was prepared with reference to step 3 for intermediate 185c. MS m/z (ESI): 703.2 [M+H]⁺.

Step 6: Compound 221c was prepared with reference to the method of step 2 for intermediate 219c. MS m/z (ESI): 603.2 [M+H]⁺.

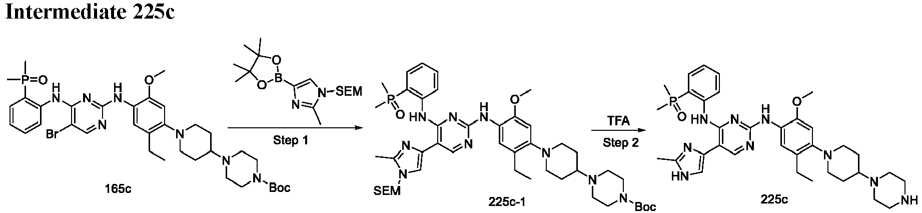

Compound 225c was prepared with reference to step 1 for intermediate 176a as step 1 and with reference to step 6 for intermediate 154c as step 2. MS m/z (ESI): 644.2 [M+H]⁺.

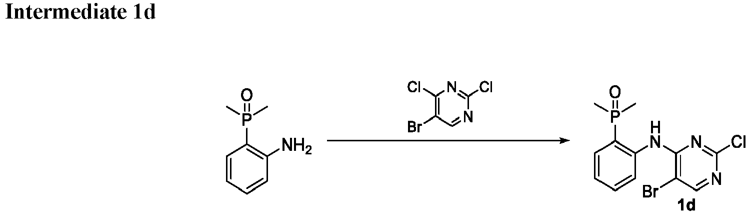

(2-Aminophenyl)dimethylphosphine oxide (1.480 g, 8.8 mmol) and 5-bromo-2,4-dichloropyrimidine (1 g, 4.4 mmol) were added to a reaction flask before DIEA (2.88 g, 22 mmol) and n-BuOH (10 mL) were added. The reaction system was stirred at 140 °C for 16 h in an argon atmosphere. After the reaction was completed, the reaction mixture was concentrated. The crude product was purified by column chromatography (DCM:MeOH = 20:1) and concentrated by rotary evaporation to give compound 1d (pale yellow solid, 1.1 g). MS m/z (ESI): 362.1 [M+H]⁺.

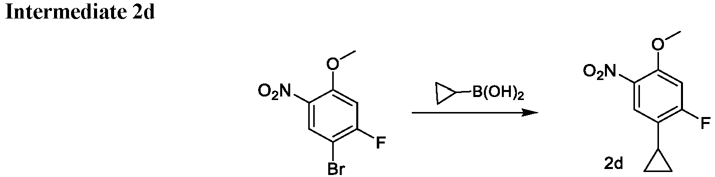

1-Bromo-2-fluoro-4-methoxy-5-nitrobenzene (1 g, 4.00 mmol), cyclopropylboronic acid (343.56 mg, 4.00 mmol), Pd(dppf)Cl₂ (585.31 mg, 799.93 µmol), and cesium carbonate (3.91 g, 12.00 mmol) were added to a mixture of ethylene glycol dimethyl ether (2 mL) and water (0.5 mL), and the reaction system was stirred at 90 °C for 12 h in a nitrogen atmosphere. After the reaction was completed, the reaction mixture was directly concentrated at reduced pressure to give a crude product. The crude product was purified by column chromatography to give compound 2d (white solid, 0.5 g). MS m/z (ESI): 212.2 [M+H]⁺.

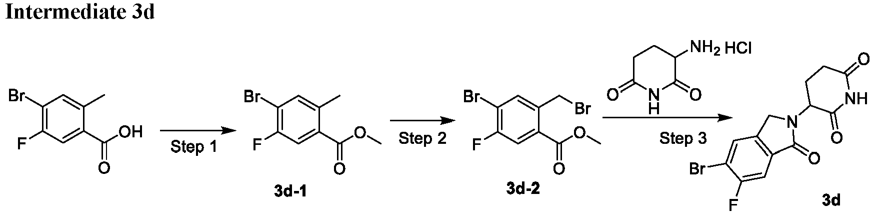

Step 1: Compound 4-bromo-5-fluoro-2-methylbenzoic acid (3 g, 12.87 mmol) was dissolved in DMF (50 mL). NaHCOs (3.24 g, 38.62 mmol) and MeI (1.2 mL, 19.31 mmol) were added with stirring, and then the reaction system was stirred at room temperature for 3 h. After the reaction was completed, the reaction system was cooled to room temperature and poured into ice water. The aqueous phase was then extracted with ethyl acetate, and the organic layer was washed with brine, dried over anhydrous sodium sulfate, filtered, concentrated, and subjected to column chromatography (EA/PE = 0-5%) to give compound 3d-1 (colorless oil, 3 g). MS m/z (ESI): 247.1 [M+H]⁺.

Step 2: At 25 °C, compound 3d-1 (2.7 g, 10.93 mmol) was added to a solution of DCE (30 mL), and NBS (2.14 g, 12.02 mmol) and AIBN (180 mg, 1.1 mmol) were added with stirring. The reaction system was stirred at 85 °C for 16 h. After the reaction was completed, the reaction mixture was diluted with water and then extracted with dichloromethane. The organic layers were combined, dried over anhydrous sodium sulfate, filtered, and concentrated to give a crude product. The crude product was separated and purified by silica gel column chromatography (PE:EA = 20:1-10:1) to give compound 3d-2 (pale yellow oil, 2 g). MS m/z (ESI): 324.8 [M+H]⁺.

Step 3: Compound 3d-2 (2 g, 6.14 mmol) was added to a solution of DMF (25 mL), and then 3-aminopiperidine-2,6-dione (1.51 g, 9.2 mmol, hydrochloride) and *N,N*-diisopropylethylamine (5.66 mL, 30.68 mol) were added. The reaction system was stirred at 85 °C for 16 h. After the reaction was completed, the reaction solution was poured into ice water, and a solid was precipitated. The mixture was filtered, and the filter cake was triturated with MTBE and dried under vacuum to give compound 3d (grey solid, 1.6 g). MS m/z (ESI): 341.2 [M+H]⁺.

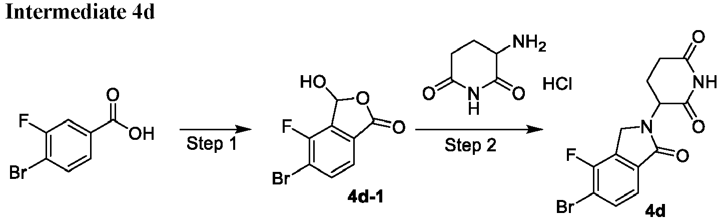

Step 1: 2,2,6,6-Tetramethylpiperidine (8.05 g, 9.61 mL, 57 mmol) was dissolved in THF (150 mL), and the system was stirred. In a nitrogen atmosphere at 0 °C, n-butyllithium (21.87 mL, 54.7 mmol, 2.5 M) was added. The reaction system was stirred at 0 °C for 1 h. The reaction system was then cooled to -45 °C, and at that temperature, a solution of 4-bromo-3-fluorobenzoic acid (4.99 g, 22.8 mmol) in THF (15 mL) was added. The system was then stirred at that temperature for another 5 h. DMF (2.65 mL, 34.2 mmol) was slowly and dropwise added to the reaction solution. The reaction system was then gradually warmed to room temperature and allowed to react overnight at room temperature. After the reaction was completed, at 0 °C, the reaction system was diluted with 3 N HCl to quench the reaction and then extracted with EA. The organic layers were combined, washed with brine (50 mL), dried over anhydrous sodium sulfate, filtered, and concentrated. The crude product was purified by column chromatography (EA/PE = 50%-100%) to give compound 4d-1 (white solid, 3.6 g). MS m/z (ESI): 247.1 [M+H]⁺.

Compound 4d was prepared with reference to step 3 for intermediate 3d as step 2. MS m/z (ESI): 341.2 [M+H]⁺.

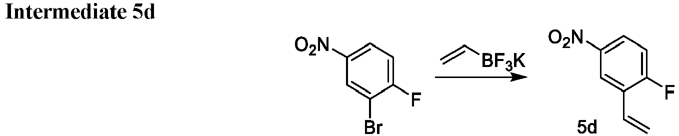

Compound 2-bromo-1-fluoro-4-nitrobenzene (1.5 g, 6.8 mmol) and compound *(3-(4-(tert-*butoxycarbonyl)piperazin-1-yl)phenyl)boronic acid (1.4 mg, 17.6 mmol) were added to a reaction flask, and dioxane (30 mL), water (10 mL), potassium carbonate (2.8 g, 20.4 mmol), and Pd(dppf)Cl₂ (250 mg, 0.34 mmol) were added sequentially. The system was then stirred at 80 °C for 16 h in a nitrogen atmosphere. After the reaction was completed, water was added to quench the reaction. The mixture was extracted with ethyl acetate, and the organic phase was washed with saturated brine, dried over anhydrous sodium sulfate, concentrated, and subjected to column chromatography (PE/EA = 0-10%) to give compound 5d (pale yellow solid, 900 mg). MS m/z (ESI): 168.1 [M+H]⁺.

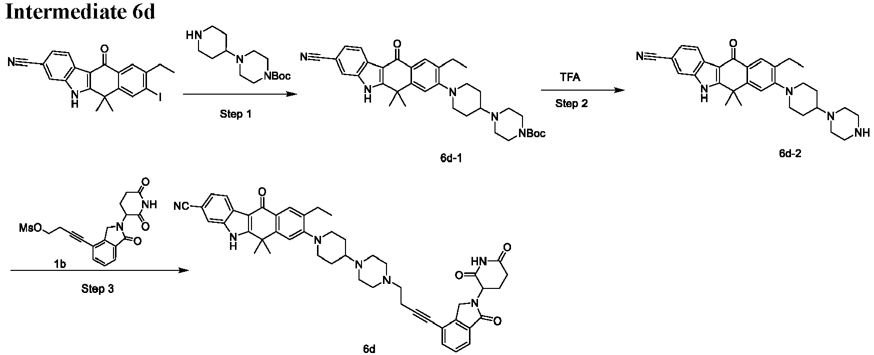

Step 1: Compound 6d-1 was prepared with reference to step 1 for intermediate 1c. MS m/z (ESI): 582.4 [M+H]⁺.

Step 2: Compound 6d-2 was prepared with reference to step 2 for intermediate 3b. MS m/z (ESI): 482.4 [M+H]⁺.

Step 2: Compound 6d-2 (200 mg, 0.415 mmol) and compound 1b (178 mg, 0.456 mmol) were dissolved in CH₃CN/CHCl₃ (1:1, 20 mL). K₂CO₃ (172 mg, 1.25 mmol) was added with stirring. In a nitrogen atmosphere, the system was stirred at 65 °C for 48 h. After the reaction was completed, H₂O was added to the reaction system. The mixture was extracted with DCM, and the organic phase was dried over anhydrous sodium sulfate, filtered, concentrated, purified by normal-phase column chromatography, and then subjected to prep-HPLC to give compound 6d (white solid, 75 mg, hydrochloride). MS m/z (ESI): 776.6 [M+H]⁺.

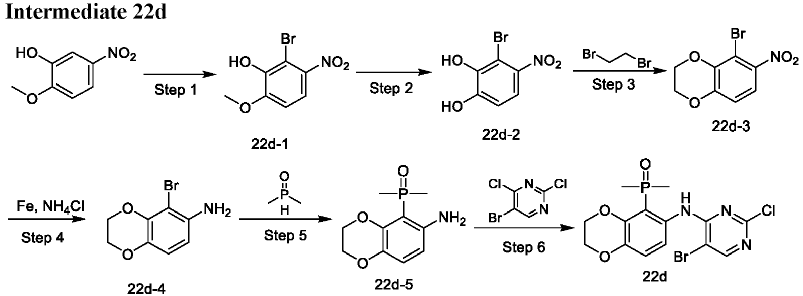

Step 1: N-Bromosuccinimide (52.62 g, 295.62 mmol) was added to a solution of 2-methoxy-5-nitrophenol (50 g, 295.62 mmol) in dichloromethane (300 mL), and the reaction system was stirred at 25 °C for 2 h. After the reaction was completed, the reaction mixture was directly concentrated at reduced pressure to give a crude product. The crude product was separated and purified by reverse-phase chromatography (formic acid system) and lyophilized to give the title compound 22d-1 (yellow solid, 20 g). MS m/z (ESI): 248.3 [M+H]⁺.

Step 2: At -75 °C, boron tribromide (80.64 mmol, 7.77 mL) was added to a solution of 22d-1 (10 g, 40.32 mmol) in dichloromethane (100 mL). The reaction system was stirred at -75 °C for 2 h, and then warmed to 25 °C and stirred for 4 h. After the reaction was completed, at 0 °C, methanol was added to the reaction solution to quench the reaction. The mixture was directly concentrated at reduced pressure to give a crude product. The crude product was purified by column chromatography to give compound 22d-2 (yellow solid, 9 g). MS m/z (ESI): 234.3 [M+H]⁺.

Step 3: Potassium carbonate (12.40 g, 89.74 mmol) and 1,2-dibromoethane (16.86 g, 89.74 mmol, 6.77 mL) were added to a solution of 22d-2 (7 g, 29.91 mmol) in DMF (40 mL), and then the reaction system was stirred at 90 °C for 4 h. After the reaction was completed, the reaction solution was extracted with water and ethyl acetate and separated into layers. The organic layer was washed with brine, dried over anhydrous sodium sulfate, filtered, and concentrated at reduced pressure to give a crude product. The crude product was purified by column chromatography (PE/EA = 10:1-5:1) to give compound 22d-3 (yellow solid, 3.8 g). MS m/z (ESI): 257.3 [M+H]⁺.

Step 4: Compound 22d-4 was prepared with reference to step 2 for intermediate 7a. MS m/z (ESI): 230.4 [M+H]⁺.

Step 5: 22d-4 (500 mg, 2.17 mmol), dimethylphosphine oxide (169.63 mg, 2.17 mmol), 4,5-bis(diphenylphosphino)-9,9-dimethylxanthene (251.51 mg, 434.67 µmol), palladium acetate (48.79 mg, 217.34 µmol), and potassium phosphate (922.66 mg, 4.35 mmol) were added to a solution of DMF (5 mL), and then the reaction system was microwaved with stirring at 145 °C for 8 h in a nitrogen atmosphere. After the reaction was completed, the reaction mixture was filtered and concentrated at reduced pressure to give a crude product. The crude product was separated and purified by reverse-phase chromatography (formic acid system) to give compound 22d-5 (off-white solid, 140 mg). MS m/z (ESI): 228.3 [M+H]⁺.

Compound 22d was prepared with reference to the method for intermediate 1d as step 6. MS m/z (ESI): 418.4 [M+H]⁺.

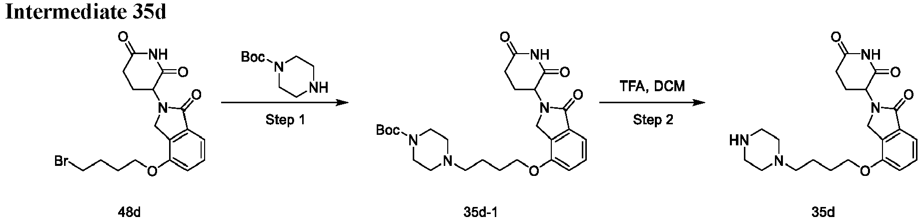

Step 1: Compound 35d-1 was prepared with reference to the method for reference compound C-1. MS m/z (ESI): 501.8 [M+H]⁺.

Step 2: Compound 35d was prepared with reference to step 2 for intermediate 3b. MS m/z (ESI): 401.6 [M+H]⁺.

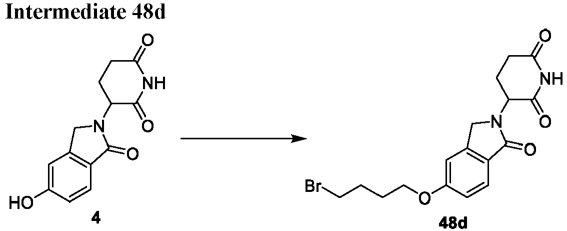

Compound 4 (200 mg, 0.77 mmol) and compound 1,4-dibromobutane (830 mg, 3.84 mmol) were dissolved in DMF (10 mL). K₂CO₃ (106.2 mg, 0.77 mmol) was added with stirring. In a nitrogen atmosphere, the system was stirred at 70 °C for 48 h. After the reaction was completed, H₂O was added to the reaction system. The mixture was extracted with DCM, and the organic phase was dried over anhydrous sodium sulfate, filtered, concentrated, and purified by normal-phase column chromatography (methanol/dichloromethane: ~8%) to give compound 48d (white solid, 50 mg). MS m/z (ESI): 395.4 [M+H]⁺.

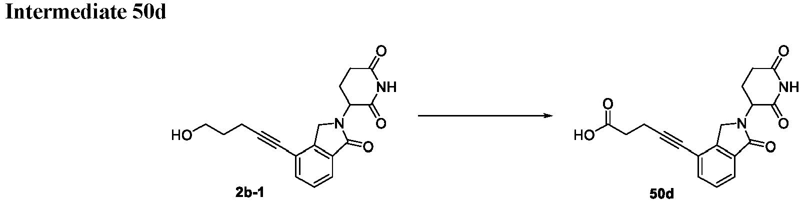

Compound 2b-1 (50 mg, 0.15 mmol) and acetone (5 mL) were added to a single-necked flask before Jones reagent (1 mL) was slowly and dropwise added in an ice-water bath. After the dropwise addition, the reaction system was stirred in the ice-water bath for 4 h. After the reaction was completed, the reaction solution was concentrated at reduced pressure, and then the crude product was directly subjected to pre-HPLC to give product 50d (white solid, 15 mg). MS m/z (ESI): 341.4 [M+H]⁺.

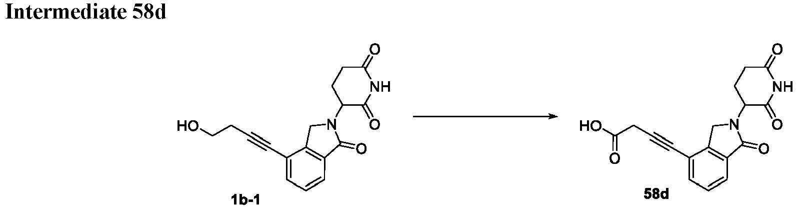

Compound 58d was prepared with reference to the method for intermediate 50d. MS m/z (ESI): 327.1 [M+H]⁺.

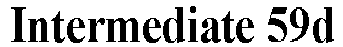

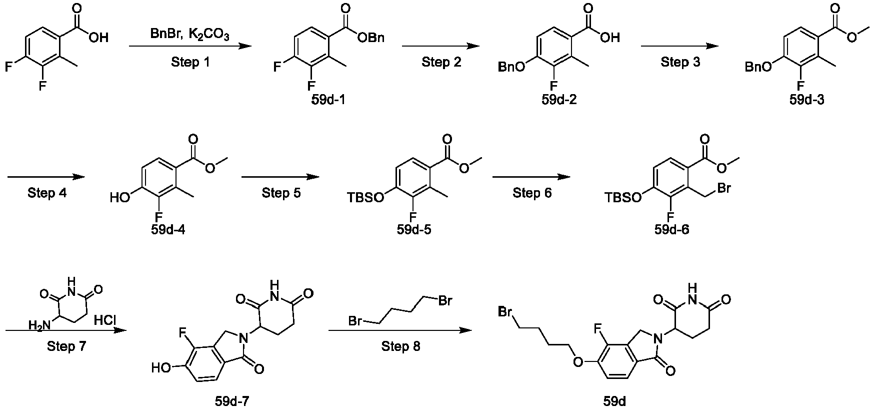

Compound 59d was prepared with reference to steps 1, 2 and 5 for intermediate 189d as steps 1, 2 and 5, with reference to step 1 for intermediate 3d as step 3, with reference to step 2 for intermediate 3a as step 4, with reference to steps 2-3 for intermediate 211d as steps 6-7, and with reference to the method for intermediate 48d as step 8. MS m/z (ESI): 413.4 [M+H]⁺.

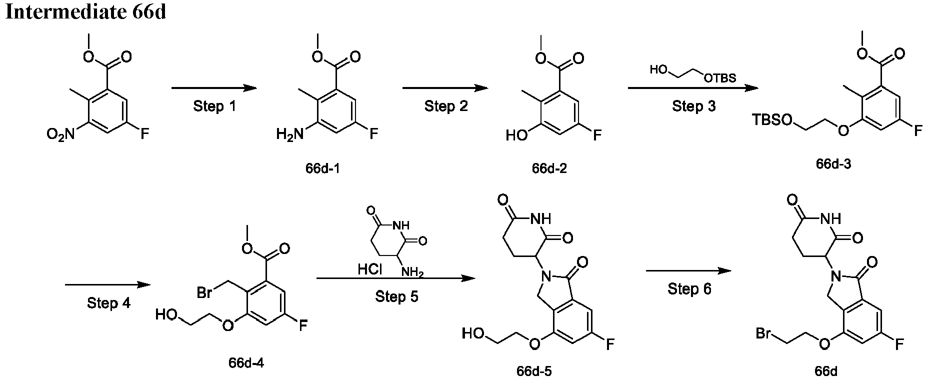

Step 1: Compound 66d-1 was prepared with reference to step 2 for intermediate 3a. MS m/z (ESI): 184.2 [M+H]⁺.

Step 2: Compound 66d-1 (10 g, 54.59 mmol) was dissolved in concentrated sulfuric acid (300.25 mmol, 16.33 mL, 98% purity) diluted with water (270 mL). A solution of sodium nitrite (4.14 g, 60.05 mmol) in water (75 mL) was slowly and dropwise added at 0 °C (over about 1 h). The reaction system was slowly warmed to 20 °C and stirred for 1 h. Concentrated sulfuric acid (136.59 g, 1.36 mol, 98% purity) and water (130 mL) were added. The reaction system was stirred at 100 °C for 2 h, then extracted with ethyl acetate, washed with saturated brine, dried over anhydrous sodium sulfate, filtered, and concentrated at reduced pressure. The crude product was purified by column chromatography to give compound 66d-2 (pale yellow solid, 8.0 g). MS m/z (ESI): 185.2 [M+H]⁺.

Step 3: DIAD (2.89 g, 16.22 mmol) was added to a solution of compound 66d-2 (2 g, 10.86 mmol), 2-[*tert-*butyl(dimethyl)silyl]oxyethanol (2.11 g, 11.95 mmol), and PPh₃ (4.84 g, 18.46 mmol) in THF (10 mL). The reaction system was stirred at 50 °C for 16 h in a nitrogen atmosphere. After the reaction was completed, the reaction solution was concentrated and purified by column chromatography to give compound 66d-3 (white solid, 3.7 g). MS m/z (ESI): 343.2 [M+H]⁺.

Step 4: NBS (2.89 g, 16.22 mmol) and AIBN (0.7 g, 4.26 mmol) were added to a solution of compound 66d-3 (3.7 g, 10.80 mmol) in carbon tetrachloride (10 mL). The reaction system was stirred at 80 °C for 6 h in a nitrogen atmosphere. After the reaction was completed, the reaction solution was concentrated and then purified by column chromatography to give compound 66d-4 (white solid, 2.7 g). MS m/z (ESI): 307.2 [M+H]⁺.

Step 5: DIEA (2.84 g, 21.98 mmol) was added to a solution of compound 66d-4 (2.7 g, 8.79 mmol) and 3-aminopiperidine-2,6-dione hydrochloride (1.52 g, 9.23 mmol) in acetonitrile (30 mL). The reaction system was stirred at 45 °C for 16 h. After the reaction was completed, the reaction solution was concentrated and then purified by column chromatography to give compound 66d-5 (white solid, 1.2 g). MS m/z (ESI): 323.3 [M+H] ⁺.

Step 6: At 0 °C, carbon tetrabromide (1.73 g, 5.21 mmol) was slowly added to a solution of compound 66d-5 (1.2 g, 3.72 mmol) and PPh₃ (1.76 g, 6.70 mmol) in dichloromethane (200 mL). The reaction system was stirred at 20 °C for 2 h. After the reaction was completed, the reaction solution was concentrated and then purified by column chromatography to give compound 66d (white solid, 1 g). MS m/z (ESI): 385.2 [M+H]⁺.

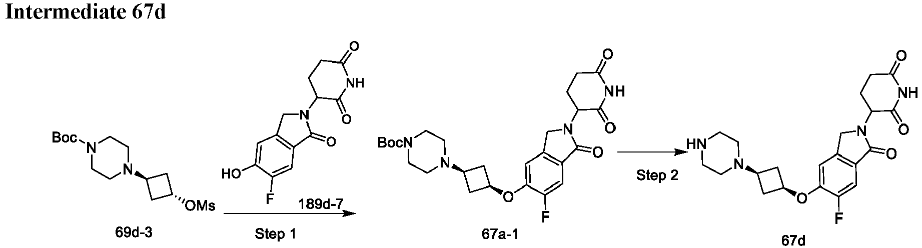

Compound 67d was prepared with reference to step 2 for intermediate 122d as step 1 and with reference to step 2 for intermediate 3b as step 2. MS m/z (ESI): 417.2 [M+H]⁺.

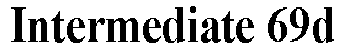

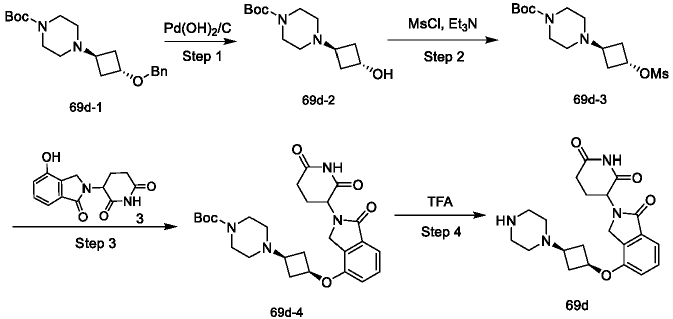

Step 1: Compound 69d-2 was prepared with reference to step 2 for intermediate 70d. ¹H NMR (400 MHz, CD₃OD) δ 4.32-4.26 (m, 1 H), 3.41 (brs, 4 H), 3.01-2.97 (m, 1 H), 2.34-2.31 (m, 4 H), 2.23-2.20 (m, 2 H), 2.03-1.97 (m, 2 H), 1.42 (s, 9 H). MS m/z (ESI): 257.2 [M+H]⁺.

Step 2: Compound 69d-2 was prepared with reference to step 1 for intermediate 3a. MS m/z (ESI): 335.2 [M+H]⁺.

Step 3: Compound 69d-3 was prepared with reference to the method of step 2 for intermediate 122d. MS m/z (ESI): 499.2 [M+H]⁺.

Step 4: Compound 69d was prepared with reference to step 5 for intermediate 70d. MS m/z (ESI): 399.5 [M+H]⁺.

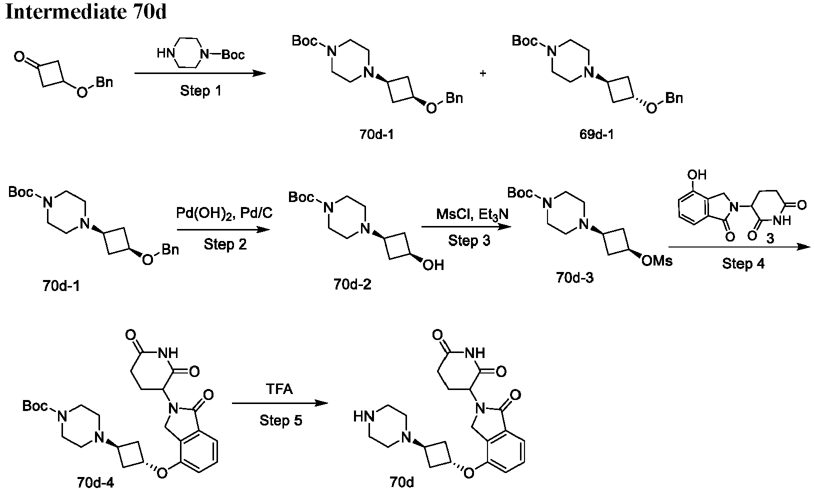

Step 1: Acetic acid (50.40 g, 839.27 mmol) was added to a solution of 3-benzyloxycyclobutanone (30 g, 170.25 mmol) and *tert*-butyl piperazine-1-carboxylate (33 g, 177.18 mmol) in methanol (400 mL). After the reaction system was stirred for 10 min, sodium cyanoborohydride (33.00 g, 525.13 mmol) was added at 0 °C. The reaction was stirred at 18 °C for 2 h, until the completion of the reaction was indicated by TLC (dichloromethane:methanol = 10:1). The reaction solution was concentrated by rotary evaporation, diluted with water, and extracted with ethyl acetate. The organic phases were combined and washed successively with sodium hydroxide solution and saturated brine, dried over anhydrous sodium sulfate, filtered, and concentrated by rotary evaporation. The crude product was separated by column chromatography to give compound 70d-1 (yellow oil, 4 g). MS m/z (ESI): 347.2 [M+H]⁺.

Step 2: Compound 70d-1 (40 g, 115.45 mmol) was dissolved in methanol (500 mL). The system was purged with nitrogen three times, and palladium hydroxide (3.0 g, 2.14 mmol, 10% purity), palladium on carbon (3 g, 10% purity), and acetic acid (525.00 mg, 8.74 mmol, 500.00 µL) were added. The reaction system was stirred in a hydrogen atmosphere at 45 °C for 36 h. The reaction solution was filtered through celite and then concentrated by rotary evaporation. The crude product was purified by column chromatography (dichloromethane:methanol = 10:1) to give compound 70d-2 (white solid, 18.2 g). ¹H NMR (400 MHz, CD₃OD) δ 3.95-3.85 (m, 1 H), 3.41-3.35 (m, 4 H), 2.47-2.44 (m, 2 H), 2.35-2.31 (m, 5 H), 1.76-1.73 (m, 2 H), 1.45 (s, 9 H). MS m/z (ESI): 257.2 [M+H]⁺.

Step 3: Compound 70d-3 was prepared with reference to step 1 for intermediate 3a. MS m/z (ESI): 335.2 [M+H]⁺.

Step 4: Compound 70d-4 was prepared with reference to step 2 for intermediate 122d. MS m/z (ESI): 499.2 [M+H]⁺.

Step 5: Compound 70d-4 (450 mg, 0.9 mmol) was dissolved in DCM (10 mL) before TFA (2 mL) was added. The reaction system was stirred at room temperature for 2 h. After the reaction was completed, the reaction solution was directly concentrated, and the residue was lyophilized with water to give crude compound 70d. MS m/z (ESI): 399.2 [M+H]⁺.

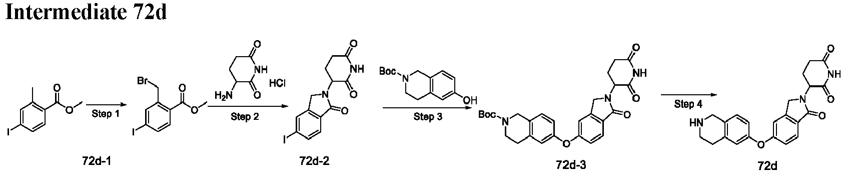

Steps 1-2: Crude compound 72d-2 was prepared with reference to steps 2-3 for intermediate 3d. MS m/z (ESI): 371.2 [M+H]⁺.

Step 3: Compound 72d-2 (200 mg, 0.54 mmol), tert-butyl 6-hydroxy-3,4-dihydroisoquinoline-2(1*H*)-carboxylate (202 mg, 0.81 mmol), CuI (21 mg, 0.11 mmol), L-proline (12 mg, 0.11 mmol), and K₂CO₃ (224 mg, 1.62 mmol) were added to a single-necked flask containing 10 mL of anhydrous DMSO. The mixture was heated to 110 °C in a nitrogen atmosphere and stirred for 16 h. After the reaction was completed, the solution was filtered at reduced pressure. A saturated NH₄Cl solution was added to the filtrate. The mixture was extracted with EA. The organic phase was washed with water and saturated brine, dried over anhydrous sodium sulfate, and subjected to normal-phase column chromatography (DCM/MeOH = 0-5%) to give compound 72d-3 (pale yellow solid, 45 mg). MS m/z (ESI): 492.5 [M+H]⁺.

Step 4: Crude compound 72d was prepared with reference to step 2 for intermediate 3b. MS m/z (ESI): 392.4 [M+H]⁺.

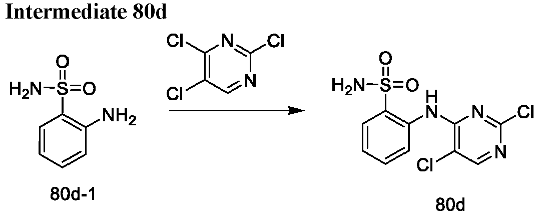

Compound 80d was prepared with reference to the method for intermediate 1d. MS m/z (ESI): 319.2 [M+H]⁺.

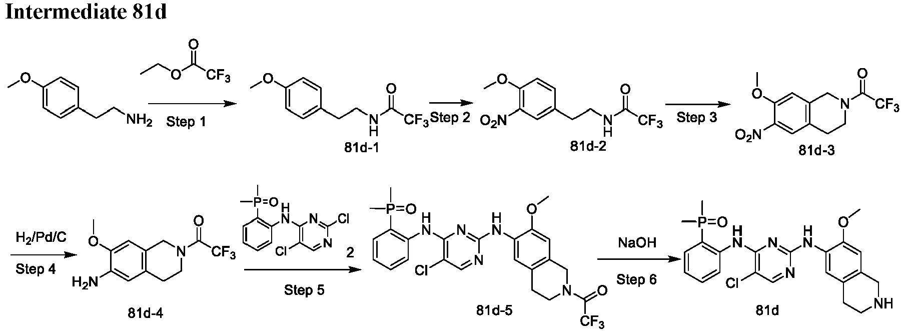

Step 1: 2-(4-Methoxyphenyl)ethan-1-amine (2 g, 13.2 mmol) and solvent MeOH were added to a reaction flask. 2,2,2-trifluoroacetic acid ethyl ester (2.8 g, 19.8 mmol) was then slowly and dropwise added. After the addition, the system was stirred for 3 h at 20 °C in a nitrogen atmosphere. After the reaction was completed, the solution was concentrated, quenched by adding a saturated aqueous NaHCOs solution, and extracted with DCM. The organic phase was washed with saturated brine, dried over anhydrous sodium sulfate, concentrated, and subjected to normal-phase column chromatography (PE/EA = 0-50%) to give compound 81d-1 (pale yellow solid, 2.2 g). MS m/z (ESI): 246.3 [M-H]⁺.

Step 2: Compound 81d-1 (2.2 g, 8.9 mmol) was dissolved in concentrated sulfuric acid (10 mL). In an ice bath, concentrated nitric acid (558 mg, 9.0 mmol) was slowly and dropwise added, and the system was stirred at 0 °C for 2 h in a nitrogen atmosphere. The reaction solution was poured into ice water after the reaction was completed, a saturated aqueous NaHCOs solution was to neutralize the pH to 8-9. A solid was precipitated, and the mixture was filtered to give a filter cake. Water and DCM were added to dissolve the filter cake. The mixture was extracted with DCM, and the organic phase was washed with saturated brine, dried over anhydrous sodium sulfate, and subjected to normal-phase column chromatography (DCM/MeOH = 0-5%) to give compound 81d-2 (pale yellow solid, 2 g). MS m/z (ESI): 293.3 [M+H]⁺.

Step 3: Compound 81d-2 (2 g, 6.8 mmol) was dissolved in HOAc (5 mL) and concentrated sulfuric acid (5 mL). Paraformaldehyde (1 g, 34.1 mmol) was slowly and dropwise added. The mixture was stirred for 5 h in a nitrogen atmosphere at 40 °C, After the reaction was completed, the reaction solution was concentrated, and neutralized to pH 8-9 by adding a saturated aqueous NaHCO₃ solution. Water and DCM were added, and the mixture was extracted with DCM. The organic phase was washed with saturated brine, dried over anhydrous sodium sulfate, and subjected to normal-phase column chromatography (DCM/MeOH = 0-5%) to give compound 81d-3 (pale yellow solid, 1 g). MS m/z (ESI): 305.4 [M+H]⁺.

Step 4: Compound 81d-4 was prepared with reference to step 2 for intermediate 3a. MS m/z (ESI): 275.1 [M+H]⁺.

Step 5: Compound 81d-5 was prepared with reference to step 3 for intermediate 21c. MS m/z (ESI): 554.7 [M+H]⁺.

Step 6: Compound 81d-5 (50 mg, 0.155 mmol) was dissolved in MeOH and water. NaOH (50 mg, 0.155 mmol) was added and the system was stirred at 20 °C for 1 h. After the reaction was completed, the solution was concentrated and extracted with DCM. The organic phase was washed with saturated brine, dried over anhydrous sodium sulfate, and subjected to normal-phase column chromatography (DCM/MeOH = 0-5%) to give compound 81d (pale yellow solid, 60 mg). MS m/z (ESI): 458.6 [M+H]⁺.

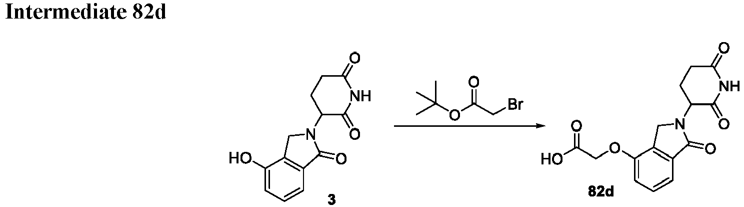

Compound 82d was prepared with reference to the method for intermediate 88d. MS m/z (ESI): 319.2 [M+H]⁺

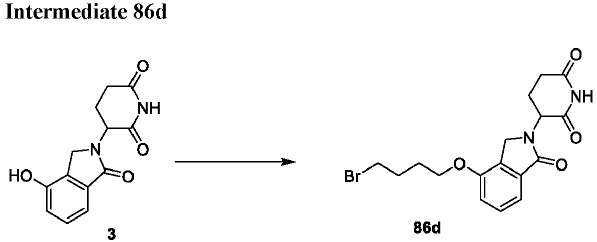

Compound 86d was prepared with reference to the method for intermediate 48d. MS m/z (ESI): 395.2 [M+H]⁺.

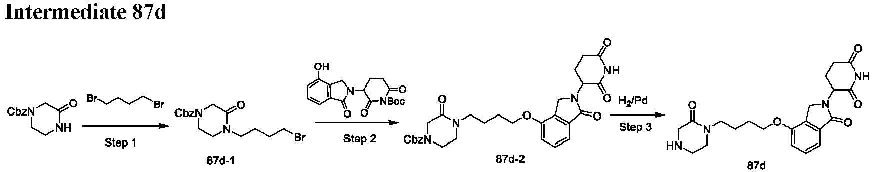

Step 1: Benzyl 3-oxopiperazine-1-carboxylate (1 g, 4.27 mmol) was dissolved in DMF (15 mL). NaH (342 mg, 8.54 mmol) was slowly added. The system was reacted at room temperature for 1 h. 1,4-Dibromobutane (4.5 g, 21.0114 mmol) was added and the system was stirred at 50 °C in a nitrogen atmosphere for 16 h. After the reaction was completed, the solution was concentrated and quenched with water. The reaction mixture was concentrated, and separated and purified by prep-HPLC to give compound 87d-1 (white solid, 1 g, hydrochloride). MS m/z (ESI): 369.1 [M+H]⁺.

Step 2: Compound 87d-1 (500 mg, 1.35 mmol), *tert*-butyl 3-(4-hydroxy-1-oxoisoindolin-2-yl)-2,6-dioxopiperidine-1-carboxylate (390 mg, 1.08 mmol), and solvent DMF (6 mL) were added to a reaction flask. K₂CO₃ (100.6 mg, 0.310 mmol) was then added and stirred at 80 °C for 2 h in a nitrogen atmosphere. After the reaction was completed, the reaction solution was filtered, concentrated, and separated and purified by prep-HPLC to give compound 87d-2 (white solid, 50 mg, hydrochloride). MS m/z (ESI): 549.7 [M+H]⁺.

Step 3: Compound 87d was prepared with reference to step 2 for intermediate 3a. MS m/z (ESI): 415.6 [M+H]⁺.

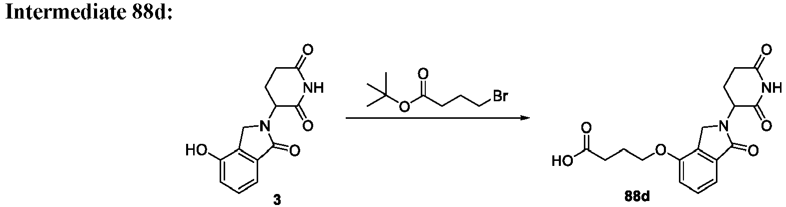

Compound 3 (100 mg, 0.38 mmol), tert-butyl 4-bromobutyrate (170 mg, 0.76 mmol), KI (63 mg, 0.38 mmol), NaHCOs (96 mg, 1.04 mmol), and anhydrous DMF (5 mL) were added in a single-necked flask. The system was heated to 60 °C and stirred for 16 h. After the reaction was completed, the reaction solution was filtered, and the filtrate was directly subjected to pre-HPLC to give compound 88d (white solid, 15 mg). MS m/z (ESI): 347.3 [M+H]⁺.

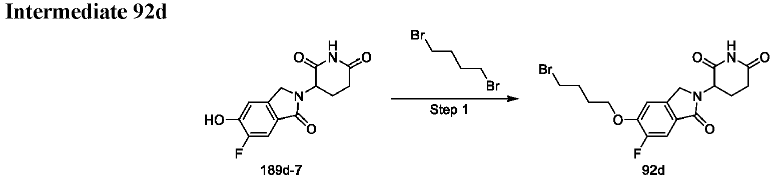

Compound 92d was prepared with reference to the method for intermediate 48d. MS m/z (ESI): 413.4 [M+H]⁺.

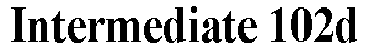

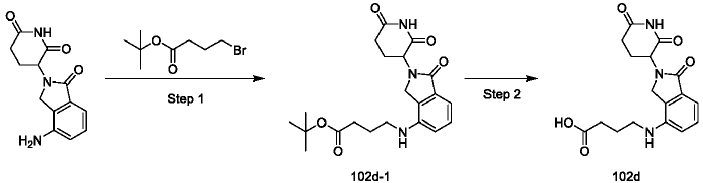

Step 1: Compound 102d-1 was prepared with reference to compound C-1. MS m/z (ESI): 402.9 [M+H]⁺.

Step 2: Compound 102d was prepared with reference to step 2 for intermediate 112d. MS m/z (ESI): 346.2 [M+H]⁺.

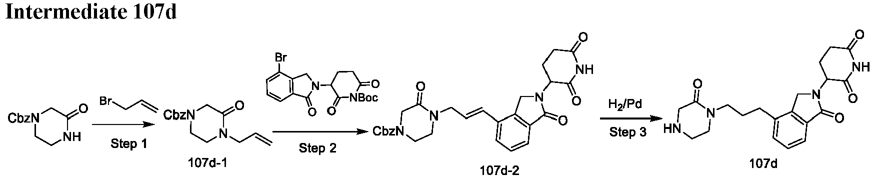

Step 1: Compound 107d-1 was prepared with reference to step 1 for intermediate 87d. MS m/z (ESI): 275.2. [M+H]⁺.

Step 2: Compound 107d-2 was prepared with reference to step 1 for intermediate 114d. MS m/z (ESI): 517.2. [M+H]⁺.

Step 3: Compound 107d was prepared with reference to step 2 for intermediate 3a. MS m/z (ESI): 385.9 [M+H]⁺.

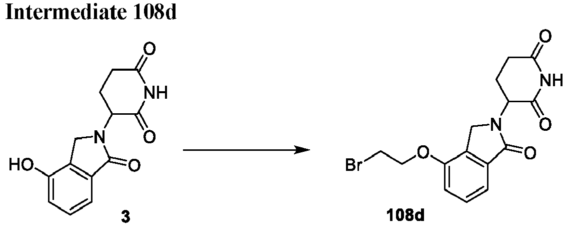

Compound 86d was prepared with reference to the method for intermediate 48d. MS m/z (ESI): 367.2 [M+H]⁺.

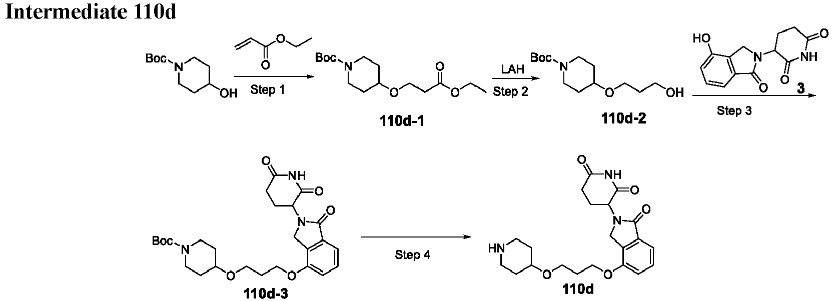

Step 1: *tert*-Butyl 4-hydroxypiperidine-1-carboxylate (3000 mg, 14.90 mmol), ethyl acrylate (2983 mg, 29.80 mmol), KOH (1672 mg, 29.80 mmol), and anhydrous THF (50 mL) were added to a single-necked flask. The reaction system was stirred at room temperature in a nitrogen atmosphere for 24 h. After the reaction was completed, water was added to quench the reaction. The mixture was extracted with EA, and the organic phase was washed with saturated brine and dried over anhydrous sodium sulfate. The crude product was subjected to normal-phase column chromatography (EA/PE = 0-15%) to give compound 110d-1 (pale yellow oil, 2100 mg).

Step 2: Compound 110d-1 (500 mg, 1.66 mmol) and anhydrous THF (10 mL) were added to a three-necked flask. In a nitrogen atmosphere, LiAlH₄ (75 mg, 1.99 mmol) was added in portions in an ice-water bath. After the reaction was completed, anhydrous Na₂SO₄ was added to the reaction solution, and water was slowly and dropwise added to quench the reaction. The mixture was filtered at reduced pressure, the residue was washed with DCM, and the filtrate was concentrated to give crude product 110d-2 (pale yellow oil, 410 mg). The crude product was directly used in the next step.

Step 3: Compound 110d-2 (400 mg, 1.54 mmol), compound 3 (400 mg, 1.54 mmol), PPh₃ (606 mg, 2.31 mmol), and anhydrous THF (10 mL) were added in a three-necked reaction flask. In a nitrogen atmosphere, DIAD (467 mg, 2.31 mmol) was slowly and dropwise added. After the reaction was completed, water was added to the reaction solution to quench the reaction. The mixture was extracted with DCM, and the organic phases were combined, washed with saturated brine, dried over anhydrous Na₂SO₄, and concentrated at reduced pressure. The crude product was subjected to normal-phase column chromatography (MeOH/DCM = 0-5%) to give product 110d-3 (pale yellow oil, 120 mg). MS m/z (ESI): 502.6 [M+H]⁺.

Step 4: Crude compound 110d was prepared with reference to step 2 for intermediate 3b. MS m/z (ESI): 402.7 [M+H]⁺.

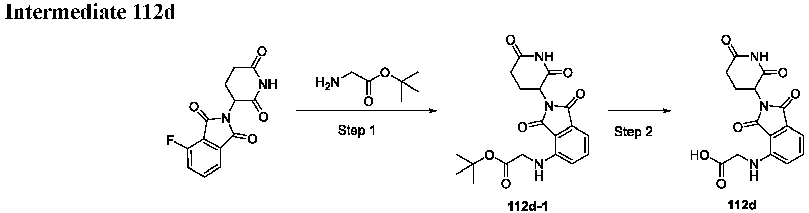

Step 1: 2-(2,6-Dioxopiperidin-3-yl)-4-fluoroisoindoline-1,3-dione (1000 mg, 3.62 mmol), DIEA (930 mg, 7.24 mmol), and NMP (10 mL) were added in a three-necked reaction flask. *tert*-Butyl glycinate (570 mg, 4.34 mmol) was then added, and the system was heated to 110 °C and stirred overnight. After the reaction was completed, water was added to the reaction solution, and the mixture was extracted with EA. The organic phases were combined, washed with saturated brine, dried over anhydrous sodium sulfate, and dried at reduced pressure. The crude product was purified by normal-phase column chromatography (EA/PE = 0-60%) to give product 112d-1 (pale yellow oil, 600 mg). MS m/z (ESI): 388.5 [M+H]⁺.

Step 2: Compound 112d-1 (600 mg, 1.55 mmol) was dissolved in DCM (10 mL) before TFA (2 mL) was added. The system was then stirred at room temperature for 1 h. After the reaction was completed, the solvent was removed at reduced pressure. Water and acetonitrile were added to the crude product, and the mixture was lyophilized to give crude compound 112d (pale yellow solid, 450 mg). MS m/z (ESI): 332.4 [M+H]⁺.

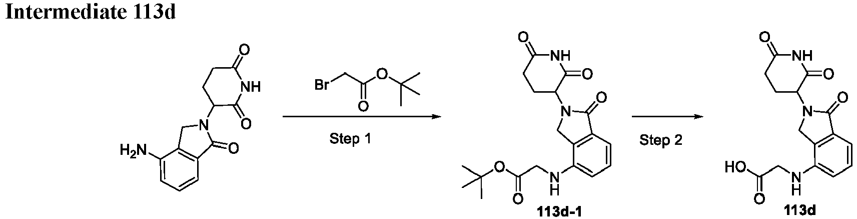

Step 1: Lenalidomide (1000 mg, 3.85 mmol) and NMP (10 mL) were added in a three-necked reaction flask. *tert*-Butyl bromoacetate (900 mg, 4.62 mmol) and DIPEA (995 mg, 7.70 mmol) were then separately added before the mixture was heated to 110 °C and stirred overnight. After the reaction was completed, water was added to the reaction solution. The mixture was extracted with EA. The organic phases were combined, washed with saturated brine, dried over anhydrous sodium sulfate, and concentrated by rotary evaporation at reduced pressure. The crude product was purified by normal-phase column chromatography (EA/PE = 0-70%) to give product 113d-1 (pale yellow oil, 700 mg). MS m/z (ESI): 374.4 [M+H]⁺.

Step 2: Compound 113d was prepared with reference to step 2 for intermediate 112d. MS m/z (ESI): 318.4 [M+H]⁺.

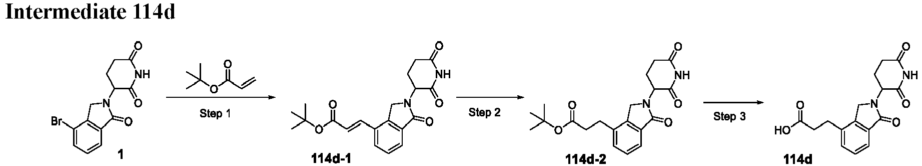

Step 1: Compound 1 (1000 mg, 3.09 mmol), tert-butyl acrylate (475 mg, 3.71 mmol), Pd₂(dba)₃ (282 mg, 0.31 mmol), *t*-Bu₃PBF₄ (90 mg, 0.31 mmol), dicyclohexylamine (1120 mg, 6.18 mmol), and anhydrous 1,4-dioxane (15 mL) were added in a single-necked flask. In a nitrogen atmosphere, the system was slowly heated to 60 °C and stirred overnight. After the reaction was completed, the reaction solution was filtered at reduced pressure. The filtrate was then concentrated at reduced pressure, and the crude product was isolated by normal-phase column chromatography (EA/PE = 0-30%) to give compound 114d-1 (pale yellow solid, 700 mg). MS m/z (ESI): 371.5 [M+H]⁺.

Step 2: Compound 114d-2 was prepared with reference to step 2 for intermediate 3a. MS m/z (ESI): 373.5 [M+H]⁺.

Step 3: Compound 114d was prepared with reference to step 2 of the synthesis method for intermediate 112d. MS m/z (ESI): 317.7 [M+H]⁺.

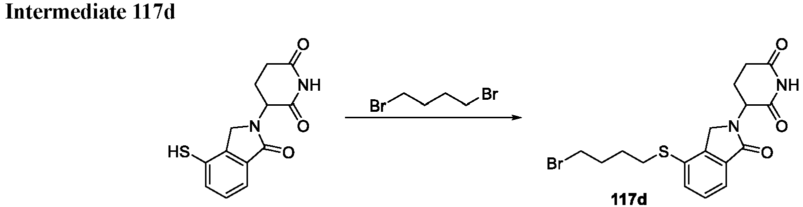

3-(4-mercapto-1-oxoisoindolin-2-yl)piperidine-2,6-dione (1000 mg, 3.62 mmol) and anhydrous DMF (10 mL) were added in a single-necked reaction flask before K₂CO₃ (600 mg, 4.34 mmol) and 1,4-dibromobutane (860 mg, 3.98 mmol) were separately added. The reaction system was stirred at room temperature for 0.5 h. After the reaction was completed, the reaction solution was filtered at reduced pressure, and then a saturated NH₄Cl solution was added to the filtrate. The mixture was extracted with EA, and the organic phases were combined, washed with saturated brine, dried over anhydrous sodium sulfate, and concentrated at reduced pressure. The crude product was purified by normal-phase column chromatography (EA/PE = 0-60%) to give product 117d (khaki solid, 500 mg). MS m/z (ESI): 411.3 [M+H]⁺.

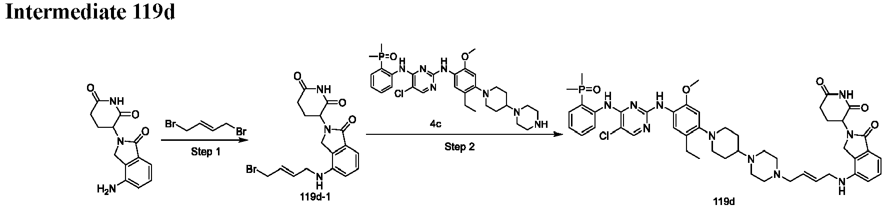

Step 1: Lenalidomide (300 mg, 1.16 mmol) was dissolved in NMP (10 mL) before DIEA (0.64 mL, 3.47 mmol) and 1,4-dibromobutene (297 mg, 1.39 mmol) were added. The reaction system was stirred at room temperature for 16 h. After the reaction was completed, the reaction solution was diluted with EA and washed with water. The organic phase was dried over anhydrous sodium sulfate, filtered, concentrated, and purified by column chromatography (80 g, MeOH/DCM = ~8%) to give compound 119d-1 (white solid, 150 mg). MS m/z (ESI): 392.7 [M+H]⁺.

Step 2: Compound 119d was prepared with reference to the method for compound C-1. MS m/z (ESI): 909.5 [M+H]⁺. E29

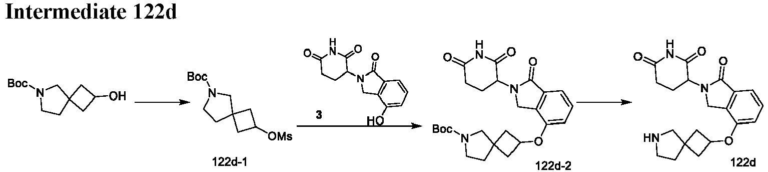

Step 1: Compound 122d-1 was prepared with reference to step 2 for intermediate 1b. M/Z (ESI): 250.1 [M+H-56]⁺.

Step 2: Cesium carbonate (1.25 g, 3.84 mmol) and compound 3 (0.50 g, 1.92 mmol) were added to a solution of compound 122d-1 (704 mg, 2.31 mmol) in DMF (10.0 mL). The reaction system was stirred at 80 °C for 16 h. After the reaction was completed, the reaction mixture was diluted with dichloromethane. The organic phase was washed with water, dried, and concentrated to give compound 122d-2 (colorless oily liquid, 0.60 g). MS m/z (ESI): 414.1 [M+H-56]⁺.

Step 3: Compound 122d was prepared with reference to step 2 for intermediate 3b. MS m/z (ESI): 370.1 [M+H]⁺

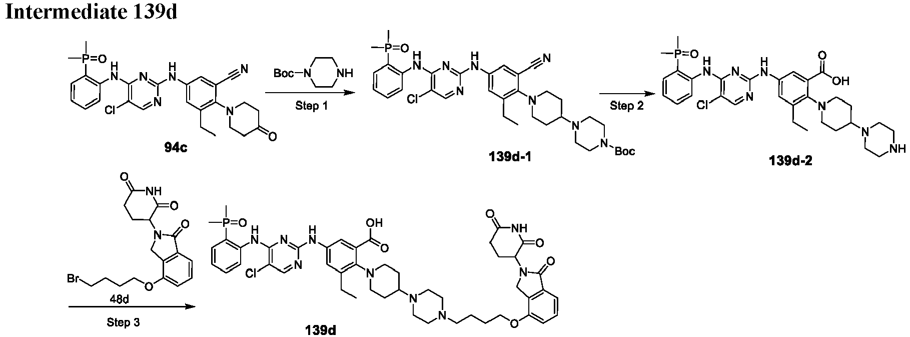

Step 1: Compound 139d-1 was prepared with reference to compound E67. MS m/z (ESI): 693.3 [M+H]⁺.

Step 2: Compound 139c-2 (1.5 g, 2.16 mmol) was added to a mixture of concentrated sulfuric acid (2.5 mL) and water (5 mL). The reaction system was purged with nitrogen 3 times, heated to 120 °C, and stirred for 3 h. After the reaction was completed, the reaction mixture was adjusted to a neutral system pH with solid NaOH, diluted with water, filtered, and concentrated to give a crude product. The crude product was subjected to reverse-phase preparative chromatography (trifluoroacetic acid system) to give compound 139d-2 (yellow solid, 1.3 g). MS m/z (ESI): 612.0 [M+H]⁺.

Step 3: Compound 139d was prepared with reference to the method for compound C-1. MS m/z (ESI): 926.3 [M+H]⁺.

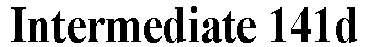

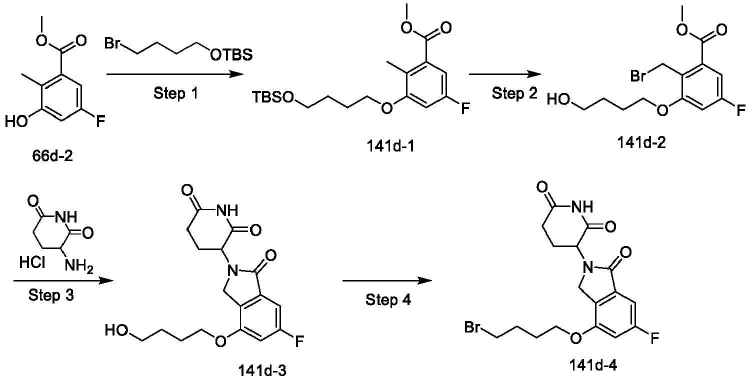

Compound 141d was prepared with reference to steps 3-6 for compound 66d. MS m/z (ESI): 413.1[M+H]⁺.

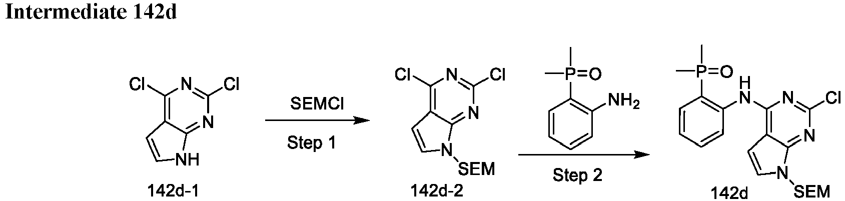

Step 1: Compound 142d-1 (10 g, 53.2 mmol), DMF (30 mL), and THF (90 mL) were added to a single-necked flask before NaH (2.34 g, 58.5 mmol, 60%) was added in portions in an ice-water bath. The system was stirred at 0 °C for 0.5 h and at 20 °C for 0.5 h. The system was then cooled to 0 °C before SEMCl (9.31 g, 55.9 mmol) was slowly and dropwise added, and stirred at 20 °C for 1.5 h. After the reaction was completed, the reaction solution was diluted with EA. The organic phase was washed with a saturated ammonium chloride solution and a saturated aqueous sodium chloride solution, dried over anhydrous sodium sulfate, filtered, and concentrated by rotary evaporation. The crude product was separated by column chromatography (eluent: EA/PE = 0-15%) to give compound 142d-2. ¹H NMR (400 MHz, CDCl₃) δ 7.38 (d, *J* = 3.7 Hz, 1 H), 6.67 (d, *J=* 3.7 Hz, 1 H), 5.61 (s, 2 H), 3.54 (dd, *J=* 8.7, 7.6 Hz, 2 H), 0.98-0.89 (m, 2 H),-0.03 (s, 9 H). MS m/z (ESI): 318.1 [M+H]⁺.

Step 2: 2-Dimethylphosphorylaniline (3.5 g, 20.7 mmol) and compound 142d-2 (6.6 g, 20.7 mmol) were dissolved in DMF (40 mL) before K₂CO₃ (3.43 g, 24.9 mmol) and DIPEA (3.2 g, 24.8 mmol, 4.3 mL) were separately added. The reaction system was then stirred at 60 °C for 16 h. After the reaction was completed, the reaction solution was diluted with ethyl acetate. The organic phase was washed twice with a saturated ammonium chloride solution, washed with a saturated aqueous sodium chloride solution, dried over anhydrous sodium sulfate, filtered, and concentrated by rotary evaporation. The crude product was separated by column chromatography (EA/PE = 0-77%) to give compound 142d. MS m/z (ESI): 451.6 [M+H] ⁺.

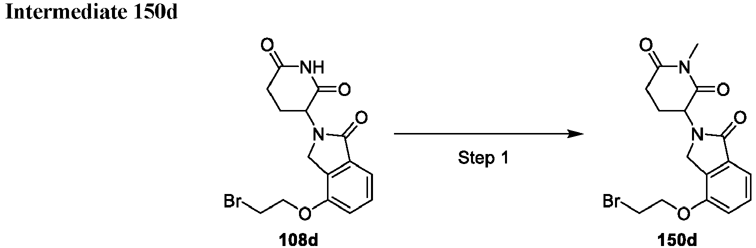

Compound 150d was prepared with reference to step 1 for intermediate 3d. MS m/z (ESI): 381.0 [M+H]⁺.

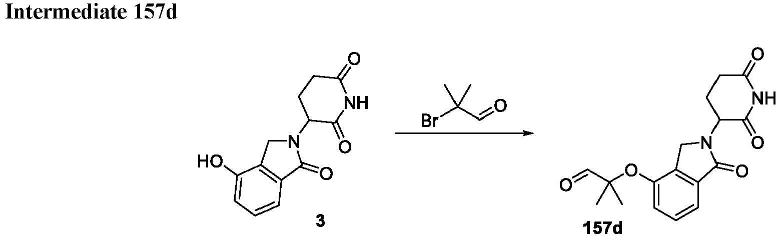

Compound 3 (150 mg, 0.58 mmol) was dissolved in DMF (5 mL) before 2-bromo-2-methylpropionaldehyde (105 mg, 0.69 mmol), KHCOs (116 mg, 1.16 mmol), and NaI (110 mg, 0.58 mmol) were separately added. The reaction system was heated to 80 °C and stirred for 16 h. After the reaction was completed, the reaction solution was filtered at reduced pressure. The filtrate was concentrated by rotary evaporation at reduced pressure, and the crude product was separated by normal-phase column chromatography (MeOH/DCM = 0-5%) to give crude compound 157d. MS m/z (ESI): 331.1 [M+H]⁺.

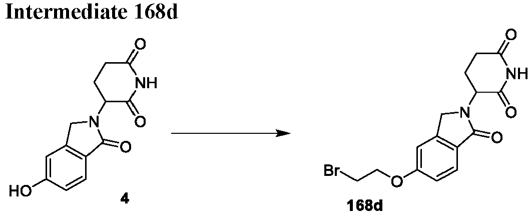

Compound 168d was prepared with reference to the method for intermediate 48d. MS m/z (ESI): 367.1, 369.1 [M+H]⁺.

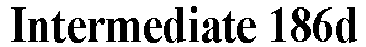

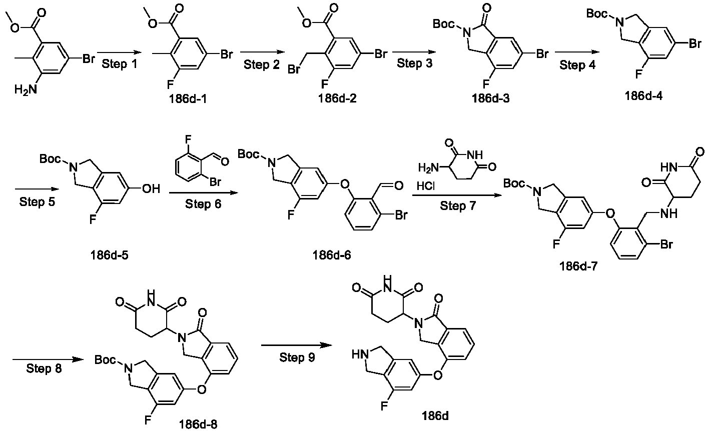

Step 1: At 0 °C, nitronium tetrafluoroborate (6.84 g, 58.59 mmol) was added to methyl 3-amino-5-bromo-2-methylbenzoate (11 g, 45.07 mmol) in DCM (100 mL). The reaction system was stirred at 0 °C in a nitrogen atmosphere for 1 h. *o*-Xylene (250 mL) was then added and the system was stirred at 80 °C for 1 h. Dichloromethane was removed and the system was stirred at 130 °C for 3 h. H₂O was added and the reaction mixture was then extracted with EA. The combined organic phases were washed with saturated brine, dried over sodium sulfate, filtered, and concentrated at reduced pressure. The crude product was separated by column chromatography to give compound 186d-1. MS m/z (ESI): 247.1 [M+H]⁺.

Step 2: NBS (9.37 g, 52.62 mmol) and AIBN (1.33 g, 8.10 mmol) were added to a solution of compound 186d-2 (10 g, 40.48 mmol) in carbon tetrachloride (100 mL). The reaction system was stirred at 80 °C for 16 h. The reaction solution was concentrated at reduced pressure. The crude product was separated by column chromatography to give compound 186d-2. MS m/z (ESI): 325.1 [M+H]⁺.

Step 3: Compound 186d-2 (8.5 g, 26.08 mmol) was added to a solution of NH₃/MeOH (7 M, 76.50 mL) in MeOH (10 mL), and the reaction system was stirred for 16 h at 20 °C. The reaction solution was concentrated at reduced pressure to give a crude product. The crude product was dissolved in THF (100 mL). DMAP (547.18 mg, 4.48 mmol) and BoczO (8.25 g, 37.82 mmol) were added. The reaction system was stirred at room temperature for 16 h. The reaction solution was concentrated by rotary evaporation. The crude product was separated by column chromatography to give compound 186d-3. MS m/z (ESI): 330.1 [M+H]⁺.

Step 4: BH₃.Me₂S (10 M, 12.12 mL) was added to a solution of compound 186d-3 (4.00 g, 12.12 mmol) in THF (100 mL) at 0 °C. The reaction system was stirred for reaction for 1 h at 0 °C, for 1 h at 20 °C, and for 14 h at 65 °C. Methanol and water were added to the reaction solution at 0 °C. The reaction solution was extracted with EA. The organic phase was dried over sodium sulfate, filtered, and concentrated at reduced pressure. The crude product was subjected to column chromatography to give compound 186d-4. MS m/z (ESI): 316.1 [M+H]⁺.

Step 5: A mixture of compound 186d-4 (2 g, 6.33 mmol), palladium acetate (150.00 mg, 668.13 µmol), boric acid (584 mg, 9.44 mmol), cesium carbonate (4.12 g, 12.65 mmol) and *t*-BuBrettPhos (600 mg, 1.24 mmol) in NMP (30 mL) was stirred at 90 °C for 16 h in a nitrogen atmosphere. Water was added to the reaction solution, and the mixture was extracted with EA. The organic phases were combined, washed with brine, dried over anhydrous sodium sulfate, filtered, and concentrated by rotary evaporation. The crude product was subjected to column chromatography to give compound 186d-5. MS m/z (ESI): 254.1 [M+H]⁺.

Steps 6-9: Compound 186d was prepared with reference to steps 2-5 for intermediate 120c. MS m/z (ESI): 396.1 [M+H]⁺.

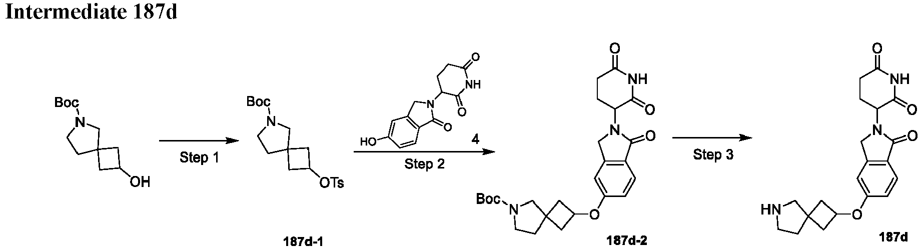

Step 1: *tert*-Butyl 2-hydroxy-6-azaspiro[3.4]octane-6-carboxylate (500 mg, 2.20 mmol) and TEA (334.42 mg, 3.30 mmol) were dissolved in DCM (20 mL) before 4-toluenesulfonyl chloride (630 mg, 3.30 mmol) was added. The reaction system was reacted at 25 °C for 18 h. The reaction solution was diluted with water and extracted with EA. The organic phases were combined, washed once with a 1 N hydrochloric acid solution, washed twice with saturated brine, dried over anhydrous sodium sulfate, filtered, and concentrated by rotary evaporation. The crude product was subjected to column chromatography to give compound 187d-1. MS m/z (ESI): 382.1 [M+H]⁺.

Step 2: Compound 4 (220 mg, 845.36 mmol), compound 187d-1 (600 mg, 1.57 mmol), and cesium carbonate (700 mg, 2.15 mmol) were dissolved in DMF (10 mL) before NaI (150 mg, 1.00 mmol) was added. The reaction system was stirred at 80 °C for 18 h. The reaction solution was diluted with water and extracted twice with DCM. The organic phases were combined, dried over anhydrous sodium sulfate, filtered, and concentrated by rotary evaporation. The crude product was subjected to column chromatography to give compound 187d-2. MS m/z (ESI): 470.2 [M+H]⁺.

Step 3: Compound 187d was prepared with reference to step 5 for intermediate 70d. MS m/z (ESI): 370.1 [M+H]⁺.

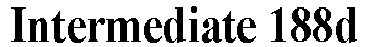

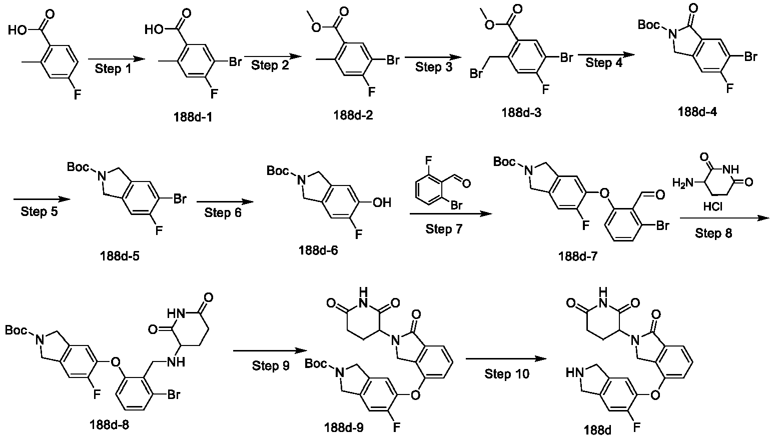

Step 1: NBS (12 g, 67.47 mmol) was added to 4-fluoro-2-methylbenzoic acid (10 g, 64.88 mmol) in sulfuric acid (100 mL) at 0 °C. The reaction system was stirred at 0 °C for 1 h. After the reaction was completed, the reaction solution was added to 2 L of ice water. The mixture was filtered and washed with water. The solid was dried to give compound 188d-1(15.1 g, white solid). MS m/z (ESI): 233.2 [M+H]⁺.

Step 2: Methyl iodide (27.59 g, 194.39 mmol) was added to compound 188d-1 (15.1 g, 64.80 mmol) and potassium carbonate (26.87 g, 194.39 mmol) in DMF (60 mL) at 0 °C. The reaction system was stirred at 20 °C for 16 h, until the completion of the reaction was indicated by TLC (PE/EA = 1:1). The reaction solution was diluted with 200 mL of ethyl acetate, washed with saturated brine (100 mL × 3), dried over anhydrous sodium sulfate, filtered, and concentrated by rotary evaporation to give compound 188d-2. MS m/z (ESI): 247.2 [M+H]⁺.

Steps 3-10: Compound 188d was prepared with reference to steps 2-9 for intermediate 186d. MS m/z (ESI): 396.2 [M+H]⁺.

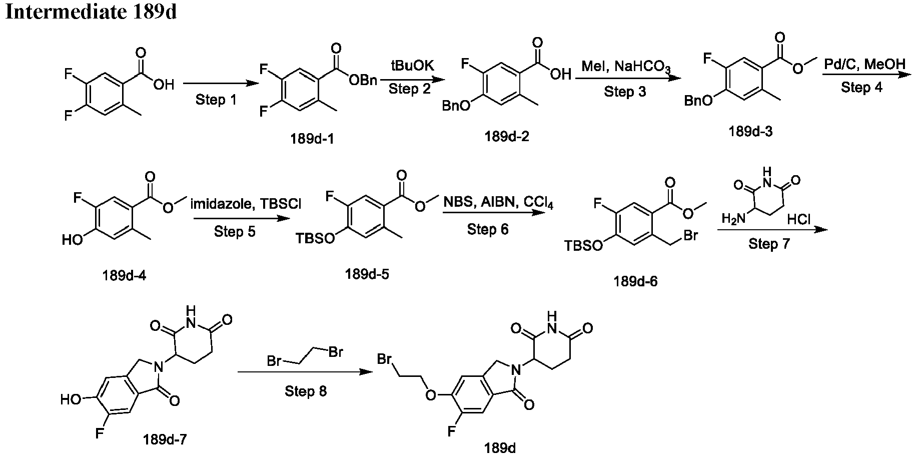

Step 1: BnBr (2.98 mL, 24.4 mmol) was added to a solution of 4,5-difluoro-2-methylbenzoic acid (3.5 g, 20.33 mmol) and K₂CO₃ (5.62 g, 40.66 mmol) in DMF (15 mL) at 0 °C with stirring. The reaction system was stirred at 25 °C for 16 h. After the reaction was completed, a saturated aqueous sodium bicarbonate solution was added. The reaction solution was concentrated to give a crude product. The crude product was separated and purified by silica gel column chromatography (PE:EA = 20:1-10:1) to give compound 189d-1. MS m/z (ESI): 263.2 [M+H]⁺.

Step 2: Compound 189d-1 (5.33 g, 20.32 mmol) was dissolved in THF (15 mL) at 0 °C. t-BuOK (6.84 g, 60.97 mmol) was added in portions with stirring. The reaction system was then stirred at 25 °C for 16 h. After the reaction was completed, a saturated sodium bicarbonate was added. The reaction solution was concentrated to give a crude product. The crude product was separated and purified by silica gel column chromatography (PE:EA = 20:1-10:1) to give compound 189d-2. MS m/z (ESI): 261.2 [M+H]⁺.

Step 3: Compound 189d-3 was prepared with reference to step 1 for intermediate 3d. MS m/z (ESI): 275.2 [M+H]⁺.

Step 4: Compound 114d-2 was prepared with reference to step 2 for intermediate 3a. MS m/z (ESI): 185.2 [M+H]⁺.

Step 5: Compound 189d-4 (0.7 g, 3.8 mmol) and imidazole (1.04 g, 15.2 mmol) were dissolved in DMF (15 mL) at 25 °C. TBSCl (1.15 g, 7.6 mmol) was added in portions with stirring. The reaction system was then stirred at 25 °C for 16 h. After the reaction was completed, a saturated sodium bicarbonate was added. The reaction solution was concentrated to give a crude product. The crude product was separated and purified by silica gel column chromatography (PE/EA = 20:1-10:1) to give compound 189d-5. MS m/z (ESI): 299.2 [M+H]⁺.

Steps 6-7: Compound 189d-7 was prepared with reference to steps 2-3 for intermediate 211d. MS m/z (ESI): 279.2 [M+H]⁺.

Step 8: Compound 189d was prepared with reference to the method for intermediate 48d. ¹H NMR (500 MHz, DMSO-*d*₆) δ 10.98 (s, 1H), 7.55 (d, *J=* 9.9 Hz, 1H), 7.46 (d, *J=* 7.3 Hz, 1H), 5.09 (dd, *J=* 13.3, 5.1 Hz, 1H), 4.49 (dd, *J=* 6.3, 4.8 Hz, 2H), 4.39 (d, *J* = 17.2 Hz, 1H), 4.27 (d, *J* = 17.2 Hz, 1H), 3.88 (t, *J=* 5.4 Hz, 2H), 2.95-2.86 (m, 1H), 2.59 (d, *J =* 16.6 Hz, 1H), 2.44-2.35 (m, 1H), 2.04-1.93 (m, 1H). MS m/z (ESI): 385.5 [M+H]⁺.

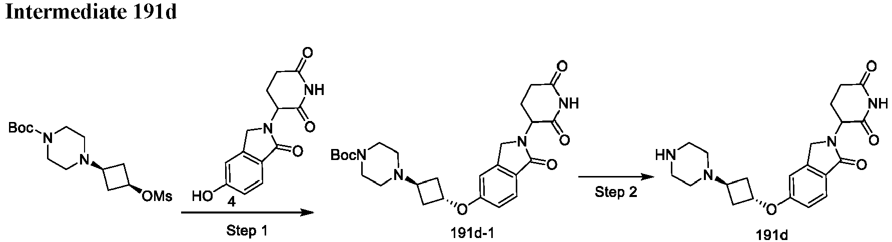

Compound 191d was prepared with reference to step 2 for intermediate 122d as step 1 and with reference to step 2 for intermediate 3b as step 2. MS m/z (ESI): 399.1 [M+H]⁺.

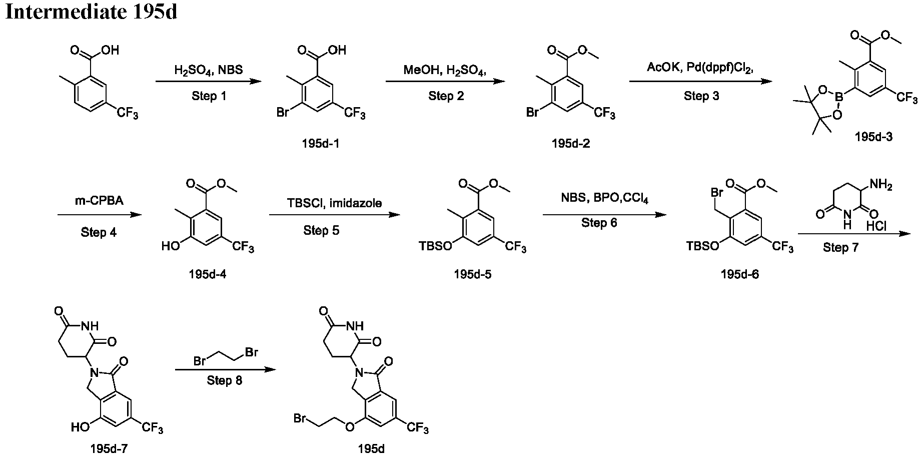

Step 1: 2-Methyl-5-trifluoromethylbenzoic acid (300 mg, 1.38 mmol) and NBS (196.58 mg, 687.53 µmol) were added to sulfuric acid (3 mL). The reaction system was stirred at 25 °C for 12 h. After the reaction was completed, the reaction mixture was diluted with ice water and extracted with EA. The organic layers were combined, washed with saturated brine, dried over anhydrous sodium sulfate, filtered, and concentrated to give a crude product. The crude product was separated and purified by a silica gel plate to give compound 195d-1 (colorless oil, 110 mg). MS m/z (ESI): 283.1 [M+H]⁺.

Step 2: Compound 195d-1 (5.33 g, 18.83 mmol) was added to methanol (50 mL) before sulfuric acid (37.66 mmol, 2.05 mL) was added. The reaction system was stirred at 65 °C for 12 h. After the reaction was completed, the reaction mixture was diluted with ice water and extracted with dichloroethane. The organic layers were combined, washed with saturated brine, dried over anhydrous sodium sulfate, filtered, and concentrated to give compound 195d-2 (yellow oil, 5.5 g), which was directly used in the next step. MS m/z (ESI): 297.1 [M+H]⁺.

Step 3: Compound 195d-2 (4 g, 13.46 mmol), bis(pinacolato)diboron (5.13 g, 20.20 mmol), potassium acetate (3.96 g, 40.39 mmol), Pd(dppf)Cl₂ (985.24 mg, 1.35 mmol) were added to dioxane (30 mL). The reaction system was stirred at 80 °C for 1 h in a nitrogen atmosphere. After the reaction was completed, the reaction mixture was filtered and concentrated to give compound 195d-3 (brown solid, 4.5 g), which was directly used in the next step. MS m/z (ESI): 345.1 [M+H]⁺.

Step 4: Compound 195d-3(4 g, 11.62 mmol) and *m*-CPBA (2.01 g, 11.62 mmol) were added to a mixed solution of water (2 mL) and ethanol (4 mL). The reaction system was stirred at 25 °C for 1 h in a nitrogen atmosphere. After the reaction was completed, the reaction solution was quenched with a sodium sulfite solution, diluted with water, and extracted with ethyl acetate. The organic layers were combined, washed with saturated brine, dried over anhydrous sodium sulfate, filtered, and concentrated to give a crude product. The crude product was purified by column chromatography to give compound 195d-4 (white solid, 2.5 g). MS m/z (ESI): 235.1 [M+H]⁺.

Step 5: Compound 195d-5 was prepared with reference to step 5 for intermediate 189d. MS m/z (ESI): 349.1 [M+H]⁺.

Step 6: Compound 195d-5 (2.5 g, 7.18 mmol), NBS (1.40 g, 7.89 mmol), and BPO (173.80 mg, 717.50 µmol) were added to CCl₄ (30 mL). The reaction system was stirred at 80 °C for 2 h in a nitrogen atmosphere. After the reaction was completed, the mixture was filtered and concentrated to give compound 195d-6 (yellow oil, 3.0 g), which was directly used in the next step. MS m/z (ESI): 427.1 [M+H]⁺.

Step 7: Compound 195d-6 (3 g, 7.02 mmol), 3-aminopiperidine-2,6-dione (989.46 mg, 7.72 mmol, hydrochloride), and DIEA (2.72 g, 21.06 mmol, 3.67 mL) were added to acetonitrile (30 mL). The reaction system was stirred at 25 °C for 2 h in a nitrogen atmosphere. After the reaction was completed, the reaction solution was filtered and concentrated to give compound 195d-7 (blue solid, 1.4 g), which was directly used in the next step. MS m/z (ESI): 329.0 [M+H]⁺.

Step 8: Compound 195d (yellow solid, 180 mg) was prepared with reference to the synthesis method for intermediate 48d. ¹H NMR (500 MHz, DMSO-*d*₆) δ 11.00 (s, 1H), 7.62 (d, *J* = 6.0 Hz, 2H), 5.15 (dd, *J=* 13.3, 5.1 Hz, 1H), 4.61 (td, *J =* 5.0, 2.6 Hz, 2H), 4.53-4.46 (m, 1H), 4.39-4.35 (m, 1H), 3.85 (t, *J =* 5.3 Hz, 2H), 2.95-2.90 (m, 1H), 2.55 (d, *J =* 20.0 Hz, 1H), 2.49-2.47 (m, 1H), 2.05-1.98 (m, 1H). MS m/z (ESI): 435.3 [M+H]⁺.

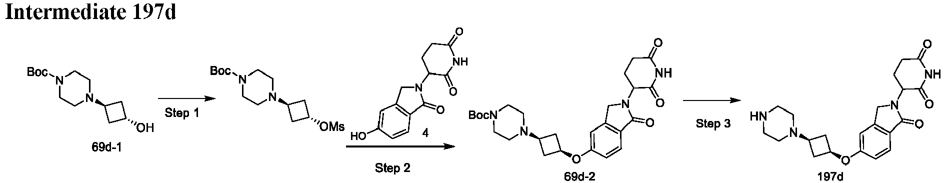

Compound 197d was prepared with reference to the method for intermediate 70d. MS m/z (ESI): 399.7 [M+H]⁺.

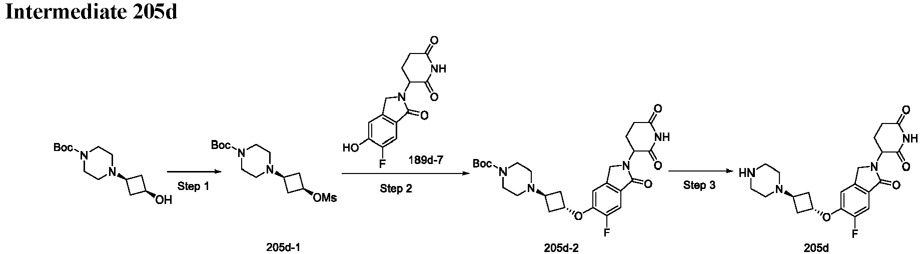

Compound 205d was prepared with reference to step 2 for intermediate 1b as step 1, with reference to step 2 for intermediate 122d as step 2, and with reference to step 2 for intermediate 3b as step 3. MS m/z (ESI): 417.7 [M+H]⁺.

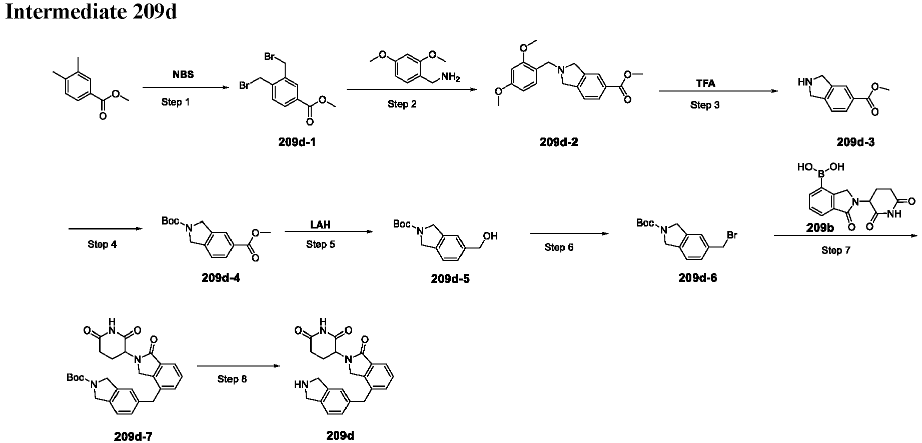

Step 1: Methyl 3,4-dimethylbenzoate (2000 mg, 12.18 mmol) and DCE (30 mL) were added together in a single-necked reaction flask before AIBN (200 mg, 1.22 mmol) and NBS (4770 mg, 26.80 mmol) were separately added. In a nitrogen atmosphere, the reaction system was stirred at 80 °C for 16 h. After the reaction was completed, the reaction solution was cooled to room temperature, adjusted to pH 8-9 with a saturated NaHCOs solution, and extracted with DCM. The organic phases were combined, dried over anhydrous Na₂SO₄, and concentrated at reduced pressure. The crude product was purified by normal-phase column chromatography to give product 209d-1 (yellowish brown solid, 3500 mg). MS m/z (ESI): 321.1 [M+H]⁺. Step 2: Compound 209d-1 (1500 mg, 4.66 mmol) and 2,4-dimethoxybenzylamine (779 mg, 4.66 mmol) were added to a single-necked reaction flask before TEA (1416 mg, 13.98 mmol) and anhydrous THF (20 mL) were added. In a nitrogen atmosphere, the system was stirred at room temperature for 5 h. After the reaction was completed, water was added to the reaction solution, and the mixture was extracted with EA. The organic phases were combined, dried over anhydrous Na₂SO₄, and concentrated at reduced pressure. The crude product was purified by normal-phase column chromatography to give product 209d-2 (reddish brown solid, 1000 mg). MS m/z (ESI): 328.6 [M+H]⁺.

Step 3: Compound 209d-2 (200 mg, 0.61 mmol) and TFA (4 mL) were added to a microwave tube. The system was heated to 140 °C by microwave and stirred for 0.5 h. After the reaction was completed, the reaction solution was concentrated at reduced pressure to give crude product 209d-3 (reddish brown oil, 150 mg), which was used directly in the next step. MS m/z (ESI): 177.9 [M+H]⁺.

Step 4: Compound 209d-3 (150 mg, 0.85 mmol), anhydrous DCM (10 mL), and TEA (430 mg, 4.25 mmol) were added in a single-necked reaction flask before (Boc)₂O (1416 mg, 13.98 mmol) was slowly added. In a nitrogen atmosphere, the system was stirred at room temperature for 2 h. After the reaction was completed, water was added to the reaction solution to quench the reaction, and the mixture was extracted with DCM. The organic phases were combined, dried over anhydrous Na₂SO₄, and concentrated at reduced pressure. The crude product was purified by normal-phase column chromatography to give product 209d-4 (pale yellow solid, 200 mg). MS m/z (ESI): 278.1 [M+H]⁺.

Step 5: Compound 209d-5 was prepared with reference to step 8 for intermediate 111b.

Step 6: Compound 209d-5 (150 mg, 0.60 mmol), anhydrous DCM (10 mL), and PPh₃ (190 mg, 0.72 mmol) were added in a single-necked reaction flask before CBr₄ (238 mg, 0.72 mmol) was slowly added. In a nitrogen atmosphere, the system was stirred at room temperature for 5 h. After the reaction was completed, water was added to the reaction solution to quench the reaction, and the mixture was extracted with DCM. The organic phases were combined, dried over anhydrous Na₂SO₄, and concentrated at reduced pressure. The crude product was purified by normal-phase column chromatography to give product 209d-6 (pale yellow solid, 80 mg). Step 7: Compound 209d-6 (50 mg, 0.16 mmol), compound 209b (46 mg, 0.16 mmol), Pd(PPh₃)₄ (18 mg, 0.02 mmol), CsF (73 mg, 0.48 mmol), and dioxane/H₂O (4.5 mL, V₁:V₂ = 8:1) were added to a single-necked flask. In a nitrogen atmosphere, the system was slowly heated to 85 °C and stirred for 16 h. After the reaction was completed, the reaction solution was filtered. The filtrate was directly concentrated at reduced pressure, and the crude product was isolated by normal-phase column chromatography (MeOH/DCM = 0-5%) to give compound 209d-7 (yellowish brown solid, 60 mg). ¹H NMR (500 MHz, CD3OD) δ 7.69 (dd, *J=* 7.2, 1.1 Hz, 1H), 7.51-7.43 (m, 2H), 7.22 (dd, *J =* 13.8, 7.8 Hz, 1H), 7.18-7.11 (m, 2H), 5.12 (dd, *J =* 13.3, 5.2 Hz, 1H), 4.65-4.57 (m, 4H), 4.38 (q, *J* = 17.0 Hz, 2H), 4.10 (s, 2H), 2.94-2.84 (m, 1H), 2.81-2.73 (m, 1H), 2.48 (qd, *J* = 13.3, 4.7 Hz, 1H), 2.18-2.10 (m, 1H), 1.51 (s, 9H). MS m/z (ESI): 376.6 [M-99]⁺.

Step 8: Compound 209d was prepared with reference to the method of step 2 for intermediate 1c. MS m/z (ESI): 376.6 [M+H]⁺.

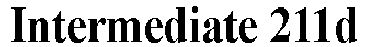

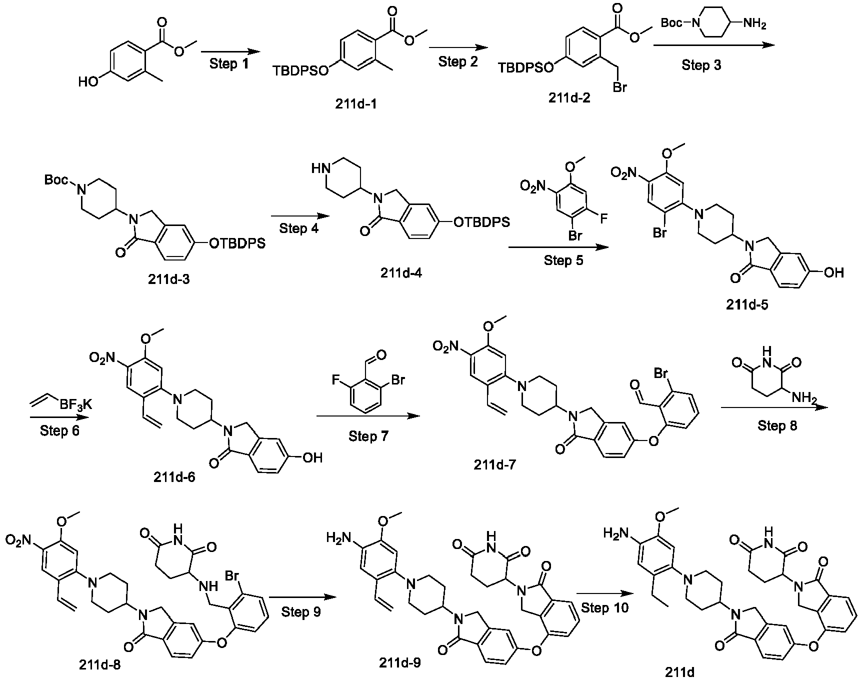

Step 1: In a nitrogen atmosphere, *tert*-butylchlorodiphenylsilane (19.85 g, 72.21 mmol, 18.55 mL) was added to a solution of methyl 4-hydroxy-2-methylbenzoate (8 g, 48.14 mmol) and imidazole (8.19 g, 120.36 mmol) in DMF (80 mL). The reaction system was then stirred at 50 °C for 10 h. After the reaction was completed, the reaction mixture was poured into water and extracted with DCM. The organic layer was washed with saturated brine, dried over anhydrous sodium sulfate, filtered, concentrated, and subjected to column chromatography to give compound 221d-1 (yellow oily liquid, 14 g) (400MHz, DMSO-*d₆*: *δ* 10.38 (s, 1H), 7.55 (d, *J=* 8.9 Hz, 1H), 7.13 (d, *J=* 9.0 Hz, 1H), 3.93 (s, 3H)). MS m/z (ESI): 405.5 [M+H]⁺.

Step 2: In a nitrogen atmosphere, azobisisobutyronitrile (284.12 mg, 1.73 mmol) and NBS (6.77 g, 38.06 mmol) were added to a solution of 211d-1 (14 g, 34.60 mmol) in carbon tetrachloride (200 mL). The mixture was stirred at 70 °C for 3 h. After the reaction was completed, the solvent was removed at reduced pressure, and the residue was subjected to column chromatography (PE/EA = 20:1 to 10: 1) to give compound 211d-2 (colorless oily liquid, 12 g). MS m/z (ESI): 483.2 [M+H]⁺.

Step 3: In a nitrogen atmosphere, DIEA (8.02 g, 62.05 mmol, 10.81 mL) was added to a solution of compound 211d-2 (10 g, 20.68 mmol) and *tert*-butyl 4-aminopiperidine-1-carboxylate (4.56 g, 22.75 mmol) in acetonitrile (100 mL). The reaction system was stirred at 60 °C for 12 h. The solvent was removed at reduced pressure to give compound 211d-3 (yellow oil, 12 g, crude). MS m/z (ESI): 515.1 [M+H-56]⁺.

Step 4: Compound 211d-4 was prepared with reference to step 2 for intermediate 3b. MS m/z (ESI): 471.1 [M+H]⁺.

Step 5: In a nitrogen atmosphere, DIEA (4.12 g, 31.87 mmol, 5.55 mL) was added to a solution of compound 211d-4 (7.5 g, 15.93 mmol) and 1-bromo-2-fluoro-4-methoxy-5-nitrobenzene (4.78 g, 19.12 mmol) in ethanol (50 mL). The reaction system was stirred at 80 °C for 12 h. After the reaction was completed, the solvent was removed at reduced pressure. The residue was dissolved in water and extracted with ethyl acetate. The organic phase was washed with saturated brine and dried over anhydrous sodium sulfate. The solvent was removed by rotary evaporation to give compound 211d-5 (yellow solid, 12.5 g). MS m/z (ESI): 463.3 [M+H]⁺.

Step 6: Compound 221d-6 was prepared with reference to the method for intermediate 5d. MS m/z (ESI): 410.0 [M+H]⁺.

Steps 7-9: Compound 211d-9 was prepared with reference to steps 2-4 for intermediate 120c. MS m/z (ESI): 622.2 [M+H]⁺.

Step 10: Wet palladium on carbon (200 mg, 10 wt%) was added to a solution of compound 3211d-9 in THF (20 mL). The system was reacted for 12 h in an H₂ atmosphere and filtered. The filter cake was rinsed with ethyl acetate and the filtrate was concentrated by rotary evaporation to give the title compound 211d (yellow solid, 56 mg, crude). MS m/z (ESI): 624.3 [M+H]⁺.

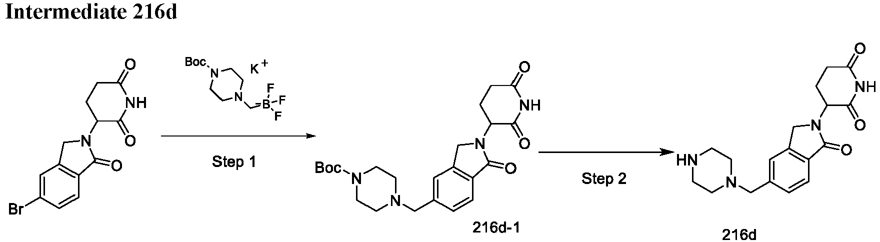

Compound 214c was prepared with reference to the method for intermediate 165a. MS m/z (ESI): 343.2 [M+H]⁺.

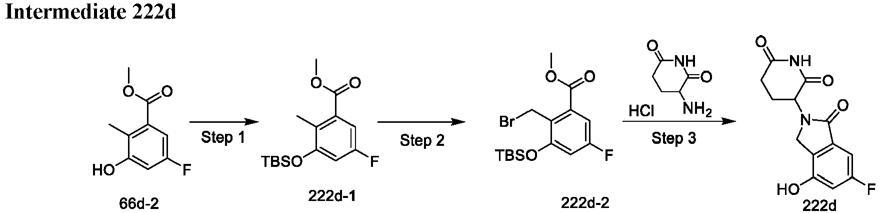

Compound 222d was prepared with reference to step 5 for intermediate 189d as step 1 and with reference to steps 6 and 7 for intermediate 195d as steps 2 and 3. MS m/z (ESI): 279.3 [M+H]⁺.

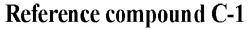

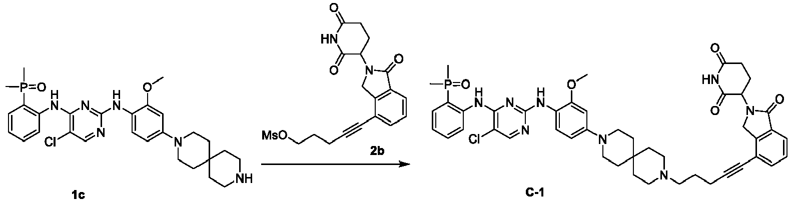

Compound 1c (10 mg, 0.018 mmol) and compound 2b (7.6 mg, 0.018 mmol) were added to a reaction flask before NMP (2 mL), sodium iodide (5.4 mg, 0.036 mmol), and DIPEA (11.6 mg, 0.09 mmol) were added. In a nitrogen atmosphere, the reaction system was heated to 80 °C and stirred for 16 h. After the reaction was completed, the reaction mixture was filtered, and the filtrate was directly separated by preparative reverse-phase column chromatography (acetonitrile/(water + 0.05% HCl)) to give compound **C-1** (white solid, 6.0 mg, hydrochloride). ¹H NMR (500 MHz, CD₃OD) δ 8.29 (s, 1H), 8.15 (s, 1H), 7.81-7.75 (m, 3H), 7.71 (dd, *J* = 16.4, 7.7 Hz, 2H), 7.61 (d, *J* = 2.3 Hz, 1H), 7.54 (q, *J* = 7.4 Hz, 2H), 7.32 (dd, *J* = 8.7, 2.3 Hz, 1H), 5.23 (dd, *J* = 13.3, 5.2 Hz, 1H), 4.58 (dd, *J* = 42.8, 17.5 Hz, 2H), 4.04 (s, 3H), 3.80-3.70 (m, 4H), 3.60 (d, *J* = 12.8 Hz, 2H), 3.42-3.36 (m, 2H), 3.27 (t, *J* = 13.1 Hz, 2H), 3.00-2.92 (m, 1H), 2.86-2.78 (m, 1H), 2.72 (t, *J* = 6.8 Hz, 2H), 2.64-2.54 (m, 1H), 2.41-2.04 (m, 9H), 1.94-1.90 (m, 2H), 1.90 (s, 3H), 1.88 (s, 3H). MS m/z (ESI): 863.6 [M+H]⁺.

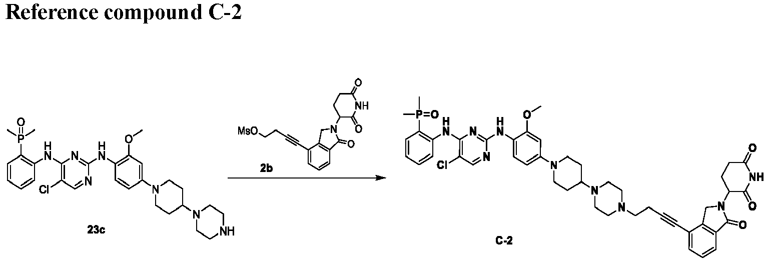

Reference compound **C-2** (pale yellow solid, 4.0 mg, hydrochloride) was prepared with reference to the method for compound C-1.

¹H NMR (500 MHz, CD₃OD) δ 8.20 (s, 2H), 7.80 (d, *J=* 6.9 Hz, 1H), 7.76-7.70 (m, 2H), 7.69-7.58 (m, 2H), 7.51 (dt, *J* = 24.8, 7.5 Hz, 2H), 7.35 (s, 1H), 7.08 (d, *J* = 8.2 Hz, 1H), 5.21 (dd, *J* = 13.3, 5.2 Hz, 1H), 4.60 (dd, *J* = 49.4, 17.6 Hz, 2H), 3.89 (d, *J* = 62.8 Hz, 14H), 3.63 (dd, *J* = 16.8, 9.9 Hz, 4H), 3.17 (dd, *J* = 8.9, 4.8 Hz, 2H), 2.99-2.86 (m, 1H), 2.86-2.75 (m, 1H), 2.65-2.56 (m, 1H), 2.52 (d, *J* = 11.4 Hz, 2H), 2.39 (d, *J* = 10.9 Hz, 2H), 2.24-2.16 (m, 1H), 1.87 (d, *J* = 13.6 Hz, 6H). MS m/z (ESI): 864.4 [M+H]⁺.

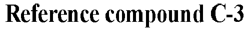

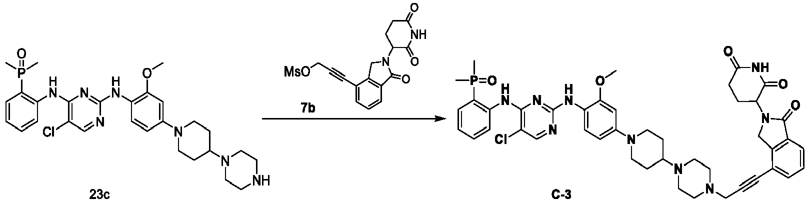

Reference compound **C-3** (white solid, 0.32 g, hydrochloride) was prepared with reference to the method for compound C-1.

¹H NMR (800 MHz, CD₃OD) δ 8.21 (s, 2H), 7.86 (d, *J* = 7.6 Hz, 1H), 7.82 (dd, *J* = 7.6, 0.6 Hz, 1H), 7.77-7.69 (m, 1H), 7.69-7.55 (m, 3H), 7.49 (t, *J* = 7.4 Hz, 1H), 7.37 (s, 1H), 7.10 (d, *J* = 7.6 Hz, 1H), 5.21 (dd, *J* = 13.4, 5.2 Hz, 1H), 4.70 (dd, *J* = 105.7, 17.7 Hz, 2H), 4.47 (s, 2H), 3.95 (d, *J* = 12.4 Hz, 5H), 3.84 (d, *J* = 39.4 Hz, 9H), 3.61 (d, *J* = 49.5 Hz, 2H), 2.93 (ddd, *J* = 17.8, 13.7, 5.4 Hz, 1H), 2.79 (ddd, *J* = 17.6, 4.4, 2.3 Hz, 1H), 2.64 (qd, *J* = 13.3, 4.5 Hz, 1H), 2.54 (d, *J* = 12.1 Hz, 2H), 2.42 (d, *J* = 10.3 Hz, 2H), 2.23-2.16 (m, 1H), 1.87 (d, *J* = 13.5 Hz, 6H). MS m/z (ESI): 850.3 [M+H]⁺.

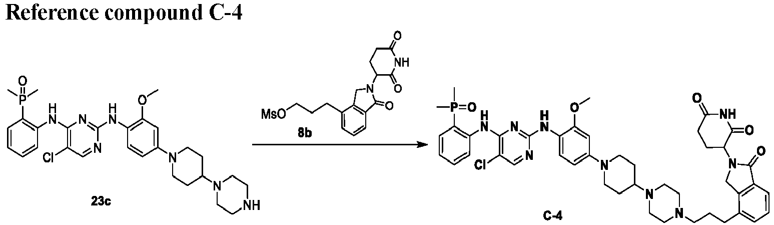

Reference compound **C-4** (white solid, 0.58 g, hydrochloride) was prepared with reference to the method for compound C-1.

¹H NMR (500 MHz, CD₃OD) δ 8.24 (s, 1H), 8.16 (s, 1H), 7.77-7.67 (m, 2H), 7.63 (t, *J* = 7.9 Hz, 1H), 7.60-7.44 (m, 4H), 7.19 (s, 1H), 6.95 (d, *J* = 8.1 Hz, 1H), 5.19 (dd, *J* = 13.3, 5.2 Hz, 1H), 4.58 (dd, *J* = 49.1, 17.1 Hz, 2H), 3.99-3.56 (m, 14H), 3.38 (dd, *J* = 20.4, 12.4 Hz, 4H), 2.98-2.75 (m, 4H), 2.57 (qd, *J* = 13.3, 4.6 Hz, 1H), 2.44 (d, *J* = 11.9 Hz, 2H), 2.31-2.16 (m, 5H), 1.87 (d, *J* = 13.6 Hz, 6H). MS m/z (ESI): 854.3 [M+H]⁺.

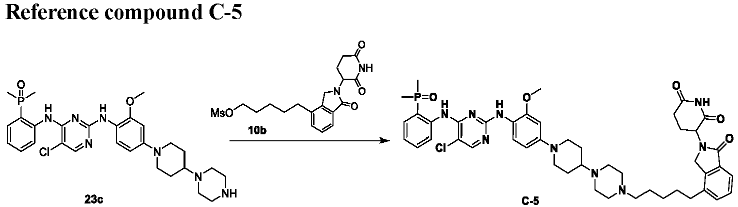

Reference compound **C-5** (white solid, 0.45 g, hydrochloride) was prepared with reference to the method for compound C-1.

¹H NMR (500 MHz, CD₃OD) δ 8.09 (s, 2H), 7.63 (ddd, *J* = 10.8, 7.7, 2.1 Hz, 2H), 7.55 (t, *J* = 7.9 Hz, 1H), 7.49 (d, *J* = 8.0 Hz, 1H), 7.42-7.35 (m, 2H), 7.30 (d, *J* = 8.0 Hz, 1H), 7.22 (s, 1H), 6.96 (d, *J* = 7.6 Hz, 1H), 5.05 (dd, *J* = 13.3, 5.2 Hz, 1H), 4.38 (q, *J* = 17.0 Hz, 2H), 3.85 (s, 7H), 3.59 (dd, *J* = 78.6, 37.9 Hz, 9H), 3.20-3.14 (m, 2H), 2.88-2.66 (m, 4H), 2.46-2.34 (m, 3H), 2.26 (d, *J* = 10.6 Hz, 2H), 2.08 (dtd, *J* = 12.8, 5.3, 2.4 Hz, 1H), 1.79 (s, 3H), 1.77-1.72 (m, 5H), 1.69 (dt, *J* = 15.2, 7.7 Hz, 2H), 1.42-1.33 (m, 2H).MS m/z (ESI): 882.3 [M+H]⁺

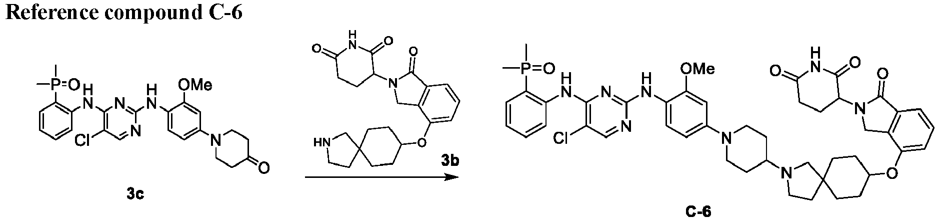

Triethylamine (22.91 mg, 226.43 µmol, 31.52 µL) was added to a solution of compound 3b (90 mg, 226.43 µmol) in 1,2-dichloroethane (3 mL) at 25 °C. The mixture was adjusted to a weakly alkaline pH before glacial acetic acid (13.60 mg, 226.43 µmol, 12.95 µL) was added, and adjusted to a weakly acidic pH before compound 3c (113.20 mg, 226.43 µmol). The reaction system was stirred for 1 h before sodium triacetoxyborohydride (95.98 mg, 452.87 µmol) was added, and was then stirred for another 2 h. After the reaction was completed, the reaction solution was subjected to reverse-phase preparative chromatography (formic acid system) and lyophilized to give compound C-6 (yellow solid, 8.88 mg, formate). ¹H NMR (400 MHz, DMSO-*d₆*) *δ* 11.12-11.24 (m, 1H), 10.81-11.06 (m, 1H), 8.38-8.58 (m, 1H), 7.93-8.18 (m, 2H), 7.42-7.62 (m, 2H), 7.21-7.41 (m, 4H), 7.00-7.17 (m, 1H), 6.57-6.70 (m, 1H), 6.44-6.54 (m, 1H), 5.06-5.18 (m, 1H), 4.47-4.62 (m, 1H), 4.30-4.43 (m, 1H), 4.14-4.28 (m, 1H), 3.76 (s, 3H), 3.62 (d, *J=* 11.76 Hz, 3H), 2.86-2.95 (m, 1H), 2.73-2.81 (m, 2H), 2.58-2.70 (m, 4H), 2.12-2.23 (m, 1H), 1.84-2.05 (m, 6H), 1.76 (d, *J* = 13.63 Hz, 6H), 1.65-1.72 (m, 2H), 1.52-1.63 (m, 6H), 1.38-1.48 (m, 2H). MS m/z (ESI): 881.2 [M+H]⁺.

### Example 1

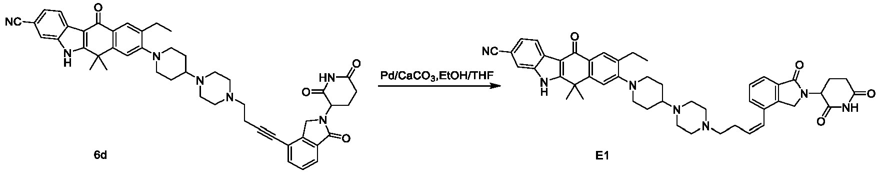

Compound 6d (20 mg, 0.026 mmol) was dissolved in EtOH (3 mL) and THF (3 mL) before Pd/CaCO₃ (20 mg) and quinoline (2 drops) were sequentially added. The reaction system was then stirred at room temperature for 1 h in a hydrogen atmosphere. The reaction mixture was filtered, and separated and purified by prep-HPLC (eluent (v/v):acetonitrile/(water + 0.05% HCl) = 10%-50%) to give title compound **E1** (pale yellow solid, 4.5 mg, hydrochloride). ¹H NMR (500 MHz, CD₃OD) δ 8.43 (d, *J* = 8.2 Hz, 1H), 8.22 (s, 1H), 7.89 (s, 1H), 7.83-7.77 (m, 1H), 7.68-7.60 (m, 2H), 7.58 (dd, *J* = 8.2, 1.2 Hz, 1H), 7.43 (s, 1H), 6.78 (d, *J* = 11.5 Hz, 1H), 5.93 (dt, *J* = 11.6, 7.2 Hz, 1H), 5.22 (dd, *J* = 13.4, 5.2 Hz, 1H), 4.54 (d, *J* = 17.4 Hz, 1H), 4.45 (d, *J* = 17.3 Hz, 1H), 4.06-3.51 (m, 9H), 3.45 (dt, *J* = 22.8, 4.8 Hz, 4H), 2.96 (ddd, *J* = 18.7, 11.9, 4.9 Hz, 3H), 2.90-2.76 (m, 5H), 2.62-2.47 (m, 1H), 2.35 (d, *J* = 11.1 Hz, 2H), 2.26-2.17 (m, 1H), 2.13-2.00 (m, 2H), 1.82 (s, 6H), 1.37 (dd, *J* = 9.5, 5.5 Hz, 3H). MS m/z (ESI): 778.6 [M+H]⁺.

### Example 3

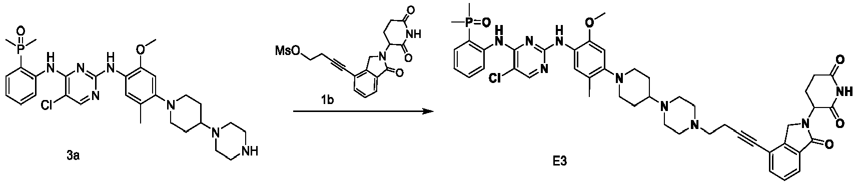

Title compound E3 (pale yellow solid, 13.6 mg, hydrochloride) was prepared with reference to the method for compound C-1.

¹H NMR (500 MHz, CD₃OD) δ 8.18 (s, 1H), 7.82 (d, *J* = 7.5 Hz, 1H), 7.75 (q, *J* = 6.7 Hz, 2H), 7.66-7.58 (m, 1H), 7.56 (dd, *J* = 14.2, 6.5 Hz, 1H), 7.47 (t, *J* = 7.2 Hz, 1H), 7.41 (s, 1H), 7.08 (s, 1H), 5.23 (dt, *J* = 11.8, 5.9 Hz, 1H), 4.62 (dd, *J* = 49.4, 17.6 Hz, 2H), 3.93 (s, 11H), 3.80-3.64 (m, 4H), 3.56 (d, *J* = 10.5 Hz, 2H), 3.26 (s, 1H), 3.20 (t, *J* = 7.0 Hz, 2H), 2.96 (ddd, *J* = 18.6, 13.6, 5.4 Hz, 1H), 2.88-2.77 (m, 1H), 2.62 (qd, *J* = 13.4, 4.7 Hz, 1H), 2.47 (d, *J* = 11.6 Hz, 2H), 2.38-2.19 (m, 7H), 1.92 (s, 3H), 1.89 (s, 3H). MS m/z (ESI): 878.5 [M+H]⁺.

### Example 4

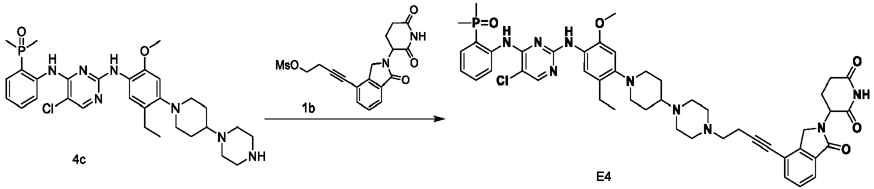

Compound 4a (1.1 g, 1.84 mmol) and compound 1b (1 g, 2.76 mmol) were added to a reaction flask before DMF (60 mL) was added. In a nitrogen atmosphere, sodium bicarbonate (1.5 g, 27.6 mmol) was added and the system was stirred at 65 °C for 24 h. The reaction mixture was concentrated and sequentially purified by normal-phase column chromatography and reverse-phase column chromatography to give title compound **E4** (white solid, 0.31 g, hydrochloride). 1H NMR (500 MHz, CD3OD) δ 8.23 (d, *J* = 53.6 Hz, 1H), 8.16 (s, 1H), 7.81 (t, *J* = 7.7 Hz, 1H), 7.73 (dd, *J* = 10.6, 7.7 Hz, 2H), 7.63-7.53 (m, 2H), 7.44 (dd, *J* = 18.8, 11.3 Hz, 2H), 7.08 (s, 1H), 5.28-5.17 (m, 1H), 4.62 (dd, *J* = 49.1, 17.7 Hz, 2H), 3.92 (s, 11H), 3.70 (dd, *J* = 25.6, 18.8 Hz, 4H), 3.46 (dd, *J* = 3.3, 1.6 Hz, 2H), 3.20 (t, *J* = 6.9 Hz, 3H), 3.02-2.89 (m, 1H), 2.82 (ddd, *J* = 17.6, 4.4, 2.3 Hz, 1H), 2.73-2.55 (m, 3H), 2.44 (d, *J* = 10.8 Hz, 2H), 2.30-2.15 (m, 3H), 1.90 (d, *J* = 13.6 Hz, 6H), 1.14 (t, *J* = 10.8, 3H). MS m/z (ESI): 892.5 [M+H]⁺.

### Examples 5 and 6

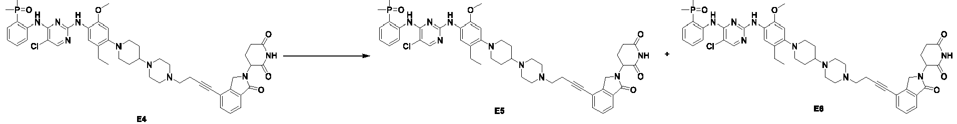

Compound E4 was isolated by chiral normal-phase chromatography (system: SHIMADZU LC-20AP; column: Chiralpak IC-column (250 × 50 mm, 10 µm particle size); mobile phase: A for Hexane (0.1% IPAm) and B for IPA + ACN (0.1% IPAm); gradient: B% = 70% isocratic; flow rate: 100 mL/min; column temperature: room temperature; wavelength: 220 and 254 nm) and prep-HPLC (system: SHIMADZU LC-20AP; column: Luna C18 (250 × 70 mm, 10 µm particle size); mobile phase: A for 0.05% HCl in H₂O and B for acetonitrile; gradient: phase B from 0% to 30% in 30 min; flow rate: 140 mL/min; column temperature: room temperature; wavelength: 220 and 254 nm) to give compound E5 (pale yellow solid, 1.1 g, hydrochloride, retention time: 3.224 min) and compound E6 (pale yellow solid, 1.1 g, hydrochloride, retention time: 5.300 min).

**E5**:1H NMR (400 MHz, DMSO-J6) δ 12.42 (br s, 1H), 12.09 (s, 1H), 11.02 (s, 1H), 8.30 (br s, 2H), 7.74 (dd, *J* = 11.8, 7.5 Hz, 2H), 7.68-7.63 (m, 1H), 7.56 (t, *J* = 7.6 Hz, 1H), 7.35 (s, 2H), 7.27 (t, *J* = 7.5 Hz, 1H), 6.86 (s, 1H), 5.17 (dd, *J* = 13.3, 5.1 Hz, 1H), 4.58-4.35 (m, 2H), 3.79-3.44 (m, 14H), 3.20-3.11 (m, 4H), 3.00-2.74 (m, 3H), 2.66-2.54 (m, 3H), 2.47-2.40 (m, 1H), 2.24 (d, *J* = 11.1 Hz, 2H), 2.08-1.88 (m, 3H), 1.82 (s, 3H), 1.78 (s, 3H), 1.07 (t, *J* = 7.4 Hz, 3H). MS m/z (ESI): 892.3 [M+H]⁺.

**E6**: 1H NMR (400 MHz, DMSO-J6) δ 12.49 (s, 1H), 12.10 (s, 1H), 11.02 (s, 1H), 8.47-8.15 (m, 2H), 7.74 (dd, *J* = 11.3, 7.6 Hz, 2H), 7.69-7.62 (m, 1H), 7.55 (t, *J* = 7.6 Hz, 1H), 7.35 (s, 2H), 7.27 (t, *J* = 7.5 Hz, 1H), 6.86 (s, 1H), 5.17 (dd, *J* = 13.3, 5.1 Hz, 1H), 4.58-4.35 (m, 2H), 3.80-3.44 (m, 14H), 3.20-3.12 (m, 4H), 3.01-2.75 (m, 3H), 2.63-2.53 (m, 3H), 2.48-2.41 (m, 1H), 2.30-2.18 (m, 2H), 2.11-1.90 (m, 3H), 1.82 (s, 3H), 1.78 (s, 3H), 1.07 (t, *J* = 7.5 Hz, 3H). MS m/z (ESI): 892.4 [M+H]⁺.

### Example 7

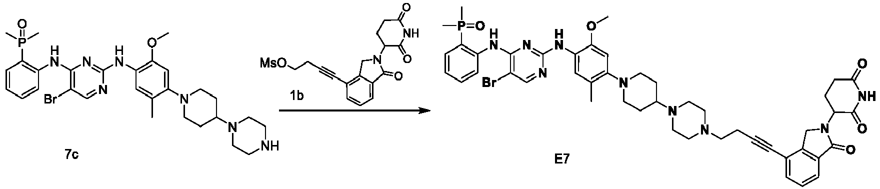

Title compound E7 (pale yellow solid, 9.2 mg, hydrochloride) was prepared with reference to the method for compound E4.

¹H NMR (500 MHz, CD₃OD) δ 8.25-8.09 (m, 2H), 7.71 (d, *J* = 7.6 Hz, 1H), 7.66-7.59 (m, 2H), 7.45 (t, *J* = 7.7 Hz, 2H), 7.34 (t, *J* = 7.2 Hz, 1H), 7.19 (s, 1H), 6.78 (s, 1H), 5.12 (dd, *J* = 13.3, 5.2 Hz, 1H), 4.49 (dd, *J* = 45.0, 17.6 Hz, 2H), 3.80-3.50 (m, 10H), 3.43 (s, 3H), 3.28 (d, *J* = 10.7 Hz, 2H), 3.01 (t, *J* = 6.4 Hz, 2H), 2.84 (ddd, *J* = 18.7, 13.6, 5.4 Hz, 3H), 2.74-2.65 (m, 1H), 2.47 (tt, *J* = 13.4, 6.7 Hz, 1H), 2.24 (d, *J* = 11.5 Hz, 2H), 2.14-2.06 (m, 4H), 1.98 (d, *J* = 10.5 Hz, 2H), 1.80 (s, 3H), 1.77 (s, 3H). MS m/z (ESI): 922.0 [M+H]⁺.

### Example 9

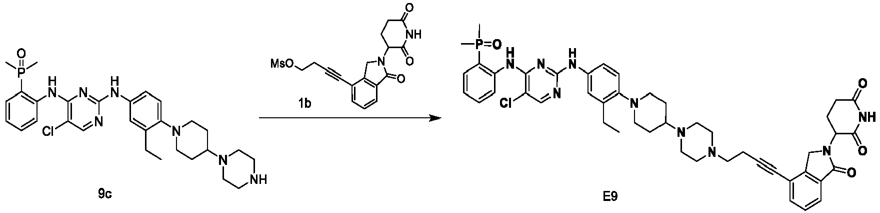

Title compound E9 (white solid, 4.4 mg, hydrochloride) was prepared with reference to the method for compound E4.

¹H NMR (500 MHz, CD₃OD) δ 8.14 (s, 1H), 8.05 (s, 1H), 7.70 (d, *J* = 7.1 Hz, 1H), 7.66-7.57 (m, 2H), 7.44 (t, *J* = 7.7 Hz, 2H), 7.34 (t, *J* = 7.1 Hz, 1H), 7.25-7.15 (m, 3H), 5.11 (dd, *J* = 13.3, 5.1 Hz, 1H), 4.51 (dd, *J* = 49.8, 17.7 Hz, 2H), 3.74 (d, *J* = 67.1 Hz, 8H), 3.56 (t, *J* = 6.8 Hz, 3H), 3.31 (d, *J* = 10.5 Hz, 2H), 3.07 (dd, *J* = 18.2, 11.6 Hz, 4H), 2.88-2.76 (m, 1H), 2.74-2.62 (m, 3H), 2.50 (tt, *J* = 13.4, 6.6 Hz, 1H), 2.31 (d, *J* = 11.2 Hz, 2H), 2.19-2.04 (m, 3H), 1.78 (d, *J* = 13.6 Hz, 6H), 1.13-1.05 (m, 3H).MS m/z (ESI): 862.5 [M+H]⁺.

### Example 10

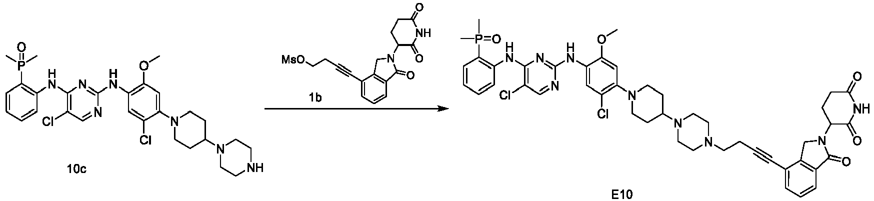

Title compound E10 (pale yellow solid, 6 mg, hydrochloride) was prepared with reference to the method for compound C-1 or E4.

¹H NMR (500 MHz, CD₃OD) δ 8.18 (br s, 2H), 7.78 (d, *J* = 7.5 Hz, 1H), 7.73-7.66 (m, 2H), 7.62-7.55 (m, 2H), 7.52 (t, *J* = 7.7 Hz, 1H), 7.42 (t, *J* = 7.3 Hz, 1H), 6.87 (s, 1H), 5.19 (dd, *J* = 13.3, 5.1 Hz, 1H), 4.66-4.52 (m, 2H), 4.13-3.76 (m, 11H), 3.68-3.53 (m, 5H), 3.18 (t, *J* = 6.8 Hz, 2H), 2.95-2.75 (m, 4H), 2.63-2.55 (m, 1H), 2.36 (d, *J* = 10.9 Hz, 2H), 2.24-2.15 (m, 1H), 2.14-2.03 (m, 2H), 1.89 (s, 3H), 1.86 (s, 3H). MS m/z (ESI): 898.7 [M+H]⁺.

### Example 11

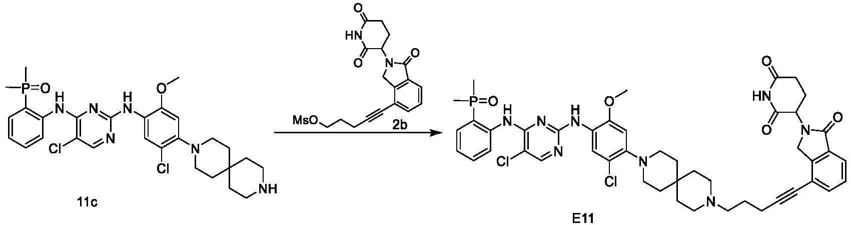

Title compound E11 (pale yellow solid, 7.2 mg, hydrochloride) was prepared with reference to the method for compound C-1.

¹H NMR (500 MHz, DMSO-*d₆*) δ 12.01 (s, 1H), 11.01 (s, 1H), 10.84 (s, 1H), 8.38-8.31 (m, 2H), 7.73 (d, *J* = 7.6 Hz, 1H), 7.71-7.62 (m, 3H), 7.56-7.53 (m, 1H), 7.45 (t, *J* = 7.8 Hz, 1H), 7.27 (t, *J* = 7.4 Hz, 1H), 6.93 (s, 1H), 5.17 (dd, *J* = 13.2, 5.2 Hz, 1H), 4.58 (d, *J* = 17.7 Hz, 1H), 4.37 (d, *J* = 17.7 Hz, 1H), 3.84 (s, 3H), 3.35 (t, *J* = 11.8 Hz, 2H), 3.24-3.15 (m, 2H), 3.13-2.86 (m, 7H), 2.64-2.52 (m, 3H), 2.13-2.05 (m, 2H), 2.04-2.00 (m, 1H), 1.94-1.86 (m, 2H), 1.85-1.74 (m, 11H), 1.56 (br s, 2H). MS m/z (ESI): 897.8 [M+H]⁺.

### Example 12

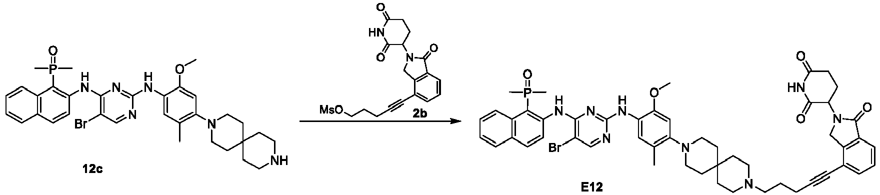

Title compound E12 (off-white solid, 5 mg, hydrochloride) was prepared with reference to the method for compound C-1.

¹H NMR (400 MHz, DMSO-*d₆*) *δ* 12.24 (s, 1H), 11.03 (s, 1H), 8.27-8.11 (m, 2H), 8.09-8.03 (m, 1H), 7.98-7.86 (m, 3H), 7.75-7.63 (m, 2H), 7.59-7.46 (m, 3H), 7.41 (s, 1H), 6.77 (s, 1H), 5.17 (dd, *J* = 5.1, 13.3 Hz, 1H), 4.52-4.42 (m, 1H), 4.36-4.24 (m, 1H), 3.85-3.74 (m, 3H), 2.98-2.89 (m, 1H), 2.76 (s, 4H), 2.64-2.56 (m, 9H), 2.46-2.35 (m, 2H), 2.07-1.92 (m, 9H), 1.84-1.75 (m, 2H), 1.57 (s, 8H). MS m/z (ESI): 971.2, 973.2 [M+H]⁺.

### Example 13

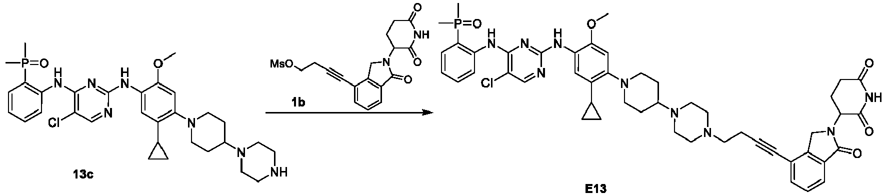

Title compound E13 (off-white solid, 5.32 mg, hydrochloride) was prepared with reference to the method for compound E4.

¹H NMR (400 MHz, DMSO-*d₆*) *δ* 11.25-11.15 (m, 1H), 11.08-10.98 (m, 1H), 8.53-8.36 (m, 1H), 8.21-8.12 (m, 1H), 8.10-8.06 (m, 1H), 7.76-7.70 (m, 1H), 7.68-7.62 (m, 1H), 7.60-7.48 (m, 2H), 7.35-7.20 (m, 1H), 7.15-7.01 (m, 1H), 6.95-6.82 (m, 1H), 6.79-6.58 (m, 1H), 5.32-4.99 (m, 1H), 4.55-4.42 (m, 1H), 4.40-4.25 (m, 1H), 3.78-3.66 (m, 3H), 3.00-2.91 (m, 1H), 2.53 (s, 13H), 2.49-2.38 (m, 4H), 2.36-2.25 (m, 2H), 2.18-2.04 (m, 2H), 1.95-1.86 (m, 2H), 1.82-1.73 (m, 6H), 1.68-1.56 (m, 2H), 0.92-0.80 (m, 2H), 0.56-0.43 (m, 2H); MS m/z (ESI): 904.4 [M+H]⁺

### Example 14

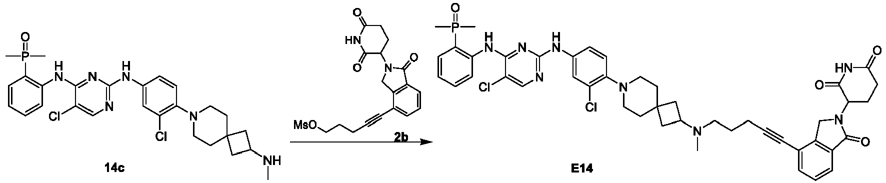

Title compound E14 (white solid, 6 mg, hydrochloride) was prepared with reference to the method for compound C-1.

¹H NMR (400 MHz, DMSO- *d₆*) *δ* 11.17 (s, 1H), 11.00 (s, 1H), 9.43 (s, 1H), 8.66-8.46 (m, 1H), 8.20 (s, 1H), 7.81 (d, *J* = 2.3 Hz, 1H), 7.71 (d, *J* = 7.0 Hz, 1H), 7.64 (d, *J* = 7.6 Hz, 1H), 7.61-7.55 (m, 1H), 7.54 (s, 1H), 7.52 (s, 1H), 7.44 (dd, *J* = 2.3, 8.8 Hz, 1H), 7.21-7.14 (m, 1H), 6.99 (d, *J* = 8.8 Hz, 1H), 5.20-5.10 (m, 1H), 4.49-4.42 (m, 1H), 4.35-4.28 (m, 1H), 2.89 (dd, *J* = 4.4, 12.8 Hz, 2H), 2.85-2.78 (m, 3H), 2.75-2.68 (m, 3H), 2.60 (s, 2H), 2.46-2.41 (m, 2H), 2.06 (s, 3H), 2.03-1.96 (m, 3H), 1.80 (s, 3H), 1.77 (s, 3H), 1.73-1.65 (m, 4H), 1.63-1.54 (m, 4H); MS m/z (ESI): 867.3 [M+H]⁺

### Example 15

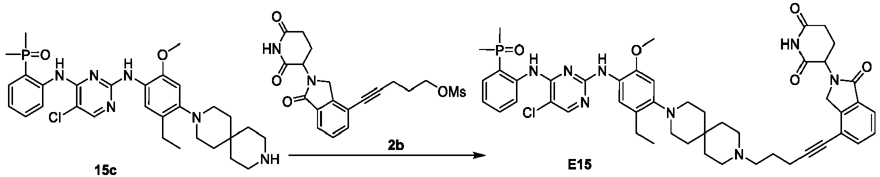

Title compound E15 (white solid, 9.0 mg) was prepared with reference to the method for compound C-1.

¹H NMR (500 MHz, CD₃OD) δ 8.18 (s, 1H), 8.01 (s, 1H), 7.69-7.53 (m, 5H), 7.43-7.39 (m, 3H), 5.10 (dd, *J* = 5.0, 10.0 Hz, 1H), 4.50 (q, *J* = 15.0 Hz, 2H), 3.94 (s, 3H), 3.66-3.62 (m, 3H), 3.47 (d, *J* = 10.0 Hz, 2H), 3.29-3.25 (m, 2H), 3.17-3.15 (m, 2H), 2.86-2.77 (m, 1H), 2.72-2.67 (m, 1H), 2.61-2.57 (m, 4H), 2.52-2.41 (m, 2H), 2.32-2.22 (m, 2H), 2.12-2.02 (m, 4H), 1.88-1.82 (m, 4H), 1.81 (s, 3H), 1.76 (s, 3H), 0.99-0.96 (m, 4H). MS m/z (ESI): 891.5 [M+H]⁺.

### Example 16

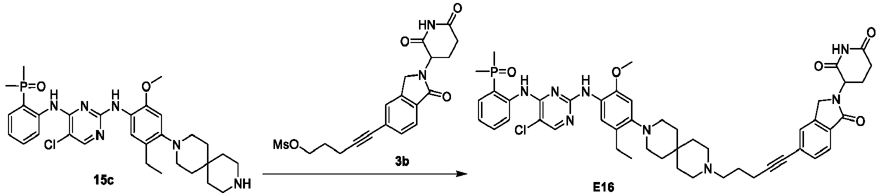

Title compound **E16** (white solid, 5.0 mg) was prepared with reference to the method for compound C-1.

¹H NMR (500 MHz, CD₃OD) δ 8.11 (s, 1H), 8.09 (s, 1H), 7.66-7.62 (m, 2H), 7.55-7.46 (m, 4H), 7.36 (t, *J* = 7.5 Hz, 1H), 7.19 (s, 1H), 5.04 (dd, *J* = 5.0, 10.0 Hz, 1H), 4.38 (q, *J* = 15.0 Hz, 2H), 3.89 (s, 3H), 3.49-3.31 (m, 6H), 3.28-3.25 (m, 2H), 3.18-3.13 (m, 2H), 2.86-2.77 (m, 1H), 2.72-2.66 (m, 1H), 2.58-2.55 (m, 4H), 2.44-2.35 (m, 1H), 2.17-2.10 (m, 7H), 1.91-1.83 (m, 2H), 1.81 (s, 3H), 1.76 (s, 3H), 1.75-1.71 (m, 2H), 0.99-0.96 (m, 3H).MS m/z (ESI): 891.4 [M+H]⁺

### Example 17

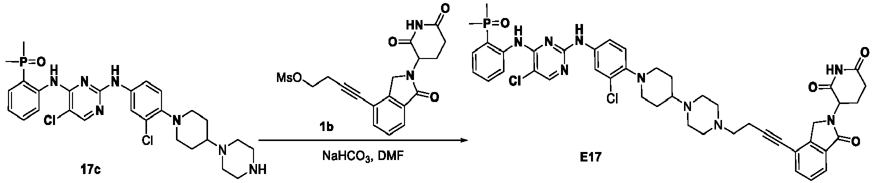

Title compound **E17** (white solid, 4.0 mg, hydrochloride) was prepared with reference to the method for compound E4.

¹H NMR (500 MHz, CD₃OD) δ 8.21 (d, *J* = 17.2 Hz, 2H), 7.82 (d, *J* = 7.6 Hz, 1H), 7.73 (dd, *J* = 18.2, 7.5 Hz, 2H), 7.62-7.54 (m, 3H), 7.44 (t, *J* = 7.4 Hz, 1H), 7.31-7.26 (m, 1H), 7.21 (d, *J* = 8.6 Hz, 1H), 5.23 (dd, *J* = 13.3, 5.0 Hz, 1H), 4.67 (d, *J* = 17.6 Hz, 1H), 4.57 (d, *J* = 17.6 Hz, 1H), 4.11-3.74 (m, 8H), 3.64 (dd, *J* = 17.1, 10.3 Hz, 3H), 3.57 (d, *J* = 11.9 Hz, 2H), 3.19 (t, *J* = 6.8 Hz, 2H), 3.00-2.91 (m, 1H), 2.90-2.80 (m, 3H), 2.68-2.55 (m, 1H), 2.37 (d, *J* = 11.2 Hz, 2H), 2.27-2.20 (m, 1H), 2.15-2.05 (m, 2H), 1.91 (s, 3H), 1.88 (s, 3H).MS m/z (ESI): 868.7 [M+H]⁺

### Example 18

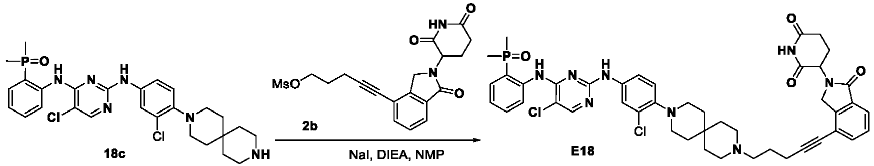

Compound E18 (white solid, 10 mg, hydrochloride) was prepared with reference to the method for compound C-1.

¹H NMR (500 MHz, CD₃OD) δ 8.28 (s, 1H), 8.14 (d, *J* = 4.5 Hz, 1H), 7.83-7.75 (m, 3H), 7.69 (dd, *J* = 15.6, 8.1 Hz, 3H), 7.56 (t, *J* = 7.6 Hz, 1H), 7.51 (t, *J* = 7.1 Hz, 1H), 7.45 (dd, *J* = 8.8, 2.1 Hz, 1H), 5.23 (dd, *J* = 13.3, 5.1 Hz, 1H), 4.58 (dd, *J* = 36.6, 17.5 Hz, 2H), 3.568-3.50 (m, 10H), 3.00-2.91 (m, 1H), 2.82 (d, *J* = 16.1 Hz, 1H), 2.76-2.69 (m, 2H), 2.62-2.52 (m, 1H), 2.26-2.15 (m, 4H), 2.12-2.01 (m, 7H), 1.91 (s, 3H), 1.89 (s, 3H). MS m/z (ESI): 867.6 [M+H]⁺.

### Example 19

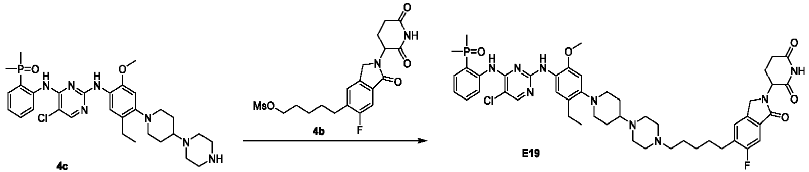

Title compound E19 (36 mg, hydrochloride) was prepared with reference to the method for compound C-1 or E4.

¹H NMR (500 MHz, DMSO-*d₆*) δ 12.02-11.76 (m, 2H), 11.00 (s, 1H), 8.30-8.26 (m, 2H), 7.69-7.54 (m, 2H), 7.48 (d, *J* = 8.8 Hz, 1H), 7.35 (s, 2H), 7.24 (s, 1H), 6.83 (s, 1H), 5.11 (dd, *J* = 13.3, 5.1 Hz, 1H), 4.44-4.28 (m, 2H), 3.85-3.73 (m, 9H), 3.15 (br s, 4H), 2.95-2.87 (m, 1H), 2.84-2.73 (m, 4H), 2.62-2.58 (m, 5H), 2.45-2.36 (m, 1H), 2.22 (br s, 2H), 2.04-1.89 (m, 3H), 1.83-1.74 (m, 9H), 1.68-1.59 (m, 2H), 1.43-1.32 (m, 2H), 1.07 (t, *J* = 7.3 Hz, 3H). MS m/z (ESI): 928.3 [M+H]⁺.

### Example 20

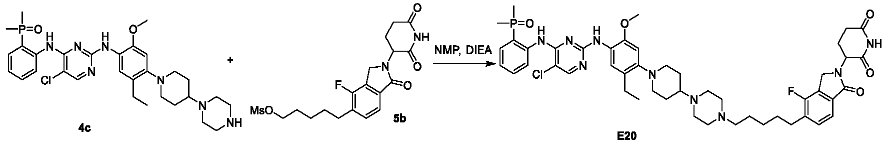

Title compound E20 (30 mg, hydrochloride) was prepared with reference to the method for compound C-1.

¹H NMR (500 MHz, DMSO-*d₆*) δ 11.90 (br s, 2H), 11.00 (s, 1H), 8.41-8.18 (m, 2H), 7.63 (d, *J* = 13.7 Hz, 1H), 7.55-7.46 (m, 2H), 7.36 (s, 2H), 7.24 (s, 1H), 6.83 (s, 1H), 5.11 (dd, *J* = 13.3, 5.1 Hz, 1H), 4.57-4.36 (m, 2H), 3.85-3.76 (m, 9H), 3.19-3.12 (m, 4H), 2.96-2.88 (m, 1H), 2.83-2.73 (m, 4H), 2.63-2.53 (m, 5H), 2.45-2.37 (m, 1H), 2.21 (s, 2H), 2.01 (s, 1H), 1.93 (s, 2H), 1.83-1.73 (m, 9H), 1.69-1.60 (m, 2H), 1.37 (d, *J* = 7.3 Hz, 2H), 1.07 (t, *J* = 7.4 Hz, 3H). MS m/z (ESI): 928.3 [M+H]⁺.

### Example 21

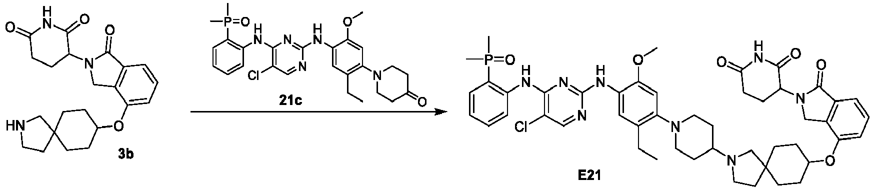

Title compound E21 (13.52 mg, formate) was prepared with reference to the method for compound C-6.

¹H NMR (400 MHz, DMSO-*d₆*) *δ* 11.22-11.06 (m, 1H), 11.0-10.86 (m, 1H), 8.57-8.38 (m, 1H), 8.15-8.03 (m, 2H), 7.60-7.51 (m, 1H), 7.50-7.39 (m, 2H), 7.36-7.22 (m, 3H), 7.15-7.00 (m, 1H), 6.86-6.61 (m, 1H), 5.16-5.05 (m, 1H), 4.64-4.48 (m, 1H), 4.41-4.30 (m, 1H), 4.27-4.16 (m, 1H), 3.81-3.71 (m, 3H), 3.07-2.80 (m, 4H), 2.76-2.65 (m, 4H), 2.63-2.52 (m, 4H), 2.31-2.14 (m, 2H), 2.09-1.85 (m, 4H), 1.82-1.74 (m, 6H), 1.46 (s, 10H), 1.11-1.02 (m, 3H). MS m/z (ESI): 909.3 [M+H]⁺.

### Example 22

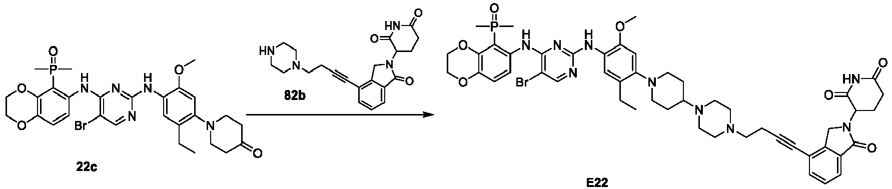

Title compound E22 (2.5 mg, formate) was prepared with reference to the method for compound C-6.

¹H NMR (400 MHz, CDCl₃) *δ* 11.56-11.23 (m, 1H), 8.34-8.26 (m, 1H), 8.12 (s, 1H), 8.08-7.91 (m, 2H), 7.83 (d, *J=* 7.5 Hz, 1H), 7.57 (d, *J=* 7.3 Hz, 1H), 7.49-7.40 (m, 1H), 6.99-6.88 (m, 1H), 6.60 (s, 1H), 5.26 (dd, *J* = 4.9, 13.1 Hz, 1H), 4.61-4.49 (m, 1H), 4.46-4.39 (m, 1H), 4.36-4.27 (m, 4H), 3.83 (s, 3H), 3.48 *(d, J=* 15.9 Hz, 2H), 3.26 (s, 2H), 3.21-3.08 (m, 5H), 3.00-2.88 (m, 3H), 2.87-2.75 (m, 3H), 2.74-2.60 (m, 2H), 2.56 (q, *J* = 7.4 Hz, 2H), 2.35-2.21 (m, 3H), 2.06-2.00 (m, 2H), 1.89 (d, *J* = 13.9 Hz, 6H), 1.39-1.15 (m, 4H), 1.11-0.99 (m, 3H), 0.93-0.80 (m, 1H). MS m/z (ESI): 498.6 [M/2+H]⁺.

### Example 23

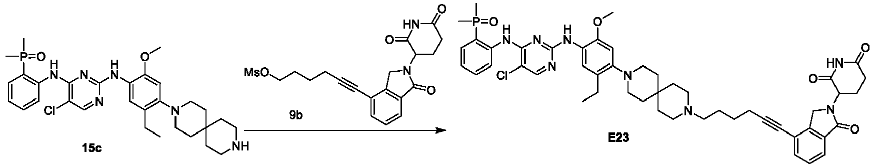

Title compound E23 (8.2 mg, hydrochloride) was prepared with reference to the method for compound C-1. ¹H NMR (500 MHz, CD₃OD) δ 8.44 (s, 1H), 8.30 (s, 1H), 7.95-7.90 (m, 2H), 7.86 (s, 1H), 7.80-7.66 (m, 2H), 7.61 (s, 1H), 5.40 (dd, *J* = 5.0, 10.0 Hz, 1H), 4.70 (q, *J* = 15.0 Hz, 2H), 4.21 (s, 3H), 4.01-3.81 (m, 3H), 3.71 (d, *J* = 10 Hz, 1H), 3.48-3.35 (m, 5H), 3.14-3.03 (m, 1H), 3.01-2.92 (m, 1H), 2.88-2.84 (m, 2H), 2.82-2.79 (m, 3H), 2.56-2.51 (m, 2H), 2.40-2.35 (m, 2H), 2.25-2.18 (m, 3H), 2.17 (s, 3H), 2.15 (s, 3H), 1.91-1.89 (m, 2H), 1.25-1.12 (m, 3H). MS m/z (ESI): 905.5 [M+H]⁺.

### Example 24

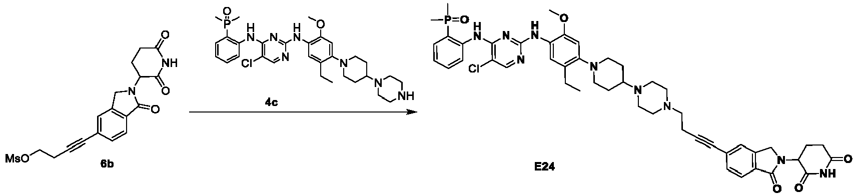

Title compound E24 (white solid, 4 mg, hydrochloride) was prepared with reference to the method for compound E4.

¹H NMR (500 MHz, CD₃OD) δ 8.42 (t, *J* = 39.3 Hz, 2H), 8.03 (d, *J* = 7.9 Hz, 1H), 8.00-7.94 (m, 2H), 7.89 (d, *J* = 7.9 Hz, 1H), 7.82 (s, 1H), 7.67 (dd, *J* = 22.9, 15.6 Hz, 2H), 7.30 (s, 1H), 5.42 (dd, *J* = 13.3, 5.1 Hz, 1H), 4.76 (q, *J* = 17.3 Hz, 2H), 4.17 (d, *J* = 13.9 Hz, 11H), 3.92 (dd, *J* = 17.7, 11.4 Hz, 4H), 3.67 (d, *J* = 9.6 Hz, 2H), 3.42 (t, *J* = 6.9 Hz, 3H), 3.15 (dd, *J* = 13.4, 5.2 Hz, 1H), 3.06 (dd, *J* = 10.0, 7.6 Hz, 1H), 2.90 (d, *J* = 7.4 Hz, 2H), 2.80-2.62 (m, 3H), 2.52-2.42 (m, 3H), 2.14 (d, *J* = 13.5 Hz, 6H), 1.38 (t, *J* = 10.8, 3H). MS m/z (ESI): 892.5 [M+H]⁺.

### Example 25

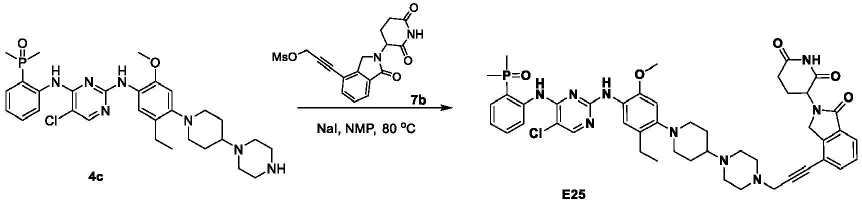

Title compound E25 (6 mg, hydrochloride) was prepared with reference to the method for compound C-1.

¹H NMR (500 MHz, CD₃OD) δ 8.21 (d, *J* = 71.9 Hz, 2H), 7.93-7.82 (m, 2H), 7.75-7.67 (m, 1H), 7.66-7.51 (m, 2H), 7.43 (dd, *J* = 24.8, 17.3 Hz, 2H), 7.03 (s, 1H), 5.23 (dd, *J* = 13.4, 5.2 Hz, 1H), 4.83-4.61 (m, 2H), 4.49 (s, 2H), 3.99-3.72 (m, 10H), 3.64 (s, 1H), 3.38 (t, *J* = 13.8 Hz, 2H), 3.12 (s, 2H), 2.96 (ddd, *J* = 19.6, 12.5, 5.6 Hz, 1H), 2.87-2.76 (m, 1H), 2.71-2.60 (m, 3H), 2.46 (dd, *J* = 39.4, 20.0 Hz, 3H), 2.30-2.13 (m, 3H), 1.90 (d, *J* = 13.6 Hz, 6H), 1.14 (s, 3H). MS m/z (ESI): 878.5 [M+H]⁺.

### Example 26

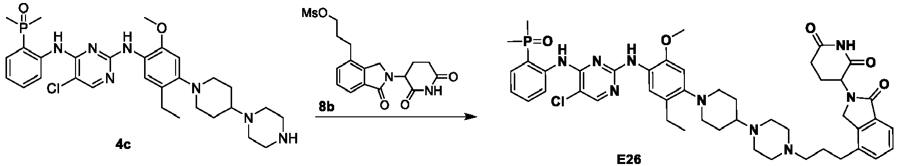

Title compound E26 (white solid, 4 mg, hydrochloride) was prepared with reference to the method for compound C-1.

¹H NMR (500 MHz, CD₃OD) δ 8.12 (dd, *J* = 54.9, 30.2 Hz, 2H), 7.79-7.68 (m, 2H), 7.60 (d, *J* = 7.1 Hz, 2H), 7.54 (t, *J* = 7.5 Hz, 1H), 7.49-7.42 (m, 2H), 7.15 (s, 1H), 5.21 (dd, *J* = 13.3, 5.1 Hz, 1H), 4.59 (dt, *J* = 45.0, 15.5 Hz, 2H), 4.16-3.56 (m, 13H), 3.57-3.35 (m, 5H), 2.90 (qdd, *J* = 17.6, 14.5, 3.7 Hz, 4H), 2.75-2.55 (m, 3H), 2.45 (t, *J* = 16.3 Hz, 2H), 2.40-2.17 (m, 5H), 1.90 (d, *J* = 13.6 Hz, 6H), 1.13 (s, 3H). MS m/z (ESI): 882.5 [M+H]⁺.

### Example 27

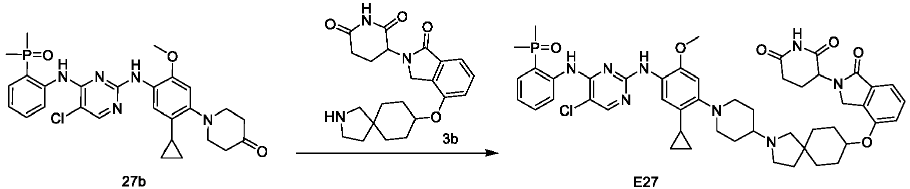

Title compound E27 (yellow solid, 12.93 mg, hydrochloride) was prepared with reference to the method for compound C-6.

¹H NMR (400 MHz, DMSO-*d₆*) δ 11.19(s, 1H), 10.98(s, 1H), 8.43(d, *J* = 4.8 Hz, 2H), 8.27(s, 1H), 8.16 (s, 1H), 8.07 (s, 1H), 7.55-7.45 (m, 2H), 7.31-7.28 (m, 3H), 7.08 (d, *J* = 7.2 Hz, 2H), 6.90(s, 1H), 6.71 (s, 1H), 5.11 (dd, *J* = 17.0, 5.2 Hz, 1H), 4.56 (d, *J* = 4.4 Hz, 1H), 4.38-4.24 (m, 2H), 3.74 (s, 4H), 3.38-3.24 (m, 4H), 2.95-2.85 (m, 1H), 2.75-2.60 (m, 4H), 2.50-2.40 (m, 2H), 2.20-2.10 (m, 2H), 2.00-1.85 (m, 5H), 1.79 (s, 3H), 1.75 (s, 3H), 1.62-1.58 (m, 6H), 1.49-1.42(m, 2H), 0.85(d, *J=* 8.8 Hz, 2H), 0.48 (t, *J* = 11.0Hz, 2H). MS m/z (ESI): 921.4 [M+H]⁺.

### Example 28

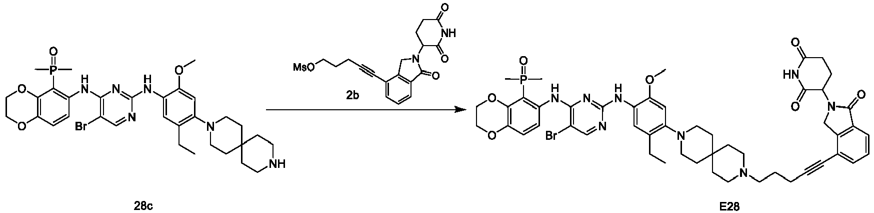

Title compound E28 (white solid, 20 mg, hydrochloride) was prepared with reference to the method for compound C-1.

¹H NMR (400 MHz, DMSO-*d₆*) δ 11.57 (s, 1H), 11.03 (s, 1H), 8.10 (s, 1H), 7.99-7.85 (m, 2H), 7.73 (d, *J* = 7.2 Hz, 1H), 7.68-7.62 (m, 1H), 7.57-7.51 (m, 1H), 7.45 (s, 1H), 6.84 (s, 1H), 6.77 (d, *J* = 8.8 Hz, 1H), 5.17 (dd, *J* = 13.6, 4.8 Hz, 1H), 4.53-4.43 (m, 1H), 4.35-4.21 (m, 5H), 3.77 (s, 3H), 3.01-2.88 (m, 2H), 2.79 (s, 4H), 2.63 (d, *J* = 5.2 Hz, 4H), 2.41 (s, 7H), 2.12-1.97 (m, 2H), 1.84-1.74 (m, 8H), 1.69-1.51 (m, 7H), 1.04 (t, *J* = 7.2 Hz, 3H).MS m/z (ESI): 993.2 [M+H]⁺.

### Example 29

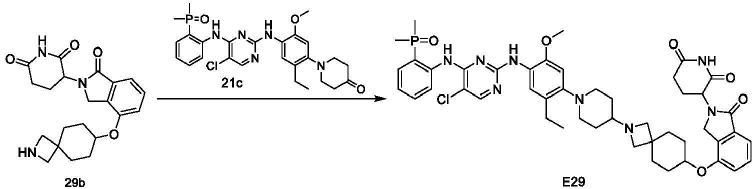

Title compound E29 (white solid, 15 mg, hydrochloride) was prepared with reference to the method for compound C-6.

¹H NMR (500 MHz, CD₃OD) δ 8.42-8.04 (m, 2H), 7.69 (dd, *J* = 13.8, 7.9 Hz, 1H), 7.59-7.45 (m, 2H), 7.42-7.38 (m, 2H), 7.31 (br s, 1H), 7.24 ((d, *J* = 10.0 Hz, 1H), 6.99-6.89 (m, 1H), 5.15 (dd, *J* = 13.4, 4.9 Hz, 1H), 4.62-4.58 (m, 1H), 4.48-4.30 (m, 2H), 4.09-3.98 (m, 4H), 3.86 (s, 3H), 3.46-3.36 (m, 1H), 3.27-3.18 (m, 2H), 2.93-2.87 (m, 3H), 2.82-2.72 (m, 1H), 2.61-2.57 (m, 2H), 2.54-2.45 (m, 1H), 2.23-2.16 (m, 4H), 2.10-2.00 (m, 2H), 1.96-1.65 (m, 13H), 1.11 (t, *J* = 9.0 Hz, 3H). MS m/z (ESI): 896.0 [M+H]⁺.

### Example 30

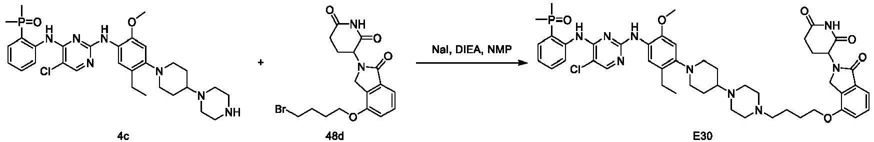

Compound E30 (pale yellow solid, 24 mg, hydrochloride) was prepared with reference to the method for compound C-1.

¹H NMR (500 MHz, CD₃OD) δ 8.33 (s, 1H), 8.10 (s, 1H), 7.72 (dd, *J* = 13.4, 8.3 Hz, 1H), 7.64-7.49 (m, 2H), 7.44-7.41 (m, 2H), 7.33 (s, 1H), 7.26 (d, *J* = 8.1 Hz, 1H), 6.93 (s, 1H), 5.20 (dd, *J* = 13.4, 5.2 Hz, 1H), 4.57-4.45 (m, 2H), 4.26 (t, *J* = 5.7 Hz, 2H), 4.02-3.39 (m, 14H), 3.30-3.26 (m, 2H), 2.99-2. 91 (m, 3H), 2.84-2.79 (m, 1H), 2.67-2.51 (m, 3H), 2.31 (d, *J* = 11.3 Hz, 2H), 2.24-2.19 (m, 1H), 2.12-2.02 (m, 4H), 2.01-1.96 (m, 2H), 1.92 (s, 3H), 1.89 (s, 3H), 1.15 (br s, 3H). MS m/z (ESI): 913.3 [M+H]⁺.

### Example 34

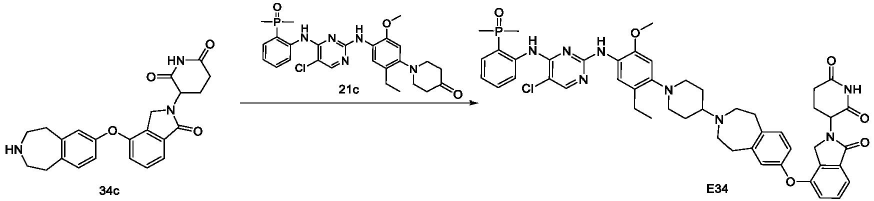

Title compound E34 (white solid, 8 mg, hydrochloride) was prepared with reference to the method for compound C-6.

¹H NMR (400 MHz, CD₃OD) δ ppm 8.25 (dd, *J* = 8. 4. Hz, 1 H), 8.06 (s, 1 H), 7.68 (s, 1 H), 7.63 (dd, *J* = 13.6, 7.6Hz, 1 H) 7.58-7.54 (m, 1 H), 7.50 (td, *J* = 7.6, 4.8 Hz, 2 H), 7.27 (brt, *J* = 8.0 Hz, 1 H) 7.15 (d, *J* = 8.0 Hz, 1 H), 7.10 (d, *J* = 7. 6Hz, 1 H), 6.88 (d, *J* = 2.0 Hz, 1 H), 6.82 (dd, *J* = 8.0, 2.4 Hz, 1 H), 6.77 (s, 1 H), 5.14 (dd, *J* = 13.6, 5.2 Hz, 1 H), 4.48-4.35 (m, 2 H), 3.84 (, s, 3H), 3.07 (br d, *J* = 11.26 Hz, 2 H), 3.00-2.82 (m, 9 H), 2.81-2.69 (m, 4 H), 2.58-2.41 (m, 3 H), 2.21-2.13 (m, 1 H), 1.95-1.75 (m, 10 H), 1.00 (t, *J* = 7.6 Hz, 3 H). MS m/z (ESI): 459.5[M/2+H]⁺.

### Example 35

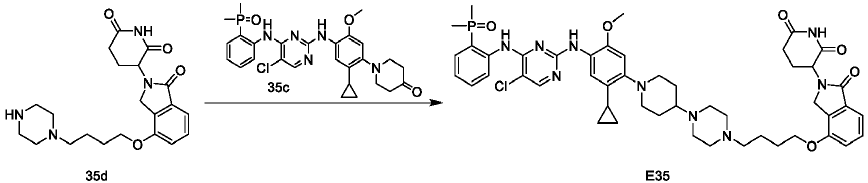

Title compound E35 (pale yellow solid, 45 mg, hydrochloride) was prepared with reference to the method for compound C-6.

¹H NMR (500 MHz, CD₃OD) δ 8.29 (s, 1H), 8.08 (s, 1H), 7.67 (dd, *J* = 14.0, 7.9 Hz, 1H), 7.56-7.47 (m, 2H), 7.41-7.35 (m, 2H), 7.23 (d, *J* = 8.1 Hz, 1H), 7.01 (s, 1H), 6.96 (s, 1H), 5.16 (dd, *J* = 13.3, 5.1 Hz, 1H), 4.56-4.44 (m, 2H), 4.23 (t, *J* = 5.6 Hz, 2H), 4.08-3.65 (m, 14H), 3.43-3.37 (m, 2H), 3.24-3.12 (m, 2H), 2.95-2.88 (m, 1H), 2.82-2.75 (m, 1H), 2.63-2.55 (m, 1H), 2.43 (d, *J* = 10.7 Hz, 2H), 2.32-2.15 (m, 4H), 2.12-2.06 (m, 2H), 1.99-1.93 (m, 2H), 1.90 (s, 3H), 1.87 (s, 3H), 0.96 (d, *J* = 7.6 Hz, 2H), 0.56 (s, 2H). MS m/z (ESI): 925.1 [M+H]⁺.

### Example 36

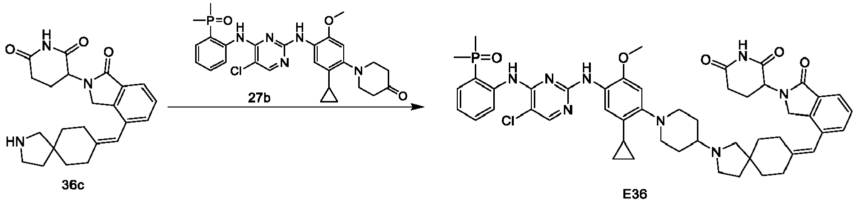

Title compound E36 (white solid, 27.82 mg, formate) was prepared with reference to the method for compound C-6.

¹H NMR (400 MHz, DMSO-*d₆*) *δ* 11.19 (s, 1H), 10.99 (s, 1H), 8.48-8.36 (m, 1H), 8.16 (s, 1H), 8.07 (s, 1H), 7.62-7.40 (m, 4H), 7.33-7.22 (m, 1H), 7.07 (t, *J* = 7.6 Hz, 1H), 6.90 (s, 1H), 6.73-6.66 (m, 1H), 6.26 (s, 1H), 5.18-5.08 (m, 1H), 4.43-4.17 (m, 2H), 3.73 (s, 3H), 3.28-3.20 (m, 3H), 2.97-2.86 (m, 1H), 2.77-2.68 (m, 4H), 2.61 (s, 3H), 2.30 (d, *J* = 3.6 Hz, 2H), 2.16-2.09 (m, 1H), 2.05-1.88 (m, 5H), 1.76 (d, *J* = 13.5 Hz, 6H), 1.69-1.46 (m, 8H), 1.26-1.12 (m, 1H), 0.84 (d, *J* = 8.3 Hz, 2H), 0.48 (d, *J* = 4.0 Hz, 2H). MS m/z (ESI): 917.2 [M+H]⁺.

### Example 37

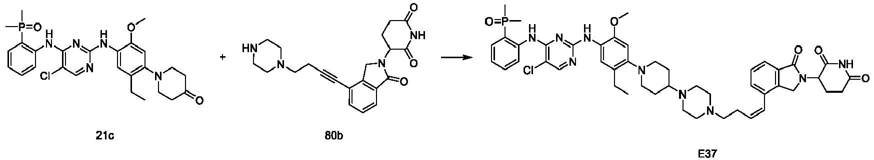

By-product E37 (50 mg, hydrochloride) was prepared with reference to the method for compound C-6.

¹H NMR (500 MHz, CD₃OD) δ 8.23 (br s, 1H), 8.13 (br s, 1H), 7.78-7.68 (m, 2H), 7.66-7.50 (m, 3H), 7.44-7.41 (m, 1H), 7.38 (br s, 1H), 7.06 (s, 1H), 6.74 (d, *J* = 11.4 Hz, 1H), 5.92-5.87 (m, 1H), 5.18 (dd, *J* = 13.4, 5.2 Hz, 1H), 4.53-4.42 (m, 2H), 3.97-3.60 (m, 12H), 3.46-3.38 (m, 4H), 3.24-3.15 (m, 2H), 2.96-2.74 (m, 4H), 2.63 (q, *J* = 7.2 Hz, 2H), 2.58-2.49 (m, 1H), 2.39 (d, *J* = 10.6 Hz, 2H), 2.30-2.15 (m, 3H), 1.89 (s, 3H), 1.86 (s, 3H), 1.10 (br s, 3H). MS m/z (ESI): 895.3 [M+H]⁺.

### Example 38

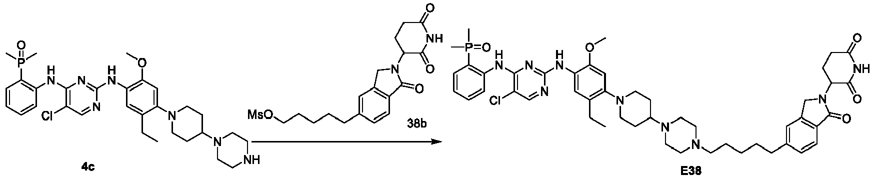

Title compound E38 (white solid, 30 mg, hydrochloride) was prepared with reference to the method for compound C-1.

¹H NMR (500 MHz, CD₃OD) δ 8.12 (s, 2H), 7.81-7.67 (m, 2H), 7.49 (s, 1H), 7.49 (s, 1H), 7.42 (d, *J* = 8.0 Hz, 2H), 7.33 (s, 1H), 6.94 (s, 1H), 5.17 (dd, *J* = 13.3, 5.2 Hz, 1H), 4.50 (q, *J* = 17.0 Hz, 2H), 3.74 (dd, *J* = 143.3, 21.1 Hz, 13H), 3.32-3.23 (m, 3H), 3.11-2.74 (m, 6H), 2.64 (s, 2H), 2.56-2.46 (m, 1H), 2.37 (s, 2H), 2.20 (ddd, *J* = 10.3, 5.2, 2.8 Hz, 3H), 1.95-1.73 (m, 10H), 1.54-1.46 (m, 2H), 1.15 (s, 3H). MS m/z (ESI): 910.5 [M+H]⁺.

### Example 39

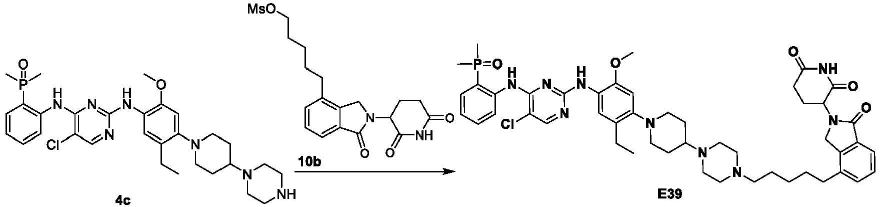

Title compound E39 (white solid, 14 mg, hydrochloride) was prepared with reference to the method for compound C-1.

¹H NMR (500 MHz, CD₃OD) δ 8.18 (s, 1H), 7.74 (dt, *J* = 8.9, 3.5 Hz, 1H), 7.67 (td, *J* = 7.3, 2.3 Hz, 1H), 7.59 (s, 1H), 7.49 (ddd, *J* = 28.7, 14.2, 7.1 Hz, 5H), 7.14 (s, 1H), 5.30-5.13 (m, 1H), 4.56 (d, *J* = 19.6 Hz, 2H), 3.96 (d, *J* = 26.9 Hz, 6H), 3.85-3.74 (m, 4H), 3.50 (t, *J* = 12.9 Hz, 2H), 3.00-2.90 (m, 2H), 2.85-2.77 (m, 4H), 2.72 (s, 5H), 2.70-2.57 (m, 3H), 2.47 (d, *J* = 12.4 Hz, 2H), 2.34 (s, 2H), 2.26-2.18 (m, 1H), 1.92 (s, 3H), 1.89 (s, 3H), 1.80 (dd, *J* = 14.9, 7.2 Hz, 2H), 1.56-1.43 (m, 3H), 1.10 (d, *J* = 26.7 Hz, 3H). MS m/z (ESI): 910.4 [M+H]⁺.

### Example 40

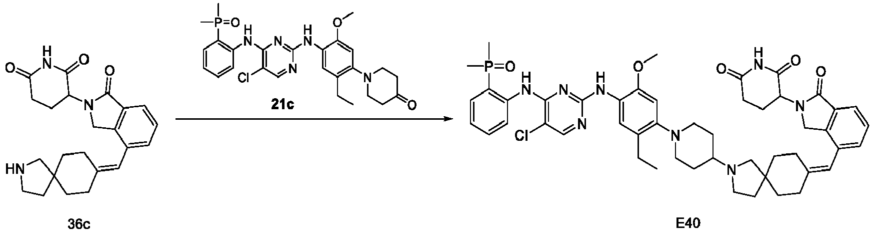

Title compound E40 (50 mg, formate) was prepared with reference to the method for compound C-6.

¹H NMR (400 MHz, DMSO-*d₆*) *δ* 11.15 (s, 1H), 10.99 (s, 1H), 8.42 (d, *J* = 4.8 Hz, 1H), 8.21 (s, 1H), 8.13-8.07 (m, 2H), 7.62-7.40 (m, 5H), 7.31 (t, *J* = 7.8 Hz, 1H), 7.10 (t, *J* = 7.3 Hz, 1H), 6.76 (d, *J* = 3.9 Hz, 1H), 6.25 (s, 1H), 5.19-5.10 (m, 1H), 4.46-4.17 (m, 2H), 3.75 (s, 3H), 3.00-2.87 (m, 3H), 2.74-2.68 (m, 1H), 2.65-2.53 (m, 5H), 2.45-2.36 (m, 4H), 2.29 (s, 2H), 2.18-2.11 (m, 1H), 2.05-1.85 (m, 5H), 1.76 (d, *J=* 13.5 Hz, 7H), 1.65-1.48 (m, 7H), 1.06 (m, 3H). MS m/z (ESI): 453.3 [M/2+H]⁺.

### Example 41

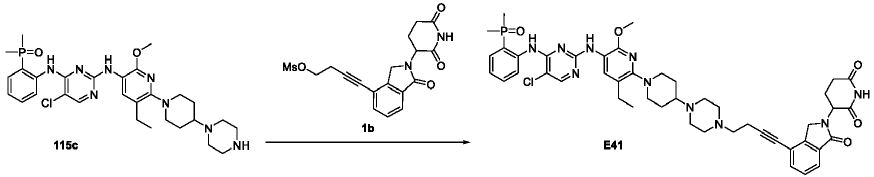

Title compound E41 (pale yellow solid, 4.2 mg, hydrochloride) was prepared with reference to the method for compound E4.

¹H NMR (500 MHz, CD₃OD) δ 8.05 (s, 1H), 8.04 (s, 1H), 7.70 (d, *J* = 7.6 Hz, 1H), 7.64-7.56 (m, 2H), 7.51 (s, 1H), 7.43 (t, *J* = 7.6 Hz, 2H), 7.33 (t, *J* = 7.2 Hz, 1H), 5.10 (dd, *J* = 13.3, 4.9 Hz, 1H), 4.53 (d, *J* = 17.6 Hz, 1H), 4.44 (d, *J* = 17.6 Hz, 1H), 3.94-3.50 (m, 15H), 3.51-3.43 (m, 3H), 3.03 (t, *J* = 6.5 Hz, 2H), 2.92 (t, *J* = 12.5 Hz, 2H), 2.87-2.78 (m, 1H), 2.70 (d, *J* = 16.3 Hz, 1H), 2.53-2.41 (m, 3H), 2.20 (d, *J=* 10.7 Hz, 2H), 2.14-2.08 (m, 1H), 1.97 (dd, *J* = 21.1, 11.3 Hz, 2H), 1.78 (d, *J* = 13.5 Hz, 6H), 1.03 (t, *J* = 7.2 Hz, 3H). MS m/z (ESI): 893.4 [M+H]⁺.

### Example 42

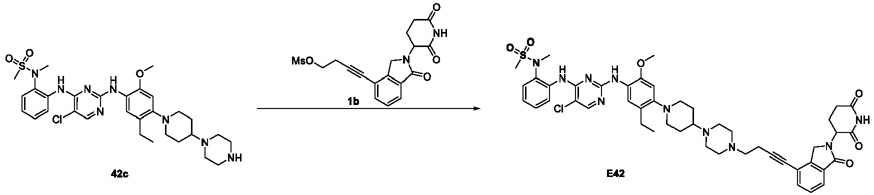

Title compound E42 (pale yellow solid, 11 mg, hydrochloride) was prepared with reference to the method for compound E4.

¹H NMR (500 MHz, CD3OD) δ 8.10 (s, 1H), 7.87 (s, 1H), 7.79 (d, *J* = 7.0 Hz, 1H), 7.72-7.69 (m, 1H), 7.67-7.63 (m, 1H), 7.53 (t, *J* = 7.7 Hz, 1H), 7.48-7.38 (m, 2H), 7.31 (s, 1H), 6.89 (s, 1H), 5.20 (dd, *J* = 13.3, 5.2 Hz, 1H), 4.62 (d, *J* = 17.6 Hz, 1H), 4.53 (d, *J* = 17.6 Hz, 1H), 4.04-3.58 (m, 11H), 3.53 (s, 3H), 3.28-3.20 (m, 2H), 3.11 (t, *J* = 6.9 Hz, 2H), 3.00 (s, 3H), 2.98-2.86 (m, 3H), 2.82-2.76 (m, 1H), 2.62-2.49 (m, 3H), 2.32 (d, *J* = 10.1 Hz, 2H), 2.24-2.17 (m, 1H), 2.10-2.00 (m, 2H), 1.06 (t, *J* = 7.1 Hz, 3H). MS m/z (ESI): 924.1 [M+H]⁺.

### Example 43

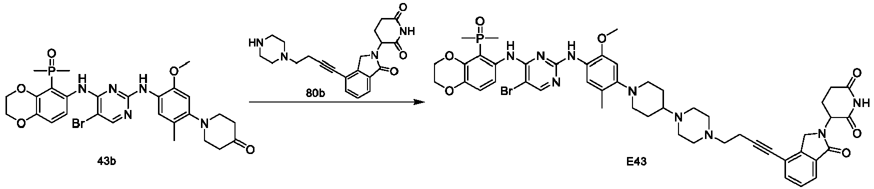

Title compound E43 (yellow solid, 5.04 mg, formate) was prepared with reference to the method for compound C-6.

¹H NMR (400 MHz, DMSO-*d₆*) *δ* 11.61 (s, 1H), 11.07-10.97 (m, 1H), 8.44-8.32 (m, 1H), 8.10 (s, 1H), 7.97-7.88 (m, 2H), 7.79-7.70 (m, 3H), 7.64 (d, *J* = 7.0 Hz, 1H), 7.58-7.48 (m, 1H), 7.44 (s, 1H), 6.81 (d, *J* = 9.3 Hz, 1H), 6.71 (s, 1H), 5.20-5.10 (m, 1H), 4.50-4.40 (m, 1H), 4.36-4.21 (m, 5H), 3.76 (s, 3H), 3.14 (d, *J* = 14.5 Hz, 2H), 3.08 (d, *J* = 10.9 Hz, 2H), 3.00-2.86 (m, 2H), 2.62 (dd, *J* = 5.8, 15.0 Hz, 6H), 2.57-2.54 (m, 3H), 2.42 (d, *J* = 4.3 Hz, 2H), 2.31-2.19 (m, 2H), 2.13 (s, 3H), 2.09-1.96 (m, 2H), 1.91-1.83 (m, 2H), 1.80 (d, *J* = 14.0 Hz, 6H), 1.64-1.50 (m, 2H). MS M/Z (ESI): 980.4 [M+H]⁺

### Example 44

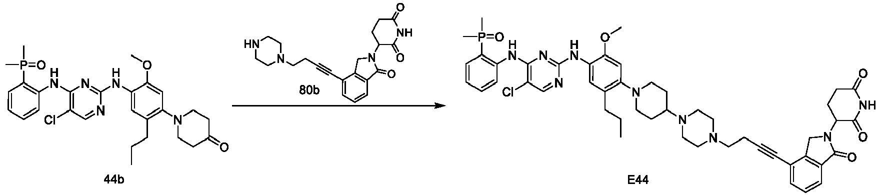

Title compound E44 (yellow solid, 8.18 mg) was prepared with reference to the method for compound C-6.

¹H NMR (400 MHz, DMSO-*d₆*) δ 11.17-11.09 (m, 1H), 11.08-10.99 (m, 1H), 8.48-8.37 (m, 1H), 8.14-8.04 (m, 2H), 7.76-7.69 (m, 1H), 7.67-7.61 (m, 1H), 7.60-7.50 (m, 2H), 7.46-7.42 (m, 1H), 7.37-7.29 (m, 1H), 7.16-7.09 (m, 1H), 6.82-6.77 (m, 1H), 5.21-5.12 (m, 1H), 4.50-4.42 (m, 1H), 4.38-4.26 (m, 1H), 3.80-3.72 (m, 3H), 3.02-2.85 (m, 3H), 2.74-2.57 (m, 10H), 2.53 (m, 3H), 2.46 (m, 4H), 2.37-2.21 (m, 2H), 2.10-2.01 (m, 1H), 1.93-1.84 (m, 2H), 1.80-1.73 (m, 6H), 1.59-1.41 (m, 4H), 0.90-0.81 (m, 3H). MS m/z (ESI): 906.4 [M+H]⁺.

### Example 45

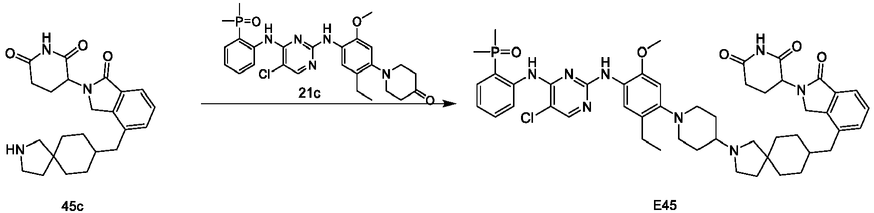

Title compound E45 (white solid, 58.4 mg, formate) was prepared with reference to the method for compound C-6.

¹H NMR (400 MHz, DMSO-*d₆*) *δ* 11.14 (s, 1H), 11.00 (s, 1H), 8.42 (s, 1H), 8.19 (s, 1H), 8.15-8.05 (m, 2H), 7.61-7.37 (m, 5H), 7.31 (s, 1H), 7.15-7.06 (m, 1H), 6.76 (d, *J* = 8.0 Hz, 1H), 5.13 (dd, *J* = 12.8, 4.8 Hz, 1H), 4.51-4.39 (m, 1H), 4.35-4.24 (m, 1H), 3.75 (d, *J* = 4.8 Hz, 4H), 3.05-2.85 (m, 4H), 2.76-2.65 (m, 4H), 2.55 (s, 4H), 2.40 (s, 2H), 2.31-2.16 (m, 1H), 2.05-1.90 (m, 3H), 1.78 (s, 3H), 1.75(s, 3H), 1.67-1.49 (m, 9H), 1.24 (m, 2H), 1.13-1.00 (m, 5H).MS m/z (ESI): 907.5 (M+H)⁺.

### Example 46

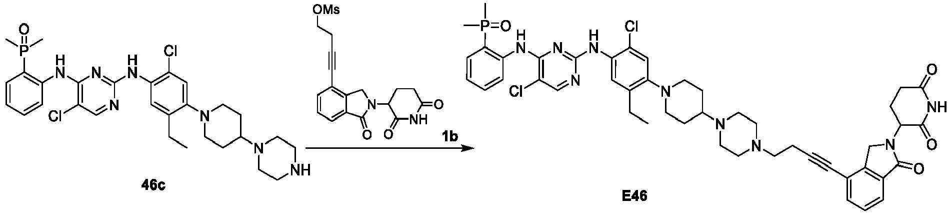

Title compound E46 (white solid, 4 mg, hydrochloride) was prepared with reference to the method for compound E4.

¹H NMR (500 MHz, CD₃OD) δ 8.18 (s, 1H), 8.17 (s, 1H), 7.83 (d, *J* = 7.6 Hz, 1H), 7.75 (d, *J* = 7.0 Hz, 1H), 7.68 (dd, *J* = 14.0, 8.2 Hz, 1H), 7.56 (t, *J* = 7.7 Hz, 1H), 7.48-7.35 (m, 3H), 7.34 (s, 1H), 5.23 (dd, *J* = 13.3, 5.2 Hz, 1H), 4.62 (dd, *J* = 49.2, 17.6 Hz, 2H), 4.30-3.47 (m, 12H), 3.29 (s, 1H), 3.19 (dd, *J* = 9.1, 4.3 Hz, 2H), 3.00-2.79 (m, 4H), 2.72 (q, *J* = 7.5 Hz, 2H), 2.61 (qd, *J* = 13.3, 4.7 Hz, 1H), 2.38 (d, *J* = 10.7 Hz, 2H), 2.28-2.16 (m, 1H), 2.10 (dt, *J* = 20.2, 10.1 Hz, 2H), 1.90 (d, *J* = 13.6 Hz, 6H), 1.20 (t, *J* = 7.4 Hz, 3H); MS m/z (ESI): 896.4 [M+H]⁺.

### Example 47

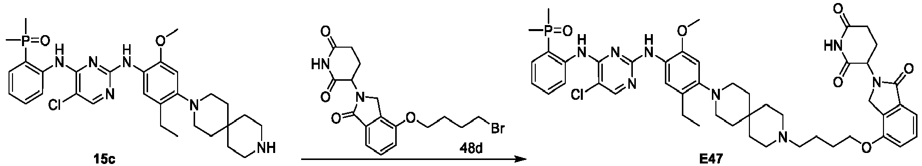

Title compound E47 (5 mg, hydrochloride) was prepared with reference to the method for compound C-1.

¹H NMR (500 MHz, CD₃OD) δ 8.35-8.13(m, 2H), 7.91 (d, *J* = 10.0 Hz, 1H), 7.76-7.56 (m, 5H), 7.40 (d, *J* = 10.0 Hz, 2H), 5.35 (dd, *J* = 10.0, 5.0 Hz, 1H), 4.60 (q, *J* = 15.0 Hz, 2H), 4.58 (t, *J* = 10.0 Hz, 2H), 4.12 (s, 3H), 3.76-3.27 (m, 8H), 3.18-2.65 (m, 5H), 2.69-2.56 (m, 3H), 2.48-2.15 (m, 9H), 2.08-2.03 (m, 4H), 1.93 (s, 3H), 1.90 (s, 3H), 1.27-1.25 (m, 3H). MS m/z (ESI): 897.4 [M+H]⁺

### Example 48

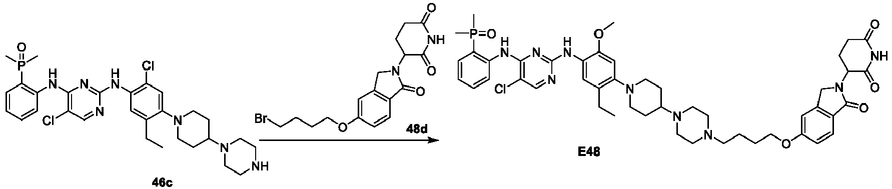

Title compound E48 (white solid, 3.5 mg, hydrochloride) was prepared with reference to the method for compound C-1.

¹H NMR (500 MHz, CD₃OD) δ 8.18 (s, 1H), 8.17 (s, 1H), 7.83 (d, *J* = 7.6 Hz, 1H), 7.75 (d, *J* = 7.0 Hz, 1H), 7.68 (dd, *J* = 14.0, 8.2 Hz, 1H), 7.56 (t, *J* = 7.7 Hz, 1H), 7.48-7.35 (m, 3H), 7.34 (s, 1H), 5.23 (dd, *J* = 13.3, 5.2 Hz, 1H), 4.62 (dd, *J* = 49.2, 17.6 Hz, 2H), 4.30-3.47 (m, 12H), 3.29 (s, 1H), 3.19 (dd, *J* = 9.1, 4.3 Hz, 2H), 3.00-2.79 (m, 4H), 2.72 (q, *J* = 7.5 Hz, 2H), 2.61 (qd, *J* = 13.3, 4.7 Hz, 1H), 2.38 (d, *J* = 10.7 Hz, 2H), 2.28-2.16 (m, 1H), 2.10 (dt, *J* = 20.2, 10.1 Hz, 2H), 1.90 (d, *J* = 13.6 Hz, 6H), 1.20 (t, *J* = 7.4 Hz, 3H); MS m/z (ESI): 912.6 [M+H]⁺.

### Example 49

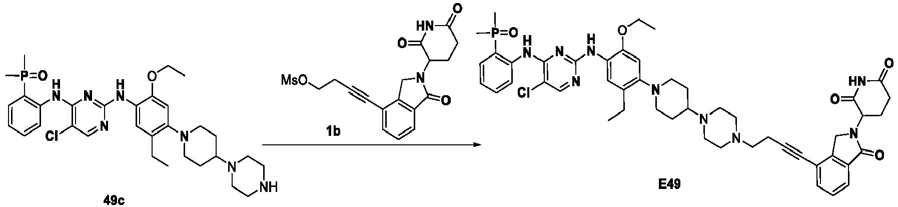

Title compound E49 (white solid, 2 mg, hydrochloride) was prepared with reference to the method for compound E4.

¹H NMR (500 MHz, CD₃OD) δ 8.24-8.20 (m, 2H), 7.78 (d, *J* = 5.0 Hz, 1H), 7.75-7.68 (m, 2H), 7.61 (t, *J* = 7.5 Hz, 1H), 7.55-7.52 (m, 2H), 7.45 (t, *J* = 7.5 Hz, 1H), 7.35 (s, 1H), 5.23 (dd, *J* =10.0, 5.0 Hz, 1H), 4.57 (q, *J* = 15.0 Hz, 2H), 4.03 (s, 3H), 3.90-3.85 (m, 4H), 3.61-3.59 (m, 2H), 3.41-3.21 (m, 5H), 2.98-2.61 (m, 5H), 2.38-2.17 (m, 11H), 1.93 (s, 3H), 1.90 (s, 3H), 1.05 (d, *J* = 5.0 Hz, 2H), 0.96 (s, 2H). MS m/z (ESI): 906.5 [M+H]⁺.

### Example 50

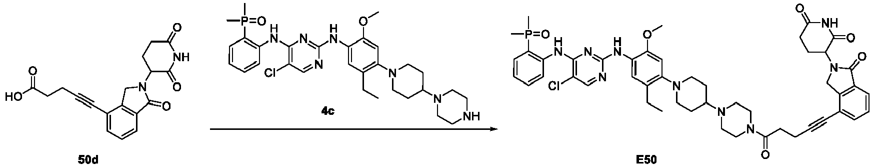

Title compound E50 (white solid, 6.7 mg, hydrochloride) was prepared with reference to the synthesis method for compound E88.

¹H NMR (500 MHz, CD₃OD) δ 8.30-7.94 (m, 2H), 7.66 (d, *J* = 7.5 Hz, 1H), 7.61 (dd, *J* = 14.1, 7.6 Hz, 1H), 7.54 (d, *J* = 7.5 Hz, 1H), 7.50-7.40 (m, 2H), 7.33 (t, *J* = 7.1 Hz, 1H), 7.26 (s, 1H), 6.92 (s, 1H), 5.13-5.07 (m, 1H), 4.41 (dd, *J* = 38.1, 17.7 Hz, 2H), 3.79 (s, 3H), 3.67-3.51 (m, 3H), 3.43-3.23 (m, 4H), 3.16-2.92 (m, 5H), 2.90-2.59 (m, 7H), 2.56-2.41 (m, 3H), 2.23-1.97 (m, 5H), 1.78 (d, *J* = 13.6 Hz, 6H), 1.01 (s, *J* = 7.0 Hz, 3H). MS m/z (ESI): 921.2 [M+H]⁺.

### Example 51

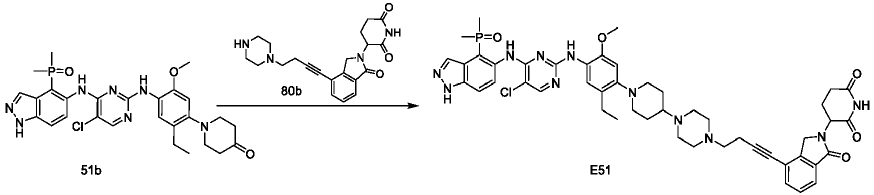

Title compound E51 (white solid, 4.22 mg, formate) was prepared with reference to the method for compound C-6.

¹H NMR (400 MHz, DMSO-*d₆*) *δ* 13.34 (s, 1H), 11.02 (d, *J* = 3.6 Hz, 2H), 8.33 (s, 1H), 8.16-8.05 (m, 2H), 7.84 (s, 1H), 7.71 (d, *J* = 7.6 Hz, 1H), 7.66-7.49 (m, 3H), 7.44 (s, 1H), 6.74 (s, 1H), 5.17 (dd, *J* = 13.2, 4.8 Hz, 1H), 4.51-4.41 (m, 1H), 4.36-4.26 (m, 1H), 3.76 (s, 3H), 3.00-2.87 (m, 4H), 2.62 (m, 10H), 2.47-2.36 (m, 4H), 2.31-2.22 (m, 3H), 2.12-1.98 (m, 2H), 1.86 (d, *J* = 13.6 Hz, 8H), 1.59-1.46 (m, 2H), 0.81 (t, *J* = 7.3 Hz, 3H). MS m/z (ESI): 932.4 [M+H]⁺.

### Example 52

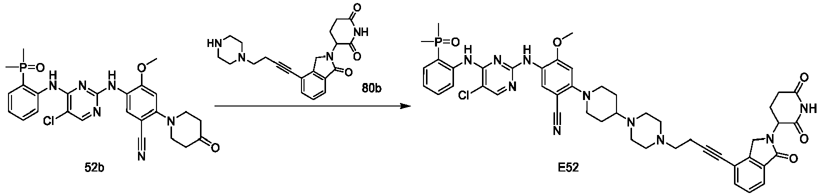

Title compound E52 (yellow solid, 46.7 mg, formate) was prepared with reference to the method for compound C-6.

¹H NMR (400 MHz, CD₃OD) δ 8.23 (m, 1H), 8.18 (s, 1H), 8.12 (s, 1H), 7.76 (d, *J* = 7.6 Hz, 1H), 7.72-7.61 (m, 3H), 7.55-7.47 (m, 1H), 7.31 (t, *J* = 7.6 Hz, 1H), 6.74 (d, *J* = 3.3 Hz, 1H), 5.19 (dd, *J* = 5.1, 13.4 Hz, 1H), 4.51 (d, *J* = 9.5 Hz, 6H), 3.97 (s, 3H), 3.58 (d, *J* = 10.1 Hz, 2H), 2.91-2.71 (m, 12H), 2.60-2.48 (m, 2H), 2.25-2.15 (m, 1H), 2.09 (d, *J* = 10.6 Hz, 2H), 1.90-1.81 (m, 6H), 1.78 (dd, *J* = 3.5, 11.9 Hz, 2H). MS m/z (ESI): 889.7 [M+H]⁺.

### Example 53

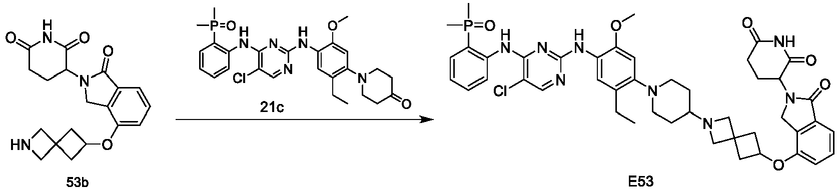

Title compound E53 (pale yellow solid, 90 mg, hydrochloride) was prepared with reference to the method of Example 21.

¹H NMR (500 MHz, CD₃OD) δ 8.48-7.90 (m, 2H), 7.70 (dd, *J* = 13.5, 6.6 Hz, 1H), 7.57 (s, 1H), 7.48-7.32 (m, 3H), 7.28 (d, *J* = 7.4 Hz, 1H), 7.21-6.95 (m, 2H), 5.16-5.05 (m, 2H), 4.46-4.18 (m, 6H), 3.90 (d, *J* = 17.6 Hz, 3H), 3.58-3.37 (m, 3H), 3.17-3.01 (m, 1H), 2.98-2.78 (m, 5H), 2.69-2.57 (m, 3H), 2.53-2.43 (m, 1H), 2.29-2.14 (m, 3H), 2.07-1.64 (m, 9H), 1.08 (br s, 3H). MS m/z (ESI): 868.0 [M+H]⁺.

### Example 54

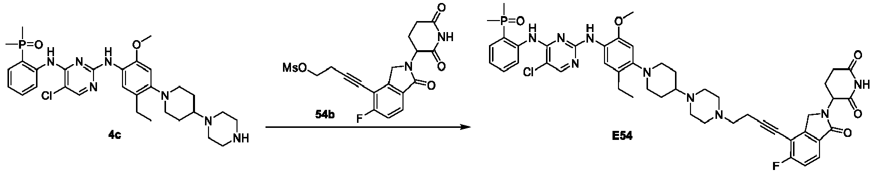

Title compound E54 (pale yellow solid, 5.2 mg, hydrochloride) was prepared with reference to the method for compound E4.

¹H NMR (500 MHz, CD₃OD) δ 8.17 (s, 2H), 7.83 (dd, *J* = 8.4, 4.5 Hz, 1H), 7.74 (dd, *J* = 13.6, 7.2 Hz, 1H), 7.59 (s, 1H), 7.47-7.34 (m, 3H), 7.09 (s, 1H), 5.21 (d, *J* = 8.9 Hz, 1H), 4.70 (d, *J* = 17.8 Hz, 1H), 4.59 (d, *J* = 18.0 Hz, 1H), 4.05-3.87 (m, 11H), 3.79-3.65 (m, 4H), 3.48-3.42 (m, 2H), 3.25 (t, *J* = 5.5 Hz, 3H), 2.98-2.91 (m, 1H), 2.82 (d, *J* = 17.2 Hz, 1H), 2.71-2.59 (m, 3H), 2.50-2.40 (m, 2H), 2.34-2.19 (m, 3H), 1.92 (s, 3H), 1.89 (s, 3H), 1.14 (t, *J* = 7.0 Hz, 3H). MS m/z (ESI): 910.9 [M+H]⁺.

### Example 55

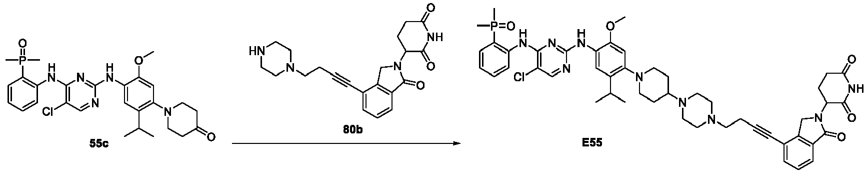

Title compound E55 (pale yellow solid, 10.2 mg, hydrochloride) was prepared with reference to the method for compound C-6.

¹H NMR (500 MHz, CD₃OD) δ 8.33 (s, 1H), 8.10 (s, 1H), 7.82 (d, *J* = 7.6 Hz, 1H), 7.75-7.66 (m, 2H), 7.56 (t, *J* = 7.6 Hz, 2H), 7.44-7.36 (m, 1H), 7.35-7.27 (m, 1H), 7.10-7.02 (m, 1H), 5.23 (dd, *J* = 13.1, 4.7 Hz, 1H), 4.67 (d, *J* = 17.6 Hz, 1H), 4.57 (d, *J* = 17.6 Hz, 1H), 4.10-3.84 (m, 10H), 3.75-3.63 (m, 3H), 3.48-3.34 (m, 3H), 3.24-3.08 (m, 4H), 3.00-2.90 (m, 1H), 2.83 (d, *J* = 16.8 Hz, 1H), 2.70-2.56 (m, 1H), 2.48-2.38 (m, 2H), 2.30-2.14 (m, 3H), 1.91 (d, *J* = 13.5 Hz, 6H), 1.17 (d, *J* = 5.7 Hz, 6H). MS m/z (ESI): 906.9 [M+H]⁺.

### Example 57

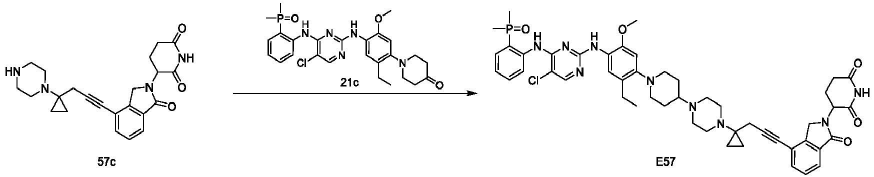

Title compound E57 (2 mg, formate) was prepared with reference to the method for compound C-6.

¹H NMR (400 MHz, CD₃OD) δ ppm 8.26-8.16 (m, 1 H), 8.10-7.99 (m, 1 H), 7.82-7.72 (m, 1 H), 7.68-7.59 (m, 3 H), 7.55-7.49 (m, 2 H), 7.31-7.22 (m, 1 H), 6.82-6.69 (m, 1 H), 5.19 (m, 1 H), 4.46 (m, 2 H), 4.28-4.28 (m, 1 H), 3.84 (s, 2 H), 3.09-3.07 (m, 2 H), 3.02-2.98 (m, 3 H), 2.84-2.82 (m, 10 H), 2.50 (br d, *J* = 7.88 Hz, 3 H), 2.16-2.27 (m, 1 H), 2.12-2.00 (m, 3 H), 1.84 (d, *J* = 13.6 Hz, 6 H), 1.79-1.66 (m, 2 H), 1.35-1.25 (m, 2 H), 0.99 (t, *J* = 7.2 Hz, 3 H), 0.85-0.68 (m, 4 H).MS m/z (ESI): 918.5 [M+H]⁺.

### Example 58

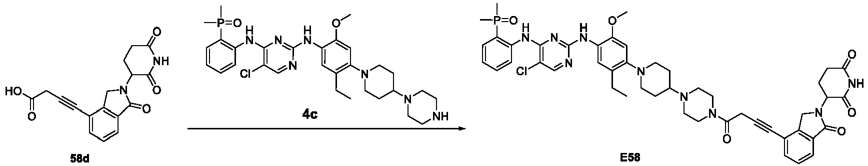

Title compound E58 (white solid, 12 mg, hydrochloride) was prepared with reference to the method for compound E88.

¹H NMR (500 MHz, CD₃OD) δ 8.40-8.06 (m, 1H), 7.81 (t, *J* = 11.5 Hz, 1H), 7.72 (dd, *J* = 14.5, 7.6 Hz, 2H), 7.63 (t, *J* = 8.0 Hz, 1H), 7.60-7.50 (m, 2H), 7.43 (t, *J* = 7.2 Hz, 1H), 7.33 (s, 1H), 6.95 (s, 1H), 5.21 (dd, *J* = 13.3, 5.3 Hz, 1H), 4.81-4.72 (m, 1H), 4.68-4.52 (m, 1H), 3.92-3.61 (m, 8H), 3.56-3.46 (m, 1H), 3.34-3.20 (m, 7H), 3.02-2.91 (m, 3H), 2.87-2.78 (m, 1H), 2.67-2.53 (m, 3H), 2.39-2.31 (m, 2H), 2.27-2.20 (m, 1H), 2.11-2.01 (m, 2H), 1.92 (s, 3H), 1.89 (s, 3H), 1.14 (t, *J* = 7.0 Hz, 3H). MS m/z (ESI): 906.6 [M+H]⁺.

### Example 59

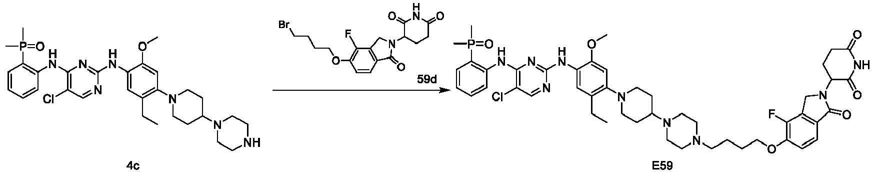

Compound E59 (pale yellow solid, 2.1 mg, hydrochloride) was prepared with reference to the method for compound C-1.

¹H NMR (500 MHz, CD₃OD) δ 8.26 (br s, 1H), 8.10 (br s, 1H), 7.69 (dd, *J* = 14.1, 7.9 Hz, 1H), 7.59 (d, *J* = 8.3 Hz, 1H), 7.57-7.48 (m, 1H), 7.41 (t, *J* = 7.7 Hz, 1H), 7.32 (t, *J* = 7.8 Hz, 2H), 6.96 (s, 1H), 5.11 (dd, *J* = 13.4, 5.1 Hz, 1H), 4.59-4.47 (m, 2H), 4.26 (t, *J* = 5.8 Hz, 2H), 3.97-3.60 (m, 12H), 3.47-3.41 (m, 2H), 3.10-2.97 (m, 2H), 2.95-2.83 (m, 1H), 2.80-2.75 (m, 1H), 2.62 (d, *J* = 7.2 Hz, 2H), 2.54-2.45 (m, 1H), 2.37 (d, *J* = 10.2 Hz, 2H), 2.20-2.06 (m, 5H), 2.02 (s, 2H), 1.92-1.84 (m, 8H), 1.11 (br s, 3H). MS m/z (ESI): 931.1 [M+H]⁺.

### Example 60

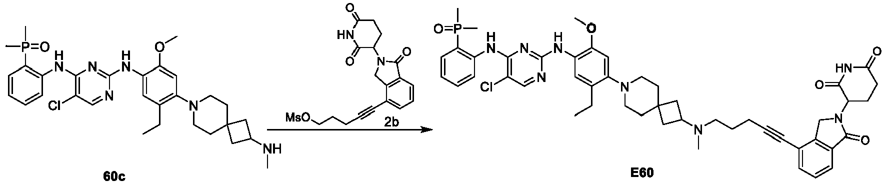

Title compound E60 (white solid, 5 mg, hydrochloride) was prepared with reference to the method for compound C-1.

¹H NMR (500 MHz, CD₃OD) δ 8.73-8.64 (m, 1H), 8.60-8.45 (m, 1H), 8.23-8.02 (m, 5H), 7.98-7.91 (m, 2H), 7.80-7.70 (m, 1H), 5.23 (dd, *J* = 10.0, 5.0 Hz, 1H), 5.03-4.95 (m, 2H), 4.44-4.36 (m, 4H), 4.16-3.78 (m, 5H), 3.75-3.48 (m, 6H), 3.39-2.91(m, 5H), 2.83-2.46 (m, 10H), 2.34 (s, 3H), 2.32 (s, 3H), 1.49-1.30 (m, 3H). MS m/z (ESI): 891.5 [M+H]⁺.

### Example 61

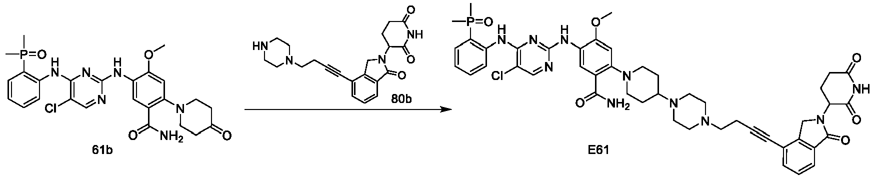

Title compound E61 (yellow solid, 7.28 mg) was prepared with reference to the method for compound C-6.

¹H NMR (400 MHz, CD₃OD) δ 8.43-8.33 (m, 2H), 8.08 (s, 1H), 7.77 (d, *J* = 7.6 Hz, 1H), 7.64 (d, *J* = 7.3 Hz, 1H), 7.60-7.46 (m, 3H), 7.24-7.15 (m, 1H), 6.96-6.86 (m, 1H), 5.21 (dd, *J* = 5.2, 13.2 Hz, 1H), 4.66-4.55 (m, 2H), 4.53-4.47 (m, 2H), 3.93 (s, 3H), 3.17-3.01 (m, 3H), 2.99-2.72 (m, 12H), 2.68 (t, *J* = 5.8 Hz, 1H), 2.59-2.41 (m, 1H), 2.26-2.16 (m, 3H), 1.84 (d, *J* = 13.4 Hz, 9H). MS m/z (ESI): 907.5 [M+H]⁺.

### Example 62

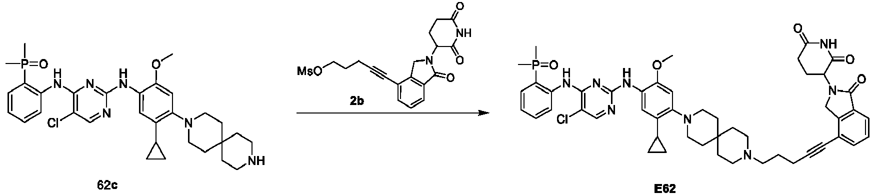

Title compound E62 (white solid, 6 mg, hydrochloride) was prepared with reference to the method for compound C-1.

¹H NMR (500 MHz, CD₃OD) δ 8.24-8.20 (m, 2H), 7.78 (d, *J* = 5.0 Hz, 1H), 7.75-7.68 (m, 2H), 7.61 (t, *J* = 7.5 Hz, 1H), 7.55-7.52 (m, 2H), 7.45 (t, *J* = 7.5 Hz, 1H), 7.35 (s, 1H), 5.23 (dd, *J* = 10.0, 5.0 Hz, 1H), 4.57 (q, *J* = 15.0 Hz, 2H), 4.03 (s, 3H), 3.90-3.85 (m, 4H), 3.61-3.59 (m, 2H), 3.41-3.21 (m, 5H), 2.98-2.61 (m, 5H), 2.38-2.17 (m, 11H), 1.93 (s, 3H), 1.90 (s, 3H), 1.05 (d, *J* = 5.0 Hz, 2H), 0.96 (s, 2H). MS m/z (ESI): 903.3 [M+H]⁺.

### Example 63

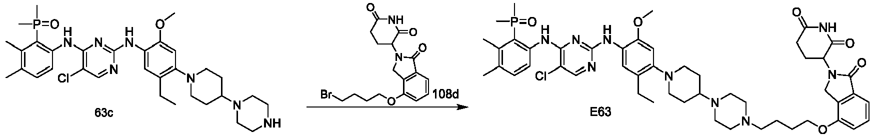

Title compound E63 (yellow solid, 34.46 mg) was prepared with reference to the method for compound C-1. ¹H NMR (400 MHz, CD3OD) δ = 8.17-7.58 (m, 2H), 7.53-7.46 (m, 1H), 7.43-7.26 (m, 3H), 7.24 (d, *J* = 8.0 Hz, 1H), 6.89 (s, 1H), 5.20-5.13 (m, 1H), 4.59-4.41 (m, 2H), 4.29-4.18 (m, 2H), 4.00-3.80 (m, 6H), 3.76-3.59 (m, 4H), 3.54-3.45 (m, 1H), 3.40-3.33 (m, 2H), 3.28-3.21 (m, 2H), 3.02-2.74 (m, 5H), 2.66-2.49 (m, 3H), 2.47-2.40 (m, 3H), 2.38-2.26 (m, 5H), 2.25-2.15 (m, 1H), 2.12-2.02 (m, 4H), 2.00 (s, 3H), 1.98-1.92 (m, 5H), 1.15-0.98 (m, 3H)MS m/z (ESI): 940.5[M+H]⁺.

### Example 64

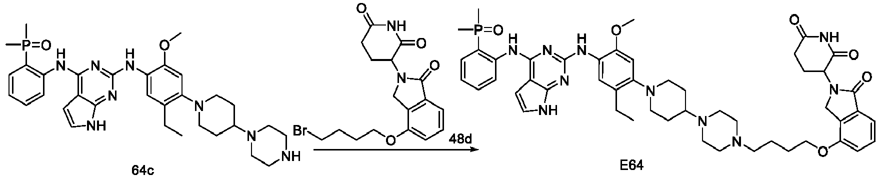

Title compound E64 (3.5 mg, white solid, hydrochloride) was prepared with reference to the method for compound C-1.

¹H NMR (500 MHz, CD₃OD) δ 8.38 (s, 1H), 7.67 (dd, *J* = 10.0, 5.0 Hz, 1H), 7.52-7.49 (m, 2H), 7.43 (t, *J* = 7.5 Hz, 1H), 7.35-7.25 (m, 3H), 7.05 (d, *J=* 5.0 Hz, 1H), 6.86 (s, 1H), 6.48 (s, 1H), 5.08 (dd, *J* = 10.0, 5.0 Hz, 1H), 4.35 (q, *J=* 15.0 Hz, 2H), 4.16 (s, 2H), 3.52-3.16 (m, 8H), 3.27-3.16 (m, 7H), 2.88-2.81 (m, 3H), 2.64-2.55 (m, 4H), 2.22-2.20 (m, 2H), 2.05-2.01 (m, 1H), 1.91-1.86 (m, 7H), 1.81 (s, 3H), 1.78 (s, 3H), 1.09 (t, *J* = 5.0 Hz, 3H). MS m/z (ESI): 917.5 [M+H]⁺.

### Example 65

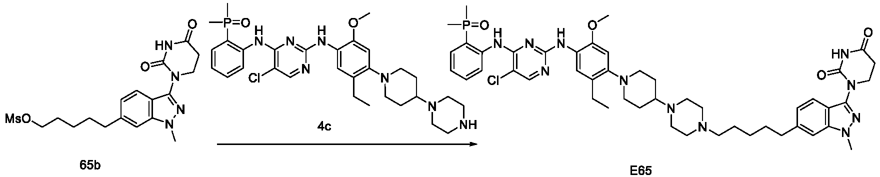

Title compound E65 (4.8 mg, white solid, 5.5 mg, hydrochloride) was prepared with reference to the method for compound C-1.

¹H NMR (500 MHz, CD₃OD) δ 8.28-8.14 (m, 2H), 7.73 (dd, *J* = 10.0, 5.0 Hz, 1H), 7.63-7.57 (m, 2H), 7.44 (t, *J* = 7.50 Hz, 1H), 7.39-7.37 (m, 2H), 7.09 (d, *J* = 10.0 Hz, 1H), 7.01 (s, 1H), 4.03 (s, 3H), 3.90 (s, 3H), 3.75-3.66 (m, 5H), 3.39-3.28 (m, 9H), 3.13-3.06 (m, 2H), 2.91-2.85 (m, 3H), 2.69-2.63(m, 2H), 2.40-2.38(m, 2H), 2.20-2.07(m, 3H), 1.93(s, 3H), 1.90(s, 3H), 1.89-1.81 (m, 5H), 1.55-1.48 (m, 2H), 1.17-1.11 (m, 3H). MS m/z (ESI): 910.6 [M+H]⁺.

### Example 66

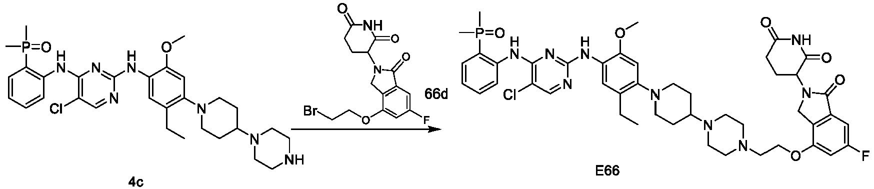

Title compound E66 (white solid, 64.76 mg) was prepared with reference to the method for compound C-1.

¹H NMR (400 MHz, DMSO-*d₆*) δ ppm 12.04 (s, 1 H), 11.04 (s, 1 H), 9.60 (s, 1 H), 8.32 (s, 2 H), 7.65 (dd, *J* =13.2, 7.6 Hz, 1 H), 7.41-7.22 (m, 4 H), 7.17 (m, 1 H), 6.84 (s, 1 H), 5.13 (dd, *J* =13.2, 5.2 Hz, 1 H), 4.65-4.52 (m, 3 H), 4.32 (m, 1 H), 3.87 (s, 4 H), 3.79 (s, 9 H), 3.18 (m, 2 H), 3.02-2.76 (m, 3 H), 2.68-2.54 (m, 3 H), 2.38 (m, 2 H), 2.24 (m, 2 H), 2.08-1.89 (m, 3 H), 1.80 (d, *J* =13.6 Hz, 6 H), 1.07 (t, *J* =7.2 Hz, 3 H); MS m/z (ESI): 902.6 [M+H]⁺.

### Example 67

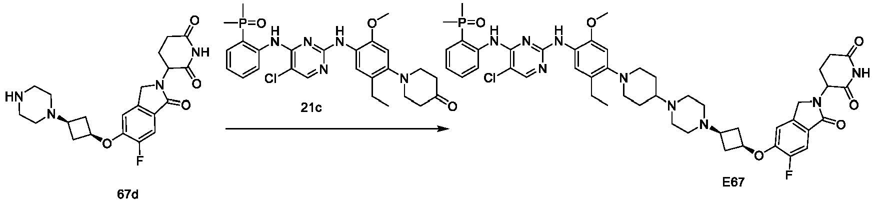

HOAc (6.7 mg, 112 µmol) was added to a solution of compound 67d (30 mg, 72.1 µmol) and compound 21c (30 mg, 56 µmol) in DCM (20 mL) at room temperature before TEA (22.5 mg, 224 µmol) was added. The reaction system was adjusted to a weakly alkaline pH, stirred for 1 h before sodium triacetoxyborohydride (47.5 mg, 224 µmol) was added, and then stirred for another 12 h for reaction. After the reaction was completed, the reaction solution was subjected to reverse-phase preparative chromatography (formic acid system) to give compound E67 (yellow solid, 6.5 mg, hydrochloride). ¹H NMR (500 MHz, CD₃OD) δ 8.13 (s, 2H), 7.73 (dd, *J* = 13.4, 8.4 Hz, 1H), 7.54 (d, *J* = 9.6 Hz, 2H), 7.44 (t, *J* = 7.0 Hz, 1H), 7.35 (s, 1H), 7.28 (d, *J* = 7.0 Hz, 1H), 7.00 (s, 1H), 5.14 (dd, *J* = 13.4, 5.2 Hz, 1H), 4.81-4.73 (m, 1H), 4.47 (q, *J* = 17.0 Hz, 2H), 4.00-3.57 (m, 14H), 3.38 (s, 1H), 3.21-2.99 (m, 4H), 2.93 (ddd, *J* = 18.7, 13.5, 5.3 Hz, 1H), 2.84-2.76 (m, 1H), 2.67 (dd, *J* = 16.9, 8.2 Hz, 4H), 2.50 (qd, *J* = 13.3, 4.7 Hz, 1H), 2.39 (d, *J* = 11.7 Hz, 2H), 2.23-2.12 (m, 3H), 1.92 (s, 3H), 1.89 (s, 3H), 1.15 (s, 3H).MS m/z (ESI): 928.5 [M+H]⁺.

### Example 68

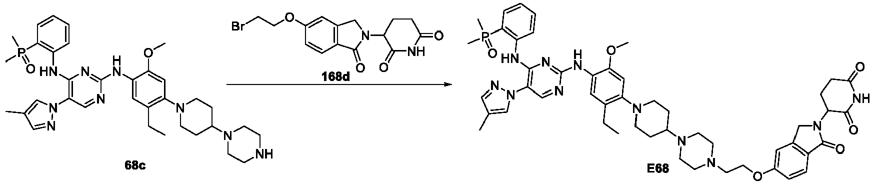

Title compound E68 (white solid, 4 mg, hydrochloride) was prepared with reference to the synthesis method for reference compound C-1.

¹H NMR (500 MHz, CD₃OD) δ 8.21-7.77 (m, 2H), 7.74 (s, 1H), 7.71-7.64 (m, 2H), 7.59 (s, 1H), 7.56-7.46 (m, 1H), 7.37 (t, *J* = 7.3 Hz, 1H), 7.31-7.15 (m, 2H), 7.11 (dd, *J* = 8.5, 2.1 Hz, 1H), 6.81 (s, 1H), 5.07-5.01 (m, 1H), 4.53-4.43 (m, 2H), 4.43-4.33 (m, 2H), 3.91-3.53 (m, 13H), 3.48-3.38 (m, 1H), 3.17 (d, *J* = 11.1 Hz, 2H), 2.90-2.64 (m, 4H), 2.54-2.32 (m, 3H), 2.23 (d, *J* = 10.6 Hz, 2H), 2.12-2.04 (m, 4H), 2.02-1.90 (m, 2H), 1.70 (d, *J* = 13.5 Hz, 6H), 0.98 (t, *J* = 7.0 Hz, 3H). MS m/z (ESI): 931.0 [M+H]⁺.

### Example 69

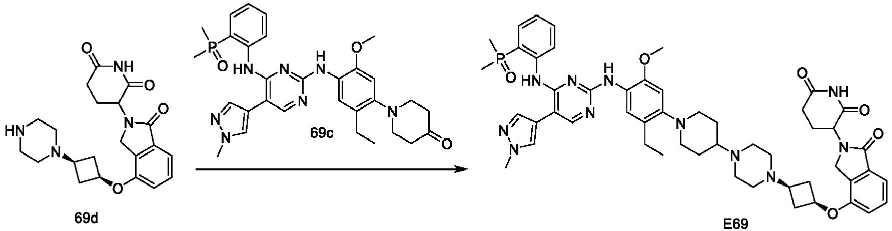

Title compound E69 (white solid, 3.5 mg, hydrochloride) was prepared with reference to the method for compound E67.

¹H NMR (500 MHz, CD₃OD) δ 8.00 (s, 1H), 7.81 (s, 2H), 7.57 (s, 1H), 7.52 (dd, *J* = 12.5, 7.7 Hz, 2H), 7.44 (t, *J* = 7.8 Hz, 1H), 7.38 (t, *J* = 7.8 Hz, 1H), 7.31 (d, *J* = 7.5 Hz, 1H), 7.21 (t, *J* = 7.3 Hz, 1H), 7.00 (d, *J* = 8.1 Hz, 1H), 6.71 (s, 1H), 5.06 (dd, *J* = 13.3, 5.1 Hz, 1H), 4.60 (s, 2H), 4.32 (q, *J* = 17.3 Hz, 2H), 3.86 (s, 3H), 3.77 (s, 3H), 3.52 (s, 2H), 3.13-2.99 (m, 5H), 2.90-2.67 (m, 7H), 2.39 (dd, *J* = 15.0, 7.2 Hz, 4H), 2.34-1.89 (m, 7H), 1.86-1.71 (m, 2H), 1.68 (d, *J* = 13.5 Hz, 6H), 0.93 (d, *J* = 21.1 Hz, 3H); MS m/z (ESI): 956.6 [M+H]⁺.

### Example 70

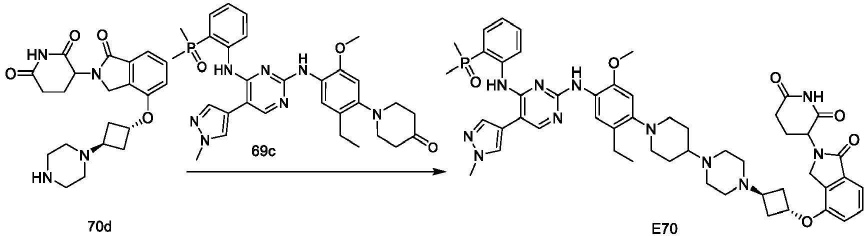

Title compound E70 (white solid, 10 mg, hydrochloride) was prepared with reference to the method for compound E67.

¹H NMR (500 MHz, DMSO-*d₆*) δ 11.21 (s, 1H), 10.99 (s, 1H), 8.27-7.90 (m, 3H), 7.67 (s, 1H), 7.61 (dd, *J* = 13.3, 8.3 Hz, 1H), 7.49 (t, *J* = 7.8 Hz, 1H), 7.42-7.33 (m, 3H), 7.27 (t, *J* = 7.3 Hz, 1H), 7.05 (d, *J* = 8.1 Hz, 1H), 6.83 (s, 1H), 5.13 (dd, *J* = 13.2, 5.1 Hz, 2H), 4.42 (d, *J* = 17.4 Hz, 1H), 4.27 (d, *J* = 17.3 Hz, 1H), 4.01-3.68 (m, 14H), 3.49-3.35 (m, 6H), 3.15 (d, *J* = 9.7 Hz, 2H), 2.98-2.90 (m, 2H), 2.77 (t, *J* = 10.8 Hz, 2H), 2.67-2.55 (m, 2H), 2.44 (dd, *J* = 13.0, 4.3 Hz, 2H), 2.21 (s, 2H), 2.05-1.84 (m, 3H), 1.72 (d, *J* = 13.6 Hz, 6H), 1.03 (s, 3H). MS m/z (ESI): 957.2 [M+H]⁺.

### Example 71

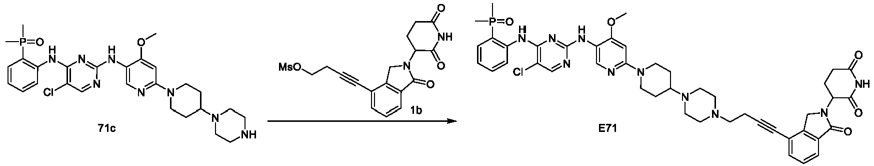

Title compound E71 (white solid, 4 mg, formate) was prepared with reference to the method for compound E4.

¹H NMR (400 M, DMSO-*d₆*) δ 11.19 (s, 1H), 11.03 (s, 1H), 8.41-8.29 (m, 1H), 8.28-8.03 (m, 1H), 7.88 (s, 1H), 7.02 (s, 1H), 7.62 (d, *J* = 7.2 Hz, 1H), 7.51 (d, *J* = 7.2 Hz, 1H), 7.47-7.25 (m, 2H), 7.06 (br, 1H), 7.04 (t, *J* = 7.2 Hz, 1H), 6.45 (s, 1H), 5.16 (dd, *J* = 13.2, 5.2 Hz, 1H), 4.45 (d, *J* = 17.2 Hz, 1H), 4.35-4.28 (m, 3H), 3.78 (s, 3H), 3.02 (s, 1H), (m, 3H), 2.67-2.63 (m, 2H), 2.62 (t, *J* = 6.0 Hz, 2H), 2.59-2.58 (m, 4H), 2.41-2.33 (m, 3H), 2.06-1.95 (m, 1H), 1.84-1.81 (m, 2H), 1.76 (d, *J* = 13.6 Hz, 6H), 1.39-1.36 (m, 2H). MS m/z (ESI): 865.3 [M+H]⁺.

### Example 72

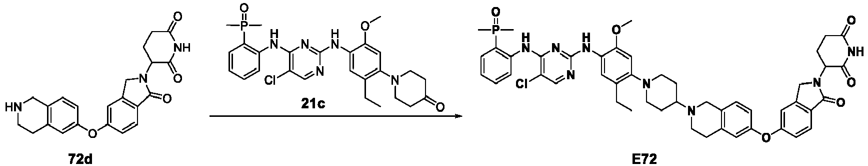

Title product E72 (white solid, 5.2 mg, hydrochloride) was prepared with reference to the method for compound C-6.

¹H NMR (500 MHz, CD₃OD) δ 8.22 (d, *J* = 74.0 Hz, 2H), 7.81 (d, *J* = 8.4 Hz, 1H), 7.73 (dd, *J* = 13.7, 7.7 Hz, 1H), 7.59 (s, 1H), 7.45 (t, *J* = 7.0 Hz, 1H), 7.37 (d, *J* = 8.3 Hz, 2H), 7.22 (d, *J* = 1.5 Hz, 1H), 7.15 (dd, *J* = 8.4, 1.9 Hz, 1H), 7.09-7.03 (m, 3H), 5.16 (dd, *J* = 13.4, 5.1 Hz, 1H), 4.68 (d, *J* = 15.3 Hz, 1H), 4.59 (d, *J* = 15.1 Hz, 1H), 4.49 (q, *J* = 17.2 Hz, 2H), 4.00-3.94 (m, 1H), 3.92 (s, 3H), 3.75 (s, 1H), 3.58-3.50 (m, 1H), 3.41 (s, 2H), 3.26-3.14 (m, 3H), 2.99-2.88 (m, 1H), 2.83-2.78 (m, 1H), 2.69 (dd, *J* = 14.5, 7.2 Hz, 2H), 2.56-2.40 (m, 3H), 2.31-2.17 (m, 3H), 1.91 (d, *J* = 13.5 Hz, 6H), 1.36-1.29 (m, 3H), 1.16 (t, *J* = 7.5 Hz, 3H). MS m/z (ESI): 904.1 [M+H]⁺.

### Example 73

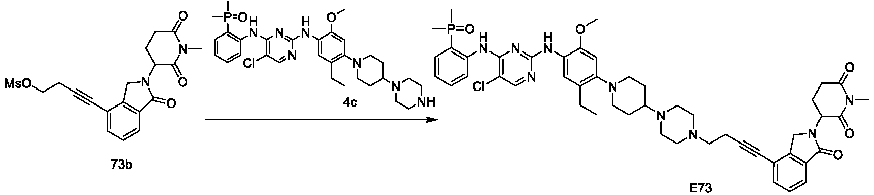

Title compound E73 (white solid, 20 mg, hydrochloride) was prepared with reference to the method for compound E4.

¹H NMR (500 MHz, CD₃OD) δ 8.14 (t, *J* = 70.0 Hz, 2H), 7.83 (d, *J* = 7.6 Hz, 1H), 7.73 (ddd, *J* = 14.0, 8.3, 1.0 Hz, 2H), 7.57 (t, *J* = 7.7 Hz, 2H), 7.44 (t, *J* = 7.1 Hz, 1H), 7.36 (s, 1H), 7.01 (s, 1H), 5.25 (dd, *J* = 13.5, 5.1 Hz, 1H), 4.61 (dd, *J* = 40.5, 17.6 Hz, 2H), 3.90 (s, 10H), 3.67 (t, *J* = 6.8 Hz, 3H), 3.39 (d, *J* = 10.2 Hz, 2H), 3.22-3.16 (m, 6H), 3.09 (s, 2H), 2.98 (ddd, *J* = 9.3, 8.0, 5.5 Hz, 2H), 2.63 (ddd, *J* = 19.1, 18.4, 11.2 Hz, 3H), 2.40 (d, *J* = 11.2 Hz, 2H), 2.27-2.12 (m, 3H), 1.90 (d, *J* = 13.6 Hz, 6H), 1.14 (s, 3H). MS m/z (ESI): 907.1 [M+H]⁺.

### Example 74

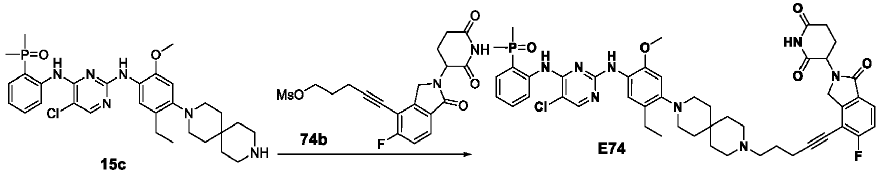

Title compound E74 (white solid, 15 mg, hydrochloride) was prepared with reference to the method for compound C-1.

¹H NMR (500 MHz, CD₃OD) δ 8.27 (s, 1H), 8.16 (s, 1H), 7.85-7.75 (m, 2H), 7.66 (dd, *J* = 15.3, 7.2 Hz, 2H), 7.51 (t, *J* = 7.1 Hz, 1H), 7.37 (dd, *J* = 19.8, 10.3 Hz, 2H), 5.20 (dd, *J* = 13.3, 5.2 Hz, 1H), 4.64 (d, *J* = 17.8 Hz, 1H), 4.54 (d, *J* = 17.7 Hz, 1H), 4.04 (s, 3H), 3.80-3.52 (m, 6H), 3.42-3.36 (m, 2H), 3.33-3.24 (m, 6H), 3.00-2.91 (m, 1H), 2.84-2.79 (m, 1H), 2.76 (t, *J* = 6.8 Hz, 2H), 2.69 (q, *J* = 7.2 Hz, 2H), 2.64-2.53 (m, 1H), 2.38-2.14 (m, 6H), 2.04 (s, 1H), 1.91 (s, 3H), 1.88 (s, 3H), 1.10 (t, *J* = 7.0 Hz, 3H). MS m/z (ESI): 909.4 [M+H]⁺.

### Example 75

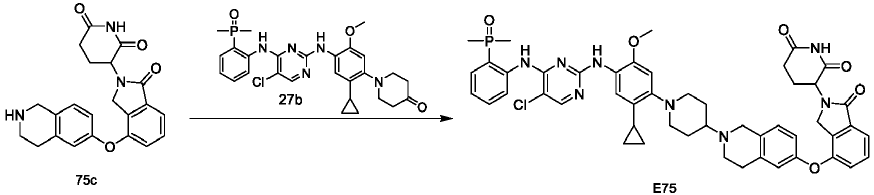

Title compound E75 (brown solid, 4.85 mg) was prepared with reference to the method for compound C-6.

¹H NMR (400 MHz, DMSO-*d₆*) *δ* 11.19 (s, 1H), 10.98 (s, 1H), 8.45 (s, 1H), 8.15 (s, 1H), 8.07 (s, 1H), 7.58-7.47 (m, 3H), 7.29 (m, 1H), 7.18-7.03 (m, 3H), 6.94-6.82 (m, 3H), 6.73 (s, 1H), 5.12 (dd, *J* = 4.8, 13.2 Hz, 1H), 4.46-4.42 (m, 1H), 4.30-4.26 (m, 1H), 3.76 (s, 2H), 3.74 (s, 3H), 2.97-2.85 (m, 1H), 2.84-2.76 (m, 5H), 2.75-2.70 (m, 2H), 2.67 (d, *J* = 1.8 Hz, 1H), 2.60 (s, 2H), 2.21-2.10 (m, 2H), 2.05-1.89 (m, 4H), 1.77 (d, *J* = 10.8 Hz, 9H), 0.92-0.81 (m, 2H), 0.49 (m, 2H). MS m/z (ESI): 915.7 [M+H]⁺.

### Example 76

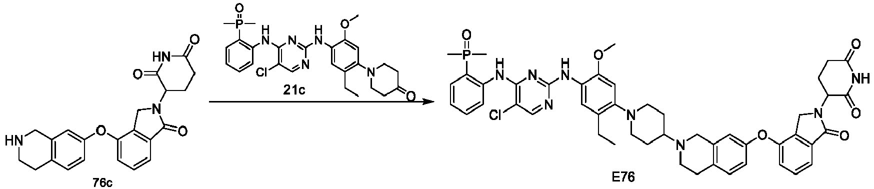

Title compound E76 (white solid, 10 mg) was prepared with reference to the method for compound C-6.

¹H NMR (400 MHz, CD₃OD) δ ppm 8.29 (1 H, d, *J* = 4.4Hz), 8.08 (1 H, s), 7.72 (1 H, s), 7.66 (1 H,m), 7.56-7.61 (1 H, m), 7.52 (2 H, m), 7.26-7.33 (1 H, m), 7.21 (1 H,m), 7.11 (1 H, m), 6.92 (1 H, d, *J* = 8.4 Hz), 6.88 (1 H, s), 6.80 (1 H, s), 5.16 (1 H, dd, *J* = 13.2, 4.8 Hz), 4.39-4.52 (2 H, m), 3.82-3.92 (5 H, m), 3.12 (2 H, d, *J* = 11.2 Hz), 3.00 (4 H, d, *J* = 8.8 Hz), 2.90 (1 H, m), 2.74-2.84 (3 H, m), 2.62-2.72 (1 H, m), 2.47-2.60 (3 H, m), 2.14-2.26 (1 H, m), 2.10 (2 H, d, *J* = 11.2 Hz), 1.78-1.93 (8 H, m), 1.03 (3 H, t, *J* = 7.2 Hz). MS m/z (ESI): 903.8 [M+H]⁺.

### Example 77

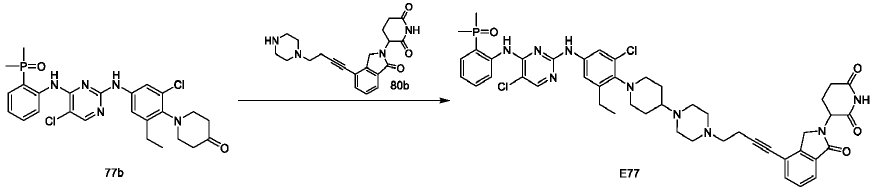

Title compound E77 (white solid, 6.6 mg) was prepared with reference to the method for compound C-6. ¹H NMR (400 MHz, DMSO-*d₆*) δ 11.16 (s, 1H), 11.01 (s, 1H), 9.46 (s, 1H), 8.55 (d, *J* = 4.0 Hz, 1H), 8.22 (s, 1H), 7.71 (d, *J* = 7.4 Hz, 1H), 7.68-7.46 (m, 5H), 7.32 (d, *J* = 2.4 Hz, 1H), 7.19 (t, *J* = 7.7 Hz, 1H), 6.28-5.21 (m, 1H), 5.16 (dd, *J* = 5.1, 13.3 Hz, 1H), 4.52-4.38 (m, 1H), 4.36-4.27 (m, 1H), 2.99-2.89 (m, 1H), 2.89-2.88 (m, 1H), 2.89 (d, *J* = 5.6 Hz, 1H), 2.79 (d, *J* = 10.5 Hz, 2H), 2.71-2.56 (m, 6H), 2.71-2.55 (m, 1H), 2.71-2.55 (m, 1H), 2.70-2.55 (m, 1H), 2.71-2.54 (m, 1H), 2.42 (d, *J* = 4.4 Hz, 2H), 2.37-2.30 (m, 1H), 2.23 (t, *J* = 11.5 Hz, 2H), 2.11-1.98 (m, 2H), 1.84-1.75 (m, 1H), 1.78 (d, *J* = 13.6 Hz, 8H), 1.55-1.40 (m, 2H), 1.09 (t, *J* = 7.6 Hz, 3H), MS m/z (ESI): 896.5 [M+H]⁺.

### Example 78

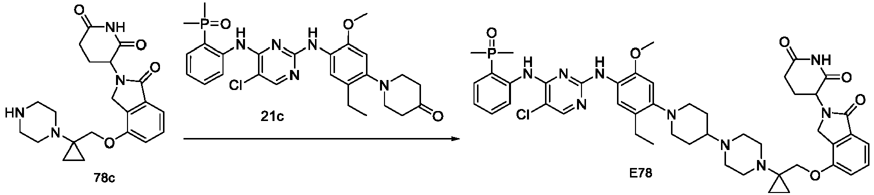

Title compound E78 (white solid, 12 mg, formate) was prepared with reference to the method for compound C-6.

¹H NMR (400 MHz, CD₃OD) δ 8.25 (br s, 1H), 8.06 (s, 1 H), 7.69 (s, 1 H), 7.67-7.60 (m, 1H), 7.56-7.45 (m, 1H), 7.42-7.20 (m, 3H), 7.02 (br d, *J* = 7.63 Hz, 1H), 6.76 (s, 1H), 5.16-5.08 (m, 1H), 4.54-4.32 (m, 2H), 4.15-3.91 (m, 2H), 3.90-3.77 (m, 3H), 3.15 (br d, *J* = 2.25 Hz, 4H), 3.05 (br s, 2H), 2.94 (br d, *J* = 3.38 Hz, 2H), 2.85-2.61 (m, 6H), 2.55-2.46 (m, 4H), 2.27-2.10 (m, 1H), 2.07-1.93 (m, 2H), 1.85 (s, 3H), 1.82 (s, 3H), 1.74-1.64 (m, 2H), 1.05-0.94 (m, 3H), 0.63 (br s, 4H). MS m/z (ESI): 910.8 [M+H]⁺.

### Example 79

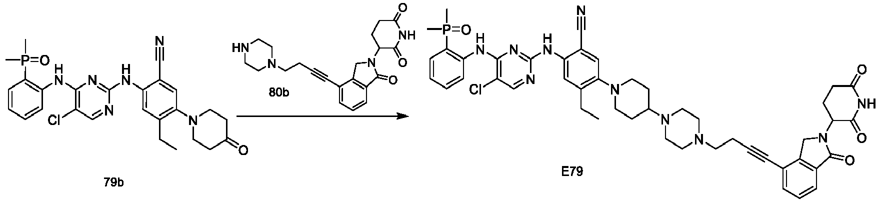

Title compound E79 (yellow solid, 5.37 mg) was prepared with reference to the method for compound C-6.

¹H NMR (400 MHz, CD₃OD) *δ* 9.05-8.89 (m, 1H), 7.90-7.82 (m, 1H), 7.80-7.75 (m, 1H), 7.74-7.62 (m, 3H), 7.57-7.31 (m, 4H), 5.24-5.17 (m, 1H), 4.53 (m, 2H), 3.28-3.22 (m, 3H), 2.99-2.92 (m, 4H), 2.85-2.74 (m, 11H), 2.59-2.48 (m, 2H), 2.26-2.20 (m, 1H), 2.17-2.09 (m, 2H), 2.07-2.01 (m, 1H), 1.95-1.88 (m, 6H), 1.87-1.75 (m, 3H), 1.36-1.29 (m, 3H).MS m/z (ESI): 887.3 [M+H]⁺.

### Example 80

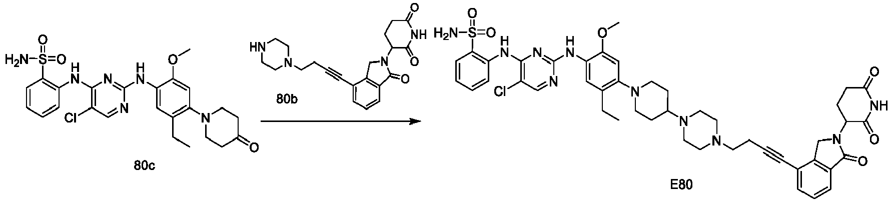

Title compound E80 (white solid, 5 mg, hydrochloride) was prepared with reference to the method for compound E67.

¹H NMR (500 MHz, CD₃OD) δ 8.16 (s, 2H), 8.07 (dd, *J* = 8.0, 1.4 Hz, 1H), 7.82 (d, *J* = 7.2 Hz, 1H), 7.73 (d, *J* = 7.5 Hz, 1H), 7.56 (t, *J* = 7.7 Hz, 2H), 7.47 (t, *J* = 7.7 Hz, 1H), 7.33 (s, 1H), 6.94 (s, 1H), 5.23 (dd, *J* = 13.3, 5.1 Hz, 1H), 4.60 (dd, *J* = 45.4, 17.6 Hz, 2H), 3.83 (d, *J* = 50.1 Hz, 11H), 3.49 (dd, *J* = 22.3, 20.6 Hz, 4H), 3.30 (s, 1H), 3.14 (d, *J* = 4.9 Hz, 2H), 2.96 (ddd, *J* = 18.8, 13.5, 5.5 Hz, 3H), 2.83 (dd, *J* = 10.1, 7.7 Hz, 1H), 2.68-2.52 (m, 3H), 2.35 (d, *J* = 11.1 Hz, 2H), 2.26-2.17 (m, 1H), 2.09 (d, *J* = 11.3 Hz, 2H), 1.14 (s, 3H). MS m/z (ESI): 895.6 [M+H]⁺.

### Example 81

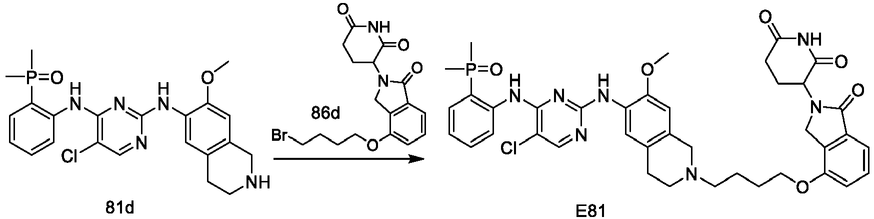

Title compound E81 (white solid, 28 mg, hydrochloride) was prepared with reference to the method for compound C-1.

¹H NMR (500 MHz, CD₃OD) δ 8.27 (dd, *J* = 8.2, 4.6 Hz, 1H), 8.12 (s, 1H), 7.74 (s, 1H), 7.71-7.56 (m, 2H), 7.50 (t, *J* = 7.8 Hz, 1H), 7.41 (d, *J* = 7.4 Hz, 1H), 7.32 (t, *J* = 6.7 Hz, 1H), 7.25 (d, *J* = 8.0 Hz, 1H), 6.70 (s, 1H), 5.16 (dd, *J* = 13.3, 5.1 Hz, 1H), 4.60 (s, 4H), 4.46 (q, *J* = 17.2 Hz, 2H), 4.23 (t, *J* = 5.9 Hz, 2H), 3.87 (s, 3H), 3.66 (s, 2H), 2.97-2.88 (m, 1H), 2.83-2.74 (m, 3H), 2.69-2.64 (m, 3H), 2.52 (ddd, *J* = 26.4, 13.3, 4.6 Hz, 1H), 2.19 (dd, *J* = 9.0, 3.8 Hz, 1H), 1.93-1.90 (m, 1H), 1.88 (s, 3H), 1.85 (s, 3H). MS m/z (ESI): 772.9 [M+H]⁺.

### Example 82

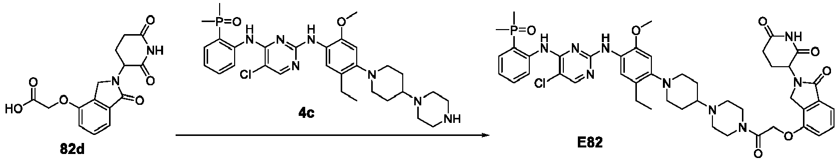

Title compound E82 (white solid, 7 mg, hydrochloride) was prepared with reference to the method of Example 88.

¹H NMR (500 MHz, CD₃OD) δ 8.20 (s, 1H), 8.17 (s, 1H), 7.74 (dd, *J* = 14.0, 7.8 Hz, 1H), 7.60 (s, 1H), 7.53 (t, *J* = 7.8 Hz, 1H), 7.49-7.40 (m, 3H), 7.26 (d, *J* = 8.0 Hz, 1H), 7.15 (d, *J* = 21.6 Hz, 1H), 5.19 (dd, *J* = 13.3, 5.1 Hz, 1H), 5.16-5.02 (m, 2H), 4.56 (dd, *J* = 35.9, 17.4 Hz, 2H), 3.93 (s, 3H), 3.83-3.35 (m, 8H), 3.32-3.14 (m, 5H), 3.00-2.90 (m, 1H), 2.85-2.79 (m, 1H), 2.72-2.64 (m, 2H), 2.62-2.52 (m, 1H), 2.45-2.19 (m, 5H), 1.92 (s, 3H), 1.89 (s, 3H), 1.14 (t, *J* = 7.0 Hz, 3H). MS m/z (ESI): 898.3 [M+H]⁺.

### Example 83

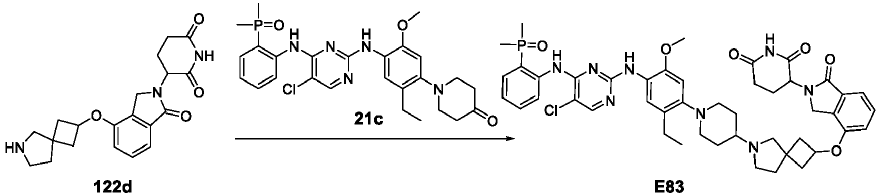

Target product E83 (15 mg, hydrochloride) was prepared with reference to the method for compound E67.

¹H NMR (500 MHz, CD₃OD) δ 8.25 (s, 1H), 8.16 (s, 1H), 7.77 (dd, *J* = 13.5, 7.6 Hz, 1H), 7.63 (s, 1H), 7.57 (s, 1H), 7.49 (dd, *J* = 10.5, 4.8 Hz, 2H), 7.40 (d, *J* = 7.6 Hz, 1H), 7.37 (d, *J* = 4.2 Hz, 1H), 7.11 (dd, *J* = 8.0, 3.0 Hz, 1H), 5.16 (dd, *J* = 13.3, 5.1 Hz, 1H), 4.52-4.39 (m, 2H), 3.99 (d, *J* = 5.3 Hz, 3H), 3.91-3.55 (m, 7H), 3.52-3.40 (m, 2H), 2.90-2.69 (m, 6H), 2.69-2.17 (m, 11H), 1.92 (d, *J* = 1.8 Hz, 3H), 1.89 (d, *J* = 1.8 Hz, 3H), 1.10 (t, *J* = 7.0 Hz, 3H). MS m/z (ESI): 882.2 [M+H]⁺.

### Example 84

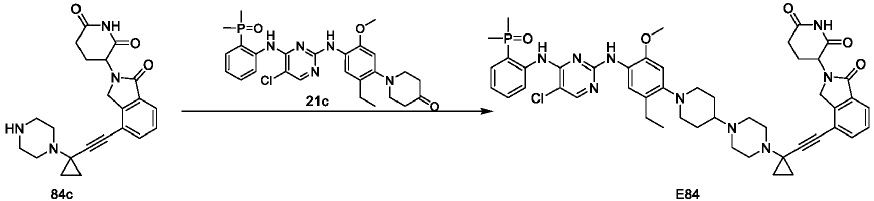

Title compound E84 (white solid, 4 mg) was prepared with reference to the method for compound C-6.

¹H NMR (400 MHz, CD₃OD) δ ppm 8.26-8.24 (m, 1 H) 8.06 (s, 1 H) 7.77 (d, *J* = 7.2 Hz, 1 H), 7.72-7.59 (m, 3 H), 7.54-7.51 (m, 2 H), 7.27 (t, *J* = 7.6 Hz, 1 H), 6.77 (s, 1 H), 5.16 (dd, *J* = 13.6, 5.6 Hz, 1 H), 4.56 (m, 2 H), 3.84 (s, 3 H), 3.06 (d, *J* = 10.8 Hz, 2H), 2.93-2.85 (5 H, m), 2.83-2.56(m, 8 H), 2.51 (m, 2 H), 2.37 (m, 1 H), 2.27-2.15 (m, 1 H), 2.06-1.93 (m, 2 H), 1.86(s, 3H), 1.83(s, 3H), 1.78-1.60 (m, 2 H), 1.12-1.06 (m, 2 H), 1.05-0.96 (m, 5 H). MS m/z (ESI): 904.8 [M+H]⁺.

### Example 85

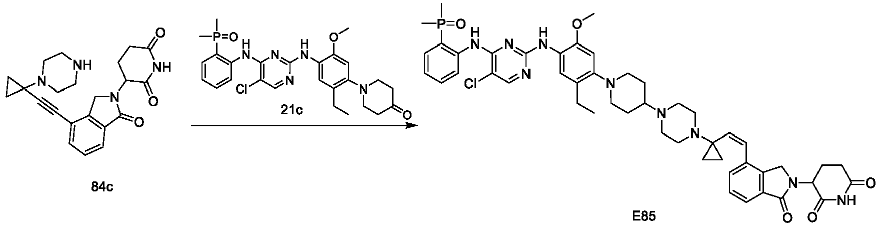

Title compound E85 (white solid, 2 mg) was prepared with reference to the method for compound C-6.

¹H NMR (400 MHz, CD₃OD) δ ppm 8.29-8.26 (m, 1H) 8.09 (s, 1 H) 7.76-7.64 (m, 4H) 7.57-7.51 (m, 2H) 7.35-7.29 (m, 1 H) 6.79 (s, 1 H),6.59 (d, *J* = 12.0 Hz, 1H), 5.98 (d, *J* = 12.0 Hz, 1 H) 5.21 (dd, *J* = 13.2, 5.2 Hz, 1 H) 4.41-4.44 (m, 2 H) 3.84 (s, 3 H) 3.05 (m, 2 H) 2.90 (m, 1 H) 2.80 (m, 2 H) 2.65-2.73 (4 H, m) 2.56-2.46 (m, 4 H) 2.42 (brs, 3 H) 2.30-2.12 (m, 3 H) 2.05-2.02 (m, 1 H) 1.92-1.80 (m, 8 H) 1.67-1.58 (m, 2 H) 1.37-1.27 (m, 3 H) 1.03 (t, *J* = 7.2 Hz, 3 H).MS m/z (ESI): 906.8 [M+H]⁺.

### Example 86

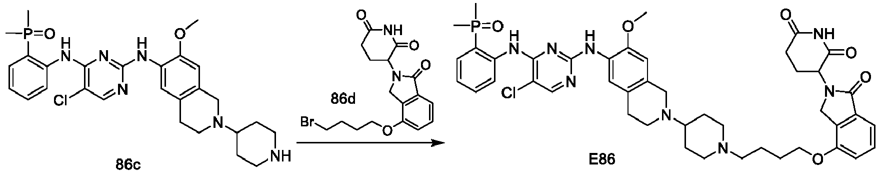

Title compound E86 (white solid, 6.7 mg, hydrochloride) was prepared with reference to the method for compound C-1.

¹H NMR (500 MHz, CD₃OD) δ 8.23 (d, *J* = 31.1 Hz, 2H), 7.77 (d, *J* = 35.3 Hz, 2H), 7.52 (dd, *J* = 16.4, 8.6 Hz, 3H), 7.43 (d, *J* = 7.2 Hz, 1H), 7.27 (d, *J* = 7.8 Hz, 1H), 7.06 (s, 1H), 5.19 (s, 1H), 4.57 (d, *J* = 16.7 Hz, 2H), 4.26 (s, 2H), 3.90 (d, *J* = 29.7 Hz, 8H), 2.88 (dd, *J* = 54.5, 14.0 Hz, 4H), 2.59 (s, 3H), 2.40 (s, 2H), 2.22 (s, 2H), 2.09 (s, 2H), 1.98 (s, 4H), 1.90 (d, *J* = 13.3 Hz, 6H), 1.38 (d, *J* = 6.5 Hz, 3H). MS m/z (ESI): 856.0 [M+H]⁺.

### Example 87

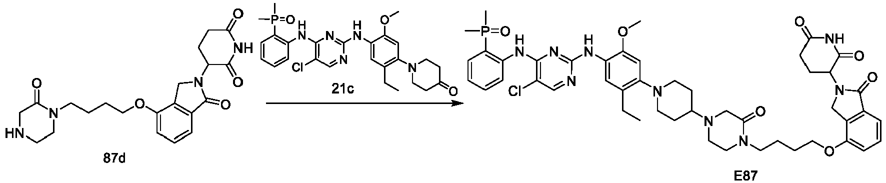

Title compound E87 (white solid, 5 mg, hydrochloride) was prepared with reference to the method for compound E67.

¹H NMR (500 MHz, CD₃OD) δ 8.15 (s, 2H), 7.77-7.65 (m, 1H), 7.54 (t, *J* = 7.9 Hz, 2H), 7.44 (dd, *J* = 12.9, 7.3 Hz, 2H), 7.34 (s, 1H), 7.27 (d, *J* = 8.1 Hz, 1H), 6.93 (s, 1H), 5.16 (dd, *J* = 13.3, 5.2 Hz, 1H), 4.48 (dd, *J* = 42.6, 17.4 Hz, 2H), 4.23 (t, *J* = 5.6 Hz, 2H), 4.05 (d, *J* = 16.7 Hz, 2H), 3.87 (s, 3H), 3.80 (d, *J* = 5.0 Hz, 2H), 3.75 (s, 2H), 3.59 (dd, *J* = 14.2, 9.1 Hz, 3H), 3.32-3.21 (m, 2H), 3.04-2.80 (m, 4H), 2.65-2.48 (m, 3H), 2.35 (d, *J* = 10.9 Hz, 2H), 2.28-2.19 (m, 1H), 2.05 (dt, *J* = 20.3, 10.0 Hz, 2H), 1.91 (s, 3H), 1.88 (s, 6H), 1.10 (s, 3H). MS m/z (ESI): 926.6 [M+H]⁺.

### Example 88

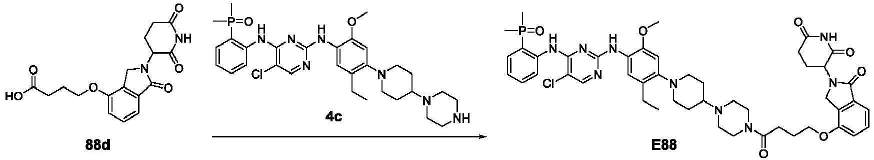

Compound 88d (12 mg, 0.03 mmol), compound 4c (18 mg, 0.03 mmol), HATU (22.8 mg, 0.06), DIPEA (11 mg, 0.09 mmol), and DMF (2 mL) were added together to a reaction flask, The system was stirred at room temperature for 1 h. After the reaction was completed, the reaction solution was filtered and subjected to pre-HPLC to give target product E88 (white solid, 20 mg, hydrochloride). ¹H NMR (500 MHz, CD₃OD) δ 8.21 (s, 2H), 7.75 (dd, *J* = 13.7, 7.6 Hz, 1H), 7.61 (t, *J* = 7.4 Hz, 1H), 7.55-7.44 (m, 3H), 7.40 (t, *J* = 12.8 Hz, 1H), 7.34-7.21 (m, 2H), 5.18 (dd, *J* = 13.2, 5.0 Hz, 1H), 4.49 (dd, *J* = 37.5, 17.4 Hz, 2H), 4.25 (t, *J* = 5.9 Hz, 2H), 3.99-3.83 (m, 4H), 3.80-3.60 (m, 4H), 3.63-3.37 (m, 5H), 3.19 (s, 3H), 2.99-2.91 (m, 1H), 2.82 (d, *J* = 15.7 Hz, 1H), 2.70 (dd, *J* = 14.6, 7.2 Hz, 4H), 2.61-2.52 (m, 1H), 2.48-2.34 (m, 4H), 2.26-2.14 (m, 3H), 1.92 (s, 3H), 1.89 (s, 3H), 1.12 (t, *J* = 7.0 Hz, 3H). MS m/z (ESI): 927.2 [M+H]⁺.

### Example 89

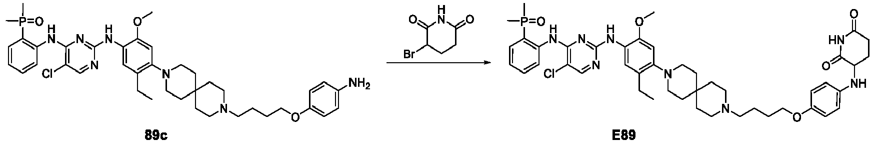

Compound 89c (25 mg, 0.03 mmol), 3-bromopiperidine-2,6-dione (6 mg, 0.03 mmol), and EtOH (3 mL) were added at room temperature to a microwave tube. The system was purged with nitrogen, heated to 140 °C by microwave, and stirred for 1 h. After the reaction was completed, the solvent was removed at reduced pressure, and the residue was subjected to prep-HPLC to give target product E89 (pale yellow solid, 12 mg, hydrochloride). ¹H NMR (500 MHz, CD₃OD) δ 8.28 (s, 1H), 8.15 (s, 1H), 7.78 (ddd, *J* = 13.8, 7.7, 1.2 Hz, 1H), 7.67 (dd, *J* = 17.8, 10.1 Hz, 2H), 7.53-7.42 (m, 4H), 7.21-7.15 (m, 2H), 4.76-4.72 (m, 1H), 4.15 (t, *J* = 5.9 Hz, 2H), 4.05 (s, 3H), 3.80-3.65 (m, 3H), 3.58 (d, *J* = 12.9 Hz, 2H), 3.33-3.20 (m, 6H), 2.78-2.67 (m, 4H), 2.40-2.38 (m, 2H), 2.13-2.00 (m, 6H), 1.98-1.87 (m, 11H), 1.10 (t, *J* = 7.2 Hz, 3H). MS m/z (ESI): 858.2 [M+H]⁺.

### Example 90

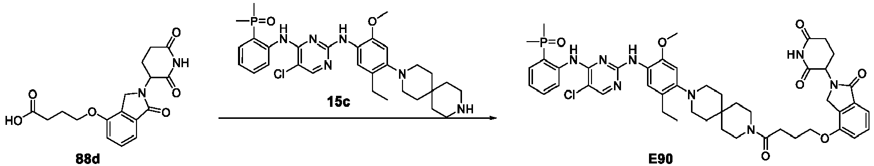

Title compound E90 (white solid, 20 mg) was prepared with reference to the synthesis method for compound E88.

¹H NMR (500 MHz, CD₃OD) δ 8.30 (s, 1H), 8.11 (s, 1H), 7.79 (dd, *J* = 13.1, 7.2 Hz, 1H), 7.74 (s, 1H), 7.66 (t, *J* = 7.8 Hz, 1H), 7.52 (t, *J* = 7.8 Hz, 2H), 7.41 (d, *J* = 7.3 Hz, 1H), 7.37 (s, 1H), 7.26 (d, *J* = 8.1 Hz, 1H), 5.18 (dd, *J* = 13.3, 5.1 Hz, 1H), 4.53 (d, *J* = 17.4 Hz, 1H), 4.47-4.42 (m, 1H), 4.28-4.22 (m, 1H), 4.22-4.15 (m, 1H), 4.04 (s, 3H), 3.88-3.48 (m, 8H), 3.00-2.90 (m, 1H), 2.85-2.72 (m, 2H), 2.66 (dd, *J* = 14.3, 6.9 Hz, 2H), 2.61-2.51 (m, 2H), 2.27-2.02 (m, 7H), 1.90 (d, *J* = 13.5 Hz, 6H), 1.78-1.44 (m, 4H), 1.08 (t, *J* = 7.1 Hz, 3H). MS m/z (ESI): 912.2 [M+H]⁺.

### Example 91

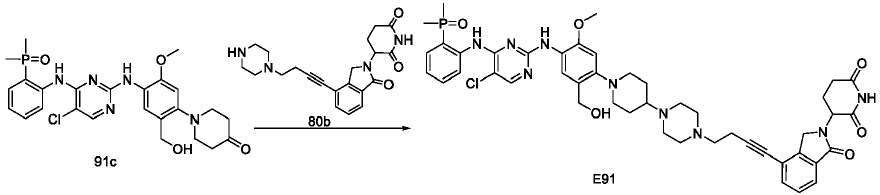

Title compound E91 (gray solid, 5 mg) was prepared with reference to the method for compound E67.

¹H NMR (500 MHz, CD₃OD) δ 8.49 (dd, *J* = 10.0, 5.0 Hz, 1H), 8.23 (s, 1H), 8.03 (s, 1H), 7.93 (d, *J* = 10.0 Hz, 1H), 7.81-7.67 (m, 5H), 7.46-7.42 (m, 1H), 7.01 (s, 1H), 5.37 (dd, *J* = 10.0, 5.0 Hz, 1H), 4.75-4.67 (m, 5H), 4.63 (q, *J* = 15.0 Hz, 2H), 4.01 (s, 3H), 3.42-3.39 (m, 2H), 3.14-3.09 (m, 4H), 2.99-2.89 (m, 10H), 2.73-2.64 (m, 1H), 2.39-2.37(m, 1H), 2.28-2.25(m, 2H), 2.03 (s, 3H), 2.00 (s, 3H), 1.95-1.92(m, 2H). MS m/z (ESI): 894.6 [M+H]⁺.

### Example 92

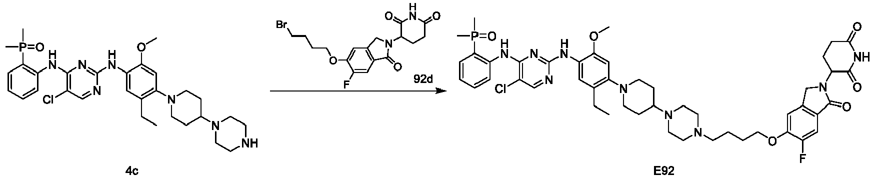

Compound E92 (pale yellow solid, 50 mg, hydrochloride) was prepared with reference to the method for compound C-1.

¹H NMR (500 MHz, DMSO-*d₆*) δ 11.55 (s, 1H), 10.98 (s, 1H), 8.89-8.61 (m, 1H), 8.39 (s, 1H), 8.19 (s, 1H), 7.60 (dd, *J* = 14.1, 8.0 Hz, 1H), 7.54 (d, *J* = 9.9 Hz, 1H), 7.45 (d, *J* = 7.3 Hz, 1H), 7.41 (s, 1H), 7.35 (s, 1H), 7.21-7.15 (m, 1H), 6.81 (s, 1H), 5.09 (dd, *J* = 13.3, 5.0 Hz, 1H), 4.40 (d, *J* = 17.4 Hz, 1H), 4.28 (d, *J* = 17.2 Hz, 1H), 4.20 (s, 2H), 3.78 (s, 8H), 3.28-3.22 (m, 4H), 3.14 (d, *J* = 10.1 Hz, 4H), 2.94-2.87 (m, 1H), 2.79 (d, *J* = 12.8 Hz, 2H), 2.61 (s, 1H), 2.39 (dd, *J* = 12.8, 4.5 Hz, 1H), 2.25-2.15 (m, 2H), 2.02-1.96 (m, 1H), 1.95-1.82 (m, 8H), 1.80 (s, 3H), 1.77 (s, 3H), 1.07 (t, *J* = 7.6 Hz, 3H). MS m/z (ESI): 931.2 [M+H]⁺.

### Example 93

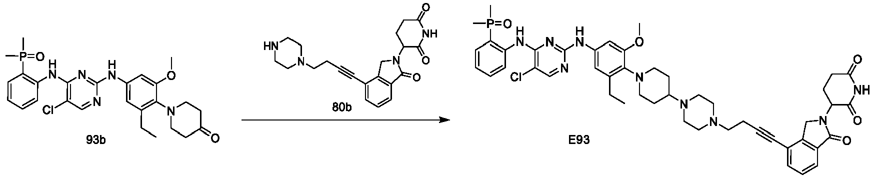

Title compound E93 (11 mg, formate) was prepared with reference to the method for compound C-6.

¹H NMR (400 MHz, CD₃OD) δ 8.32 (s, 1H), 8.12 (s, 1H), 7.84-7.76 (m, 1H), 7.75-7.65 (m, 2H), 7.54-7.52 (m, 2H), 7.45-7.36 (m, 1H), 6.87 (d, *J* = 7.6 Hz, 2H), 5.25-5.17 (m, 1H), 4.68-4.50 (m, 3H), 4.10-3.75 (m, 8H), 3.74-3.46 (m, 9H), 3.19-3.13 (m, 2H), 3.03-2.53 (m, 8H), 2.29-2.17 (m, 3H), 2.04-1.94 (m, 2H), 1.90(s, 3H), 1.87(s, 3H), 1.15 (t, *J* = 7.2 Hz, 3H). MS m/z (ESI): 892.4 [M+H]⁺.

### Example 94

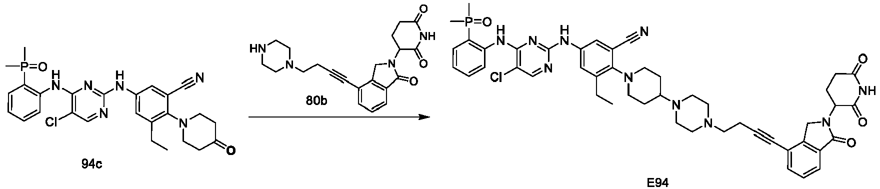

Title compound E94 (5 mg, hydrochloride) was prepared with reference to the method for compound E67.

¹H NMR (400 MHz, CD₃OD) δ 8.31-8.22 (m, 1H), 8.19-8.14 (m, 1H), 7.90-7.80 (m, 1H), 7.80-7.73 (m, 1H), 7.72-7.60 (m, 3H), 7.58-7.45 (m, 2H), 7.37-7.30 (m, 1H), 5.20 (dd, *J* = 13.3, 5.1 Hz, 1H), 5.24-5.15 (m, 1H), 4.60-4.46 (m, 1H), 4.60-4.46 (m, 1H), 3.92-3.34 (m, 6H), 3.23-2.74 (m, 11H), 2.73-2.61 (m, 2H), 2.61-2.47 (m, 1H), 2.28-2.16 (m, 3H), 2.06-1.90 (m, 2H), 1.86 (d, *J* = 13.5 Hz, 6H), 1.41-1.26 (m, 1H), 1.17 (t, *J* = 7.5 Hz, 2H), 1.22-1.11 (m, 1H). MS m/z (ESI): 887.3 [M+H]⁺.

### Example 95

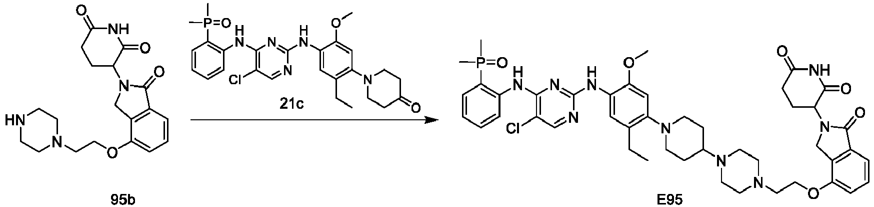

Title compound E95 (white solid, 5 mg, hydrochloride) was prepared with reference to the method for compound E67.

¹H NMR (400 MHz, CD₃OD) δ 8.41-8.18 (m, 1H), 8.09 (t, *J* = 11.9 Hz, 1H), 7.75-7.66 (m, 1H), 7.59-7.51 (m, 2H), 7.51-7.45 (m, 1H), 7.44-7.37 (m, 1H), 7.31 (d, *J* = 7.6 Hz, 2H), 6.95 (s, 1H), 5.18 (dd, *J* = 5.1, 13.2 Hz, 1H), 4.74-4.49 (m, 4H), 4.03-3.78 (m, 13H), 3.67-3.52 (m, 2H), 3.06-2.86 (m, 3H), 2.85-2.74 (m, 1H), 2.69-2.49 (m, 3H), 2.36 (d, *J* = 10.5 Hz, 2H), 2.27-2.01 (m, 4H), 1.91-1.85 (m, 6H), 1.20-1.07 (m, 3H).MS m/z (ESI): 884.7 [M+H]⁺.

### Example 97

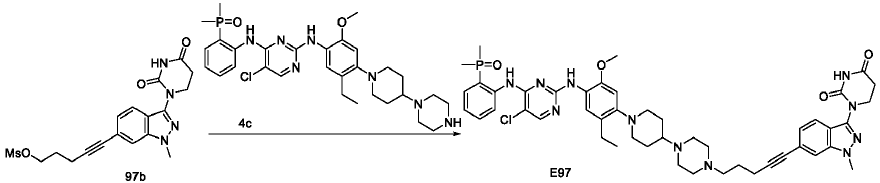

Title compound E97 (white solid, 3 mg, hydrochloride) was prepared with reference to the method for compound C-1.

¹H NMR (500 MHz, CD₃OD) δ 8.22-8.20 (m, 2H), 7.78-7.74 (m, 1H), 7.69-7.62 (m, 3H), 7.50-7.47 (m, 2H), 7.22-7.20 (m, 2H), 4.08-3.86 (m, 16H), 3.61-3.47 (m, 6H), 2.91-2.89 (m, 2H), 2.74-2.69 (m, 4H), 2.54-2.51(m, 4H), 2.44-2.21(m, 2H), 1.93(s, 3H), 1.90(s, 3H), 1.16-1.13(m, 3H). MS m/z (ESI): 906.7 [M+H]⁺.

### Example 98

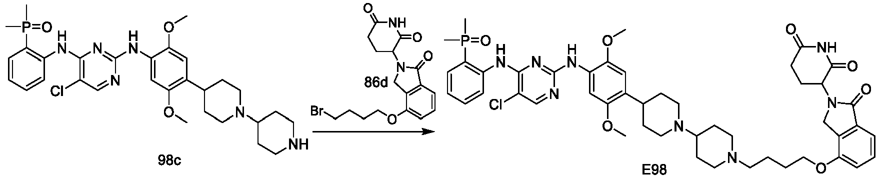

Title compound E98 (white solid, 30 mg, hydrochloride) was prepared with reference to the method for compound C-1.

¹H NMR (500 MHz, CD₃OD) δ 8.27 (d, *J* = 70.1 Hz, 1H), 8.12 (d, *J* = 41.9 Hz, 1H), 7.72 (dd, *J* = 13.7, 8.1 Hz, 1H), 7.53 (t, *J* = 7.8 Hz, 2H), 7.44 (d, *J=* 7.4 Hz, 2H), 7.27 (d, *J* = 8.1 Hz, 1H), 7.14 (s, 1H), 7.06 (s, 1H), 5.20 (dd, *J* = 13.3, 5.1 Hz, 1H), 4.60-4.38 (m, 2H), 4.26 (t, *J* = 5.7 Hz, 2H), 3.95-3.82 (m, 5H), 3.70 (dd, *J* = 62.8, 30.1 Hz, 6H), 3.50-3.37 (m, 2H), 3.33-3.16 (m, 5H), 3.02-2.90 (m, 1H), 2.83 (d, *J* = 15.6 Hz, 1H), 2.66-2.49 (m, 3H), 2.31 (dd, *J* = 23.6, 11.9 Hz, 4H), 2.26-2.13 (m, 3H), 2.12-2.02 (m, 2H), 2.01-1.95 (m, 2H), 1.92 (d, *J* = 13.6 Hz, 6H). MS m/z (ESI): 913.5 [M+H]⁺.

### Example 99

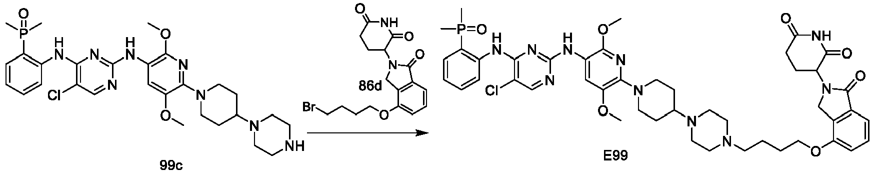

Title compound E99 (white solid, 30 mg, hydrochloride) was prepared with reference to the method for compound C-1.

¹H NMR (500 MHz, CD₃OD) δ 8.33 (s, 1H), 8.10 (s, 1H), 7.72 (s, 1H), 7.60 (ddd, *J=* 14.1, 7.7, 1.4 Hz, 1H), 7.52-7.35 (m, 3H), 7.24 (dd, *J* = 9.5, 4.5 Hz, 2H), 5.17 (dd, *J* = 13.4, 5.2 Hz, 1H), 4.60 (s, 3H), 4.45 (q, *J* = 17.2 Hz, 2H), 4.20 (t, *J* = 6.1 Hz, 2H), 3.95 (d, *J* = 6.4 Hz, 3H), 3.58 (s, 3H), 3.39 (t, *J* = 15.7 Hz, 2H), 2.99-2.78 (m, 5H), 2.70 (s, 5H), 2.53-2.45 (m, 3H), 2.43 (d, *J* = 11.6 Hz, 1H), 2.20 (ddt, *J* = 12.8, 5.3, 2.7 Hz, 1H), 1.94 (d, *J* = 14.2 Hz, 2H), 1.88 (s, 4H), 1.86 (s, 3H), 1.76 (dt, *J* = 14.3, 7.2 Hz, 2H), 1.64 (dt, *J* = 12.1, 8.6 Hz, 2H); MS m/z (ESI): 915.6 [M+H]⁺.

### Example 100

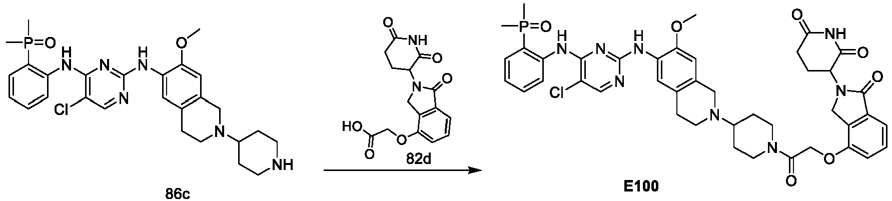

Title compound E100 (white solid, 5 mg, hydrochloride) was prepared with reference to the method for compound E88.

¹H NMR (500 MHz, CD₃OD) δ 8.24 (d, *J* = 21.8 Hz, 1H), 8.18 (s, 1H), 7.80 (dd, *J* = 13.4, 7.7 Hz, 1H), 7.71 (t, *J* = 7.5 Hz, 1H), 7.58-7.45 (m, 4H), 7.29-7.20 (m, 1H), 7.05-6.95 (m, 1H), 5.24-5.10 (m, 3H), 5.03 (t, *J* = 13.4 Hz, 2H), 4.61-4.13 (m, 3H), 3.93 (s, 3H), 3.82 (ddd, *J* = 15.8, 9.8, 6.8 Hz, 1H), 3.74 (d, *J* = 6.7 Hz, 1H), 3.47-3.34 (m, 2H), 3.30-3.17 (m, 2H), 3.12 (s, 1H), 2.98-2.88 (m, 1H), 2.87-2.72 (m, 3H), 2.55 (d, *J* = 11.7 Hz, 1H), 2.25 (dd, *J* = 38.0, 30.7 Hz, 3H), 1.89 (d, *J* = 13.5 Hz, 6H), 1.82-1.54 (m, 1H). MS m/z (ESI): 841.6 [M+H]⁺.

### Example 101

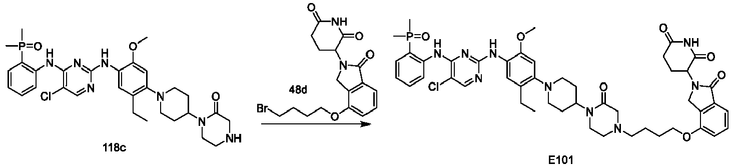

Compound E101 (pale yellow solid, 18 mg, hydrochloride) was prepared with reference to the method for compound C-1.

¹H NMR (500 MHz, DMSO-*d₆*) δ 11.80 (br s, 1H), 11.47 (br s, 1H), 10.99 (s, 1H), 8.36 (s, 1H), 8.24 (s, 1H), 7.62 (dd, *J* = 13.7, 8.2 Hz, 1H), 7.50 (t, *J* = 7.8 Hz, 1H), 7.42-7.32 (m, 3H), 7.28-7.19 (m, 2H), 6.93 (br s, 1H), 5.12 (dd, *J* = 13.3, 5.1 Hz, 1H), 4.44 (d, *J* = 17.3 Hz, 1H), 4.26 (d, *J* = 17.4 Hz, 1H), 4.19-4.14 (m, 1H), 3.95 (d, *J* = 15.8 Hz, 1H), 3.86-3.56 (m, 12H), 3.24-3.07 (m, 5H), 2.95-2.89 (m, 1H), 2.62-2.55 (m, 3H), 2.44-2.40 (m, 1H), 2.04-1.88 (m, 4H), 1.85-1.75 (m, 8H), 1.77-1.60 (m, 2H), 1.08 (t, *J* = 7.4 Hz, 3H). MS m/z (ESI): 927.6 [M+H]⁺.

### Example 102

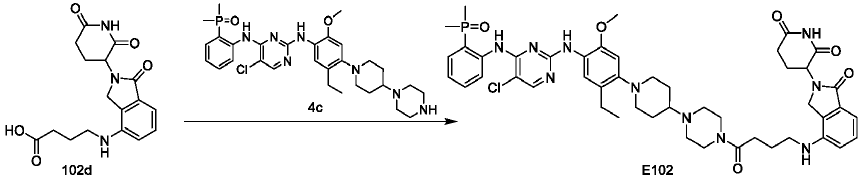

Title compound E102 (pale yellow solid, 45 mg, hydrochloride) was prepared with reference to the method for compound E88.

¹H NMR (500 MHz, CD₃OD) δ 8.16 (br s, 2H), 7.76-7.68 (m, 2H), 7.66 (t, *J* = 7.7 Hz, 1H), 7.58 (d, *J* = 7.7 Hz, 2H), 7.44 (t, *J* = 7.0 Hz, 2H), 7.14 (s, 1H), 5.21 (dd, *J* = 13.3, 5.2 Hz, 1H), 4.74-4.62 (m, 3H), 4.23 (br s, 1H), 3.91 (s, 3H), 3.75-3.44 (m, 9H), 3.37-3.34 (m, 2H), 3.25-3.09 (m, 3H), 2.97-2.90 (m, 1H), 2.83-2.78 (m, 1H), 2.66 (dd, *J* = 14.6, 7.3 Hz, 3H), 2.60-2.51 (m, 1H), 2.39 (d, *J* = 11.0 Hz, 2H), 2.34-2.20 (m, 3H), 2.12-2.07 (m, 2H), 1.89 (s, 3H), 1.86 (s, 3H), 1.10 (t, *J* = 7.5 Hz, 3H). MS m/z (ESI): 926.7 [M+H]⁺.

### Example 103

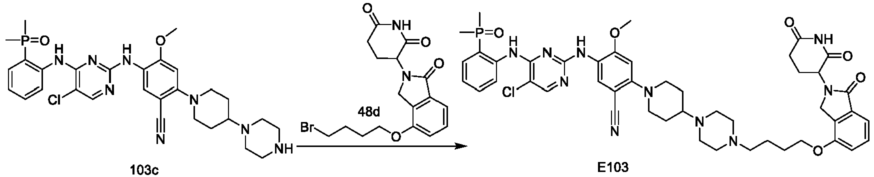

Title compound E103 (white solid, 14.48 mg, formate) was prepared with reference to the method for compound C-1.

¹ HNMR (400 MHz, DMSO-*d₆*) δ 11.22 (s, 1H), 10.97 (s, 1H), 8.51-8.36 (m, 1H), 8.25 (s, 1H), 8.18-8.10 (m, 1H), 7.94 (s, 1H), 7.60-7.51 (m, 1H), 7.51-7.42 (m, 2H), 7.31 (d, *J* = 7.4 Hz, 1H), 7.25 (d, *J* = 8.1 Hz, 1H), 7.14 (t, *J* = 7.0 Hz, 1H), 6.74 (s, 1H), 5.11 (dd, *J* = 5.1, 13.3 Hz, 1H), 4.43-4.32 (m, 1H), 4.28-4.19 (m, 1H), 4.15 (t, *J* = 6.3 Hz, 2H), 3.88 (s, 3H), 3.55 (d, *J* = 11.1 Hz, 2H), 2.97-2.88 (m, 1H), 2.83 (t, *J* = 11.6 Hz, 2H), 2.63-2.53 (m, 6H), 2.46-2.36 (m, 7H), 2.03-1.97 (m, 1H), 1.94-1.88 (m, 2H), 1.77 (d, *J* = 13.6 Hz, 8H), 1.65-1.55 (m, 4H). MS m/z (ESI): 909.4 [M+H]⁺.

### Example 104

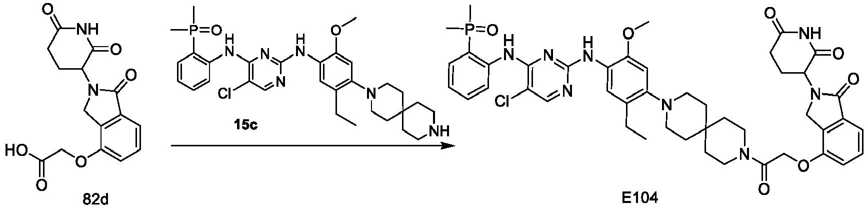

Title compound E100 (white solid, 5 mg, hydrochloride) was prepared with reference to the method for compound E88.

¹H NMR (400 MHz, DMSO-*d₆*) *δ* 11.75-11.34 (m, 1H), 10.98 (s, 1H), 8.46-8.33 (m, 1H), 8.19 (s, 1H), 7.60 (dd, *J* = 7.5, 14.3 Hz, 1H), 7.54-7.22 (m, 4H), 7.22-7.14 (m, 2H), 6.98-6.82 (m, 1H), 5.12 (dd, *J* = 5.3, 12.9 Hz, 1H), 5.06-4.97 (m, 2H), 4.44-4.36 (m, 1H), 4.32-4.23 (m, 1H), 3.86-3.75 (m, 3H), 3.46 (s, 6H), 2.99-2.83 (m, 4H), 2.64-2.56 (m, 3H), 2.04-1.98 (m, 1H), 1.78 (d, *J* = 13.6 Hz, 7H), 1.73-1.63 (m, 3H), 1.62-1.48 (m, 4H), 1.07 (t, *J* = 7.4 Hz, 3H);MS m/z (ESI): 883.6[M+H]⁺.

### Example 105

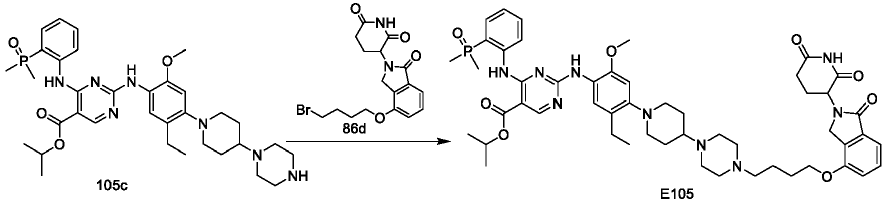

Title compound E105 (white solid, 30 mg, hydrochloride) was prepared with reference to the method for compound C-1.

¹H NMR (500 MHz, CD₃OD) δ 7.79 (dd, *J* = 11.8, 7.7 Hz, 1H), 7.60 (s, 1H), 7.57-7.50 (m, 2H), 7.42-7.30 (m, 2H), 7.29 (d, *J* = 7.2 Hz, 1H), 7.12 (d, *J* = 7.9 Hz, 1H), 6.62 (s, 1H), 5.21-5.12 (m, 1H), 5.06 (dd, *J* = 13.3, 5.2 Hz, 1H), 4.34 (q, *J* = 17.3 Hz, 2H), 4.09 (t, *J* = 6.1 Hz, 2H), 3.74 (s, 3H), 2.92 (d, *J* = 10.9 Hz, 2H), 2.72 (dddd, *J* = 57.5, 55.3, 17.5, 9.7 Hz, 10H), 2.41 (dt, *J* = 13.3, 6.6 Hz, 4H), 2.26 (d, *J* = 44.4 Hz, 3H), 2.11-2.01 (m, 1H), 1.89 (d, *J* = 11.3 Hz, 2H), 1.76 (dd, *J* = 14.0, 6.6 Hz, 2H), 1.68 (t, *J* = 13.2 Hz, 9H), 1.61-1.51 (m, 2H), 1.29 (d, *J* = 6.3 Hz, 6H), 0.81-0.71 (m, 3H). MS m/z (ESI): 964.9 [M+H]⁺.

### Example 106

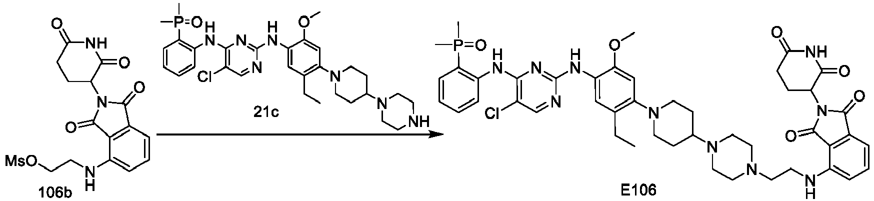

Title compound E106 (white solid, 3 mg, hydrochloride) was prepared with reference to the method for compound C-1.

¹H NMR (500 MHz, CD₃OD) δ 78.00 (s, 1H), 7.58 (ddd, *J* = 15.7, 11.3, 7.6 Hz, 2H), 7.42 (d, *J* = 34.1 Hz, 1H), 7.28 (ddd, *J* = 29.0, 18.1, 10.6 Hz, 3H), 7.15 (d, *J* = 8.6 Hz, 1H), 7.08 (d, *J* = 7.1 Hz, 1H), 6.87 (s, 1H), 4.99 (dd, *J* = 12.6, 5.4 Hz, 2H), 3.84-3.65 (m, 10H), 3.45 (dd, *J* = 27.0, 21.5 Hz, 4H), 2.92 (s, 2H), 2.82-2.72 (m, 1H), 2.69-2.58 (m, 2H), 2.53 (d, *J* = 6.5 Hz, 2H), 2.27 (d, *J* = 11.2 Hz, 2H), 2.02 (d, *J* = 4.8 Hz, 3H), 1.79 (d, *J* = 13.5 Hz, 6H), 1.32-1.17 (m, 3H), 1.03 (s, 3H). MS m/z (ESI): 897.8 [M+H]⁺.

### Example 107

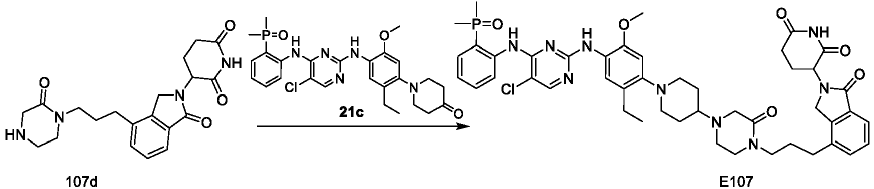

Title compound E107 (white solid, 8 mg, hydrochloride) was prepared with reference to the method for compound E67.

¹H NMR (500 MHz, CD₃OD) δ 8.13 (s, 1H), 7.78-7.67 (m, 3H), 7.64-7.47 (m, 3H), 7.41 (dd, *J* = 37.4, 30.3 Hz, 2H), 6.94 (s, 1H), 5.22 (dd, *J* = 13.4, 5.1 Hz, 1H), 4.58 (dd, *J* = 56.5, 17.2 Hz, 2H), 4.05 (s, 2H), 3.88 (s, 4H), 3.83-3.50 (m, 6H), 3.00-2.90 (m, 3H), 2.86-2.78 (m, 3H), 2.61 (ddd, *J* = 26.6, 13.1, 4.4 Hz, 3H), 2.24 (ddd, *J* = 20.8, 16.7, 9.6 Hz, 3H), 2.06 (dd, *J* = 11.6, 6.7 Hz, 5H), 1.91 (d, *J* = 13.6 Hz, 6H), 1.57-1.34 (m, 1H), 1.19 (d, *J* = 41.1 Hz, 3H); MS m/z (ESI): 897.0 [M+H]⁺.

### Example 108

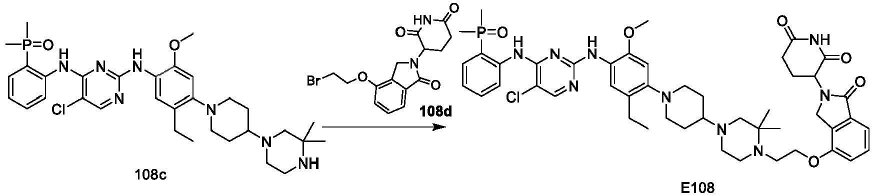

Title compound E108 (white solid, 10 mg, hydrochloride) was prepared with reference to the method for compound C-1.

¹H NMR (500 MHz, CD₃OD) δ 8.11 (s, 2H), 7.64 (dd, *J* = 13.8, 7.7 Hz, 1H), 7.49 (dd, *J* = 18.1, 10.5 Hz, 1H), 7.43 (t, *J* = 7.8 Hz, 2H), 7.37 (t, *J* = 5.5 Hz, 2H), 7.20 (d, *J* = 8.1 Hz, 1H), 7.14 (s, 1H), 5.07 (dd, *J* = 13.3, 5.1 Hz, 1H), 4.66-4.35 (m, 4H), 4.23-3.54 (m, 12H), 3.50 (d, *J* = 4.0 Hz, 4H), 2.87-2.66 (m, 2H), 2.59 (q, *J* = 7.2 Hz, 2H), 2.50-2.32 (m, 5H), 2.10 (dd, *J* = 9.0, 3.7 Hz, 1H), 1.78 (d, *J* = 13.6 Hz, 6H), 1.69 (s, 6H), 1.00 (s, 3H). MS m/z (ESI): 912.9 [M+H]⁺.

### Example 109

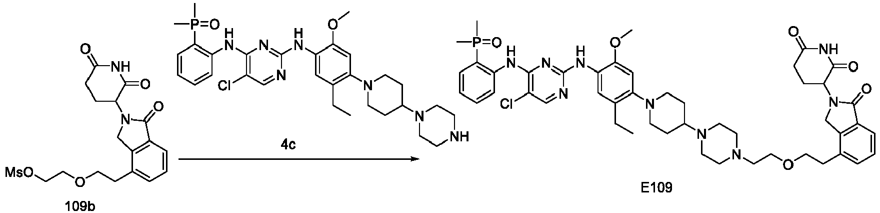

Title compound E109 (white solid, 5 mg, hydrochloride) was prepared with reference to the method for compound C-1.

¹H NMR (500 MHz, CD₃OD) δ 8.25-8.19 (m, 2H), 7.77-7.73 (m, 2H), 7.64-7.56 (m, 3H), 7.47 (t, *J* = 5.0 Hz, 2H), 7.19-7.16 (m, 1H), 5.22 (dd, *J* = 10.0, 5.0 Hz, 1H), 4.65 (q, *J* = 15.0 Hz, 2H), 3.96 (s, 3H), 3.94-3.50 (m, 20H), 3.37-3.35 (m, 1H), 3.11-2.81 (m, 3H), 2.71-2.59 (m, 1H), 2.50-2.24 (m, 6H), 1.92 (s, 3H), 1.88 (s, 3H), 1.16-1.10 (m, 3H). MS m/z (ESI): 912.5 [M+H]⁺.

### Example 110

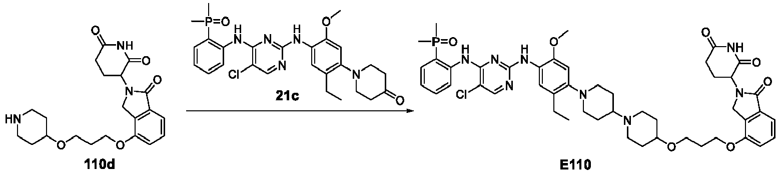

Title product E110 (pale yellow solid, 15 mg, hydrochloride) was prepared with reference to the synthesis method for compound E67.

¹H NMR (500 MHz, CD₃OD) δ 8.08 (s, 2H), 7.63 (dd, *J* = 13.9, 7.7 Hz, 1H), 7.53-7.45 (m, 1H), 7.46-7.40 (m, 1H), 7.40-7.30 (m, 2H), 7.29 (d, *J* = 7.5 Hz, 1H), 7.21 (d, *J* = 8.1 Hz, 1H), 7.05 (d, *J* = 21.5 Hz, 1H), 5.06 (dd, *J* = 13.4, 5.1 Hz, 1H), 4.45-4.29 (m, 2H), 4.27-4.19 (m, 1H), 3.83 (d, *J* = 9.2 Hz, 3H), 3.71-3.62 (m, 2H), 3.58-3.52 (m, 2H), 3.43-3.24 (m, 4H), 3.19-3.05 (m, 2H), 3.02-2.88 (m, 1H), 2.87-2.78 (m, 1H), 2.76-2.68 (m, 1H), 2.62-2.41 (m, 3H), 2.30-2.18 (m, 1H), 2.16-1.88 (m, 11H), 1.80 (s, 3H), 1.77 (s, 3H), 0.99 (t, *J* =7.2 Hz, 3H). MS m/z (ESI): 914.1 [M+H]⁺.

### Example 111

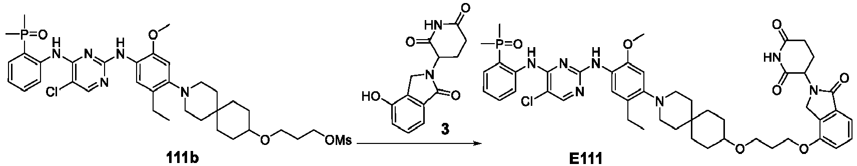

Compound 111b (20 mg, 0.03 mmol), compound 3 (7 mg, 0.03 mmol), NaI (6 mg, 0.03), KHCO₃ (6 mg, 0.06 mmol), and DMF (2 mL) were added in a reaction flask. The system was heated to 90 °C and stirred for 16 h. After the reaction was completed, the reaction solution was filtered and subjected to pre-HPLC to give title product E111 (white solid, 1.3 mg). ¹H NMR (500 MHz, CDCl₃) δ 8.39 (dd, *J* = 8.0, 4.2 Hz, 1H), 8.03 (d, *J* = 76.6 Hz, 2H), 7.49-7.42 (m, 2H), 7.33-7.27 (m, 1H), 7.21 (d, *J* = 7.1 Hz, 3H), 7.09-7.04 (m, 1H), 5.46-5.26 (m, 1H), 5.25-5.19 (m, 1H), 4.42 (d, *J* = 16.3 Hz, 1H), 4.30 (d, *J* = 16.4 Hz, 1H), 4.19 (t, *J* = 6.0 Hz, 2H), 3.89 (s, 3H), 3.66-3.56 (m, 2H), 3.41-3.23 (m, 2H), 2.96-2.53 (m, 6H), 2.42-2.32 (m, 1H), 2.30-2.16 (m, 2H), 2.10-1.96 (m, 3H), 1.86 (s, 3H), 1.83 (s, 3H), 1.34-1.23 (m, 10H), 1.07 (t, *J* = 7.1 Hz, 3H). MS m/z (ESI): 898.5 [M+H]⁺.

### Example 112

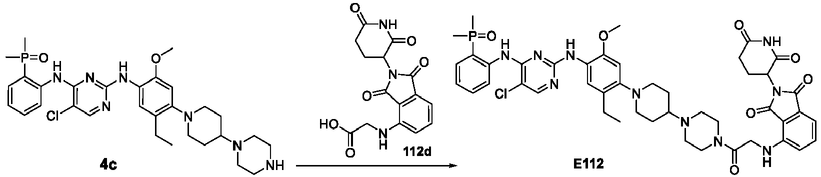

Compound E112 (white solid, 20 mg) was prepared with reference to the method for compound E88.

¹H NMR (500 MHz, CD₃OD) δ 8.13 (s, 1H), 8.11 (s, 1H), 7.72 (dd, *J* = 14.0, 9.0 Hz, 1H), 7.60 (t, *J* = 7.8 Hz, 2H), 7.43 (t, *J* = 6.9 Hz, 1H), 7.33 (s, 1H), 7.14 (d, *J* = 7.0 Hz, 1H), 7.06 (d, *J* = 8.4 Hz, 1H), 6.94 (s, 1H), 5.11 (dd, *J* = 12.5, 5.5 Hz, 1H), 4.42-4.22 (m, 3H), 3.88 (s, 3H), 3.80-3.66 (m, 3H), 3.56-3.48 (m, 1H), 3.43-3.35 (m, 2H), 3.32-3.20 (m, 4H), 3.02-2.92 (m, 2H), 2.92-2.85 (m, 1H), 2.82-2.72 (m, 2H), 2.71-2.57 (m, 2H), 2.34 (d, *J* = 10.4 Hz, 2H), 2.18-2.12 (m, 1H), 2.12-2.02 (m, 2H), 1.92 (s, 3H), 1.89 (s, 3H), 1.16 (t, *J* = 7.2 Hz, 3H). MS m/z (ESI): 911.5 [M+H]⁺.

### Example 113

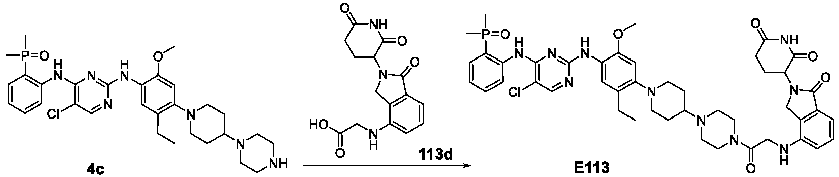

Compound E113 (white solid, 12 mg, hydrochloride) was prepared with reference to the method for compound E88.

¹H NMR (500 MHz, CD₃OD) δ 8.24 (d, *J* = 54.0 Hz, 1H), 8.15 (s, 1H), 7.73 (dd, *J* = 14.0, 7.7 Hz, 1H), 7.58 (s, 1H), 7.47-7.35 (m, 3H), 7.25 (d, *J* = 7.4 Hz, 1H), 7.05 (s, 1H), 6.97 (d, *J* = 8.0 Hz, 1H), 5.20 (dd, *J* = 13.3, 5.1 Hz, 1H), 4.51-4.21 (m, 5H), 3.91 (s, 3H), 3.81-3.65 (m, 3H), 3.65-3.55 (m, 1H), 3.37 (d, *J* = 30.7 Hz, 4H), 3.32-3.08 (m, 4H), 2.99-2.89 (m, 1H), 2.86-2.79 (m, 1H), 2.67 (dd, *J* = 14.5, 7.4 Hz, 2H), 2.60-2.50 (m, 1H), 2.38 (d, *J* = 10.5 Hz, 2H), 2.27-2.12 (m, 3H), 1.92 (s, 3H), 1.89 (s, 3H), 1.14 (t, *J* = 7.2 Hz, 3H). MS m/z (ESI): 897.4 [M+H]⁺.

### Example 114

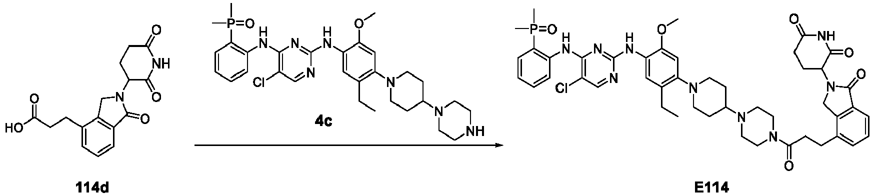

Compound E114 (white solid, 13 mg, hydrochloride) was prepared with reference to the method for compound E88.

¹H NMR (500 MHz, CD₃OD) δ 8.16 (s, 2H), 7.72 (dd, *J* = 13.4, 7.6 Hz, 2H), 7.58 (s, 3H), 7.48-7.40 (m, 2H), 7.12 (s, 1H), 5.26-5.04 (m, 1H), 4.79-4.39 (m, 4H), 4.20-4.00 (m, 1H), 3.91 (s, 3H), 3.73-3.33 (m, 8H), 3.14-3.04 (m, 3H), 3.01-2.88 (m, 3H), 2.85-2.75 (m, 2H), 2.67 (dd, *J* = 14.5, 7.1 Hz, 2H), 2.61-2.51 (m, 1H), 2.35-2.15 (m, 5H), 1.89 (s, 3H), 1.87 (s, 3H), 1.11 (t, *J* = 7.2 Hz, 3H). MS m/z (ESI): 896.6 [M+H]⁺.

### Example 115

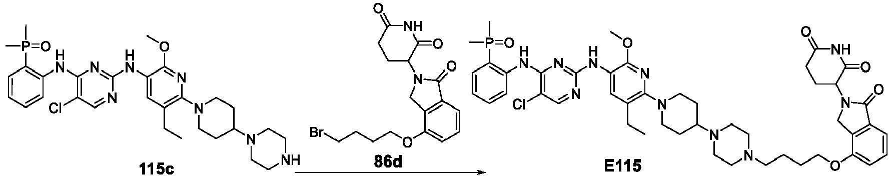

Title compound E115 (pale yellow solid, 40 mg, hydrochloride) was prepared with reference to the method for compound C-1.

¹H NMR (500 MHz, CD₃OD) δ 8.30 (s, 2H), 7.87 (dd, *J* = 14.0, 7.7 Hz, 1H), 7.82-7.64 (m, 3H), 7.62-7.54 (m, 2H), 7.40 (d, *J* = 8.0 Hz, 1H), 5.34 (dd, *J* = 13.4, 5.1 Hz, 1H), 4.71 (d, *J* = 17.4 Hz, 1H), 4.62 (d, *J* = 17.4 Hz, 1H), 4.40 (t, *J* = 5.5 Hz, 2H), 4.15-3.96 (m, 7H), 3.86-3.50 (m, 9H), 3.17 (t, *J* = 11.9 Hz, 2H), 3.12-3.03 (m, 1H), 3.00-2.92 (m, 1H), 2.80-2.69 (m, 3H), 2.44 (d, *J* = 10.6 Hz, 2H), 2.39-2.32 (m, 1H), 2.28-2.18 (m, 4H), 2.15-2.09 (m, 2H), 2.05 (d, *J* = 13.6 Hz, 6H), 1.29 (t, *J* =7.2 Hz, 3H). MS m/z (ESI): 914.1 [M+H]⁺.

### Example 116

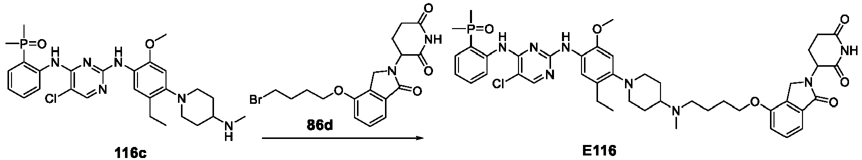

Title compound E116 (pale yellow solid, 15 mg, hydrochloride) was prepared with reference to the method for compound C-1.

¹H NMR (500 MHz, CD₃OD) δ 8.03 (s, 2H), 7.62 (dd, *J* = 14.0, 8.7 Hz, 1H), 7.42 (t, *J* = 7.8 Hz, 2H), 7.32 (t, *J* = 8.4 Hz, 2H), 7.21 (d, *J* = 28.4 Hz, 1H), 7.16 (d, *J* = 8.1 Hz, 1H), 6.87 (s, 1H), 5.08 (dd, *J* = 13.4, 5.1 Hz, 1H), 4.44-4.30 (m, 2H), 4.16 (t, *J* = 5.5 Hz, 2H), 3.78 (d, *J* = 1.9 Hz, 3H), 3.52-3.40 (m, 1H), 3.35-3.27 (m, 1H), 3.20-3.13 (m, 4H), 3.00-2.90 (m, 1H), 2.84 (s, 3H), 2.82-2.76 (m, 1H), 2.68-2.61 (m, 1H), 2.58-2.48 (m, 2H), 2.46-2.36 (m, 1H), 2.17-2.00 (m, 4H), 2.00-1.85 (m, 5H), 1.79 (d, *J* = 13.6 Hz, 6H), 1.02 (t, *J* = 7.2 Hz, 3H). MS m/z (ESI): 858.1 [M+H]⁺.

### Example 117

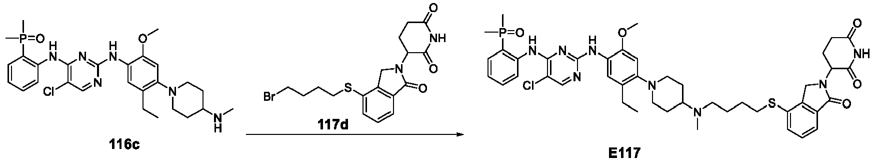

Title compound E117 (white solid, 25 mg, hydrochloride) was prepared with reference to the method for compound C-1.

¹H NMR (500 MHz, CD₃OD) δ 8.40-7.90 (m, 2H), 7.64-7.60 (m, 2H), 7.60-7.56 (m, 1H), 7.47 (t, *J* = 7.7 Hz, 2H), 7.32 (t, *J* = 7.2 Hz, 1H), 7.21 (s, 1H), 6.85 (s, 1H), 5.09 (ddd, *J* = 13.3, 5.1, 2.5 Hz, 1H), 4.38 (dt, *J* = 33.3, 9.5 Hz, 2H), 3.77 (s, 3H), 3.46-3.36 (m, 1H), 3.13-3.01 (m, 4H), 2.98-2.76 (m, 7H), 2.71-2.65 (m, 1H), 2.56-2.48 (m, 2H), 2.46-2.39 (m, 1H), 2.11-2.05 (m, 3H), 2.02-1.81 (m, 5H), 1.80 (s, 3H), 1.78 (s, 3H), 1.73-1.65 (m, 2H), 1.02 (t, *J* = 7.2 Hz, 3H). MS m/z (ESI): 874.1 [M+H]⁺.

### Example 118

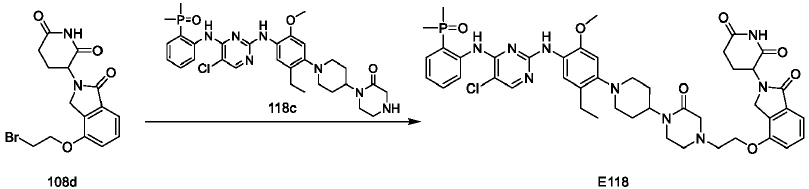

Compound E118 (pale yellow solid, 5 mg, hydrochloride) was prepared with reference to the method for compound C-1.

¹H NMR (500 MHz, CD₃OD) δ 8.24 (dd, *J* = 8.2, 4.3 Hz, 1H), 8.05 (s, 1H), 7.69 (s, 1H), 7.63 (ddd, *J* = 13.9, 7.7, 1.4 Hz, 1H), 7.50 (t, *J* = 7.9 Hz, 2H), 7.41 (d, *J* = 7.1 Hz, 1H), 7.29-7.22 (m, 2H), 6.79 (s, 1H), 5.15 (dd, *J* = 13.4, 5.2 Hz, 1H), 4.57 (br s, 1H), 4.52-4.42 (m, 2H), 4.32 (t, *J* = 5.1 Hz, 2H), 3.84 (s, 3H), 3.44-3.38 (m, 2H), 3.34 (s, 2H), 3.06 (d, *J* = 11.7 Hz, 2H), 2.96-2.74 (m, 8H), 2.58-2.48 (m, 3H), 2.20-2.16 (m, 1H), 2.02-1.94 (m, 2H), 1.85 (s, 3H), 1.82 (s, 3H), 1.70 (d, *J* = 11.9 Hz, 2H), 1.00 (t, *J* = 7.5 Hz, 3H). MS m/z (ESI): 899.5 [M+H]⁺.

### Example 119

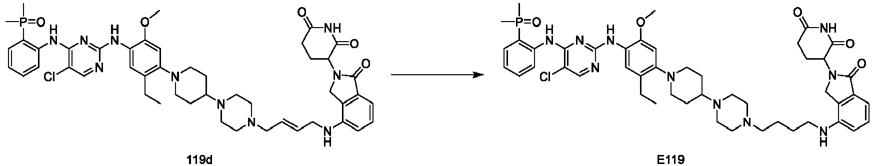

Compound 119d (60 mg, 0.065 mmol) was dissolved in methanol (80 mL). In a nitrogen atmosphere, Pd/C (2 g) and Pd(OH)₂/C (1 g) were added. The reaction system was purged with hydrogen and stirred at 45 °C for 48 h in the hydrogen atmosphere. After the reaction was completed, the reaction solution was filtered, and the filtrate was concentrated at reduced pressure and subjected to prep-HPLC to give compound E119 (pale yellow solid, 10 mg, hydrochloride). ¹H NMR (500 MHz, DMSO-*d*₆) δ 12.41 (br s, 1H), 12.11 (br s, 1H), 11.01 (s, 1H), 8.34 (br s, 2H), 7.66 (dd, *J* = 13.7, 8.4 Hz, 1H), 7.38-7.26 (m, 4H), 6.98 (d, *J* = 7.4 Hz, 1H), 6.88-6.79 (m, 2H), 5.12 (dd, *J* = 13.3, 5.1 Hz, 1H), 4.30 (d, *J* = 17.2 Hz, 1H), 4.17 (d, *J* = 17.2 Hz, 1H), 3.88-3.71 (m, 12H), 3.26-3.14 (m, 6H), 2.97-2.78 (m, 3H), 2.66-2.53 (m, 3H), 2.37-2.21 (m, 3H), 2.10-1.91 (m, 3H), 1.89-1.74 (m, 8H), 1.70-1.61 (m, 2H), 1.07 (t, *J* = 7.3 Hz, 3H). MS m/z (ESI): 912.5 [M+H]⁺.

### Example 120

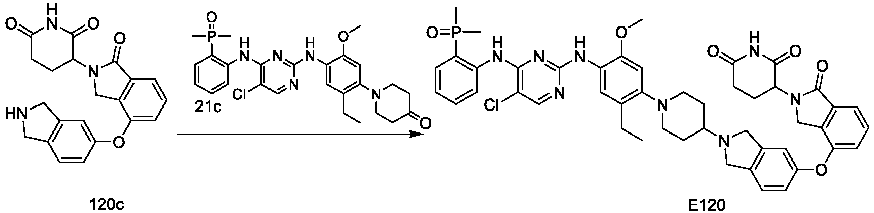

Title compound E120 (white solid, 8 mg, hydrochloride) was prepared with reference to the method for compound E67.

¹H NMR (500 MHz, CD₃OD) δ ppm 8.26 (br dd, *J* = 7.94, 4.19 Hz, 1H), 8.07 (s, 1H), 7.72 (s, 1H), 7.67-7.52 (m, 2H), 7.52 (br t, *J* = 7.75 Hz, 2H), 7.36 (d, *J* = 8.13 Hz, 1H), 7.28 (br t, *J* = 7.07 Hz, 1H), 7.12 (d, *J* = 7.75 Hz, 1H), 7.08- 6.99 (m, 2H), 6.80 (s, 1H), 5.15 (dd, *J* = 13.38, 5.13 Hz, 1H), 4.58 (br d, *J* = 1.25 Hz, 1H) 4.44 (d, *J* = 13.38 Hz, 2H), 4.26 (s, 3H), 3.85 (s, 3H), 3.10 (br d, *J* = 10.88 Hz, 2H), 2.96-2.72 (m, 6H), 2.58-2.45 (m, 3H), 2.24-2.11 (m, 3H), 1.91-1.76 (m, 7H), 1.01 (, t, *J* = 7.50 Hz, 3H). MS m/z (ESI): 889.6 [M+H]⁺.

### Example 121

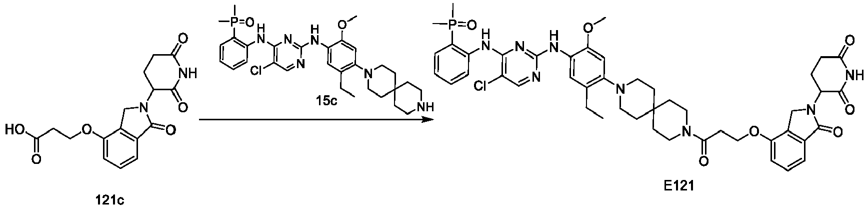

Title compound E121 (white solid, 5 mg, hydrochloride) was prepared with reference to the method for compound E88.

¹H NMR (400 MHz, DMSO-*d*₆) δ 11.99-11.62 (m, 1H), 10.43-10.04 (m, 1H), 8.42-8.21 (m, 2H), 7.64 (dd, *J* = 7.3, 11.7 Hz, 1H), 7.56-7.12 (m, 6H), 7.11-6.96 (m, 2H), 5.21-5.09 (m, 1H), 4.38-4.29 (m, 1H), 4.24-4.16 (m, 1H), 3.83 (s, 3H), 3.26-2.90 (m, 9H), 2.70-2.55 (m, 4H), 2.11-1.92 (m, 2H), 1.79 (d, *J* = 13.4 Hz, 9H), 1.64-1.40 (m, 6H), 1.38-1.20 (m, 2H), 1.05 (s, 3H);MS m/z (ESI): 897.6 [M+H]⁺

### Examples 122 and 123

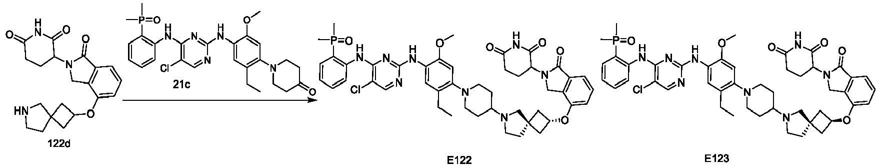

Title compounds E122 (white solid, 50 mg, hydrochloride) and E123 (white solid, 40 mg, hydrochloride) were prepared with reference to the method for compound E88.

¹H NMR (400 MHz, CD₃OD) δ 8.26 (m, 1H), 8.06 (s, 1H), 7.71 (d, *J* = 2.1 Hz, 1H), 7.65-7,62 (m, 1H), 7.51-7,47 (m, 2H), 7.41-7,39 (m, 1H), 7.28-7,26 (m, 1H), 7.09-7,07 (m, 1H), 6.76 (s, 1H), 5.16 (dd, *J* = 5.4, 12.9 Hz, 1H), 4.51-4,49 (m, 1H), 4.48-4.37 (m, 2H), 3.84 (s, 3H), 3.43-3.41 (m, 3H), 3.23-3.20 (m, 2H), 3.07-3.06 (m, 2H), 2.80-2.78 (m, 1H), 2.69-2.65 (m, 3H), 2.63-2.61 (m, 2H), 2.51- 2.49 (m, 2H), 2.47- 2.17 (m, 8H), 1.85-1.75 (m, 8H), 1.02-0.98 (m, 3H). MS m/z (ESI): 881.7 [M+H]⁺.

¹H NMR (400 MHz, CD₃OD) δ ppm 8.28 (dt, *J* = 5.82, 3.47 Hz, 1H) 8.09 (s, 1H) 7.75 (s, 1H), 7.72-7.62 (m, 1H), 7.58-7.45 (m, 2H), 7.42 (br d, *J* = 7.13 Hz, 1H), 7.30 (br t, *J* = 6.88 Hz, 1H), 7.08 (br d, *J* = 7.13 Hz, 1H), 6.79 (s, 1H), 5.21-5.13 (m, 1H) 4.60-4.38 (m, 3H), 3.87 (s, 3H), 3.24 (s, 4H) 3.17-3.01 (m, 4H), 2.95-2.80 (m, 1H), 2.84-2.70 (m, 5H) 2.60-2.45 (m, 3H) 2.40- 2.07 (m, 8H), 1.96-1.43 (m, 6H), 1.01-0.92 (m, 3H). MS m/z (ESI): 881.7 [M+H]⁺.

### Example 124

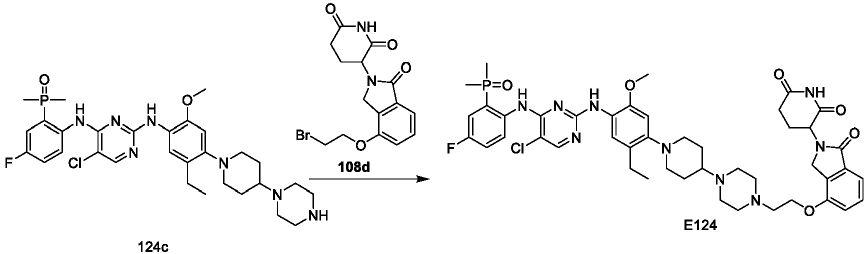

Title compound E124 (white solid, 9 mg, hydrochloride) was prepared with reference to the method for compound C-1.

¹H NMR (500 MHz, CD₃OD) δ 8.14 (s, 2H), 7.62-7.53 (m, 2H), 7.50 (d, *J* = 7.2 Hz, 1H), 7.35 (t, *J* = 12.8 Hz, 3H), 7.01 (s, 1H), 5.20 (dd, *J* = 13.4,5.2 Hz, 1H), 4.76-4.53 (m, 4H), 4.09-3.89 (m, 13H), 3.67 (t, *J* = 6.1 Hz, 1H), 3.38 (d, *J* = 12.5 Hz, 2H), 3.10 (s, 2H), 2.95 (ddd, *J* = 18.8, 13.6, 5.4 Hz, 1H), 2.82 (ddd, *J* = 17.6, 4.5, 2.4 Hz, 1H), 2.70-2.57 (m, 3H), 2.41 (d, *J* = 10.5 Hz, 2H), 2.22 (ddd, *J* = 19.1, 9.5, 7.0 Hz, 3H), 1.90 (d, *J* = 13.6 Hz, 6H), 1.14 (s, 3H). MS m/z (ESI): 903.0 [M+H]⁺.

### Example 125

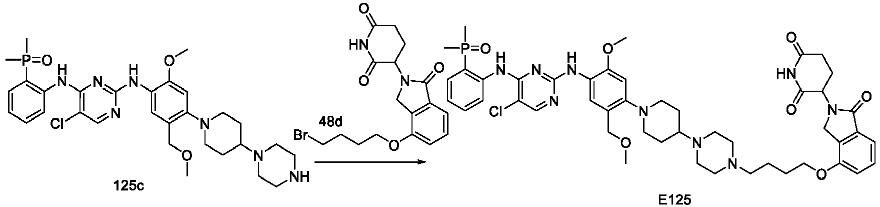

Title compound E125 (white solid, 1.5 mg, hydrochloride) was prepared with reference to the method for compound C-1.

¹H NMR (500 MHz, CD₃OD) δ 8.14-8.07(m, 2H), 7.63 (dd, *J* = 10.0, 5.0 Hz, 1H), 7.51-7.48 (m, 1H), 7.42-7.31 (m, 3H), 7.30 (d, *J* = 10.0 Hz, 1H), 7.14 (d, *J* = 10.0 Hz, 1H), 7.04 (s, 1H), 5.07 (dd, *J* = 10.0, 5.0 Hz, 1H), 4.42 (q, *J* = 15.0 Hz, 2H), 4.35 (s, 2H), 4.14 (t, *J* = 5.0 Hz, 2H), 3.94-3.62 (m, 13H), 3.48-3.46 (m, 2H), 3.34 (s, 3H), 3.31-3.30 (m, 1H), 3.21-3.19 (m, 2H), 2.86-2.79 (m, 1H), 2.72-2.67 (m, 1H), 2.55-2.46(m, 1H), 2.35-2.33(m, 2H), 2.18-1.97 (m, 5H), 1.89-1.82(m, 2H), 1.80 (s, 3H), 1.76(s, 3H). MS m/z (ESI): 929.0 [M+H]⁺.

### Example 126

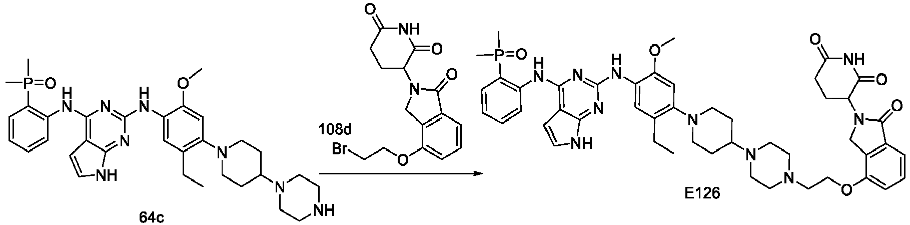

Title compound E126 (white solid, 10 mg, hydrochloride) was prepared with reference to the method for compound C-1.

¹H NMR (500 MHz, CD₃OD) δ 8.28-8.16 (m, 1H), 7.69-7.61 (m, 2H), 7.52-7.42 (m, 2H), 7.38 (d, *J* = 10 Hz, 1H), 7.36-7.30 (m, 1H), 7.21 (d, *J* = 10.0 Hz, 1H), 6.96-6.54 (m, 2H), 6.55-6.42(m, 1H), 5.08 (dd, *J* = 10.0, 5.0 Hz, 1H), 4.54 (q, *J* = 15.0 Hz, 2H), 4.53 (t, *J* = 5.0 Hz, 2H), 3.95-3.73 (m, 10H), 3.63-3.57 (m, 2H), 3.41-3.31 (m, 2H), 3.16-3.08 (m, 2H), 2.87-2.80 (m, 1H), 2.74-2.63 (m, 2H), 2.62-2.45 (m, 3H), 2.33-2.25 (m, 2H), 2.18-2.09 (m, 3H), 1.97-1.91 (m, 1H), 1.92 (s, 3H), 1.88 (s, 3H), 1.09-1.06 (m, 3H). MS m/z (ESI): 889.6 [M+H]⁺.

### Example 127

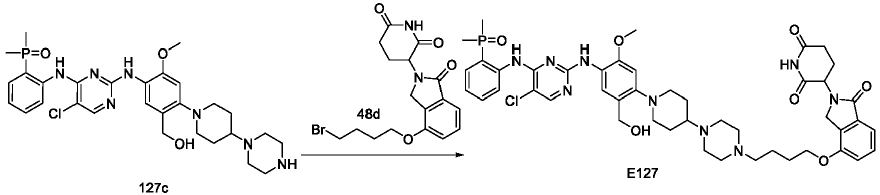

Title compound E127 (white solid, 5 mg, hydrochloride) was prepared with reference to the method for compound C-1.

¹H NMR (500 MHz, CD₃OD) δ 8.25-8.23(m, 2H), 7.77 (dd, *J* = 10.0, 5.0 Hz, 1H), 7.66 (t, *J* = 10.0 Hz, 1H), 7.56-7.49 (m, 3H), 7.42 (d, *J* = 10.0 Hz, 1H), 7.33 (s, 1H), 7.25 (d, *J* = 10.0 Hz, 1H), 5.19 (dd, *J* = 10.0, 5.0 Hz, 1H), 4.72 (s, 2H), 4.53 (q, *J* = 15.0 Hz, 2H), 4.25 (t, *J* = 5.0 Hz, 2H), 4.04-3.65 (m, 14H), 3.63-3.50 (m, 2H), 3.44-3.41 (m, 2H), 2.98-2.79 (m, 1H), 2.84-2.79 (m, 1H), 2.66-2.52 (m, 3H), 2.34-2.19 (m, 3H), 2.14-2.08 (m, 2H), 2.01-1.96 (m, 2H), 1.90 (s, 3H), 1.88 (s, 3H). MS m/z (ESI): 915.0 [M+H]⁺.

### Example 128

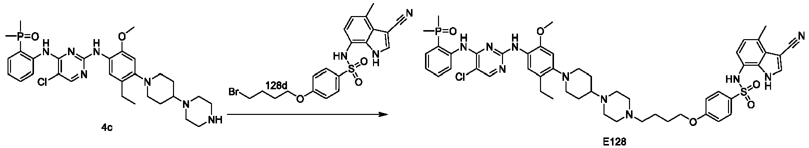

Compound E128 (pale yellow solid, 30 mg, hydrochloride) was prepared with reference to the method for compound C-1.

¹H NMR (500 MHz, DMSO-*d*₆) δ 12.37 (s, 1H), 12.24-11.97 (m, 1H), 11.78 (br s, 1H), 10.64 (s, 1H), 8.44-8.19 (m, 3H), 7.63 (s, 1H), 7.41 (d, *J* = 8.8 Hz, 2H), 7.37 (s, 2H), 7.23 (s, 1H), 6.92 (d, *J* = 8.7 Hz, 2H), 6.87 (d, *J* = 8.2 Hz, 1H), 6.82 (s, 1H), 6.39 (d, *J* = 7.8 Hz, 1H), 3.91-3.65 (m, 12H), 3.21-3.09 (m, 4H), 3.07-2.96 (m, 2H), 2.84-2.75 (m, 2H), 2.64 (s, 3H), 2.56 (d, *J* = 7.4 Hz, 2H), 2.21 (d, *J* = 9.5 Hz, 2H), 1.97-1.86 (m, 2H), 1.83-1.75 (m, 8H), 1.34-1.24 (m, 2H), 1.07 (t, *J* = 7.4 Hz, 3H). MS m/z (ESI): 979.8 [M+H]⁺.

### Example 129

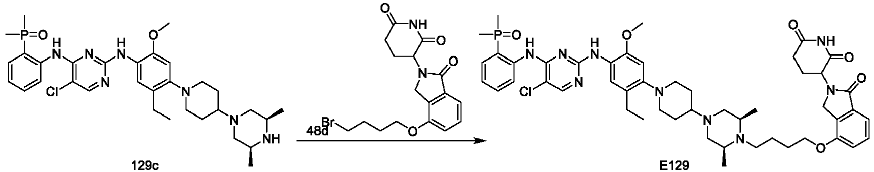

Compound E129 (pale yellow solid, 5 mg, hydrochloride) was prepared with reference to the method for compound C-1.

¹H NMR (500 MHz, CD₃OD) δ 8.46 (br s, 1H), 8.33 (br s, 1H), 7.94-7.87 (m, 1H), 7.78-7.66 (m, 2H), 7.64-7.59 (m, 2H), 7.57 (s, 1H), 7.46 (d, *J* = 8.0 Hz, 1H), 7.24 (s, 1H), 5.36 (dd, *J* = 13.3, 5.2 Hz, 1H), 4.72 (dd, *J* = 38.8, 17.5 Hz, 2H), 4.52-4.36 (m, 4H), 4.15 (t, *J* = 11.8 Hz, 2H), 4.08 (s, 3H), 3.87-3.78 (m, 3H), 3.73 (s, 2H), 3.65-3.57 (m, 2H), 3.41-3.25 (m, 2H), 3.19-3.05 (m, 1H), 3.02-2.95 (m, 1H), 2.92-2.79 (m, 3H), 2.62 (d, *J* = 10.5 Hz, 2H), 2.49-2.35 (m, 3H), 2.26-2.15 (m, 4H), 2.09 (s, 3H), 2.06 (s, 3H), 1.73 (dd, *J* = 11.2, 6.5 Hz, 6H), 1.31 (br s, 3H). MS m/z (ESI): 941.5 [M+H]⁺.

### Example 130

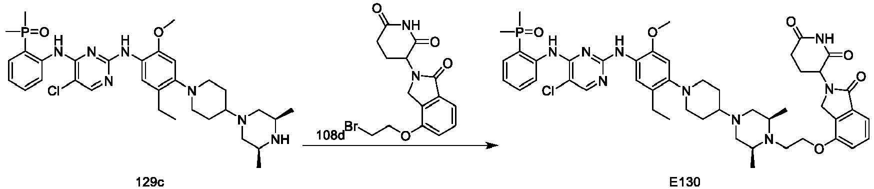

Compound E130 (pale yellow solid, 10 mg, hydrochloride) was prepared with reference to the method for compound C-1.

¹H NMR (500 MHz, CD₃OD) δ 8.26 (br s, 1H), 8.14 (br s, 1H), 7.77-7.67 (m, 1H), 7.55 (t, *J* = 7.7 Hz, 2H), 7.48 (d, *J* = 7.6 Hz, 1H), 7.45-7.30 (m, 3H), 6.98 (s, 1H), 5.15 (dd, *J* = 13.3, 5.1 Hz, 1H), 4.69 (d, *J* = 17.0 Hz, 1H), 4.60 (s, 2H), 4.52 (d, *J* = 17.3 Hz, 1H), 4.35 (d, *J* = 29.5 Hz, 2H), 4.11-3.94 (m, 4H), 3.88 (d, *J* = 12.9 Hz, 3H), 3.77-3.57 (m, 3H), 3.54-3.34 (m, 2H), 3.25-3.01 (m, 2H), 2.97-2.85 (m, 1H), 2.78 (d, *J* = 17.7 Hz, 1H), 2.69-2.52 (m, 3H), 2.50-2.12 (m, 5H), 1.89 (s, 3H), 1.86 (s, 3H), 1.73-1.62 (m, 6H), 1.10 (br s, 3H); MS m/z (ESI): 913.5 [M+H]⁺.

### Example 131

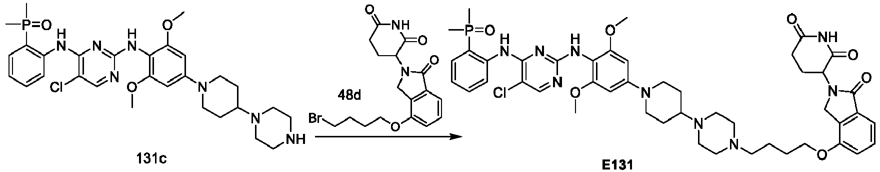

Title compound E131 (6.99 mg, formate) was prepared with reference to the method for compound C-1.

¹H NMR (400 MHz, CD₃OD) δ 8.48 (s, 1H), 7.92 (s, 1H), 7.72 (d, *J* = 8.0, 1H), 7.39-7.27 (m, 2H), 7.23-7.21 (m, 2H), 7.10-7.08 (m, 1H), 6.31 (s, 2H), 5.18-5.15 (m, 1H), 4.21-4.19 (m, 2H), 3.85-3.83 (m, 2H), 3.31 (s, 6H), 2.90-2.77 (m, 12H), 2.68-2.66(m, 4 H), 2.48-2.42 (m, 1 H), 2.36-2.32 (m, 1 H), 2.16-2.10 (m, 2 H), 1.89-1.85 (m, 11 H), 1.75-1.72 (m, 2 H). MS m/z (ESI): 914.7 [M+H]⁺.

### Example 132

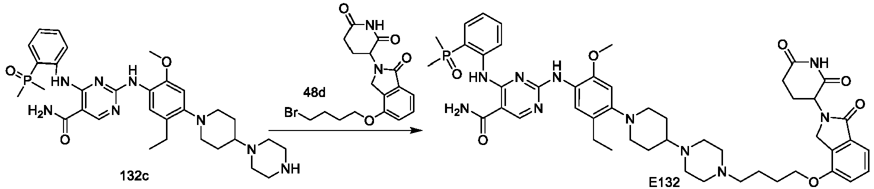

Title compound E132 (white solid, 19.31 mg, formate) was prepared with reference to the method for compound C-1.

¹H NMR (400 MHz, DMSO-*d*6) δ 12.46-11.92 (m, 1H), 11.84-11.64 (m, 1H), 11.06-10.84 (m, 1H), 9.76-9.38 (m, 1H), 8.95-8.64 (m, 1H), 8.47-8.21 (m, 1H), 7.81 (br dd, *J* = 7.8, 11.8 Hz, 2H), 7.68-7.59 (m, 1H), 7.59-7.57 (m, 1H), 7.55-7.21 (m, 4H), 6.77 (s, 1H), 5.12 (dd, *J* = 5.0, 13.1 Hz, 1H), 4.47 (d, *J* = 17.4 Hz, 1H), 4.27 (d, *J* = 17.5 Hz, 1H), 4.19-4.10 (m, 2H), 3.80 (s, 7H), 3.25 (br s, 3H), 3.15-3.04 (m, 3H), 2.99-2.86 (m, 4H), 2.81-2.66 (m, 3H), 2.59 (br d, *J* = 18.1 Hz, 1H), 2.24-2.11 (m, 3H), 2.05-1.98 (m, 1H), 1.98-1.87 (m, 4H), 1.85-1.76 (m, 2H), 1.68 (br d, *J* = 13.3 Hz, 6H), 1.31-1.21 (m, 1H), 0.99-0.73 (m, 3H);MS m/z (ESI): 921.4 [M+H]⁺

### Example 133

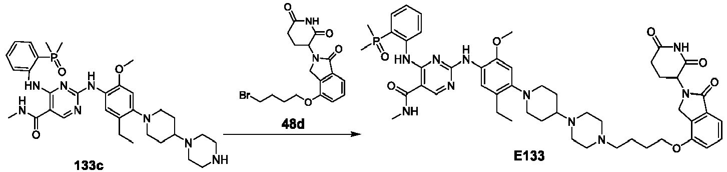

Title compound E133 (4.7 mg, hydrochloride) was prepared with reference to the method for compound C-1. ¹H NMR (400 MHz, DMSO-*d*₆) δ 12.60-11.37 (m, 3H), 10.98 (s, 1H), 9.25 (s, 1H), 9.12-8.84 (m, 1H), 8.77-8.57 (m, 1H), 7.84-7.76 (m, 1H), 7.67 (d, *J* = 3.0 Hz, 1H), 7.58-7.47 (m, 2H), 7.43 (t, *J* = 7.4 Hz, 1H), 7.35-7.29 (m, 2H), 7.26 (d, *J* = 8.1 Hz, 1H), 5.16-5.07 (m, 1H), 4.47 (d, *J* = 17.5 Hz, 1H), 4.27 (d, *J* = 17.5 Hz, 1H), 4.17 (d, *J* = 3.5 Hz, 2H), 3.79 (s, 6H), 3.31-3.18 (m, 6H), 3.14-3.02 (m, 3H), 2.99-2.86 (m, 2H), 2.78 (d, *J* = 4.4 Hz, 3H), 2.75-2.69 (m, 2H), 2.61 (s, 1H), 2.60-2.53 (m, 1H), 2.39-2.31 (m, 2H), 2.24-2.12 (m, 2H), 2.06-1.98 (m, 1H), 1.95-1.87 (m, 3H), 1.84 (d, *J* = 6.0 Hz, 3H), 1.69 (d, *J* = 13.4 Hz, 6H), 1.04-0.75 (m, 3H). MS m/z (ESI): 935.4 [M+H]⁺.

### Example 134

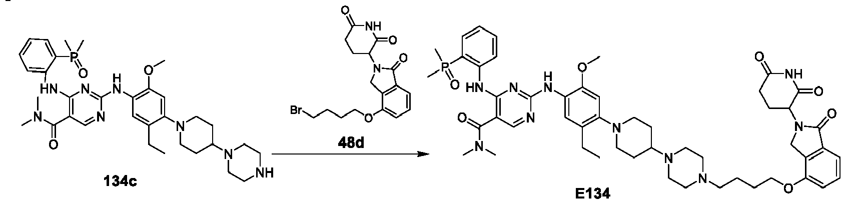

Title compound E134 (white solid, 28.5 mg, hydrochloride) was prepared with reference to the method for compound C-1.

¹H NMR (400 MHz, CD₃OD) δ 8.13-7.99 (m, 1H), 7.74 (dd, *J* = 7.9, 13.3 Hz, 1H), 7.66-7.33 (m, 5H), 7.24 (d, *J* = 8.0 Hz, 1H), 7.04 (s, 1H), 5.17 (dd, *J* = 5.1, 13.3 Hz, 1H), 4.58-4.42 (m, 2H), 4.23 (t, *J* = 5.6 Hz, 2H), 3.97-3.66 (m, 12H), 3.45-3.37 (m, 4H), 3.15 (s, 6H), 2.85-2.74 (m, 1H), 3.00-2.73 (m, 1H), 2.66-2.51 (m, 3H), 2.41 (d, *J* = 8.9 Hz, 2H), 2.29-2.15 (m, 3H), 2.12-2.05 (m, 2H), 2.01-1.91 (m, 2H), 1.84 (d, *J* = 13.6 Hz, 6H), 1.40-1.33 (m, 3H), 1.09 (s, 3H). MS m/z (ESI): 949.8 [M+H]⁺.

### Example 135

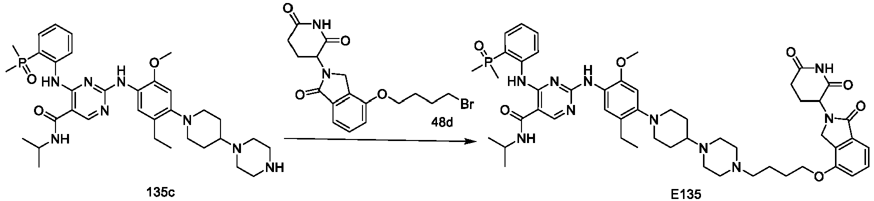

Compound E135 (white solid, 35.22 mg, hydrochloride) was prepared with reference to the method for reference compound C-1.

¹H NMR (400 MHz, CD₃OD) δ 8.51 (br s, 1H), 7.87 (dd, *J* = 7.8, 13.1 Hz, 1H), 7.66 (d, *J* = 4.0 Hz, 2H), 7.57-7.46 (m, 2H), 7.34-7.22 (m, 3H), 6.89 (s, 1H), 5.19-5.14 (m, 1H), 4.59-4.42 (m, 2H), 4.29-4.17 (m, 3H), 4.09-3.51 (m, 12H), 3.44-3.35 (m, 2H), 3.25 (d, *J* = 9.9 Hz, 2H), 3.00-2.86 (m, 3H), 2.85-2.74 (m, 1H), 2.66-2.41 (m, 2H), 2.33 (d, *J* = 11.4 Hz, 3H), 2.25-2. 15 (m, 1H), 2.08 (d, *J* = 9.3 Hz, 4H), 2.01-1.90 (m, 2H), 1.84 (d, *J* = 13.4 Hz, 6H), 1.27 (s, 3H), 1.25 (s, 3H), 1.00 (br s, 3H). MS m/z (ESI): 964.0 [M+H]⁺.

### Example 136

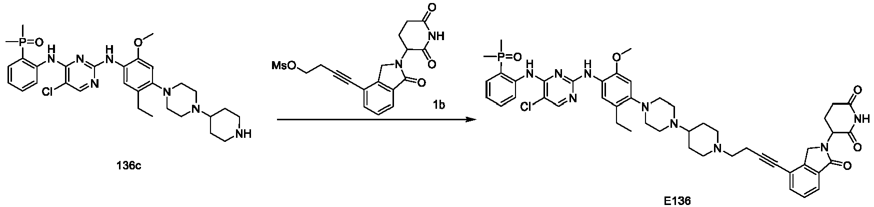

Compound E136 (white solid, 26 mg, hydrochloride) was prepared with reference to the method for compound E4.

¹H NMR (500 MHz, DMSO-*d*₆) δ 11.42-11.01 (m, 2H), 8.61 (s, 1H), 8.39 (s, 1H), 8.18 (s, 1H), 7.77 (d, *J* = 7.6 Hz, 1H), 7.70 (d, *J* = 7.1 Hz, 1H), 7.58 (dt, *J* = 15.1, 7.7 Hz, 2H), 7.47 (s, 1H), 7.35 (s, 1H), 7.16 (t, *J* = 7.1 Hz, 1H), 6.76 (s, 1H), 5.18 (dd, *J* = 13.3, 5.1 Hz, 1H), 4.52 (d, *J* = 17.7 Hz, 1H), 4.35 (d, *J* = 17.7 Hz, 1H), 3.82-3.72 (m, 5H), 3.65-3.47 (m, 4H), 3.29-3.00 (m, 11H), 2.98-2.91 (m, 1H), 2.59-2.55 (m, 3H), 2.48-2.41 m, 3H), 2.22 (d, *J* = 11.6 Hz, 2H), 2.10-1.99 (m, 1H), 1.79 (s, 3H), 1.76 (s, 3H), 1.07 (t, *J* = 7.5 Hz, 3H). MS m/z (ESI): 447 [M/2+H]⁺.

### Example 137

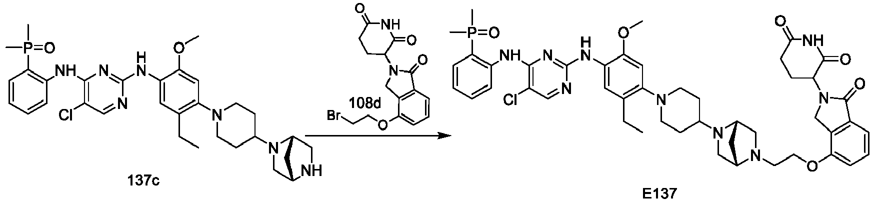

Compound E137 (white solid, 35 mg, hydrochloride) was prepared with reference to the method for compound C-1.

¹H NMR (500 MHz, CD₃OD) δ 8.17 (s, 2H), 7.72 (dd, *J* = 13.9, 7.7 Hz, 1H), 7.61-7.50 (m, 2H), 7.49-7.41 (m, 3H), 7.30 (d, *J* = 8.1 Hz, 1H), 7.18 (s, 1H), 5.19-5.15 (m, 1H), 4.99-4.93 (m, 2H), 4.75-4.36 (m, 6H), 4.28 (t, *J* = 10.0 Hz, 1H), 4.13-3.85 (m, 7H), 3.63-3.36 (m, 4H), 2.98-2.86 (m, 1H), 2.81-2.56 (m, 6H), 2.50-2.30 (m, 4H), 2.23-2.14 (m, 1H), 1.89 (s, 3H), 1.86 (s, 3H), 1.10 (br s, 3H). MS m/z (ESI): 897.1 [M+H]⁺.

### Example 138

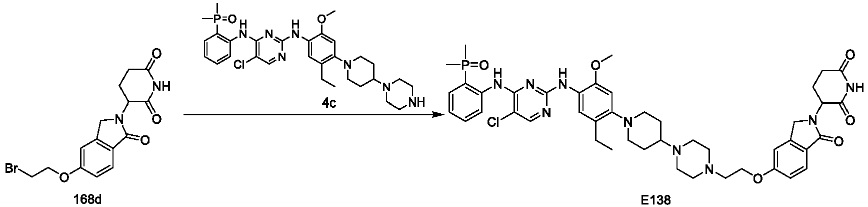

Compound E138 (white solid, 5 mg, hydrochloride) was prepared with reference to the method for reference compound C-1.

¹H NMR (500 MHz, CD₃OD) δ 8.29 (br s, 1H), 8.19 (br s, 1H), 7.76 (d, *J* = 8.5 Hz, 1H), 7.73-7.66 (m, 1H), 7.53 (s, 1H), 7.40 (t, *J* = 7.1 Hz, 1H), 7.29 (t, *J* = 6.4 Hz, 2H), 7.21 (dd, *J* = 8.5, 2.2 Hz, 1H), 6.94 (s, 1H), 5.12 (dd, *J* = 13.4, 5.2 Hz, 1H), 4.62-4.54 (m, 2H), 4.46 (t, *J* = 14.1 Hz, 2H), 4.09-3.66 (m, 13H), 3.59 (s, 1H), 3.34-3.31 (m, 2H), 3.03-2.87 (m, 3H), 2.80-2.75 (m, 1H), 2.65-2.57 (m, 2H), 2.53-2.44 (m, 1H), 2.35 (d, *J* = 11.3 Hz, 2H), 2.21-2.03 (m, 3H), 1.89 (s, 3H), 1.86 (s, 3H), 1.12 (br s, 3H). MS m/z (ESI): 884.8 [M+H]⁺.

### Example 139

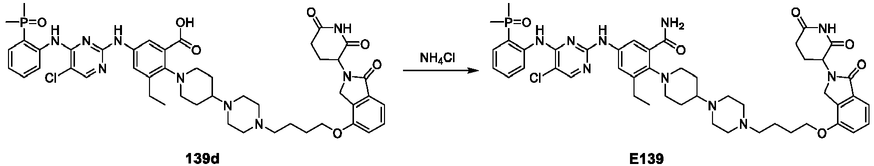

Compound 139d (30 mg, 0.03 mmol), NH₄Cl (5.20 mg, 0.09 mmol), HATU (18.47 mg, 0.05 mmol), and DIPEA (12.56 mg, 0.10 mmol) were added to DMF (1 mL). The reaction system was stirred at 25 °C for 1 h. The reaction mixture was filtered and concentrated at reduced pressure to give a crude product. The crude product was isolated by reverse-phase pre-HPLC (formic acid system) to give title compound E139. ¹H NMR (400 MHz, DMSO-*d*₆) δ 11.17 (s, 1H), 11.05-10.91 (m, 1H), 9.40-9.29 (m, 1H), 8.67-8.55 (m, 1H), 8.31 (s, 2H), 8.22-8.16 (m, 1H), 7.79-7.66 (m, 1H), 7.64-7.51 (m, 2H), 7.51-7.37 (m, 3H), 7.34-7.22 (m, 3H), 7.17 (t, *J* = 7.1 Hz, 1H), 5.16-5.06 (m, 1H), 4.43-4.33 (m, 1H), 4.27-4.20 (m, 1H), 4.14 (t, *J* = 6.2 Hz, 2H), 3.14-3.02 (m, 3H), 2.95-2.82 (m, 3H), 2.64-2.55 (m, 4H), 2.40 (td, *J* = 4.2, 6.7 Hz, 3H), 2.35-2.27 (m, 4H), 2.15 (s, 1H), 2.04-1.96 (m, 1H), 1.84-1.71 (m, 12H), 1.63-1.56 (m, 2H), 1.52-1.40 (m, 2H), 1.14-1.07 (m, 3H). MS m/z (ESI): 925.5 [M+H]⁺.

### Example 140

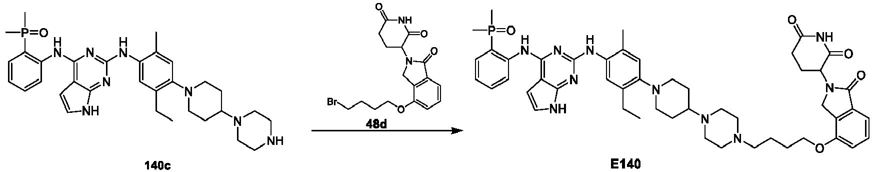

Title compound E140 (white solid, 100 mg, hydrochloride) was prepared with reference to the synthesis method for compound C-1.

¹H NMR (400 MHz, DMSO-*d*₆) δ 12.86-12.02 (m, 2 H), 10.99 (s, 1 H), 9.95-9.70 (m, 1 H), 8.37 (s, 1 H), 7.65 (dd, *J* = 13.51, 7.52 Hz, 1 H), 7.54-7.44 (m, 1 H), 7.32 (d, *J* =4.89 Hz, 2 H), 7.29-7.15 (m, 3 H), 7.07 (d, *J* = 14.43 Hz, 2 H), 6.46 (s, 1 H), 5.12 (dd, *J* = 13.02, 4.95 Hz, 1 H), 4.47 (d, *J* =17.48 Hz, 1 H), 4.27 (d, *J* = 17.36 Hz, 2 H), 4.16 (d, *J* = 3.55 Hz, 3 H), 3.82 (d, *J* = 11.00 Hz, 2 H), 3.48-3.14 (m, 8 H), 2.99-2.73 (m, 4 H), 2.72-2.54 (m, 4 H), 2.21 (s, 5 H) 2.06-1.89 (m, 5 H), 1.83 (d, *J* =13.69 Hz, 8 H), 1.15 (t, *J* = 7.40 Hz, 3 H). MS m/z (ESI): 901.4 [M+H]⁺.

### Example 141

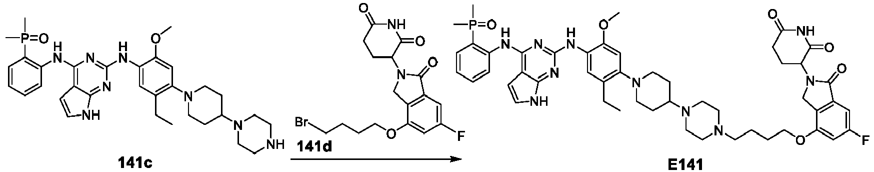

Title compound E141 (white solid, 95 mg, hydrochloride) was prepared with reference to the synthesis method for compound C-1.

¹H NMR (400 MHz, DMSO-*d*₆) δ 12.28 (s, 1 H), 11.82 (s, 1 H), 11.01 (s, 1 H), 8.50 (s, 1 H), 7.73-7.49 (m, 2 H), 7.40 (s, 1 H), 7.25-7.18(m, 2 H), 7.13-7.03 (m, 2 H), 6.86 (s, 1 H), 6.47 (s, 1 H), 5.12 (dd, *J* = 13.33, 4.77 Hz, 1 H), 4.48-4.40 (m, 1 H), 4.31-4.21 (m, 1 H), 4.18 (s, 2 H), 4.28-4.13 (m, 1 H), 3.81 (s, 7 H), 3.51 (s, 1 H), 3.19 (d, *J* = 11.37 Hz, 7 H), 2.96-2.75(m, 4 H), 2.69-2.58 (m, 4 H), 2.33 (s, 1 H), 2.21 (s, 1 H), 2.05-1.88 (m, 5 H), 1.83 (d, *J* = 13.57 Hz, 8 H), 1.13 (t, *J* = 7.34 Hz, 3 H). MS m/z (ESI): 935.7 [M+H]⁺.

### Example 142

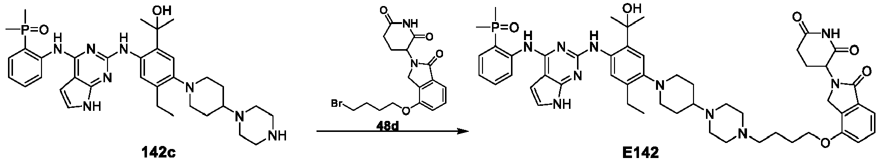

Title compound E142 (white solid, 21 mg, formate) was prepared with reference to the synthesis method for compound C-1.

¹H NMR (400 MHz, DMSO-*d*₆) δ 11.56 (s, 1 H), 11.18 (s, 1 H), 11.01 (s, 1 H), 9.39 (s, 1 H), 8.90 (dd, *J* = 8.3, 4.5 Hz, 1 H), 8.23 (s, 1 H), 8.08 (s, 1 H), 7.60-7.46 (m, 1 H), 7.49 (t, *J* = 7.8 Hz, 1 H), 7.41 (t, *J* = 7.8 Hz, 1 H), 7.31 (d, *J* = 7.5 Hz, 1 H), 7.25 (d, *J* = 8.1 Hz, 1 H), 7.06 (t, *J* = 7.3 Hz, 1 H), 6.97 (s, 1 H), 6.95-6.90 (m, 1 H), 6.35 (s, 1 H), 5.79 (s, 1 H), 5.11 (dd, *J* = 13.6, 5.2 Hz, 1 H), 4.42-4.35 (m, 1 H), 4.22 (d, *J* = 17.9 Hz, 1 H), 4.14 (t, *J* = 5.9 Hz, 2 H), 3.06-2.83 (m, 4 H), 2.62 (d, *J* = 7.9 Hz, 2 H), 2.66 (d, *J* = 8.7 Hz, 2 H), 2.57-2.54 (m, 1 H), 2.44-2.16 (m, 9 H), 2.18 (d, *J* = 15.4 Hz, 2 H), 2.03-1.97 (m, 1 H), 1.86-1.72 (m, 10 H), 1.62-1.51 (m, 9 H), 1.52-1.49 (m, 1 H), 1.36 (d, *J* = 13.7 Hz, 1H), 1.14 (t, *J* = 7.5 Hz, 3 H). MS m/z (ESI): 945.7 [M+H]⁺.

### Example 143

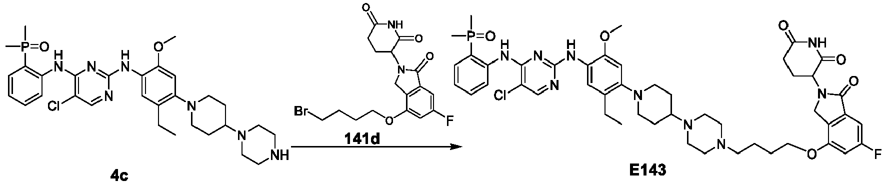

Title compound E143 (21.3 mg, formate) was prepared with reference to the synthesis method for compound C-1.

¹H NMR (400 MHz, DMSO-*d6*) δ 11.15 (s, 1 H), 11.02 (s, 1 H), 8.51-8.36 (m, 1 H), 8.24 (s, 1 H), 8.14 (s, 1 H), 8.10 (s, 1 H), 7.60-7.51 (m, 1 H), 7.42 (s, 1 H), 7.35-7.28 (m, 1 H), 7.25-7.18 (m, 1 H), 7.11 (s, 2 H), 6.77 (s, 1 H), 5.18-5.06 (m, 1 H), 4.34 (s, 1 H), 4.26-4.11 (m, 3 H), 3.75 (s, 3 H), 3.06-2.86 (m, 4 H), 2.76-2.64 (m, 3 H), 2.45-2.21 (m, 12 H), 2.05-1.93 (m, 1 H), 1.90-1.82 (m, 2 H), 1.77 (d, *J*=13.6 Hz, 9 H), 1.63-1.49 (m, 5 H), 1.06 (s, 3 H). MS m/z (ESI): 930.6 [M+H]⁺.

### Example 144

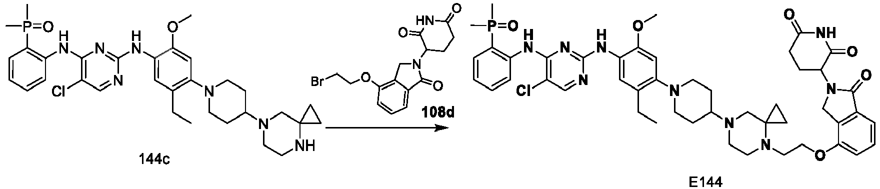

Title compound E144 (white solid, 10 mg, hydrochloride) was prepared with reference to the method for compound C-1.

¹H NMR (500 MHz, CD₃OD) δ 8.02 (s, 2H), 7.62 (dd, *J* = 13.9, 7.7 Hz, 1H), 7.44 (t, *J* = 7.8 Hz, 2H), 7.37-7.30 (m, 2H), 7.26 (s, 1H), 7.18 (d, *J* = 8.0 Hz, 1H), 6.93 (s, 1H), 5.08 (dd, *J* = 13.3, 5.2 Hz, 1H), 4.40 (dd, *J* = 28.5, 19.3 Hz, 4H), 3.87-3.70 (m, 9H), 3.59 (d, *J* = 23.2 Hz, 2H), 3.28 (s, 2H), 3.12-2.98 (m, 2H), 2.87-2.75 (m, 2H), 2.72-2.63 (m, 1H), 2.59-2.45 (m, 3H), 2.32 (d, *J* = 9.4 Hz, 2H), 2.16-2.02 (m, 3H), 1.79 (d, *J* = 13.6 Hz, 6H), 1.31 (s, 2H), 1.11 (s, 2H), 1.02 (s, 3H). MS m/z (ESI): 910.8 [M+H]⁺.

### Example 145

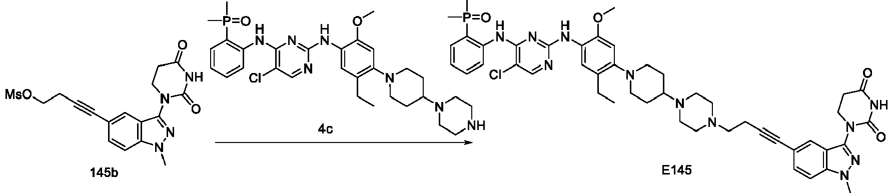

Title compound E145 (white solid, 6 mg, hydrochloride) was prepared with reference to the method for compound E4.

¹H NMR (500 MHz, CD₃OD) δ 8.32-8.14 (m, 2H), 7.91(s, 1H), 7.72 (dd, *J* = 10.0, 5.0 Hz, 1H), 7.58(s, 1H), 7.52 (t, *J* = 5.0 Hz, 1H), 7.44 (t, *J* = 5.0 Hz, 1H), 7.36(s, 1H), 7.02(s, 1H), 4.08 (t, *J* = 7.5 Hz, 2H), 4.04 (s, 3H), 3.98-3.81 (m, 11H), 3.69-3.64 (m, 3H), 3.40-3.37 (m, 2H), 3.14-3.08 (m, 4H), 2.90 (t, *J* = 7.5 Hz, 2H), 2.67-2.63(m, 2H), 2.42-2.40(m, 2H), 2.23-2.16(m, 2H), 1.92(s, 3H), 1.90(s, 3H), 1.14-1.11(m, 3H). MS m/z (ESI): 893.0 [M+H]⁺.

### Example 146

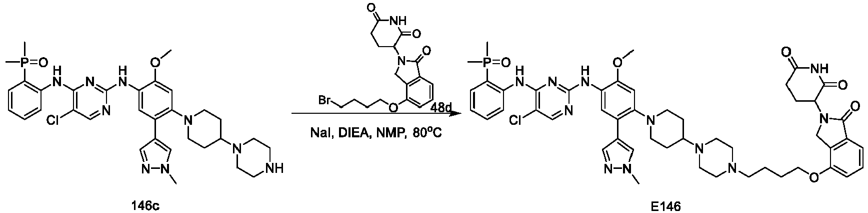

Compound E146 (pale yellow solid, 1.2 mg, hydrochloride) was prepared with reference to the method for compound C-1.

¹H NMR (500 MHz, CD₃OD) δ 8.39-8.00 (m, 3H), 7.85 (s, 1H), 7.70-7.59 (m, 1H), 7.56-7.46 (m, 2H), 7.39 (dd, *J* = 7.4, 4.0 Hz, 1H), 7.35-7.13 (m, 3H), 6.91 (s, 1H), 5.17 (dd, *J* = 13.2, 5.1 Hz, 1H), 4.56-4.44 (m, 2H), 4.23 (t, *J* = 5.9 Hz, 2H), 4.09-3.82 (m, 10H), 3.79-3.61 (m, 4H), 3.52 (s, 1H), 3.42-3.37 (m, 2H), 3.34 (s, 2H), 2.95-2.87 (m, 1H), 2.85-2.73 (m, 3H), 2.64-2.57 (m, 1H), 2.28 (d, *J* = 12.8 Hz, 2H), 2.22-2.15 (m, 1H), 2.12-2.02 (m, 4H), 1.96 (d, *J* = 6.5 Hz, 2H), 1.89 (s, 3H), 1.86 (s, 3H). MS m/z (ESI): 964.8 [M+H]⁺.

### Example 147

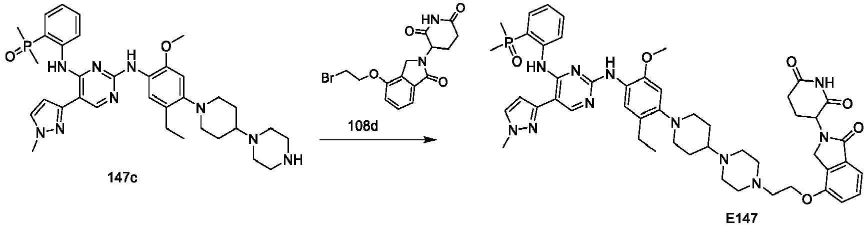

Title compound E147 (white solid, 10 mg, hydrochloride) was prepared with reference to the method for compound C-1.

¹H NMR (500 MHz, CD₃OD) δ 8.46 (s, 1H), 7.95 (dd, *J* = 13.0, 7.6 Hz, 1H), 7.78-7.70 (m, 3H), 7.65-7.53 (m, 2H), 7.50 (d, *J* = 7.3 Hz, 2H), 7.33 (d, *J* = 7.9 Hz, 1H), 7.06 (s, 1H), 6.81 (d, *J* = 2.4 Hz, 1H), 5.20 (dd, *J* = 13.3, 5.1 Hz, 1H), 4.75-4.52 (m, 4H), 3.96 (dd, *J* = 42.1, 20.6 Hz, 16H), 3.69 (dd, *J* = 16.5, 10.5 Hz, 2H), 3.42 (s, 2H), 3.26 (s, 1H), 2.99-2.90 (m, 1H), 2.86-2.79 (m, 1H), 2.60 (dt, *J* = 13.3, 8.6 Hz, 1H), 2.44 (dd, *J* = 29.9, 18.0 Hz, 4H), 2.23 (ddd, *J* = 16.9, 14.3, 10.9 Hz, 3H), 1.86 (d, *J* = 13.4 Hz, 6H), 0.99 (s, 3H). MS m/z (ESI): 931.0 [M+H]⁺.

### Example 148

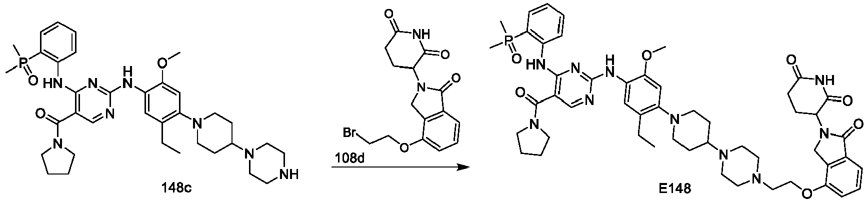

Compound E148 (pale yellow solid, 30 mg, hydrochloride) was prepared with reference to the method for compound C-1.

¹H NMR (500 MHz, CD₃OD) δ 8.28-7.92 (m, 2H), 7.74 (dd, *J* = 13.5, 8.8 Hz, 1H), 7.65-7.51 (m, 2H), 7.49-7.40 (m, 2H), 7.35-7.29 (m, 2H), 6.89 (s, 1H), 5.17 (dd, *J* = 13.3, 5.1 Hz, 1H), 4.69-4.47 (m, 4H), 4.00-3.71 (m, 12H), 3.66-3.60 (m, 4H), 3.57-3.49 (m 1H), 3.25 (s, 2H), 2.96-2.89 (m, 3H), 2.82-2.77 (m, 1H), 2.59-2.49 (m, 3H), 2.32 (d, *J* = 11.1 Hz, 2H), 2.24-2.14 (m, 1H), 2.12-1.93 (m, 7H), 1.84 (s, 3H), 1.82 (s, 3H), 1.05 (br s, 3H). MS m/z (ESI): 947.9 [M+H]⁺.

### Example 149

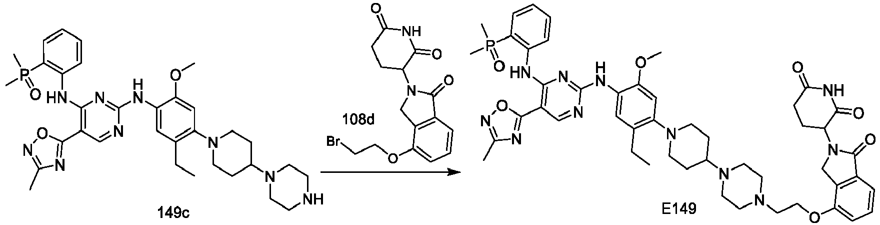

Compound E149 (pale yellow solid, 42 mg, hydrochloride) was prepared with reference to the method for compound C-1.

¹H NMR (500 MHz, DMSO-*d*₆) δ 12.19 (s, 1H), 11.01 (s, 1H), 10.76 (s, 1H), 8.88 (s, 1H), 7.85-7.68 (m, 2H), 7.52 (dt, *J* = 8.4, 4.4 Hz, 2H), 7.44 (s, 1H), 7.38 (dt, *J* = 7.6, 3.9 Hz, 2H), 7.30 (d, *J* = 8.1 Hz, 1H), 6.79 (s, 1H), 5.12 (dd, *J* = 13.3, 5.1 Hz, 1H), 4.62-4.55 (m, 3H), 4.34 (d, *J* = 17.6 Hz, 1H), 3.98-3.64 (m, 16H), 3.11 (s, 2H), 2.98-2.89 (m, 1H), 2.79 (s, 2H), 2.66-2.57 (m, 1H), 2.45-2.33 (m, 4H), 2.21 (d, *J* = 10.0 Hz, 2H), 2.06-1.99 (m, 1H), 1.93 (d, *J* = 9.0 Hz, 2H), 1.70 (s, 3H), 1.67 (s, 3H), 0.89 (s, 3H). MS m/z (ESI): 932.8 [M+H]⁺.

### Example 150

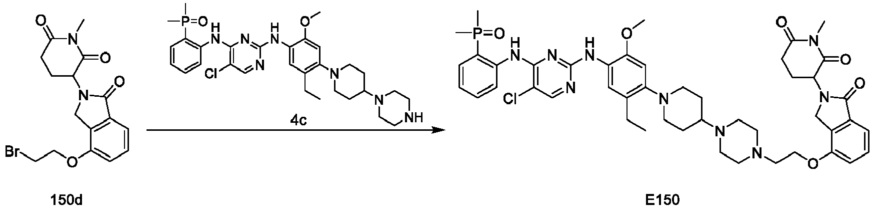

Compound E150 (yellow solid, 34 mg, hydrochloride) was prepared with reference to the method for compound C-1.

¹H NMR (500 MHz, DMSO-*d*₆) δ 12.38 (br s, 1H), 12.11 (s, 1H), 8.41-8.21 (m, 2H), 7.66 (dd, *J* = 13.7, 8.7 Hz, 1H), 7.53 (t, *J* = 7.8 Hz, 1H), 7.39-7.26 (m, 5H), 6.86 (s, 1H), 5.19 (dd, *J* = 13.4, 5.1 Hz, 1H), 4.64-4.55 (m, 3H), 4.34 (d, *J* = 17.6 Hz, 1H), 3.94-3.75 (m, 13H), 3.19 (d, *J* = 10.3 Hz, 2H), 3.06-2.95 (m, 5H), 2.86-2.74 (m, 3H), 2.58-2.53 (m, 2H), 2.46-2.37 (m, 1H), 2.24 (d, *J* = 10.5 Hz, 2H), 2.08-1.89 (m, 3H), 1.81 (s, 3H), 1.78 (s, 3H), 1.06 (t, *J* = 7.4 Hz, 3H). MS m/z (ESI): 898.8 [M+H]⁺.

### Example 151

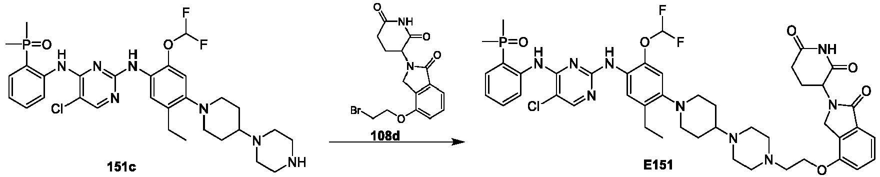

Title compound E151 (white solid, 41.92 mg, hydrochloride) was prepared with reference to the synthesis method for compound C-1.

¹H NMR (400 MHz, DMSO-*d*₆) δ 12.43 (s, 1 H), 11.92 (s, 1 H), 11.03 (s, 1 H), 9.62 (s, 1 H), 8.38-8.15 (m, 2 H), 7.62 (dd, *J* = 13.63, 7.52 Hz, 1 H), 7.57-7.48 (m, 1 H), 7.45-7.35 (m, 2 H), 7.34-7.18 (m, 3 H), 7.15-6.85 (m, 2 H), 5.13 (dd, *J* = 13.27, 4.83 Hz, 1 H), 4.69-4.50 (m, 3 H), 4.33 (d, *J* = 17.73 Hz, 1 H), 3.75 (s, 8 H), 3.44 (s, 3 H), 3.17 (d, *J* =10.15 Hz, 2 H), 3.01-2.86 (m, 1 H), 2.80-2.66 (m, 2 H), 2.65-2.55 (m, 3 H), 2.45-2.21 (m, 3 H), 2.09-1.86 (m, 3 H), 1.84-1.73 (m, 6 H), 1.10 (t, *J* = 7.40 Hz, 3 H). MS m/z (ESI): MS m/z (ESI): 920.5 [M+Na]⁺.

### Example 152

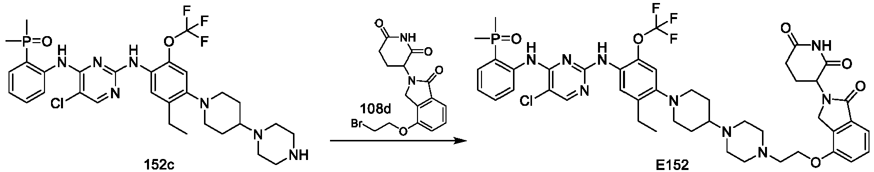

Compound E152 (white solid, 51.1 mg, formate) was prepared with reference to the method for reference compound C-1.

¹H NMR (400 MHz, DMSO-*d*₆) δ 11.27 (s, 1H), 11.01 (s, 1H), 8.89 (s, 1H), 8.40 (br s, 2H), 8.11 (s, 1H), 7.59-7.38 (m, 2H), 7.33-7.27 (m, 4H), 7.10-7.05 (m, 1H), 7.02 (s, 1H), 5.12 (dd, *J* = 13.20, 4.40 Hz, 1H), 4.40-4.36 (m, 1H), 4.26-4.21 (m, 3H), 3.30-3.20 (m, 2H), 3.05-3.02 (m, 2H), 2.74-2.30 (m, 15 H), 2.00-1.95 (m, 1H), 1.88-1.79 (m, 2H), 1.75 (m, 3H), 1.59 (m, 3H), 1.50-1.40 (m, 2H), 1.12 (t, *J* = 7.20 Hz, 3H). MS m/z (ESI): 960.5 [M+Na] ⁺.

### Example 153

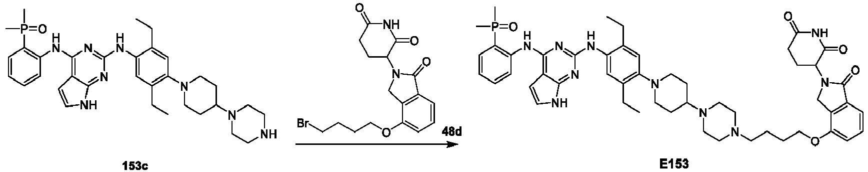

Title compound E153 (white solid, 8 mg, formate) was prepared with reference to the method for compound C-1.

¹H NMR (400 MHz, DMSO-*d*₆) δ ppm 11.54 (s, 1 H), 11.10 (s, 1 H), 11.01 (s, 1 H), 8.85 (dd, *J*=8.3, 3.4 Hz, 1 H), 8.19 (s, 1 H), 7.95 (s, 1 H), 7.55-7.46 (m, 2 H), 7.31 (d, *J*=7.3 Hz, 1 H), 7.28-7.20 (m, 2 H), 7.16 (s, 1 H), 7.05-6.93 (m, 1 H), 6.94-6.93 (m, 1 H), 6.87-6.81 (m, 1 H), 6.31-6.28 (m, 1 H), 5.11 (dd, *J*=13.4, 5.2 Hz, 1 H), 4.42-4.34 (m, 1 H), 4.26-4.11 (m, 4 H), 3.04 (d, *J*=10.9 Hz, 2 H), 2.99-2.85 (m, 1 H), 2.72-2.55 (m, 10 H), 2.47-2.28 (m, 8 H), 2.03-1.97 (m, 1 H), 1.90-1.72 (m, 10 H), 1.59 (dd, *J*=12.6, 8.2 Hz, 4 H), 1.14 (t, *J*=7.5 Hz, 3 H), 1.08 (t, *J*=7.6 Hz, 3 H). MS m/z (ESI): 915.6 [M+H]⁺.

### Example 154

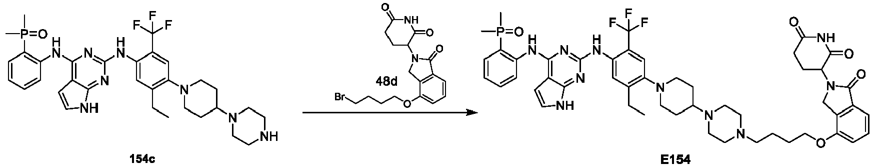

Compound E154 (white solid, 31.3 mg, formate) was prepared with reference to the method for reference compound C-1.

¹H NMR (400 MHz, DMSO-*d*₆) δ 11.83 (s, 1H), 11.24 (s, 1H), 11.00 (s, 1H), 8.77 (br s, 1H), 7.91 (s, 1H), 7.57 (s, 1H), 7.51-7.49 (m, 3H), 7.32-7.24 (m, 4H), 6.91-6.90 (m, 1H), 6.89 (s, 1H), 6.33 (s, 1H), 5.15-5.11 (m, 1H), 4.40-4.21 (m, 4H), 4.15 (s, 2H), 3.10-3.07 (m, 1H), 2.95-2.90 (m, 5H), 2.34-2.31 (m, 10H), 2.00-1.95 (m, 1H), 1.90-1.80 (m, 11H), 1.61-1.58 (m, 4H), 1.17 (t, *J* = 7.6, 3H). MS m/z (ESI): 955.6 [M+H]⁺.

### Example 155

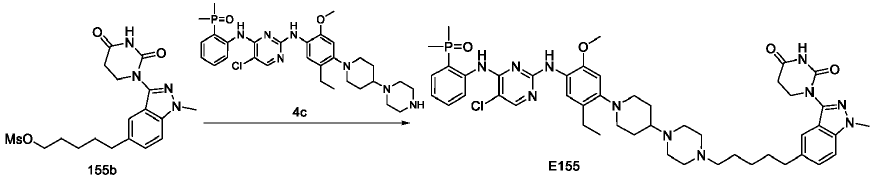

Title compound E155 (white solid, 5 mg, hydrochloride) was prepared with reference to the method for compound C-1.

¹H NMR (500 MHz, CD₃OD) δ 8.27-8.16 (m, 2H), 7.73 (dd, *J* = 10.0, 5.0 Hz, 1H), 7.59-7.41 (m, 5H), 7.36 (d, *J* = 10.0 Hz, 1H), 7.09 (s, 1H), 4.05 (t, *J* = 7.5 Hz, 2H), 4.04 (s, 3H), 3.92-3.68 (m, 12H), 3.47-3.45 (m, 2H), 3.29-3.26 (m, 4H), 2.92 (t, *J* = 7.5 Hz, 2H), 2.81 (t, *J* = 7.5 Hz, 2H), 2.69-2.65(m, 2H), 2.45-2.40(m, 2H), 2.29-2.26(m, 2H), 1.92(s, 3H), 1.89(s, 3H), 1.88-1.87(m, 2H), 1.83-1.77 (m, 2H), 1.50-1.43(m, 2H), 1.16-1.09(m, 3H). MS m/z (ESI): 910.5 [M+H]⁺.

### Example 156

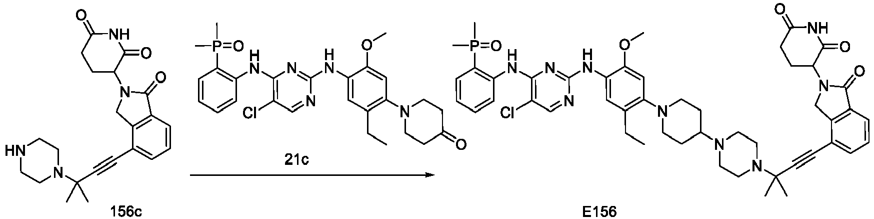

Title compound E156 (white solid, 6 mg, hydrochloride) was prepared with reference to the method for compound 21.

¹H NMR (500 MHz, CD₃OD) δ 8.30-8.12 (m, 2H), 7.88-7.85 (m, 2H), 7.72 (dd, *J* = 20.0, 10.0 Hz, 1H), 7.62-7.55 (m, 2H), 7.43 (t, *J* = 5.0 Hz, 1H), 7.32 (s, 1H), 6.95 (s, 1H), 5.23 (dd, *J* = 10.0, 5.0 Hz, 1H), 4.83-4.64 (m, 2H), 4.02-3.88 (m, 8H), 3.73-3.49 (m, 4H), 3.31-3.29 (m, 3H), 3.02-2.91 (m, 3H), 2.85-2.79 (m, 1H), 2.73-2.62(m, 3H), 2.37 (d, *J* = 15.0 Hz, 2H), 2.25-2.21 (m, 1H), 2.16-2.09 (m, 1H), 1.92 (s, 3H), 1.89 (s, 3H), 1.88 (s, 6H), 1.17-1.14 (m, 3H). MS m/z (ESI): 906.8 [M+H]⁺.

### Example 157

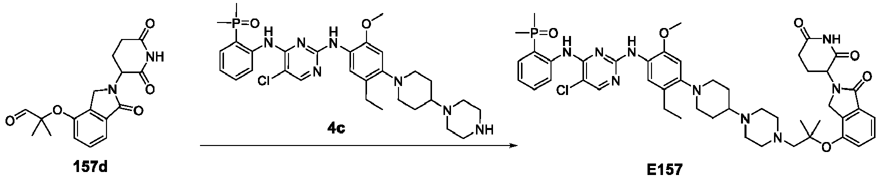

Compound 157d (30 mg, 0.09 mmol) and compound 4c (54 mg, 0.09 mmol) were added to a single-necked flask at room temperature before DCE (5 mL) was added. Then AcOH (11 mg, 0.18 mmol) and TEA (27 mg, 0.27 mmol) were sequentially added. The reaction system was stirred at room temperature in a nitrogen atmosphere for 1 h. NaBH(OAc)₃ (57 mg, 0.27 mmol) was then added. The system was then stirred at room temperature for 16 h. The solvent was removed at reduced pressure, and the crude product was separated and purified by prep-HPLC to give target product E157 (pale yellow solid, 30 mg, hydrochloride). ¹H NMR (500 MHz, CD3OD) δ 8.40-8.10 (m, 2H), 7.74 (dd, *J* = 13.9, 7.7 Hz, 1H), 7.63-7.51 (m, 4H), 7.49-7.41 (m, 2H), 7.14 (s, 1H), 5.20 (dd, *J* = 13.3, 5.1 Hz, 1H), 4.69 (d, *J* = 17.4 Hz, 1H), 4.56 (d, *J* = 16.5 Hz, 1H), 4.20-3.98 (m, 8H), 3.93 (s, 3H), 3.81 (s, 3H), 3.55-3.47 (m, 2H), 3.35 (d, *J* = 16.1 Hz, 2H), 3.00-2.91 (m, 1H), 2.86-2.79 (m, 1H), 2.72-2.62 (m, 3H), 2.48 (d, *J* = 11.2 Hz, 2H), 2.40-2.28 (m, 2H), 2.26-2.19 (m, 1H), 1.92 (s, 3H), 1.89 (s, 3H), 1.67 (d, *J* = 7.1 Hz, 6H), 1.13 (t, *J* = 7.2 Hz, 3H). MS m/z (ESI): 912.5 [M+H]⁺.

### Example 158

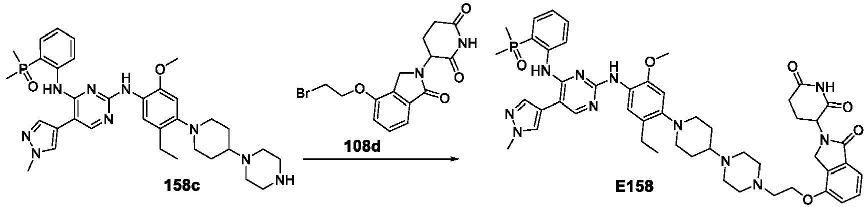

Title compound E158 (pale yellow solid, 5.2 mg, hydrochloride) was prepared with reference to the method for compound C-1.

¹H NMR (500 MHz, CD3OD) δ 8.18-8.02 (m, 2H), 7.93 (d, *J* = 31.0 Hz, 2H), 7.75-7.53 (m, 4H), 7.48 (d, *J* = 6.2 Hz, 2H), 7.33 (s, 2H), 5.28-5.16 (m, 1H), 4.78-4.58 (m, 4H), 4.13-3.88 (m, 17H), 3.81-3.61 (m, 4H), 3.02-2.78 (m, 2H), 2.76-2.48 (m, 7H), 2.30-2.12 (m, 1H), 1.82 (d, *J* = 9.2 Hz, 6H), 1.07 (t, *J* = 7.2 Hz, 3H). MS m/z (ESI): 930.6 [M+H]⁺.

### Examples 159 and 160

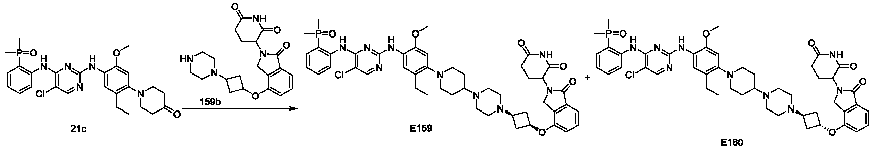

Compounds E159 (white solid, 6 mg, hydrochloride) and E160 (white solid, 10 mg, hydrochloride) were prepared with reference to the method for compound E67.

E159: ¹H NMR (500 MHz, CD₃OD) δ 8.26 (s, 1H), 8.10 (s, 1H), 7.72-7.67 (m, 1H), 7.60-7.49 (m, 2H), 7.45-7.38 (m, 2H), 7.33 (s, 1H), 7.12 (d, *J* = 8.0 Hz, 1H), 6.99 (s, 1H), 5.14 (dd, *J* = 13.3, 5.2 Hz, 1H), 4.55-4.42 (m, 2H), 4.14-3.47 (m, 14H), 3.39-3.32 (m, 2H), 3.14-2.96 (m, 4H), 2.91 (ddd, *J* = 18.7, 13.5, 5.3 Hz, 1H), 2.82-2.75 (m, 1H), 2.63 (s, 4H), 2.52 (qd, *J* = 13.2, 4.5 Hz, 1H), 2.36 (s, 2H), 2.23-2.07 (m, 3H), 1.89 (s, 3H), 1.86 (s, 3H), 1.11 (br s, 3H). MS m/z (ESI): 910.8 [M+H]⁺.

E160: ¹H NMR (500 MHz, CD₃OD) δ 8.26 (s, 1H), 8.11 (s, 1H), 7.75 (dd, *J* = 13.5, 7.6 Hz, 1H), 7.65-7.57 (m, 2H), 7.51-7.36 (m, 4H), 7.03 (d, *J* = 8.0 Hz, 1H), 5.18-5.15 (m, 2H), 4.47 (dd, *J* = 38.1, 17.3 Hz, 2H), 4.26 (s, 1H), 4.15-3.71 (m, 16H), 3.12-3.01 (m, 2H), 3.00-2.88 (m, 1H), 2.81-2.58 (m, 9H), 2.53-2.49 (m, 1H), 2.25-2.10 (m, 1H), 1.89 (s, 3H), 1.86 (s, 3H), 1.07 (br s, 3H). MS m/z (ESI): 910.8 [M+H]⁺.

### Example 161

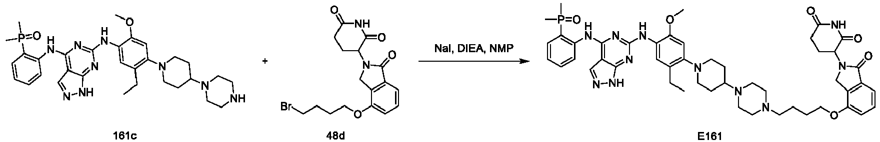

Compound E161 (yellow solid, 8.11 mg, formate) was prepared with reference to the method for reference compound C-1.

¹H NMR (400 MHz, DMSO-*d*₆) δ 13.18-12.83 (m, 1H), 12.68-12.42 (m, 1H), 12.35-12.14 (m, 1H), 11.03-10.90 (m, 1H), 10.02-9.66 (m, 1H), 8.41-8.28 (m, 1H), 7.81-7.71 (m, 1H), 7.51-7.45 (m, 1H), 7.39 (br s, 1H), 7.36-7.29 (m, 2H), 7.26 (d, *J* = 8.1 Hz, 1H), 6.90 (br s, 1H), 5.17-5.07 (m, 1H), 4.47 (br d, *J* = 17.4 Hz, 3H), 4.25 (br s, 3H), 4.20-4.13 (m, 4H), 3.84 (br s, 3H), 3.74-3.67 (m, 3H), 3.65-3.55 (m, 3H), 3.53-3.42 (m, 2H), 3.26-3.19 (m, 3H), 2.90 (br dd, *J* = 12.6,4.2 Hz, 3H), 2.69 (s, 3H), 2.31-2.21 (m, 2H), 2.21-2.14 (m, 2H), 1.94-1.87 (m, 3H), 1.84 (s, 3H), 1.81 (s, 3H), 1.09 (br s, 3H). MS m/z (ESI): 918.5 [M+H]⁺.

### Example 162

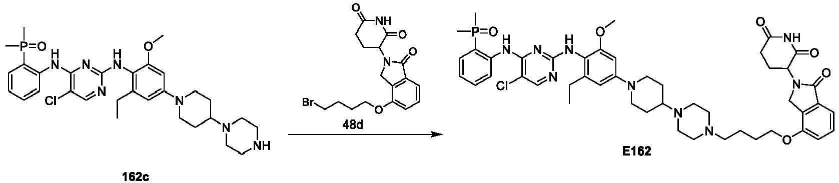

Title compound E162 (yellow solid, 8 mg, hydrochloride) was prepared with reference to the method for compound C-1.

¹H NMR (400 MHz, DMSO-d6) δ 11.23 (s, 1H), 11.01 (s, 1H), 8.46-8.33 (m, 1H), 8.25-8.16 (m, 1H), 7.66-7.46 (m, 3H), 7.37-7.28 (m, 3H), 7.22-7.17 (m, 2H), 7.06 (dd, *J* = 13.8, 7.3 Hz, 2H), 5.17-5.10 (m, 1H), 4.89-4.72 (m, 1H), 4.67-4.49 (m, 2H), 4.43-4.00 (m, 8H), 3.87-3.80 (m, 3H), 3.09-2.88 (m, 4H), 2.71-2.65 (m, 2H), 2.42-2.39 (m, 1H), 2.44-2.38 (m, 2H), 2.43-2.37 (m, 1H), 2.37-2.36 (m, 1H), 2.06-1.93 (m, 3H), 1.84-1.73 (m, 8H), 1.67-1.56 (m, 2H), 1.54-1.43 (m, 2H), 1.40-1.34 (m, 2H), 1.16-1.07 (m, 3H) MS m/z (ESI): 912.6 [M+H]⁺.

### Example 163

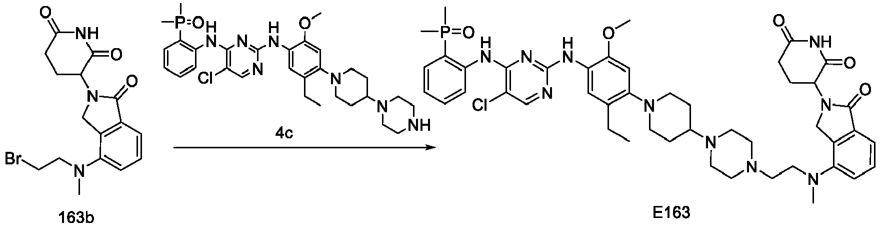

Title compound E163 (white solid, 5 mg, hydrochloride) was prepared with reference to the method for compound C-1.

¹H NMR (500 MHz, CD₃OD) δ 8.13 (dd, *J* = 10.0, 5.0 Hz, 1H), 7.96 (s, 1H), 7.57-7.52 (m, 2H), 7.41 (t, *J* = 5.0 Hz, 1H), 7.34 (t, *J* = 5.0 Hz, 1H), 7.24 (d, *J* = 5.0 Hz, 1H), 7.19 (t, *J* = 10.0 Hz, 1H), 7.07 (d, *J* = 5.0 Hz, 1H), 6.67 (s, 1H), 5.05 (dd, *J* = 10.0, 5.0 Hz, 1H), 3.74 (s, 3H), 3.33-3.20 (m, 2H), 3.07-3.05 (m, 1H), 2.98-2.95 (m, 2H), 2.84 (s, 3H), 2.81-2.72(m, 1H), 2.71-2.40(m, 15H), 2.27-2.21(m, 1H), 2.13-2.06 (m, 2H), 1.91-1.88(m, 2H), 1.76(s, 3H), 1.73(s, 3H), 1.60-1.49(m, 3H), 0.90-0.88 (m, 3H). MS m/z (ESI): 897.5 [M+H]⁺.

### Example 164

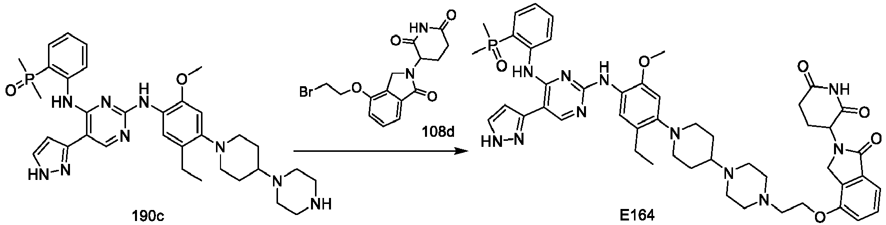

Title compound E164 (white solid, 8 mg, hydrochloride) was prepared with reference to the method for compound C-1.

¹H NMR (500 MHz, CD₃OD) δ 8.48 (s, 1H), 8.02-7.93 (m, 1H), 7.89-7.85 (m, 1H), 7.78-7.68 (m, 2H), 7.65-7.59 (m, 1H), 7.57 (t, *J* = 7.8 Hz, 1H), 7.50 (d, *J* = 7.4 Hz, 1H), 7.46 (s, 1H), 7.33 (d, *J* = 8.0 Hz, 1H), 6.99 (s, 1H), 6.87 (d, *J* = 2.6 Hz, 1H), 5.20 (dd, *J* = 13.4, 5.2 Hz, 1H), 4.75-4.52 (m, 4H), 4.03-3.83 (m, 12H), 3.69-3.58 (m, 1H), 3.40-3.33 (m, 3H), 3.20-3.04 (m, 2H), 3.02-2.91 (m, 1H), 2.86-2.79 (m, 1H), 2.65-2.55 (m, 1H), 2.54-2.32 (m, 4H), 2.29-2.13 (m, 3H), 1.85 (d, *J* = 13.4 Hz, 6H), 1.00 (s, 3H). MS m/z (ESI): 916.8 [M+H]⁺.

### Example 165

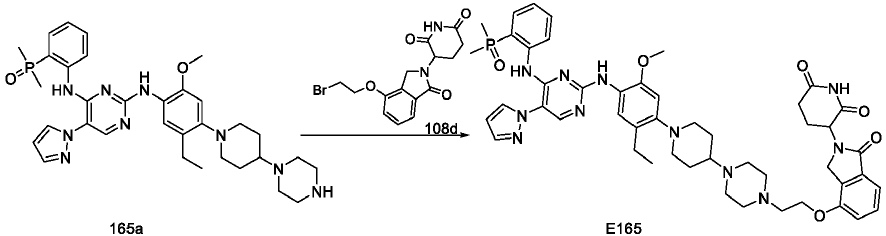

Title compound E165 (yellow solid, 12 mg, hydrochloride) was prepared with reference to the method for compound C-1.

¹H NMR (500 MHz, CD₃OD) δ 8.18 (s, 1H), 8.07 (d, *J* = 2.5 Hz, 1H), 7.97 (s, 1H), 7.87 (d, *J* = 2.0 Hz, 1H), 7.76-7.72 (m, 1H), 7.59 (s, 1H), 7.56-7.53 (m, 1H), 7.48-7.44 (m, 2H), 7.36-7.26 (m, 2H), 6.91 (s, 1H), 6.64-6.58 (m, 1H), 5.17 (dd, *J* = 13.5, 5.5 Hz, 1H), 4.68 (d, *J* = 17.5 Hz, 1H), 4.63-4.58 (m, 2H), 4.53 (d, *J* = 17.5 Hz, 1H), 3.84 (m, 13H), 3.56 (t, *J* = 10.5 Hz, 1H), 3.26 (m, 2H), 3.03-2.86 (m, 3H), 2.86-2.75 (m, 1H), 2.57 (m, 3H), 2.34 (d, *J* = 11.5 Hz, 2H), 2.20 (m, 1H), 2.14-2.02 (m, 2H), 1.80 (s, 3H), 1.77 (s, 3H), 1.07 (s, 3H). MS m/z (ESI): 916.9 [M+H]⁺.

### Example 166

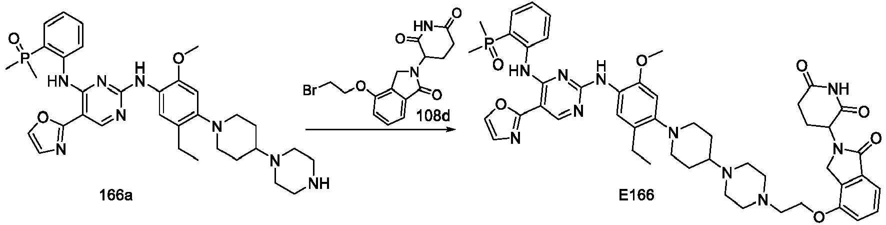

Title compound E166 (yellow solid, 20 mg, hydrochloride) was prepared with reference to the method for compound C-1.

¹H NMR (500 MHz, CD₃OD) δ 8.73 (s, 1H), 8.11 (s, 1H), 7.95-7.87 (m, 1H), 7.74 (t, *J* = 7.5 Hz, 2H), 7.61 (t, *J* =7.5 Hz, 2H), 7.57 (t, *J* =7.5 Hz, 2H), 7.50 (d, *J* =7.0 Hz, 2H), 7.46 (s, 1H), 7.33-7.32 (m, 1H), 6.90 (s, 1H), 5.20 (dd, *J* = 13.0, 5.0 Hz, 1H), 4.69 (d, *J* = 17.5 Hz, 1H), 4.62 (t, *J* = 5.0 Hz, 2H), 4.55 (d, *J* = 17.5 Hz, 1H), 4.00-3.64 (m, 13H), 3.58-3.54 (m, 1H), 3.26 (d, *J* = 12.5 Hz, 2H), 2.99-2.92 (m, 3H), 2.87-2.80 (m, 1H), 2.63-2.33 (m, 1H), 2.25-2.20 (m, 1H), 2.12-2.04 (m, 2H), 1.86 (s, 3H), 1.84 (s, 3H), 0.99 (s, 3H). MS m/z (ESI): 917.7 [M+H]⁺.

### Example 167

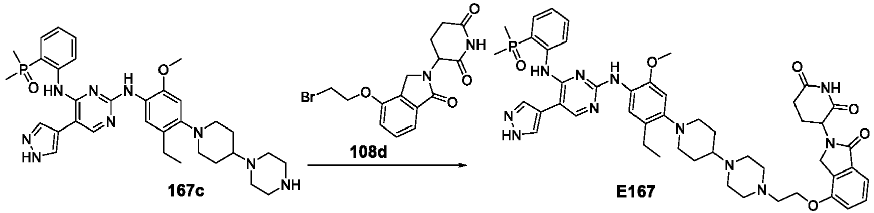

Title compound E167 (pale yellow solid, 5.0 mg, hydrochloride) was prepared with reference to the method for compound C-1.

¹H NMR (500 MHz, CD₃OD) δ 8.11 (s, 3H), 7.87 (s, 1H), 7.67 (dd, *J* = 13.8, 7.7 Hz, 1H), 7.57 (t, *J* = 7.8 Hz, 2H), 7.50 (d, *J* = 7.5 Hz, 1H), 7.42 (t, *J* = 6.9 Hz, 2H), 7.33 (d, *J* = 8.1 Hz, 1H), 7.02 (s, 1H), 5.22-5.18 (m, 1H), 4.88-4.50 (m, 4H), 4.06-3.83 (m, 12H), 3.71-3.61 (m, 1H), 3.43-3.34 (m, 3H), 3.18-3.02 (m, 2H), 3.01-2.91 (m, 1H), 2.87-2.78 (m, 1H), 2.68-2.54 (m, 3H), 2.41 (d, *J* = 11.4 Hz, 2H), 2.26-2.12 (m, 3H), 1.80 (d, *J* = 13.5 Hz, 6H), 1.11 (t, *J* = 7.2 Hz, 3H). MS m/z (ESI): 916.4 [M+H]⁺.

### Example 168

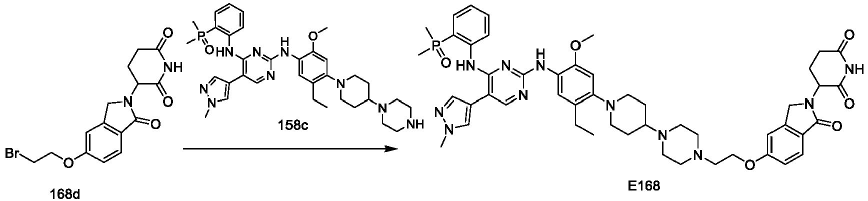

Title compound E168 (white solid, 6 mg, hydrochloride) was prepared with reference to the method for compound C-1.

¹H NMR (500 MHz, CD₃OD) δ7.98 (dd, *J* = 8.2, 4.2 Hz, 1H), 7.91 (s, 1H), 7.78 (s, 1H), 7.72 (s, 1H), 7.62 (t, *J* = 7.2 Hz, 1H), 7.56 (s, 1H), 7.53-7.40 (m, 2H), 7.16 (t, *J* = 7.6 Hz, 1H), 7.05 (d, *J* = 10.1 Hz, 1H), 7.02-6.97 (m, 1H), 6.66 (s, 1H), 5.05-4.97 (m, 1H), 4.48 (s, 2H), 4.41-4.27 (m, 2H), 4.16 (t, *J* = 5.3 Hz, 2H), 3.86 (s, 3H), 3.76 (s, 3H), 2.95 (d, *J* = 11.6 Hz, 2H), 2.85-2.75 (m, 4H), 2.71-2.58 (m, 8H), 2.40-2.30 (m, 4H), 2.07 (dd, *J* = 11.5, 6.2 Hz, 1H), 1.92 (d, *J* = 10.5 Hz, 2H), 1.69 (t, *J* = 17.8 Hz, 6H), 1.64-1.56 (m, 2H), 0.83 (t, *J* = 7.5 Hz, 3H). MS m/z (ESI): 930.7 [M+H]⁺.

### Example 170

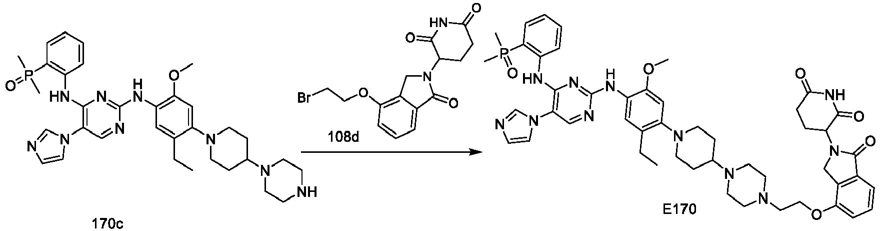

Title compound E170 (white solid, 8 mg, hydrochloride) was prepared with reference to the method for compound C-1.

¹H NMR (500 MHz, CD₃OD) δ8.05 (s, 1H), 7.93 (s, 1H), 7.79 (s, 1H), 7.64 (s, 1H), 7.50 (dd, *J* = 13.1, 7.1 Hz, 1H), 7.42 (t, *J* = 7.8 Hz, 2H), 7.33 (d, *J* = 7.5 Hz, 1H), 7.19 (t, *J* = 16.3 Hz, 3H), 7.12 (s, 1H), 6.69 (s, 1H), 5.10-5.06 (m, 1H), 4.64 (s, 1H), 4.49 (s, 8H), 4.35 (dt, *J* = 47.8, 23.9 Hz, 2H), 4.26 (s, 1H), 3.76 (s, 3H), 3.05 (dd, *J* = 14.5, 5.8 Hz, 4H), 2.91 (s, 3H), 2.73-2.64 (m, 3H), 2.44-2.32 (m, 3H), 2.10 (d, *J* = 5.4 Hz, 2H), 1.74 (d, *J* = 13.6 Hz, 2H), 1.64 (d, *J* = 13.5 Hz, 6H), 0.89 (t, *J* = 7.4 Hz, 3H). MS m/z (ESI): 916.6 [M+H]⁺.

### Example 171

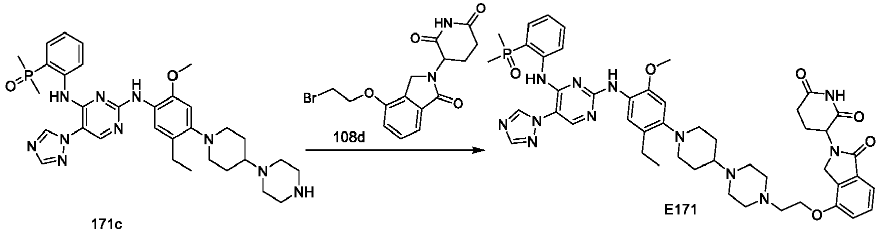

Title compound E171 (white solid, 6 mg, hydrochloride) was prepared with reference to the method for compound C-1.

¹H NMR (500 MHz, CD₃OD) δ9.03 (s, 1H), 8.39 (d, *J* = 29.2 Hz, 2H), 7.72 (dd, *J* = 13.6, 7.7 Hz, 1H), 7.57 (t, *J* = 7.8 Hz, 3H), 7.52-7.36 (m, 3H), 7.33 (d, *J* = 7.9 Hz, 1H), 7.05 (s, 1H), 5.20 (dd, *J* = 13.3, 5.1 Hz, 1H), 4.79-4.42 (m, 2H), 4.22-3.82 (m, 10H), 3.80-3.50 (m, 4H), 3.41 (s, 2H), 3.32 (s, 2H), 3.26-3.04 (m, 2H), 3.02-2.88 (m, 1H), 2.82 (d, *J* = 17.7 Hz, 1H), 2.71-2.53 (m, 3H), 2.43 (d, *J* = 11.1 Hz, 2H), 2.23 (d, *J* = 5.4 Hz, 3H), 1.81 (d, *J* = 13.5 Hz, 6H), 1.15 (s, 3H). MS m/z (ESI): 917.5 [M+H]⁺.

### Example 172

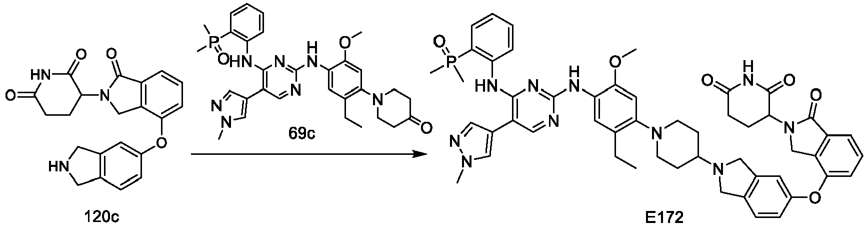

Title compound E172 (white solid, 10 mg, hydrochloride) was prepared with reference to the method for compound E67.

¹H NMR (500 MHz, CD₃OD) δ 8.16-8.01 (m, 2H), 7.90 (s, 1H), 7.80 (s, 1H), 7.72-7.53 (m, 5H), 7.51-7.47 (m, 1H), 7.45 (t, *J* = 7.0 Hz, 1H), 7.22 (d, *J* = 7.5 Hz, 2H), 7.17 (d, *J* = 5.7 Hz, 2H), 5.17 (dd, *J* = 13.3, 5.1 Hz, 1H), 4.98 (dd, *J* = 23.5, 11.3 Hz, 2H), 4.78 (d, *J* = 9.2 Hz, 2H), 4.45 (q, *J* = 17.3 Hz, 2H), 4.03 (d, *J* = 12.0 Hz, 3H), 3.98 (s, 3H), 3.73-3.33 (m, 5H), 2.95-2.84 (m, 1H), 2.79 (ddd, *J* = 17.5, 4.4, 2.3 Hz, 1H), 2.68 (dd, *J* = 14.6, 7.2 Hz, 2H), 2.61-2.24 (m, 5H), 2.22-2.11 (m, 1H), 1.82 (d, *J* = 13.5 Hz, 6H), 1.09 (d, *J* = 7.2 Hz, 3H). MS m/z (ESI): 935.6 [M+H]⁺.

### Example 173

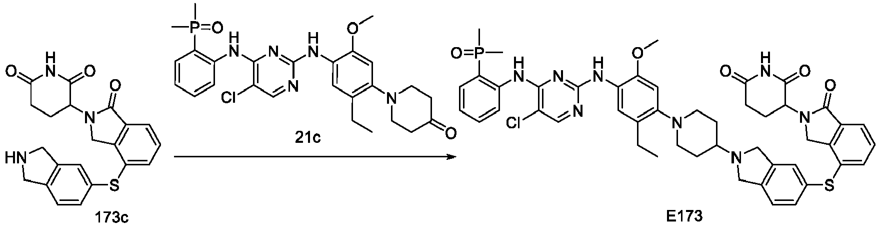

Title compound E173 (white solid, 3 mg, hydrochloride) was prepared with reference to the method for compound E67.

¹H NMR (500 MHz, CD₃OD) δ 8.27-8.19 (m, 2H), 7.84 (d, *J* = 10.0 Hz, 1H), 7.77-7.58 (m, 5H), 7.48-7.37 (m, 5H), 7.18 (s, 1H), 5.16 (dd, *J* = 10.0, 5.0 Hz, 1H), 4.81-4.75 (m, 2H), 3.94-3.87 (m, 4H), 3.53-3.37 (m, 4H), 2.95-2.67 (m, 4H), 2.54-2.16 (m, 6H), 1.92 (s, 3H), 1.89 (s, 3H), 1.15-1.13(m, 3H). MS m/z (ESI): 905.6 [M+H]⁺.

### Example 174

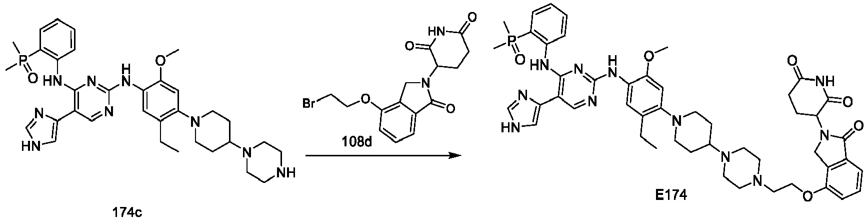

Title compound E174 (white solid, 6 mg, hydrochloride) was prepared with reference to the method for compound C-1.

¹H NMR (500 MHz, CD₃OD) δ 9.01 (s, 1H), 8.08-8.06 (m, 2H), 7.82 (s, 1H), 7.56 (dd, *J* = 10.0, 5.0 Hz, 1H), 7.48-7.32 (m, 5H), 7.21 (d, *J* = 5.0 Hz, 1H), 7.10 (s, 1H), 5.09 (dd, *J* = 10.0, 5.0 Hz, 1H), 4.64-4.44 (m, 5H), 3.98-3.70 (m, 15H), 3.50-3.29 (m, 4H), 2.87-2.75(m, 2H), 2.67-2.25(m, 8H), 2.25-2.07(m, 1H), 1.72(s, 3H), 1.70(s, 3H), 1.10-0.95 (m, 3H). MS m/z (ESI): 916.6 [M+H]⁺.

### Example 175

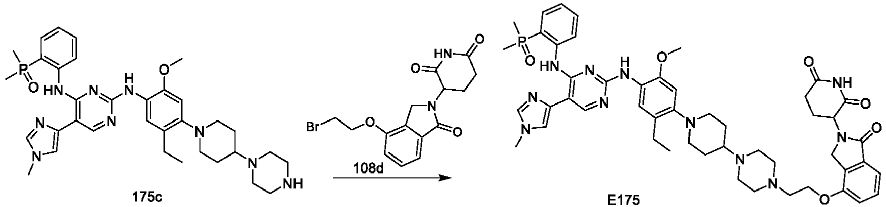

Title compound E175 (white solid, 5 mg, hydrochloride) was prepared with reference to the method for compound C-1.

¹H NMR (500 MHz, CD₃OD) δ 9.12 (s, 1H), 8.24-8.23 (m, 2H), 7.95 (s, 1H), 7.68 (dd, *J* = 10.0, 5.0 Hz, 1H), 7.61-7.44 (m, 5H), 7.32 (d, *J* = 5.0 Hz, 2H), 5.20 (dd, *J* = 10.0, 5.0 Hz, 1H), 4.74-4.56 (m, 5H), 4.05-3.91 (m, 16H), 3.72-3.60 (m, 4H), 2.99-2.91(m, 1H), 2.84-2.71(m, 3H), 2.66-2.48(m, 5H), 2.24-2.19(m, 1H), 1.86(s, 3H), 1.83(s, 3H), 1.19-1.05 (m, 3H). MS m/z (ESI): 930.7 [M+H]⁺.

### Example 176

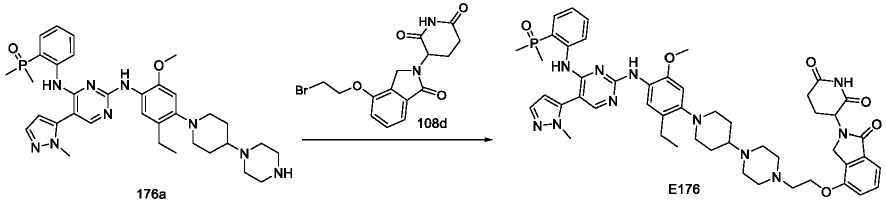

Title compound E176 (yellow solid, 14 mg, hydrochloride) was prepared with reference to the method for compound C-1.

¹H NMR (500 MHz, CD₃OD) δ 8.10 (d, *J* = 8.5 Hz, 2H), 7.82 (d, *J* = 2.0 Hz, 1H), 7.70-7.65 (m, 1H), 7.57 (t, *J* = 8.0 Hz, 2H), 7.50 (d, *J* = 7.5 Hz, 2H), 7.44-7.42 (m, 1H), 7.33 (d, *J* = 8.0 Hz, 1H), 7.16 (s, 1H), 6.70 (d, *J* = 2.0 Hz, 1H), 5.20 (dd, *J* = 13.5, 5.0 Hz, 1H), 4.73 (d, *J* = 17.5 Hz, 1H), 4.66 (t, *J* = 5.0 Hz, 2H), 4.57 (d, *J* = 18.0 Hz, 1H), 4.14-3.85 (m, 16H), 3.82-3.74 (m, 1H), 3.60-3.35 (m, 4H), 2.99-2.92 (m, 1H), 2.88-2.80 (m, 1H), 2.67-2.56 (m, 3H), 2.49 (d, *J* = 12.0 Hz, 2H), 2.37 (s, 2H), 2.25-2.20 (m 1H), 1.79 (s, 3H), 1.77 (s, 3H), 1.12 (s, 3H). MS m/z (ESI): 930.5 [M+H]⁺.

### Example 177

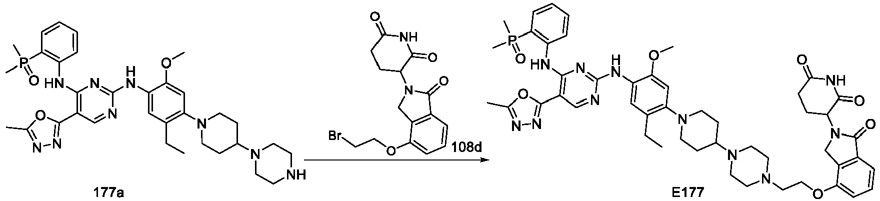

Title compound E177 (yellow solid, 5 mg) was prepared with reference to the method for compound C-1.

¹H NMR (500 MHz, CD₃OD) δ 8.68 (s, 1H), 7.96 (dd, *J* = 12.5, 7.5 Hz, 1H), 7.73-7.65 (m, 2H), 7.62 (s, 1H), 7.56-7.50 (m, 2H), 7.43 (d, *J* = 7.5 Hz, 1H), 7.27 (dd, *J* = 8.0, 0.5 Hz, 1H), 6.75 (s, 1H), 5.17 (dd, *J* = 13.5, 5.0 Hz, 1H), 4.46 (q, *J* = 17.5 Hz, 2H), 4.40-4.32 (m, 2H), 3.85 (s, 3H), 3.26-2.87 (m, 9H), 2.85-2.69 (m, 4H), 2.63 (s, 3H), 2.56-2.47 (m, 1H), 2.32 (s, 2H), 2.12-2.16 (m, 3H), 1.88 (t, *J* = 12.0 Hz, 2H), 1.82 (s, 3H), 1.79 (s, 3H), 1.35-1.25 (m, 3H), 0.94-0.82 (m, 3H). MS m/z (ESI): 932.7 [M+H]⁺.

### Example 178

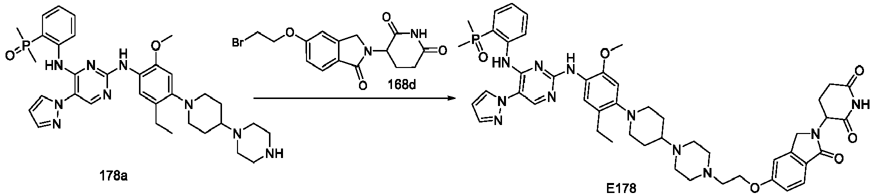

Title compound E178 (yellow solid, 6 mg) was prepared with reference to the method for compound C-1.

¹H NMR (500 MHz, CD₃OD) δ 8.16 (s, 1H), 8.02 (dd, *J* = 8.0, 4.0 Hz, 1H), 7.99 (d, *J* = 2.0 Hz, 1H), 7.83 (d, *J* = 2.0 Hz, 1H), 7.75-7.71 (m, 3H), 7.58 (t, *J* = 7.5 Hz, 1H), 7.36 (t, *J* = 7.5 Hz, 1H), 7.18 (d, 7 = 2.0 Hz, 1H), 7.13 (dd, *J* = 8.5, 2.0 Hz, 1H), 6.78 (s, 1H), 6.57 (t, *J* = 2.0 Hz, 1H), 5.14 (dd, *J* = 13.5, 5.5 Hz, 1H), 4.52-4.43 (m, 2H), 4.28 (t, *J* = 5.5 Hz, 2H), 3.87 (s, 3H), 3.07 (d, *J* = 11.5 Hz, 2H), 2.95-2.89 (m, 3H), 2.84-2.72 (m, 9H), 2.52-2.41 (m, 4H), 2.22-2.16 (m, 1H), 2.04 (d, *J* = 10.5 Hz, 2H), 1.80 (s, 3H), 1.78 (s, 3H), 1.75-1.67 (m, 2H), 1.36-1.31 (m, 2H), 0.97 (t, *J* = 7.5 Hz, 3H). MS m/z (ESI): 916.7 [M+H]⁺.

### Example 179

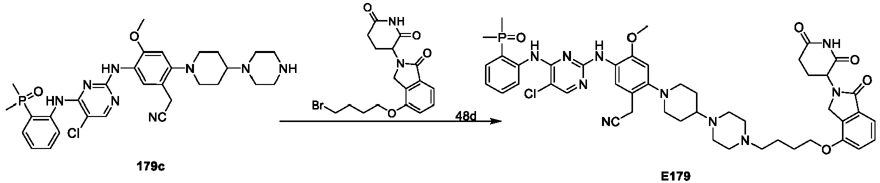

Title compound E179 (yellow solid, 4.5 mg, hydrochloride) was prepared with reference to the method for compound C-1.

¹H NMR (400 MHz, DMSO-*d*₆) δ 12.60-12.40 (m, 1H), 12.55-12.21 (m, 1H), 12.12-11.92 (m, 1H), 10.99 (s, 1H), 9.84-9.62 (m, 1H), 8.40-8.31 (m, 1H), 8.21 (brs, 1H), 7.65 (dd, *J* = 13.4, 7.7 Hz, 1H), 7.53-7.46 (m, 1H), 7.44-7.36 (m, 1H), 7.33 (d, *J* = 7.5 Hz, 1H), 7.28-7.22 (m, 1H), 7.00 (s, 1H), 5.13 (dd, *J* = 13.2, 5.1 Hz, 1H), 4.47 (d, *J* = 17.5 Hz, 2H), 4.40-4.34 (m, 1H), 4.33-4.23 (m, 2H), 4.17 (d, *J* = 4.3 Hz, 2H), 3.88 (s, 2H), 3.82 (s, 4H), 3.80-3.67 (m, 4H), 3.66-3.54 (m, 2H), 3.51-3.37 (m, 1H), 3.29 (d, *J* = 7.1 Hz, 1H), 3.25 (brs, 1H), 3.14 (d, *J* = 10.6 Hz, 2H), 2.99-2.80 (m, 3H), 2.64-2.60 (m, 1H), 2.27-2.14 (m, 2H), 2.05-1.89 (m, 5H), 1.85 (br s, 1H), 1.82 (s, 3H), 1.78 (s, 3H). MS m/z (ESI): 923.5 [M+H]⁺.

### Example 180

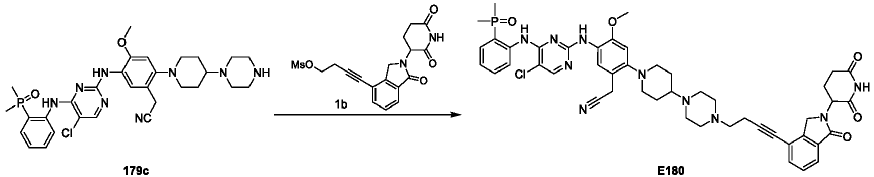

Title compound E180 (yellow solid, 6.6 mg, hydrochloride) was prepared with reference to the method for compound E4.

¹H NMR (400 MHz, DMSO-*d*₆) δ 11.18 (s, 1H), 11.06-11.01 (m, 1H), 8.46-8.35 (m, 1H), 8.26 (s, 1H), 8.12 (s, 1H), 7.75-7.68 (m, 1H), 7.66-7.58 (m, 2H), 7.49-7.49 (m, 1H), 7.58-7.49 (m, 2H), 7.42-7.33 (m, 1H), 7.12 (t, *J* = 7.4 Hz, 1H), 6.92 (s, 1H), 5.22-5.14 (m, 1H), 4.51-4.41 (m, 1H), 4.35-4.24 (m, 1H), 3.81 (brs, 2H), 3.79 (s, 3H), 3.66-3.58 (m, 1H), 3.04-2.87 (m, 5H), 2.81-2.68 (m, 4H), 2.68-2.63 (m, 4H), 2.60 (brs, 3H), 2.33-2.24 (m, 4H), 2.09-1.96 (m, 2H), 1.78 (s, 3H), 1.75 (s, 3H), 1.66-1.58 (m, 2H). MS m/z (ESI): 903.4 [M+H]⁺.

### Examples 181, 182, 183, and 184

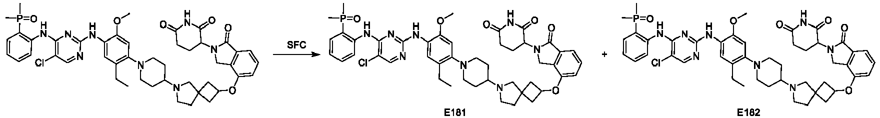

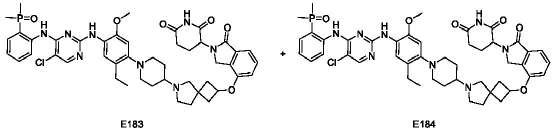

Compound E83 was subjected to 3 SFC separations (mobile phase: 80% EtOH + ACN (0.1% NH₃-H₂O) at supercritical CO₂, flow rate: 120 g/min, gradient time: 12 min, total time: 120 min, volume per injection: 2.0 mL) & (mobile phase: 80% EtOH + ACN (0.1% NH₃·H₂O) at supercritical CO₂, flow rate: 120 g/min, gradient time: 20 min, total time: 40 min, volume per injection: 10.0 mL) & (mobile phase: 30% EtOH + ACN (0.1% TEA) at supercritical CO₂, flow rate: 100 g/min, gradient time: 20 min, total time: 40 min, volume per injection: 10.0 mL) and reverse-phase preparative chromatography (hydrochloric acid system) to give compound E181 (yellow solid, 36.31 mg, hydrochloride, retention time: 5.406 min), compound E182 (yellow solid, 53.5 mg, hydrochloride, retention time: 6.466 min), compound E183 (yellow solid, 65.57 mg, hydrochloride, retention time: 3.052 min), and compound E184 (yellow solid, 44.13 mg, hydrochloride, retention time: 4.406 min).

**E181:** ¹H NMR (400 MHz, DMSO-*d*₆) δ 12.22 (br s, 1H), 11.70 (br s, 1H), 10.99 (s, 1H), 8.42 s, 1H), 8.27 (br s, 1H), 7.75-7.61 (m, 1H), 7.48 (t, *J* = 8.0 Hz, 1H), 7.44-7.25 (m, 4H), 7.08 (d, *J* = 8.0 Hz, 1H), 6.93 (s, 1H), 5.16-5.08 (m, 1H), 4.93-4.88 (m, 2H), 4.39-4.23 (m, 1H), 3.81 (s, 3H), 3.74-3.65 (m, 1H), 3.47 (d, *J* = 5.4 Hz, 1H), 3.26-3.19 (m, 4H), 2.98-2.79 (m, 4H), 2.77-2.67 (m, 1H), 2.65-2.54 (m, 3H), 2.48-2.38 (m, 1H), 2.29-2.16 (m, 3H), 2.15-2.04 (m, 6H), 2.03-1.93 (m, 1H), 1.82 (s, 3H), 1.78 (s, 3H), 1.06 (t, *J* = 7.1 Hz, 3H). MS m/z (ESI): 881.6 [M+H]⁺.

**E182:** ¹H NMR (400 MHz, DMSO-*d*₆) δ 12.20 (br s, 1H), 11.74 (br s, 1H), 10.99 (br s, 1H), 8.54-8.13 (m, 2H), 7.77-7.60 (m, 1H), 7.53-7.44 (m, 1H), 7.42-7.23 (m, 4H), 7.12 (br d, *J* = 8.0 Hz, 1H), 6.93 (s, 1H), 5.16-5.08 (m, 1H), 4.99-4.86 (m, 2H), 4.39-4.23 (m, 1H), 3.90 (br d, *J* = 10.5 Hz, 1H), 3.80 (s, 3H), 3.53 (br s, 2H), 3.28-3.22 (m, 4H), 2.98-2.77 (m, 3H), 2.70-2.53 (m, 5H), 2.47-2.38 (m, 1H), 2.34-2.26 (m, 1H), 2.26-2.09 (m, 5H), 2.09-1.95 (m, 3H), 1.81 (s, 3H), 1.78 (s, 3H), 1.05 (t, *J* = 7.0 Hz, 3H). MS m/z (ESI): 881.3 [M+H]⁺.

**E183:** ¹H NMR (400 MHz, DMSO-*d*₆) δ 12.23 (br s, 1H), 11.73 (s, 1H), 10.99 (s, 1H), 8.44 (br s, 1H), 8.22 (br s, 1H), 7.68 (dd, *J* = 13.6, 7.8 Hz, 1H), 7.54-7.43 (m, 1H), 7.43-7.24 (m, 4H), 7.12-6.91 (m, 2H), 5.15-5.08 (m, 1H), 4.95-4.88 (m, 2H), 4.39-4.19 (m, 2H), 3.81 (s, 3H), 3.74-3.66 (m, 1H), 3.47 (br d, *J* = 5.4 Hz, 1H), 3.28-3.16 (m, 4H), 3.02-2.85 (m, 4H), 2.72 (br s, 1H), 2.65-2.55 (m, 3H), 2.48-2.37 (m, 1H), 2.30-2.19 (m, 3H), 2.19-2.05 (m, 5H), 2.04-1.94 (m, 1H), 1.82 (s, 3H), 1.78 (s, 3H), 1.12-1.00 (m, 3H). MS m/z (ESI): 881.3 [M+H]⁺.

**E184:** ¹H NMR (400 MHz, DMSO-*d*₆) δ 12.21 (br s, 1H), 11.73 (br s, 1H), 11.00 (s, 1H), 8.51 (br s, 1H), 8.34-8.13 (m, 1H), 7.68 (br dd, *J* = 13.6, 7.7 Hz, 1H), 7.52-7.45 (m, 1H), 7.43-7.24 (m, 4H), 7.13 (d, *J* = 8.0 Hz, 1H), 6.94 (br s, 1H), 5.16-5.09 (m, 1H), 4.95-4.89 (m, 2H), 4.42-4.24 (m, 2H), 3.80 (s, 3H), 3.54 (br s, 2H), 3.28-3.20 (m, 4H), 2.99-2.80 (m, 3H), 2.66 (br s, 1H), 2.63- 2.55 (m, 4H), 2.49-2.39 (m, 1H), 2.34-2.27 (m, 1H), 2.26-2.11 (m, 5H), 2.10-1.95 (m, 3H), 1.82 (s, 3H), 1.78 (s, 3H), 1.05 (t, *J* = 7.0 Hz, 3H). MS m/z (ESI): 881.6 [M+H]⁺.

### Example 185

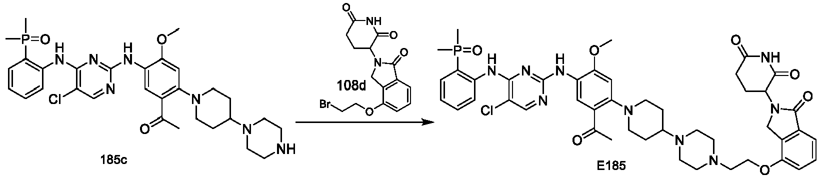

Compound E185 (white solid, 25 mg) was prepared with reference to the method forreference compound C-1.

¹H NMR (400 MHz, DMSO-*d*₆) δ 11.23 (s, 1H), 10.99 (s, 1H), 8.41 (brs, 1H), 8.30 (s, 1H), 8.10 (s, 1H), 7.62 (s, 1H), 7.50-7.47 (m, 2H), 7.32-7.27 (m, 3H), 7.10-7.07 (m, 1H), 6.77 (s, 1H), 5.13 (d, *J* =4.80, 1H), 4.39-4.35 (m, 1H), 4.25-4.21 (m, 3H), 3.84 (s, 3H), 3.18-3.21 (m, 6H), 2.82-2.79 (m, 2H), 2.74-2.71 (m, 4H), 2.60-2.55 (m, 8H), 2.25-2.20 (m, 3H), 1.90-1.88 (m, 1H), 1.88 (s, 3H), 1.75 (s, 3H), 1.61-1.59 (m, 2H). MS m/z (ESI): 898.4 [M+H]⁺.

### Example 186

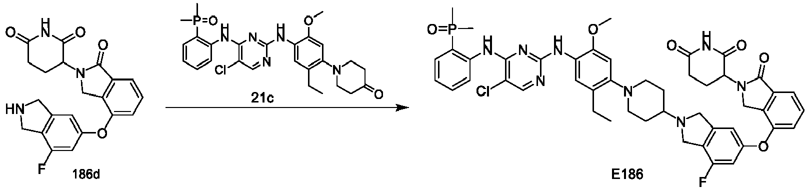

Title compound E186 (pale yellow solid, 45 mg) was prepared with reference to the method for compound E67.

¹H NMR (400 MHz, DMSO-*d*₆) δ 11.15 (s, 1H), 11.00 (s, 1H), 8.43 (br s, 1H), 8.13-8.11 (m, 2H), 7.58-7.54 (m, 3H), 7.50 (s, 1H), 7.48-7.43 (m, 1H), 7.19-7.17 (m, 2H), 6.90-6.87 (m, 2H), 6.80 (s, 1H), 5.15-5.12 (m, 1H), 4.46-4.27 (m, 2H), 3.98-3.95 (m, 4H), 3.76 (s, 3H), 3.05-2.60 (m, 5H), 2.59-2.53 (m, 4H), 2.48-2.33 (m, 1H), 2.03-1.98 (m, 3H), 1.79 (s, 3H), 1.75 (s, 3H), 1.69-1.60 (m, 2H), 1.07 (t, *J* = 7.2, 3H). MS m/z (ESI): 907.3 [M+H]⁺.

### Example 187

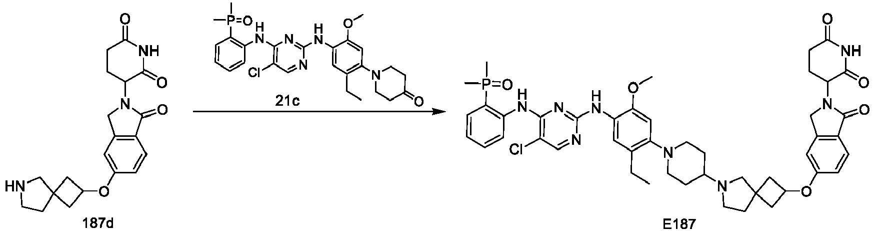

Title compound E187 (white solid, 62.46 mg) was prepared with reference to the method for compound E67. ¹H NMR (400 MHz, DMSO-*d*₆) δ 11.14(s, 1H), 10.98 (s, 1H), 8.43 (br s, 1H), 8.26-8.20 (m, 1H), 8.15-8.00 (m, 2H), 7.61-7.31 (m, 3H), 7.20-7.07 (m, 3H), 6.72 (s, 1H), 5.10-5.05 (m, 1H), 4.80-4.75 (m, 1H), 4.40-4,20 (m, 2H), 3.75 (s, 3H), 3.00-2.90 (m, 6H), 2.75-2.65 (m, 7H), 2.19-2.09 (m, 3H), 2.00-1.93 (m, 7H), 1.71-1.75 (m, 6H), 1.65-1.57 (m, 2H), 1.06 (br s, 3H). MS m/z (ESI): 881.4 [M+H]⁺.

### Example 188

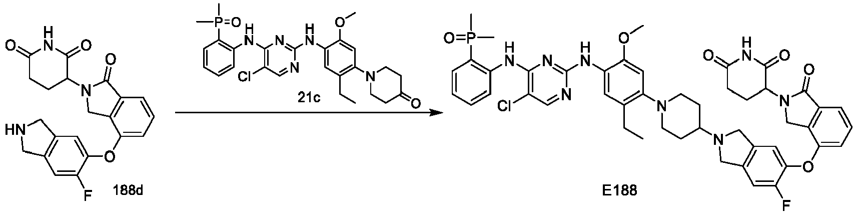

Compound E188 (white solid, 35 mg) was prepared with reference to the method for compound E67.

¹H NMR (400 MHz, DMSO-*d*₆) δ 11.16 (s, 1H), 11.01 (s, 1H), 8.44 (br s, 1H), 8.15-8.10 (m, 1H), 7.56-7.45 (m, 5H), 7.39-7.36 (m, 2H), 7.25-7.23 (m, 1H), 7.20-7.15 (m, 1H), 7.11-6.98 (m, 1H), 6.80 (s, 1H), 5.16-5.12 (m, 1H), 4.55-4.35(m, 2H), 3.95-3.90 (m, 4H), 3.78 (s, 3H), 3.05-3.02 (m, 2H), 2.95-2.85 (m, 1H), 2.56 (t, *J* =6.8, 2H), 2.55-2.53 (m, 5H), 2.03-1.99 (m, 3H), 1.79 (s, 3H), 1.75 (s, 3H), 1.67-1.60 (m, 2H), 1.07 (t, *J=* 7.6, 3H). MS m/z (ESI): 907.4 [M+H]⁺.

### Example 189

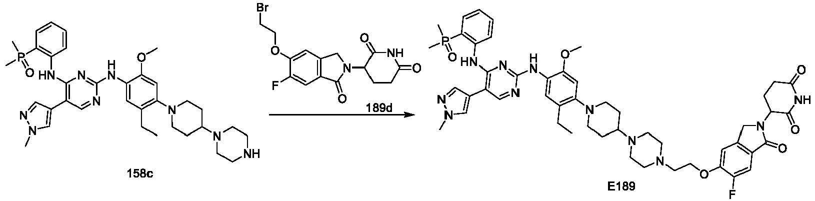

Compound E189 (pale yellow solid, 200 mg, hydrochloride) was prepared with reference to the method for compound C-1.

¹H NMR (500 MHz, DMSO-*d*₆) δ 12.44 (s, 1H), 11.22 (s, 1H), 10.99 (s, 1H), 8.20-7.92 (m, 3H), 7.68 (s, 1H), 7.64-7.57 (m, 2H), 7.53 (d, *J* = 7.2 Hz, 1H), 7.37 (s, 2H), 7.28 (t, *J* = 7.3 Hz, 1H), 6.85 (s, 1H), 5.11-5.07 (m, 1H), 4.66 (s, 2H), 4.42 (d, *J* = 17.4 Hz, 1H), 4.30 (d, *J* = 17.3 Hz, 1H), 3.96-3.59 (m, 16H), 3.42 (d, *J* = 6.4 Hz, 1H), 3.17 (d, *J* = 8.7 Hz, 2H), 2.94-2.87 (m, 1H), 2.81 (s, 2H), 2.60 (d, *J* = 17.1 Hz, 1H), 2.53 (s, 2H), 2.40 (ddd, *J* = 26.4, 13.2, 4.3 Hz, 1H), 2.24 (d, *J* = 8.6 Hz, 2H), 2.04-1.89 (m, 3H), 1.73 (s, 3H), 1.70 (s, 3H), 1.02 (s, 3H). MS m/z (ESI): 949.1 [M+H]⁺.

### Example 190

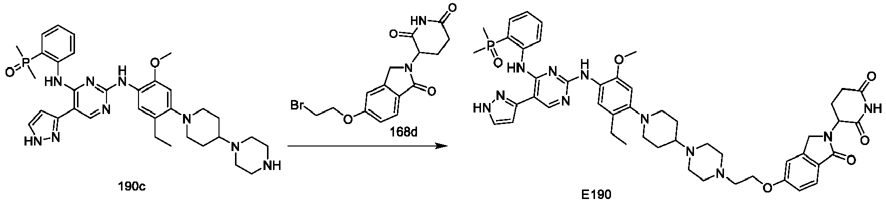

Title compound E190 (white solid, 8 mg, hydrochloride) was prepared with reference to the method for compound C-1.

¹H NMR (500 MHz, CD₃OD) δ ¹H NMR (500 MHz, MeOD) δ 8.17 (dd, *J* = 8.2, 4.2 Hz, 1H), 7.97 (s, 1H), 7.65-7.58 (m, 2H), 7.55 (ddd, *J* = 13.9, 7.7, 1.3 Hz, 1H), 7.42 (t, *J* = 7.9 Hz, 1H), 7.18 (dd, *J* = 11.3, 3.8 Hz, 1H), 6.92-6.84 (m, 2H), 6.68 (s, 1H), 5.09-4.91 (m, 1H), 4.76 (s, 3H), 4.48-4.29 (m, 2H), 3.75 (s, 3H), 3.09-2.90 (m, 4H), 2.87-2.59 (m, 7H), 2.49-2.33 (m, 7H), 2.24 (dd, *J* = 12.9, 8.5 Hz, 3H), 2.13-2.00 (m, 1H), 1.93 (t, *J* = 18.5 Hz, 2H), 1.75 (d, *J* = 13.5 Hz, 6H), 1.69-1.60 (m, 2H), 0.91 (t, *J* = 7.5 Hz, 3H). MS m/z (ESI): 917.1 [M+H]⁺.

### Example 191

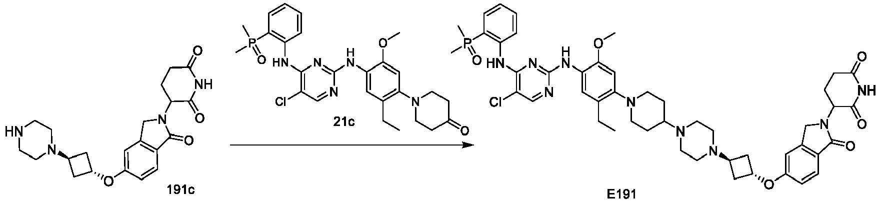

Title compound E191 (white solid, 10 mg, hydrochloride) was prepared with reference to the method for compound C-6.

¹H NMR (500 MHz, CD₃OD) δ 8.17 (dd, *J* = 8.2, 4.2 Hz, 1H), 7.97 (s, 1H), 7.64-7.58 (m, 2H), 7.55 (ddd, *J* = 13.9, 7.7, 1.3 Hz, 1H), 7.42 (t, *J* = 7.9 Hz, 1H), 7.18 (dd, *J* = 11.3, 3.8 Hz, 1H), 6.92-6.85 (m, 2H), 6.68 (s, 1H), 5.10-4.96 (m, 1H), 4.84-4.77 (m, 2H), 4.40-4.28 (m, 2H), 3.75 (s, 3H), 3.10-2.93 (m, 4H), 2.87-2.51 (m, 8H), 2.47-2.32 (m, 7H), 2.24 (dd, *J* = 12.9, 8.5 Hz, 3H), 2.15-2.01 (m, 1H), 1.95 (d, *J* = 12.9 Hz, 2H), 1.75 (d, *J* = 13.5 Hz, 6H), 1.68-1.55 (m, 2H), 0.91 (t, *J* = 7.5 Hz, 3H). MS m/z (ESI): 911.0 [M+H]⁺.

### Example 192

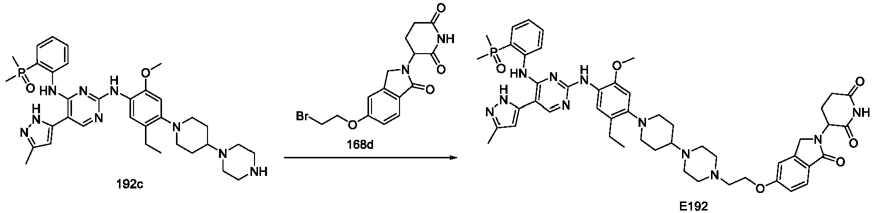

Title compound E192 (off-white solid, 7.6 mg, hydrochloride) was prepared with reference to the method for compound C-1.

¹H NMR (500 MHz, CD₃OD) δ 8.34 (s, 1H), 7.92 (dd, *J* = 10.0, 5.0 Hz, 1H), 7.65 (s, 1H), 7.64-7.55 (m, 3H), 7.40 (t, *J* = 10.0 Hz, 1H), 7.07 (s, 1H), 7.01 (d, *J* = 10.0 Hz, 1H), 6.62 (s, 1H), 6.40 (s, 1H), 5.02 (dd, *J* = 10.0, 5.0 Hz, 1H), 4.48 (s, 2H), 4.40-4.31 (m, 2H), 4.16 (t, *J* = 5.0 Hz, 2H), 3.76 (s, 3H), 2.93-2.52 (m, 16H), 2.43-2.34 (m, 2H), 2.26 (s, 3H), 2.22-2.18 (m, 2H), 2.09-2.04(m, 1H), 1.96-1.94 (m, 2H), 1.74(s, 3H), 1.71(s, 3H), 1.65-1.58(m, 2H), 0.76-0.74 (m, 3H). MS m/z (ESI): 931.0 [M+H]⁺.

### Example 193

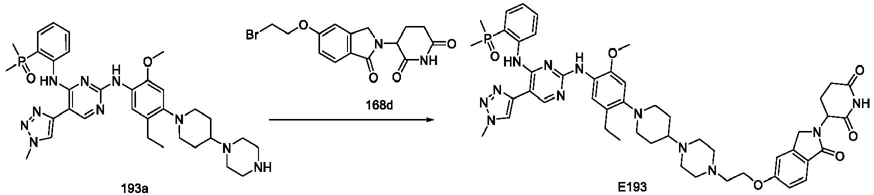

Title compound E193 (yellow solid, 15 mg, hydrochloride) was prepared with reference to the method for compound C-1.

¹H NMR (500 MHz, CD₃OD) δ 8.50 (s, 1H), 8.42 (s, 1H), 7.92-7.88 (m, 1H), 7.84 (d, *J* = 7.5 Hz, 1H), 7.79 (dd, *J* = 8.5, 2.0 Hz, 1H), 7.76-7.69 (m, 1H), 7.64-7.51 (m, 2H), 7.32 (d, *J* = 2.5 Hz, 1H), 7.29-7.17 (m, 2H), 5.15 (dd, *J* = 13.0, 5.0 Hz, 1H), 4.62 (t, *J* = 5.0 Hz, 2H), 4.58-4.44 (m, 2H), 4.24 (s, 3H), 4.04-3.89 (m, 12H), 3.59-3.46 (m, 4H), 2.99-2.78 (m, 2H), 2.64-2.35 (m, 7H), 2.22-2.17 (m, 1H), 1.87 (s, 3H), 1.84 (s, 3H), 1.03 (s, 3H). MS m/z (ESI): 932.2 [M+H]⁺.

### Example 194

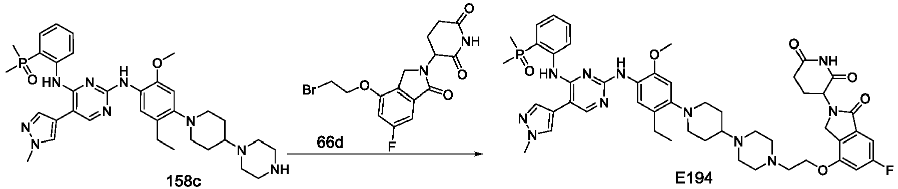

Title compound E194 (pale yellow solid, 10 mg, hydrochloride) was prepared with reference to the method for compound C-1.

¹H NMR (500 MHz, CD₃OD) δ 8.21 (s, 1H), 8.10-7.95 (m, 3H), 7.73-7.65 (m, 2H), 7.62 (t, *J* = 7.8 Hz, 1H), 7.49-7.39 (m, 2H), 7.19-7.13 (m, 2H), 5.17 (dd, *J* = 13.3, 5.1 Hz, 1H), 4.69 (d, *J* = 17.5 Hz, 1H), 4.64 (t, *J* = 4.5 Hz, 2H), 4.53 (d, *J* = 17.5 Hz, 1H), 4.08-3.96 (m, 14H), 3.92-3.75 (m, 6H), 2.98-2.88 (m, 1H), 2.82-2.54 (m, 8H), 2.24-2.16 (m, 1H), 1.82 (s, 3H), 1.79 (s, 3H), 1.05 (t, *J* = 7.1 Hz, 3H). MS m/z (ESI): 949.1 [M+H]⁺.

### Example 195

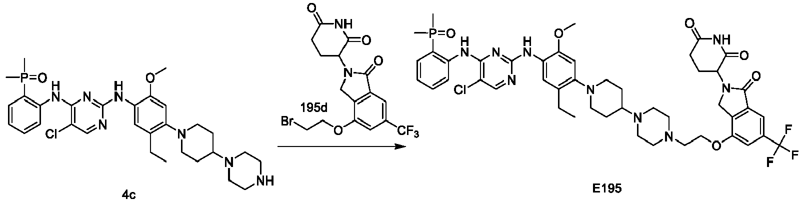

Compound E195 (pale yellow solid, 72 mg, hydrochloride) was prepared with reference to the method for compound C-1.

¹H NMR (500 MHz, DMSO-*d*₆) δ 12.51-12.43 (m, 1H), 12.14 (s, 1H), 11.04 (s, 1H), 8.35 (br s, 2H), 7.68-7.62 (m, 3H), 7.35 (br s, 2H), 7.27 (t, *J* = 7.2 Hz, 1H), 6.86 (s, 1H), 5.16 (dd, *J* = 13.3, 5.1 Hz, 1H), 4.84-4.65 (m, 3H), 4.46 (d, *J* = 18.4 Hz, 1H), 3.97-3.66 (m, 13H), 3.50-3.38 (m, 1H), 3.19 (d, *J* = 6.5 Hz, 2H), 3.00-2.90 (m, 1H), 2.81 (d, *J* = 18.8 Hz, 2H), 2.66-2.53 (m, 3H), 2.41 (ddd, *J* = 27.0, 13.6, 4.8 Hz, 1H), 2.25 (d, *J* = 8.9 Hz, 2H), 2.09-1.90 (m, 3H), 1.81 (s, 3H), 1.79 (s, 3H), 1.07 (t, *J=* 7.2 Hz, 3H). MS m/z (ESI): 953.3 [M+H]⁺.

### Example 196

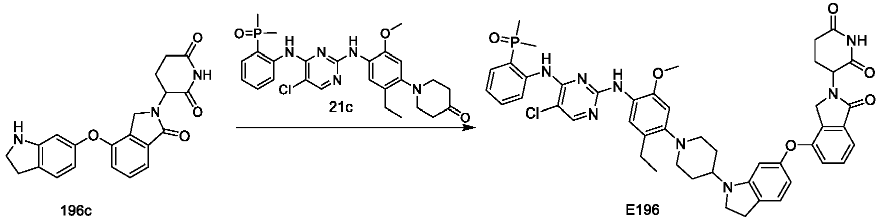

Title compound E196 (white solid, 7.2 mg, hydrochloride) was prepared with reference to the method for compound C-6.

¹H NMR (400 MHz, DMSO-*d*₆) δ 2.24-11.92 (m, 1H), 10.99 (s, 1H), 9.97-9.70 (m, 1H), 8.50-8.13 (m, 2H), 7.72-7.60 (m, 1H), 7.57-7.36 (m, 4H), 7.31-7.17 (m, 2H), 7.05 (br dd, *J* = 7.8, 11.3 Hz, 2H), 6.41 (br s, 1H), 6.29 (br d, *J* = 7.8 Hz, 1H), 5.12 (br dd, *J* = 4.8, 13.3 Hz, 1H), 3.82 (s, 1H), 3.94-3.73 (m, 1H), 3.98-3.65 (m, 1H), 3.52 (br t, *J* = 7.9 Hz, 2H), 3.39-3.30 (m, 1H), 3.42-3.26 (m, 3H), 3.00-2.84 (m, 3H), 2.72 (br s, 2H), 2.53-2.52 (m, 1H), 2.58 (br d, *J* = 17.1 Hz, 1H), 2.47-2.38 (m, 2H), 2.48-2.30 (m, 2H), 2.04-1.95 (m, 1H), 1.86 (br d, *J* = 10.8 Hz, 2H), 1.81 (s, 3H), 1.78 (s, 3H), 1.46-1.45 (m, 1H), 1.05 (t, *J* = 6.9 Hz, 3H);MS m/z (ESI): 889.3 [M+H]⁺.

### Example 197

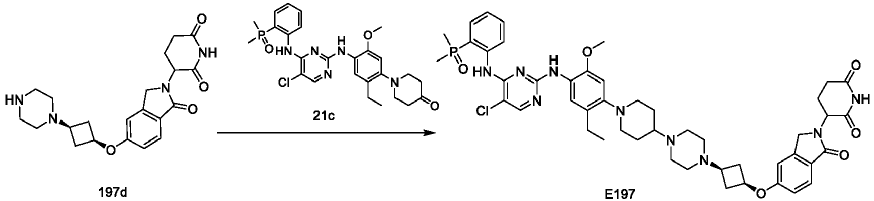

Title compound E197 (white solid, 4 mg, hydrochloride) was prepared with reference to the method for compound E67.

¹H NMR (500 MHz, CD₃OD) δ 8.25-8.19 (m, 2H), 7.81-7.69 (m, 2H), 7.58 (s, 1H), 7.44 (t, *J* = 7.1 Hz, 1H), 7.35 (s, 1H), 7.12 (s, 1H), 7.07 (dd, *J* = 8.4, 2.0 Hz, 1H), 6.99 (s, 1H), 5.15 (dd, *J* = 13.3, 5.2 Hz, 1H), 4.75 (dd, *J* = 13.7, 6.9 Hz, 1H), 4.54-4.41 (m, 2H), 4.08-3.50 (m, 15H), 3.13 (ddd, *J* = 57.2, 38.5, 9.1 Hz, 4H), 2.93 (ddd, *J* = 16.6, 11.1, 4.3 Hz, 1H), 2.85-2.76 (m, 1H), 2.65 (d, *J* = 6.7 Hz, 4H), 2.47-2.26 (m, 3H), 2.11 (dd, *J* = 28.7, 23.6 Hz, 3H), 1.79 (d, *J* = 13.5 Hz, 6H), 1.02 (s, 3H). MS m/z (ESI): 910.6 [M+H]⁺.

### Example 198

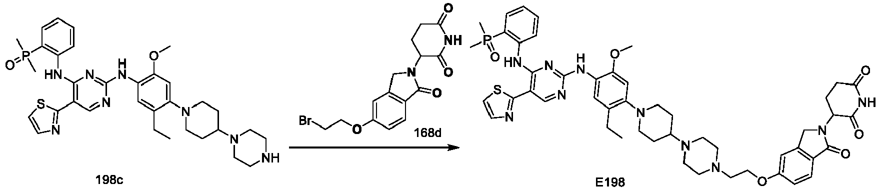

Title compound E198 (white solid, 10 mg, hydrochloride) was prepared with reference to the method for compound C-1.

¹H NMR (500 MHz, CD₃OD) δ 7.88 (d, *J* = 3.3 Hz, 1H), 7.81 (dd, *J* = 13.2, 7.8 Hz, 1H), 7.69-7.58 (m, 4H), 7.49 (s, 1H), 7.17 (s, 1H), 7.11 (dd, *J* = 8.4, 2.2 Hz, 1H), 6.78 (s, 1H), 5.04 (dd, *J* = 13.3, 5.2 Hz, 1H), 4.44 (d, *J* = 10.7 Hz, 2H), 4.36 (dd, *J* = 17.7, 9.9 Hz, 2H), 3.97-3.48 (m, 14H), 3.13 (s, 2H), 2.87-2.71 (m, 3H), 2.35 (ddd, *J* = 84.9, 44.7, 9.9 Hz, 6H), 2.07 (dd, *J* = 6.6, 3.7 Hz, 1H), 1.94 (d, *J* = 10.0 Hz, 2H), 1.73 (d, *J* = 13.4 Hz, 6H), 0.88 (s, 3H). MS m/z (ESI): 933.6 [M+H]⁺.

### Example 199

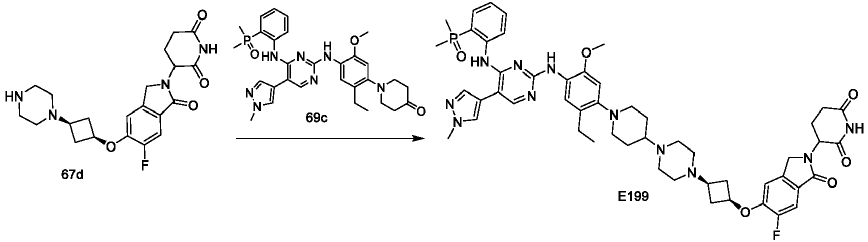

Title compound E199 (white solid, 4 mg, hydrochloride) was prepared with reference to the method for compound C-6.

¹H NMR (500 MHz, CD₃OD) δ 8.13 (s, 1H), 8.03 (s, 1H), 7.84 (s, 1H), 7.77 (s, 1H), 7.68 (dd, *J* = 14.3, 7.4 Hz, 1H), 7.56 (t, *J* = 14.3 Hz, 2H), 7.44 (dd, *J* = 18.6, 11.1 Hz, 2H), 7.28 (d, *J* = 7.0 Hz, 1H), 7.08 (s, 1H), 5.14 (dd, *J* = 13.3, 5.2 Hz, 1H), 4.84-4.77 (m, 1H), 4.47 (q, *J* = 16.8 Hz, 2H), 4.01 (s, 3H), 3.86 (d, *J* = 76.6 Hz, 12H), 3.51-3.42 (m, 3H), 3.28-3.12 (m, 4H), 2.93 (ddd, *J* = 18.9, 13.7, 5.5 Hz, 1H), 2.84-2.76 (m, 1H), 2.75-2.58 (m, 4H), 2.56-2.48 (m, 1H), 2.43 (d, *J* = 10.1 Hz, 2H), 2.27 (s, 2H), 2.19 (dd, *J* = 9.0, 3.7 Hz, 1H), 1.81 (d, *J* = 13.5 Hz, 6H), 1.10 (s, 3H).MS m/z (ESI): 975.1 [M+H]⁺.

### Example 200

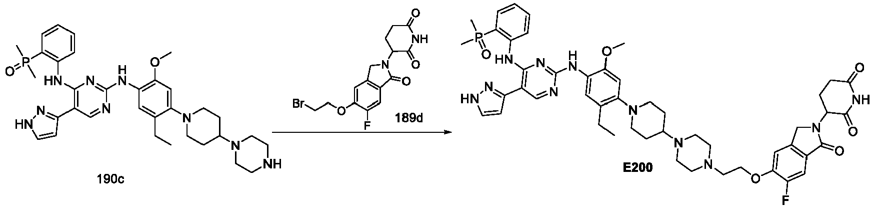

Title compound E200 (white solid, 8 mg, hydrochloride) was prepared with reference to the method for compound C-1.

¹H NMR (500 MHz, CD₃OD) δ 8.35 (s, 1H), 7.85 (dd, *J* = 13.0, 7.7 Hz, 1H), 7.75 (d, *J* = 2.6 Hz, 1H), 7.65-7.57 (m, 2H), 7.56-7.42 (m, 2H), 7.39-7.27 (m, 2H), 6.85 (s, 1H), 6.75 (d, *J* = 2.5 Hz, 1H), 5.03 (dd, *J* = 13.3, 5.2 Hz, 1H), 4.67-4.50 (m, 2H), 4.46-4.24 (m, 2H), 4.00-3.62 (m, 12H), 3.54-3.45 (m, 1H), 3.23-3.21 (m, 3H), 3.04-2.88 (m, 2H), 2.87-2.63 (m, 2H), 2.5-2.30 (m, 3H), 2.26 (d, *J* = 11.8 Hz, 2H), 2.13-1.96 (m, 3H), 1.73 (d, *J* = 13.4 Hz, 6H), 0.89 (s, 3H). MS m/z (ESI): 935.1 [M+H]⁺.

### Example 201

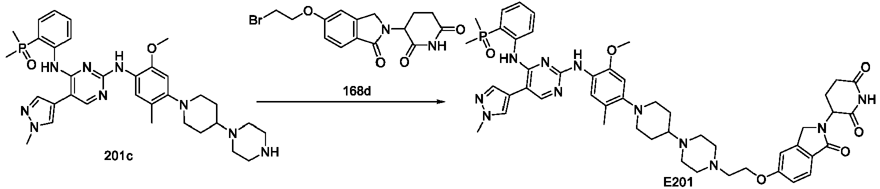

Title compound E201 (white solid, 5 mg, hydrochloride) was prepared with reference to the method for compound C-1.

¹H NMR (500 MHz, CD₃OD) δ 8.29 (dd, *J* = 10.0, 5.0 Hz, 1H), 8.15 (s, 1H), 8.02 (s, 1H), 7.94 (s, 1H), 7.88 (d, *J* = 5.0 Hz, 1H), 7.81 (s, 1H), 7.78-7.69 (m, 2H), 7.42 (t, *J* = 10.0 Hz, 1H), 7.32 (s, 1H), 7.26 (dd, *J* = 20.0, 5.0 Hz, 1H), 6.87 (s, 1H), 5.29 (dd, *J* = 10.0, 5.0 Hz, 1H), 4.65-4.56 (m, 2H), 4.41 (t, *J* = 5.0 Hz, 2H), 4.11 (s, 3H), 4.02 (s, 3H), 3.28 (d, *J* = 10.0 Hz, 2H), 3.07-2, 80 (m, 14H), 2.68-2.59 (m, 2H), 2.34-2.29 (m, 1H), 2.20-2.18 (m, 5H), 1.94(s, 3H), 1.92(s, 3H), 1.91-1.83(m, 2H). MS m/z (ESI): 917.0 [M+H]⁺.

### Example 202

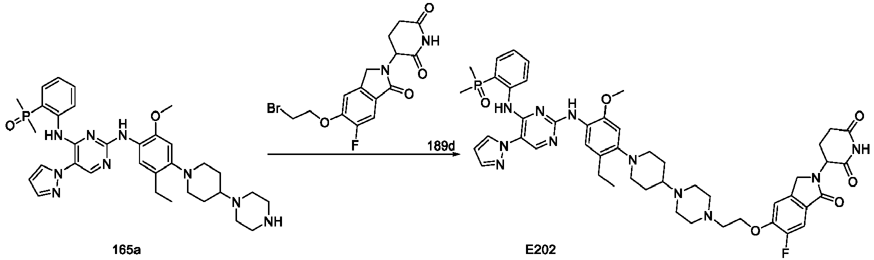

Title compound E202 (yellow solid, 30 mg, hydrochloride) was prepared with reference to the method for compound C-1.

¹H NMR (500 MHz, CD₃OD) δ 8.20 (s, 1H), 8.07 (d, *J* = 2.5 Hz, 1H), 7.90(s, 1H), 7.88 (d, *J* = 2.0 Hz, 1H), 7.78-7.73 (m, 1H), 7.57-7.48 (m, 2H), 7.47-7.46 (m, 2H), 7.44 (s, 1H), 6.92 (s, 1H), 6.62 (t, *J* = 2.0 Hz, 1H), 5.13 (dd, *J* = 8.5, 5.5 Hz, 1H), 4.63 (t, *J* = 4.5 Hz, 2H), 4.48 (dd, *J* = 27.5, 17.0 Hz, 2H), 4.04-3.45 (m, 14H), 3.33-3.27 (m, 2H), 2.95-2.87 (m, 3H), 2.81-2.77 (m, 1H), 2.56-2.47 (m, 3H), 2.33 (d, *J* = 11.5 Hz, 2H), 2.19-2.16 (m, 1H), 2.07-2.03 (m, 2H), 1.81 (s, 3H), 1.78 (s, 3H), 1.09 (s, 3H). MS m/z (ESI): 935.2 [M+H]⁺.

### Example 203

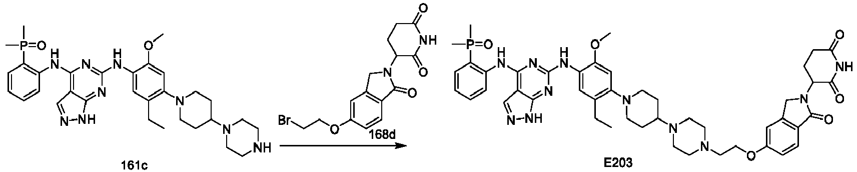

Compound E203 (white solid, 25 mg, hydrochloride) was prepared with reference to the method for reference compound C-1.

¹H NMR (500 MHz, DMSO-*d*₆) δ 13.04-11.92 (m, 2H), 10.98 (s, 1H), 8.53-8.21 (m, 1H), 8.17-7.97 (m, 1H), 7.75-7.66 (m, 3H), 7.43 (br s, 2H), 7.27 (s, 2H), 7.17 (dd, *J* = 8.4, 2.0 Hz, 1H), 6.84 (s, 1H), 5.09 (dd, *J* = 13.3, 5.0 Hz, 1H), 4.53 (s, 2H), 4.42 (d, *J* = 17.3 Hz, 1H), 4.30 (d, *J* = 17.3 Hz, 1H), 3.80 (s, 5H), 3.70-3.56 (m, 7H), 3.42 (d, *J* = 6.5 Hz, 1H), 3.18 (s, 2H), 2.97-2.86 (m, 1H), 2.81 (s, 2H), 2.62 (dd, *J* = 17.4, 7.3 Hz, 4H), 2.44-2.34 (m, 1H), 2.24 (s, 2H), 2.03-1.89 (m, 3H), 1.84 (d, *J* = 13.7 Hz, 6H), 1.12 (s, 3H). MS m/z (ESI): 891.0 [M+H]⁺.

### Example 204

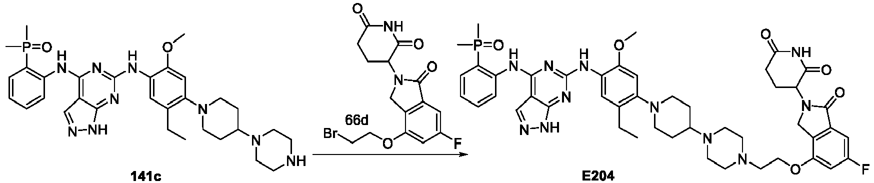

Title compound E204 (pale yellow solid, 8.2 mg, hydrochloride) was prepared with reference to the method for compound C-1.

¹H NMR (500 MHz, CD₃OD) δ 8.31 (s, 2H), 7.62 (s, 1H), 7.38 (d, *J* = 60.6 Hz, 3H), 7.12-7.04 (m, 2H), 6.94 (s, 1H), 5.07 (dd, *J* = 13.3, 5.1 Hz, 1H), 4.59 (d, *J* = 17.5 Hz, 1H), 4.53 (t, *J* = 4.3 Hz, 2H), 4.43 (d, *J* = 17.5 Hz, 1H), 4.01-3.72 (m, 13H), 3.64-3.54 (m, 1H), 3.38-3.28 (m, 2H), 3.15-2.96 (m, 2H), 2.92-2.78 (m, 1H), 2.76-2.66 (m, 1H), 2.63-2.53 (m, 2H), 2.52-2.42 (m, 1H), 2.32 (d, *J* = 10.1 Hz, 2H), 2.19-2.05 (m, 3H), 1.81 (d, *J* = 13.5 Hz, 6H), 1.07 (t, *J* = 7.2 Hz, 3H). MS m/z (ESI): 909.0 [M+H]⁺.

### Example 205

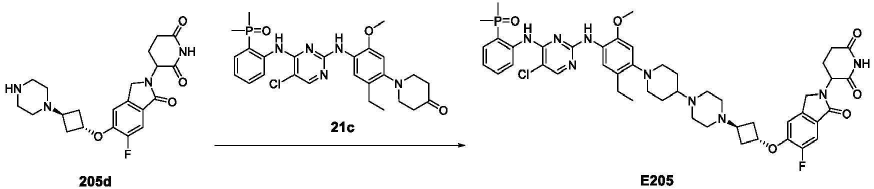

Title product E205 (pale yellow solid, 3.6 mg, hydrochloride) was prepared with reference to the synthesis method for compound E67.

¹H NMR (500 MHz, CD₃OD) δ 8.50-8.02 (m, 2H), 7.73 (dd, *J=* 13.8, 7.6 Hz, 1H), 7.64-7.52 (m, 2H), 7.45 (t, *J* = 7.3 Hz, 1H), 7.41-7.31 (m, 1H), 7.20 (d, *J* = 7.0 Hz, 1H), 7.04 (s, 1H), 5.24-5.17 (m, 1H), 5.14 (dd, *J* = 13.3, 5.1 Hz, 1H), 4.47 (q, *J* = 17.1 Hz, 2H), 4.32-4.21 (m, 1H), 4.04-3.62 (m, 12H), 3.47-3.37 (m, 2H), 3.21-3.07 (m, 4H), 2.98-2.87 (m, 1H), 2.84-2.72 (m, 3H), 2.66 (d, *J* = 7.3 Hz, 2H), 2.55-2.38 (m, 3H), 2.28-2.16 (m, 3H), 1.91 (d, *J=* 13.5 Hz, 6H), 1.15 (t, *J* = 7.3 Hz, 3H). MS m/z (ESI): 929.1 [M+H]⁺.

### Example 206

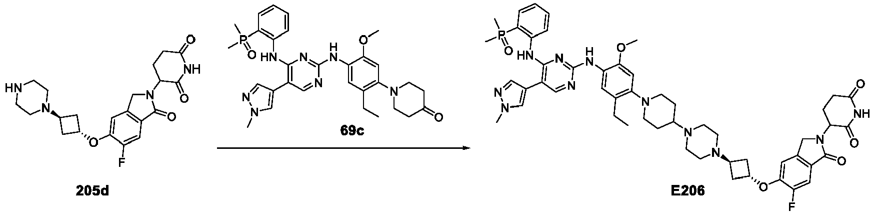

Title product E206 (pale yellow solid, 6.2 mg, hydrochloride) was prepared with reference to the synthesis method for compound E67.

¹H NMR (500 MHz, CD₃OD) δ 8.16 (s, 1H), 8.08 (s, 1H), 7.96 (d, *J* = 10.3 Hz, 1H), 7.95 (s, 1H), 7.74-7.60 (m, 3H), 7.53 (d, *J* = 9.6 Hz, 1H), 7.47 (t, *J* = 7.0 Hz, 1H), 7.38 (s, 1H), 7.21 (d, *J* = 7.0 Hz, 1H), 5.19 (t, *J* = 6.9 Hz, 1H), 5.14 (dd, *J* = 13.3, 5.1 Hz, 1H), 4.53-4.42 (m, 2H), 4.32-4.23 (m, 1H), 4.06 (s, 3H), 4.04-3.70 (m, 15H), 3.19-3.09 (m, 2H), 2.98-2.88 (m, 1H), 2.83-2.55 (m, 9H), 2.55-2.45 (m, 1H), 2.23-2.15 (m, 1H), 1.82 (d, *J* = 13.5 Hz, 6H), 1.07 (t, *J* = 7.1 Hz, 3H). MS m/z (ESI): 974.5 [M+H]⁺.

### Example 207

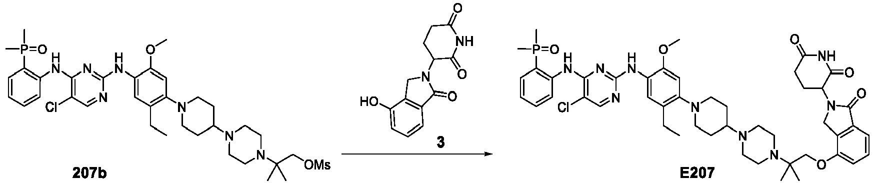

Title compound E207 (white solid, 4.0 mg) was prepared with reference to the synthesis method for compound E111.

¹H NMR (500 MHz, CD₃OD) δ 8.39-8.19 (m, 1H), 8.16 (s, 1H), 7.77-7.70 (m, 1H), 7.62 (dd, *J* = 7.1, 1.2 Hz, 1H), 7.60-7.52 (m, 3H), 7.45 (t, *J* = 7.1 Hz, 1H), 7.40 (s, 1H), 7.08 (s, 1H), 5.20 (dd, *J* = 13.3, 5.1 Hz, 1H), 4.69 (d, *J* = 17.4 Hz, 1H), 4.57 (d, *J* = 17.4 Hz, 1H), 4.15-3.97 (m, 8H), 3.92 (s, 3H), 3.80-3.70 (m, 3H), 3.46 (dd, *J* = 9.8, 6.0 Hz, 2H), 3.29-3.16 (m, 2H), 2.99-2.91 (m, 1H), 2.86-2.80 (m, 1H), 2.70-2.63 (m, 3H), 2.45 (d, *J* = 10.7 Hz, 2H), 2.31-2.19 (m, 3H), 1.92 (s, 3H), 1.89 (s, 3H), 1.68 (s, 3H), 1.66 (s, 3H), 1.14 (t, *J* =7.2 Hz, 3H). MS m/z (ESI): 912.6 [M+H]+.

### Example 208

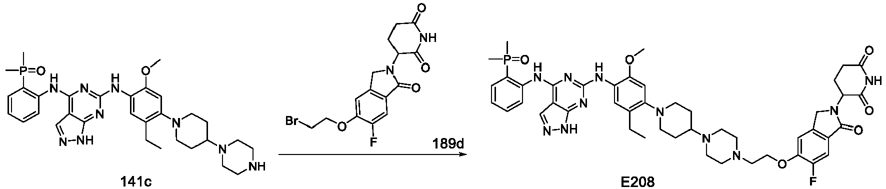

Title compound E208 (pale yellow solid, 10 mg, hydrochloride) was prepared with reference to the method for compound C-1.

¹H NMR (500 MHz, CD₃OD) δ 8.43 (s, 2H), 7.79-7.69 (m, 1H), 7.61-7.41 (m, 5H), 7.17 (s, 1H), 5.16 (dd, *J* = 13.3, 5.1 Hz, 1H), 4.74-4.70 (m, 2H), 4.50 (q, *J =* 17.1 Hz, 2H), 4.14-3.75 (m, 16H), 3.60-3.50 (m, 2H), 2.96-2.88 (m, 1H), 2.83-2.78 (m, 1H), 2.78-2.68 (m, 2H), 2.54-2.46 (m, 3H), 2.40-2.30 (m, 2H), 2.24-2.17 (m, 1H), 1.94 (s, 3H), 1.91 (s, 3H), 1.19 (t, *J* = 7.2 Hz, 3H). MS m/z (ESI): 909.0 [M+H]⁺.

### Example 209

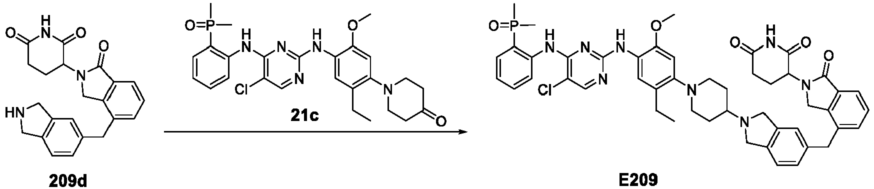

Target product E209 (pale yellow solid, 15 mg, hydrochloride) was prepared with reference to the synthesis method for compound E67.

¹H NMR (500 MHz, CD₃OD) δ 8.22 (s, 2H), 7.79-7.70 (m, 2H), 7.62 (t, *J* = 7.7 Hz, 1H), 7.56-7.45 (m, 4H), 7.41 (d, *J* = 7.9 Hz, 1H), 7.38-7.27 (m, 3H), 5.16 (dd, *J* = 13.3, 5.2 Hz, 1H), 5.01-4.92 (m, 2H), 4.76 (d, *J* = 12.8 Hz, 2H), 4.42-4.38 (m, 2H), 4.20 (s, 2H), 4.07-3.93 (m, 4H), 3.97 (s, 3H), 3.78-3.46 (m, 4H), 2.96-2.86 (m, 1H), 2.83-2.74 (m, 1H), 2.72 (q, *J* = 7.3 Hz, 2H), 2.58-2.38 (m, 5H), 2.21-2.13 (m, 1H), 1.90 (d, *J* = 13.6 Hz, 6H), 1.12 (t, *J* = 6.8 Hz, 3H). MS m/z (ESI): 887.4 [M+H]⁺.

### Example 210

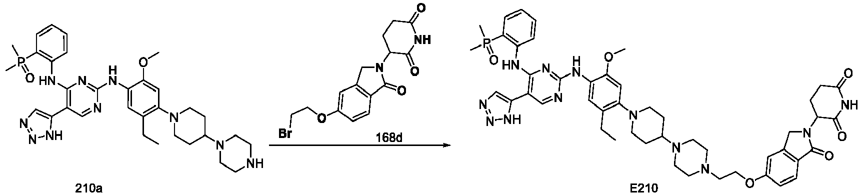

Title compound E210 (yellow solid, 1.5 mg, hydrochloride) was prepared with reference to the method for compound C-1.

¹H NMR (500 MHz, CD₃OD) δ 8.47 (s, 1H), 8.37 (s, 1H), 7.87 (dd, *J* = 13.0, 8.0 Hz, 2H), 7.78 (d, *J* = 8.5 Hz, 1H), 7.71-7.69 (m, 1H), 7.57 (t, *J* = 7.5 Hz, 1H), 7.43 (s, 1H), 7.30 (d, *J* = 2.0 Hz, 1H), 7.24 (dd, *J* = 8.5, 2.5 Hz, 1H), 7.01 (s, 1H), 5.14 (dd, *J* = 13.5, 5.0 Hz, 1H), 4.59 (t, *J* = 4.5 Hz, 2H), 4.48 (dd, *J* = 27.5, 17.0 Hz, 2H), 3.97-3.85 (m, 14H), 3.38-3.32 (m, 2H), 3.18-3.14(m, 2H), 2.84-2.80 (m, 2H), 2.52-2.40 (m, 5H), 2.21-2.18 (m, 3H), 1.85 (s, 3H), 1.82 (s, 3H), 1.02 (s, 3H). MS m/z (ESI): 917.9 [M+H]⁺.

### Example 211

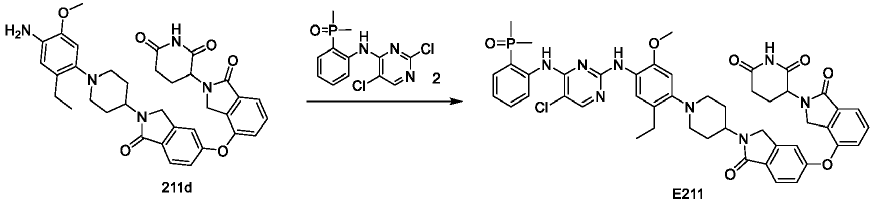

Compound **2** (10.14 mg, 32.07 µmol) and p-toluenesulfonic acid (5.52 mg, 32.07 µmol) were added to a solution of compound 211d (20 mg, 32.07 µmol) in ethylene glycol (0.5 mL). The reaction system was then reacted at 80 °C for 10 h. The reaction system was directly separated by preparative chromatography (formic acid system) to give title compound E211 (yellow solid, 2.6 mg). ¹H NMR (400 M, DMSO-*d*6) δ 11.16 (s, 1H), 10.99 (s, 1H), 8.46-8.43 (m, 1H), 8.15-8.11 (m, 2H), 7.67 (d, *J* = 8.0 Hz, 1H), 7.61-7.52 (m, 3H), 7.49-7.46 (m, 1H), 7.40-7.32 (m, 2H), 7.28-7.25 (m, 1H), 7.20-7.01 (m, 2H), 6.88 (s, 1H), 5.12 (dd, *J* = 13.2, 5.2 Hz, 1H), 4.58-4.11 (m, 5H), 3.78 (s, 4H), 3.11-3.08 (m, 2H), 3.00-2.87 (m, 3H), 2.04-1.95 (m, 3H), 1.87-1.80 (m, 3H), 1.77 (d, *J=* 13.2 Hz, 6H), 1.68-1.65 (m, 2H), 1.09 (t, *J=* 7.6 Hz, 3H). MS m/z (ESI): 903.7 [M+H]⁺.

### Example 212

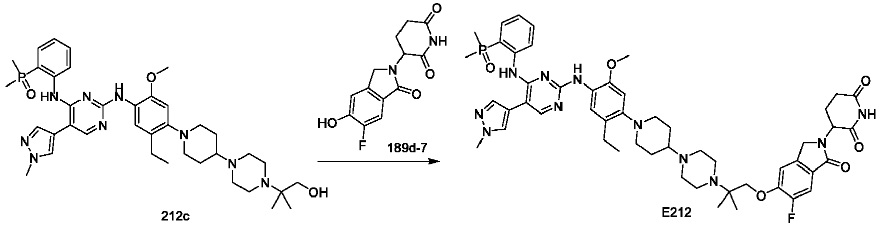

Compound 212c (135 mg, 188.58 µmol) was dissolved in THF (2 mL) before PPh₃ (80.00 mg, 3.05 µmol) and DEAD (45.00 mg, 258 µmol) were added. The system was stirred for 1 min before compound 189d-7 (50.00 mg, 179.7 µmol) was added, and then stirred overnight at 45 °C in a N₂ atmosphere, and concentrated at reduced pressure. The crude product was separated by column chromatography and then subjected to prep-HPLC to give compound E212 (white solid, 4.4 mg). ¹H NMR (400 MHz, DMSO-*d*₆) δ 11.01 (s, 1H), 10.16 (s, 1H), 8.22-8.15 (m, 1H), 8.07 (s, 1H), 8.01 (s, 1H), 7.74-7.66 (m, 3H), 7.56-7.48 (m, 3H), 7.40 (t, *J* = 7.3Hz, 1H), 7.10 (t, *J* = 7.0 Hz, 1H), 6.75 (s, 1H), 5.10 (dd, *J* = 12.8, 4.3Hz, 1H), 4.43-4.26 (m, 2H), 3.84 (s, 3H), 3.80 (s, 3H), 3.04-2.84 (m, 5H), 2.66-2.57 (m, 8H), 2.55-2.33 (m, 6H), 2.04-1.97 (m, 2H), 1.90-1.81 (m, 2H), 1.71 (s, 3H), 1.69 (s, 3H), 1.61-1.48 (m, 2H), 1.32 (s, 6H), 0.88-1.07 (m, 3H). MS m/z (ESI): 976.5 [M+H]⁺.

### Example 213

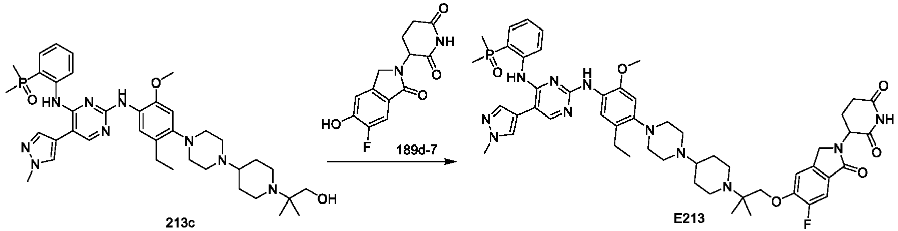

Compound E213 (white solid, 1.0 mg) was prepared with reference to the method of Example 212.

¹H NMR (400 MHz, DMSO-*d*₆) δ 10.99 (brs, 1H), 10.16 (s, 1H), 8.21-8.16 (m, 1H), 8.07 (s, 1H), 8.01 (s, 1H), 7.74 (s, 1H), 7.69 (s, 1H), 7.66 (s, 1H), 7.53-7.48 (m, 3H), 7.41-7.37 (m, 1H), 7.12-7.09 (m, 1H), 6.75 (s, 1H), 5.06-5.12 (m, 1H), 4.42-4.24 (m, 2H), 4.03 (br s, 2H), 3.84 (s, 3H), 3.79 (s, 3H), 2.93-3.00 (m, 5H), 2.67-2.62 (m, 8H), 2.44-2.24 (m, 5H), 2.01-1.98 (m, 1H), 1.86-1.82 (m, 2H), 1.72 (s, 3H), 1.69 (s, 3H), 1.24 (s, 2H), 1.15 (s, 6H), 0.88-1.07 (m, 3H). MS m/z (ESI): 976.5 [M+H]⁺.

### Example 214

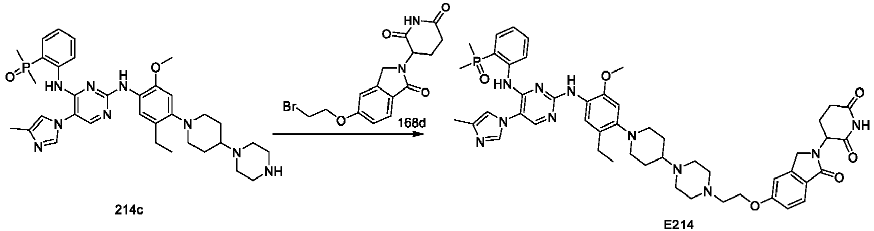

Title compound E214 (white solid, 10 mg, hydrochloride) was prepared with reference to the method for reference compound C-1.

¹H NMR (500 MHz, CD₃OD) δ 8.01 (s, 1H), 7.90 (s, 1H), 7.64-7.62 (m, 2H), 7.51 (dd, *J* = 14.3, 8.2 Hz, 1H), 7.42 (t, *J* = 7.8 Hz, 1H), 7.18 (t, *J* = 7.2 Hz, 1H), 7.07 (s, 1H), 7.01 (dd, *J* = 8.5, 2.2 Hz, 1H), 6.91 (s, 1H), 6.68 (s, 1H), 5.10-4.99 (m, 2H), 4.36 (q, *J* = 17.2 Hz, 2H), 4.18 (s, 2H), 3.76 (d, *J* = 4.4 Hz, 3H), 3.35 (t, *J* = 7.1 Hz, 1H), 3.02 (d, *J* = 11.6 Hz, 4H), 2.93-2.75 (m, 6H), 2.74-2.64 (m, 5H), 2.38 (dd, *J* = 13.0, 5.1 Hz, 3H), 2.17 (s, 3H), 2.11-2.02 (m, 3H), 1.72 (s, 2H), 1.64 (d, *J* = 13.5 Hz, 6H), 0.89 (t, *J* = 7.5 Hz, 3H). MS m/z (ESI): 931.0 [M+H]⁺.

### Example 215

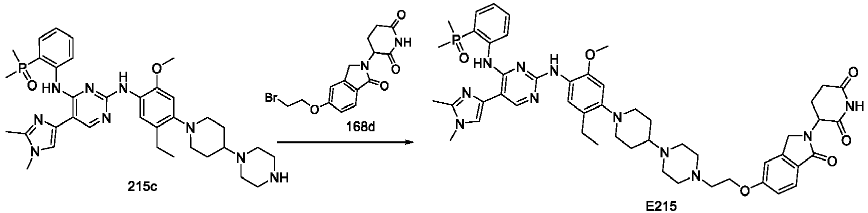

Title compound E215 (white solid, 4.4 mg, hydrochloride) was prepared with reference to the method for reference compound C-1.

¹H NMR (500 MHz, CD₃OD) δ 8.24-8.16 (m, 2H), 7.81 (s, 1H), 7.79 (d, *J* = 10.0 Hz, 1H), 7.69 (dd, *J* = 15.0, 5.0 Hz, 1H), 7.61-7.56 (m, 2H), 7.45 (t, *J* = 7.5 Hz, 1H), 7.33 (s, 1H), 7.24 (dd, *J* = 20.0, 5.0 Hz, 1H), 5.15 (dd, *J* = 10.0, 5.0 Hz, 1H), 4.63-4.46 (m, 4H), 4.04-3.90 (m, 16H), 3.68-3.62 (m, 4H), 2.97-2.66(m, 8H), 2.58-2.47(m, 5H), 2.21-2.18(m, 1H), 1.87 (s, 3H), 1.84 (s, 3H), 1.22-1.10 (m, 3H). MS m/z (ESI): 944.5 [M+H]⁺.

### Example 216

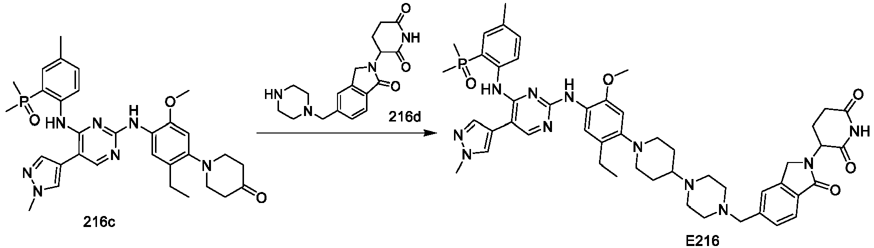

Title compound E216 (white solid, 4 mg, hydrochloride) was prepared with reference to the method for compound E67.

¹H NMR (500 MHz, CD₃OD) δ 8.01 (s, 1H), 7.95 (d, *J* = 7.7 Hz, 3H), 7.83 (d, *J* = 8.2 Hz, 2H), 7.74 (s, 1H), 7.50 (d, *J* = 12.9 Hz, 1H), 7.41 (d, *J* = 7.5 Hz, 2H), 7.00 (s, 1H), 5.20 (dd, *J* = 13.3, 5.1 Hz, 1H), 4.61 (dd, *J* = 30.7, 18.1 Hz, 4H), 4.09-3.58 (m, 17H), 3.10 (s, 2H), 2.96 (d, *J* = 15.4 Hz, 1H), 2.83 (d, *J* = 2.4 Hz, 1H), 2.56 (dd, *J* = 13.0, 8.5 Hz, 3H), 2.43 (s, 3H), 2.38 (d, *J* = 11.9 Hz, 2H), 2.24-2.09 (m, 3H), 1.80 (s, 3H), 1.78 (s, 3H), 1.06 (s, 3H). MS m/z (ESI): 915.0 [M+H]⁺.

### Example 217

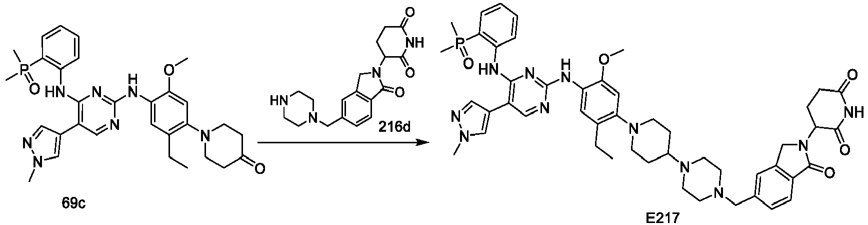

Title compound E217 (white solid, 4 mg, hydrochloride) was prepared with reference to the method for compound E67.

¹H NMR (500 MHz, CD₃OD) δ 8.01 (s, 1H), 7.89 (s, 1H), 7.87-7.81 (m, 2H), 7.70 (s, 2H), 7.63 (s, 1H), 7.55 (dd, *J* = 12.9, 7.8 Hz, 1H), 7.46 (s, 1H), 7.35-7.23 (m, 2H), 6.91 (s, 1H), 5.09 (dd, *J* = 13.4, 5.2 Hz, 1H), 4.63-4.19 (m, 4H), 3.90 (d, *J* = 16.2 Hz, 3H), 3.84-3.44 (m, 12H), 3.26 (s, 2H), 3.06 (dd, *J* = 14.1, 12.4 Hz, 2H), 2.89-2.64 (m, 2H), 2.52-2.38 (m, 3H), 2.27 (d, *J* = 11.6 Hz, 2H), 2.10 (dd, *J* = 9.1, 3.8 Hz, 3H), 1.69 (d, *J* = 13.5 Hz, 6H), 0.97 (s, 3H). MS m/z (ESI): 901.0 [M+H]⁺.

### Example 218

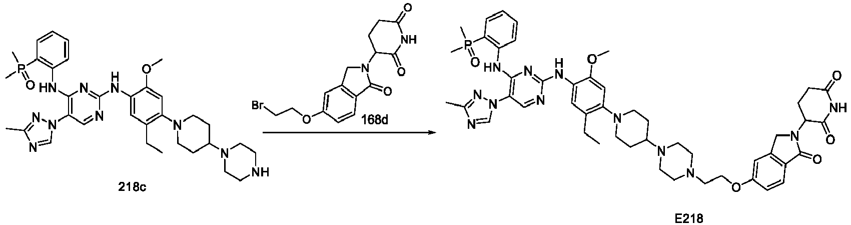

Title compound E218 (white solid, 10 mg, hydrochloride) was prepared with reference to the method for reference compound C-1.

¹H NMR (500 MHz, DMSO-*d*₆) δ 12.47 (s, 1H), 11.40 (s, 1H), 10.98 (s, 1H), 9.92 (s, 1H), 8.76 (s, 1H), 8.50-7.92 (m, 2H), 7.69 (d, *J* = 8.5 Hz, 1H), 7.61 (dd, *J* = 13.6, 7.9 Hz, 1H), 7.51-7.21 (m, 4H), 7.18 (dd, *J* = 8.4, 2.2 Hz, 1H), 6.88 (s, 1H), 5.12-5.08 (m, 1H), 4.58-4.53 (m, 2H), 4.43 (d, *J* = 17.3 Hz, 1H), 4.31 (d, *J* = 17.3 Hz, 1H), 3.99-3.65 (m, 13H), 3.51-3.34 (m, 1H), 3.27-3.11 (3, 2H), 3.01-2.73 (m, 3H), 2.65-2.53 (m, 3H), 2.45-2.36 (m, 1H), 2.34 (s, 3H), 2.29-2.21 (m, 2H), 2.09-1.90 (m, 3H), 1.70 (d, *J=* 13.6 Hz, 6H), 1.08 (d, *J* = 8.7 Hz, 3H). MS m/z (ESI): 932.1 [M+H]⁺.

### Example 219

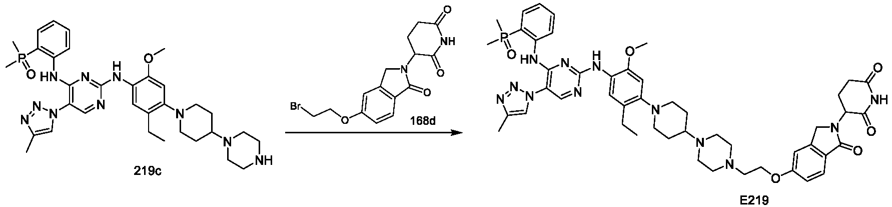

Compound E219 (white solid, 3.71 mg) was prepared with reference to the method for reference compound C-1.

¹H NMR (400 MHz, DMSO-*d*₆) δ 10.99 (s, 1H), 10.46 (s, 1H), 8.29-8.50 (m, 2H), 8.08 (s, 1H), 8.05 (s, 1H), 7.64 (d, *J* = 7.8Hz, 1H), 7.47-7.28 (m, 3H), 7.20 (s, 1H), 7.12-7.04 (m, 2H), 6.79 (s, 1H), 5.09 (dd, *J* = 13.5, 5.2Hz, 1H), 4.36-4.16(m, 4H), 3.78 (s, 3H), 3.33-3.11 (m, 5H), 2.92-2.76 (m, 10H), 2.42-2.32 (m, 5H), 2.29 (s, 3H), 2.02-1.81(m, 5H), 1.66 (s, 3H), 1.62 (s, 3H), 1.07 (t, *J* = 7.3Hz, 3H). MS m/z (ESI): 931.4 [M+H]⁺.

### Example 220

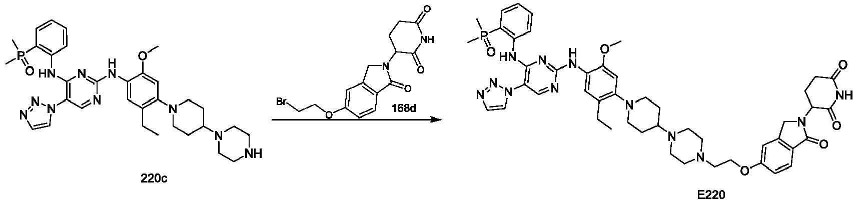

Compound E220 (white solid, 2.31 mg) was prepared with reference to the method for reference compound C-1.

¹H NMR (400 MHz, DMSO-*d*₆) δ 10.99 (s, 1H), 10.58 (s, 1H), 8.52- 8.33 (m, 3H), 8.05 (s, 1H), 7.89 (s, 1H), 7.63 (d, *J=* 8.7Hz, 1H), 7.55-7.33 (m, 2H), 7.32-7.23 (m, 1H), 7.21-7.15 (m, 1H), 7.07 (d, *J* = 6.8Hz, 2H), 6.80 (s, 1H), 5.08 (dd, *J* = 13.2, 4.4Hz, 1H), 4.42-4.10 (m, 4H), 3.77 (s, 3H), 3.08-2.98 (m, 3H), 2.96-2.85 (m, 2H), 2.71-2.60 (m, 11H), 2.42-2.23 (m, 4H), 2.01-1.94 (m, 1H), 1.92-1.83 (m, 2H), 1.70-1.50 (m, 8H), 1.11-1.02 (m, 3H). MS m/z (ESI): 917.4 [M+H]⁺.

### Example 221

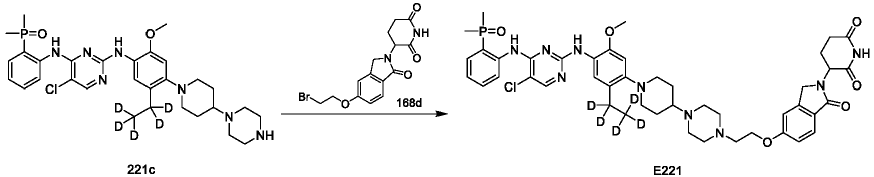

Compound E221 (white solid, 4.55 mg) was prepared with reference to the method for reference compound C-1.

¹H NMR (400 MHz, DMSO-*d*₆) δ 11.16 (s, 1H), 10.99 (s, 1H), 8.49-8.39 (m, 1H), 8.34-8.23 (m, 1H), 8.12 (d, *J=* 11.7Hz, 1H), 7.65-7.60 (m, 1H), 7.58-7.51 (m, 1H), 7.45-7.40 (m, 1H), 7.36-7.28 (m, 1H), 7.20 (s, 1H), 7.13-7.04 (m, 2H), 6.77 (s, 1H), 5.13-5.04 (m, 1H), 4.43-4.37 (m, 1H), 4.31-4.25 (m, 1H), 4.22-4.15 (m, 2H), 3.75 (s, 3H), 3.07-2.97 (m, 4H), 2.75-2.65 (m, 6H), 2.45-2.35 (m, 5H), 2.04-1.94 (m, 2H), 1.91-1.83 (m, 3H), 1.78 (s, 3H), 1.75 (s, 3H), 1.66-1.50 (m, 3H). MS m/z (ESI): 889.5 [M+H]⁺.

### Example 222

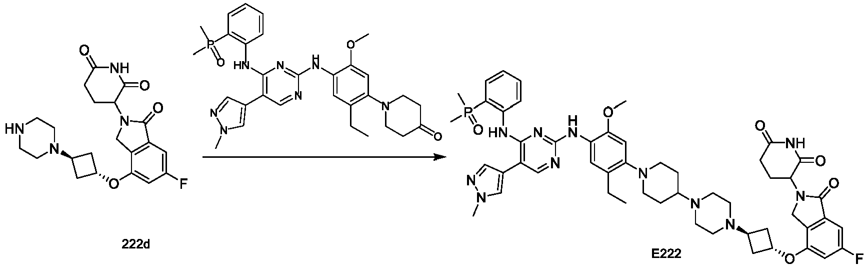

Compound E222 (white solid, 9.56 mg, formate) was prepared with reference to the method for compound E67.

¹H NMR (400 MHz, DMSO-*d*₆) δ 11.02 (s, 1H), 10.16 (s, 1H), 8.32 (s, 1H), 8.19-8.18 (m, 1H), 8.07 (s, 1H), 8.00 (s, 1H), 7.73-7.72 (m, 2H), 7.66-7.51 (m, 1H), 7.47-7.39 (m, 1H), 7.11-7.09 (m, 2H), 6.96-6.94 (m, 1H), 6.75 (s, 1H), 5.14-5.13 (m, 1H), 4.95-4.90 (m, 1H), 4.40-4.19 (m, 2H), 3.84 (s, 3H), 3.79 (s, 3H), 3.70-3.60 (m, 3H), 2.99- 2.96 (m, 3H), 2.90-2.29 (m, 12H), 2.20-1.83 (m, 8H), 1.71 (s, 3H), 1.68 (s, 3H), 1.56-1.50 (m, 2H), 0.96 (t, *J* = 7.3Hz, 3H). MS m/z (ESI): 974.5 [M+H]⁺.

### Example 223

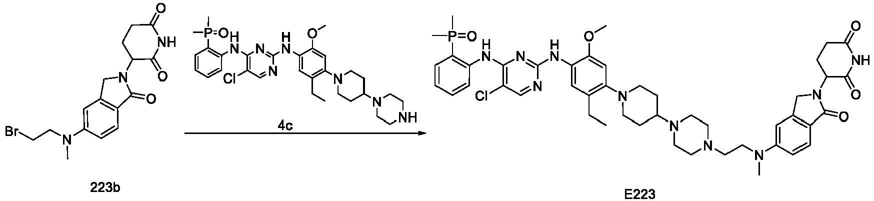

Title compound E223 (white solid, 5 mg, hydrochloride) was prepared with reference to the method for compound C-1.

¹H NMR (500 MHz, CD₃OD) δ 8.19-8.00 (m, 2H), 7.59 (d, *J* = 10.0 Hz, 2H), 7.45-7.21 (m, 3H), 7.00 (s, 1H), 6.95 (d, *J* = 10.0 Hz, 1H), 6.85 (s, 1H), 5.02 (dd, *J* = 10.0, 5.0 Hz, 1H), 4.39-4.30 (m, 2H), 3.91-3.63 (m, 14H), 3.50-3.34 (m, 4H), 3.06 (s, 3H), 2.92-2.66 (m, 4H), 2.56-2.51(m, 2H), 2.42-2.34 (m, 1H), 2.27-2.25 (m, 2H), 2.07-1.95 (m, 3H), 1.80(s, 3H), 1.77(s, 3H), 1.07-0.98 (m, 3H). MS m/z (ESI): 897.8 [M+H]⁺.

### Example 224

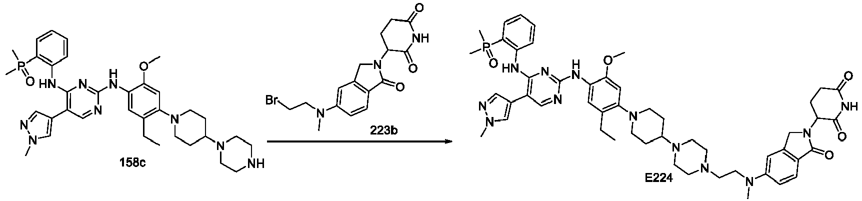

Title compound E224 (white solid, 5.5 mg, hydrochloride) was prepared with reference to the method for reference compound C-1.

¹H NMR (500 MHz, CD₃OD) δ 8.03 (s, 1H), 7.84(s, 1H), 7.73(s, 1H), 7.58 (s, 1H), 7.56-7.51 (m, 2H), 7.44-7.34 (m, 2H), 7.25 (t, *J* = 7.5 Hz, 1H), 6.81 (d, *J* = 10.0 Hz, 2H), 6.75 (s, 1H), 5.00 (dd, *J* = 10.0, 5.0 Hz, 1H), 4.35-4.26 (m, 2H), 3.87 (s, 3H), 3.77 (s, 3H), 3.62-3.45 (m, 4H), 3.13-3.06 (m, 4H), 3.02-2.93 (m, 5H), 2.83-2.32(m, 12H), 2.17-1.78 (m, 6H), 1.70(s, 3H), 1.67 (s, 3H), 0.95-0.94 (m, 3H). MS m/z (ESI): 944.1 [M+H]⁺.

### Example 225

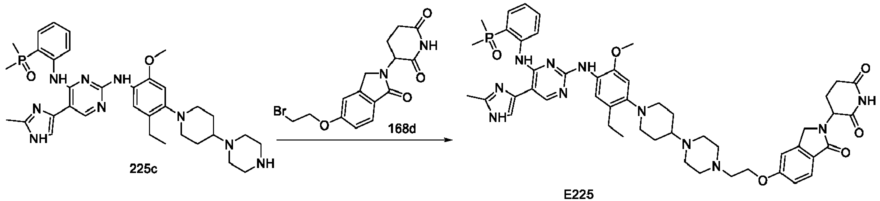

Title compound E225 (white solid, 8 mg, hydrochloride) was prepared with reference to the method for compound C-1.

¹H NMR (500 MHz, CD₃OD) δ 8.21 (s, 1H), 7.80 (dd, *J* = 10.0, 5.0 Hz, 1H), 7.70-7.68 (m, 2H), 7.62 (d, *J* = 5.0 Hz, 1H), 7.54 (t, *J* = 7.5 Hz, 1H), 7.25 (s, 1H), 7.06 (s, 1H), 6.98 (dd, *J* = 10.0, 5.0 Hz, 1H), 6.62 (s, 1H), 5.01 (dd, *J* = 10.0, 5.0 Hz, 1H), 4.40-4.31 (m, 2H), 4.16 (t, *J* = 7.5 Hz, 2H), 3.76 (s, 3H), 2.94-2.53 (m, 15H), 2.44-2.34(m, 2H), 2.30 (s, 3H), 2.26-2.21(m, 2H), 2.09-1.94 (m, 4H), 1.75 (s, 3H), 1.72(s, 3H), 1.66-1.59 (m, 2H), 0.78-0.75 (m, 3H). MS m/z (ESI): 930.6 [M+H]⁺.

The present invention is further described with reference to the following test examples, which, however, are not intended to limit the scope of the present invention.

### Biological Assay

### Cells

The cell lines used in the present invention (supplied by WUXI APPTEC) are shown in the following table.

| Cell | Abbreviation | Matrix |
|---|---|---|
| BaF3 (EGFR Del19/T790M/C797S) | DTC | RPMI1640+10%FBS |
| BaF3 (EGFR Del19/C797S) | DC | RPMI1640+10%FBS |
| BaF3 (EGFR Del19) | D | RPMI1640+10%FBS |
| BaF3 (EGFR L858R/T790M/C797S) | LTC | RPMI1640+10%FBS |
| BaF3 (EGFR L858R/C797S) | LC | RPMI1640+10%FBS |
| BaF3 (EGFR L858R) | L | RPMI1640+10%FBS |
| BaF3 (EGFR WT) | WT | RPMI1640+10 %FBS+10 ng/ml EGF |
| BaF3 patent | parental | RPMI1640+10%FBS+10ng/ml mIL3 |

Half maximal inhibitory concentration (IC₅₀) assay of compounds:
IC₅₀ of compounds in the present invention was determined by Cell Titer GLO reagent from Promega. The procedures are as follows:
Tumor cells were cultured in specified medium in an incubator at 37 °C and 5% CO₂. The cells were passaged periodically. Cells at the logarithmic growth phase were collected for plating at a density of 2000-4000 cells/well. The next day, the compounds of the present invention were serially diluted and added to the cells, with DMSO being the negative control and the culture media without cells being the blank control. The plate was incubated in the incubator for 72 h. The cell viability assay was performed by adding the detection reagents to the cell culture media according to Cell Titer GLO reagent instructions and detecting luminescent signals on an EnVision microplate reader (PerkinElmer).

Inhibition of cell growth by the compounds of the present invention was plotted by Prism Graphpad software for statistically calculating the IC₅₀ of the compound of the present invention. The specific data are shown in Tables 5 and 6 below:

**Table 5**

| Compound | Structure | DTC IC50 (nM) | LTC IC50 (nM) | Parental IC50 (nM) | WT IC50 (nM) | A431 IC50 (nM) |
|---|---|---|---|---|---|---|
| C-2 | 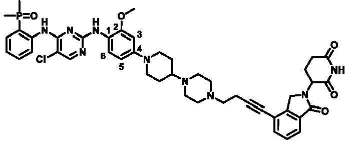 | 42.8 | 82.9 | 73.8 | 531.0 | 1380.2 |
| E3 | 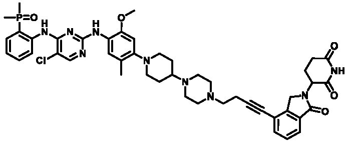 | 14.3 | 46.6 | 521.4 | 287.0 | |
| E4 | 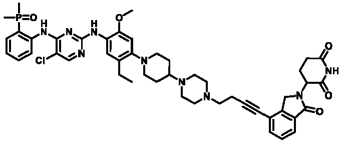 | 6.6 | 37.6 | >4000 | >4000 | 2063.0 |
| E10 | 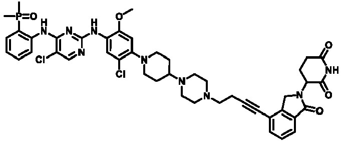 | 15.5 | 28.4 | 54.9 | 101.0 | 1213.4 |
| E13 | 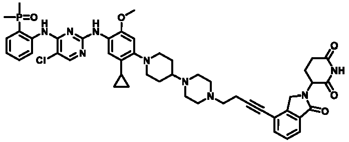 | 19.6 | 66.5 | >4000 | >4000 | >4000 |
| E24 | 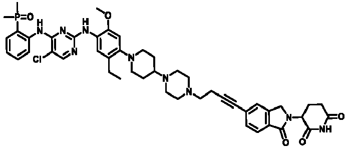 | 35.4 | 70.6 | 1262.2 | 1554.9 | 1460.5 |
| C-3 | 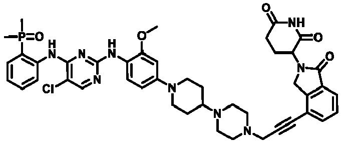 | 43.7 | 129.3 | 475.2 | 586.9 | 1206.2 |
| E25 | 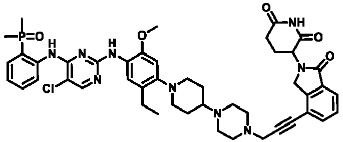 | 13.7 | 57.2 | 1206.4 | 1372.9 | 1263.3 |
| C-4 | 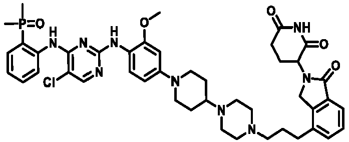 | 41.7 | 70.7 | 142.5 | | |
| E26 | 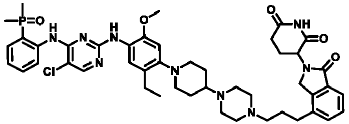 | 8.0 | 53.5 | 545.8 | 517.1 | 1087.2 |
| C-5 | 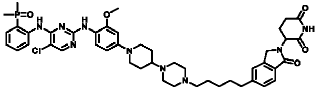 | 22.5 | 102.7 | 569.5 | 1625.4 | 1229.7 |
| E19 | 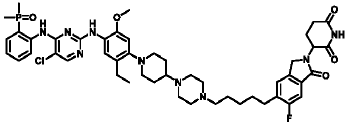 | 8.9 | 61.1 | >4000 | >4000 | 1072.0 |
| E20 | 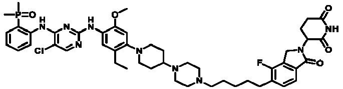 | 5.6 | 54.3 | >4000 | >4000 | 1270.4 |
| C-1 | 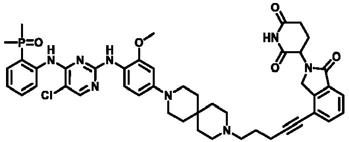 | 28.0 | 199.2 | 376.2 | 380.1 | 2820.5 |
| E11 | 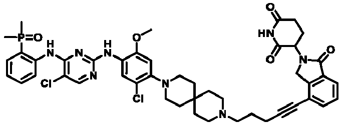 | 17.1 | 61.3 | 146.8 | 236.4 | >4000 |
| E15 | 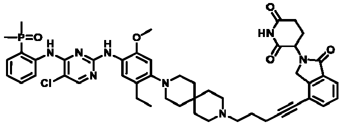 | 10.6 | 120.4 | >4000 | >4000 | >4000 |
| E16 | 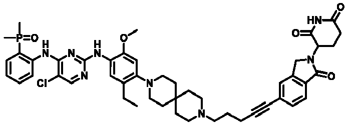 | 55.6 | 347.6 | >4000 | >4000 | >4000 |
| E23 | 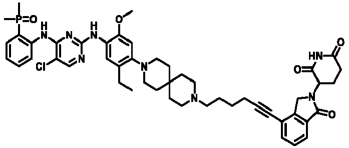 | 9.4 | 105.5 | >4000 | >4000 | >4000 |
| C-6 | 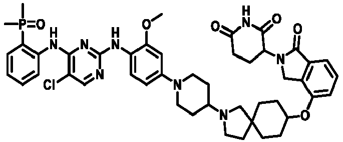 | 7.8 | 33.6 | 93.0 | 79.2 | 341.7 |
| E21 | 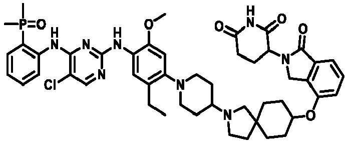 | 1.9 | 16.8 | 410.0 | 636.0 | 1007.5 |
| E27 | 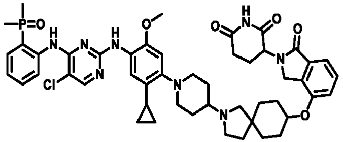 | 3.3 | 29.8 | 375.0 | 937.0 | 1192.0 |
| E28 | 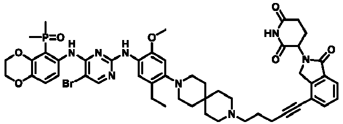 | 11.2 | 57.9 | 695.0 | 955.0 | 1393.0 |
| E29 | 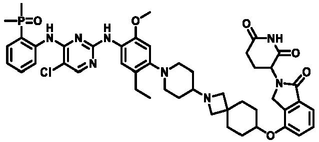 | 14.0 | 20.9 | 1372.0 | 1001.0 | 1021.0 |
| E30 | 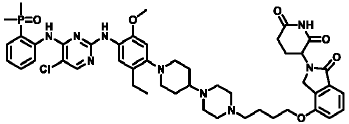 | 1.6 | 11.9 | 410.0 | 636.0 | 1021.0 |
| E9 | 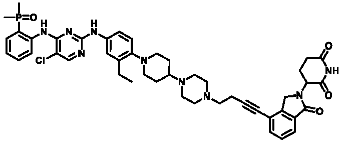 | 5.6 | 11.2 | 20.7 | 28.4 | 392.5 |
| E12 | 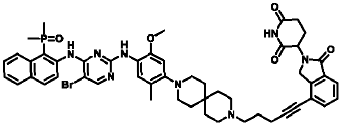 | 18.3 | 111.2 | >4000 | >4000 | >4000 |
| E14 | 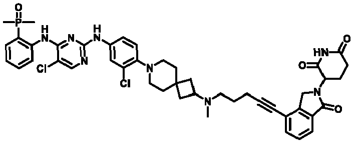 | 7.8 | 55.2 | 84.2 | 69.0 | 376.6 |
| E17 | 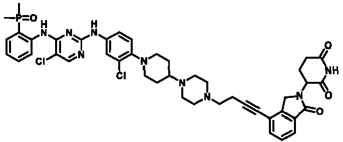 | 3.7 | 15.5 | 23.4 | 25.1 | 283.1 |
| E18 | 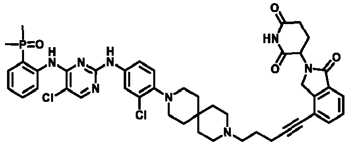 | 30.9 | 266.4 | 786.2 | 723.8 | 2179.2 |

**Table 6**

| Compound | DTC IC50 (nM) | LTC IC50 (nM) | DC IC50 (nM) | Parental IC50 (nM) | WT IC50 (nM) | A431 IC50 (nM) |
|---|---|---|---|---|---|---|
| E7 | | 42.7 | 14.9 | 270.8 | 133.6 | |
| E22 | | 34.1 | 4.1 | 384.7 | 275.2 | 1049.6 |
| E34 | | 16.9 | 2.5 | | 1259.6 | 1483.7 |
| E35 | 5.59 | 25.7 | 8.9 | | 2501.9 | 1641.2 |
| E36 | 9.49 | 24.7 | 3.1 | | 540.9 | 1132.9 |
| E37 | 4.84 | 15.3 | 3.1 | | 1073.3 | 1206.4 |
| E38 | 6.23 | 16.5 | 3.4 | | 461.0 | 1018.3 |
| E39 | | 19.2 | 4.2 | | 250.0 | 927.5 |
| E40 | | 17.5 | 4.9 | 131.1 | 260.2 | 1213.1 |
| E41 | | 44.4 | 8.2 | | 1058->4000 | 2238.9 |
| E42 | | 43.4 | 5.6 | | 90.7 | 1140.5 |
| E43 | | 15.8 | 6.6 | 121.6 | 330.3 | 1064.2 |
| E44 | | 17.9 | 6.6 | | >4000 | 3493.1 |
| E45 | | 21.2 | 8.5 | 221.2 | 585.3 | 1243.3 |
| E46 | 10.1 | 17.7 | 9.2 | | 1199.1 | 1605.8 |
| E47 | | 36.9 | 9.5 | | 461.0 | >4000 |
| E48 | 15.5 | 22.8 | 10 | | 1021.5 | 1024.1 |
| E49 | | 53.1 | 10.1 | | >4000 | 3768.2 |
| E50 | 21.9 | 58.1 | 12.6 | | 1261.3 | 1345.2 |
| E51 | | 57.8 | 14 | | >4000 | 1087.7 |
| E52 | | 41.7 | 15.2 | | >4000 | 1041.6 |
| E54 | | 51.4 | 20.3 | | 352.7 | 1178.9 |
| E57 | 72.5 | 456.0 | 22.1 | | >4000 | >4000 |
| E59 | | 67.1 | 22.7 | | 1321.3 | 1441.9 |
| E60 | | 77.1 | 39.7 | | >4000 | >4000 |
| E61 | 280 | 621.4 | 37.8 | | >4000 | >4000 |
| E62 | | 96.1 | 51.1 | | >4000 | >4000 |
| E63 | 7.31 | 49.3 | 2.6 | | 658.6 | 1152.3 |
| E64 | | 41.5 | 11.7 | | >4000 | >4000 |
| E65 | 35.3 | 266.6 | 15.8 | | 1734.2 | 1057.9 |
| E66 | | 59.7 | 10.6 | | 2491.9 | 1985.2 |
| E67 | 4.09 | 20.6 | 4.4 | | 306.3 | 2487.4 |
| E68 | | >4000 | 18.6 | | >4000 | >4000 |
| E69 | 15.3 | 114.3 | 0.7 | | 167.5 | 343.8 |
| E70 | | >4000 | 0.7 | | 266.6 | 1253.9 |
| E71 | | 62.3 | 54.4 | 719.3 | 1548.9 | >4000 |
| E74 | | 160.3 | 37.6 | | >4000 | >4000 |
| E75 | 26.3 | 64.2 | 20.2 | | 80.3 | >4000 |
| E76 | | 146.8 | 30.1 | | >4000 | >4000 |
| E77 | 16.3 | 44.6 | 13.5 | | 67.6 | 650.6 |
| E82 | 17 | 161.6 | 9.1 | | 781.0 | 1778.2 |
| E83 | | 18.0 | 1.2 | | 1448.5 | 1469.7 |
| E84 | | 85.5 | 25.4 | | >4000 | 1970.2 |
| E85 | | 53.0 | 13.7 | | 1876.3 | 1372.1 |
| E86 | 40.7 | 196.8 | 33 | | 211.6 | 1671.3 |
| E87 | 11.5 | 51.8 | 5.3 | | >4000 | 3797.0 |
| E88 | | 59.9 | 9.1 | | 2700.5 | 1613.6 |
| E89 | 89.5 | 1037.0 | 79.9 | | 1152.4 | 3646.2 |
| E90 | | 142.8 | 13.1 | | 2067.4 | 3698->4000 |
| E91 | 18.5 | 115.4 | 5.2 | | 304.9 | 1425.7 |
| E92 | | 72.6 | 12.7 | | 691.6 | 1165.1 |
| E93 | 9.74 | 68.6 | 15 | | 75.4 | 826.7 |
| E94 | | 57.0 | 40.2 | | 50.6 | 524.4 |
| E95 | 5.37 | 48.4 | | | 1815->4000 | 1730.2 |
| E97 | 45.6 | 173.1 | 20.6 | | 1059.7 | 1074.4 |
| E98 | 15.5 | 45.2 | 5 | | 566.3 | 1165.4 |
| E100 | | 1131.9 | 515.2 | | 1231.1 | >4000 |
| E101 | | 51.6 | 6.8 | | 1693.7 | 1435.7 |
| E103 | 5.54 | 21.8 | 4.6 | | 639.4 | 1010.4 |
| E104 | | 43.8 | 9.1 | | 2270.2 | >4000 |
| E105 | | 2058.2 | 10.5 | | 1491.2 | 1966.3 |
| E106 | | 226.0 | 16.8 | | >4000 | 1370.6 |
| E107 | | 158.1 | 12.1 | | >4000 | 1336.2 |
| E108 | 10.5 | 76.7 | 3.2 | | 1750.5 | 3545.3 |
| E109 | 10.5 | 83.3 | 6.5 | | >4000 | 1137.8 |
| E110 | 6.55 | 56.5 | 2.4 | | >4000 | 1140.7 |
| E112 | 18.4 | 85.4 | 10.7 | | >4000 | 1374.8 |
| E115 | 5.19 | 24.4 | 1.4 | | 779.2 | 1161.7 |
| E116 | 7.32 | 56.3 | 4.3 | | >4000 | 1221.5 |
| E118 | | 111.6 | 6.3 | | >4000 | 1338.4 |
| E119 | | 67.3 | 5.6 | | >4000 | 1398.7 |
| E120 | 14.74 | 114.9 | | | 2165->4000 | 1556->4000 |
| E122 | | 109.9 | 3.4 | | 2654.6 | 1093.5 |
| E123 | 3.98 | 28.2 | | | 1473.2 | 1688.0 |
| E124 | 6.8 | 106.3 | 106.1 | | 3966.5 | 1265.7 |
| E125 | 20.3 | 103.6 | 18.4 | | >4000 | >4000 |
| E126 | 18.9 | 72.3 | 44 | | >4000 | >4000 |
| E127 | | 79.6 | 8.7 | | 594.7 | 1270.0 |
| E129 | 6.13 | 47.7 | 3.3 | | 888.9 | 1331.2 |
| E137 | 11.5 | 76.3 | 6.6 | | 1081.4 | 1274.8 |
| E138 | | 70.6 | 13.7 | | 2501.0 | 1097.9 |
| E139 | | 694.3 | 60.9 | | 554.1 | 962.7 |
| E140 | 7.97 | 54.6 | 8.1 | | >4000 | >4000 |
| E141 | | 50.3 | 14.7 | | >4000 | >4000 |
| E142 | | 1101.5 | 123.8 | | 791.7 | >4000 |
| E143 | | 25.5 | 3.3 | | 344.6 | 1062.7 |
| E144 | | 105.0 | 35.2 | | 1204.5 | 1371.2 |
| E145 | | 505.5 | 79.1 | | >4000 | 1113.3 |
| E146 | 15.7 | 55.8 | 5.6 | | 188.2 | 908.1 |
| E147 | | 2004.0 | 56.1 | | 1572.9 | >4000 |
| E148 | | >4000 | 9.3 | | >4000 | >4000 |
| E149 | | 3482.8 | 37.2 | | 1290 | >4000 |
| E151 | 3.85 | 56.1 | 6.7 | | >4000 | 3158.1 |
| E152 | 7.63 | 227.4 | 16.2 | | >4000 | 1469.7 |
| E153 | | 65.9 | 26.3 | | >4000 | >4000 |
| E154 | | 3429.0 | 446.3 | | >4000 | >4000 |
| E155 | | 921.4 | 121.7 | | 3979.1 | 2092.2 |
| E156 | 10.7 | 217.5 | 16.7 | | >4000 | 1542->4000 |
| E157 | 11.2 | 103.3 | 33.7 | | 3777.5 | 3171.1 |
| E158 | 33 | 2756.8 | | | 729.9 | 1196->4000 |
| E159 | | 21.9 | 1.4 | | 646.0 | 1853.2 |
| E160 | 2.89 | 14.3 | 0.8 | | 1292.8 | 2411.0 |
| E161 | | 134.8 | 2 | | 1186.2 | 1289.4 |
| E164 | | >4000 | 7.7 | | >4000 | >4000 |
| E165 | 780 | >4000 | 4.2 | | >4000 | >4000 |
| E167 | 37.8 | 3049.5 | 1.5 | | 975.2 | 1100.6 |
| E168 | | >4000 | 1.1 | | 505.7 | 1222.9 |
| E170 | | >4000 | 3.5 | | >4000 | >4000 |
| E171 | | >4000 | 6.3 | | >4000 | >4000 |
| E172 | | >4000 | 2.4 | | 1073.4 | >4000 |
| E173 | | 181.1 | 21.7 | | 1525.5 | >4000 |
| E176 | | >4000 | 2.4 | | >4000 | >4000 |
| E177 | | >4000 | 15.7 | | >4000 | >4000 |
| E179 | 5.94 | 44.6 | 1.3 | | 152.6 | 1165.0 |
| E180 | 5.35 | 22.6 | 2.4 | | 887.7 | 1098.6 |
| E181 | 1.76 | 6.3 | 0.7 | | 1107.5 | 1134.7 |
| E182 | 3.87 | 41.0 | 6 | | 1078.8 | 1330.7 |
| E183 | 3.16 | 9.6 | 1 | | 1007.4 | 1540.5 |
| E184 | | 127.8 | 20.5 | | 1034.2 | 1780.5 |
| E185 | 18.8 | 144.3 | 20.3 | | 1649.5 | 1831.7 |
| E187 | | 50.3 | 19.5 | | 814.8 | 1464.5 |
| E189 | | >4000 | 0.8 | | 495.5 | 1005.6 |
| E191 | | 69.2 | 3.1 | | 947.3 | 1145.4 |
| E192 | | >4000 | 43.8 | | >4000 | >4000 |
| E193 | | >4000 | 14.9 | | 1323.7 | >4000 |
| E194 | | >4000 | 0.5 | | 535.2 | 1364.4 |
| E205 | 6.87 | 51.7 | 9.9 | | >4000 | 1047.6 |
| E208 | | 531.5 | 16.1 | | 1010.6 | 1353.6 |
| E211 | | 59.2 | | | 3298.2 | >4000 |
| E213 | | >4000 | 8.6 | | 1356.1 | >4000 |
| E214 | | >4000 | 3.4 | | 615.8 | 1463.2 |
| E216 | | >4000 | 1.0 | | 926.3 | >4000 |
| E217 | | >4000 | 0.9 | | 622.8 | >4000 |
| E218 | | >4000 | 1.0 | | 2088.6 | >4000 |
| E219 | | >4000 | 0.9 | | 829.4 | >4000 |
| E220 | | >4000 | 4 | | >4000 | >4000 |
| E221 | 12.1 | 22.5 | 6.4 | | 1747.7 | >4000 |
| E222 | | 89.7 | <0.24 | | 132.9 | >4000 |
| E223 | | 69.4 | 11.5 | | >4000 | >4000 |
| E224 | | >4000 | 0.9 | | 654.3 | >4000 |

The inhibitory effect on cell growth of the compounds of the present invention was determined using the same procedures as described above, and IC₅₀ was calculated by plotting using Prism Graphpad software. The specific data are shown in Table 7 below:

**Table 7**

| Compound | DC IC₅₀ (nM) | LC IC₅₀ (nM) | D IC₅₀ (nM) | L IC₅₀ (nM) | DT IC₅₀ (nM) | LT IC₅₀ (nM) |
|---|---|---|---|---|---|---|
| C-2 | 15.5 | 49.5 | 4.5 | 17.1 | 13.3 | 20.9 |
| E4 | 4.8 | 23.0 | 2.1 | 6.7 | 5.5 | 7.4 |
| E13 | 20.4 | 128.1 | 6.1 | 23.5 | 7.7 | 16.5 |
| E20 | 2.7 | 19.4 | 1.7 | 5.2 | 7.0 | 10.0 |
| E15 | 9.1 | 54.4 | 5.3 | 20.1 | 13.7 | 20.7 |
| E12 | 16.2 | 105.7 | 7.8 | 37.8 | 24.8 | 36.0 |
| E95 | 1.3 | 16.9 | 0.8 | 6.6 | 4.0 | 3.5 |
| E120 | 3.2 | 33.9 | 1.8 | 16.6 | 7.8 | 9.3 |
| E123 | 0.4 | 3.6 | 0.3 | 1.4 | 3.0 | 2.8 |
| E158 | 0.1 | 1.3 | 0.2 | 0.6 | 15.3 | 69.1 |

The data in Tables 5, 6, and 7 show that the compounds of the examples of the present invention have good inhibitory effects and selectivity in the proliferation activity test of BaF3 cells with various EGFR mutants.

### EGFR protein abundance assay

Mouse Ba/F3 cells stably expressing human EGFR-DTC, EGFR-LTC, or EGFR-DC were transferred to Petri dishes with RPMI-1640 medium (Gibco, Product No. 11875093) containing 10% fetal bovine serum (FBS, Biological Industries, Product No. 04-001-1A) and 0.1% penicillin/streptomycin (P/S) and incubated in an aseptic incubator at 37 °C, 95% relative humidity, and 5% CO₂. Cells at the logarithmic growth phase were taken and seeded at a density of 50000 cells/well in 12-well plates (Corning, Product No. 3513) containing 1 mL of medium per well. After 24 h, the compounds disclosed herein with different concentrations were added to the wells at a final DMSO concentration of 0.1%. After 24 h, cells were lysed in SDS lysis buffer (1.45 g of SDS, 0.2 g of Tris base, 6 mL of glycerol, 20 mg of bromophenol blue, 310 mg DTT; brought to 40 mL with d₂H₂O), and the EGFR levels were detected and analyzed by Western Blotting (immunoblotting) using standard molecular biological techniques. Cell lysates were boiled at 95 °C for 8 min, mixed and centrifuged. The total cell proteins in the lysates were subjected to SDS-PAGE (GenScript, Product No. M00654), transferred to NC membranes (GE, Product No. 1060002) by blotting, incubated in a blocking buffer (TAKARA, Product No. T7131A) at room temperature for 60 min, and co-incubated overnight at 4 °C with the following primary antibodies: anti-EGFR (#4267S, 1:1000), anti-phoso-EGFR (#3777S, 1:1000), anti-GAPDH (#5174S, 1:10000), anti-Actin (#5125S, 1:10000) (all purchased from Cell Signaling Technology). The samples were washed three times with TBST at room temperature, each for 10 min, and then incubated for 60 minutes in a blocking buffer containing a secondary antibody (#7074S, 1:10000, purchased from Cell Signaling Technology) at room temperature. The samples were washed three times with TBST at room temperature, each for 10 min. Chemiluminescent substrates were added, and an Azure c300 system was used for imaging.

The DC₅₀ of the compound of the present invention for degrading EGFR-DTC, EGFR-LTC and EGFR-DC is < 50 nM, preferably < 20 nM, more preferably < 10 nM, and even more preferably < 5 nM. See Table 8 for the specific results:

**Table 8**

| Compound | EGFR-DTC | | EGFR-LTC | | EGFR-DC | |
|---|---|---|---|---|---|---|
| | DC50 (nM) | Dmax | DC50 (nM) | Dmax | DC50 (nM) | Dmax |
| E4 | 2.27 | 96% | 6.26 | 91% | 18.6 | 75% |
| E5 | 9.31 | 86% | <1 | 93% | | |
| E6 | 3.37 | 70% | 10.1 | 89% | | |
| E95 | 3.02 | 91% | 2.13 | 94% | 10.1 | 98% |
| E120 | 7.17 | 86% | 13.1 | 85% | | |
| E123 | 1.06 | 97% | 2.6 | 95% | 1.12 | 97% |

The above is a detailed description of the present invention with reference to specific preferred embodiments, and should not be construed as limitations to the present invention. For those skilled in the art of the present invention, various simple deductions or substitutions may be made without departing from the spirit of the present invention, and should be construed as falling within the scope of the present invention.

## Claims

1. A compound of formula (I), or a pharmaceutically acceptable salt, an enantiomer, a diastereomer, a racemate, a solvate, a hydrate, a polymorph, a prodrug or an isotopic variant thereof, and a mixture thereof,
PIN-Linker-E (I)
wherein,
PIN represents: 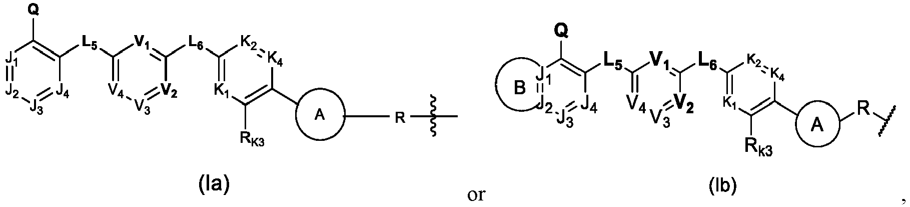
wherein Q is C(R^{q1})₃, C(O)R^{q1}, S(O)R^{q1}, SO₂R^{q1}, P(O)R^{q1}R^{q2}, or NR^{q1}R^{q2}, and R^{q1} and R^{q2} are each independently selected from hydrogen, amino, -C₁₋₅ alkylene-R_{qn}, -C₁₋₅ haloalkylene-R_{qn}, -C₁₋₅ alkyleneoxy-R_{qn}, -C₃₋₈ cycloalkylene-R_{qn}, -3- to 8-membered heterocycloalkylene-R_{qn}, -C(O)R_{qn}, -S(O)R_{qn}, SO₂R_{qn}, - C(O)NR₀₀R_{qn}, -NR₀₀C(O)R_{qn}, and -SO₂NR₀₀R_{qn};
R_{qn} is selected from hydrogen, C₁₋₁₀ alkyl, C₁₋₁₀ haloalkyl, C₁₋₁₀ alkoxy, C₁₋₁₀ alkylthio, C₂₋₁₀ alkenyl, C₂₋₁₀ alkynyl, NRₐR_{b}, C₃₋₈ cycloalkyl, 3- to 8-membered heterocycloalkyl, C₆₋₁₀ aryl, and 5- to 14-membered heteroaryl;
J₁ is N or CR_{J1}, J₂is N or CR_{J2}. J₃is N or CR_{J3}, and J₄ is N or CR_{J4};
V₁ is N or CR₁, V₂ is N or CR₂, V₃ is N or CR₃, and V₄ is N or CR₄;
R₁, R₂, R₃, R₄, R_{J1}, R_{J2}, R_{J3}, and R_{J4} are each independently selected from hydrogen, hydroxy, cyano, acetyl, carboxy, nitro, halogen, amino, C₁₋₁₀ alkyl, C₁₋₁₀ haloalkyl, C₁₋₁₀ alkoxy, C₁₋₁₀ alkylthio, C₂₋₁₀ alkenyl, C₂₋₁₀ alkynyl, NRₐR_{b}, C₃₋₈ cycloalkyl, 3- to 8-membered heterocycloalkyl, C₆₋₁₀ aryl, and 5- to 14-membered heteroaryl;
or, R₃ and R₄, together with the atoms to which they are attached, form a substituted or unsubstituted 5- to 6-membered heteroaryl or benzene;
or, R_{J2} and R_{J3}, together with the atoms to which they are attached, form a substituted or unsubstituted 5- to 6-membered heteroaryl or benzene, or R_{J3} and R_{J4}, together with the atoms to which they are attached, form a substituted or unsubstituted 5- to 6-membered heteroaryl or benzene; the substitution refers to that the 5- to 6-membered heteroaryl or benzene is optionally substituted with 1, 2, 3, or 4 substituents selected from hydrogen, halogen, amino, cyano, acetyl, C₁₋₅ alkyl, C₁₋₅ alkoxy, C₁₋₃ haloalkyl, C₃₋₈ cycloalkyl, and 3- to 8-membered heterocycloalkyl;
K₁ is N or CRₖ₁, K₂ is N or CRₖ₂, and K₄ is N or CRₖ₄,
Rₖ₁, Rₖ₂, and Rₖ₄ are identical or different, and are each independently selected from hydrogen, hydroxy, cyano, acetyl, -C(O)NRₐR_{b}, halogen, C₁₋₁₀ alkyl, C₁₋₅ alkylene-OC₁₋₅ alkyl, C₁₋₁₀ haloalkyl, C₁₋₁₀ alkoxy, C₁₋₁₀ alkylthio, C₂₋₁₀ alkenyl, C₂₋₁₀ alkynyl, NRₐR_{b}, C₃₋₈ cycloalkyl, 3- to 8-membered heterocycloalkyl, -O-C₃₋₈ cycloalkyl, -O-3- to 8-membered heterocycloalkyl, C₆₋₁₀ aryl, and 5- to 14-membered heteroaryl;
Rₖ₃ is not hydrogen, and Rₖ₃ represents -X₁-Rx,
X₁ is selected from O, S, NR₀₀, -C(O)-, -S(O)-, SO₂, -C(O)NR₀₀-, -NR₀₀C(O)-, -SO₂NR₀₀-, -NR₀₀SO₂-, - NR₀₀C(O)NR₀₀-, -NR₀₀C(O)O-, -OC(O)O-, -C(O)O-, -OC(O)-, -CH=CH-, -C≡C-, -(CRₓ₁Rₓ₂)ₓ-, C₃₋₈ cycloalkylene, 3- to 8-membered heterocycloalkylene, C₆₋₁₀ arylene, and 5- to 14-membered heteroarylene; Rx represents -(CRₓ₃Rₓ₄)ₓ₂-Rₓₙ, or deuterium,
Rₓ₁, Rₓ₂, Rₓ₃, Rₓ₄, and Rₓₙ are each independently selected from hydrogen, cyano, hydroxy, acetyl, halogen, nitro, formyl, carboxy, amino, C₁₋₁₀ alkyl, C₁₋₁₀ haloalkyl, C₁₋₁₀ alkoxy, C₁₋₁₀ alkylthio, C₂₋₁₀ alkenyl, C₂₋₁₀ alkynyl, C₃₋₈ cycloalkyl, 3- to 8-membered heterocycloalkyl, C₆₋₁₀ aryl, and 5- to 14-membered heteroaryl; or, Rₓ₁ and Rₓ₂, together with the C atom to which they are attached, form a 3- to 8-membered cycloalkyl or 3- to 8-membered heterocycloalkyl;
x and x2 are each independently 0, 1, 2, 3, 4, or 5, and x and x2 are not both 0;
or, R_{J3} and Rₖ₃ are connected to form a bond;
L₅ and L₆ are each independently selected from O, S, NR₀₀, C(O)NR₀₀, NR₀₀C(O), C₁₋₁₀ alkylene, C₁₋₁₀ haloalkylene, C₁₋₁₀ alkyleneoxy, C₂₋₁₀ alkenylene, and C₂₋₁₀ alkynylene;
ring A is a saturated or unsaturated monocyclic alkylene, monocyclic heterocyclylene, polycyclic alkylene, or polycyclic heterocyclylene;
ring B is a saturated or unsaturated monocyclic alkylene, monocyclic heterocyclylene, polycyclic alkylene, or polycyclic heterocyclylene;
R is a bond, a saturated or unsaturated monocyclic alkylene, monocyclic heterocyclylene, polycyclic alkylene or polycyclic heterocyclylene, or a 7- to 11-membered mono-spiroheterocyclylene, 3- to 8-membered monocyclic heterocyclylene and 8- to 10-membered bicyclic heteroarylene substituted with 1, 2, or 3 R₀s, wherein R₀ is selected from hydrogen, deuterium, halogen, =O, C₁₋₆ alkyl, C₁₋₆ haloalkyl, C₁₋₆ alkoxy, C₃₋₈ cycloalkyl, and 3- to 8-membered heterocyclyl, or two R₀s, together with the C atom to which they are attached, form a 3- to 6-membered cycloalkyl; the heteroatom of the mono-spiroheterocyclylene is selected from one or more of nitrogen, oxygen, and sulfur, and the number of the heteroatom is 1, 2, or 3; the heteroatom of the bicyclic heteroarylene is selected from one or more of nitrogen, oxygen, and sulfur, and the number of the heteroatom is 1, 2, or 3; the heteroatom of the monocyclic heterocyclylene is selected from one or more of nitrogen, oxygen, and sulfur, and the number of the heteroatom is 1, 2, or 3; the heteroatom of the 3- to 8-membered heterocyclyl is selected from one or more of nitrogen, oxygen, and sulfur, and the number of the heteroatom is 1, 2, or 3;
Linker is absent, or represents: a C₁₋₂₀ alkylene chain, and any one or more methylene groups may optionally be substituted by one or more R^{L}s, R^{L}s are identical or different, and R^{L} is selected from O, S, NR₀₀, -C(O)-, SO, SO₂, -C(O)NR₀₀-, -NR₀₀C(O)-, -SO₂NR₀₀-, -NR₀₀SO₂-, -NR₀₀C(O)NR₀₀-, -NR₀₀C(O)O-, -OC(O)-, - C(O)O-, -OC(O)O-, -CR₀₀=CR₀₀-, -C≡C-, -(CH₂)ᵣ-, C₃₋₈ cycloalkylene, 3- to 8-membered heterocyclylene, C₆₋₁₀ arylene, and 5- to 14-membered heteroarylene; and any one methylene group may be substituted by 1 or 2 R^{L'}s, and R^{L'}s are identical or different and are hydrogen, halogen, amino, nitro, cyano, acetyl, C₁₋₁₀ alkyl, C₁₋₁₀ alkoxy, C₁₋₁₀ haloalkyl, C₃₋₈ cycloalkyl, 3- to 8-membered heterocyclyl, C₆₋₁₀ aryl, or 5- to 14-membered heteroaryl, or two R^{L'}s, together with the C atoms to which they are attached, form a C₃₋₈ cycloalkyl or 3- to 8-membered heterocyclyl; r is 1, 2, 3, 4, or 5;
E represents: 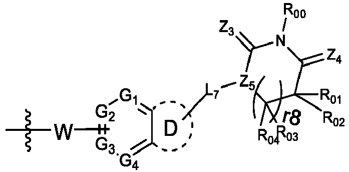
wherein G₁ is N or CR_{G1}, G₂ is N or CR_{G2}, G₃ is N or CR_{G3}, and G₄ is N or CR_{G4};
one of R_{G1}, R_{G2}, R_{G3}, and R_{G4} is attached to W, and the others are each independently selected from the group consisting of hydrogen, hydroxy, cyano, acetyl, acylamino, halogen, C₁₋₁₀ alkyl, C₁₋₁₀ haloalkyl, C₁₋₁₀ alkoxy, C₁₋₁₀ alkylthio, NRₐR_{b}, C₃₋₈ cycloalkyl, 3- to 8-membered heterocyclyl, C₆₋₁₀ aryl, and 5- to 14-membered heteroaryl;
ring D is absent, such that L₇ is attached to a C atom or an N atom on the aromatic ring on which G₁ is located, or ring D is a substituted or unsubstituted C₆₋₁₀ aryl or a substituted or unsubstituted 5- to 14-membered heteroaryl; the substitution refers to that the C₆₋₁₀ aryl or 5- to 14-membered heteroaryl is optionally substituted, within their respective valence-permitted ranges, with 1, 2, or 3 substituents selected from hydrogen, halogen, C₁₋₁₀ alkyl, C₁₋₁₀ alkoxy, C₁₋₁₀ haloalkyl, C₁₋₁₀ haloalkoxy, C₂₋₁₀ alkenyl, C₂₋₁₀ alkynyl, cyano, nitro, acylamino, amino, and acetyl;
or, ring D is 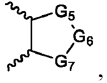 G₅, G₆, and G₇ are each independently O, S, N, or C atom optionally substituted with 1 or 2 R^{G}s, and R^{G} is hydrogen, hydroxy, amino, cyano, acetyl, carboxy, nitro, halogen, C₁₋₁₀ alkyl, C₁₋₁₀ haloalkyl, C₁₋₁₀ alkoxy, C₁₋₁₀ alkylthio, C₂₋₁₀ alkenyl, C₂₋₁₀ alkynyl, C₃₋₈ cycloalkyl, 3- to 8-membered heterocyclyl, C₆₋₁₀ aryl, or 5- to 14-membered heteroaryl; or two R^{G}s, together with the C atoms to which they are attached, form C=O, 3- to 8-membered cycloalkyl, or 3- to 8-membered heterocyclyl;
Z₃ and Z₄ are each independently selected from O, S, or NR₀₀; Z₅ is CR₀₀ or N; or the ring in which Z₅ is located is absent;
L₇ is a bond, O, S, NR₀₀, C(O)NR₀₀, NR₀₀C(O), C₁₋₅ alkylene, C₁₋₅ haloalkylene, C₁₋₅ alkyleneoxy, C₂₋₆ alkenylene, or C₂₋₆ alkynylene;
W is a bond, O, S, NR₀₀, -C(O)-, -S(O)-, SO₂, -C(O)NR₀₀-, -NR₀₀C(O)-, -SO₂NR₀₀-, -NR₀₀SO₂-, - NR₀₀C(O)NR₀₀-, -NR₀₀C(O)O-, -OC(O)O-, -C(O)O-, -OC(O)-, -CR₀₀=CR₀₀-, -C≡C-, -(CR₂₃R₂₄)ᵣ₂-, C₃₋₈ cycloalkylene, 3- to 8-membered heterocyclylene, C₆₋₁₀ arylene, and 5- to 14-membered heteroarylene;
Rₐ, R_{b}, R₀₀, R₀₁, R₀₂, R₀₃, R₀₄, R₂₃, and R₂₄ are each independently selected from hydrogen, cyano, acetyl, hydroxy, carboxy, nitro, halogen, C₁₋₁₀ alkyl, C₁₋₁₀ haloalkyl, C₁₋₁₀ alkoxy, C₁₋₁₀ alkylthio, NR_{c}R_{d}, C₃₋₈ cycloalkyl, 3- to 8-membered heterocyclyl, C₆₋₁₀ aryl, and 5- to 14-membered heteroaryl;
or R₀₁ and R₀₂, or R₀₃ and R₀₄, together with the C atom to which they are attached, form a 3- to 8-membered cycloalkyl or 3- to 8-membered heterocycloalkyl; or R₂₃ and R₂₄, together with the C atom to which they are attached, form a 3- to 8-membered cycloalkyl or 3- to 8-membered heterocyclyl; or Rₐ and R_{b}, together with the N atom to which they are attached, form a 3- to 8-membered heterocyclyl;
r2 is 0, 1, 2, 3, or 4;
R_{c} and Rₐ are each independently hydrogen, C₁₋₅ alkyl, C₁₋₅ alkoxy, or C₁₋₅ haloalkyl; or R_{c} and R_{d}, together with the N atom to which they are attached, form a 3- to 8-membered heterocyclyl;
the above C₁₋₁₀ alkyl, C₁₋₁₀ haloalkyl, C₁₋₁₀ alkoxy, C₁₋₁₀ alkylthio, C₁₋₅ alkyl, C₁₋₅ haloalkyl, C₁₋₅ alkoxy, C₃₋₈ cycloalkyl, 3- to 8-membered heterocyclyl, C₆₋₁₀ aryl, or 5- to 14-membered heteroaryl, saturated or unsaturated monocycle, monocyclic heterocycle, polycycle and polycyclic heterocycle are unsubstituted or, within a valence-permitted range, each independently substituted by 1, 2, 3, 4, or more groups selected from the following B2 groups, and the B2 group is selected from hydrogen, =O, cyano, acetyl, hydroxy, carboxy, nitro, halogen, C₁₋₅ alkyl, C₁₋₅ alkoxy, C₁₋₅ alkylthio, NRₑR_{f}, 3- to 8-membered cycloalkyl, 3- to 8-membered heterocyclyl, C₆₋₁₀ aryl, or 5- to 14-membered heteroaryl, wherein Rₑ and R_{f} are each independently hydrogen, C₁₋₅ alkyl, C₁₋₅ alkoxy, and C₁₋₅ haloalkyl;
provided that:
when PIN is formula (Ia) and Q is S(O)₂-isopropyl, neither Rₖ₃ nor Rₖ₄ is methyl;
when PIN is formula (Ib), ring B is 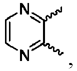 Q is P(O)(CH₃)₂, J₁, J₂, J₃, J₄, V₃ and K₁ are CH, and V₁ and V₂ are N, R₄ is not bromine, or neither Rₖ₃ nor Rₖ₄ is methyl.

2. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 1, wherein, in the compound of formula (I),
E represents: 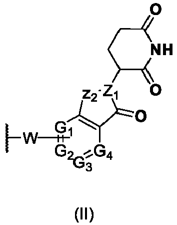
wherein R₁, R₂, R₃, R₄, R_{J1}, R_{J2}, R_{J3}, and R_{J4} are each independently selected from: hydrogen, hydroxy, cyano, acetyl, carboxy, nitro, halogen, amino, C₁₋₁₀ alkyl, C₁₋₁₀ haloalkyl, C₁₋₁₀ alkoxy, C₁₋₁₀ alkylthio, C₂₋₁₀ alkenyl, C₂₋₁₀ alkynyl, NRₐR_{b}, C₃₋₈ cycloalkyl, 3- to 8-membered heterocycloalkyl, C₆₋₁₀ aryl, and 5- to 14-membered heteroaryl;
or, R₃ and R₄, together with the atoms to which they are attached, form a substituted or unsubstituted 5- to 6-membered heteroaryl or benzene;
or, R_{J2} and R_{J3}, together with the atoms to which they are attached, form a substituted or unsubstituted 5- to 6-membered heteroaryl or benzene, or R_{J3} and R_{J4}, together with the atoms to which they are attached, form a substituted or unsubstituted 5- to 6-membered heteroaryl or benzene,
the substitution refers to that the 5- to 6-membered heteroaryl or benzene is optionally substituted with 1, 2, 3, or 4 substituents selected from hydrogen, halogen, amino, cyano, acetyl, C₁₋₅ alkyl, C₁₋₅ alkoxy, C₁₋₃ haloalkyl, C₃₋₈ cycloalkyl, and 3- to 8-membered heterocycloalkyl;
x and x2 are each independently 0, 1, 2, 3, 4, or 5, and x and x2 are not both 0;
or, R_{J3} and Rₖ₃ are connected to form a bond;
Linker represents 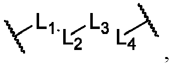 wherein L₁, L₂, L₃, and L₄ each independently represents -R_{L1}-(CR₂₁R₂₂)ₙ₃-R_{L2}-(CR₃₁R₃₂)ₙ₂-,
R_{L1} and R_{L2} are each independently a bond, O, S, NR₀₀, -C(O)-, SO, SO₂, -C(O)NR₀₀-, -NR₀₀C(O)-, - SO₂NR₀₀-, -NR₀₀SO₂-, -NR₀₀C(O)NR₀₀-, -NR₀₀C(O)O-, -OC(O)-, -C(O)O-, -OC(O)O-, -CH=CH-, -C≡C-, - (CR₄₁R₄₂)ᵣ-, -(CR₄₁R₄₂)ᵣ-O-, -O-(CR₄₁R₄₂)ᵣ-, C₃₋₈ cycloalkylene or 3- to 8-membered heterocycloalkylene, C₆₋₁₀ arylene, or 5- to 14-membered heteroarylene;
n2, n3, and r are each independently 0, 1, 2, 3, or 4;
R₂₁, R₂₂, R₃₁, R₃₂, R₄₁, and R₄₂ are each independently selected from hydrogen, cyano, acetyl, hydroxy, halogen, C₁₋₁₀ alkyl, C₁₋₁₀ haloalkyl, C₁₋₁₀ alkoxy, C₁₋₁₀ alkylthio, NRₐR_{b}, C₃₋₈ cycloalkyl, 3- to 8-membered heterocycloalkyl, C₆₋₁₀ aryl, and 5- to 14-membered heteroaryl; or R₂₁ and R₂₂, together with the C atom to which they are attached, form a 3- to 8-membered cycloalkyl or 3- to 8-membered heterocycloalkyl;
L₅ and L₆ are each independently selected from O, S, NR₀₀, C(O)NR₀₀, NR₀₀C(O), C₁₋₁₀ alkylene, C₁₋₁₀ haloalkylene, C₁₋₁₀ alkyleneoxy, C₂₋₁₀ alkenylene, and C₂₋₁₀ alkynylene;
W is a bond, O, S, NR₀₀, -C(O)-, -S(O)-, SO₂, -C(O)NR₀₀-, -NR₀₀C(O)-, -SO₂NR₀₀-, -NR₀₀SO₂-, - NR₀₀C(O)NR₀₀-, -NR₀₀C(O)O-, -OC(O)O-, -C(O)O-, -OC(O)-, -CH=CH-, -C≡C-, -(CR₂₃R₂₄)ᵣ₂-, C₃₋₈ cycloalkylene, 3- to 8-membered heterocycloalkylene, C₆₋₁₀ arylene, or 5- to 14-membered heteroarylene; R₂₃ and R₂₄ are each independently selected from hydrogen, cyano, acetyl, hydroxy, halogen, C₁₋₁₀ alkyl, C₁₋₁₀ haloalkyl, C₁₋₁₀ alkoxy, C₁₋₁₀ alkylthio, NRₐR_{b}, C₃₋₈ cycloalkyl, 3- to 8-membered heterocycloalkyl, C₆₋₁₀ aryl, and 5- to 14-membered heteroaryl; or R₂₃ and R₂₄, together with the C atom to which they are attached, form a 3- to 8-membered cycloalkyl or 3- to 8-membered heterocycloalkyl; r2 is 0, 1, 2, 3 or 4;
Z₁ is CH or N, and Z₂ is CR_{z1}R_{z2}, NR₀₀, or C(O);
R_{z1} and R_{z2} are each independently selected from hydrogen, hydroxy, cyano, acetyl, halogen, C₁₋₁₀ alkyl, C₁₋₁₀ haloalkyl, C₁₋₁₀ alkoxy, C₁₋₁₀ alkylthio, NRₐR_{b}, C₃₋₈ cycloalkyl, 3- to 8-membered heterocycloalkyl, C₆₋₁₀ aryl, and 5- to 14-membered heteroaryl; or R_{z1} and R_{z2}, together with the C atoms to which they are attached, form a 3- to 8-membered cycloalkyl or 3- to 8-membered heterocycloalkyl;
G₁ is N or CR_{G1}, G₂ is N or CR_{G2}, G₃ is N or CR_{G3}, and G₄ is N or CR_{G4};
one of R_{G1}, R_{G2}, R_{G3}, and R_{G4} is attached to W, and the others are each independently selected from the group consisting of hydrogen, hydroxy, cyano, acetyl, halogen, amino, C₁₋₁₀ alkyl, C₁₋₁₀ haloalkyl, C₁₋₁₀ alkoxy, C₁₋₁₀ alkylthio, NRₐR_{b}, C₃₋₈ cycloalkyl, 3- to 8-membered heterocycloalkyl, C₆₋₁₀ aryl, and 5- to 14-membered heteroaryl;
R₀₀, Rₐ, and R_{b} are each independently hydrogen, cyano, acetyl, hydroxy, carboxy, nitro, halogen, C₁₋₁₀ alkyl, C₁₋₁₀ haloalkyl, C₁₋₁₀ alkoxy, C₁₋₁₀ alkylthio, NR_{c}R_{d}, C₃₋₈ cycloalkyl, 3- to 8-membered heterocycloalkyl, C₆₋₁₀ aryl or 5- to 14-membered heteroaryl; or Rₐ and R_{b}, together with the N atom to which they are attached, form a 3- to 8-membered heterocycloalkyl;
R_{c} and R_{d} are each independently hydrogen, C₁₋₅ alkyl, C₁₋₅ alkoxy, or C₁₋₅ haloalkyl; or R_{c} and R_{d}, together with the N atom to which they attached, form a 3- to 8-membered heterocycloalkyl;
the above C₁₋₁₀ alkyl, C₁₋₁₀ haloalkyl, C₁₋₁₀ alkoxy, C₁₋₁₀ alkylthio, C₁₋₅ alkyl, C₁₋₅ haloalkyl, C₁₋₅ alkoxy, C₃₋₈ cycloalkyl, 3- to 8-membered heterocycloalkyl, C₆₋₁₀ aryl, or 5- to 14-membered heteroaryl, saturated or unsaturated monocycle, monocyclic heterocycle, polycycle and polycyclic heterocycle are unsubstituted or, within a valence-permitted range, each independently substituted by 1, 2, 3, 4, or more groups selected from the following B2 groups, and the B2 group is selected from hydrogen, cyano, acetyl, hydroxy, carboxy, nitro, halogen, C₁₋₅ alkyl, C₁₋₅ alkoxy, C₁₋₅ alkylthio, NRₑR_{f}, 3- to 8-membered cycloalkyl, 3- to 8-membered heterocycloalkyl, C₆₋₁₀ aryl, and 5- to 14-membered heteroaryl, wherein Rₑ and R_{f} are each independently hydrogen, C₁₋₅ alkyl, C₁₋₅ alkoxy, or C₁₋₅ haloalkyl;
provided that:
when PIN is formula (Ia) and Q is S(O)₂-isopropyl, neither Rₖ₃ nor Rₖ₄ is methyl;
when PIN is formula (Ib) and ring B is pyrazine, neither Rₖ₃ nor Rₖ₄ is methyl.

3. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 1 or 2, wherein,
Rₖ₂ and Rₖ₄ are each independently selected from hydrogen, deuterium, halogen, cyano, acylamino, C₁₋₅ alkyl, C₁₋₅ haloalkyl, C₁₋₅ alkoxy, C₁₋₃ alkylene-OC₁₋₃ alkyl, C₃₋₆ cycloalkyl, and C₃₋₆ heterocycloalkyl; the C₁₋₅ alkyl, C₁₋₅ haloalkyl, C₁₋₅ alkoxy, C₁₋₃ alkylene-OC₁₋₃ alkyl, C₃₋₆ cycloalkyl, and C₃₋₆ heterocycloalkyl are optionally substituted with 0, 1, 2, 3, or more substituents selected from deuterium, hydroxy, cyano, C₁₋₅ alkyl, C₁₋₅ haloalkyl, and C₁₋₅ alkoxy; or Rₖ₁ and Rₖ₂ are each independently hydrogen, F, Cl, Br, I, amino, hydroxy, cyano, acetyl, C₁₋₅ alkyl, C₁₋₅ haloalkyl, C₁₋₅ alkoxy, C₁₋₃ alkylene-OC₁₋₃ alkyl, C₃₋₆ cycloalkyl, or C₃₋₆ heterocycloalkyl; preferably, Rₖ₂ is hydrogen, methyl, ethyl, propyl, isopropyl, methoxy, ethoxy, propoxy, isopropoxy, cyclopropyl, cyclobutyl, or -O-oxetanyl; preferably R_{K4} is selected from hydrogen, deuterium, acylamino, cyano or C₁₋₅ alkyl, preferably methyl, ethyl, propyl, isopropyl, C₁₋₅ haloalkyl and C₁₋₅ alkoxy; and/or, X₁ is selected from O, S, NR₀₀, -C(O)-, -S(O)-, SO₂, -C(O)NR₀₀-, -NR₀₀C(O)-, -SO₂NR₀₀-, -NR₀₀SO₂-, -NR₀₀C(O)NR₀₀-, -NR₀₀C(O)O-, -OC(O)O-, -CH=CH-, -C≡C-, C₁₋₅ alkylene, C₃₋₆ cycloalkylene, 3- to 6-membered heterocycloalkylene, phenylene, 5- to 6-membered heteroarylene, and 8- to 10-membered bicyclic heteroarylene, wherein R₀₀ is selected from hydrogen and C₁₋₃ alkyl (preferably methyl, ethyl, or propyl;
preferably hydrogen or methyl);
and/or, Rₖ₃ is selected from F, Cl, Br, I, hydroxy, cyano, formyl, nitro, carboxy, acetyl, -CO-amino, SOC₁₋₅ alkyl, SO₂C₁₋₅ alkyl, C₁₋₅ alkyl, C₁₋₅ haloalkyl, C₁₋₅ alkoxy, C₁₋₅ alkylene-OC₁₋₅ alkyl, C₃₋₆ cycloalkyl, 3- to 6-membered heterocycloalkyl, -O-C₃₋₆ cycloalkyl, and -O-3- to 6-membered heterocycloalkyl; preferably, Rₖ₃ is hydroxy, cyano, formyl, nitro, carboxy, acetyl, -CO-amino, or -SO₂-methyl; or Rₖ₃ is F, Cl, Br, I, methyl, ethyl, propyl, isopropyl, methoxy, ethoxy, propoxy, isopropoxy, cyclopropyl, cyclobutyl, oxetanyl, fluoromethyl, difluoromethyl, trifluoromethyl, -CH₂CH₂F, -CHFCH₃, -CH₂CHF₂, -CF₂CH₃, -CHFCH₂F, -CH₂CF₃, - CF₂CH₂F, or -CHFCHF₂; or, Rₖ₃ is a C₁₋₆ alkyl, C₁₋₆ haloalkyl, C₁₋₆ alkoxy, C₁₋₅ alkylene-OC₁₋₅ alkyl, C₃₋₆ cycloalkyl, 3- to 6-membered heterocycloalkyl, -O-C₃₋₆ cycloalkyl, or -O-3- to 6-membered heterocycloalkyl; the C₁₋₆ alkyl, C₁₋₆ haloalkyl, C₁₋₆ alkoxy, C₁₋₅ alkylene, C₁₋₅ alkyl, C₃₋₆ cycloalkyl, or 3- to 6-membered heterocycloalkyl is optionally substituted with 0, 1, 2, 3, 4, 5, or more substituents selected from deuterium, halogen, hydroxy, cyano, C₁₋₃ alkyl, C₁₋₃ haloalkyl, and C₁₋₃ alkoxy;
and/or, R₄ is hydroxy, cyano, acetyl, carboxy, nitro, halogen, amino, C₁₋₆ alkyl, C₁₋₆ haloalkyl, C₁₋₆ alkoxy, C₁₋₆ alkylthio, C₂₋₆ alkenyl, C₂₋₆ alkynyl, NRₐR_{b}, C₃₋₈ cycloalkyl, 3- to 8-membered heterocyclyl, C₆₋₁₀ aryl, or 5-to 14-membered heteroaryl; the C₁₋₆ alkyl, C₁₋₆ haloalkyl, C₁₋₆ alkoxy, C₁₋₆ alkylthio, C₂₋₆ alkenyl, C₂₋₆ alkynyl, C₃₋₈ cycloalkyl, 3- to 8-membered heterocyclyl, C₆₋₁₀ aryl, and 5- to 14-membered heteroaryl are optionally substituted with 0, 1, 2, or 3 R⁴¹s; or, R₄ is halogen, C(O)NRₐR_{b}, C₃₋₈ cycloalkyl, 3- to 8-membered heterocyclyl, phenyl, 5- or 6-membered heteroaryl, 7- to 11-membered spirocyclyl, or 8- to 12-membered spiroheterocyclyl; the C₃₋₈ cycloalkyl, 3- to 8-membered heterocyclyl, phenyl, 5- or 6-membered heteroaryl, 7- to 11-membered spirocyclyl, and 8- to 12-membered spiroheterocyclyl are optionally substituted with 0, 1, 2, or 3 R⁴¹s; preferably, R₄ is selected from fluorine, chlorine, bromine, amino, acylamino, acetyl, cyclopropyl, cyclobutyl, oxetanyl, cyclopentyl, trifluoromethyl, and C₁₋₆ alkyl; preferably, R₄ is hydrogen, -O-C₀₋₅ alkylene-C₃₋₈ cycloalkyl, or -OC₀₋₅ alkylene-3- to 8-membered heterocyclyl; preferably, R₄ is phenyl or 5- or 6-membered heteroaryl substituted with 0, 1, or 2 R⁴¹s; preferably, R₄ is selected from furanyl, thienyl, pyrrolyl, pyrazolyl, imidazolyl, thiazolyl, isothiazolyl, oxazolyl, isoxazolyl, triazolyl, phenyl, pyridinyl, pyrimidinyl, cyclopentyl, tetrahydrofuranyl, tetrahydrothienyl, tetrahydropyrrolyl, cyclohexyl, oxacyclohexyl, piperidinyl, piperazinyl, and morpholinyl substituted with 0, 1, or 2 R⁴¹s; R⁴¹ is selected from hydrogen, deuterium, halogen, C₁₋₆ alkyl, C₁₋₆ haloalkyl, C₁₋₆ alkoxy, C₁₋₆ alkylthio, C₃₋₈ cycloalkyl, 3- to 8-membered heterocyclyl, OC₃₋₈ cycloalkyl, and -O-3- to 8-membered heterocyclyl (R⁴¹ is preferably hydrogen, deuterium, hydroxy, methyl, ethyl, propyl, isopropyl, trifluoromethyl, trifluoroethyl, methoxy, ethoxy, propoxy, cyclopropyl, cyclobutyl, oxetanyl, cyclopentyl, or azacyclopentyl 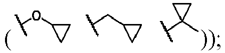
and/or, ring A is a 3- to 8-membered monocyclic heterocyclylene, 7- to 16-membered spiroheterocyclylene, 7-to 14-membered fused heterocyclylene, 7- to 10-membered bridged heterocyclylene, phenylene, 5- or 6-membered monocyclic heteroarylene, or 8- to 10-membered bicyclic heteroarylene; preferably ring A is a 5-to 7-membered monocyclic heterocyclylene; preferably, ring A is a 7- to 11-membered mono-spiroheterocyclylene;
and/or, the pharmaceutically acceptable salt is a hydrochloride, a formate, or a trifluoroacetate;
and/or, Linker represents 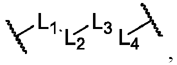 and L₁, L₂, L₃ and L₄ each independently represent -R_{L1}-(CR₂₁R₂₂)ₙ₃-R_{L2}-(CR₃₁R₃₂)ₙ₂-, R_{L1} and R_{L2} are each independently a bond, O, NR₀₀, -C(O)-, -CH=CH-, -C≡C-, -(CR₄₁R₄₂)ᵣ-, or C₃₋₈ cycloalkylene; n2, n3, and r are each independently 0, 1, 2, 3, or 4; R₄₁ and R₄₂ are each independently selected from hydrogen, acetyl, halogen, C₁₋₅ alkyl, C₁₋₅ haloalkyl, or C₆₋₁₀ aryl; or, R₂₁ and R₂₂, together with the C atom to which they are attached, form a 3- to 8-membered cycloalkyl; preferably, Linker represents 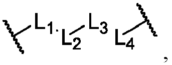 and L₁, L₂, L₃ and L₄ each independently represent -R_{L1}-(CR₂₁R₂₂)ₙ₃-R_{L2}-(CR₃₁R₃₂)ₙ₂-; R_{L1} is each independently a bond, O, NR₀₀, -C(O)-, -(CR₄₁R₄₂)ᵣ-, or C₃₋₈ cycloalkylene; R_{L2} is each independently a bond, O, NR₀₀, -C(O)-, -CH=CH-, -C≡C-, or C₃₋₈ cycloalkylene; n2, n3, and r are each independently 0, 1, 2, 3, or 4; R₄₁ and R₄₂ are each independently selected from hydrogen or C₁₋₅ alkyl; or, R₂₁ and R₂₂, together with the C atom to which they are attached, form a 3- to 8-membered cycloalkyl.

4. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 1 or 2, wherein, Rₖ₂ is -CONH₂.

5. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 1 or 2, wherein,
X₁ is -(CRₓ₁Rₓ₂)ₓ-,
Rₓ₁ and Rₓ₂ are each independently selected from hydrogen, C₁₋₅ alkyl (preferably C₁₋₃ alkyl, more preferably methyl, ethyl, propyl or isopropyl) and C₁₋₅ alkoxy, or Rₓ₁ and Rₓ₂, together with the C atoms to which they are attached, form a C₃₋₆ cycloalkyl (preferably cyclopropyl or cyclobutyl) or 3- to 6-membered heterocycloalkyl (preferably oxetanyl); x is 0, 1, 2, 3, or 4.

6. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 1 or 2, wherein,
Rx is hydrogen, cyano, hydroxy, acetyl, halogen, amino, C₁₋₅ alkyl, C₁₋₅ haloalkyl, C₁₋₅ alkoxy, C₂₋₁₀ alkenyl, C₂₋₁₀ alkynyl, C₃₋₆ cycloalkyl, 3- to 6-membered heterocycloalkyl, benzene, 5- to 6-membered heteroaryl, or 8- to 10-membered bicyclic heteroaryl.

7. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 1 or 2, wherein,
Rₖ₃ represents -X₁-Px, and X₁ is selected from a C₃₋₈ cycloalkylene, 3- to 8-membered heterocycloalkylene, C₆₋₁₀ arylene, and 5- to 14-membered heteroarylene; Rx represents -(CRₓ₃Rₓ₄)ₓ₂-Rₓₙ, and Rₓ₃, Rₓ₄ and Rₓₙ are each independently selected from hydrogen, cyano, hydroxy, acetyl, halogen, nitro, formyl, carboxy, amino, C₁₋₅ alkyl, C₁₋₅ haloalkyl, C₁₋₅ alkoxy, C₁₋₅ alkylthio, C₃₋₈ cycloalkyl, 3- to 8-membered heterocycloalkyl, C₆₋₁₀ aryl, and 5- to 14-membered heteroaryl; or Rₓ₁ and Rₓ₂, together with the C atom to which they are attached, form a 3- to 8-membered cycloalkyl or 3- to 8-membered heterocycloalkyl; x2 is 0, 1, 2, 3, or 4.

8. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 3, wherein,
X₁ is a 5- to 6-membered heteroarylene or 8- to 10-membered bicyclic heteroarylene, and
Rx is H, F, Cl, Br, I, methyl, ethyl, propyl, isopropyl, methoxy, ethoxy, propoxy, isopropoxy, cyclopropyl, cyclobutyl, oxetanyl, fluoromethyl, difluoromethyl, trifluoromethyl, -CH₂CH₂F, -CHFCH₃, -CH₂CHF₂, - CF₂CH₃, -CHFCH₂F, -CH₂CF₃, -CF₂CH₂F, or -CHFCHF₂.

9. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 2, wherein,
Rₖ₃ is selected from 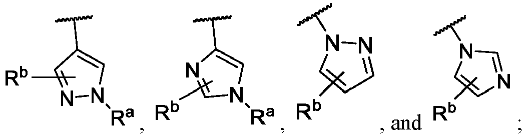
R^{a} and R^{b} are H, F, Cl, Br, I, methyl, ethyl, propyl, isopropyl, methoxy, ethoxy, propoxy, isopropoxy, cyclopropyl, cyclobutyl, oxetanyl, fluoromethyl, difluoromethyl, trifluoromethyl, -CH₂CH₂F, -CHFCH₃, - CH₂CHF₂, -CF₂CH₃, -CHFCH₂F, -CH₂CF₃, -CF₂CH₂F, or -CHFCHF₂; preferably, Rₖ₃ is a 5- or 6-membered heterocyclyl substituted with 0, 1, or 2 substituents selected from R^{b}, and R^{b} is selected from hydrogen, deuterium, halogen, cyano, C₁₋₅ alkyl, C₁₋₅ haloalkyl, and C₁₋₅ alkoxy.

10. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 2, wherein,
ring A represents: 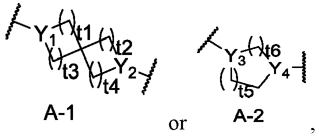 wherein, Y₁ and Y₂ are each independently CH or N, Y₃ and Y₄ are each independently CH or N, t1, t2, t3 and t4 are each independently 0, 1, 2 or 3, and t5 and t6 are each independently 0, 1, 2 or 3, provided that: t1 and t3 are not both 0, and t2 and t4 are not both 0.

11. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 3, wherein, ring A is selected from 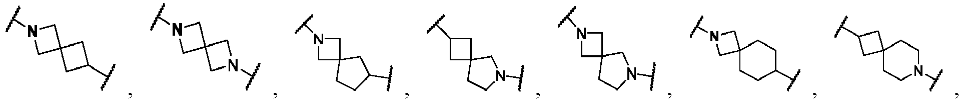 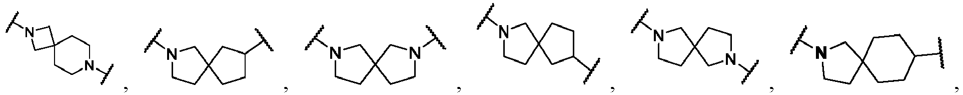 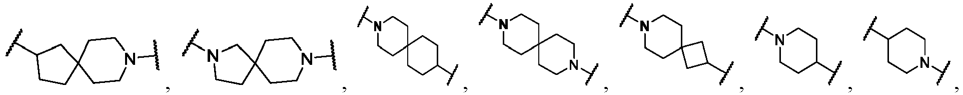 and

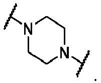

12. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 2, wherein,
R is a bond, or R is a 3- to 8-membered monocyclic heterocyclylene, 7- to 16-membered spiro heterocyclylene,
7- to 10-membered bicyclic fused heterocyclylene, 7- to 10-membered bridged heterocyclylene, 5- or 6-membered heteroarylene, or 8- to 10-membered bicyclic heteroarylene; preferably, R is a bond; preferably, R is a 5- to 7-membered monocyclic heterocyclylene; preferably, R is a 7- to 11-membered mono-spiroheterocyclylene or 8- to 10-membered bicyclic heteroarylene; preferably, R is a 7- to 11-membered mono-spiroheterocyclylene or 8- to 10-membered bicyclic heteroarylene substituted with 0, 1, 2, or 3 R₀s; R₀ is selected from hydrogen, deuterium, halogen, =O, C₁₋₆ alkyl, C₁₋₆ haloalkyl, C₁₋₆ alkoxy, C₃₋₈ cycloalkyl, or 3-to 8-membered heterocyclyl, or two R₀s, together with the C atom to which they are attached, form a 3- to 6-membered cycloalkyl or heterocyclyl.

13. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 2, wherein, R represents: 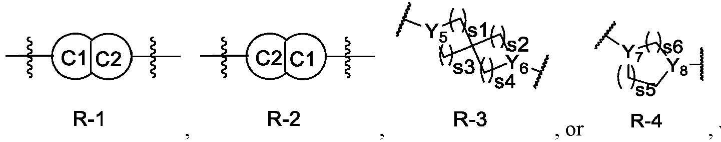 wherein
ring C1 is a 5- to 6-membered heteroaryl or benzene, and ring C2 is a 5- to 6-membered saturated or unsaturated monocycle or a 5- to 6-membered saturated or unsaturated monoheterocycle, wherein the 5- to 6-membered heteroaryl, benzene, 5- to 6-membered saturated or unsaturated monocycle, or 5- to 6-membered saturated or unsaturated monoheterocycle is optionally substituted with 0, 1, 2, 3, or more substituents selected from hydrogen, D, halogen, amino, hydroxy, cyano, acetyl, C₁₋₅ alkyl, C₁₋₅ alkoxy, C₃₋₈ cycloalkyl, 5- to 7-membered heterocycloalkyl, 6- to 10-membered aryl, and 5- to 14-membered heteroaryl; preferably, ring C2 is a 5- to 7-membered azacycle;
Y₅ and Y₆ are each independently CH or N, Y₇ and Y₈ are each independently CH or N, s1, s2, s3 and s4 are each independently 0, 1, 2 or 3, and s5 and s6 are each independently 0, 1, 2 or 3, provided that: s1 and s3 are not both 0, and s2 and s4 are not both 0.

14. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 12, wherein,
ring C1 is selected from furan, thiophene, pyrrole, pyrazole, imidazole, thiazole, isothiazole, oxazole, isoxazole, triazole, benzene, pyridine, pyrimidine, pyrazine, or pyridazine; ring C2 is selected from cyclopentane, tetrahydrofuran, tetrahydrothiophene, tetrahydropyrrole, furan, thiophene, pyrrole, pyrazole, imidazole, thiazole, isothiazole, oxazole, isoxazole, triazole, cyclohexane, oxacyclohexane, piperidine, piperazine, morpholine, benzene, pyridine, pyrimidine, pyrazine, and pyridazine;
and/or R is selected from 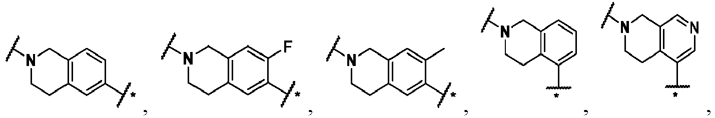 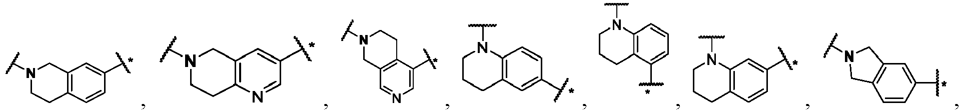 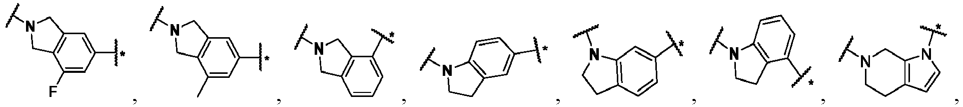 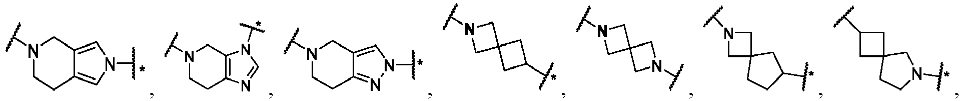 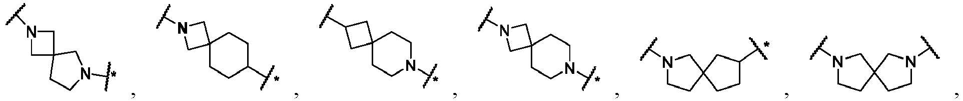 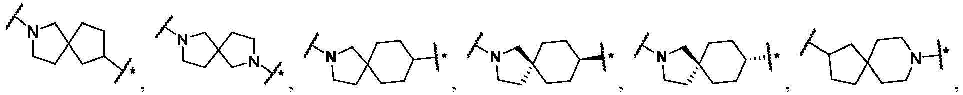 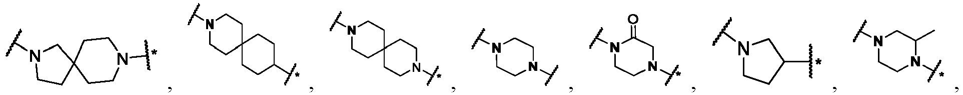 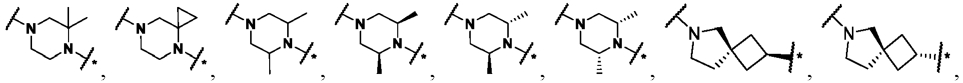 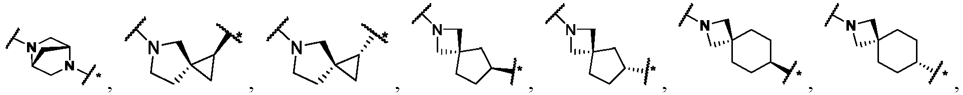 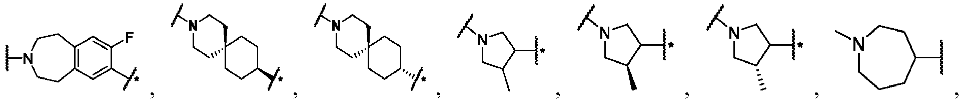 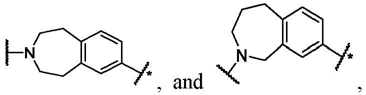 wherein 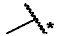 is connected with Linker while the other end is connected with ring A, or 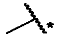 is connected with ring A while the other end is connected with Linker;
and/or, L₁, L₂, L₃, and L₄ are each independently selected from -R_{L1}-(CH₂)ₙ₅-R_{L2}-(CH₂)ₙ₆-, wherein R_{L1} and R_{L2} are each independently O, S, NR₀₀, -C(O)-, -C(O)NR₀₀-, -NR₀₀C(O)-, -SO₂NR₀₀-, -NR₀₀SO₂-, - NR₀₀C(O)NR₀₀-, -NR₀₀C(O)O-, -OC(O)-, -C(O)O-, -OC(O)O-, and (CH₂)ᵣ₄; n5, n6, and r4 are each independently 0, 1, 2, 3, or 4, and R₀₀ is a C₁₋₃ alkyl (preferably methyl);
preferably, R_{L1} and R_{L2} are each independently selected from a bond, 5- or 6-membered heteroarylene, phenylene, C₃₋₈ cycloalkylene, C₃₋₈ heterocyclylene, CH₂, CH₂CH₂, OCH₂CH₂, CH₂CH₂O, CH₂CH₂CH₂, ethenylene, ethynylene, 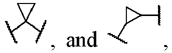 wherein, the 5- or 6-membered heteroarylene, phenylene, C₃₋₈ cycloalkylene, C₃₋₈ heterocyclylene, and any methylene are optionally substituted, within their respective valence-permitted ranges, with 0, 1, 2, or 3 substituents selected from hydroxy, cyano, halogen, C₁₋₆ alkyl, C₁₋₆ haloalkyl, C₁₋₆ alkoxy, C₁₋₆ alkylthio, C₂₋₆ alkenyl, C₂₋₆ alkynyl, NRₐR_{b}, acetyl, -C(O)NRₐR_{b}, C₃₋₈ cycloalkyl, 3- to 8-membered heterocyclyl, C₆₋₁₀ aryl, and 5- to 14-membered heteroaryl.

15. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 2, wherein,
L₁ is (CR₂₁R₂₂)ₙ₃, wherein R₂₁ and R₂₂ are each independently hydrogen, methyl, ethyl, propyl, isopropyl, cyclopropyl, cyclobutyl, oxetanyl, methoxy, ethoxy or propoxy, and n3 is 0, 1, 2 or 3; preferably L₁ is (CR₂₁R₂₂)ₙ₃, wherein R₂₁ and R₂₂, together with the C atom to which they are attached, form cyclopropyl, cyclobutyl, cyclopentyl or oxetanyl, and n₃ is 1 or 2;
preferably, L₁ is O, S, NR₀₀, C(O), C(O)NR₀₀, NR₀₀C(O), -C(O)O-, -OC(O)-, SO, SO₂, SO₂NR₀₀, or NR₀₀SO₂; R₀₀ is hydrogen or methyl;
preferably, L₁ is -CH=CH- or -C≡C-;
preferably, L₁ is CH₂, CH₂CH₂, or CH₂CH₂CH₂ (preferably CH₂ or CH₂CH₂);
preferably, L₁ is a bond;
preferably, L₁ is a C₃₋₆ cycloalkylene, 4- to 7-membered heterocyclylene, phenylene, or 5- or 6-membered heteroarylene (preferably cyclopropylene, cyclobutylene, cyclopentylene, azacyclopentylene, cyclohexylene, piperidylene, piperazinylene, 1,4-phenylene, 2,5-1*H-*pyrrolylene, 3,5-1*H-*pyrrolylene, 3,5-1*H*-pyrazolylene, 2,5-1*H*-triazolylene, or tetrazolylene);
and/or, L₂ is (CR₂₁R₂₂)ₙ₃, wherein R₂₁ and R₂₂ are each independently hydrogen, methyl, ethyl, propyl, isopropyl, cyclopropyl, cyclobutyl, oxetanyl, methoxy, ethoxy or propoxy, and n3 is 0, 1, 2 or 3; preferably L₂ is (CR₂₁R₂₂)ₙ₃, wherein R₂₁ and R₂₂, together with the C atom to which they are attached, form cyclopropyl, cyclobutyl, cyclopentyl or oxetanyl, and n₃ is 1 or 2;
preferably, L₂ is O, S, NR₀₀, C(O), C(O)NR₀₀, NR₀₀C(O), -C(O)O-, -OC(O)-, SO, SO₂, SO₂NR₀₀, or NR₀₀SO₂;
R₀₀ is hydrogen or methyl;
preferably, L₂ is -CH=CH- or -C≡C-;
preferably, L₂ is CH₂, CH₂CH₂, or CH₂CH₂CH₂ (preferably CH₂ or CH₂CH₂);
preferably, L₂ is a bond;
preferably, L₂ is a C₃₋₆ cycloalkylene, 4- to 7-membered heterocyclylene, phenylene, or 5- or 6-membered heteroarylene;
and/or, L₃ is (CR₂₁R₂₂)ₙ₃, wherein R₂₁ and R₂₂ are each independently hydrogen, methyl, ethyl, propyl, isopropyl, cyclopropyl, cyclobutyl, oxetanyl, methoxy, ethoxy or propoxy, and n3 is 0, 1, 2 or 3; preferably L₃ is (CR₂₁R₂₂)ₙ₃, wherein R₂₁ and R₂₂, together with the C atom to which they are attached, form cyclopropyl, cyclobutyl, cyclopentyl or oxetanyl, and n₃ is 1 or 2;
preferably, L₃ is O, S, NR₀₀, C(O), C(O)NR₀₀, NR₀₀C(O), -C(O)O-, -OC(O)-, SO, SO₂, SO₂NR₀₀, or NR₀₀SO₂; R₀₀ is hydrogen or C₁₋₃ alkyl (preferably methyl, ethyl, or propyl);
preferably, L₃ is -CH=CH- or -C≡C-;
preferably, L₃ is CH₂, CH₂CH₂, or CH₂CH₂CH₂ (preferably CH₂ or CH₂CH₂);
preferably, L₃ is a bond;
preferably, L₃ is a C₃₋₆ cycloalkylene, 4- to 7-membered heterocyclylene, phenylene, or 5- or 6-membered heteroarylene;
and/or, L₄ is (CR₂₁R₂₂)ₙ₃, wherein R₂₁ and R₂₂ are each independently hydrogen, methyl, ethyl, propyl, isopropyl, cyclopropyl, cyclobutyl, oxetanyl, methoxy, ethoxy or propoxy, and n3 is 0, 1, 2 or 3; preferably L₄ is (CR₂₁R₂₂)ₙ₃, wherein R₂₁ and R₂₂, together with the C atom to which they are attached, form cyclopropyl, cyclobutyl, cyclopentyl or oxetanyl, and n₃ is 1 or 2;
preferably, L₄ is O, S, NR₀₀, C(O), C(O)NR₀₀, NR₀₀C(O), -C(O)O-, -OC(O)-, SO, SO₂, SO₂NR₀₀, or NR₀₀SO₂; R₀₀ is hydrogen or methyl;
preferably, L₄ is -CH=CH- or -C≡C-;
preferably, L₄ is CH₂, CH₂CH₂, or CH₂CH₂CH₂ (preferably CH₂ or CH₂CH₂);
preferably, L₄ is a bond;
preferably, L₄ is a C₃₋₆ cycloalkylene, 4- to 7-membered heterocyclylene, phenylene, or 5- or 6-membered heteroarylene;
and/or, W is a bond, O, S, NR₀₀, -C(O)-, -S(O)-, SO₂, -C(O)NR₀₀-, -NR₀₀C(O)-, -NR₀₀C(O) NR₀₀-, -SO₂NR₀₀-, -NR₀₀SO₂-, -NR₀₀C(O)O-, -C(O)O-, -OC(O)-, -OC(O)O-, -CH=CH-, -C=C-, -(CR₄₃R₄₄)ₙ₈-, C₃₋₆ cycloalkylene, 3- to 6-membered heterocycloalkylene, phenylene, 5- to 6-membered heteroarylene, or 8- to 10-membered arylene or heteroarylene, wherein R₀₀, R₄₃, and R₄₄ are each independently hydrogen, methyl, ethyl, propyl, isopropyl, methoxy, ethoxy, isopropoxy, cyclopentyl, or cyclohexyl, or R₄₃ and R₄₄, together with the C atom to which they are attached, form cyclopropyl, cyclobutyl, cyclopentyl, or oxetanyl, and n₈ is 0, 1, 2, or 3;
preferably, W is 1,2-phenylene, 1,3-phenylene, 1,4-phenylene, piperazinylene, or pyridinylene; preferably, W is CH₂, O, S, -N(methyl)-, -CH=CH-, or C≡C; preferably, W is CH₂; preferably, W is O or S; preferably, W is -CH=CH- or C≡C;
and/or, ring B is selected from a 3- to 8-membered saturated or unsaturated cycloalkyl, 3- to 8-membered saturated or unsaturated heterocycloalkyl, 6- to 10-membered aryl, and 5- to 14-membered heteroaryl; preferably, ring B is a 5- to 7-membered saturated monocyclic heterocyclyl, benzene, or 5- to 6-membered heteroaryl; preferably, ring B is benzene or 5- to 6-membered heteroaryl; preferably, ring B is a 5- to 7-membered saturated monoheterocyclyl; preferably, ring B is cyclopentane, tetrahydropyrrole, 1,3-dioxolane, oxolane, 1,4-dioxane, furan, thiophene, thiazole, isothiazole, oxazole, isoxazole, pyrrole, imidazole, pyrazole, piperidine, piperazine, benzene, pyridine, pyridazine, or pyrimidine; preferably, ring B is 1,3-dioxolane, oxolane, 1,4-dioxane, imidazole, benzene, or pyrimidine; preferably, ring B is a 5- to 6-membered heteroaryl; preferably, ring B is 1,4-dioxane or 1,3-dioxolane.

16. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 2, wherein, ring B is selected from 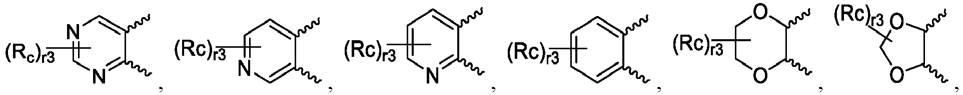 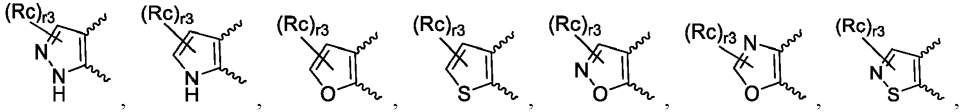 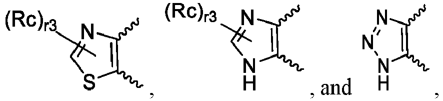
" 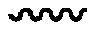 " represents a connection site;
Rc is hydrogen, F, Cl, Br, I, hydroxy, cyano, formyl, nitro, carboxy, acetyl, SOC₁₋₅ alkyl, SO₂C₁₋₅ alkyl, C₁₋₅ alkyl, C₁₋₅ haloalkyl, C₁₋₅ alkoxy, C₁₋₅ alkyl-OC₁₋₅ alkyl, C₃₋₆ cycloalkyl, 3- to 6-membered heterocycloalkyl, -O-C₃₋₆ cycloalkyl, or -O-3- to 6-membered heterocycloalkyl; r3 is 0, 1, 2, or 3;
and/or, V₁ and V₂ are both N, V₃ is CR₃, and V₄ is CR₄, wherein R₃ is hydrogen, and R₄ is hydrogen, halogen, amino, cyano, acetyl, C₁₋₃ alkyl, C₁₋₃ alkoxy, or C₁₋₃ haloalkyl (preferably fluorine or chlorine);
preferably, V₁ and V₂ are both N, V₃ is CR₃, and V₄ is CR₄, wherein R₃ and R₄, together with the atoms to which they are attached, form a substituted or unsubstituted 5- to 6-membered heteroaryl; the substitution refers to that the 5- to 6-membered heteroaryl is optionally substituted with 1, 2, or 3 substituents selected from hydrogen, halogen, amino, cyano, acetyl, C₁₋₃ alkyl, C₁₋₃ alkoxy, and C₁₋₃ haloalkyl;
preferably, R₃ and R₄, together with the atoms to which they are attached, form 1*H-*pyrrole, 1*H*-pyrazole, or 1*H-*imidazole;
and/or, L₅ and L₆ are each independently selected from a bond, NH, CONH, NHCO, C₁₋₃ alkylene (preferably methylene or ethylene), C₂₋₆ alkenylene, and C₂₋₆ alkynylene;
and/or, E is selected from 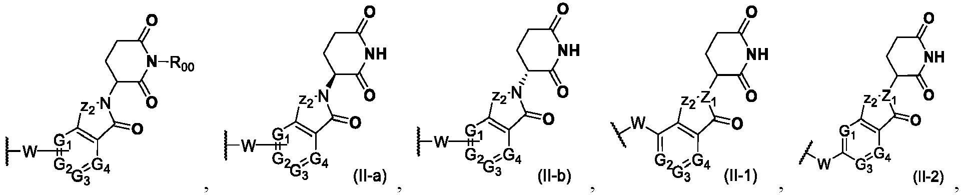 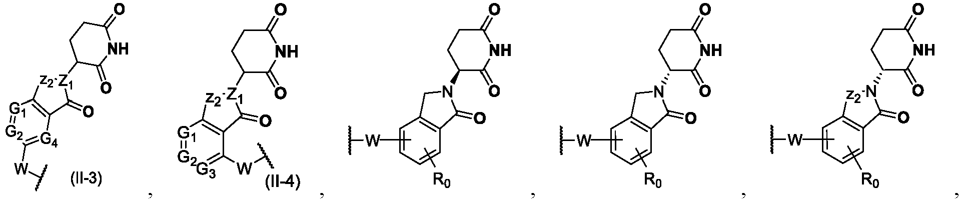 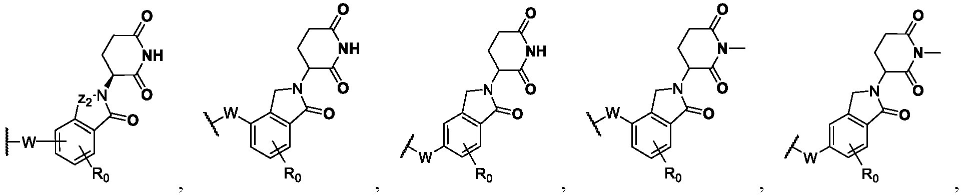 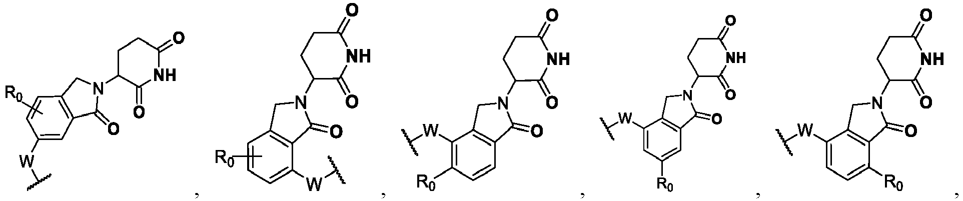 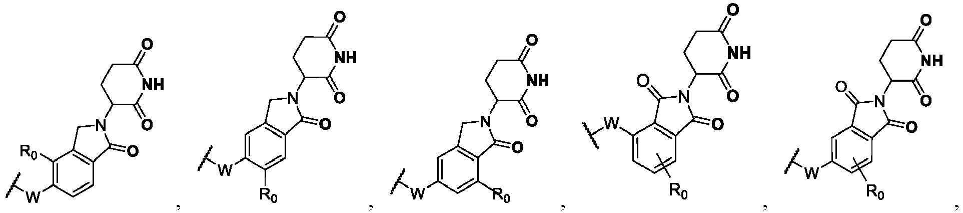 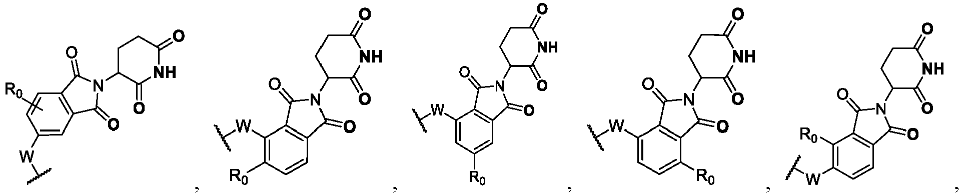 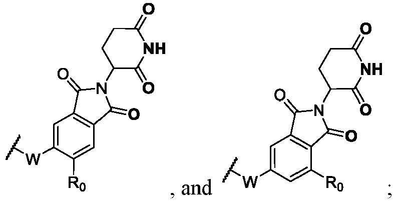
one of R_{G1}, R_{G2}, R_{G3}, and R_{G4} is attached to W, the others are each independently selected from hydrogen, hydroxy, cyano, acetyl, fluorine, chlorine, bromine, amino, methyl, ethyl, propyl, isopropyl, methoxy, ethoxy, fluoromethyl, difluoromethyl, trifluoromethyl, -CH₂CH₂F, -CHFCH₃, -CH₂CHF₂, -CF₂CH₃, -CHFCH₂F, - CH₂CF₃, -CF₂CH₂F, and -CHFCHF₂; preferably, R_{G1}, R_{G2}, R_{G3}, and R_{G4} are each independently hydrogen, halogen (preferably F), C₁₋₃ alkyl, or C₁₋₃ haloalkyl;
preferably, R_{G1}, R_{G2}, R_{G3}, R_{G4}, and R₀ are each independently hydrogen, deuterium, cyano, halogen (preferably F), C₁₋₃ alkyl, or C₁₋₃ haloalkyl (preferably methyl or trifluoromethyl).

17. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 2, wherein,
J₁ is N, J₂ is CR_{J2}, J₃ is CR_{J3}, and J₄ is CR_{J4}; or J₁ is CR_{J1}, J₂ is N, J₃ is CR_{J3}, and J₄ is CR_{J4}; or J₁ is CR_{J1}, J₂ is CR_{J2}, J₃ is N, and J₄ is CR_{J4}; or J₁ is CR_{J1}, J₂ is CR_{J2}, J₃ is CR_{J3}, and J₄ is N; or J₁ is N, J₂ is N, J₃ is CR_{J3}, and J₄ is CR_{J4}; or J₁ is N, J₂ is CR_{J2}, J₃ is N, and J₄ is CR_{J4}; or J₁ is N, J₂ is CR_{J2}, J₃ is CR_{J3}, and J₄ is N; or J₁ is CR_{J1}, J₂ is N, J₃ is N, and J₄ is CR_{J4}; or J₁ is CR_{J1}, J₂ is N, J₃ is CR_{J3}, and J₄ is N; or J₁ is CR_{J1}, J₂ is CR_{J2}, J₃ is N, and J₄ is N.

18. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 2, wherein,
R_{J1}, R_{J2}, R_{J3}, and R_{J4} are each independently selected from H, F, Cl, Br, I, methyl, ethyl, propyl, isopropyl, methoxy, ethoxy, propoxy, isopropoxy, cyclopropyl, cyclobutyl, oxetanyl, fluoromethyl, difluoromethyl, trifluoromethyl, -CH₂CH₂F, -CHFCH₃, -CH₂CHF₂, -CF₂CH₃, -CHFCH₂F, -CH₂CF₃, -CF₂CH₂F, and - CHFCHF₂;
preferably, R_{J3} and R_{J4} are hydrogen, and R_{J1} and R_{J2} are each independently selected from F, Cl, Br, I, methyl, ethyl, propyl, cyclopropyl, or a substituted or unsubstituted benzene or 5- to 6-membered heteroaryl; the substitution refers to that the benzene or 5- to 6-membered heteroaryl is optionally substituted with 1, 2, or 3 substituents selected from hydrogen, halogen, amino, cyano, acetyl, C₁₋₃ alkyl, C₁₋₃ alkoxy, and C₁₋₃ haloalkyl; preferably, R_{J1}, R_{J3}, and R_{J4} are hydrogen, and R_{J2} is selected from H, F, Cl, Br, methyl, ethyl, 3- to 8-membered cycloalkyl, benzene, or 5- to 6-membered heteroaryl; or R_{J1}, R_{J2}, R_{J3}, and R_{J4} are hydrogen; preferably, R_{J1}, R_{J3}, and R_{J4} are hydrogen, and R_{J2} is methyl, ethyl, cyclopropyl, benzene, or 1-methyl-1*H*-pyrazol-4-yl, 1-methyl-1*H*-imidazol-4-yl, 1-ethyl-1*H*-pyrazol-4-yl, 1-ethyl-1*H*-imidazol-4-yl, 1-isopropyl-1*H*-pyrazol-4-yl or 1-isopropyl-1*H*-imidazol-4-yl;
and/or, Q is C(R^{q1})₃, C(O)R^{q1}, S(O)R^{q1}, SO₂R^{q1}, P(O)R^{q1}R^{q2}, or NR^{q1}R^{q2}, wherein R^{q1} and R^{q2} are each independently selected from methyl, ethyl, propyl, isopropyl, amino, acetyl, methylsulfonyl, acylamino, and aminoacyl, and R^{q1} and R^{q2} are preferably C₁₋₆ alkyl or C₁₋₆ alkoxy.

19. The compound of formula (I), or the pharmaceutically acceptable salt, the enantiomer, the diastereomer, the racemate, the solvate, the hydrate, the polymorph, the prodrug or the isotopic variant thereof, and the mixture thereof according to any one of claims 2-18, wherein, PIN represents: 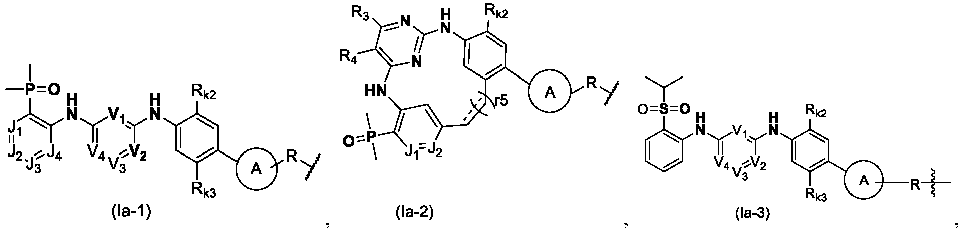 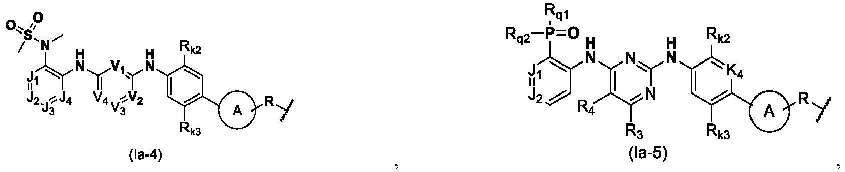 or 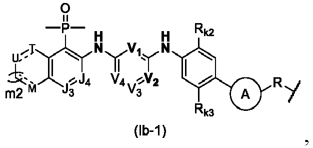 wherein V₁, V₂, V₃, V₄, J₁, J₂, J₃, J₄, Rₖ₂, Rₖ₃, R₃, R₄, ring A, and R are defined in any one of claims 2-18;
R₃ and R₄, together with the atoms to which they are attached, form 1*H*-pyrrole, wherein "---" is a single bond or absent; r5 is 0, 1, 2, 3, 4, or 5;
U, T, and M are each independently C, N, O, or S, and U, T, and M are each independently substituted, within a valence-permitted range, with 1 or 2 R⁰s, wherein R⁰ is selected from hydrogen, hydroxy, halogen, cyano, acetyl, C₁₋₅ alkyl (preferably C₁₋₃ alkyl), C₁₋₅ alkoxy (preferably C₁₋₃ alkoxy), C₁₋₅ haloalkyl (preferably C₁₋₃ haloalkyl), C₃₋₆ cycloalkyl, or 3- to 6-membered heterocycloalkyl; m₂ is 0, 1, or 2; preferably R⁰ is hydrogen or halogen (preferably fluorine or chlorine).

20. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 2, wherein, the compound of formula (I) is any one of the following compounds: 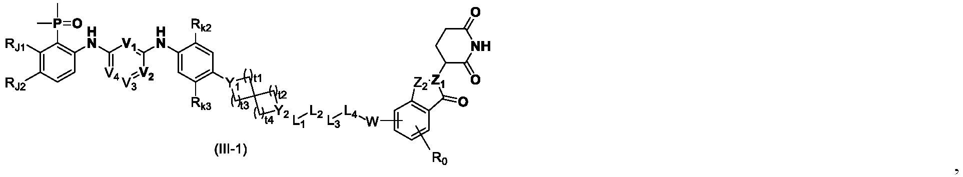 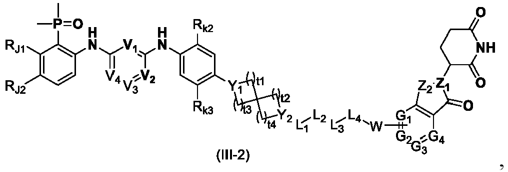 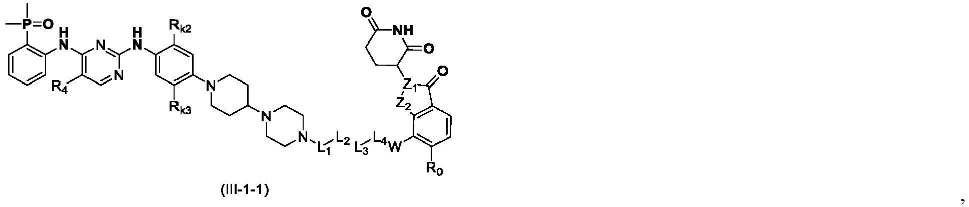 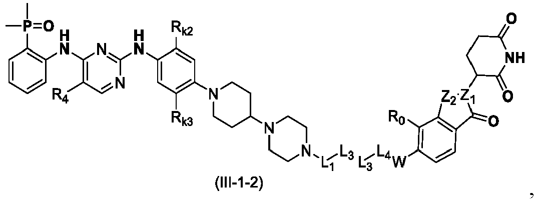 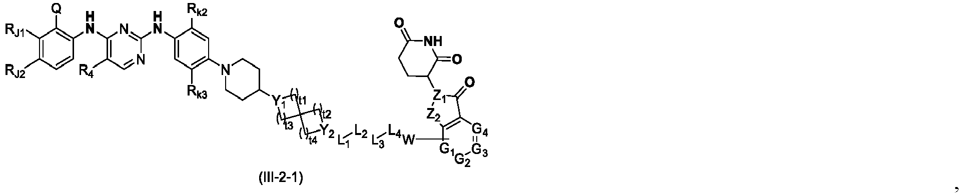 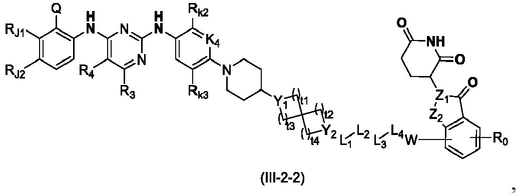 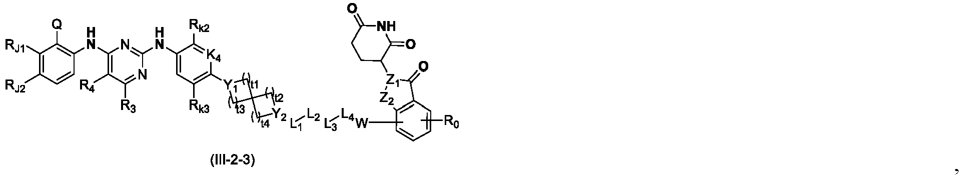 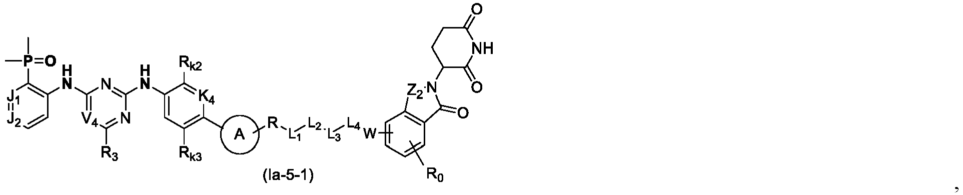 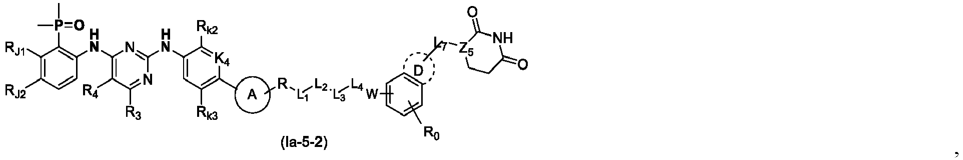 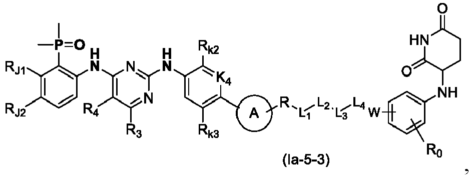 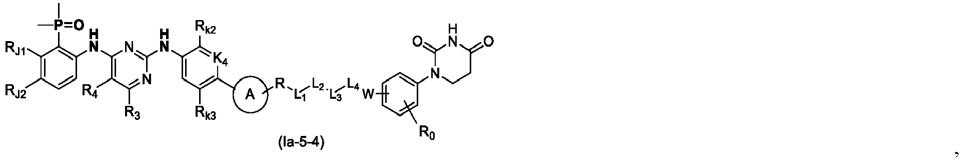 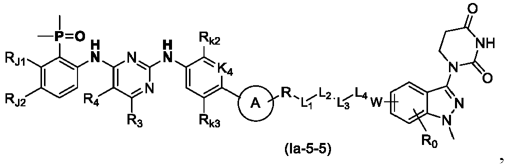 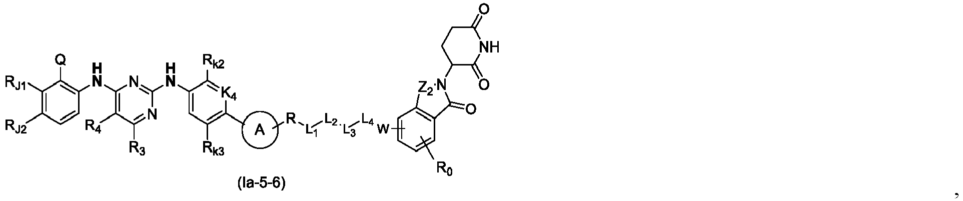 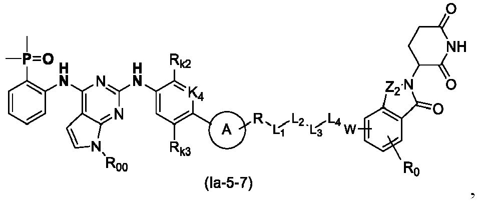 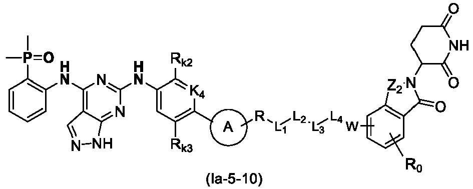 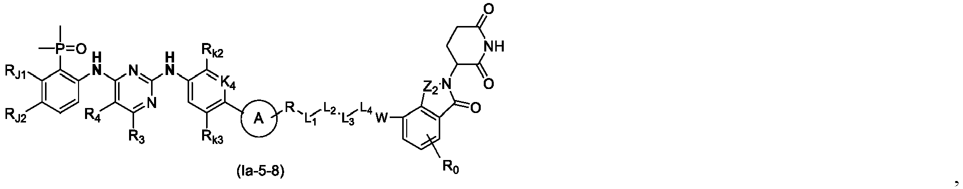 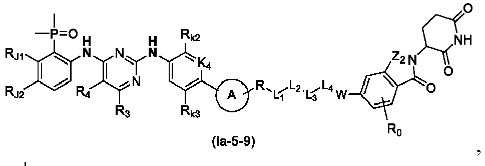 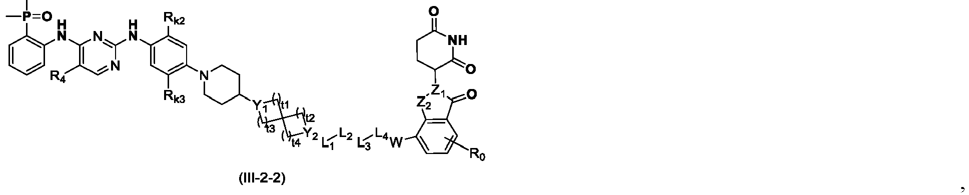 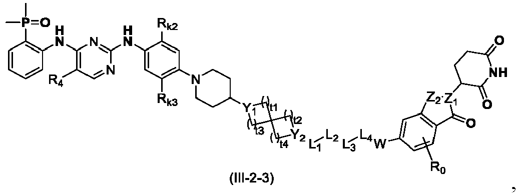 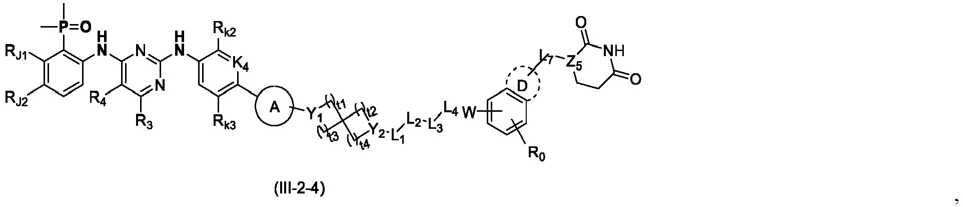 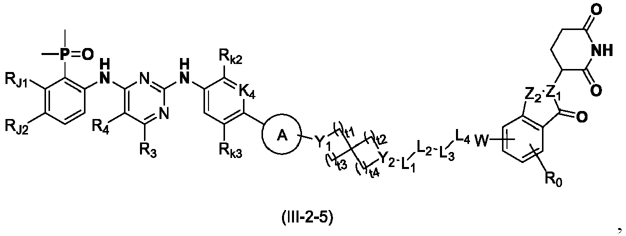 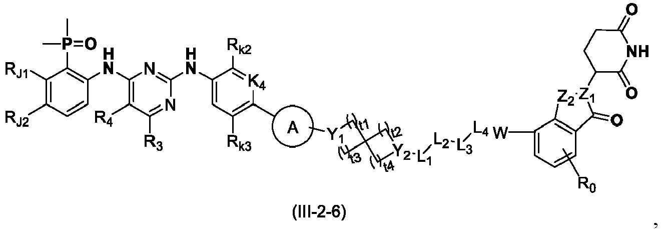 or 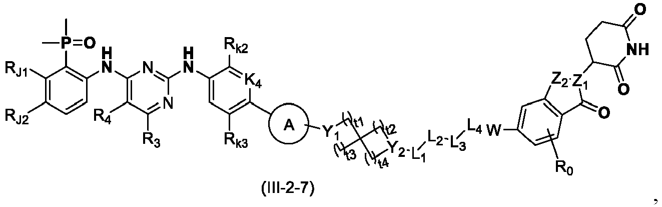 wherein
preferably, hydrogen of the compound of formula (I) may optionally be substituted by 1, 2, 3, 4, 5, or more deuterium.

21. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 2, wherein, the compound of formula (I) is the compound of formula (III-1-1) or (III-1-2): 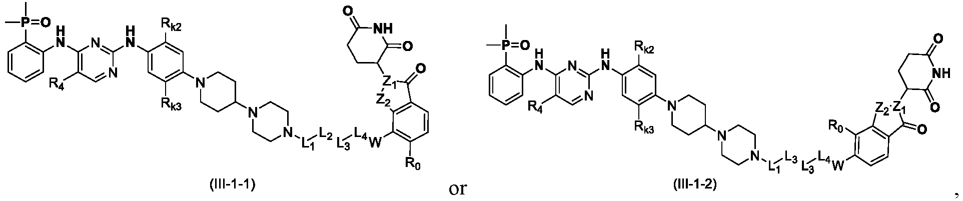 wherein
R₄ is halogen (preferably chlorine or bromine); Rₖ₂ is hydrogen or C₁₋₅ alkoxy (preferably C₁₋₃ alkoxy, more preferably methoxy);
Rₖ₃ is halogen, acetyl, cyano, C₁₋₅ alkyl, C₃₋₆ cycloalkyl, C₁₋₅ haloalkyl, or C₁₋₅ alkoxy;
L₁ is a bond, CH₂, CH₂CH₂, CH₂CH₂CH₂, CO, NH, CONH, NHCO, -N(methyl)-, O, C(O)O, OC(O), or (CR₂₁R₂₂)n₃, wherein R₂₁ and R₂₂ are each independently hydrogen or methyl, or R₂₁ and R₂₂, together with the C atom to which they are attached, form a C₃₋₆ cycloalkyl or 3- to 6-membered heterocycloalkyl, and n₃ is 0, 1, 2, or 3;
L₂ is a bond, CH₂, CH₂CH₂, CH₂CH₂CH₂, CO, NH, CONH, NHCO, O, C(O)O, OC(O), or (CR₂₁R₂₂)n₃, wherein R₂₁ and R₂₂ are each independently hydrogen or methyl, or R₂₁ and R₂₂, together with the C atom to which they are attached, form a C₃₋₆ cycloalkyl or 3- to 6-membered heterocycloalkyl, and n₃ is 0, 1, 2, or 3; L₃ is a bond, CH₂, CH₂CH₂, CH₂CH₂CH₂, CO, NH, CONH, NHCO, O, C(O)O, OC(O), or (CR₂₁R₂₂)n₃, wherein R₂₁ and R₂₂ are each independently hydrogen or methyl, or R₂₁ and R₂₂, together with the C atom to which they are attached, form a C₃₋₆ cycloalkyl or 3- to 6-membered heterocycloalkyl, and n₃ is 0, 1, 2, or 3; L₄ is a bond, CH₂, CH₂CH₂, CH₂CH₂CH₂, CO, NH, CONH, NHCO, O, C(O)O, OC(O), or (CR₂₁R₂₂)n₃, wherein R₂₁ and R₂₂ are each independently hydrogen or methyl, or R₂₁ and R₂₂, together with the C atom to which they are attached, form a C₃₋₆ cycloalkyl or 3- to 6-membered heterocycloalkyl, and n₃ is 0, 1, 2, or 3; Z₁ is CH or N, and Z₂ is CH₂ or C(O); W is CH₂, O, S, alkenylene, or C≡C; R₀ is H, D, halogen, hydroxy, cyano, C₁₋₃ alkyl, or C₁₋₃ alkoxy.

22. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 21, wherein,
R₄ is chlorine or bromine (preferably chlorine); Rₖ₂ is hydrogen or methoxy; Rₖ₃ is selected from fluorine, chlorine, bromine, acetyl, methyl, ethyl, isopropyl, propyl, cyclopropyl, -O-oxetanyl, trifluoromethyl, - CH₂CH₂F, -CHFCH₃, -CH₂CHF₂, -CF₂CH₃, -CHFCH₂F, -CH₂CF₃, -CF₂CH₂F, and -CHFCHF₂;
L₁, L₂, L₃, and L₄ are each independently a bond, CH₂, or CH₂CH₂ (preferably, L₁, L₂, L₃, and L₄ are each independently CH₂, more preferably, L₁ and L₂ are each a bond or CH₂, and L₃ and L₄ are CH₂); Z₁ is CH or N, Z₂ is CH₂ or C(O); W is CH₂, O, S, or C≡C (preferably CH₂ or C≡C, more preferably C≡C); R₀ is H, D, fluorine, chlorine, bromine, hydroxy, cyano, methyl, ethyl, propyl, or isopropyl (preferably hydrogen, more preferably F, even more preferably C₁₋₃ alkyl or C₁₋₃ haloalkyl).

23. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 2, wherein, the compound of formula (I) is the compound of formula (III-2-1) or (III-2-5): 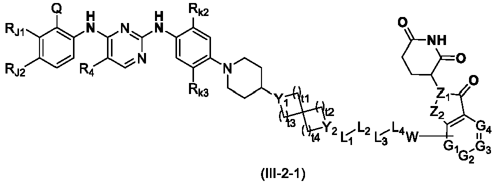 or 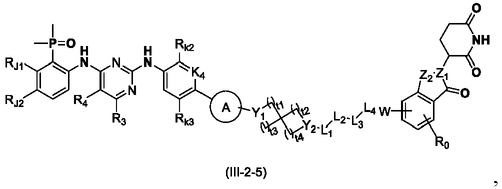 wherein
Q is P(O)(C₁₋₃ alkyl)₂ or N(C₁₋₃ alkyl)methylsulfonyl;
Y₁ is CH or N, Y₂ is CH or N, and t1, t2, t3 and t4 are each independently 1 or 2; R_{J1}, R_{J2}, R₄, R_{K2}, R_{K3}, L₁, L₂, L₃, L₄, W, G₁, G₂, G₃, G₄, Z₁, and Z₁ are defined in any one of claims 1-22; ring A is a 5- to 7-membered heterocyclylene or 3- to 8-membered cycloalkylene; R₀ is selected from hydrogen, deuterium, cyano, F, C₁₋₆ alkyl, or C₁₋₆ alkoxy.

24. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 23, wherein, the compound of formula (I) is the compound of formula (III-2-2) or (III-2-3): 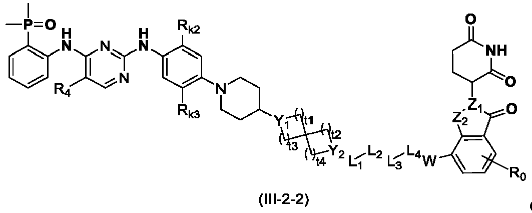 or 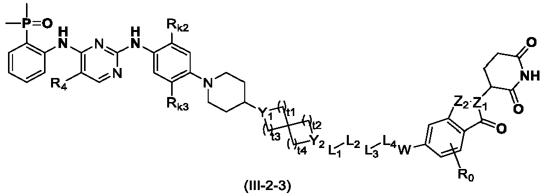
wherein R₄ is chlorine or bromine; Y₁ is N, Y₂ is CH, and t1, t2, t3 and t4 are each independently 1 or 2; Rₖ₂ is hydrogen, methoxy, ethoxy, or O-oxetanyl; Rₖ₃ is selected from fluorine, chlorine, bromine, cyano, acetyl, methyl, ethyl, isopropyl, propyl, cyclopropyl, -O-oxetanyl, trifluoromethyl, -CH₂CH₂F, -CHFCH₃, -CH₂CHF₂, -CF₂CH₃, -CHFCH₂F, -CH₂CF₃, -CF₂CH₂F, or -CHFCHF₂;
L₁, L₂, L₃, and L₄ are each independently a bond, CH₂, or CH₂CH₂ (preferably, L₁, L₂, L₃, and L₄ are each independently CH₂, more preferably, L₁ and L₂ are each a bond or CH₂, and L₃ and L₄ are CH₂, even more preferably, L₁, L₂, L₃, and L₄ are each independently a bond);
W is CH₂, O, or S (preferably O); Z₁ is CH or N, and Z₂ is CH₂ or C(O); R₀ is selected from hydrogen, fluorine, chlorine, and methyl.

25. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 2, wherein, the compound of formula (I) is the compound of formula (Ib-1) or (Ib-2): 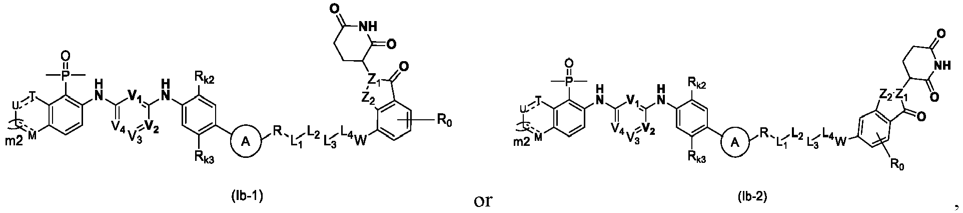 wherein
U, T, M, V₁, V₂, V₃, V₄, Rₖ₂, Rₖ₃, A, R, L₁, L₂, L₃, L₄, W, Z₁, Z₂, R₀, and m2 are defined in claim 22; preferably, T is O, S, or C; preferably, T is O or N; M is O, S, or C, and preferably M is O or N; U is C or N.

26. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 2, wherein,
E represents (II-i-1): 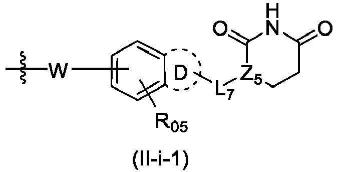
wherein L₇ is a bond or NH, and Z₅ is N or CH;
R₀₅ is hydrogen, halogen, cyano, acetyl, acylamino, C₁₋₃ alkyl, C₁₋₃ alkoxy, C₁₋₃ haloalkyl, or C₁₋₃ haloalkoxy; ring D is absent, L₇ is attached to a benzene ring, or ring D is selected from benzene, pyridine, pyrimidine, pyrazine, pyridazine, cyclohexane, oxocyclohexane, cyclopentane, tetrahydrofuran, tetrahydrothiophene, tetrahydropyrrole, furan, thiophene, pyrrole, pyrazole, imidazole, thiazole, isothiazole, oxazole, isoxazole, or triazole; and ring D is substituted, within a valence-permitted range, with 1, 2, or 3 substituents selected from hydrogen, C₁₋₃ alkyl, C₁₋₃ alkoxy, C₁₋₃ haloalkyl, C₁₋₃ haloalkoxy, C₃₋₆ cycloalkyl, C₃₋₆ heterocycloalkyl, or halogen; W is defined in any one of claims 1-25.

27. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 26, wherein, E is selected from: 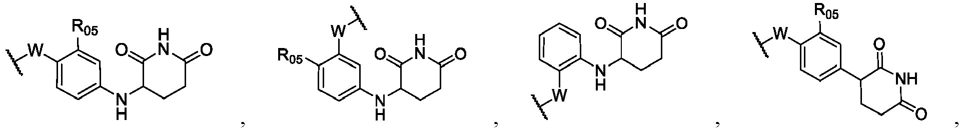 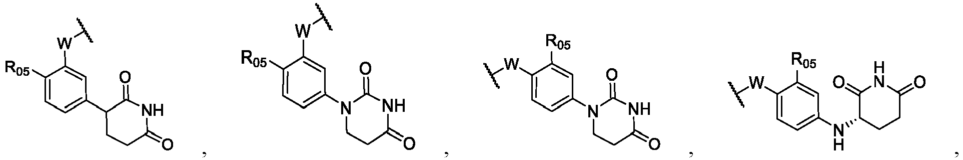 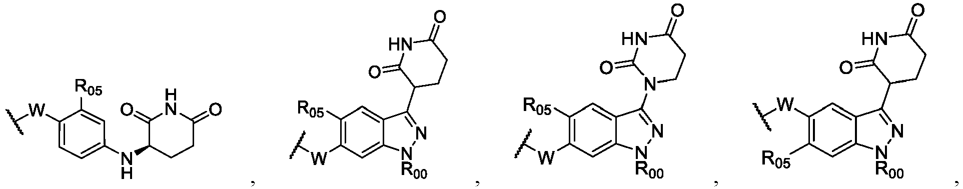 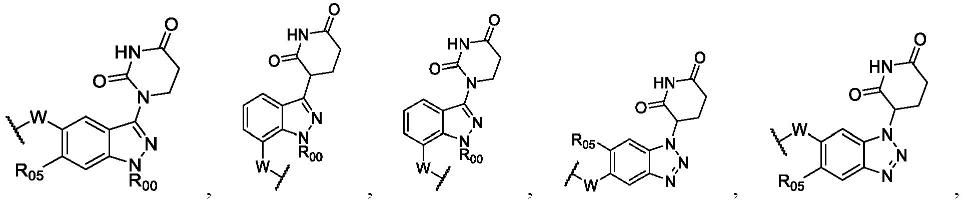 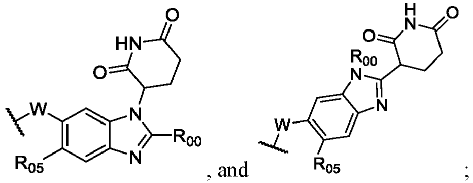 R₀₅ is hydrogen, fluorine, cyano, acetyl, acylamino, fluoromethyl, difluoromethyl, or trifluoromethyl; preferably R₀₅ is hydrogen, deuterium, C₁₋₆ alkyl, C₁₋₆ alkoxy, cyano, or C₁₋₆ haloalkyl; R₀₀ is hydrogen, methyl, ethyl, propyl, isopropyl, or cyclopropyl; W is CH₂, O, S, NH, -N(methyl)-, or -C≡C.

28. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 1 or 2, wherein the compound is selected from Table 1.

29. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 1 or 2, wherein,
Rₖ₃ is selected from 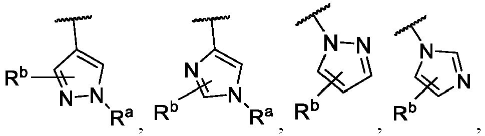 halogen, cyano, acetyl, C₁₋₅ alkyl, C₁₋₅ alkylene-OC₁₋₅ alkyl, and C₃₋₆ cycloalkyl, wherein the C₁₋₅ alkyl is optionally substituted with 0, 1, 2, 3, 4, or 5 substituents selected from deuterium, halogen, hydroxy, cyano, C₁₋₃ alkyl, C₁₋₃ haloalkyl, and C₁₋₃ alkoxy; R^{a} and R^{b} are H, F, Cl, Br, I, methyl, ethyl, propyl, isopropyl, methoxy, ethoxy, propoxy, isopropoxy, cyclopropyl, cyclobutyl, oxetanyl, fluoromethyl, difluoromethyl, trifluoromethyl, -CH₂CH₂F, -CHFCH₃, - CH₂CHF₂, -CF₂CH₃, -CHFCH₂F, -CH₂CF₃, -CF₂CH₂F, or -CHFCHF₂; R^{a} and R^{b} are H, F, Cl, Br, I, methyl, ethyl, propyl, isopropyl, methoxy, ethoxy, propoxy, isopropoxy, cyclopropyl, cyclobutyl, oxetanyl, fluoromethyl, difluoromethyl, trifluoromethyl, -CH₂CH₂F, -CHFCH₃, -CH₂CHF₂, -CF₂CH₃, -CHFCH₂F, - CH₂CF₃, -CF₂CH₂F, or -CHFCHF₂; preferably, Rₖ₃ is selected from 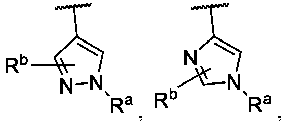 F, Cl, Br, I, cyano, acetyl, ethyl, C₁₋₅ alkylene-OC₁₋₅ alkyl, and C₃₋₆ cycloalkyl, wherein the C₁₋₅ alkyl is optionally substituted with 0, 1, 2, 3, 4, or 5 substituents selected from deuterium, hydroxy, cyano, C₁₋₃ alkyl, and C₁₋₃ alkoxy; R^{a} is H, methyl, ethyl, propyl, or isopropyl; R^{b} is H, methyl, ethyl, propyl, isopropyl, methoxy, ethoxy, propoxy, isopropoxy, cyclopropyl, cyclobutyl, oxetanyl, fluoromethyl, difluoromethyl, trifluoromethyl, - CH₂CH₂F, -CHFCH₃, -CH₂CHF₂, -CF₂CH₃, -CHFCH₂F, -CH₂CF₃, -CF₂CH₂F, or -CHFCHF₂; more preferably,
Rₖ₃ is selected from 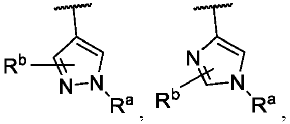 F, Cl, Br, I, cyano, acetyl, ethyl, C₁₋₅ alkylene-OC₁₋₅ alkyl, and C₃₋₆ cycloalkyl, wherein the C₁₋₅ alkyl is optionally substituted with 0, 1, 2, or 3 substituents selected from deuterium, hydroxy, cyano, C₁₋₃ alkyl, and C₁₋₃ alkoxy; R^{a} is H, methyl, ethyl, propyl, or isopropyl; R^{b} is H, methyl, ethyl, propyl, or isopropyl;
and/or, R₄ is hydrogen, hydroxy, cyano, C(O)NRₐR_{b}, halogen, C₁₋₁₀ alkyl substituted with 0, 1 or 2 R⁴¹s, or 5-to 6-membered heteroaryl substituted with 0, 1 or 2 R⁴¹s; the heteroatom of the 5- to 6-membered heteroaryl is selected from one or more of nitrogen, oxygen, and sulfur, and the number of the heteroatom is 1, 2, or 3; or, R₃ and R₄, together with the atoms to which they are attached, form a 5- to 6-membered heteroaryl or benzene substituted with 0, 1 or 2 R⁴¹s; the heteroatom of the 5- to 6-membered heteroaryl is selected from one or more of nitrogen, oxygen, and sulfur, and the number of the heteroatom is 1, 2, or 3; Rₐ and R_{b} are each independently hydrogen, cyano, acetyl, hydroxy, carboxy, nitro, halogen, C₁₋₁₀ alkyl, C₁₋₁₀ haloalkyl, C₁₋₁₀ alkoxy, C₁₋₁₀ alkylthio, NR_{c}R_{d}, C₃₋₈ cycloalkyl, 3- to 8-membered heterocycloalkyl, C₆₋₁₀ aryl, or 5- to 14-membered heteroaryl; or Rₐ and R_{b}, together with the N atom to which they are atttached, form a 3- to 8-membered heterocycloalkyl; the heteroatom of the 5- to 14-membered heteroaryl is selected from one or more of nitrogen, oxygen, and sulfur, and the number of heteroatom is 1, 2, or 3; the heteroatom of the 3- to 8-membered heterocycloalkyl is selected from one or more of nitrogen, oxygen, and sulfur, and the number of the heteroatom is 1, 2, or 3; R⁴¹ is selected from hydrogen, deuterium, halogen, C₁₋₆ alkyl, C₁₋₆ haloalkyl, C₁₋₆ alkoxy, C₁₋₆ alkylthio, C₃₋₈ cycloalkyl, 3- to 8-membered heterocyclyl, OC₃₋₈ cycloalkyl, and -O-3- to 8-membered heterocyclyl; the heteroatom of the 3- to 8-membered heterocycloalkyl is selected from one or more of nitrogen, oxygen, and sulfur, and the number of the heteroatom is 1, 2, or 3; preferably, R₄ is hydrogen, hydroxy, cyano, C(O)NRₐR_{b}, halogen, C₁₋₁₀ alkyl substituted with 0, 1 or 2 R⁴¹s, or 5- to 6-membered heteroaryl substituted with 0, 1 or 2 R⁴¹s; the heteroatom of the 5- to 6-membered heteroaryl is nitrogen and/or oxygen, and the number of the heteroatom is 1, 2, or 3; or R₃ and R₄, together with the atoms to which they are attached, form a 5- to 6-membered heteroaryl or benzene substituted with 0, 1, or 2 R⁴¹s; the heteroatom of the 5- to 6-membered heteroaryl is nitrogen and/or oxygen, and the number of the heteroatom is 1, 2, or 3; Rₐ and R_{b} are each independently hydrogen or C₁₋₅ alkyl; R⁴¹ is selected from hydrogen, deuterium, halogen, C₁₋₅ alkyl, C₁₋₅ haloalkyl, and C₁₋₅ alkoxy; more preferably, R₄ is hydrogen, halogen, or 5- to 6-membered heteroaryl; the heteroatom of the 5- to 6-membered heteroaryl is nitrogen, and the number of heteroatom is 1, 2, or 3; or, R₃ and R₄, together with the atoms to which they are attached, form a 5- to 6-membered heteroaryl or benzene; the heteroatom of the 5- to 6-membered heteroaryl is nitrogen, and the number of heteroatom is 1, 2, or 3; Rₐ and R_{b} are each independently hydrogen or C₁₋₁₀ alkyl;
and/or, ring A is a 3- to 8-membered monocyclic heterocyclylene or 7- to 16-membered spiroheterocyclylene; the heteroatom of the monocyclic heterocyclylene is selected from one or more of nitrogen, oxygen, and sulfur, and the number of the heteroatom is 1, 2, or 3; the heteroatom of the spiroheterocyclylene is selected from one or more of nitrogen, oxygen, and sulfur, and the number of the heteroatoms is 1, 2, or 3; preferably, ring A is a 3- to 6-membered monocyclic heterocyclylene or 7- to 11-membered spiroheterocyclylene; the heteroatom of the monocyclic heterocyclylene is nitrogen, oxygen, or sulfur, and the number of the heteroatom is 1, 2, or 3; the heteroatom of the spiroheterocyclylene is nitrogen, oxygen, or sulfur, and the number of the heteroatom is 1, 2, or 3; more preferably, ring A is a 3- to 8-membered monocyclic heterocyclylene or 7- to 11-membered spiroheterocyclylene; the heteroatom of the monocyclic heterocyclylene is N, and the number of heteroatom is 1 or 2; the heteroatom of the spiroheterocyclylene is N, and the number of heteroatom is 1 or 2;
and/or, R is a bond, or 7- to 11-membered mono-spiroheterocyclylene, 3- to 8-membered monocyclic heterocyclylene, and 8- to 10-membered bicyclic heteroarylene substituted with 0, 1, 2, or 3 R₀s, wherein R₀ is selected from hydrogen, deuterium, halogen, =O, C₁₋₆ alkyl, C₁₋₆ haloalkyl, C₁₋₆ alkoxy, C₃₋₈ cycloalkyl, and 3- to 8-membered heterocyclyl, or two R₀s, together with the C atom to which they are attached, form a 3- to 6-membered cycloalkyl; the heteroatom of the mono-spiroheterocyclylene is selected from one or more of nitrogen, oxygen, and sulfur, and the number of the heteroatom is 1, 2, or 3; the heteroatom of the bicyclic heteroarylene is selected from one or more of nitrogen, oxygen, and sulfur, and the number of the heteroatom is 1, 2, or 3; the heteroatom of the monocyclic heterocyclylene is selected from one or more of nitrogen, oxygen, and sulfur, and the number of the heteroatom is 1, 2, or 3; the heteroatom of the 3- to 8-membered heterocyclyl is selected from one or more of nitrogen, oxygen, and sulfur, and the number of the heteroatom is 1, 2, or 3; preferably, R is a bond, or 7- to 11-membered mono-spiroheterocyclylene, 3- to 8-membered monocyclic heterocyclylene, and 8- to 10-membered bicyclic heteroarylene substituted with 0, 1, 2, or 3 R₀s; R₀ is selected from hydrogen, =O and C₁₋₆ alkyl, or two R₀s, together with the C atom to which they are attached, form a 3- to 6-membered cycloalkyl; the heteroatom of the bicyclic heteroarylene is nitrogen, oxygen, or sulfur, and the number of the heteroatom is 1, 2, or 3; the heteroatom of the spiroheterocyclylene is nitrogen, oxygen, or sulfur, and the number of the heteroatom is 1, 2, or 3; the heteroatom of the monocyclic heterocyclylene is nitrogen, oxygen, or sulfur, and the number of the heteroatom is 1, 2, or 3; more preferably, R is a bond, or 7- to 11-membered mono-spiroheterocyclylene, 5- or 6-membered monocyclic heterocyclylene, and 8- to 10-membered bicyclic heteroarylene substituted with 0, 1, 2, or 3 R₀s; R₀ is selected from hydrogen, =O, and C₁₋₆ alkyl, or two R₀s, together with the C atom to which they are attached, form a 3- to 6-membered cycloalkyl; the heteroatom of the mono-spiroheterocyclylene is N, and the number of the heteroatom is 1 or 2; the heteroatom of the bicyclic heteroarylene is N, and the number of the heteroatom is 1 or 2; the heteroatom of the 6-membered monocyclic heterocyclylene is N, and the number of the heteroatom is 1 or 2;
and/or, Linker represents: 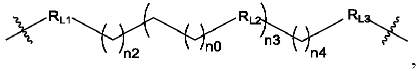 wherein R_{L1} and R_{L3} are each independently O, S, CO, SO, SO₂, N(R₀₀), or (CR₂₁R₂₂)ᵣ; R₂₁ and R₂₂ are hydrogen, acetyl, halogen, amino, C₁₋₁₀ alkyl, C₁₋₁₀ haloalkyl, C₁₋₁₀ alkoxy, C₂₋₁₀ alkenyl, C₂₋₁₀ alkynyl, C₃₋₈ cycloalkyl, 3- to 8-membered heterocycloalkyl, C₆₋₁₀ aryl, or 5- to 14-membered heteroaryl; or R₂₁ and R₂₂, together with the C atom to which they are attached, form a 3- to 8-membered cycloalkyl or 3- to 8-membered heterocycloalkyl; R_{L2}s are identical or different and R_{L2} is O, S, CO, N(R₀₀), C(O)O, OC(O), C(O)NH, NHC(O) or NHC(O)NH, phenylene, alkynylene, cyclopropylene, 1,4-piperazinylene, or triazolylene; R₀₀ is hydrogen or C₁₋₁₀ alkyl, and n2 and n4 are each independently 0, 1, 2, 3, 4, 5, 6, 7, 8, 9, or 10; r, n0, and n3 are 0, 1, 2, 3, or 4; preferably, Linker represents: 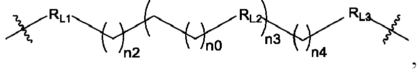 wherein R_{L1} and R_{L3} are each independently O, CO, N(R₀₀), or (CR₂₁R₂₂)ᵣ, and R₂₁ and R₂₂ are hydrogen, acetyl, C₁₋₅ alkyl, C₆₋₁₀ aryl, or C₁₋₅ haloalkyl; or,
R₂₁ and R₂₂, together with the C atom to which they are attached, form a 3- to 8-membered cycloalkyl; R_{L2}s are identical or different, and R_{L2} is O, CO, N(R₀₀), alkynylene 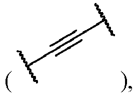 or cyclopropylene; R₀₀ is hydrogen or C₁₋₅ alkyl, and n2 and n4 are each independently 0, 1, 2, or 3; r, n0, and n3 are 0, 1, 2, 3, or 4; more preferably, Linker represents: 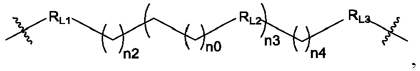 wherein R_{L1} and R₁₃ are each independently O, N(R₀₀), or (CR₂₁R₂₂)ᵣ, and R₂₁ and R₂₂ are hydrogen or C₁₋₅ alkyl; or, R₂₁ and R₂₂, together with the C atom to which they are attached, form a 3- to 8-membered cycloalkyl; R_{L2}s are identical or different, and R_{L2} is O, CO, N(R₀₀), alkynylene 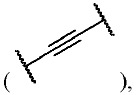 alkenylene 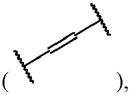 or cyclopropylene 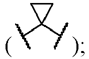 R₀₀ is hydrogen or C₁₋₅ alkyl, and n2 and n4 are each independently 0, 1, 2, or 3; r, n0, and n3 are 0, 1, 2, 3, or 4; even more preferably, Linker represents: 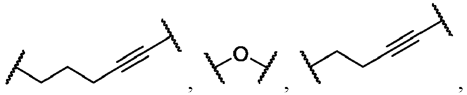 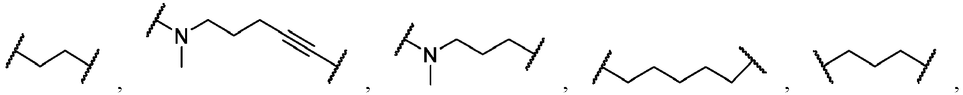 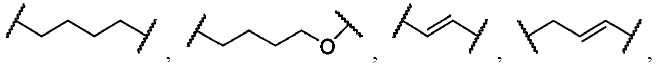 methylene, 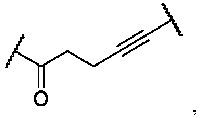 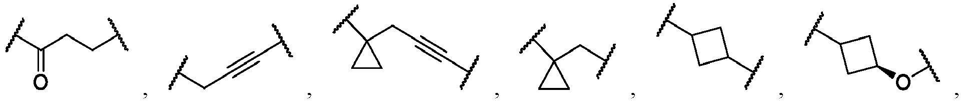 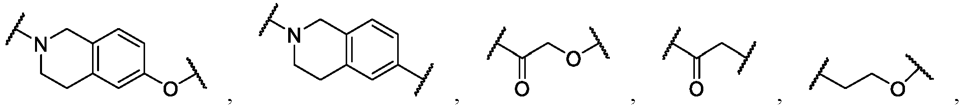 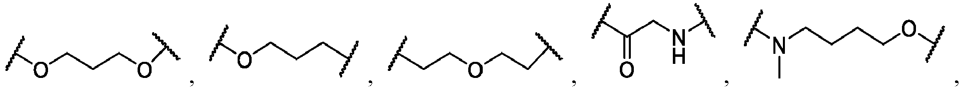 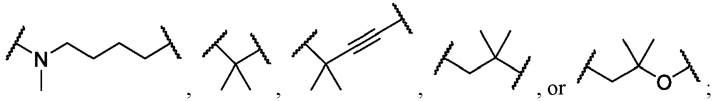
and/or, R_{J1}, R_{J3}, and R_{J4} are hydrogen;
and/or, R_{J2} is selected from hydrogen, F, Cl, Br, and C₁₋₅ alkyl;
and/or, R_{J3} and R_{J4} are hydrogen;
and/or, R_{J1} and R_{J2} are each independently selected from hydrogen, F, Cl, Br, and C₁₋₅ alkyl;
and/or, V₃ is N or CR³, R₃ is hydrogen, or R₃ and R₄, together with the atoms to which they are attached, form a 5- to 6-membered heteroaryl or benzene substituted with 0, 1, or 2 R⁴¹s; the heteroatom of the 5- to 6-membered heteroaryl is nitrogen, and the number of the heteroatom is 1, 2, or 3; V₃ is N or CR³;
and/or, W is O or -C≡C-;
and/or, Q is P(O)R^{q1}R^{q2}, wherein R^{q1} and R^{q2} are each independently C₁₋₆ alkyl or C₁₋₆ alkoxy;
and/or, V₄ is N or CR₄, wherein R₄ is hydrogen, hydroxy, cyano, C(O)NRₐR_{b}, halogen, C₁₋₁₀ alkyl substituted with 0, 1, or 2 R⁴¹s, or 5- to 6-membered heteroaryl substituted with 0, 1, or 2 R⁴¹s; the heteroatom of the 5-to 6-membered heteroaryl is selected from one or more of nitrogen, oxygen, and sulfur, and the number of the heteroatom is 1, 2, or 3.

30. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 2, wherein,
Rₖ₃ is selected from 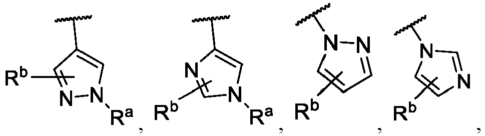 halogen, cyano, acetyl, C₁₋₅ alkyl, C₁₋₅ alkylene-OC₁₋₅ alkyl, or C₃₋₆ cycloalkyl, wherein the C₁₋₅ alkyl is optionally substituted with 0, 1, 2, 3, 4, or 5 substituents selected from deuterium, halogen, hydroxy, cyano, C₁₋₃ alkyl, C₁₋₃ haloalkyl, and C₁₋₃ alkoxy; R^{a} and R^{b} are H, F, Cl, Br, I, methyl, ethyl, propyl, isopropyl, methoxy, ethoxy, propoxy, isopropoxy, cyclopropyl, cyclobutyl, oxetanyl, fluoromethyl, difluoromethyl, trifluoromethyl, -CH₂CH₂F, -CHFCH₃, - CH₂CHF₂, -CF₂CH₃, -CHFCH₂F, -CH₂CF₃, -CF₂CH₂F, or -CHFCHF₂; R^{a} and R^{b} are H, F, Cl, Br, I, methyl, ethyl, propyl, isopropyl, methoxy, ethoxy, propoxy, isopropoxy, cyclopropyl, cyclobutyl, oxetanyl, fluoromethyl, difluoromethyl, trifluoromethyl, -CH₂CH₂F, -CHFCH₃, -CH₂CHF₂, -CF₂CH₃, -CHFCH₂F, - CH₂CF₃, -CF₂CH₂F, and -CHFCHF₂;
Q, V₁, V₂, V₃, V₄, J₁, J₂, J₃, J₄, K₁, K₂, K₄, R, L₅, L₆, ring A, E, and Linker are defined in any one of claims 1-29.

31. The compound of formula (I), or the pharmaceutically acceptable salt, the enantiomer, the diastereomer, the racemate, the solvate, the hydrate, the polymorph, the prodrug or the isotopic variant thereof, and the mixture thereof according to claim 2, wherein,
R is a bond, or a 7- to 11-membered mono-spiroheterocyclylene, 3- to 8-membered monocyclic heterocyclylene, and 8- to 10-membered bicyclic heteroarylene substituted with 0, 1, 2, or 3 R₀s, wherein R₀ is selected from hydrogen, deuterium, halogen, =O, C₁₋₆ alkyl, C₁₋₆ haloalkyl, C₁₋₆ alkoxy, C₃₋₈ cycloalkyl, and 3- to 8-membered heterocyclyl, or two R₀s, together with the C atom to which they are attached, form a 3- to 6-membered cycloalkyl; the heteroatom of the mono-spiroheterocyclylene is selected from one or more of nitrogen, oxygen, and sulfur, and the number of the heteroatom is 1, 2, or 3; the heteroatom of the bicyclic heteroarylene is selected from one or more of nitrogen, oxygen, and sulfur, and the number of the heteroatom is 1, 2, or 3; the heteroatom of the monocyclic heterocyclylene is selected from one or more of nitrogen, oxygen, and sulfur, and the number of the heteroatom is 1, 2, or 3; the heteroatom of the 3- to 8-membered heterocyclyl is selected from one or more of nitrogen, oxygen, and sulfur, and the number of the heteroatom is 1, 2, or 3;
Q, V₁, V₂, V₃, V₄, J₁, J₂, J₃, J₄, K₁, K₂, K₄, Rₖ₃, L₅, L₆, ring A, E and Linker are defined in any one of claims 1-29.

32. A compound of formula (1c-1), (1e-1), (1c-1-1), (1e-1-1), (1c-1-2), or (1e-1-2): 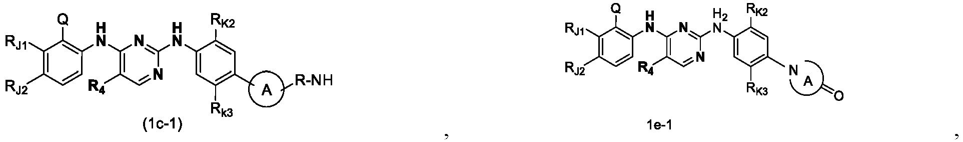 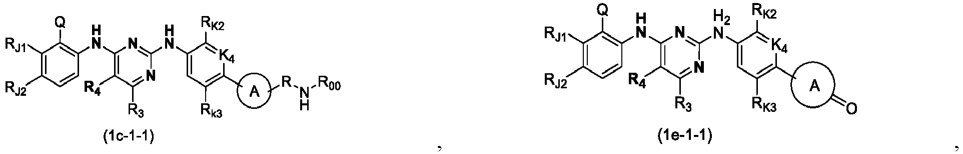 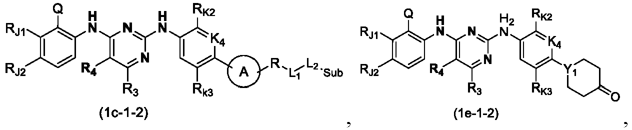 wherein, in formula (1c-1), when R is a bond, NH is NH on ring A; when R is not a bond, NH is NH on ring R;
Sub is selected from OH, SH, C(O)OH, S(O)OH, S(O)₂OH, NH, or a leaving group selected from fluorine, chlorine, bromine, iodine, boronic acid, boronate, -OMs or -OTF;
the variables in the formula are defined in any one of claims 1-29.

33. A pharmaceutical composition, comprising the compound of formula (I), or the pharmaceutically acceptable salt, the enantiomer, the diastereomer, the racemate, the solvate, the hydrate, the polymorph, the prodrug or the isotopic variant thereof, and the mixture thereof according to any one of claims 1-31, and a pharmaceutically acceptable excipient, wherein preferably, the pharmaceutical composition further comprises an additional therapeutic agent.

34. Use of the compound of formula (I), or the pharmaceutically acceptable salt, the enantiomer, the diastereomer, the racemate, the solvate, the hydrate, the polymorph, the prodrug or the isotopic variant thereof, and the mixture thereof according to any one of claims 1-31, or the pharmaceutical composition according to claim 33, in preparing a protein inhibitor or degrading agent, wherein the protein of the protein inhibitor or degrading agent is selected from at least one of EGFR, ROS1, or ALK.

35. Use of the compound of formula (I), or the pharmaceutically acceptable salt, the enantiomer, the diastereomer, the racemate, the solvate, the hydrate, the polymorph, the prodrug or the isotopic variant thereof, and the mixture thereof according to any one of claims 1-31, in preparing a medicament for treating and/or preventing a cancer.

36. The use according to claim 35, wherein the cancer is selected from lung cancer; lymphoma; inflammatory myofibroblastoma; colorectal cancer; cerebral glioma; astroblastoma; ovarian cancer; bone marrow cancer; transplantation-related cancer; neutropenia; leukemia; Wagner-Unverricht syndrome; bronchial carcinoma; prostate cancer; breast cancer; thyroid cancer; pancreatic cancer; neuroblastoma; extramedullary plasmacytoma; plasmacytoma; gastric cancer; gastrointestinal stromal tumor; esophageal cancer; large intestine adenocarcinoma; esophageal squamous cell carcinoma; liver cancer; renal cell carcinoma; bladder cancer; endometrial cancer; melanoma; brain cancer; oral cancer; sarcoma; a tumor resistant to targeted therapies; or a tumor or disease dependent on ALK, ROS1 or EGFR, or any mutein thereof.

37. The use according to claim 35, wherein the cancer is selected from small cell lung cancer; non-small cell lung cancer; diffuse large B-cell lymphoma; non-Hodgkin's lymphoma; anaplastic lymphoma; anaplastic large cell lymphoma; CD20-positive lymphoma; primary lymphoma; B cell lymphoma; recurrent B-cell non-Hodgkin's lymphoma; recurrent diffuse large B-cell lymphoma; recurrent mediastinal (thymic) large B-cell lymphoma; primary mediastinal (thymic) large B-cell lymphoma; recurrent transformed non-Hodgkin's lymphoma; refractory B-cell non-Hodgkin's lymphoma; refractory diffuse large B-cell lymphoma; refractory primary mediastinal (thymic) large B-cell lymphoma; refractory transformed non-Hodgkin's lymphoma; multiple myeloma; myelodysplastic syndrome (MDS); previously treated myelodysplastic syndrome; plasma cell myeloma; smoldering myeloma; smoldering multiple myeloma; myelofibrosis; acute myeloid leukemia (AML); leukemia-associated anemia; chronic granulocytic leukemia; B cell chronic lymphocytic leukemia; Wagner-Unverricht syndrome; bronchial carcinoma; prostate cancer; triple-negative breast cancer; sporadic breast cancer; a patient with Cowden disease; thyroid cancer; pancreatic cancer; neuroblastoma; extramedullary plasmacytoma; plasmacytoma; gastric cancer; gastrointestinal stromal tumor; esophageal cancer; large intestine adenocarcinoma; esophageal squamous cell carcinoma; liver cancer; renal cell carcinoma; bladder cancer; endometrial cancer; melanoma; brain cancer; oral cancer; rhabdomyosarcoma; various adipose-derived tumors; Ewing sarcoma/primitive neuroectodermal tumor (Ewing/PNET); leiomyosarcoma; or a tumor resistant to EGFR-, ROS1- or ALK-targeted therapies.

38. The use of claim 35, wherein the cancer is selected from anaplastic lymphoma kinase (ALK) mutationpositive non-small cell lung cancer (NSCLC); ROS1-positive non-small cell lung cancer; EGFR mutant non-small cell lung cancer; lung adenocarcinoma; lung cancer resistant to EGFR-, ROS1-, or ALK-targeted therapies; lymphoma resistant to ALK-targeted therapies; or the following tumor, cancer or disease dependent on a protein selected from ALK, ROS 1 or EGFR, or any mutein thereof: lung cancer, lymphoma, inflammatory myofibroblastoma, colorectal cancer, cerebral glioma, astroblastoma, ovarian cancer, leukemia, breast cancer, thyroid cancer, neuroblastoma, extramedullary plasmacytoma, plasmacytoma, esophageal squamous cell carcinoma, renal cell carcinoma, bronchial carcinoma, prostate cancer, breast cancer, thyroid cancer, pancreatic cancer, neuroblastoma, extramedullary plasmacytoma, plasmacytoma, gastric cancer, gastrointestinal stromal tumor, esophageal cancer, large intestine adenocarcinoma, esophageal squamous cell carcinoma, liver cancer, renal cell carcinoma, bladder cancer, endometrial cancer, melanoma, brain cancer, oral cancer, or sarcoma.

39. A compound, wherein the compound is any one of the compounds in Table 2 or a salt thereof.
